# EUROPEAN PATENT APPLICATION

(11) **EP 4 137 584 A1**
(43) Date of publication of application: **22.02.2023**
(21) Application number: 22188159.2
(22) Date of filing: 29.09.2017
(51) Int. Cl.: C12Q 1/6883

(54) **ASSAY FOR DISTINGUISHING BETWEEN SEPSIS AND SYSTEMIC INFLAMMATORY RESPONSE SYNDROME**

(30) Priority: 29.09.2016 GB 201616557
(62) Divisional of application: 17780869.8
(71) Applicant: Secretary of State for Health and Social Care, London SW1H 0EU (GB); University College Cardiff Consultants Limited, Cardiff CF24 0DE (GB)
(72) Inventor: HALL, Judith, Cardiff CF5 6JH (GB); SZAKMANY, Tamas, Cardiff CF5 2HW (GB); SHAH, Sanjoy, Bristol BS6 6QP (GB); KEMPSELL, Karen, Salisbury SP4 0JG (GB); BALL, Graham, Nottingham NG11 8NS (GB)
(74) Representative: MacLean, Martin Robert

(57) **Abstract**

There is provided a method for distinguishing between sepsis and systemic inflammatory response syndrome (SIRS) in a patient, comprising:
(i) determining the amount of one or more biomarker for sepsis, and one or more biomarker for SIRS in a sample obtained from a patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124,
(ii) comparing the amount of the one or more biomarker for sepsis determined in said sample in (i) to a corresponding reference value representative of a healthy individual,
(iii) comparing the amount of the one or more biomarker for SIRS determined in said sample in (i) to a corresponding reference value representative of a healthy individual;

wherein the patient is diagnosed as having sepsis, when an increase is observed in the one or more biomarker for sepsis, and no increase is observed in the one or more biomarker for SIRS, in the sample obtained from the patient relative to the corresponding reference value; and
wherein the patient is diagnosed as having SIRS, when an increase is observed in the one or more biomarker for SIRS, and no increase is observed in the one or more biomarker for sepsis, in the sample obtained from the patient relative to the corresponding reference value.

## Description

The present invention relates to one or more biomarkers associated with systemic inflammatory conditions, such as Severe Inflammatory Response Syndrome (SIRS) and sepsis. More particularly, the invention relates to methods for diagnosing, monitoring and prognosing systemic inflammatory conditions, such as Severe Inflammatory Response Syndrome (SIRS), sepsis, abdominal sepsis and pulmonary sepsis, and for distinguishing between sepsis and SIRS in a patient.

Systemic inflammatory conditions such as Severe Inflammatory Response Syndrome (SIRS) and sepsis are life-threatening conditions that can result in organ failure and death.

Sepsis (or blood poisoning) is characterised by a systemic host response to infection. Sepsis affects approximately 25% of intensive care patients, and is estimated to cause over 37,000 deaths in the UK every year, with a mortality rate of between 28% and 50%. Diagnosis of sepsis is typically performed using culture-based methods, involving microbial growth followed by taxonomic identification of the pathogen. However, these culture-based techniques are time-consuming, taking over 24 hours to obtain results, and have poor sensitivity and specificity. Other more recently developed diagnostic methods involve assessment of single blood protein biomarkers such as CRP and pro-calcitonin. These methods allow quicker diagnosis, but there is growing evidence that these markers suffer from poor specificity. Furthermore, none of the available methods provide an insight into the underlying origin or aetiology of the disease, nor do they provide any way of predicting recovery from sepsis or the likelihood of progression to severe sepsis or septic shock. Treatment of sepsis typically involves administration of antimicrobials such as broad-spectrum antibiotics together with intravenous fluids. It is estimated that mortality increases by approximately 5% for every hour that treatment is delayed. Rapid diagnosis and initiation of treatment of patients having sepsis is therefore essential.

Severe Inflammatory Response Syndrome (SIRS) is also characterised by a systemic host response. However, this condition does not result from infection, but instead results from injury or trauma. Clinical symptoms of SIRS are similar to sepsis, and thus it can be difficult to distinguish between patients in the early stages of sepsis and patients who have infection-negative systemic inflammation (SIRS). Making an incorrect distinction between these two conditions has both clinical and economic implications, including inappropriate patient management, and unnecessary over-prescription of antibiotics. There is currently no clinical test available to distinguish between sepsis and non-infective SIRS. Development of a method for effectively stratifying SIRS and sepsis patients would therefore be beneficial, allowing sepsis patients to receive early, effective and aggressive treatment with antimicrobials and supportive care, and reducing the unnecessary exposure of SIRS patients to antibiotics.

There is therefore a need to provide improved ways to diagnose, monitor and/or prognose patients that have or are at risk of developing systemic inflammatory conditions (such as sepsis and SIRS) in order to facilitate early intervention and appropriate treatment. In particular, there is a need to provide for effective ways of distinguishing between sepsis and SIRS in patients in order to ensure that an appropriate treatment regimen is selected.

### SUMMARY OF THE INVENTION

By conducting extensive investigations into expression patterns associated with systemic inflammatory conditions, the present inventors have identified biomarkers that may be used to evaluate various aspects of systemic inflammatory conditions, such as SIRS and sepsis. The biomarkers may be used to diagnose the presence (or absence) of a systemic inflammatory condition, and to distinguish between different types of systemic inflammatory conditions in a patient. The biomarkers may also be used to monitor a patient having a systemic inflammatory condition, and to determine whether a patient is suitable for discharge from medical care. The present invention therefore provides a solution to one or more of the above mentioned problems.

In one aspect, the present invention provides a method for distinguishing between sepsis and systemic inflammatory response syndrome (SIRS) in a patient, comprising:
(i) determining the amount of one or more biomarker for sepsis, and one or more biomarker for SIRS in a sample obtained from a patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124,
(ii) comparing the amount of the one or more biomarker for sepsis determined in said sample in (i) to a corresponding reference value representative of a healthy individual,
(iii) comparing the amount of the one or more biomarker for SIRS determined in said sample in (i) to a corresponding reference value representative of a healthy individual;
wherein the patient is diagnosed as having sepsis, when an increase is observed in the one or more biomarker for sepsis, and no increase is observed in the one or more biomarker for SIRS, in the sample obtained from the patient relative to the corresponding reference value; and wherein the patient is diagnosed as having SIRS, when an increase is observed in the one or more biomarker for SIRS, and no increase is observed in the one or more biomarker for sepsis, in the sample obtained from the patient relative to the corresponding reference value.

In a related aspect, the present invention provides a method for distinguishing between sepsis and systemic inflammatory response syndrome (SIRS) in a patient, comprising:
(a) diagnosing a patient as having a systemic inflammatory condition by performing a method comprising:
   (i) determining the amount of one or more biomarker in a sample obtained from the patient, wherein the one or more biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1,
   (ii) comparing the amount of the one or more biomarker determined in said sample in (i) to a corresponding reference value to determine that the patient has a systemic inflammatory condition; and
(b) determining whether the patient diagnosed as having a systemic inflammatory condition has sepsis or SIRS by performing a method comprising:
   (i) determining the amount of one or more biomarker for sepsis, and one or more biomarker for SIRS in a sample obtained from the patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124,
   (ii) comparing the amount of the one or more biomarker for sepsis determined in said sample in (i) to a corresponding reference value representative of a healthy individual,
   (iii) comparing the amount of the one or more biomarker for SIRS determined in said sample in (i) to a corresponding reference value representative of a healthy individual;
   wherein the patient is diagnosed as having sepsis, when an increase is observed in the one or more biomarker for sepsis, and no increase is observed in the one or more biomarker for SIRS, in the sample obtained from the patient relative to the corresponding reference value; and wherein the patient is diagnosed as having SIRS, when an increase is observed in the one or more biomarker for SIRS, and no increase is observed in the one or more biomarker for sepsis, in the sample obtained from the patient relative to the corresponding reference value.

In a related aspect, the present invention provides a method for distinguishing between sepsis and systemic inflammatory response syndrome (SIRS) in a patient diagnosed as having a systemic inflammatory condition, comprising:
(i) determining the amount of one or more biomarker for sepsis in a sample obtained from a patient, wherein the one or more biomarker for sepsis is selected from the group consisting of: ITGB3, ITGA2B, MYL9, LCN2, TREML1, LCN15, CMTM5, PPBP, and PF4,
(ii) comparing the amount of the one or more biomarker for sepsis determined in said sample in (i) to a corresponding reference value representative of a healthy individual;
wherein the patient is diagnosed as having sepsis, when an increase is observed in the one or more biomarker for sepsis in the sample obtained from the patient relative to the corresponding reference value; and wherein the patient is diagnosed as having SIRS, when no increase is observed in the one or more biomarker for sepsis, in the sample obtained from the patient relative to the corresponding reference value.

In a related aspect, the present invention provides a method for distinguishing between sepsis and systemic inflammatory response syndrome (SIRS) in a patient diagnosed as having a systemic inflammatory condition, comprising:
(i) determining the amount of one or more biomarker for SIRS in a sample obtained from a patient, wherein the one or more biomarker for SIRS is selected from the group consisting of: PLA2G7, ARHGEF10L, MYCL, TGFBI, and GPR124,
(ii) comparing the amount of the one or more biomarker for SIRS determined in said sample in (i) to a corresponding reference value representative of a healthy individual;
wherein the patient is diagnosed as having SIRS, when an increase is observed in the one or more biomarker for SIRS in the sample obtained from the patient relative to the corresponding reference value; and wherein the patient is diagnosed as having sepsis, when no increase or a decrease is observed in the one or more biomarker for SIRS, in the sample obtained from the patient relative to the corresponding reference value.

In a further related aspect, the present invention provides the use of one or more of: ITGB3, ITGA2B, MYL9, LCN2, TREML1, LCN15, CMTM5, PPBP, and PF4 and/or one or more of: PLA2G7, ARHGEF10L, MYCL, TGFBI, and GPR124 for distinguishing between sepsis and SIRS in a patient.

In a further aspect, the present invention provides a method for diagnosing whether a patient has a systemic inflammatory condition, comprising:
(i) determining the amount of FAM20A and OLAH in a sample obtained from a patient,
(ii) comparing the amount of FAM20A determined in said sample in (i) to a corresponding reference value representative of a healthy individual,
(iii) comparing the amount of OLAH determined in said sample in (i) to a corresponding reference value representative of a healthy individual;
wherein the patient is diagnosed as having a systemic inflammatory condition, when an increase is observed in FAM20A and OLAH in the sample obtained from the patient relative to the corresponding reference value; and wherein the patient is diagnosed as not having a systemic inflammatory condition, when no increase is observed in FAM20A and OLAH, in the sample obtained from the patient relative to the corresponding reference value.

In a further related aspect, the present invention provides the use of FAM20A and OLAH for diagnosing a systemic inflammatory condition in a patient.

In a further aspect, the present invention provides a method for diagnosing whether a patient has abdominal sepsis, comprising:
(i) determining the amount of one or more biomarker in a sample obtained from a patient, wherein the one or more biomarker is selected from the group consisting of: SLC39A8, CIQC, CIQA, MRAS, TMEM37, CIQB, PCOLCE2, KIF2C, TNF, IFI44, IFIT1, and RPGRIP1,
(ii) comparing the amount of the one or more biomarker determined in said sample in (i) to a corresponding reference value; and thereby determining whether the patient has abdominal sepsis.

In a related aspect, the present invention provides a method for diagnosing whether a patient has abdominal sepsis, comprising:
(a) diagnosing a patient as having sepsis by performing a method to distinguish between sepsis and SIRS in the patient, comprising:
   (i) determining the amount of one or more biomarker for sepsis, and one or more biomarker for SIRS in a sample obtained from the patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124,
   (ii) comparing the amount of the one or more biomarker for sepsis determined in said sample in (i) to a corresponding reference value representative of a healthy individual,
   (iii) comparing the amount of the one or more biomarker for SIRS determined in said sample in (i) to a corresponding reference value representative of a healthy individual; wherein the patient is diagnosed as having sepsis, when an increase is observed in the one or more biomarker for sepsis, and no increase is observed in the one or more biomarker for SIRS, in the sample obtained from the patient relative to the corresponding reference value; and
(b) determining whether the patient diagnosed as having sepsis has abdominal sepsis by perfoming a method comprising:
   (i) determining the amount of one or more biomarker in a sample obtained from the patient, wherein the one or more biomarker is selected from the group consisting of: SLC39A8, CIQC, CIQA, MRAS, TMEM37, CIQB, PCOLCE2, KIF2C, TNF, IFI44, IFIT1, and RPGRIP1,
   (ii) comparing the amount of the one or more biomarker determined in said sample in (i) to a corresponding reference value; and thereby determining whether the patient has abdominal sepsis.

In a related aspect, the present invention provides a method for diagnosing whether a patient has abdominal sepsis, comprising:
(a) diagnosing a patient as having a systemic inflammatory condition by performing a method comprising:
   (i) determining the amount of one or more biomarker in a sample obtained from the patient, wherein the one or more biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1,
   (ii) comparing the amount of the one or more biomarker determined in said sample in (i) to a corresponding reference value to determine that the patient has a systemic inflammatory condition;
(b) determining that the patient diagnosed as having a systemic inflammatory condition has sepsis by performing a method to distinguish between sepsis and SIRS in the patient, comprising:
   (i) determining the amount of one or more biomarker for sepsis, and one or more biomarker for SIRS in a sample obtained from the patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124,
   (ii) comparing the amount of the one or more biomarker for sepsis determined in said sample in (i) to a corresponding reference value representative of a healthy individual,
   (iii) comparing the amount of the one or more biomarker for SIRS determined in said sample in (i) to a corresponding reference value representative of a healthy individual; wherein the patient is diagnosed as having sepsis, when an increase is observed in the one or more biomarker for sepsis, and no increase is observed in the one or more biomarker for SIRS, in the sample obtained from the patient relative to the corresponding reference value; and
(c) determining whether the patient diagnosed as having sepsis has abdominal sepsis by performing a method comprising:
   (i) determining the amount of one or more biomarker in a sample obtained from a patient, wherein the one or more biomarker is selected from the group consisting of: SLC39A8, CIQC, CIQA, MRAS, TMEM37, CIQB, PCOLCE2, KIF2C, TNF, IFI44, IFIT1, and RPGRIP1,
   (ii) comparing the amount of the one or more biomarker determined in said sample in (i) to a corresponding reference value; and thereby determining whether the patient has abdominal sepsis.

In a related aspect, the present invention provides the use of one or more of: SLC39A8, CIQC, CIQA, MRAS, TMEM37, CIQB, PCOLCE2, KIF2C, TNF, IFI44, IFIT1, and RPGRIP1 as a biomarker of abdominal sepsis in a patient.

In a further aspect, the present invention provides a method for diagnosing whether a patient has pulmonary sepsis, comprising:
(i) determining the amount of one or more biomarker in a sample obtained from a patient, wherein the one or more biomarker is selected from the group consisting of: HCAR2, CXCR1, DISC1, EPSTI1, and IFI44,
(ii) comparing the amount of the one or more biomarker determined in said sample in (i) to a corresponding reference value; and thereby determining whether the patient has pulmonary sepsis.

In a related aspect, the present invention provides a method for diagnosing whether a patient has pulmonary sepsis, comprising:
(a) diagnosing a patient as having sepsis by performing a method to distinguish between sepsis and SIRS in the patient, comprising:
   (i) determining the amount of one or more biomarker for sepsis, and one or more biomarker for SIRS in a sample obtained from the patient, wherein the one or more biomarker for sepsis is selected from the group consisting of: ITGB3, ITGA2B, MYL9, LCN2, TREML1, LCN15, CMTM5, PPBP, and PF4 and the one or more biomarker for SIRS is selected from the group consisting of: PLA2G7, ARHGEF10L, MYCL, TGFBI, and GPR124L,
   (ii) comparing the amount of the one or more biomarker for sepsis determined in said sample in (i) to a corresponding reference value representative of a healthy individual,
   (iii) comparing the amount of the one or more biomarker for SIRS determined in said sample in (i) to a corresponding reference value representative of a healthy individual; wherein the patient is diagnosed as having sepsis, when an increase is observed in the one or more biomarker for sepsis, and no increase is observed in the one or more biomarker for SIRS, in the sample obtained from the patient relative to the corresponding reference value;
(b) determining whether the patient diagnosed as having sepsis has pulmonary sepsis by performing a method comprising:
   (i) determining the amount of one or more biomarker in a sample obtained from the patient, wherein the one or more biomarker is selected from the group consisting of: HCAR2, CXCR1, DISC1, EPSTI1, and IFI44,
   (ii) comparing the amount of the one or more biomarker determined in said sample in (i) to a corresponding reference value; and thereby determining whether the patient has pulmonary sepsis.

In a further related aspect, the present invention provides a method for diagnosing whether a patient has pulmonary sepsis, comprising:
(a) diagnosing a patient as having a systemic inflammatory condition, by performing a method comprising:
   (i) determining the amount of one or more biomarker in a sample obtained from the patient, wherein the one or more biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1,
   (ii) comparing the amount of the one or more biomarker determined in said sample in (i) to a corresponding reference value to determine that the patient has a systemic inflammatory condition;
(b) determining that the patient diagnosed as having a systemic inflammatory condition has sepsis by performing a method to distinguish between sepsis and SIRS in the patient, comprising:
   (i) determining the amount of one or more biomarker for sepsis, and one or more biomarker for SIRS in a sample obtained from the patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124,
   (ii) comparing the amount of the one or more biomarker for sepsis determined in said sample in (i) to a corresponding reference value representative of a healthy individual,
   (iii) comparing the amount of the one or more biomarker for SIRS determined in said sample in (i) to a corresponding reference value representative of a healthy individual; wherein the patient is diagnosed as having sepsis, when an increase is observed in the one or more biomarker for sepsis, and no increase is observed in the one or more biomarker for SIRS, in the sample obtained from the patient relative to the corresponding reference value;
(c) determining whether the patient diagnosed as having sepsis has pulmonary sepsis by performing a method comprising:
   (i) determining the amount of one or more biomarker in a sample obtained from the patient, wherein the one or more biomarker is selected from the group consisting of: HCAR2, CXCR1, DISC1, EPSTI1, and IFI44,
   (ii) comparing the amount of the one or more biomarker determined in said sample in (i) to a corresponding reference value; and thereby determining whether the patient has pulmonary sepsis.

In a related aspect, the present invention provides the use of one or more of: HCAR2, CXCR1, DISC1, EPSTI1, and IFI44as a biomarker of pulmonary sepsis in a patient.

In a further aspect, the present invention provides a method for monitoring a systemic inflammatory condition in a patient, comprising:
(i) determining the amount of one or more biomarker in a sample obtained from a patient at a first (or earlier) time point;
(ii) determining the amount of the one or more biomarker in a sample obtained from the patient at one or more later time points;
(iii) comparing the amount of the one or more biomarker determined in step (ii) to the amount of the one or more biomarker determined in step (i);
wherein the one or more biomarker is selected from the group consisting of: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1, and LILRB5.

In one embodiment, the method is for monitoring a patient having abdominal sepsis, and comprises the steps of:
(i) determining the amount of one or more biomarker in a sample obtained from a patient having abdominal sepsis at a first time point;
(ii) determining the amount of the one or more biomarker in a sample obtained from the patient at one or more later time points;
(iii) comparing the amount of the one or more biomarker determined in step (ii) to the amount of the one or more biomarker determined in step (i);
wherein the one or more biomarker is selected from the group consisting of: ITM2A, CCL5, NPPC, KLRK1, KLRB1, HLA-DRA, BCL11B, HLA-DPB1, SLAMF6, CD160, KLRF1, and MX1.

In one embodiment, the method is for monitoring a patient having SIRS, and comprises the steps of:
(i) determining the amount of one or more biomarker in a sample obtained from a patient having SIRS at a first time point;
(ii) determining the amount of the one or more biomarker in a sample obtained from the patient at one or more later time points;
(iii) comparing the amount of the one or more biomarker determined in step (ii) to the amount of the one or more biomarker determined in step (i);
wherein the one or more biomarker is selected from the group consisting of: CCL5, NPPC, PKD1, LGALS2, MYCL, NECAB1, and PKHD1.

In a related aspect, the present invention provides the use of one or more of: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1 and LILRB5, as a biomarker for monitoring a patient having a systemic inflammatory condition.

In a related aspect, the invention provides a method for determining whether a patient having a systemic inflammatory condition is suitable for discharge from medical care, comprising:
(i) determining the amount of one or more biomarker selected from the group consisting of: NECAB1, NECAB2, PKD1, PKHD1, LILRB4, and LILRB5 in a sample obtained from a patient,
(ii) comparing the amount of the one or more biomarker determined in said sample in (i) to a corresponding reference value, and thereby determining whether the patient is suitable for discharge from medical care.

In one embodiment, the method is for determining whether a patient being treated for SIRS is suitable for discharge from medical care, and comprises the steps of:
(i) determining the amount of one or more biomarker selected from: NECAB1, PKDI, PKHD1, LILRB4, and LILRB5 in a sample obtained from a patient being treated for SIRS,
(ii) comparing the amount of one or more biomarker determined in said sample in (i) to a corresponding reference value; and thereby determining whether the patient is suitable for discharge from medical care.

In one embodiment, the method is for determining whether a patient being treated for sepsis is suitable for discharge from medical care, and comprises the steps of:
(i) determining the amount one or more biomarker selected from: NECAB2, PKD1, PKHD1 and LILRB5 in a sample obtained from a patient being treated for sepsis,
(ii) comparing the amount of the one or more biomarker determined in said sample in (i) to a corresponding reference value; and thereby determining whether the patient is suitable for discharge from medical care.

In a related aspect, the present invention provides the use of one or more of: NECAB1, NECAB2, PKD1, PKHD1, LILRB4, and LILRB5, for determining whether a patient having a systemic inflammatory condition is suitable for discharge from medical care.

In a further aspect, the present invention provides a device for carrying out the methods and uses of the invention. In one embodiment, the device comprises one or more binding agent specific for the one or more biomarker.

### DETAILED DESCRIPTION OF THE INVENTION

As described herein, the present inventors have conducted a temporal differential gene expression study in peripheral blood leukocytes (PBLs) in patients having SIRS, abdominal sepsis and pulmonary sepsis, and in normal healthy individuals. Using this method, the inventors have identified host biomarkers associated with different systemic inflammatory conditions. In particular, the present inventors have identified biomarkers that are elevated in patients having systemic inflammatory conditions, and can thus be used for diagnosis, monitoring and/or prognosis of these conditions. The present inventors have further identified biomarkers that are differentially regulated in different types of systemic inflammatory condition (e.g. SIRS and sepsis) and biomarkers that are differentially regulated in different types of sepsis (e.g. in abdominal sepsis and pulmonary sepsis). These biomarkers can therefore be used to specifically diagnose SIRS and sepsis (e.g. abdominal sepsis and pulmonary sepsis), and can also be used to distinguish between SIRS and sepsis, and/or between abdominal sepsis and pulmonary sepsis. These biomarkers may also be used to monitor a systemic inflammatory condition in a patient (e.g. to monitor the recovery of a patient). The present inventors have also identified biomarkers that are differentially regulated in patients that recover from a systemic inflammatory condition, and those that do not recover form a systemic inflammatory condition, and can thus be used to determine whether a patient is suitable for discharge from medical care. The new biomarkers for the systemic inflammatory conditions are listed in Tables 1-4 herein (together with corresponding sequence identifiers (SEQ ID NOs)). Tables 1-4 provide the HGNC gene IDs for the biomarkers of the invention. As would be understood by a person skilled in the art, the HGNC gene ID information can be used to determine the sequence of all the RNA transcripts, and thus all of the proteins which correspond to the biomarkers of the invention. Accesion numbers for each of the biomarkers are also provided in the "Sequence Information" Section of the description.

Based on these findings, the present inventors have thus developed methods and uses that allow for rapid, sensitive and accurate diagnosis, monitoring and/or prognosis of systemic inflammatory conditions (such as sepsis and/or SIRS) using one or more biological samples obtained from a patient at a single time point, or during the course of disease progression. The inventors have also developed methods and uses that allow for different systemic inflammatory conditions (such as sepsis and/or SIRS) to reliably distinguished allowing for appropriate therapeutic intervention.

### Diagnosis of a systemic inflammatory condition

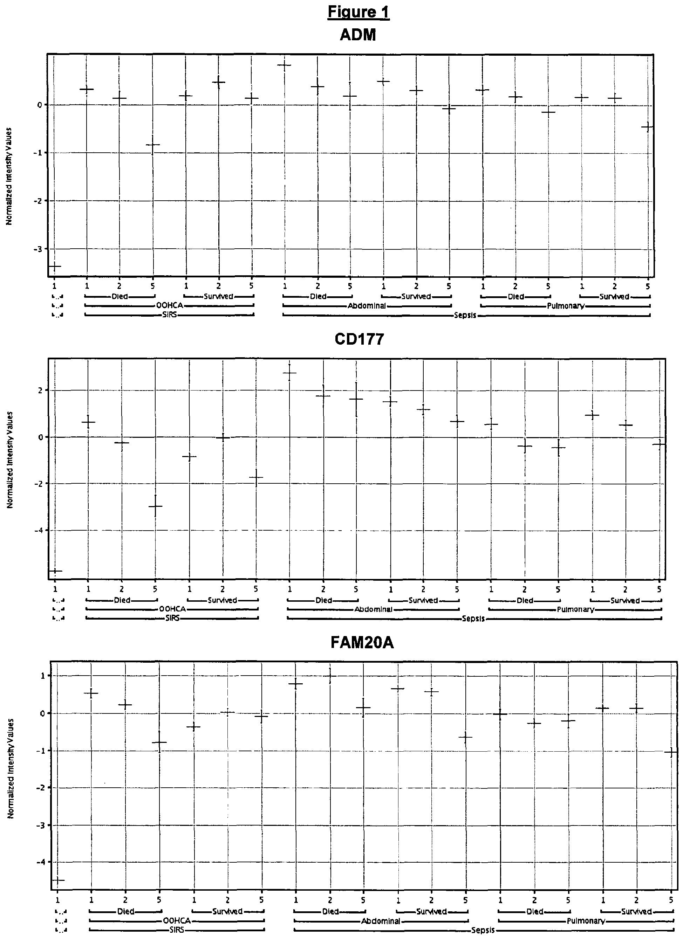

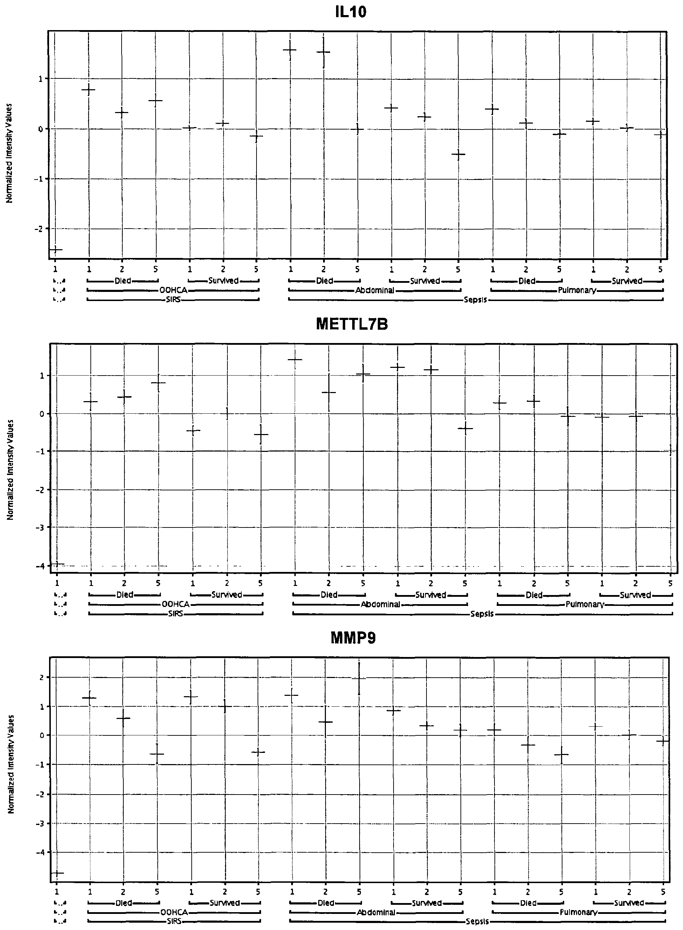

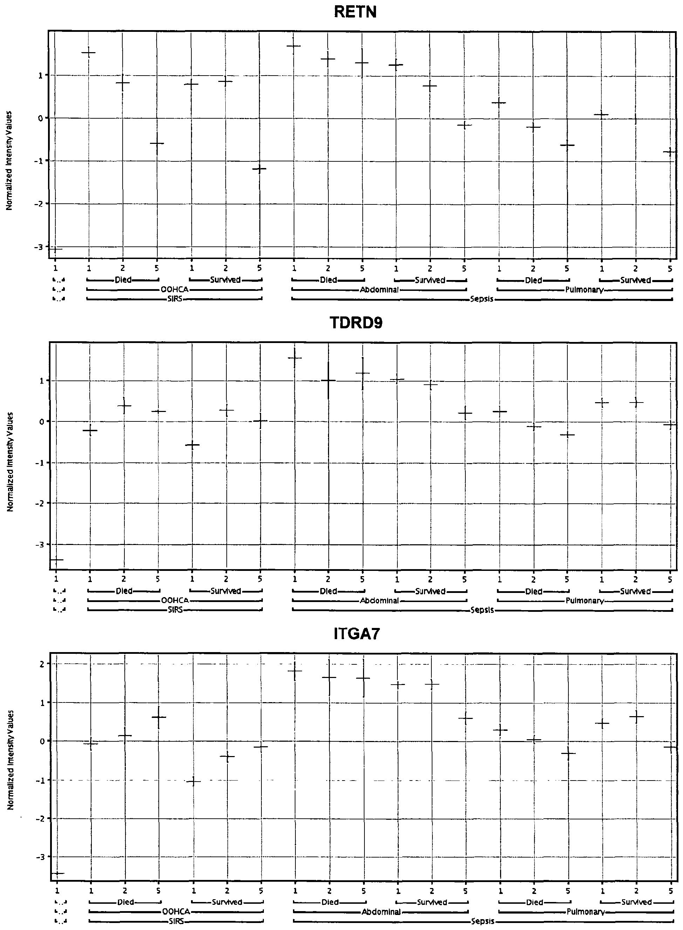

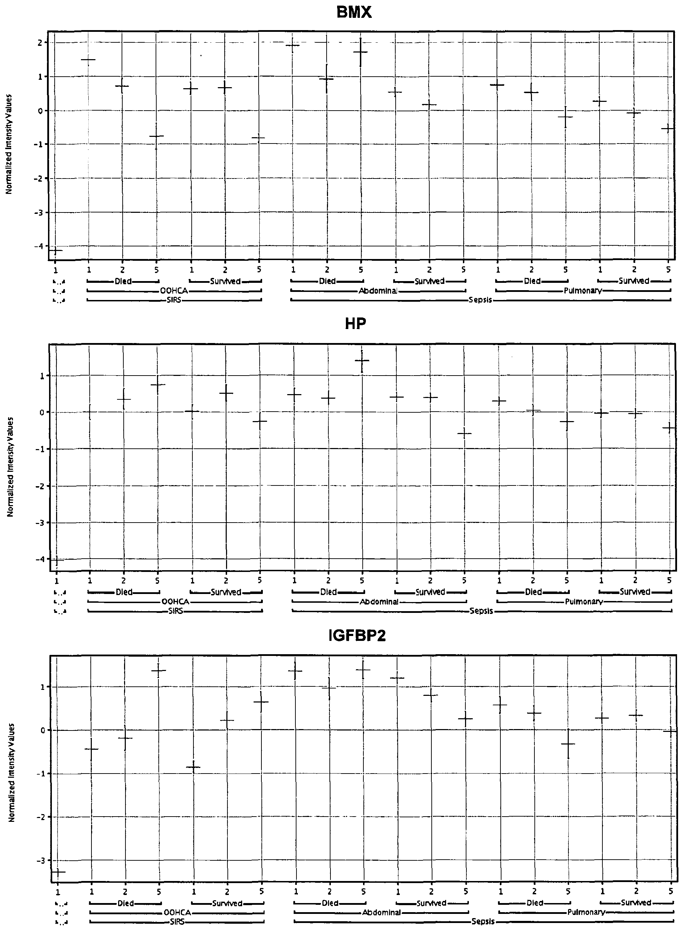

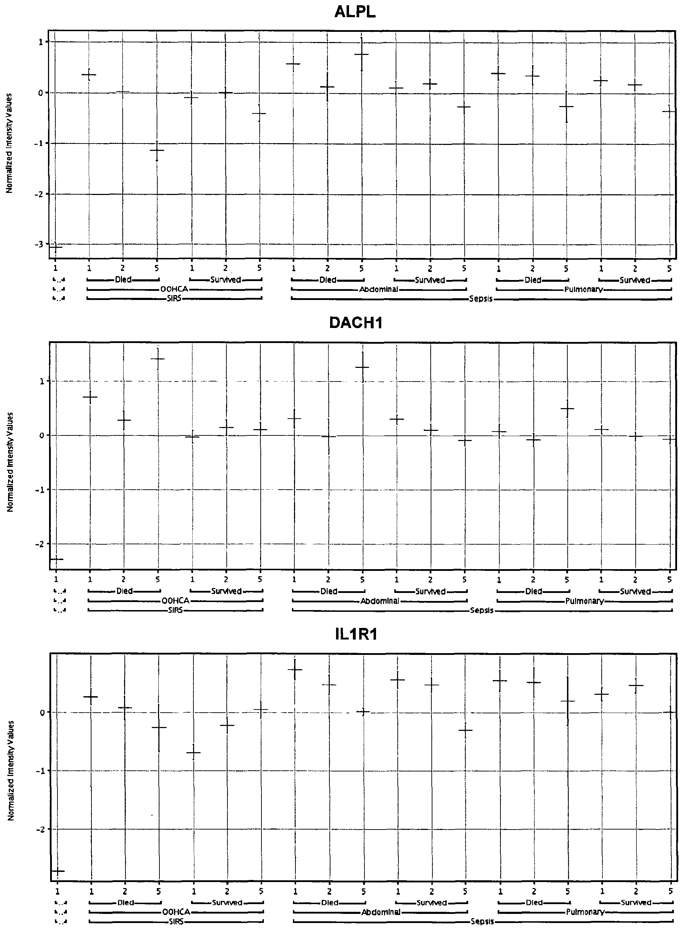

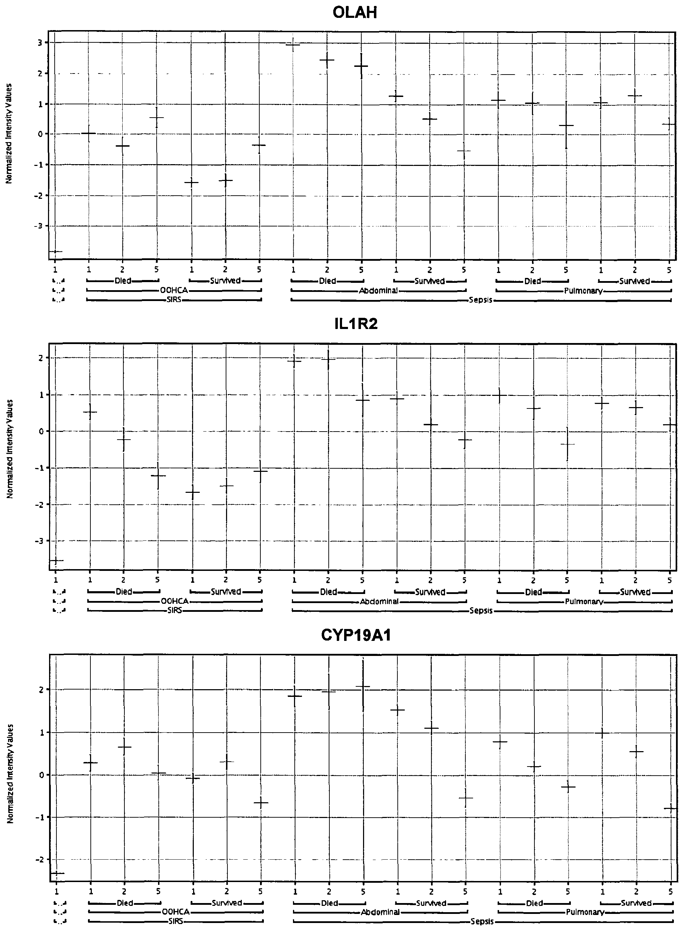

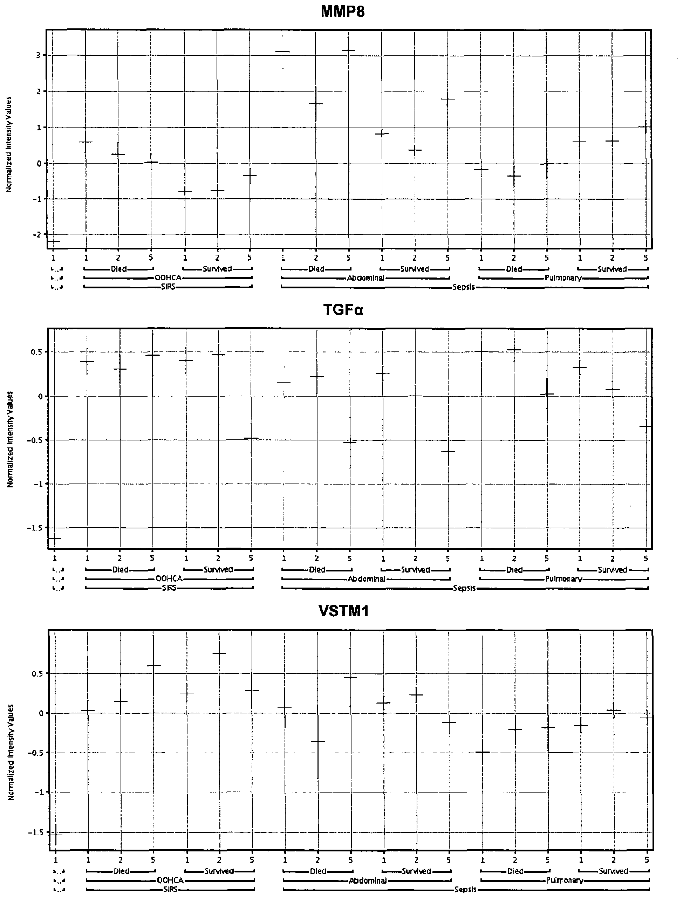

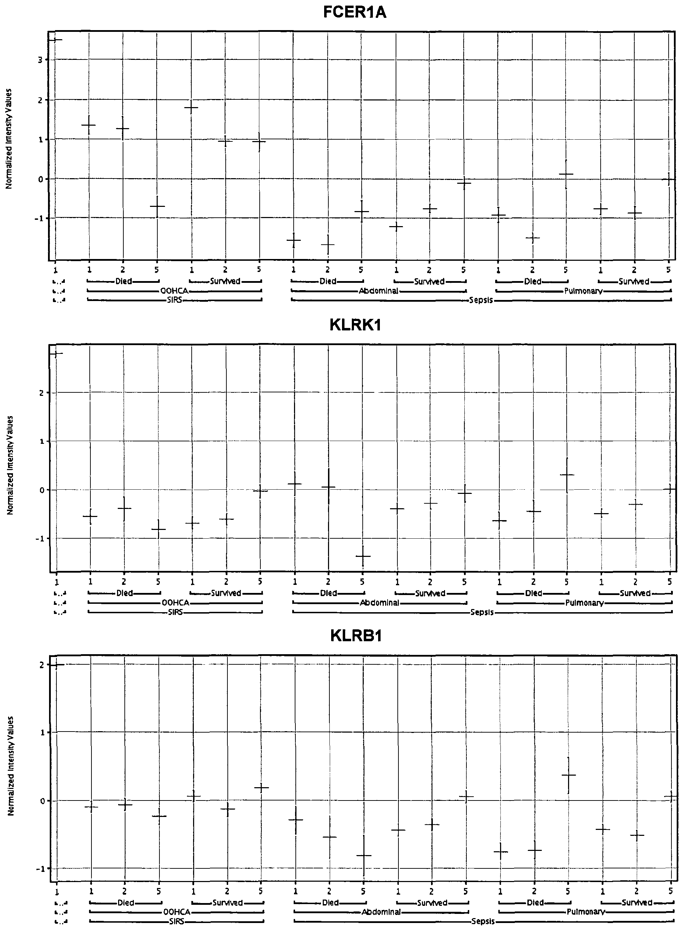

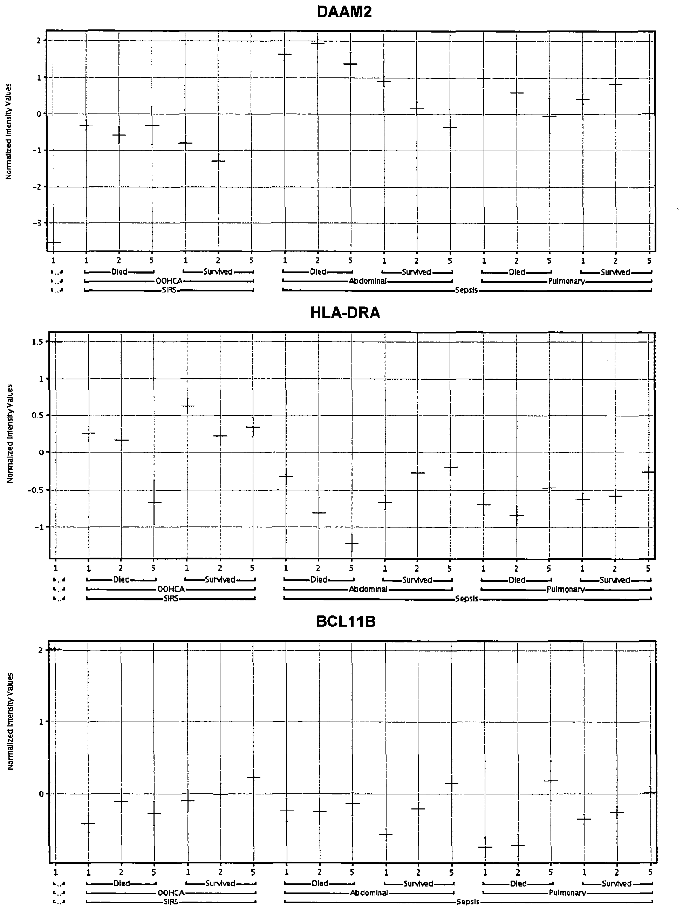

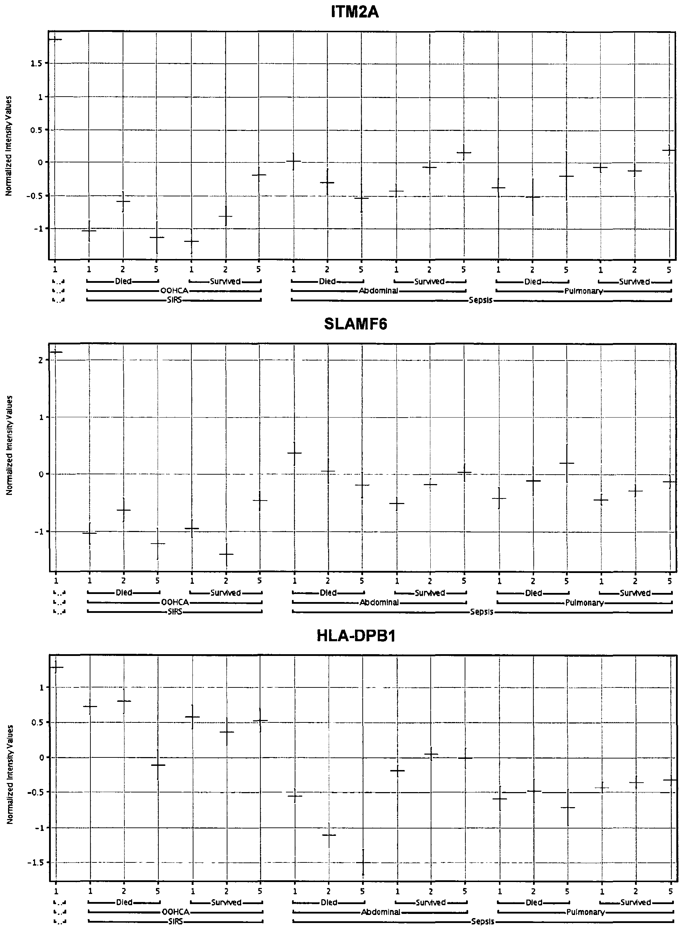

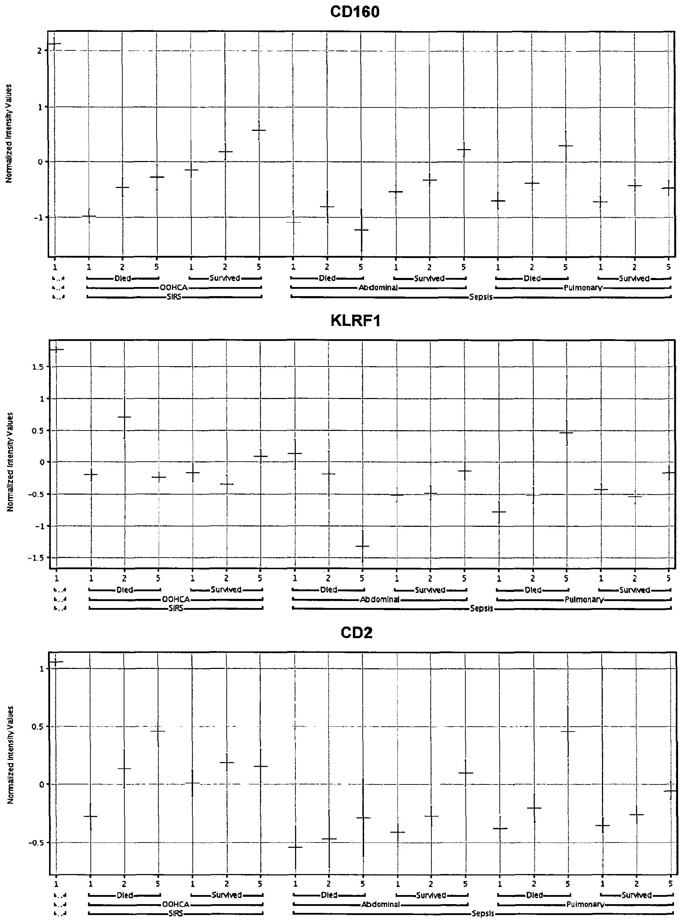

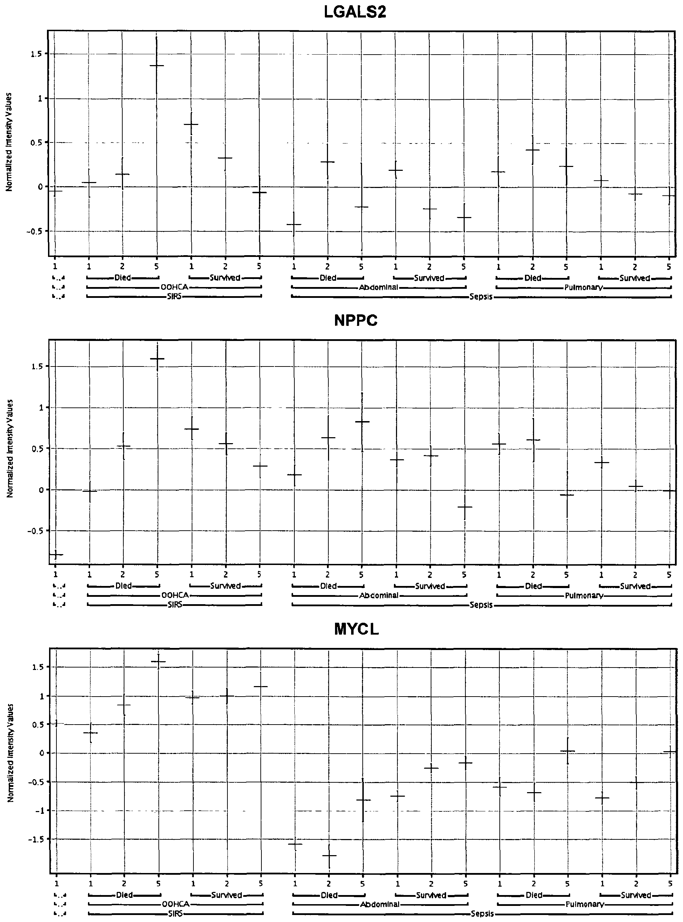

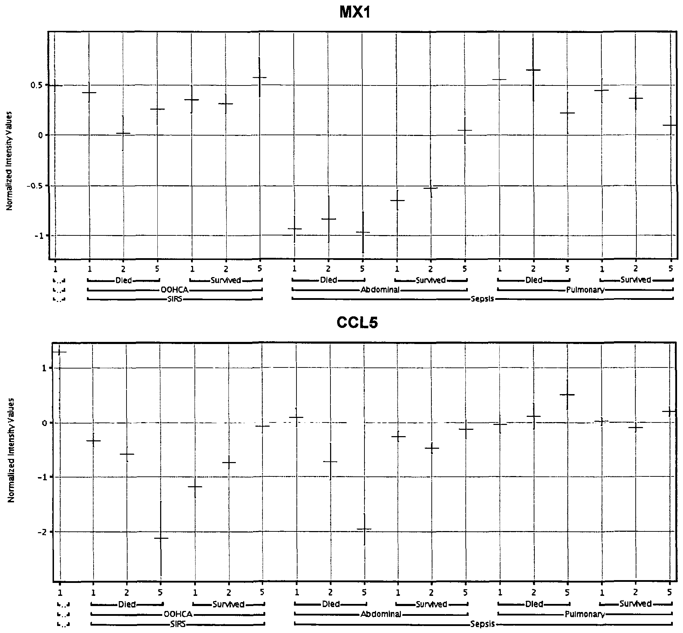

As illustrated by Example 1 and Figure 1, the present inventors observed that the levels of FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1, are elevated in patients having systemic inflammatory conditions (see Table 1), and can thus be used as biomarkers for diagnosis of systemic inflammatory conditions.

The present invention therefore provides a method for diagnosing a systemic inflammatory condition in a patient, comprising:
(i) determining the presence and/or amount of one or more inflammation biomarker in a sample obtained from a patient, wherein the one or more biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1;
(ii) comparing the presence and/or amount of the one or more inflammation biomarker determined in said sample in (i) to a corresponding reference value; and thereby determining whether the patient has or is at risk of developing a systemic inflammatory condition.

As used herein, the terms "diagnosis", "diagnosing" and "diagnose(d)" refer to the process or act of recognising, deciding on or concluding on a disease or condition in a patient on the basis of symptoms and signs and/or from results of various diagnostic procedures (such as for example, from knowing the presence, absence or quantity of one or more biomarkers characteristic of the diagnosed disease or condition). In one embodiment, diagnosis of a systemic inflammatory condition in a patient comprises determining whether the patient has or is at risk of developing a systemic inflammatory condition.

As used herein, the term "systemic inflammatory condition" refers to a disease or condition comprising a systemic inflammatory response. In one embodiment, the term encompasses SIRS and sepsis. In one embodiment, the systemic inflammatory condition is one or more of SIRS and sepsis. In one embodiment, the systemic inflammatory condition is one or more of SIRS, abdominal sepsis and pulmonary sepsis.

As used herein, the term "systemic inflammatory response syndrome (SIRS)" refers to a systemic inflammatory response syndrome with no signs of infection. This condition may also be referred to as "non-infective SIRS" or "infection-free SIRS". SIRS may be characterised by the presence of at least two of the four following clinical symptoms: fever or hypothermia (temperature of 38.0°C (100.4°F) or more, or temperature of 36.0°C (96.8°F) or less); tachycardia (at least 90 beats per minute); tachypnea (at least 20 breaths per minute or PaCC>2 less than 4.3 kPa (32.0 mm Hg) or the need for mechanical ventilation); and an altered white blood cell (WBC) count of 12×10⁶ cells/mL or more, or an altered WBC count of 4×10⁶ cells/mL or less, or the presence of more than 10% band forms (immature neutrophils).

As used herein, the term "sepsis" refers to the systemic inflammatory condition that occurs as a result of infection. Defined focus of infection is indicated by either (i) an organism grown in blood or sterile site; or (ii) an abscess or infected tissue (e.g. pneumonia, peritonitis, urinary tract, vascular line infection, soft tissue). In one embodiment, the infection may be a bacterial infection. The presence of sepsis is also characterised by the presence of at least two (of the four) systemic inflammatory response syndrome (SIRS) criteria defined above.

Sepsis may be characterised as mild sepsis, severe sepsis (sepsis with acute organ dysfunction), septic shock (sepsis with refractory arterial hypotension), organ failure, multiple organ dysfunction syndrome and death.

"Mild sepsis" can be defined as the presence of sepsis without organ dysfunction.

"Severe sepsis" can be defined as the presence of sepsis and at least one of the following manifestations of organ hypoperfusion or dysfunction: hypoxemia, metabolic acidosis, oliguria, lactic acidosis, or an acute alteration in mental status without sedation. Organ hypoperfusion or dysfunction is defined as a Sequential Organ Failure Assessment (SOFA) score ≥ 2 for the organ in question.

"Septic shock" can be defined as the presence of sepsis accompanied by a sustained decrease in systolic blood pressure (90 mm Hg or less, or a drop of at least 40 mm Hg from baseline systolic blood pressure) despite fluid resuscitation, and the need for vasoactive amines to maintain adequate blood pressure.

The term "sepsis" may include one or more of abdominal sepsis and pulmonary sepsis.

As used herein, the term "abdominal sepsis" refers to severe bacterial infection in the abdominal cavity (for example, but not restricted to perforated small and large bowel, pyelonephritis, spontaneous bacterial peritonitis, abscess in the peritoneal cavity, infection of the retroperitoneal space, infection in the liver, kidneys, pancreas, spleen); causing organ dysfunction. Organ hypoperfusion or dysfunction is defined as a Sequential Organ Failure Assessment (SOFA) score ≥ 2 for the organ in question.

As used herein, the term "pulmonary sepsis" refers to severe bacterial infection in the thoracic cavity, primarily affecting the lung and pleural space (for example, but not restricted to pneumonia, lung abscess, empyaema, mediastinitis, tracheobronchitis); causing organ dysfunction. Organ hypoperfusion or dysfunction is defined as a Sequential Organ Failure Assessment (SOFA) score ≥ 2 for the organ in question.

The terms "patient", "individual", and "subject", are used interchangeably herein to refer to a mammalian subject for whom diagnosis, monitoring, prognosis, and/or treatment is desired. The mammal can be a human, non-human primate, mouse, rat, dog, cat, horse or cow, but is not limited to these examples. In one preferred embodiment, the individual, subject, or patient is a human, e.g. a male or female.

In one embodiment, the patient is suspected of having or being at risk of developing a systemic inflammatory condition (such as SIRS, sepsis, abdominal sepsis or pulmonary sepsis). For example, the patient may be a critically ill patient, e.g. a patient admitted to an intensive care unit (ICU) or emergency department (ED), in whom the incidence of SIRS and sepsis is known to be elevated. The patient may be admitted to ICU or ED with one or more of: serious trauma, chronic obstructive pulmonary disease (COPD), patients having undergone surgery, complications from surgery, medical shock, bacterial, fungal or viral infections, Acute Respiratory Distress Syndrome (ARDS), pulmonary and systemic inflammation, pulmonary tissue injury, severe pneumonia, respiratory failure, acute respiratory failure, respiratory distress, subarachnoidal hemorrhage (SAH), (severe) stroke, asphyxia, neurological conditions, organ dysfunction, single or multi-organ failure (MOF), poisoning and intoxication, severe allergic reactions and anaphylaxis, burn injury, acute cerebral hemorrhage or infarction, and any condition for which the patient requires assisted ventilation.

In one embodiment, the patient has been previously diagnosed as having or being at risk of developing a systemic inflammatory condition (eg. SIRS, sepsis, abdominal sepsis or pulmonary sepsis). In one embodiment, the patient may have been previously diagnosed as having or being at risk of developing a systemic inflammatory condition (eg. SIRS, sepsis, abdominal sepsis or pulmonary sepsis) using any of the methods described herein, or any combination of methods described herein.

In one embodiment, the patient has not been previously diagnosed as having a systemic inflammatory condition (eg. SIRS, sepsis, abdominal sepsis or pulmonary sepsis).

As used herein, the term "sample" encompasses any suitable biological material, for example blood, plasma, saliva, serum, sputum, urine, cerebral spinal fluid, cells, a cellular extract, a tissue sample, a tissue biopsy, a stool sample and the like. Furthermore, pools or mixtures of the above-mentioned samples may be employed. Typically the sample is blood sample. The precise biological sample that is taken from the individual may vary, but the sampling preferably is minimally invasive and is easily performed by conventional techniques. In a preferred embodiment, the sample is a whole blood sample, a purified peripheral blood leukocyte sample or a cell type sorted leukocyte sample, such as a sample of the individual's neutrophils.

The methods and uses of the present invention may utilise samples that have undergone minimal or zero processing before testing. They may also utilise samples that have been manipulated, in any way, after procurement, such as treatment with reagents, solubilisation, or enrichment for certain components.

The methods of the present invention are *in vitro* methods. Thus, the methods of the present invention can be carried out *in vitro* on an isolated sample that has been obtained from a patient.

For those embodiments described herein which involve a multi-step method, the sample used in each step of the method may be the same sample obtained from the patient. When the method comprises multiple quantification steps, all the steps may be performed at the same time using the same sample.

The sample may be obtained from the patient before, during, and/or after treatment for the systemic inflammatory condition. In one embodiment, the sample is taken before treatment for the systemic inflammatory condition has been initiated. In one embodiment, the sample is taken after treatment for the systemic inflammatory condition has been initiated (eg. so as to monitor the effectiveness of a treatment regimen).

In one embodiment, the sample may be obtained from the patient at least 1 hour (e.g. at least 2 hours, at least 4 hours, at least 6 hours, at least 8 hours, at least 12 hours, at least 24 hours, at least 36 hours, at least 48 hours, at least 72 hours, at least 96 hours, or at least 120 hours) after the patient presents with one or more clinical symptoms of a systemic inflammatory condition.

In one embodiment, the sample may be obtained from the patient up to 1 hour (eg. up to 2 hours, up to 4 hours, up to 6 hours, up to 8 hours, up to 12 hours, up to 24 hours, up to 36 hours, up to 48 hours, up to 72 hours, up to 96 hours, or up to 120 hours) after the patient presents with one or more clinical symptoms of a systemic inflammatory condition. For example, the sample may be obtained from the patient up to 24 hours after the patient presents with one or more clinical symptoms of a systemic inflammatory condition. For example, the sample may be obtained from the patient up to 48 hours after the patient presents with one or more clinical symptoms of a systemic inflammatory condition. For example, the sample may be obtained from the patient up to 72 hours after the patient presents with one or more clinical symptoms of a systemic inflammatory condition. For example, the sample may be obtained from the patient up to 96 hours after the patient presents with one or more clinical symptoms of a systemic inflammatory condition. For example, the sample may be obtained from the patient up to 120 hours after the patient presents with one or more clinical symptoms of a systemic inflammatory condition.

In one embodiment, the sample may be obtained from the patient between about 1 hour and 120 hours (eg. between about 1 hour and 96 hours, between about 1 hour and 72 hours, between about 1 hour and 48 hours, or between about 1 hour and 24 hours) after the patient presents with one or more clinical symptoms of a systemic inflammatory condition.

For example, the sample may be obtained from the patient between about 12 hours and 120 hours (eg. between about 12 hours and 96 hours, between about 12 hours and 72 hours, between about 12 hours and 48 hours, or between about 12 hours and 24 hours) after the patient presents with one or more clinical symptoms of a systemic inflammatory condition.

For example, the sample may be obtained from the patient between about 24 hours and 120 hours (eg. between about 24 hours and 96 hours, between about 24 hours and 72 hours, or between about 24 hours and 48 hours) after the patient presents with one or more clinical symptoms of a systemic inflammatory condition. For example, the sample may be obtained between about 24 hours and 48 hours. For example, the sample may be obtained between about 24 hours and 72 hours. For example, the sample may be obtained between about 24 hours and 96 hours.

For example, the sample may be obtained from the patient between about 48 hours and 120 hours (eg. between about 48 hours and 96 hours, or between about 48 hours and 72 hours) after the patient presents with one or more clinical symptoms of a systemic inflammatory condition.

For example, the sample may be obtained from the patient between about 72 hours and 120 hours or between about 72 hours and 96 hours, after the patient presents with one or more clinical symptoms of a systemic inflammatory condition.

For example, the sample may be obtained from the patient between about 96 hours and 120 hours after the patient presents with one or more clinical symptoms of a systemic inflammatory condition.

Presentation of the patient with one or more clinical symptoms of a systemic inflammatory condition means that the patient displays or presents with one or more (eg. 2 or more, 3 or more, or all 4) clinical symptoms of a systemic inflammatory condition. The skilled person will be aware of the clinical symptoms associated with a systemic inflammatory condition. Clinical symptoms of a systemic inflammatory condition include: (i) fever (temperature of 38.0°C (100.4°F) or more) or hypothermia (temperature of 36.0°C (96.8°F) or less); (ii) tachycardia (at least 90 beats per minute); (iii) tachypnea (at least 20 breaths per minute or PaCC>2 less than 4.3 kPa (32.0 mm Hg) or the need for mechanical ventilation); and (iv) an altered white blood cell (WBC) count of 12×10⁶ cells/mL or more, or an altered WBC count of 4×10⁶ cells/mL or less, or the presence of more than 10% band forms.

The patient does not necessarily have to present with one or more clinical symptoms of a systemic inflammatory condition before they are tested for the presence (or absence) of a systemic inflammatory condition.

Thus, in one embodiment, the sample may be obtained from the patient at least 1 hour (e.g. at least 2 hours, at least 4 hours, at least 6 hours, at least 8 hours, at least 12 hours, at least 24 hours, at least 36 hours, at least 48 hours, at least 72 hours, at least 96 hours, or at least 120 hours) after the patient is admitted to a medical care facility.

In one embodiment, the sample may be obtained from the patient up to 1 hour (eg. up to 2 hours, up to 4 hours, up to 6 hours, up to 8 hours, up to 12 hours, up to 24 hours, up to 36 hours, up to 48 hours, up to 72 hours, up to 96 hours, or up to 120 hours) after the patient is admitted to a medical care facility. For example, the sample may be obtained from the patient up to 24 hours after the patient is admitted to a medical care facility. For example, the sample may be obtained from the patient up to 48 hours after the patient is admitted to a medical care facility. For example, the sample may be obtained from the patient up to 72 hours after the patient is admitted to a medical care facility. For example, the sample may be obtained from the patient up to 96 hours after the patient is admitted to a medical care facility. For example, the sample may be obtained from the patient up to 120 hours after the patient is admitted to a medical care facility.

In one embodiment, the sample may be obtained from the patient between about 1 hour and 120 hours (eg, between about 1 hour and 96 hours, between about 1 hour and 72 hours, between about 1 hour and 48 hours, or between about 1 hour and 24 hours) after the patient is admitted to a medical care facility.

For example, the sample may be obtained from the patient between about 12 hours and 120 hours (eg, between about 12 hours and 96 hours, between about 12 hours and 72 hours, between about 12 hours and 48 hours, or between about 12 hours and 24 hours) after the patient is admitted to a medical care facility.

For example, the sample may be obtained from the patient between about 24 hours and 120 hours (eg, between about 24 hours and 96 hours, between about 24 hours and 72 hours, or between about 24 hours and 48 hours) after the patient is admitted to a medical care facility. For example, the sample may be obtained between about 24 hours and 48 hours. For example, the sample may be obtained between about 24 hours and 72 hours. For example, the sample may be obtained between about 24 hours and 96 hours.

For example, the sample may be obtained from the patient between about 48 hours and 120 hours (eg. between about 48 hours and 96 hours, or between about 48 hours and 72 hours) after the patient is admitted to a medical care facility.

For example, the sample may be obtained from the patient between about 72 hours and 120 hours or between about 72 hours and 96 hours, after the patient is admitted to a medical care facility.

For example, the sample may be obtained from the patient between about 96 hours and 120 hours after the patient is admitted to a medical care facility.

As used herein, the phrase "after the patient is admitted to a medical care facility" refers to the admission of a patient for clinical observation and/or treatment. Admission to a medical care facility includes admittance of the patient into hospital (eg. into an intensive care unit). The term "medical care facility" is not limited hospitals, but includes any environment in which a patient can be clinically monitored and/or treated (eg. including doctors surgeries, or expedition medical tents).

As used herein, the term "biomarker" refers to virtually any biological compound, such as a protein and a fragment thereof, a peptide, a polypeptide, a proteoglycan, a glycoprotein, a lipoprotein, a carbohydrate, a lipid, a nucleic acid, an organic or inorganic chemical, a natural polymer, and a small molecule, that is present in the biological sample and that may be isolated from, or measured in, the biological sample. Furthermore, a biomarker can be the entire intact molecule, or it can be a portion thereof that may be partially functional or recognized, for example, by an antibody or other specific binding protein.

In one embodiment, the one or more biomarker is a nucleic acid (e.g., DNA, such as cDNA or amplified DNA, or RNA, such as mRNA). The one or more biomarker may have a nucleic acid sequence as shown in the sequences in the Sequence Information section herein. The relevant sequence identifiers are also shown in Tables 1-4.

In another embodiment, the one or more biomarker is a protein. As used herein, the terms "protein", "peptide", and "polypeptide" are, unless otherwise indicated, interchangeable. When the presence and/or amount of two or more biomarkers are determined, the biomarkers may all be protein biomarkers or all nucleic acid biomarkers. Alternatively, the biomarkers may be both protein and nucleic acid biomarkers.

The present invention also encompasses, without limitation, polymorphisms, isoforms, metabolites, mutants, variants, derivatives, modifications, subunits, fragments, protein-ligand complexes and degradation products of the biomarkers listed in Tables 1-4.

The protein fragments can be 200, 150, 100, 50, 25, 10 amino acids or fewer in length. The nucleic acid fragments can be 1000, 500, 250 150, 100, 50, 25, 10 nucleotides or fewer in length.

Variants of the protein biomarkers of the present invention include polypeptides with altered amino acid sequences due to amino acid substitutions, deletions, or insertions. Variant polypeptides may comprise conservative or non-conservative amino acid substitutions, deletions or additions. Variants include polypeptides that have an amino acid sequence being at least 70%, at least 80%, at least 90%, at least 95%, at least 98% or at least 99% identical to the amino acid sequences of the polypeptides listed in Tables 1-4. Variants may be allelic variants, splice variants or any other species specific homologs, paralogs, or orthologs.

Derivatives of the protein biomarkers of the present invention are polypeptides which contain one or more naturally occurring amino acid derivatives of the twenty standard amino acids. For example, 4-hydroxyproline may be substituted for proline; 5-hydroxylysine may be substituted for lysine; 3-methylhistidine may be substituted for histidine; homoserine may be substituted for serine; and ornithine may be substituted for lysine.

Variants of the nucleic acid biomarkers of the present invention may have a sequence identity of at least 80% with the corresponding nucleic acid sequence shown in the Sequence Information section. Sequence identity may be calculated as described herein. A sequence identity of at least 80% includes at least 82%, at least 84%, at least 86%, at least 88%, at least 90%, at least 91%, at least 92%, at least 93%, at least 94%, at least 95%, at least 96%, at least 97%, at least 98%, at least 99%, and 100% sequence identity (to each and every nucleic acid sequence presented herein and/ or to each and every SEQ ID NO presented herein).

The one or more inflammation biomarker used in the method may be selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1.

In one embodiment, the one or more inflammation biomarker may be selected from the group consisting of: IGFBP2, CYP19A1, and VSTM1. In one embodiment, the one or more biomarker may be selected from the group consisting of: CD177, IL10, IL1R1, IL1R2, VSTM1, ADM, and HP, wherein said biomarkers are associated with immune response and/ or inflammation. In one embodiment, the one or more biomarker may be selected from the group consisting of: METTL7B, RETN, and CYP19A1, wherein said biomarkers are associated with lipid metabolism. In one embodiment, the one or more biomarker may be selected from the group consisting of: MMP9 and MMP8, wherein said biomarkers are associated with extracellular matrix maintenance or composition.

Each of the biomarkers for a systemic inflammatory condition may be used alone, or in combination with any of the biomarkers for a systemic inflammatory condition in the method of the invention. For example, any combination of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more 19 or more, 20 or more, up to and including all of the biomarkers may be used to diagnose a systemic inflammatory condition according to the method of the invention.

In one embodiment, the one or more biomarker is FAM20A. In one embodiment, the one or more biomarker is OLAH. In one embodiment, the one or more biomarker is CD177. In one embodiment, the one or more biomarker is ADM. In one embodiment, the one or more biomarker is IL10. In one embodiment, the one or more biomarker is METTL7B. In one embodiment, the one or more biomarker is MMP9. In one embodiment, the one or more biomarker is RETN. In one embodiment, the one or more biomarker is TDRD9. In one embodiment, the one or more biomarker is ITGA7. In one embodiment, the one or more biomarker is BMX. In one embodiment, the one or more biomarker is HP. In one embodiment, the one or more biomarker is IGFBP2. In one embodiment, the one or more biomarker is ALPL. In one embodiment, the one or more biomarker is DACH1. In one embodiment, the one or more biomarker is IL1R1. In one embodiment, the one or more biomarker is IL1R2. In one embodiment, the one or more biomarker is CYP19A1. In one embodiment, the one or more biomarker is MMP8. In one embodiment, the one or more biomarker is TGFA. In one embodiment, the one or more biomarker is VSTM1.

In one embodiment, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more 19 or more, 20 of more, or all 21) of the biomarkers selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1, may be used to diagnose a systemic inflammatory condition in a patient.

As demonstrated by ROC analysis performed in Example 2, the inflammation biomarkers FAM20A, OLAH and CD177 were all shown to provide highly accurate diagnosis of patients having a systemic inflammatory condition when used on their own or in combination. In one embodiment, a combination of FAM20A and OLAH may be used to diagnose a systemic inflammatory condition in a patient. In one embodiment, a combination of FAM20A, OLAH and CD177 may be used to diagnose a systemic inflammatory condition in a patient.

One or more additional biomarker for inflammation may also be used in the method of the invention to diagnose a systemic inflammatory condition. Any combination of the one or more additional biomarker may be used in combination with the one or more biomarker of the invention. For example at least 1, 2, 3, 4, 5, 6, 7, 8, 9, 10, 11, 12, 13, 14, 15, 16, 17, 18, 19, or all 20 additional biomakers for inflammation may be used in combination with the one or more biomarker of the invention (as described herein). Typically, the one or more additional biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1.

In one embodiment, the one or more biomarker is FAM20A, and the one or more additional biomarker is selected from at least 1 (eg. at least 2, 3, 4, 5, 6, 7, 8, 9, 10, 11, 12, 13, 14, 15, 16, 17, 18, 19, up to and including all) of the biomarkers: OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1.

In one embodiment, the one or more biomarker is OLAH, and the one or more additional biomarker is selected from at least 1 (eg. at least 2, 3, 4, 5, 6, 7, 8, 9, 10, 11, 12, 13, 14, 15, 16, 17, 18, 19, up to and including all) of the biomarkers: FAM20A, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1.

In one embodiment, the one or more biomarker is CD177, and the one or more additional biomarker is selected from at least 1 (eg. at least 2, 3, 4, 5, 6, 7, 8, 9, 10, 11, 12, 13, 14, 15, 16, 17, 18, 19, up to and including all) of the biomarkers: FAM20A, OLAH, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1.

A biomarker is considered to be informative if a measurable aspect or characteristic of the biomarker is associated with a given state of an individual, such as the diagnosis, monitoring or prognosis of a systemic inflammatory condition. Such a measurable aspect or characteristic may include, for example, the presence, absence, or concentration of the biomarker in the biological sample from the individual and/or its presence as part of a profile of biomarkers. Such a measurable aspect of a biomarker is defined herein as a "feature." For example, the presence of a biomarker in a sample may be a feature. As another example, the amount of a biomarker in a sample, or the amount of a biomarker in a sample compared with a control or reference sample may be a feature. A feature may also be a ratio of two or more measurable aspects of biomarkers, which biomarkers may or may not be of known identity. A "biomarker profile" comprises at least two such features, where the features can correspond to the same or different classes of biomarkers such as, for example, two nucleic acids or a nucleic acid and a protein. A biomarker profile may also comprise at least three, four, five, 10, 20, 30 or more features. In one embodiment, a biomarker profile comprises hundreds, or even thousands, of features. In another embodiment, the biomarker profile comprises at least one measurable aspect of at least one internal standard.

A "phenotypic change" is a detectable change in a parameter associated with a given state of the individual. For instance, a phenotypic change may include an increase or decrease of a biomarker in a bodily fluid, where the change is associated with a systemic inflammatory condition (such as sepsis or SIRS) or distinguishing between sepsis and SIRS. The presence and/or amount of each of the one or more biomarkers of the invention is a feature or phenotypic change according to the present invention. For example, the presence of each of the one or more biomarkers of the invention is a feature or phenotypic change according to the present invention. For example, the amount of each of the one or more biomarkers of the invention is a feature or phenotypic change according to the present invention. In a further example, the presence and amount of each of the one or more biomarkers of the invention is a feature or phenotypic change according to the present invention.

A phenotypic change may further include a change in a detectable aspect of a given state of the individual that is not a change in a measurable aspect of a biomarker. For example, a change in phenotype may include a detectable change in body temperature, weight loss, fatigue, respiration rate or other physiological parameter. Such changes can be determined via clinical observation and measurement using conventional techniques that are well-known to the skilled artisan. As used herein, "conventional techniques" are those techniques that classify an individual based on phenotypic changes without obtaining a biomarker profile according to the present invention.

According to the present invention, systemic inflammatory conditions may be diagnosed, monitored, and/or prognosed by obtaining a profile of biomarkers from a sample obtained from a patient. As used herein, "obtain" means "to come into possession of".

A feature as defined herein for the diagnosis, monitoring or prognosis of a systemic inflammatory condition may be detected, quantified or determined by any appropriate means. For example, the one or more biomarker of the invention, a measurable aspect or characteristic of the one or more biomarker or a biomarker profile of the invention may be detected by any appropriate means. The presence of the one or more biomarkers of the invention may be considered together as a "biomarker profile" of the invention. The presence of the individual biomarkers within any of the biomarker combinations disclosed herein may be considered together as a "biomarker profile" of the invention.

The presence and/or amount of the one or more biomarker of the invention may be determined by quantitative and/or qualitative analysis. Measurement of the one or more biomarkers can be performed by any method that provides satisfactory analytical specificity, sensitivity and precision. The invention encompasses the use of those methods known to a person skilled in the art to measure the presence and/or amount of one or more biomarkers.

In one embodiment, the methods described herein involve determining the "presence and amount of the one or more biomarker". In one embodiment, the methods described herein involve determining the "presence of the one or more biomarker". In one embodiment, the methods described herein involve determining the "amount of the one or more biomarker".

Determining the "amount of one or more biomarker" in a sample means quantifying the biomarker by determining the relative or absolute amount of the biomarker. The amount of the one or more biomarker of the invention encompasses the mass of the one or more biomarker, the molar amount of the one or more biomarker, the concentration of the one or biomarker and the molarity of the one or more biomarker. This amount may be given in any appropriate units. For example, the concentration of the one or more biomarker may be given in pg/ml, ng/ml or µg/ml. It will be appreciated that the assay methods do not necessarily require measurement of absolute values of biomarker, unless it is desired, because relative values are sufficient for many applications of the invention. Accordingly, the "amount" can be the "absolute" total amount of the biomarker that is detected in a sample, or it can be a "relative" amount, e.g., the difference between the biomarker detected in a sample and e.g. another constituent of the sample. In some embodiments, the amount of the biomarker may be expressed by its concentration in a sample, or by the concentration of an antibody that binds to the biomarker. Thus, the actual amount of the one or more biomarker, such as the mass, molar amount, concentration or molarity of the one or biomarker may be assessed and compared with the corresponding reference value. Alternatively, the amount of one or more biomarker may be compared with that of the reference value without quantifying the mass, molar amount, concentration or molarity of the one or more biomarker.

The presence and/or amount of the one or more biomarker can be determined at the protein or nucleic acid level using any method known in the art. The particular preferred method for determining the presence and/or amount of the one or more biomarkers will depend in part on the identity and nature of the biomarker.

The biomarkers of the invention may be detected at the nucleic acid or protein level. Thus, the biomarkers of the invention may be DNA, RNA or protein and may be detected using any appropriate technique. The presence and/or amount of the one or more biomarker of the invention may be measured directly or indirectly. Any appropriate agent may be used to determine the presence and/or amount of the one or more biomarker of the invention. For example, the presence and/or amount of the one or more biomarker of the invention may be determined using an agent selected from peptides and peptidomimetics, antibodies, small molecules and single-stranded DNA or RNA molecules, as described herein. Suitable standard techniques are known in the art.

For example, when the one or more biomarker is detected at the nucleic acid level this may be carried out using: (i) biomarker-specific oligonucleotide DNA or RNA or any other nucleic acid derivative probes bound to a solid surface; (ii) purified RNA (labelled by any method, for example using reverse transcription and amplification) hybridised to probes; (iii) whole lysed blood, from which the RNA is labelled by any method and hybridised to probes; (iv) purified RNA hybridised to probes and a second probe (labelled by any method) hybridised to the purified RNA; (v) whole lysed blood from which the RNA is hybridised to probes, and a second probe (labelled by any method) which is hybridised to the RNA; (vi) purified peripheral blood leukocytes, obtaining purified RNA (labelled by any method), and hybridising the purified labelled RNA to probes; (vii) purified peripheral blood leukocytes, obtaining purified RNA and hybridising the RNA to probes, then using a second probe (labelled by any method) which hybridises to the RNA; (viii) RT-PCR using any primer/probe combination or inter-chelating fluorescent label, for example SYBRGreen; (ix) end-point PCR; (x) digital PCR; (xi) sequencing; (xii) array cards (RT-PCR); (xiii) lateral flow devices/methodology; and/or (xiv) digital microfluidics.

In one embodiment, quantitative real-time PCR is used to determine the presence and/or amount of the one or more biomarker of the invention. Quantitative real-time PCR may be performed using forward and reverse oligonucleotide primers that amplify the target sequence (such as those described herein). Detection of the amplified product is done in real-time, and may be performed using oligonucleotide probes that produce a fluorescent signal when the target DNA is amplified (e.g. Taqman^{®} fluorgenic probes), or using SYBR Green dye that binds to double-stranded DNA and emits fluorescence when bound.

In one embodiment, oligonucleotide microarray analysis is used to detect and/or quantify the one or more biomarker of the invention using biomarker-specific oligonucleotide DNA or RNA or any other nucleic acid derivative probes bound to a solid surface.

In a preferred embodiment, RNA from a sample (either purified or unpurified) is labelled via any method (typically amplification) and used to interrogate one or more probe immobilised on a surface. Typically the one or more probes are 50 to 100 nucleotides in length.

In another preferred embodiment, one or more probe is immobilised on a surface and the RNA from a sample is hybridised to one or more second probe (labelled by any method). The RNA hybridised with the second (labelled) probe is then used to interrogate the one or more probe immobilised on the surface. Examples of such methodology are known in the art, including the Vantix^{™} system.

For example, when the one or more biomarker is detected at the protein acid level this may be carried out using: (i) biomarker-specific primary antibodies or antibody fragments bound to a solid surface; (ii) whole lysed blood biomarker antigen bound to antibodies or antibody fragments; (iii) secondary biomarker-specific antibodies or antibody fragments used to detect biomarker antigen bound to primary antibody (labelled using any method); (iv) biomarker-specific primary aptamers bound to a solid surface; (v) whole lysed blood - biomarker antigen bound to aptamers; (vi) secondary biomarker-specific aptamer used to detect biomarker antigen bound to primary aptamer (labelled using any method); (vii) any antibody derivative i.e. phage display etc. used as above; (viii) lateral flow devices/methodology; (ix) chromatography; (x) mass spectrometry; (xi) nuclear magnetic resonance (NMR); (xii) protein gels/transfers to filter; and/or (xiii) immunoprecipitation. In a preferred embodiment, a lateral flow device may be used to detect the one or more protein biomarker.

Any agent for the detection of or for the determination of the amount of the one or more biomarker of the invention may be used to determine the amount of the one or more biomarker. Similarly, any method that allows for the detecting of the one or more biomarker, the quantification, or relative quantification of the one or more biomarker may be used.

Agents for the detection of or for the determination of the amount of one or more biomarker may be used to determine the amount of the one or more biomarker in a sample obtained from the patient. Such agents typically bind to the one or more biomarker. Such agents may bind specifically to the one or more biomarker. The agent for the detection of or for the determination of the amount of the one or more biomarker may be an antibody or other binding agent specific for the one or more biomarker. By specific, it will be understood that the agent or antibody binds to the molecule of interest, in this case the one or more biomarker, with no significant cross-reactivity to any other molecule, particularly any other protein. Cross-reactivity may be assessed by any suitable method. Cross-reactivity of an agent or antibody for the one or more biomarker with a molecule other than the one or more biomarker may be considered significant if the agent or antibody binds to the other molecule at least 5%, 10%, 15%, 20%, 25%, 30%, 35%, 40%, 45%, 50%, 55%, 60%, 65%, 70%, 75%, 80%, 85%, 90% or 100% as strongly as it binds to the one or more biomarker. Preferably, the agent or antibody binds to the other molecule at less than 20%, less than 15%, less than 10% or less than 5%, less than 2% or less than 1% the strength that it binds to the one or more biomarker.

As described herein, the presence and/or amount of the one or more biomarker, and hence the biomarker profile may be determined immunologically by reacting antibodies, or functional fragments thereof, specific to the biomarkers. A functional fragment of an antibody is a portion of an antibody that retains at least some ability to bind to the antigen to which the complete antibody binds. The fragments, which include, but are not limited to, scFv fragments, Fab fragments, F(ab) fragments and F(ab)2 fragments, can be recombinantly produced or enzymatically produced. Specific binding molecules other than antibodies, such as aptamers, may be used to bind the biomarkers.

The antibody may be monoclonal or polyclonal. The antibody may be produced by any suitable method known in the art. For example, polyclonal antibodies may be obtained by immunising a mammal, typically a rabbit or a mouse, with the one or more biomarker under suitable conditions and isolating antibody molecules from, for example, the serum of said mammal. Monoclonal antibodies may be obtained by hybridoma or recombinant methods.

Hybridoma methods may involve immunising a mammal, typically a rabbit or a mouse, with the one or more biomarker under suitable conditions, then harvesting the spleen cells of said mammal and fusing them with myeloma cells. The mixture of fused cells is then diluted and clones are grown from single parent cells. The antibodies secreted by the different clones are then tested for their ability to bind to the one or more biomarker, and the most productive and stable clone is then grown in culture medium to a high volume. The secreted antibody is collected and purified.

Recombinant methods may involve the cloning into phage or yeast of different immunoglobulin gene segments to create libraries of antibodies with slightly different amino acid sequences. Those sequences which give rise to antibodies which bind to the one or more biomarker may be selected and the sequences cloned into, for example, a bacterial cell line, for production.

Typically the antibody is a mammalian antibody, such as a primate, human, rodent (e.g. mouse or rat), rabbit, ovine, porcine, equine or camel antibody. The antibody may be a camelid antibody or shark antibody. The antibody may be a nanobody. The antibody can be any class or isotype of antibody, for example IgM, but is preferably IgG. The antibody may be a humanised antibody.

The antibody or fragment may be associated with other moieties, such as linkers which may be used to join together 2 or more fragments or antibodies. Such linkers may be chemical linkers or can be present in the form of a fusion protein with the fragment or whole antibody. The linkers may thus be used to join together whole antibodies or fragments which have the same or different binding specificities, e.g. that can bind the same or different polymorphisms. The antibody may be a bispecific antibody which is able to bind to two different antigens, typically any two of the polymorphisms mentioned herein. The antibody may be a *'diabody'* formed by joining two variable domains back to back. In the case where the antibodies used in the method are present in any of the above forms which have different antigen binding sites of different specificities then these different specificities are typically to polymorphisms at different positions or on different proteins. In one embodiment the antibody is a chimeric antibody comprising sequence from different natural antibodies, for example a humanised antibody.

Methods to assess an amount of the one or more biomarker may involve contacting a sample with an agent or antibody capable of binding specifically to the one or more biomarker. Such methods may include dipstick assays and Enzyme-linked Immunosorbant Assay (ELISA), or similar assays, such as those using a lateral flow device. Other immunoassay types may also be used to assess the one or more biomarker amounts. Typically dipsticks comprise one or more antibodies or proteins that specifically bind to the one or more biomarker. If more than one antibody is present, the antibodies preferably have different non-overlapping determinants such that they may bind to the one or more biomarker simultaneously.

ELISA is a heterogeneous, solid phase assay that requires the separation of reagents. ELISA is typically carried out using the sandwich technique or the competitive technique. The sandwich technique requires two antibodies. The first specifically binds the one or more biomarker and is bound to a solid support. The second antibody is bound to a marker, typically an enzyme conjugate. A substrate for the enzyme is used to quantify the one or more biomarker -antibody complex and hence the amount of the one or more biomarker in a sample. The antigen competitive inhibition assay also typically requires a one or more biomarker -specific antibody bound to a support. A biomarker -enzyme conjugate is added to the sample (containing the one or more biomarker) to be assayed. Competitive inhibition between the biomarker - enzyme conjugate and unlabelled biomarker allows quantification of the amount of the one or more biomarker in a sample. The solid supports for ELISA reactions preferably contain wells.

Antibodies capable of binding specifically to the one or more biomarker may be used in methods of immunofluorescence to detect the presence of the one or more biomarker.

The present invention may also employ methods of determining the amount of the one or more biomarker that do not comprise antibodies. High Performance Liquid Chromatography (HPLC) separation and fluorescence detection is preferably used as a method of determining the amount of the one or more biomarker. HPLC apparatus and methods as described previously may be used (Tsikas D et al. J Chromatogr B Biomed Sci Appl 1998; 705: 174-6) Separation during HPLC is typically carried out on the basis of size or charge. Prior to HPLC, endogenous amino acids and an internal standard L-homoarginine are typically added to assay samples and these are phase extracted on CBA cartridges (Varian, Harbor City, CA). Amino acids within the samples are preferably derivatized with o-phthalaldehyde (OPA). The accuracy and precision of the assay is preferably determined within quality control samples for all amino acids.

Other methods of determining the amount the one or more biomarker that do not comprise antibodies include mass spectrometry. Mass spectrometric methods may include, for example, matrix-assisted laser desorption/ionization mass spectrometry (MALDI MS), surface-enhanced laser desorption/ionization mass spectrometry (SELDI MS), time of flight mass spectrometry (TOF MS) and liquid chromatography mass spectrometry (LC MS).

A separation method may be used to determine the presence and/or amount of the one or more biomarker and hence to create a profile of biomarkers, such that only a subset of biomarkers within the sample is analysed. For example, the biomarkers that are analysed in a sample may consist of mRNA species from a cellular extract, which has been fractionated to obtain only the nucleic acid biomarkers within the sample, or the biomarkers may consist of a fraction of the total complement of proteins within the sample, which have been fractionated by chromatographic techniques. One or more, two or more, three or more, four or more, or five or more separation methods may be used according to the present invention.

Determination of the presence and/or amount of the one or more biomarker, and hence the creation of a profile of biomarkers may be carried out without employing a separation method. For example, a biological sample may be interrogated with a labelled compound that forms a specific complex with a biomarker in the sample, where the intensity of the label in the specific complex is a measurable characteristic of the biomarker. A suitable compound for forming such a specific complex is a labelled antibody. A biomarker may be measured using an antibody with an amplifiable nucleic acid as a label. The nucleic acid label may become amplifiable when two antibodies, each conjugated to one strand of a nucleic acid label, interact with the biomarker, such that the two nucleic acid strands form an amplifiable nucleic acid.

The presence and/or amount of the one or more biomarker, and hence the biomarker profile may be derived from an assay, such as an array, of nucleic acids, where the biomarkers are the nucleic acids or complements thereof. For example, the biomarkers may be ribonucleic acids. The presence and/or amount of the one or more biomarker, and hence the biomarker profile may be obtained using a method selected from nuclear magnetic resonance, nucleic acid arrays, dot blotting, slot blotting, reverse transcription amplification and Northern analysis.

The determination of the presence and/or amount of the one or more biomarker, and hence a biomarker profile may be generated by the use of one or more separation methods. For example, suitable separation methods may include a mass spectrometry method, such as electrospray ionization mass spectrometry (ESI-MS), ESI-MS/MS, ESI- MS/(MS)n (n is an integer greater than zero), matrix-assisted laser desorption ionization time- of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SLMS), quadrupole time-of-flight (Q-TOF), atmospheric pressure chemical ionization mass spectrometry (APCI-MS), APCI-MS/MS, APCI-(MS)n, atmospheric pressure photoionization mass spectrometry (APPI-MS), APPI-MS/MS, and APPI-(MS)n. Other mass spectrometry methods may include, inter alia, quadrupole, fourier transform mass spectrometry (FTMS) and ion trap. Other suitable separation methods may include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE) or other chromatography, such as thin-layer, gas or liquid chromatography, or any combination thereof. The sample may be fractionated prior to application of the separation method.

The determination of the presence and/or amount of the one or more biomarker, and hence a biomarker profile may be generated by methods that do not require physical separation of the biomarkers themselves. For example, nuclear magnetic resonance (NMR) spectroscopy may be used to resolve a profile of biomarkers from a complex mixture of molecules. An analogous use of NMR to classify tumours is disclosed in Hagberg, NMR Biomed. 11: 148-56 (1998), for example. Additional procedures include nucleic acid amplification technologies, which may be used to generate a profile of biomarkers without physical separation of individual biomarkers. (See Stordeur et al, J. Immunol. Methods 259: 55-64 (2002) and Tan et al, Proc. Nat'lAcad. Sci. USA 99: 11387-11392 (2002), for example.)

In one embodiment, laser desorption/ionization time-of-flight mass spectrometry is used to determine the presence and/or amount of the one or more biomarker, and hence create a biomarker profile where the biomarkers are proteins or protein fragments that have been ionized and vaporized off an immobilizing support by incident laser radiation. A profile is then created by the characteristic time-of-flight for each protein, which depends on its mass-to-charge ("m/z") ratio. A variety of laser desorption/ionization techniques are known in the art. (See, e.g., Guttman et al, Anal Chem. 73: 1252-62 (2001) and Wei et al, Nature 399: 243-46 (1999).)

Laser desorption/ionization time-of-flight mass spectrometry allows the generation of large amounts of information in a relatively short period of time. A sample is applied to one of several varieties of a support that binds all of the biomarkers, or a subset thereof, in the sample. Cell lysates or samples are directly applied to these surfaces in volumes as small as 0.5 µL, with or without prior purification or fractionation. The lysates or sample can be concentrated or diluted prior to application onto the support surface. Laser desorption/ionization is then used to generate mass spectra of the sample, or samples, in as little as three hours.

In a preferred embodiment, the total mRNA from a cellular extract of the patient is assayed, and the various mRNA species that are obtained from the sample are used as biomarkers. Biomarker profiles may be obtained, for example, by hybridizing these mRNAs to an array of probes, which may comprise oligonucleotides or cDNAs, using standard methods known in the art. Alternatively, the mRNAs may be subjected to gel electrophoresis or blotting methods such as dot blots, slot blots or Northern analysis, all of which are known in the art. (See, e.g., Sambrook et al. in "Molecular Cloning, 3rd ed.," Cold Spring Harbor Laboratory Press, Cold Spring Harbor, New York (2001).) mRNA profiles also may be obtained by reverse transcription followed by amplification and detection of the resulting cDNAs, as disclosed by Stordeur *et al,* supra, for example. In another embodiment, the profile may be obtained by using a combination of methods, such as a nucleic acid array combined with mass spectroscopy.

Different methods have different advantages and may be preferred depending on numerous factors, such as the particular circumstances of the patients to be tested and/or the availability of reagents/equipment in the diagnostics laboratory. For example, qPCR using probe/quencher hydrolysis probes is highly specific and stringent. As another example, microarray analysis can resolve subtle differences in expression of transcript variants, which may be important in disease pathology and diagnosis.

Different one or more biomarkers may be used with different detection methods according to the present invention.

When the amount of two or more biomarkers are determined, the amount of each biomarker may be determined, or the cumulative amount of all the biomarkers may be determined. Alternatively, the amount of the two or more biomarkers can be combined with each other in a formula to form an index value.

In the methods and uses of the invention, the presence and/or amount of the one or more biomarker of the invention in a patient (or the profile of biomarkers in a patient) may be measured relative to a corresponding reference value. As such, the presence and/or amount of the one of more biomarker of the invention (or the profile of biomarkers) may be "compared" to a corresponding reference value.

The terms "comparison", "comparing" and "compared" are used herein interchangeably, and includes any means to discern at least one difference in the presence and/or amount of the one or more biomarker in the test sample as compared to a reference value (or as compared to a further sample obtained from the patient where monitoring of a systemic inflammatory condition takes place). In one embodiment, the methods of the invention described herein may involve comparison of "the amount of the one or more biomarker" in the test sample as compared to a reference value. In one embodiment, the methods of the invention described herein may involve comparison of "the presence and amount of the one or more biomarker" in the test sample as compared to a reference value.

A comparison may include a visual inspection of chromatographic spectra, and a comparison may include arithmetical or statistical comparisons of values assigned to the features of the profiles. Such statistical comparisons include, but are not limited to, applying a decision rule. If the biomarker profiles comprise at least one internal standard, the comparison to discern a difference in the biomarker profiles may also include features of these internal standards, such that features of the biomarker are correlated to features of the internal standards. As described herein, the comparison can be used to diagnose, monitor or prognose a systemic inflammatory condition, such as sepsis, abdominal sepsis, pulmonary sepsis or SIRS, and can be used to distinguish between sepsis and SIRS in a patient, or it can be used to distinguish between abdominal sepsis and pulmonary sepsis in a patient.

The term "reference value" refers to a value that is representative of a control individual or population whose disease state is known. A reference value can be determined for any particular population, subpopulation, or group of individuals according to standard methods well known to those of skill in the art. The actual amount of the one or more biomarkers, such as the mass, molar amount, concentration or molarity of the one or more biomarker of the invention may be assessed and compared with the corresponding reference population. Alternatively, the amount of one or more biomarker of the invention may be compared with that of the reference population without quantifying the mass, molar amount, concentration or molarity of the one or more biomarker.

The reference value may be obtained from a healthy individual or a population of healthy individuals eg. by quantifying the amount of the one or more biomarker in a sample obtained from the healthy individual or the population of healthy individuals. As used herein, "healthy" refers to a subject or group of individuals who are in a healthy state, e.g. patients who have not shown any symptoms of the disease, have not been previously diagnosed with the disease and/or are not likely to develop the disease. In one embodiment, the healthy individual (or population of healthy individuals) is not on medication affecting the disease and has not been diagnosed with any other disease. In one embodiment, the healthy individual (or population of healthy individuals) has similar sex, age and body mass index (BMI) as compared with the test patient. In one embodiment, the healthy individual (or population of healthy individuals) does not have a current infection or a chronic infection. Application of standard statistical methods used in medicine permits determination of normal levels of expression, as well as significant deviations from such normal levels.

The reference value may be obtained from an individual or a population of individuals suffering from the disease eg. by quantifying the amount of the one or more biomarker in a sample obtained from the individual or the population of individuals suffering from the disease. The reference data is typically collected from individuals that present at a medical centre with clinical signs relating to the relevant disease of interest. The reference value may be obtained, for example, from an individual or population of individuals having a systemic inflammatory condition, such as those having sepsis (including those having abdominal sepsis or pulmonary sepsis) or those having SIRS. Such individual(s) may have similar sex, age and body mass index (BMI) as compared with the test patient.

In one embodiment, the reference value is obtained from an individual or population of individuals having sepsis. In one embodiment, the reference value is obtained from an individual or population of individuals having abdominal sepsis. In one embodiment, the reference value is obtained from an individual or population of individuals having pulmonary sepsis. In one embodiment, the individual (or population of individuals) presents at hospital with sepsis (such as abdominal sepsis or pulmonary sepsis) of less than 72 hours duration. The reference values may be obtained from individuals having sepsis may be obtained at any stage in the progression of sepsis, such as infection, bacteremia, severe sepsis, septic shock of multiple organ failure. For example, the reference values may be obtained from patients having severe sepsis and/or septic shock. Diagnosis of sepsis (such as severe sepsis and/or septic shock) is based on the conventional diagnosis methods defined herein.

In one embodiment, the reference value is obtained from an individual or a population of individuals having SIRS. Diagnosis of SIRS is based on the SIRS criteria defined herein. In one embodiment, the individual (or population of individuals) may have organ failure defined as SOFA score > 2. In one embodiment, the individual or a population of individuals having SIRS has not been treated with antibiotics for treatment of known or suspected infection. In one embodiment, the individual or a population of individuals having SIRS have been admitted to a medical care facility following out-of hospital cardiac arrest.

The reference value may be obtained from an individual or a population of individuals who are diagnosed as having sepsis (eg. abdominal or pulmonary sepsis) or SIRS by conventional methods about 24, 48, 72, 96 or 120 hours or more after biological samples were taken for the purpose of generating a reference sample. In one embodiment, the individual or a population of individuals is diagnosed as having sepsis (eg. abdominal or pulmonary sepsis) or SIRS using conventional methods about 24-48 hours, about 48-72 hours, about 72-96 hours, or about 96-120 hours after the biological samples were taken. Conventional methods for confirming diagnosis of sepsis and SIRS are as defined herein.

The sample(s) used to generate the reference values may be obtained from the individual (or population of individuals) that present at a medical centre with clinical signs relating to the relevant disease of interest at any of the time points described herein for sample collection from the test patient. All embodiments described herein for the timing of sample collection from a test patient thus apply equally to the time point at which samples are obtained from the reference individual (or population of individuals) for the purpose of generating a reference value. For example, the sample used to generate the reference value may be obtained from an individual (or population of individuals) up to 24 hours after the individual (or population of individuals) presents with one or more clinical symptoms of a systemic inflammatory condition, or is admitted to a medical care facility. The individual (or population of individuals) from which the sample is obtained is then later on confirmed as having a systemic inflammatory condition using the conventional methods described herein.

In one embodiment, the reference values used in the comparison step of the method are generated from a sample obtained at the same time point (or time period) as the sample obtained from the test patient. For example, if a sample is obtained from a test patient up to 24 hours after the patient presents with one or more clinical symptoms of a systemic inflammatory condition or is admitted to a medical care facility, then the corresponding reference value may be obtained from an individual (or population of individuals) up to 24 hours after the individual (or population of individuals) presents with one or more clinical symptoms of a systemic inflammatory condition or is admitted to a medical care facility. Likewise, if a sample is obtained from a test patient up to 48 hours after the patient presents with one or more clinical symptoms of a systemic inflammatory condition or is admitted to a medical care facility, then the corresponding reference value may be obtained from an individual (or population of individuals) up to 48 hours after the individual (or population of individuals) presents with one or more clinical symptoms of a systemic inflammatory condition or is admitted to a medical care facility.

The individuals from which samples are obtained for generation of reference data may be subject to further follow-on consultations to confirm clinical assessments, as well as to identify further changes in biomarkers, changes in the severity of clinical signs over a period of time, and/or survival outcome. The reference data collected may include series data to indicate the progression or regression of the disease, so that the data can be used to determine if the condition of a test individual is improving, worsening or static. The reference data collected from patients that recover from the systemic inflammatory disease, can be used as a reference value that is representative of an individual having a (good) prognosis of recovery from the systemic inflammatory condition. The reference data collected from patients that do not recover from the systemic inflammatory disease, can be used as a reference value that is representative of an individual having a prognosis of non-recovery from the systemic inflammatory condition (or a poor prognosis of recovery from the systemic inflammatory condition).

Multiple separate reference values may be used in the methods of the invention. For example, reference values may include those that are representative of one or more of (eg. two or more, three or more, four or more, or all five of): (i) an individual (or a population of individuals) having sepsis, (ii) an individual (or a population of individuals) having SIRS; (iii) an individual (or a population of individuals) having abdominal sepsis; (iv) an individual (or a population of individuals) having pulmonary sepsis; and/or (v) a healthy individual (or a population of healthy individuals).

For example, the methods of the invention may use reference values that are representative of: (i) an individual (or a population of individuals) having sepsis; (ii) an individual (or a population of individuals) having SIRS; and (iii) a healthy individual (or a population of healthy individuals). For example, the methods of the invention may use reference values that are representative of: (i) an individual (or a population of individuals) having sepsis; and (ii) an individual (or a population of individuals) having SIRS. For example, the methods of the invention may use reference values that are representative of: (i) an individual (or a population of individuals) having sepsis; and (ii) a healthy individual (or a population of healthy individuals). For example, the methods of the invention may use reference values that are representative of: (i) an individual (or a population of individuals) having SIRS; and (ii) a healthy individual (or a population of healthy individuals).

For example, the methods of the invention may use reference values that are representative of: (i) an individual (or a population of individuals) having abdominal sepsis; (ii) an individual (or a population of individuals) having pulmonary sepsis; (iii) an individual (or a population of individuals) having SIRS; and (iv) a healthy individual (or a population of healthy individuals). For example, the methods of the invention may use reference values that are representative of: (i) an individual (or a population of individuals) having abdominal sepsis; (ii) an individual (or a population of individuals) having pulmonary sepsis; and (iii) an individual (or a population of individuals) having SIRS. For example, the methods of the invention may use reference values that are representative of: (i) an individual (or a population of individuals) having abdominal sepsis; (ii) an individual (or a population of individuals) having pulmonary sepsis; and (iii) a healthy individual (or a population of healthy individuals). For example, the methods of the invention may use reference values that are representative of: (i) an individual (or a population of individuals) having abdominal sepsis; (ii) an individual (or a population of individuals) having SIRS; and (iii) a healthy individual (or a population of healthy individuals). For example, the methods of the invention may use reference values that are representative of: (i) an individual (or a population of individuals) having pulmonary sepsis; (ii) an individual (or a population of individuals) having SIRS; and (iii) a healthy individual (or a population of healthy individuals). For example, the methods of the invention may use reference values that are representative of: (i) an individual (or a population of individuals) having abdominal sepsis; and (ii) an individual (or a population of individuals) having pulmonary sepsis. For example, the methods of the invention may use reference values that are representative of: (i) an individual (or a population of individuals) having abdominal sepsis; and (ii) a healthy individual (or a population of healthy individuals). For example, the methods of the invention may use reference values that are representative of: (i) an individual (or a population of individuals) having pulmonary sepsis; and (ii) a healthy individual (or a population of healthy individuals). For example, the methods of the invention may use reference values that are representative of: (i) an individual (or a population of individuals) having abdominal sepsis; and (ii) an individual (or a population of individuals) having SIRS. For example, the methods of the invention may use reference values that are representative of: (i) an individual (or a population of individuals) having pulmonary sepsis; and (ii) an individual (or a population of individuals) having SIRS.

The reference value may be obtained from the same (test) patient, provided that the test and reference values are generated from biological samples taken at different time points and compared to one another. For example, a sample may be obtained from a patient at the start of a study period. A reference value taken from that sample may then be compared to biomarker profiles generated from subsequent samples from the same patient. Such a comparison may be used, for example, to monitor a systemic inflammatory condition (ie. determine the progression of a systemic inflammatory condition in the patient by repeated classifications over time). Although the invention does not require a monitoring period to classify a patient, it will be understood that repeated classifications of the patient, i.e., repeated snapshots, may be taken over time until the individual is no longer at risk. Alternatively, a profile of biomarkers obtained from the patient may be compared to one or more profiles of biomarkers obtained from the same patient at different points in time.

In one embodiment, the reference value is obtained from a single individual eg. by quantifying the amount of a biomarker in a sample or samples derived from a single individual. Alternatively, the reference value may be derived by pooling data obtained from two or more (e.g. at least three, four, five, 10, 15, 20 or 25) individuals (ie. a population of individuals) and calculating an average (for example, mean or median) amount for a biomarker. Thus, the reference value may reflect the average amount of a biomarker in a given population of individuals. Said amounts may be expressed in absolute or relative terms, in the same manner as described above in relation to the sample that is to be tested using the method of the invention. As used herein, the term "population of individuals" refers to a group of two or more individuals, such as at least three, four, five, 10, 15, 20 or 25 individuals.

When comparing between the sample and the reference value, the way in which the amounts are expressed is matched between the sample and the reference value. Thus, an absolute amount can be compared with an absolute amount, and a relative amount can be compared with a relative amount.

The reference value may be derived from the same sample as the sample that is being tested, thus allowing for an appropriate comparison between the two. Thus, by way of example, if the sample is derived from a blood sample, then the reference value will also be a blood sample.

When the amounts of two or more biomarkers are determined, the amount of each biomarker may be compared to its corresponding reference value. Alternatively, when the cumulative amount of all the biomarkers is determined, the cumulative amount the biomarkers may be compared to a cumulative corresponding reference value. Alternatively, when the amount of the two or more biomarkers are combined with each other in a formula to form an index value, the index value can be compared to a corresponding reference index value derived in the same manner.

The reference values may be obtained either within (ie. constituting a step of) or separately to (ie. not constituting a step of) the methods of the invention. In one embodiment, the methods of the invention may comprise a step of establishing a reference value for the quantity of the markers. In one embodiment, the reference values are obtained separately to the method of the invention and accessed (eg. on a database) during the comparison step of the invention.

As illustrated in Figure 1, the present inventors observed that all of biomarkers shown in Table 1 increased in abundance in samples obtained from patients having a systemic inflammatory condition (such as sepsis or SIRS), as compared to healthy individuals. Detecting elevated levels of one or more of these biomarkers in a patient can thus be used to diagnose the presence of a systemic inflammatory condition in a patient. In particular, the differences in marker abundance between individuals having a systemic inflammatory condition and individuals that are healthy provides a way to classify individuals as having a systemic inflammatory condition or not having a systemic inflammatory condition by determining their marker profile.

By comparing the presence and/or amount of markers quantified in a sample obtained from a test patient to the presence and/or amount of markers quantified for a reference value (such as that obtained from a population of healthy individuals, or from a population of individuals having sepsis (eg. abdominal sepsis or pulmonary sepsis) or SIRS), it is possible to diagnose whether the patient has a systemic inflammatory condition (such as abdominal sepsis, pulmonary sepsis or SIRS). The method permits classification of the patient as belonging to or not belonging to the reference population (ie. by determining whether the amounts of marker quantified in the patient are statistically similar to the reference population or statistically deviate from the reference population). Hence, classification of the patient's marker profile (ie. the overall pattern of change observed for the markers quantified) as corresponding to the profile derived from a particular reference population is predictive that the patient falls (or does not fall) within the reference population.

In one embodiment, a patient may be diagnosed as having or being at risk of having a systemic inflammatory condition (such as sepsis (eg. abdominal sepsis or pulmonary sepsis) or SIRS), when the amount of the one or more biomarker is statistically similar to the amount determined for the corresponding reference value representative of an individual (or a population of individuals) having sepsis (eg. abdominal sepsis or pulmonary sepsis) and/or the amount determined for the corresponding reference value representative of an individual (or a population of individuals) having SIRS. In one embodiment, a patient may be diagnosed as not having or not being at risk of having a systemic inflammatory condition (such as sepsis (eg. abdominal sepsis or pulmonary sepsis) or SIRS) when the amount of the one or more biomarker is statistically similar to the amount determined for the corresponding reference value representative of a healthy individual (or a population of healthy individuals).

As used herein, the term "statistically similar" means that the amount of the one or more biomarker quantified for the test patient is similar to the amount quantified for the reference population to a statistically significant level. The term "statistically significant" means that the alteration is greater than what might be expected to happen by chance alone (p = < 0.05). Statistical significance can be determined by any method known in the art.

In one embodiment, a patient may be diagnosed as having or being at risk of having a systemic inflammatory condition (such as sepsis (eg. abdominal sepsis or pulmonary sepsis) or SIRS) when the amount of the one or more biomarker statistically deviates from the amount determined for the corresponding reference value representative of a healthy individual (or a population of healthy individuals). In one embodiment, a patient may be diagnosed as not having or not being at risk of having a systemic inflammatory condition (such as sepsis (eg. abdominal sepsis or pulmonary sepsis) or SIRS) when the amount of the one or more biomarker statistically deviates from the amount determined for the corresponding reference value representative of an individual (or a population of individuals) having sepsis (eg. abdominal sepsis or pulmonary sepsis) and/or an individual (or a population of individuals) having SIRS.

As used herein, the term "statistically deviates" means that the amount of the one or more biomarker quantified for the test patient differs from the amount quantified for the reference population to a statistically significant level. The term "statistically significant" means that the alteration is greater than what might be expected to happen by chance alone (p = < 0.05). Statistical significance can be determined by any method known in the art. The deviation in biomarker abundance may be an increase or decrease. The increase or decrease may be statistically significant.

The amount of the one or more biomarker of the invention, for example in a biomarker profile, may differ by at least 10%, at least 20%, at least 30%, at least 40%, at least 50%, at least 60, at least 70%, at least 80%, at least 90%, at least 100%, at least 150%, at least 200% or more compared with a corresponding reference value.

The amount of the one or more biomarker of the invention, for example in a biomarker profile, may differ by at least 0.1, at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, or at least 50 fold as compared to a corresponding reference value.

For example, if the amount of the one or more biomarker of the invention, typically in a biomarker profile, is reduced compared with a corresponding reference value, the expression may be reduced partially or totally compared with the corresponding reference value. Typically the amount is reduced by at least 10%, at least 20%, at least 30%, at least 40%, at least 50%, at least 60, at least 70%, at least 80%, at least 90%, at least 95%, at least 99%, up to total elimination of the one or more biomarker. Typically the amount is reduced by at least 0.1, at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, or at least 50 fold as compared to a corresponding reference value. For example, the fold decrease may be at least 0.5 fold. For example, the fold decrease may be at least 1 fold. For example, the fold decrease may be at least 1.5 fold. For example, the fold decrease may be at least 2 fold. For example, the fold decrease may be at least 2.5 fold. For example, the fold decrease may be at least 3 fold. For example, the fold decrease may be at least 3.5 fold. For example, the fold decrease may be at least 4 fold. For example, the fold decrease may be at least 4.5 fold. For example, the fold decrease may be at least 5 fold. The decrease in the amount of the marker may be statistically significant.

For example, if the amount of one or more biomarker of the invention, typically in a biomarker profile, is increased compared with a corresponding reference value, the amount may be increased by at least 10%, at least 20%, at least 30%, at least 40%, at least 50%, at least 60, at least 70%, at least 80%, at least 90&, at least 100%, at least 150%, at least 200% compared with the corresponding reference value. The amount may be increased by at least 0.1, at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, or at least 50 fold as compared to a corresponding reference value. For example, the fold increase may be at least 0.5 fold. For example, the fold increase may be at least 1 fold. For example, the fold increase may be at least 1.5 fold. For example, the fold increase may be at least 2 fold. For example, the fold increase may be at least 2.5 fold. For example, the fold increase may be at least 3 fold. For example, the fold increase may be at least 3.5 fold. For example, the fold increase may be at least 4 fold. For example, the fold increase may be at least 4.5 fold. For example, the fold increase may be at least 5 fold. The increase in the amount of the marker may be statistically significant.

The amount of the one or more biomarker may be altered compared with a corresponding reference value for at least 12 hours, at least 24 hours, at least 30 hours, at least 48 hours, at least 72 hours, at least 96 hours, at least 120 hours, at least 144 hours, at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks or more.

As described herein, the present inventors observed that all of biomarkers shown in Table 1 increased in abundance in samples obtained from patients having a systemic inflammatory condition, as compared to healthy individuals. Detecting elevated levels of one or more of these biomarkers in a patient can thus be used to diagnose the presence of a systemic inflammatory condition in a patient.

Thus, in one embodiment, when the reference value is representative of a healthy individual (or population of healthy individuals), an increase in the one or more biomarker in the sample obtained from the patient relative to the corresponding reference value, indicates that the patient has or is at risk of having a systemic inflammatory condition. Likewise, no increase in the one or more biomarker in the sample obtained from the patient relative to the corresponding reference value, indicates that the patient does not have a systemic inflammatory condition.

The present inventors observed that the overall increase in biomarker abundance observed in patients having a systemic inflammatory condition varied between different biomarkers, with some biomarkers showing very significant increases in abundance, and others showing more subtle changes.

In one embodiment, the patient may be diagnosed as having a systemic inflammatory condition, or being at risk of developing a systemic inflammatory condition, when the one or more biomarker (or the one or more additional biomarker) increases by at least 0.1 (e.g. at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value representative of a healthy individual.

The 'comparison' step of the methods of the invention may comprise applying a decision rule, or using a decision tree. A "decision rule" or a "decision tree" is a method used to classify individuals. This rule can take on one or more forms that are known in the art, as exemplified in Hastie et al., in "The Elements of Statistical Learning" Springer-Nerlag (Springer, New York (2001)). Analysis of biomarkers in the complex mixture of molecules within the sample generates features in a data set. A decision rule or a decision tree may be used to act on a data set of features to diagnose, monitor, or prognose a systemic inflammatory condition (such as sepsis or SIRS), to distinguish between sepsis and SIRS in a patient, or to distinguish between abdominal sepsis and pulmonary sepsis.

The decision rule or decision tree can comprise a data analysis algorithm, such as a computer pattern recognition algorithm. Other suitable algorithms include, but are not limited to, logistic regression or a nonparametric algorithm that detects differences in the distribution of feature values (e.g., a Wilcoxon Signed Rank Test). The decision rule may be based upon one, two, three, four, five, 10, 20 or more features. In one embodiment, the decision rule or decision tree is based on hundreds or more of features. Applying the decision rule or decision tree may also comprise using a classification tree algorithm. For example, the reference value (or reference biomarker profile) may comprise at least three features or biomarkers, where the features are predictors in a classification tree algorithm. The data analysis algorithm predicts membership within a population (or class) with an accuracy of at least about 60%, at least about 70%, at least about 80% and at least about 90%.

Suitable algorithms are known in the art, some of which are reviewed in Hastie *et al,* supra. Such algorithms classify complex spectra from biological materials, such as a blood sample, to distinguish individuals as normal or as possessing biomarker expression levels characteristic of a particular disease state. While such algorithms may be used to increase the speed and efficiency of the application of the decision rule and to avoid investigator bias, one of ordinary skill in the art will realise that computer-based algorithms are not required to carry out the methods of the present invention.

Algorithms may be applied to the comparison of the one or more biomarker or the biomarker profiles, regardless of the method that was used to generate the data for the one or more biomarker or the biomarker profile. For example, suitable algorithms can be applied to biomarker profiles generated using gas chromatography, as discussed in Harper, "Pyrolysis and GC in Polymer Analysis" Dekker, New York (1985). Further, Wagner et al, Anal Chem 74: 1824-35 (2002) disclose an algorithm that improves the ability to classify individuals based on spectra obtained by static time-of-flight secondary ion mass spectrometry (TOF-SIMS). Additionally, Bright et al, J. Microbiol Methods 48: 127-38 (2002) disclose a method of distinguishing between bacterial strains with high certainty (79- 89% correct classification rates) by analysis of MALDI-TOF-MS spectra. Dalluge, Fresenius J. Anal. Chem. 366: 701-11 (2000) discusses the use of MALDI-TOF-MS and liquid chromatography-electrospray ionization mass spectrometry (LC/ESI-MS) to classify profiles of biomarkers in complex biological samples.

The methods and uses of the invention may thus comprise applying a decision rule as described herein. Applying the decision rule may comprise using a data analysis algorithm, also as described herein. The data analysis algorithm may comprise at least two, at least three, at least four, at least five, at least six, at least seven, at least eight, at least nine, at least ten, at least 15, at least 20, at least 25, at least 50 or more input parameters. The data analysis algorithm may use any of the biomarkers of the invention, or combination of biomarkers of the invention as input parameters. Typically, the data analysis algorithm uses at least two, at least three, at least four, at least five, at least six, at least seven, at least eight, at least nine, at least ten, at least 15, at least 20, at least 25, at least 50 of the biomarkers of the invention (e.g. as listed in any one of Tables 1 to 4) as input parameters.

The application of the decision rule or the decision tree does not require perfect classification. A classification may be made with at least about 90% certainty, or even more, in one embodiment. In other embodiments, the certainty is at least about 80%, at least about 70%, or at least about 60%. The useful degree of certainty may vary, depending on the particular method of the present invention. "Certainty" is defined as the total number of accurately classified individuals divided by the total number of individuals subjected to classification. As used herein, "certainty" means "accuracy".

Classification may also be characterized by its "sensitivity". The "sensitivity" of classification relates to the percentage of individuals who were correctly identified as having a particular disease or condition eg. the percentage of individuals who were correctly identified as having a systemic inflammatory condition (such as sepsis or SIRS). "Sensitivity" is defined in the art as the number of true positives divided by the sum of true positives and false negatives.

The "specificity" of a method is defined as the percentage of patients who were correctly identified as not having particular disease or condition, eg. the percentage of individuals who were correctly identified as not having a systemic inflammatory condition (such as sepsis or SIRS). That is, "specificity" relates to the number of true negatives divided by the sum of true negatives and false positives.

Typically, the accuracy, sensitivity and/or specificity of the methods and uses of the invention is at least about 90%, at least about 80%, at least about 70% or at least about 60%.

The method for diagnosing a systemic inflammatory condition in a patient as described herein can be used in a decision tree process to investigate the health of a patient having or suspected of having a systemic inflammatory condition. For example, the method for diagnosing a systemic inflammatory condition in a patient can be performed before, after, or in addition to any of the other methods described herein.

In one embodiment, the method for diagnosing a systemic inflammatory condition in a patient is performed as described herein. If the patient tests positive for a systemic inflammatory condition, they may be tested using the method for distinguishing between sepsis and SIRS described herein to determine whether the patient has sepsis and/or SIRS. In one embodiment, the above combination of methods are performed as described, and if the patient tests positive for sepsis, the patient may be further tested for sepsis, abdominal sepsis and/or pulmonary sepsis using the diagnostic methods described herein, so as to confirm whether the patient has or is at risk of developing sepsis, and/or determine whether the patient has or is at risk of developing abdominal sepsis and/or pulmonary sepsis. If the patient tests positive for SIRS, the patient may be further tested for SIRS using the diagnostic method described herein, so as to confirm whether the patient has or is at risk of developing SIRS. If the patient tests positive for sepsis using the method for diagnosis of sepsis (as described herein), the patient may be further tested for abdominal sepsis and/or pulmonary sepsis using the methods described herein.

In one embodiment, the method of the invention for diagnosing a systemic inflammatory condition in a patient is performed as described herein. If the patient tests positive for a systemic inflammatory condition, they may be tested for sepsis, abdominal sepsis, pulmonary sepsis and/or SIRS using the diagnostic methods described herein. The methods for diagnosis of sepsis, abdominal sepsis, pulmonary sepsis and/or SIRS may be performed simultaneously or sequentially in any order.

The above described combination of methods may also be performed in parallel to determine the disease status of a patient by simultaneously (or substantially simultaneously) investigating the expression of all the biomarkers in a sample obtained from the patient, and determining whether the patient has or is at risk of having a systemic inflammatory condition, sepsis (such as abdominal or pulmonary sepsis) and/or SIRS.

When performing these different methods in a decision tree process, the sample used in each step of the method may be the same sample obtained from the patient. When the method comprises multiple quantification steps, these multiple steps may be performed at the same time (e.g. in parallel) and/or using the same sample. When the method comprises multiple comparison steps, these multiple steps may be performed at the same time (e.g. in parallel).

In all methods described herein, any appropriate technique may be used to confirm the diagnosis. Standard techniques are known in the art. For example, confirmation of a diagnosis of a systemic inflammatory condition in a patient may include: testing for the presence of other known biomarkers of a systemic inflammatory condition including: C-reactive protein (CRP), Procalcitonin (PCT), lactate, Cystatin C (CYTC), Neutrophil gelatinase-associated lipocalin (NGAL) and interleukin 6 (IL6).

Additional clinical parameters that may be used to confirm the diagnosis also include: white blood cell count, kidney function tests (such as serum creatinine, or urine output), respiratory system function tests (such as PaO2/FiO2), nervous system function tests (expressed as Glasgow coma scale), cardiovascular function tests (expressed as mean arterial pressure), liver function tests (such as bilirubin concentration), and coagulation function tests (such as platelet concentration). The methods and uses of the invention may further comprise determining such clinical parameters in the patient.

In a related aspect, the present invention also provides the use of one or more of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1 as a biomarker for a systemic inflammatory condition. For example, the one or more biomarker may be selected from: FAM20A, OLAH and/or CD177.

In one embodiment, the use is of the one or more biomarker in the diagnosis of a systemic inflammatory condition in a patient. For example, the use may comprise (i) determining the presence and/or amount of one or more biomarker in a sample obtained from a patient; and (ii) comparing the presence and/or amount of the one or more biomarker determined in said sample in (i) to a corresponding reference value to determine whether the patient has a systemic inflammatory condition.

All embodiments described above for the method of diagnosing a systemic inflammatory condition in a patient apply equally to the 'use' of the invention described herein. This includes all embodiments relating to the terms "systemic inflammatory condition", "patient", "sample", and "the one or more biomarker".

### Diagnosis of SIRS

When investigating gene expression patterns in patients having systemic inflammatory conditions, the inventors observed that certain biomarkers (see Table 2) were particularly elevated in abundance in patients having SIRS, as compared to patients having other systemic inflammatory conditions, and healthy individuals. As a result of these findings, the inventors thus observed that PLA2G7, ARHGEF10L, MYCL, TGFBI, GPR124, IL1RN, NLRP3, RBP4, and MPP3 can be used as biomarkers for diagnosis of SIRS.

The present invention therefore provides a method for diagnosing SIRS in a patient, comprising:
(i) determining the presence and/or amount of one or more biomarker in a sample obtained from a patient, wherein the one or more biomarker is selected from the group consisting of: PLA2G7, ARHGEF10L, MYCL, TGFBI, GPR124, IL1RN, NLRP3, RBP4, and MPP3;
(ii) comparing the presence and/or amount of the one or more biomarker determined in said sample in (i) to a corresponding reference value; and thereby determining whether the patient has or is at risk of developing SIRS.

All embodiments described above for the "method for diagnosing a systemic inflammatory condition in a patient" apply equally to the "method for diagnosing SIRS in a patient". This includes all embodiments relating to the "sample", "patient", "biomarker", and "reference value", and all embodiments relating to the quantification step for "determining the presence and/or amount of one or more biomarker in a sample" and the "comparison" step used to make a conclusion about the disease state of the patient.

As used herein, the phrase "diagnosis of SIRS in a patient" means determining whether the patient has or is risk of developing SIRS. The systemic inflammatory condition "SIRS" diagnosed using the method of the invention is as described above for the "method for diagnosing a systemic inflammatory condition in a patient".

The "patient" for which diagnosis is performed is as described above for the "method for diagnosing a systemic inflammatory condition in a patient". In one embodiment, the patient is suspected of having or being at risk of developing a systemic inflammatory condition. In one embodiment, the patient has been diagnosed as having or being at risk of developing a systemic inflammatory condition. In one embodiment, the patient has been diagnosed as having a systemic inflammatory condition using the method described herein. In one embodiment, the patient has been diagnosed as having or being at risk of developing SIRS and/or sepsis using the method of the invention for distinguishing between sepsis and SIRS in a patient as described herein. In one embodiment, the patient is suspected of having or being at risk of developing SIRS.

The "sample" obtained from the patient is as described above for the "method for diagnosing a systemic inflammatory condition in a patient", including all embodiments relating to the time point at which the sample is obtained.

The "one or more biomarker" of the invention is as described above for the "method for diagnosing a systemic inflammatory condition in a patient".

As illustrated in Example 1, the present inventors observed that PLA2G7, ARHGEF10L, MYCL, TGFBI, GPR124, IL1RN, NLRP3, RBP4, and MPP3 are elevated in patients having SIRS, and can thus be used as biomarkers for diagnosis of SIRS.

The reference to the biomarker MYCL throughout the entire description, includes the transcript variant 1 of MYCL (as encoded by SEQ ID NO: 37) and the transcript variant 3 of MYCL (as encoded by SEQ ID NO: 38). In one embodiment, the reference to the biomarker MYCL is a reference to the transcript variant 1 of MYCL (as encoded by SEQ ID NO: 37). In one embodiment, the reference to the biomarker MYCL is a reference to the transcript variant 3 of MYCL (as encoded by SEQ ID NO: 38).

In one embodiment, the one or more biomarker may be selected from the group consisting of: PLA2G7, ARHGEF10L, MYCL, TGFBI, GPR124, IL1RN, NLRP3, RBP4, and MPP3. For example, the one or more biomarker may be selected from the group consisting of: PLA2G7, ARHGEF10L, MYCL, TGFBI, GPR124, RBP4, and MPP3. For example, the one or more biomarker may be selected from the group consisting of: MYCL, TGFBI, GPR124, NLRP3, and MPP3. For example, the one or more biomarker may be selected from the group consisting of: ARHGEF10L, TGFBI, GPR124, IL1RN, NLRP3, RBP4, and MPP3. For example, the one or more biomarker may be selected from the group consisting of: TGFBI, GPR124, IL1RN, NLRP3, RBP4, and MPP3. For example, the one or more biomarker may be selected from the group consisting of: GPR124, IL1RN, NLRP3, RBP4, and MPP3. For example, the one or more biomarker may be selected from the group consisting of: GPR124, NLRP3, and MPP3.

The present inventors observed that a sub-set of the biomarkers for SIRS (GPR124, TGFBI, PLA2G7, MYCL, and ARHGEF10L) increase in abundance in patients having SIRS compared to healthy individuals, but do not increase in abundance in patients having sepsis as compared to healthy individuals. These markers therefore provide highly specific biomarkers for diagnosing SIRS. Thus, in one embodiment, the one or more biomarker may be selected from the group consisting of: PLA2G7, ARHGEF10L, MYCL, TGFBI, and GPR124. For example, the one or more biomarker may be selected from the group consisting of: PLA2G7, ARHGEF10L, MYCL, and TGFBI.

Each of the biomarkers of SIRS may be used alone, or in combination with any of the SIRS biomarkers in the method of the invention. For example, any combination of 1 or more, 2 or more, 3 or more, 4 or more, 5 or more, 6 or more, 7 or more, 8 or more, up to and including all of the SIRS biomarkers may be used to diagnose SIRS in a patient according to the method of the invention.

In one embodiment, any combination of 1 or more (eg. 2 or more, 3 or more, 4 or more, 5 or more, 6 or more, 7 or more, 8 or more, or all 9) of the biomarkers selected from the group consisting of: PLA2G7, ARHGEF10L, MYCL, TGFBI, GPR124, IL1RN, NLRP3, RBP4, and MPP3, may be used to diagnose SIRS in a patient. In one embodiment, any combination of 1 or more (eg. 2 or more, 3 or more, 4 or more, or all 5) of the biomarkers selected from the group consisting of: PLA2G7, ARHGEF10L, MYCL, TGFBI, and GPR124, may be used to diagnose SIRS in a patient. For example, any combination of 1 or more (eg. 2 or more, 3 or more, or all 4) of the biomarkers selected from the group consisting of: ARHGEF10L, MYCL, TGFBI, and GPR124 may be used to diagnose SIRS in a patient. For example, any combination of 1 or more (eg. 2 or more, 3 or more, or all 4) of the biomarkers selected from the group consisting of:ARHGEF10L, MYCL, TGFBI ,and GPR124, may be used to diagnose SIRS in a patient. For example, any combination of 1 or more (eg. 2 or more, or all 3) of the biomarkers selected from the group consisting of: MYCL, TGFBI and GPR124, may be used to diagnose SIRS in a patient. For example, any combination of 1 or more (eg. 2 or more, or all 3) of the biomarkers selected from the group consisting of: PLA2G7, TGFBI and GPR124, may be used to diagnose SIRS in a patient. For example, any combination of 1 or more (or both) of the biomarkers selected from the group consisting of: MYCL and GPR124 , may be used to diagnose SIRS in a patient. For example, any combination of 1 or more (or both) of the biomarkers selected from the group consisting of: PLA2G7 and GPR124 , may be used to diagnose SIRS in a patient.

In a further example, the following combinations of SIRS biomarkers may be used in the method of the invention to diagnose SIRS: (i) TGFBI and PLA2G7; (ii) TGFBI and GPR124; (iii) TGFBI and MYCL; (iv) TGFBI and ARHGEF10L;(v) PLA2G7 and GPR124; (vi) PLA2G7 and MYCL; (vii) PLA2G7 and ARHGEF10L; (viii) GPR124 and MYCL; (ix) GPR124 and ARHGEF10L; (x) MYCL and ARHGEF10L.

As described in Example 2, a subset of the SIRS biomarkers (PLA2G7, ARHGEF10L, MYCL, and TGFBI) were shown to be particularly effective in diagnosing SIRS when tested by ROC analysis. Specifically, AUC values of 0.89, 0.8, 0.8, 0.79 were achieved for PLA2G7, ARHGEF10L, MYCL, and TGFBI. Thus, in one embodiment, any combination of 1 or more (eg. 2 or more, 3 or more, or all 4) of the biomarkers selected from the group consisting of: PLA2G7, ARHGEF10L, MYCL, and TGFBI, may be used to diagnose SIRS in a patient. For example, 2 or more of the biomarkers selected from the group consisting of: PLA2G7, ARHGEF10L, MYCL, and TGFBI, may be used to diagnose SIRS in a patient. For example, 3 or more of the biomarkers selected from the group consisting of: PLA2G7, ARHGEF10L, MYCL, and TGFBI, may be used to diagnose SIRS in a patient. In one embodiment, the method of the invention may be preferably performed using the combination of: PLA2G7, ARHGEF10L, MYCL, and TGFBI.

In one embodiment, the one or more biomarker is TGFBI. In one embodiment, the one or more biomarker is PLA2G7. In one embodiment, the one or more biomarker is MYCL. In one embodiment, the one or more biomarker is ARHGEF10L. In one embodiment, the one or more biomarker is GPR124. In one embodiment, the one or more biomarker is IL1RN. In one embodiment, the one or more biomarker is NLRP3. In one embodiment, the one or more biomarker is RBP4. In one embodiment, the one or more biomarker is MPP3.

One or more additional biomarker for SIRS may also be used in the diagnosis of SIRS according to the method of the invention. Any combination of the one or more additional biomarker may be used in combination with the one or more biomarker of the invention. For example at least 1, at least 2, at least 3, at least 4, at least 5, at least 6, at least 7, or all 8 additional biomakers for SIRS may be used in combination with the one or more biomarker of the invention (as described herein). Typically, the one or more additional biomarker is selected from the group consisting of: PLA2G7, ARHGEF10L, MYCL, TGFBI, GPR124, IL1RN, NLRP3, RBP4, and MPP3. For example, the one or more additional biomarker is selected from the group consisting of: PLA2G7, ARHGEF10L, MYCL, TGFBI and GPR124.

In one embodiment, the one or more biomarker is TGFBI, and the one or more additional biomarker is selected from at least 1 (eg. at least 2, at least 3, at least 4, at least 5, at least 6, at least 7, up to and including all) of the biomarkers: PLA2G7, ARHGEF10L, MYCL, GPR124, IL1RN, NLRP3, RBP4, and MPP3. In one embodiment, the one or more additional biomarker is selected from at least 1 (eg. at least 2, at least 3, up to and including all) of the biomarkers: PLA2G7, ARHGEF10L, MYCL, and GPR124. In one embodiment, the one or more additional biomarker is selected from at least 1 (eg. at least 2, up to and including all) of the biomarkers: PLA2G7, ARHGEF10L and MYCL.

In one embodiment, the one or more biomarker is PLA2G7, and the one or more additional biomarker is selected from at least 1 (eg. at least 2, at least 3, at least 4, at least 5, at least 6, at least 7, up to and including all) of the biomarkers: ARHGEF10L, MYCL, TGFBI, GPR124, IL1RN, NLRP3, RBP4, and MPP3. In one embodiment, the one or more additional biomarker is selected from at least 1 (eg. at least 2, at least 3, up to and including all) of the biomarkers: ARHGEF10L, MYCL, TGFBI, and GPR124, . In one embodiment, the one or more additional biomarker is selected from at least 1 (eg. at least 2, up to and including all) of the biomarkers: ARHGEF10L, MYCL and TGFBI.

In one embodiment, the one or more biomarker is MYCL, and the one or more additional biomarker is selected from at least 1 (eg. at least 2, at least 3, at least 4, at least 5, at least 6, at least 7, up to and including all) of the biomarkers: PLA2G7, ARHGEF10L, TGFBI, GPR124, IL1RN, NLRP3, RBP4, and MPP3. In one embodiment, the one or more additional biomarker is selected from at least 1 (eg. at least 2, at least 3, up to and including all) of the biomarkers: PLA2G7, ARHGEF10L, TGFBI and GPR124. In one embodiment, the one or more additional biomarker is selected from at least 1 (eg. at least 2, up to and including all) of the biomarkers: PLA2G7, ARHGEF10L and TGFBI.

In one embodiment, the one or more biomarker is ARHGEF10L, and the one or more additional biomarker is selected from at least 1 (eg. at least 2, at least 3, at least 4, at least 5, at least 6, at least 7, up to and including all) of the biomarkers: PLA2G7, MYCL, TGFBI, GPR124, IL1RN, NLRP3, RBP4, and MPP3. In one embodiment, the one or more additional biomarker is selected from at least 1 (eg. at least 2, at least 3, up to and including all) of the biomarkers: PLA2G7, MYCL, TGFBI, and GPR124. In one embodiment, the one or more additional biomarker is selected from at least 1 (eg. at least 2, up to and including all) of the biomarkers: PLA2G7, MYCL and TGFBI.

In one embodiment, the one or more biomarker is GPR124, and the one or more additional biomarker is selected from at least 1 (eg. at least 2, at least 3, at least 4, at least 5, at least 6, at least 7, up to and including all) of the biomarkers: PLA2G7, ARHGEF10L, MYCL, TGFBI, IL1RN, NLRP3, RBP4, and MPP3. In one embodiment, the one or more additional biomarker is selected from at least 1 (eg. at least 2, at least 3, up to and including all) of the biomarkers: PLA2G7, ARHGEF10L, MYCL, and TGFBI.

In one embodiment, the one or more biomarker is IL1RN, and the one or more additional biomarker is selected from at least 1 (eg. at least 2, at least 3, at least 4, at least 5, at least 6, at least 7, up to and including all) of the biomarkers: PLA2G7, ARHGEF10L, MYCL, TGFBI,GPR124, NLRP3, RBP4, and MPP3. In one embodiment, the one or more additional biomarker is selected from at least 1 (eg. at least 2, at least 3, at least 4, up to and including all) of the biomarkers: PLA2G7, ARHGEF10L, MYCL, TGFBI, and GPR124.

In one embodiment, the one or more biomarker is NLRP3, and the one or more additional biomarker is selected from at least 1 (eg. at least 2, at least 3, at least 4, at least 5, at least 6, at least 7, up to and including all) of the biomarkers: PLA2G7, ARHGEF10L, MYCL, TGFBI, GPR124, IL1RN, RBP4, and MPP3. In one embodiment, the one or more additional biomarker is selected from at least 1 (eg. at least 2, at least 3, at least 4, up to and including all) of the biomarkers: PLA2G7, ARHGEF10L, MYCL, TGFBI, and GPR124.

In one embodiment, the one or more biomarker is RBP4, and the one or more additional biomarker is selected from at least 1 (eg. at least 2, at least 3, at least 4, at least 5, at least 6, at least 7, up to and including all) of the biomarkers: PLA2G7, ARHGEF10L, MYCL, TGFBI, GPR124, IL1RN, NLRP3, and MPP3. In one embodiment, the one or more additional biomarker is selected from at least 1 (eg. at least 2, at least 3, at least 4, up to and including all) of the biomarkers: PLA2G7, ARHGEF10L, MYCL, TGFBI, and GPR124.

In one embodiment, the one or more biomarker is MPP3, and the one or more additional biomarker is selected from at least 1 (eg. at least 2, at least 3, at least 4, at least 5, at least 6, at least 7, up to and including all) of the biomarkers: PLA2G7, ARHGEF10L, MYCL, TGFBI, GPR124, IL1RN, NLRP3, and RBP4. In one embodiment, the one or more additional biomarker is selected from at least 1 (eg. at least 2, at least 3, at least 4, up to and including all) of the biomarkers: PLA2G7, ARHGEF10L, MYCL, TGFBI, and GPR124.

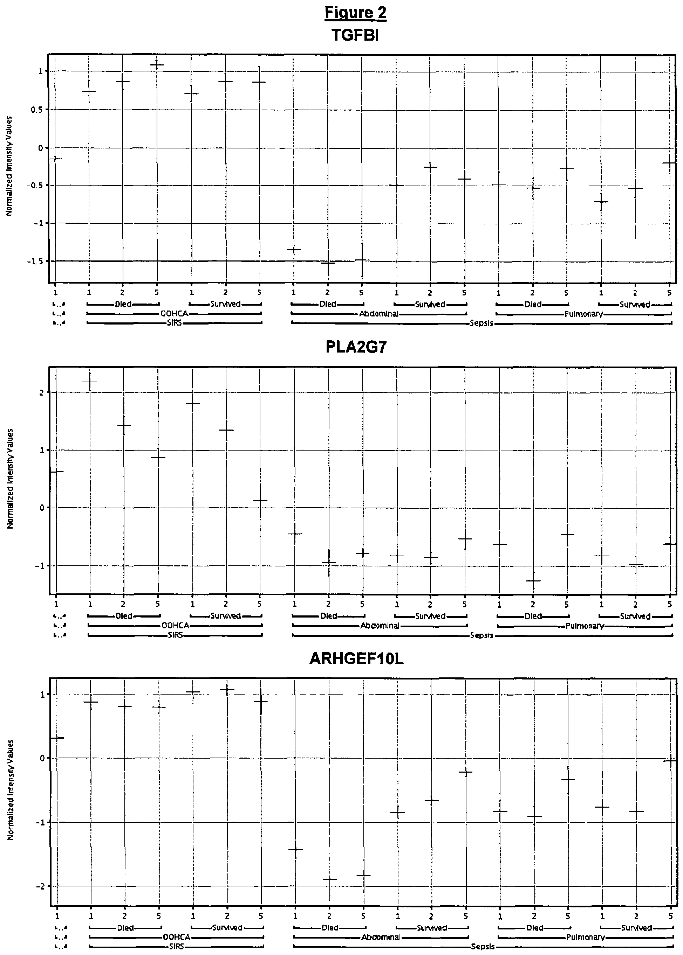

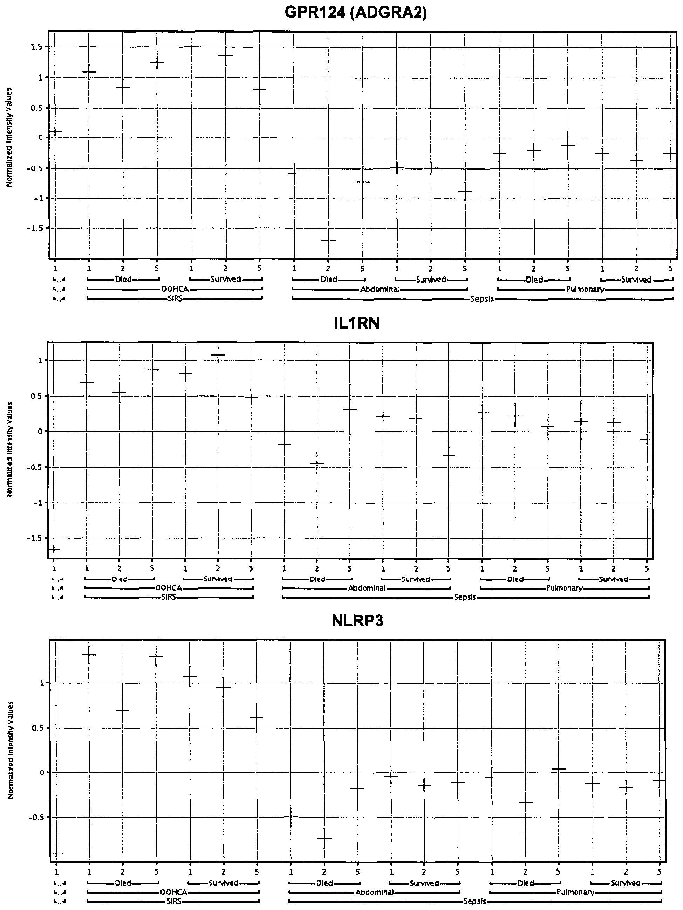

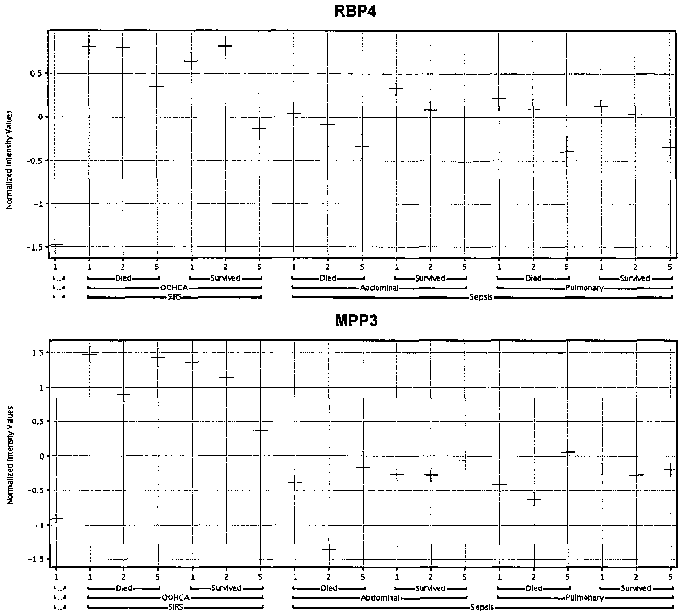

As illustrated in Figure 2, the present inventors observed that the "SIRS" biomarkers described herein increase in abundance in patients having SIRS as compared to patients having other systemic inflammatory conditions (such as abdominal sepsis or pulmonary sepsis), and as compared to healthy individuals. These differences in marker abundance can be used to diagnose whether an individual has or is at risk of developing SIRS.

For example, by comparing the presence and/or amount of markers quantified in a sample obtained from a patient to the presence and/or amount of markers quantified for a reference value (such as a reference value that is representative of a healthy individual (or a population of healthy individuals), a reference value that is representative of an individual (or a population of individuals) having sepsis (eg. abdominal and/or pulmonary sepsis), or a reference value that is representative of an individual (or a population of individuals) having SIRS, it is possible to diagnose the presence (or absence) of SIRS in a patient. The method permits classification of the patient as belonging to or not belonging to the reference population (ie. by determining whether the amounts of marker quantified in the individual are statistically similar to the reference population or statistically deviate from the reference population). Hence, classification of the patient's marker profile (ie. the overall pattern of change observed for the markers quantified) as corresponding to the profile derived from a particular reference population is predictive that the patient falls (or does not fall) within the reference population.

In one embodiment, a patient may be diagnosed as having or being at risk of having SIRS, when the amount of the one or more biomarker is statistically similar to the amount determined for the corresponding reference value representative of an individual (or a population of individuals) having SIRS. In one embodiment, a patient may be diagnosed as not having or not being at risk of having SIRS when the amount of the one or more biomarker is statistically similar to the amount determined for the corresponding reference value representative of a healthy individual (or a population of healthy individuals). In one embodiment, a patient may be diagnosed as not having or not being at risk of having SIRS when the amount of the one or more biomarker quantified is statistically similar to the amount determined for the corresponding reference value representative of an individual having sepsis (or a population of individuals having sepsis).

In one embodiment, a patient may be diagnosed as having or being at risk of having SIRS when the amount of the one or more biomarker statistically deviates from the amount determined for the corresponding reference value representative of a healthy individual (or a population of healthy individuals). In one embodiment, a patient may be diagnosed as having or being at risk of having SIRS when the amount of the one or more biomarker statistically deviates from the amount determined for the corresponding reference value representative of an individual having sepsis (or a population of individuals having sepsis). In one embodiment, a patient may be diagnosed as not having or not being at risk of having SIRS when the amount of the one or more biomarker statistically deviates from the amount determined for the corresponding reference value representative of an individual (or a population of individuals) having SIRS.

All embodiments described above for the classification of a patient as having or being at risk of having a systemic inflammatory condition (or as not having or not being at risk of having a systemic inflammatory condition) apply equally to the method for diagnosing whether a patient has or is at risk of having SIRS. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may as defined above for the method of diagnosing a systemic inflammatory condition in a patient. In one embodiment, the reference value is representative of a healthy individual (or a population of healthy individuals). In one embodiment, the reference value is representative of an individual having SIRS (or a population of individuals having SIRS). In one embodiment, the reference value is representative of an individual having sepsis (or a population of individuals having sepsis).

As described above for the "method for diagnosing a systemic inflammatory condition in a patient", the method of the invention may involve the use of multiple separate reference values. For example, the reference value may include one of more (eg. two or more, or all 3) of the reference values selected from: a reference value that is representative of a healthy individual (or a population of healthy individuals); a reference value that is representative of an individual having SIRS (or a population of individuals having SIRS); and a reference value that is representative of an individual having sepsis (or a population of individuals having sepsis).

The present inventors observed that the SIRS biomarkers described herein each increase in abundance in samples obtained from patients having SIRS, as compared to healthy individuals. Detection of increased levels of these biomarkers in a patient as compared to the levels detected for healthy individuals can thus be used to diagnose the presence of SIRS. In one embodiment, when the reference value is representative of a healthy individual (or population of healthy individuals), an increase in the one or more biomarker (and/ or one or more additional biomarker) in the sample obtained from the patient relative to the corresponding reference value indicates that the patient has or is at risk of developing SIRS. Likewise, no increase in the one or more biomarker (and/ or one or more additional biomarker) in the sample obtained from the patient relative to the corresponding reference value indicates that the patient does not have SIRS.

For some of the SIRS biomarkers identified by the present inventors, increased levels of these markers were also observed in patients having sepsis as compared to healthy individuals, although much bigger increases were observed for these biomarkers in the patients having SIRS. The accuracy of SIRS diagnosis can thus be improved by looking for a "minimum" fold change or % change in the levels of the one or more biomarkers as compared to the corresponding reference value that is representative of a healthy individual. The fold change or % change may be as defined above for the method for diagnosis of a systemic inflammatory condition.

In one embodiment, the patient may be diagnosed as having SIRS, or being at risk of developing SIRS, when the one or more biomarker (or the one or more additional biomarker) increases by at least 0.1 (e.g. at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value representative of a healthy individual.

For example, an increase of at least 1.1 (eg. at least 1.2, at least 1.3, at least 1.4, at least 1.5) fold in GPR124 in the sample obtained from the patient relative to the corresponding reference value, indicates that the patient has, or is at risk of developing SIRS. In one embodiment, no increase or an increase of less than 1.1 (eg. less than 1.2, less than 1.3, less than 1.4, less than 1.5, less than 1.6) fold in GPR124 in the sample obtained from the patient relative to the corresponding reference value, indicates that the patient does not have, or is not at risk of developing SIRS.

For example, an increase of at least 1.5 (eg. at least 1.6, at least 1.7, at least 1.8, at least 1.9, or at least 2) fold in TGFBI in the sample obtained from the patient relative to the corresponding reference value, indicates that the patient has, or is at risk of developing SIRS. In one embodiment, no increase or an increase of less than 1.5 (eg. less than 1.6, less than 1.7, or less than 1.8) fold in TGFBI in the sample obtained from the patient relative to the corresponding reference value, indicates that the patient does not have, or is not at risk of developing SIRS.

For example, an increase of at least 1.1 (eg. at least 1.2, at least 1.3, at least 1.4, at least 1.5, or at least 1.6) fold in PLA2G7 in the sample obtained from the patient relative to the corresponding reference value, indicates that the patient has, or is at risk of developing SIRS. In one embodiment, no increase or an increase of less than 1.1 (eg. less than 1.2, less than 1.3, less than 1.4, less than 1.5, or less than 1.6) fold in PLA2G7 in the sample obtained from the patient relative to the corresponding reference value, indicates that the patient does not have, or is not at risk of developing SIRS. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.1 (eg. at least 1.2, at least 1.3, or at least 1.4) fold in MYCL in the sample obtained from the patient relative to the corresponding reference value, indicates that the patient has, or is at risk of developing SIRS. In one embodiment, no increase or an increase of less than 1.1 (eg. less than 1.2, or less than 1.3, or less than 1.4) fold in MYCL in the sample obtained from the patient relative to the corresponding reference value, indicates that the patient does not have, or is not at risk of developing SIRS. In one embodiment, when detecting this biomarker, the method is performed using a sample obtained from a patient at least 24 (eg. at least 36, at least 48, at least 72, at least 96, or at least 120) hours after the patient presents with one or more clinical symptoms of a systemic inflammatory condition, or is admitted to a medical care facility.

For example, an increase of at least 1.1 (eg. at least 1.2, at least 1.3, at least 1.4, at least 1.5) fold in ARHGEF10L in the sample obtained from the patient relative to the corresponding reference value, indicates that the patient has, or is at risk of developing SIRS. In one embodiment, no increase or an increase of less than 1.1 (eg. less than 1.2, less than 1.3, less than 1.4, or less than 1.5) fold in ARHGEF10L in the sample obtained from the patient relative to the corresponding reference value, indicates that the patient does not have, or is not at risk of developing SIRS.

For example, an increase of at least 4 (eg. at least 4.1, at least 4.2, at least 4.3, at least 4.4, or at least 4.5) fold in IL1RN in the sample obtained from the patient relative to the corresponding reference value, indicates that the patient has, or is at risk of developing SIRS. In one embodiment, no increase or an increase of less than 4 (eg. less than 4.1, less than 4.2, less than 4.3, less than 4.4, or less than 4.5) fold in IL1RN in the sample obtained from the patient relative to the corresponding reference value, indicates that the patient does not have, or is not at risk of developing SIRS.

For example, an increase of at least 2 (eg. at least 2.1, at least 2.1, at least 2.3, at least 2.4, at least 2.5, at least 2.6, at least 2.7, at least 2.8, at least 2.9, at least 3) fold in NLRP3 in the sample obtained from the patient relative to the corresponding reference value, indicates that the patient has, or is at risk of developing SIRS. In one embodiment, no increase or an increase of less than 2 (eg. less than 2.1, less than 2.2, less than 2.3, less than 2.4, or less than 2.5) fold in NLRP3 in the sample obtained from the patient relative to the corresponding reference value, indicates that the patient does not have, or is not at risk of developing SIRS.

For example, an increase of at least 3.5 (eg. at least 3.6, at least 3.7, at least 3.8, at least 3.9, or at least 4) fold in RBP4 in the sample obtained from the patient relative to the corresponding reference value, indicates that the patient has, or is at risk of developing SIRS. In one embodiment, no increase or an increase of less than 3.5 (eg. less than 3.6, less than 3.7, less than 3.8, less than 3.9, or less than 4) fold in RBP4 in the sample obtained from the patient relative to the corresponding reference value, indicates that the patient does not have, or is not at risk of developing SIRS. In one embodiment, when detecting this biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 2 (eg. at least 2.1, at least 2.2, at least 2.3, at least 2.4, at least 2.5) fold in MPP3 in the sample obtained from the patient relative to the corresponding reference value, indicates that the patient has, or is at risk of developing SIRS. In one embodiment, no increase or an increase of less than 2 (eg. less than 2.1, less than 2.2, less than 2.3, less than 2.4, less than 2.5) fold in MPP3 in the sample obtained from the patient relative to the corresponding reference value, indicates that the patient does not have, or is not at risk of developing SIRS.

As described herein, the present inventors observed that the levels of the one or more SIRS biomarkers were elevated in patients having SIRS as compared to patients having sepsis. Detection of increased levels of these biomarkers in a patient as compared to the levels detected for patients having sepsis can thus be used to diagnose the presence of SIRS.

Thus, in one embodiment, the reference value used in the method of the invention is representative of an individual (or population of individuals) having sepsis (such as abdominal sepsis and/or pulmonary sepsis). The reference value that is representative of an individual having sepsis is as described above for the "method for diagnosing a systemic inflammatory condition in a patient".

In one embodiment, an increase in the one or more biomarker (and/ or one or more additional biomarker) in the sample obtained from the patient relative to the corresponding reference value representative of an individual having sepsis, indicates that the patient has or is at risk of developing SIRS. Likewise, no increase in the one or more biomarker (and/ or one or more additional biomarker) in the sample obtained from the patient relative to the corresponding reference value representative of an individual having sepsis, indicates that the patient does not have SIRS.

In one embodiment, the patient may be diagnosed as having SIRS, or being at risk of developing SIRS, when the one or more biomarker (or the one or more additional biomarker) increases by at least 1 (e.g. at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value of an individual having sepsis.

The method for diagnosis of SIRS as described herein can be used in a decision tree process to investigate the health of a patient having or suspected of having a systemic inflammatory condition. For example, the method for diagnosis of SIRS in a patient can be performed before, after, or in addition to any of the other methods described herein.

In one embodiment, the method for diagnosing SIRS in a patient (as described herein) can be performed subsequent to (or in addition to) the method for diagnosing whether a patient has a systemic inflammatory condition (as described herein). If the patient tests positive for a systemic inflammatory condition (using the method of the invention for diagnosing whether a patient has a systemic inflammatory condition), they may be tested for SIRS using the diagnostic method described herein. Furthermore, the method for diagnosis of SIRS may be performed before, after, or in addition to the method for diagnosis of sepsis in a patient, as described herein.

In one embodiment, the method of the invention for diagnosing SIRS in a patient (as described herein) can be performed subsequent to (or in addition to) the method for distinguishing between sepsis and SIRS in a patient (as described herein). If the patient tests positive for SIRS using the distinguishing method of the invention, they may then be tested for SIRS using the diagnostic method described herein, so as to further confirm the diagnosis of SIRS in the patient.

In one embodiment, the method for diagnosis of SIRS may be performed subsequent to (or in addition to) the method for diagnosing whether a patient has a systemic inflammatory condition (as described herein), and the method for distinguishing between sepsis and SIRS in a patient (as described herein). For example, the patient may be tested first using the method for diagnosing whether a patient has a systemic inflammatory condition (as described herein). If the patient tests positive for a systemic inflammatory condition, they may be tested using the distinguishing method of the invention (as described herein) to determine whether they have sepsis and/or SIRS. If the patient tests positive for SIRS using the distinguishing method of the invention, they may be tested for SIRS using the diagnostic method described herein, so as to further confirm the diagnosis of SIRS in the patient. Furthermore, the method for diagnosis of SIRS may be performed before, after, or in addition to the method for diagnosis of sepsis in a patient, as described herein.

The methods for diagnosis of a systemic inflammatory condition, sepsis, abdominal sepsis, pulmonary sepsis and/or SIRS may be performed simultaneously or sequentially in any order. The above described combination of methods may be performed in parallel to determine the disease status of a patient by simultaneously (or substantially simultaneously) investigating the expression of all the biomarkers in a sample obtained from the patient, and determining whether the patient has or is at risk of having a systemic inflammatory condition, sepsis (such as abdominal or pulmonary sepsis) and/or SIRS.

When performing these different methods in a decision tree process, the sample used in each step of the method may be the same sample obtained from the patient (as described herein). When the method comprises multiple quantification steps, all the steps may be performed at the same time (e.g. in parallel) and/or using the same sample.

In a related aspect, the present invention also provides the use of one or more of: PLA2G7, ARHGEF10L, MYCL, TGFBI, GPR124, IL1RN, NLRP3, RBP4, and MPP3, as a biomarker for SIRS. In one embodiment, the use is of the one or more biomarker in the diagnosis of SIRS in a patient. In one embodiment, the use is of one or more of: PLA2G7, ARHGEF10L, MYCL, TGFBI, and GPR124. In one embodiment, the use is of one or more of: PLA2G7, ARHGEF10L, MYCL, and TGFBI. For example, the use may be of the combination of: PLA2G7, ARHGEF10L, MYCL, and TGFBI.

For example, the use may comprise (i) determining the presence and/or amount of one or more biomarker in a sample obtained from a patient; and (ii) comparing the presence and/or amount of the one or more biomarker determined in said sample in (i) to a corresponding reference value to determine whether the patient has or is at risk of developing SIRS.

All embodiments described above for the method of diagnosing SIRS in a patient apply equally to the 'use' of the invention described herein. This includes all embodiments relating to the terms "SIRS", "patient", "sample", and "the one or more biomarker".

### Diagnosis of sepsis

When investigating gene expression patterns in patients having systemic inflammatory conditions, the inventors identified a subset of biomarkers (see Table 3) that were expressed at different levels in patients having sepsis, as compared to patients having other systemic inflammatory conditions, and healthy individuals. As a result of these findings, the inventors thus observed that ITGB3, ITGA2B, MYL9, LCN2, TREML1, LCN15, CMTM5, PPBP, PF4, KIF2C, MAP1A, SELP, NEXN, NLRC4, CLEC1B, MRAS, PCOLCE2, TMEM37, SLC39A8, KIF2C, CIQC, CIQB, CIQA, TNF, IFI44, IFIT1, RPGRIP1, EPSTI1, DISC1, CXCR1, and HCAR2, can be used as biomarkers for diagnosis of sepsis.

In particular, the present inventors have identified that ITGB3, ITGA2B, MYL9, LCN2, TREML1, LCN15, CMTM5, PPBP, PF4, KIF2C, MAP1A, SELP, NEXN, NLRC4, and CLEC1B, are elevated in all types of sepsis tested, and thus can be used as biomarkers for sepsis including abdominal sepsis and pulmonary sepsis.

The inventors also observed that the levels of SLC39A8, CIQC, CIQA, MRAS, TMEM37, CIQB, PCOLCE2, KIF2C, and TNF are elevated in patients having abdominal sepsis, compared to patients having pulmonary sepsis or SIRS, and healthy individuals. The inventors also observed that the levels of IFI44, IFIT1, and RPGRIP1 were decreased in patients having abdominal sepsis, compared to patients having pulmonary sepsis or SIRS, and healthy individuals. SLC39A8, CIQC, CIQA, MRAS, TMEM37, CIQB, PCOLCE2, KIF2C, TNF, IFI44, IFIT1, and RPGRIP1 can thus be used as biomarkers for abdominal sepsis. Likewise, the inventors observed that the levels of HCAR2, CXCR1, DISC1, EPSTI1, and IFI44 are elevated in patients having pulmonary sepsis, compared to patients having abdominal sepsis and/or SIRS, and healthy individuals. HCAR2, CXCR1, DISC1, EPSTI1, and IFI44 can thus be used as biomarkers for pulmonary sepsis.

The present invention therefore provides a method for diagnosing sepsis in a patient, comprising:
(i) determining the presence and/or amount of one or more biomarker in a sample obtained from a patient, wherein the one or more biomarker is selected from the group consisting of: ITGB3, ITGA2B, MYL9, LCN2, TREML1, LCN15, CMTM5, PPBP, PF4, KIF2C, MAP1A, SELP, NEXN, NLRC4, CLEC1B, SLC39A8, CIQC, CIQA, MRAS, TMEM37, CIQB, PCOLCE2, KIF2C, TNF, IFI44, IFIT1, RPGRIP1, HCAR2, CXCR1, DISC1, and EPSTI1;
(ii) comparing the presence and/or amount of the one or more biomarker determined in said sample in (i) to a corresponding reference value; and thereby determining whether the patient has sepsis.

All embodiments described above for the "method for diagnosing a systemic inflammatory condition in a patient" apply equally to the "method for diagnosing sepsis in a patient". This includes all embodiments relating to the "sample", "patient", "biomarker", and "reference value", and all embodiments relating to the step for "determining the presence and/or amount of one or more biomarker in a sample" and for the "comparison" step for making a conclusion about the disease status of the patient.

As used herein, the phrase "diagnosis of sepsis in a patient" means determining whether the patient has or is risk of developing sepsis. The systemic inflammatory condition "sepsis" diagnosed using the method of the invention is as described above for the "method for diagnosing a systemic inflammatory condition in a patient". In one embodiment, the method is for diagnosing one or more of: abdominal sepsis and pulmonary sepsis. In one embodiment, the method is for diagnosing abdominal sepsis. In one embodiment, the method is for diagnosing pulmonary sepsis.

The "patient" for which diagnosis is performed is as described above for the "method for diagnosing a systemic inflammatory condition in a patient". In one embodiment, the patient is suspected of having or being at risk of developing a systemic inflammatory condition. In one embodiment, the patient has been diagnosed as having or being at risk of developing a systemic inflammatory condition. In one embodiment, the patient has been diagnosed as having a systemic inflammatory condition using the method described herein. In one embodiment, the patient is suspected of having or being at risk of developing sepsis. In one embodiment, the patient has been diagnosed as having or being at risk of developing sepsis using the method described herein for distinguishing between sepsis and SIRS in a patient.

The "sample" obtained from the patient is as described above for the "method for diagnosing a systemic inflammatory condition in a patient", including all embodiments relating to the time point at which the sample is obtained.

The optimum time point at which a sample is obtained from a patient may depend on the biomarker being tested. For example, when testing for any one or more of the biomarkers MAP1A, SELP, NEXN, ITGA2B, MYL9, CMTM5, PPBP, TREML1, PF4, CLEC1B or ITGB3, the sample may be obtained up to 1 hour, 2 hours, 4 hours, 6 hours, 8 hours, 12 hours, 24 hours, 36 hours, 48 hours, 72 hours, or 96 hours after the patient presents with one or more clinical symptoms of a systemic inflammatory condition, or is admitted to a medical care facility. Preferably, the sample is obtained up to 24 hours after the patient presents with one or more clinical symptoms of a systemic inflammatory condition, or is admitted to a medical care facility. Preferably, the sample is obtained up to 48 hours after the patient presents with one or more clinical symptoms of a systemic inflammatory condition, or is admitted to a medical care facility.

The "one or more biomarker" of the invention is as described above for the "method for diagnosing a systemic inflammatory condition in a patient".

As illustrated in Example 1, the present inventors observed that ITGB3, ITGA2B, MYL9, LCN2, TREML1, LCN15, CMTM5, PPBP, PF4, KIF2C, MAP1A, SELP, NEXN, NLRC4, CLEC1B, SLC39A8, CIQC, CIQA, MRAS, TMEM37, CIQB, PCOLCE2, KIF2C, TNF, IFI44, IFIT1, RPGRIP1, HCAR2, CXCR1, DISC1, and EPSTI1, are biomarkers of sepsis, and thus can be used in the diagnosis of sepsis.

The reference to the biomarker SLC39A8 throughout the entire description, includes the transcript variant 1 of SLC39A8 (as encoded by SEQ ID NO: 70) and the transcript variant 3 of SLC39A8 (as encoded by SEQ ID NO: 71). In one embodiment, the reference to the biomarker SLC39A8 is a reference to the transcript variant 1 of SLC39A8 (as encoded by SEQ ID NO: 70). In one embodiment, the reference to the biomarker SLC39A8 is a reference to the transcript variant 3 of SLC39A8 (as encoded by SEQ ID NO: 71).

In one embodiment, the one or more biomarker may be selected from the group consisting of ITGB3, ITGA2B, MYL9, LCN2, TREML1, LCN15, CMTM5, PPBP, PF4, KIF2C, MAP1A, SELP, NEXN, NLRC4, CLEC1B, SLC39A8, CIQC CIQA,, MRAS, TMEM37, CIQB, PCOLCE2, KIF2C, TNF, IFI44, IFIT1, RPGRIP1, HCAR2, CXCR1, DISC1, and EPSTI1.

In one embodiment, the one or more biomarker may be selected from the group consisting of ITGB3, ITGA2B, MYL9, LCN2, TREML1, LCN15, CMTM5, PPBP, PF4, MAP1A, SELP, NLRC4, IFI44, HCAR2, CXCR1, DISC1, and EPSTI1. When detecting one or more biomarker selected from this sub-group, the systemic inflammatory condition diagnosed using the method may be pulmonary sepsis.

The present inventors observed that a sub-set of the biomarkers for sepsis specifically increased in abundance in all types of sepsis tested (including abdominal and pulmonary sepsis) as compared to healthy individuals and patients having SIRS. These markers are therefore useful for diagnosis of sepsis in a patient. In one embodiment, the one or more biomarker may be selected from the group consisting of: ITGB3, ITGA2B, MYL9, LCN2, TREML1, LCN15, CMTM5, PPBP, PF4, KIF2C, MAP1A, SELP, NEXN, NLRC4, and CLEC1B.

The present inventors also observed that a sub-set of the biomarkers for sepsis (ITGB3, ITGA2B, MYL9, LCN2,TREML1, LCN15, CMTM5, PPBP, and PF4) increase in abundance in patients having sepsis as compared to healthy individuals, and show no increase (or a decrease) in patients having SIRS as compared to healthy individuals (eg. in patients tested at days 1 and 2 post-hospitalisation). These markers therefore provide highly specific biomarkers for diagnosing sepsis. Thus, in one embodiment, the one or more biomarker may therefore be selected from the group consisting of: ITGB3, ITGA2B, MYL9, LCN2, TREML1, LCN15, CMTM5, PPBP, and PF4.

Furthermore, of this sub-set of sepsis biomarkers, the inventors also observed that the specific biomarkers ITGB3, ITGA2B, MYL9, LCN2, and TREML1 were particularly effective at diagnosing sepsis when tested using ROC analysis, as described in Example 2. Specifically, AUC values of 0.86, 0.83, 0.82, 0.82 and 0.8 were observed for ITGB3, ITGA2B, MYL9, LCN2, and TREML1. Thus, in one embodiment, the one or more biomarker is preferably selected from the group consisting of: ITGB3, ITGA2B, MYL9, LCN2, and TREML1.

Each of the biomarkers of sepsis may be used alone, or in combination with any of the sepsis biomarkers in the method of the invention. For example, any combination of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, up to and including all of the sepsis biomarkers may be used to diagnose sepsis in a patient according to the method of the invention.

In one embodiment, the one or more biomarker is LCN2. In one embodiment, the one or more biomarker is ITGA2B. In one embodiment, the one or more biomarker is MYL9. In one embodiment, the one or more biomarker is ITGB3. In one embodiment, the one or more biomarker is TREML1. In one embodiment, the one or more biomarker is LCN15. In one embodiment, the one or more biomarker is CMTM5. In one embodiment, the one or more biomarker is PPBP. In one embodiment, the one or more biomarker is PF4. In one embodiment, the one or more biomarker is KIF2C. In one embodiment, the one or more biomarker is MAP1A. In one embodiment, the one or more biomarker is SELP. In one embodiment, the one or more biomarker is NEXN. In one embodiment, the one or more biomarker is NLRC4. In one embodiment, the one or more biomarker is CLEC1B. In one embodiment, the one or more biomarker is MRAS. In one embodiment, the one or more biomarker is CIQC. In one embodiment, the one or more biomarker is CIQB. In one embodiment, the one or more biomarker is PCOLCE2. In one embodiment, the one or more biomarker is CIQA. In one embodiment, the one or more biomarker is TMEM37. In one embodiment, the one or more biomarker is SLC39A8. In one embodiment, the one or more biomarker is TNF. In one embodiment, the one or more biomarker is IFI44. In one embodiment, the one or more biomarker is IFIT1. In one embodiment, the one or more biomarker is RPGRIP1. In one embodiment, the one or more biomarker is EPSTI1. In one embodiment, the one or more biomarker is DISC1. In one embodiment, the one or more biomarker is CXCR1. In one embodiment, the one or more biomarker is HCAR2.

In one embodiment, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, or all 29) of the biomarkers selected from the group consisting of: ITGB3, ITGA2B, MYL9, LCN2, TREML1, LCN15, CMTM5, PPBP, PF4, KIF2C, MAP1A, SELP, NEXN, NLRC4, CLEC1B, SLC39A8, CIQC, CIQA, MRAS, TMEM37, CIQB, PCOLCE2, KIF2C, TNF, IFI44, IFIT1, RPGRIP1, HCAR2, CXCR1, DISC1, and EPSTI1, may be used to diagnose sepsis in a patient.

In one embodiment, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, or all 15) of the biomarkers selected from the group consisting of: ITGB3, ITGA2B, MYL9, LCN2, TREML1, LCN15, CMTM5, PPBP, PF4, KIF2C, MAP1A, SELP, NEXN, NLRC4, and CLEC1B, may be used to diagnose sepsis in a patient.

In one embodiment, any combination of 1 or more (eg. 2 or more, 3 or more, 4 or more, 5 or more, 6 or more, 7 or more, 8 or more, or all 9) of the biomarkers selected from the group consisting of: ITGB3, ITGA2B, MYL9, LCN2, TREML1, LCN15, CMTM5, PPBP, and PF4, may be used to diagnose sepsis in a patient.

In one embodiment, any combination of 1 or more (eg. 2 or more, 3 or more, 4 or more, or all 5) of the biomarkers selected from the group consisting of: ITGB3, ITGA2B, MYL9, LCN2, and TREML1, may be used to diagnose sepsis in a patient. For example, the method may be performed using 2 or more of the biomarkers selected from the group consisting of: ITGB3, ITGA2B, MYL9, LCN2, and TREML1. For example, the method may be performed using 3 or more of the biomarkers selected from the group consisting of: ITGB3, ITGA2B, MYL9, LCN2, and TREML1. For example, the method may be performed using 4 or more of the biomarkers selected from the group consisting of: ITGB3, ITGA2B, MYL9, LCN2, and TREML1. For example, the method may be performed using all 5 biomarkers: ITGB3, ITGA2B, MYL9, LCN2, and TREML1. This combination of sepsis biomarkers was shown to be particularly effective in diagnosing sepsis when tested by ROC analysis described in Example 2.

For example, the following combinations of sepsis biomarkers may be used in the method of the invention to diagnose sepsis: (i) LCN15 and ITGA2B; (ii) LCN15 and MYL9; (iii) LCN15 and CMTM5; (iv) LCN15 and PPBP; (v) LCN15 and TREML1; (vi) LCN15 and PF4; (vii) LCN15 and LCN2; (viii) LCN15 and ITGB3; (ix) ITGA2B and MYL9; (x) ITGA2B and CMTM5; (xi) ITGA2B and PPBP; (xii) ITGA2B and TREML1; (xiii) ITGA2B and PF4; (xiv) ITGA2B and LCN2; (xv) ITGA2B and ITGB3; (xvi) MYL9 and CMTM5; (xvii) MYL9 and PPBP; (xviii) MYL9 and TREML1; (xix) MYL9 and PF4; (xx) MYL9 and LCN2; (xxi) MYL9 and ITGB3; (xxii) CMTM5 and PPBP; (xxiii) CMTM5 and TREML1; (xxiv) CMTM5 and PF4; (xxv) CMTM5 and LCN2; (xxvi) CMTM5 and ITGB3; (xxvii) PPBP and TREML1; (xxviii) PPBP and PF4; (xxix) PPBP and LCN2; (xxx) PPBP and ITGB3; (xxxi) TREML1 and PF4; (xxxii) TREMLI and LCN2; (xxxiii) TREML1 and ITGB3; (xxxiv) PF4 and LCN2; (xxxv) PF4 and ITGB3; and (xxxvi) LCN2 and ITGB3.

One or more additional biomarker for sepsis may also be used in the diagnosis of sepsis according to the method of the invention. Any combination of the one or more additional biomarker may be used in combination with the one or more biomarker of the invention. For example at least 1, at least 2, at least 3, at least 4, at least 5, at least 6, at least 7, at least 8, at least 9, at least 10, at least 11, at least 12, at least 13, or all 14 additional biomakers for sepsis may be used in combination with the one or more biomarker of the invention (as described herein). Typically, the one or more additional biomarker is selected from the group consisting of: ITGB3, ITGA2B, MYL9, LCN2, TREML1, LCN15, CMTM5, PPBP, PF4, KIF2C, MAP1A, SELP, NEXN, NLRC4, and CLEC1B. For example, the one or more additional biomarker may be selected from the group consisting of: ITGB3, ITGA2B, MYL9, LCN2, TREML1, LCN15, CMTM5, PPBP, and PF4. For example, the one or more additional biomarker may be selected from the group consisting of: ITGB3, ITGA2B, MYL9, LCN2, and TREML1.

In one embodiment, the one or more biomarker is LCN2, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, TREML1, LCN15, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, TREML1, LCN15, CMTM5, PPBP, and PF4. In one embodiment, the one or more additional biomarker is selected from at least 1 (eg. at least 2, at least 3, up to and including all) of the biomarkers: ITGB3, ITGA2B, MYL9, and TREML1.

In one embodiment, the one or more biomarker is ITGA2B, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, MYL9, LCN2, TREML1, LCN15, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, MYL9, LCN2, TREML1, LCN15, MYL9, ITGB3, CMTM5, PPBP, and PF4. In one embodiment, the one or more additional biomarker is selected from at least 1 (eg. at least 2, at least 3, up to and including all) of the biomarkers: ITGB3, MYL9, LCN2, and TREML1.

In one embodiment, the one or more biomarker is MYL9, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, LCN2, TREML1, LCN15, ITGA2B, ITGB3, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, LCN2, TREML1, LCN15, ITGA2B, ITGB3, CMTM5, PPBP, and PF4. In one embodiment, the one or more additional biomarker is selected from at least 1 (eg. at least 2, at least 3, up to and including all) of the biomarkers: ITGB3, ITGA2B, LCN2, and TREML1.

In one embodiment, the one or more biomarker is ITGB3, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGA2B, MYL9, LCN2, TREML1, LCN15, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGA2B, MYL9, LCN2, TREML1, LCN15, ITGA2B, MYL9, CMTM5, PPBP, and PF4. In one embodiment, the one or more additional biomarker is selected from at least 1 (eg. at least 2, at least 3, up to and including all) of the biomarkers: ITGA2B, MYL9, LCN2, and TREML1.

In one embodiment, the one or more biomarker is TREML1, and the one or more additional biomarker is selected from at least 1 (eg. at least 2, at least 3, at least 4, at least 5, at least 6, at least 7, at least 8, at least 9, at least 10, at least 11, up to and including all) of the biomarkers: ITGB3, ITGA2B, MYL9, LCN2, LCN15, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, LCN2, LCN15, CMTM5, PPBP, and PF4. In one embodiment, the one or more additional biomarker is selected from at least 1 (eg. at least 2, at least 3, up to and including all) of the biomarkers: ITGB3, ITGA2B, MYL9, and LCN2.

In one embodiment, the one or more biomarker is LCN15, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2 TREML1,, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, LCN2, TREML1, CMTM5, PPBP, and PF4.

In one embodiment, the one or more biomarker is CMTM5, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, LCN2, TREML1, LCN15, PPBP, and PF4.

In one embodiment, the one or more biomarker is PPBP, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, ITGA2B, MYL9, ITGB3, CMTM5, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, LCN2, TREML1, LCN15, ITGA2B, MYL9, ITGB3, CMTM5, and PF4.

In one embodiment, the one or more biomarker is PF4, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, ITGA2B, MYL9, ITGB3, CMTM5, PPBP, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, LCN2, TREML1, LCN15, ITGA2B, MYL9, ITGB3, CMTM5, and PPBP.

In one embodiment, the one or more biomarker is KIF2C, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, LCN15, TREML1, ITGA2B, MYL9, ITGB3, CMTM5, PPBP, PF4, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, at least 8, up to and including all) of the biomarkers: ITGB3, ITGA2B, MYL9, LCN2, TREML1, LCN15, ITGA2B, MYL9, ITGB3, CMTM5, PPBP, and PF4.

In one embodiment, the one or more biomarker is MAP1A, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, ITGA2B, MYL9, ITGB3, CMTM5, PPBP, PF4, KIF2C, SELP, NEXN, NLRC4, and CLEC1B. In one embodiment, the one or more additional biomarker is selected from at least 1 (eg. at least 2, at least 3, at least 4, at least 5, at least 6, at least 7, at least 8, up to and including all) of the biomarkers: ITGB3, ITGA2B, MYL9, LCN2, TREML1, LCN15, ITGA2B, MYL9, ITGB3, CMTM5, PPBP, and PF4.

In one embodiment, the one or more biomarker is SELP, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, ITGA2B, MYL9, ITGB3, CMTM5, PPBP, PF4, KIF2C, MAP1A, NEXN, NLRC4, and CLEC1B. In one embodiment, the one or more additional biomarker is selected from at least 1 (eg. at least 2, at least 3, at least 4, at least 5, at least 6, at least 7, at least 8, up to and including all) of the biomarkers: ITGB3, ITGA2B, MYL9, LCN2, LCN15, TREML1, ITGA2B, MYL9, ITGB3, CMTM5, PPBP, and PF4.

In one embodiment, the one or more biomarker is NEXN, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, ITGA2B, MYL9, ITGB3, CMTM5, PPBP, PF4, KIF2C, MAP1A, SELP, NLRC4, and CLEC1B. In one embodiment, the one or more additional biomarker is selected from at least 1 (eg. at least 2, at least 3, at least 4, at least 5, at least 6, at least 7, at least 8, up to and including all) of the biomarkers: ITGB3, ITGA2B, MYL9, LCN2, TREML1, LCN15, ITGA2B, MYL9, ITGB3, CMTM5, PPBP, and PF4.

In one embodiment, the one or more biomarker is NLRC4, and the one or more additional biomarker is selected from at least 1 (eg. at least 2, at least 3, at least 4, at least 5, at least 6, at least 7, at least 8, at least 9, at least 10, at least 11, up to and including all) of the biomarkers: ITGB3, ITGA2B, MYL9, LCN2, TREML1, LCN15, ITGA2B, MYL9, ITGB3, CMTM5, PPBP, PF4, KIF2C, MAP1A, SELP, NEXN, and CLEC1B. In one embodiment, the one or more additional biomarker is selected from at least 1 (eg. at least 2, at least 3, at least 4, at least 5, at least 6, at least 7, at least 8, up to and including all) of the biomarkers: ITGB3, ITGA2B, MYL9, LCN2, TREML1, LCN15, ITGA2B, MYL9, ITGB3, CMTM5, PPBP, and PF4.

In one embodiment, the one or more biomarker is CLEC1B, and the one or more additional biomarker is selected from at least 1 (eg. at least 2, at least 3, at least 4, at least 5, at least 6, at least 7, at least 8, at least 9, at least 10, at least 11, at least 12, at least 13, up to and including all) of the biomarkers: ITGB3, ITGA2B, MYL9, LCN2, TREML1, LCN15, ITGA2B, MYL9, ITGB3, CMTM5, PPBP, PF4, KIF2C, MAP1A, SELP, NEXN, and NLRC4. In one embodiment, the one or more additional biomarker is selected from at least 1 (eg. at least 2, at least 3, at least 4, at least 5, at least 6, at least 7, at least 8, up to and including all) of the biomarkers: ITGB3, ITGA2B, MYL9, LCN2, TREML1, LCN15, ITGA2B, MYL9, ITGB3, CMTM5, PPBP, and PF4.

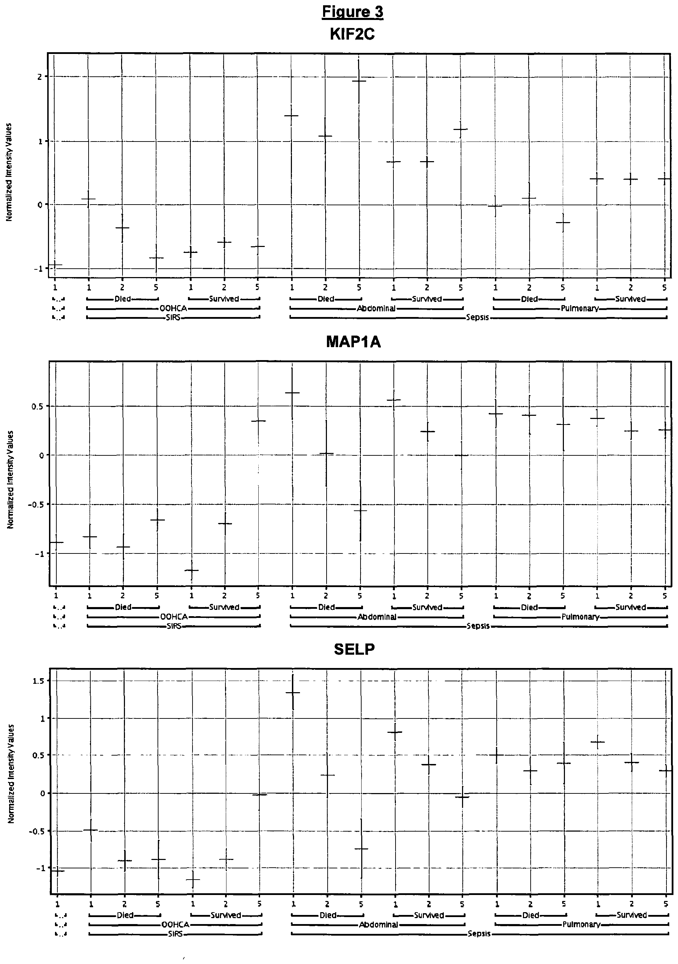

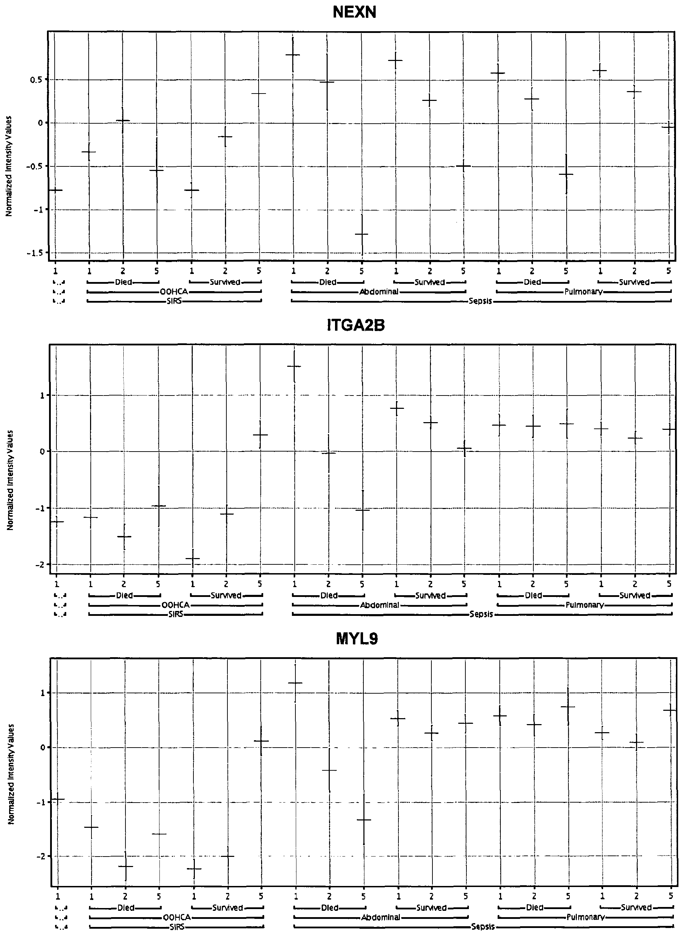

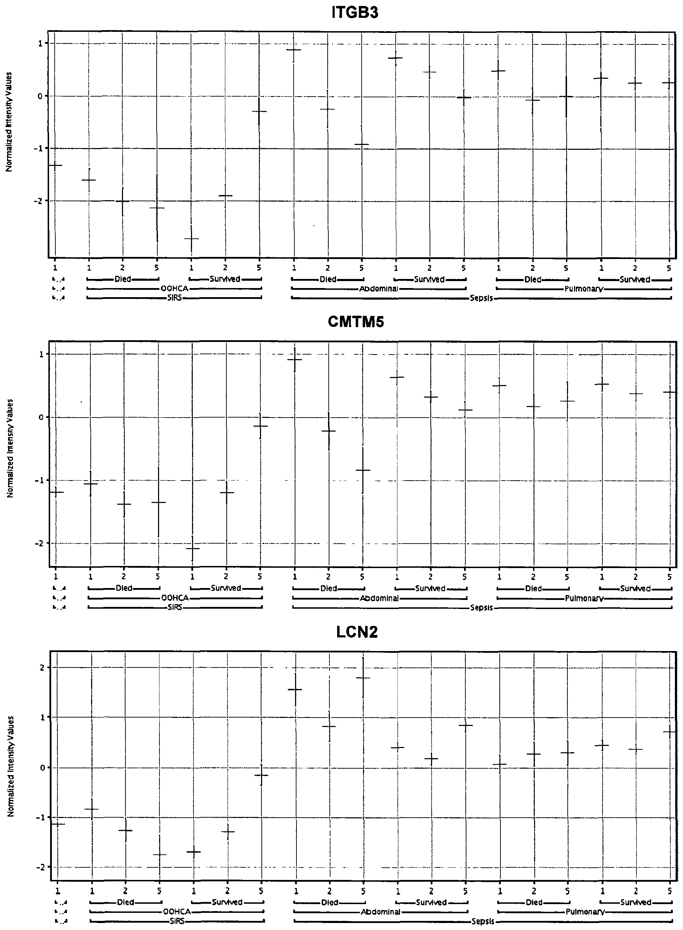

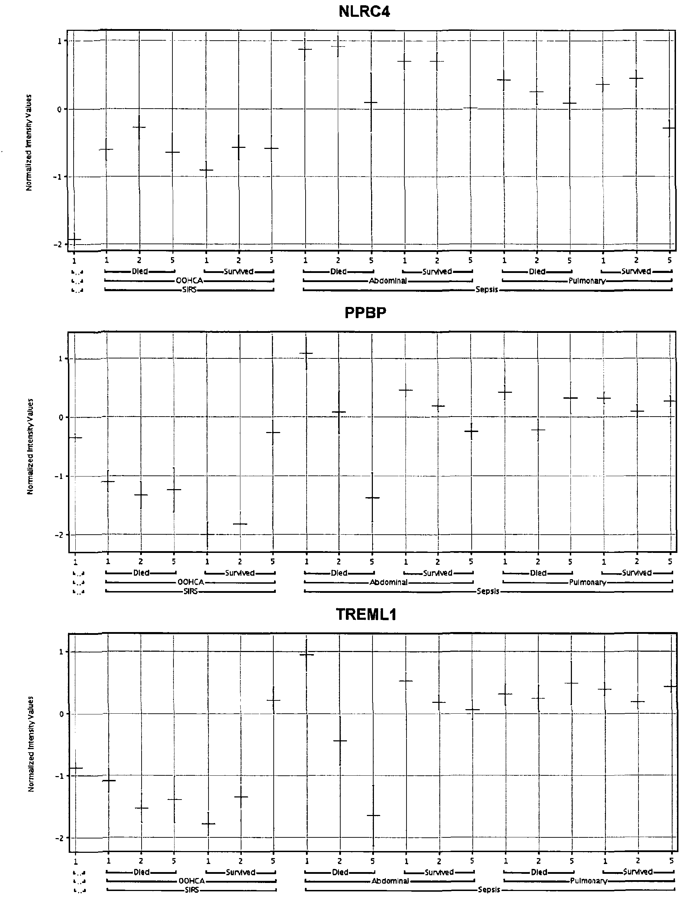

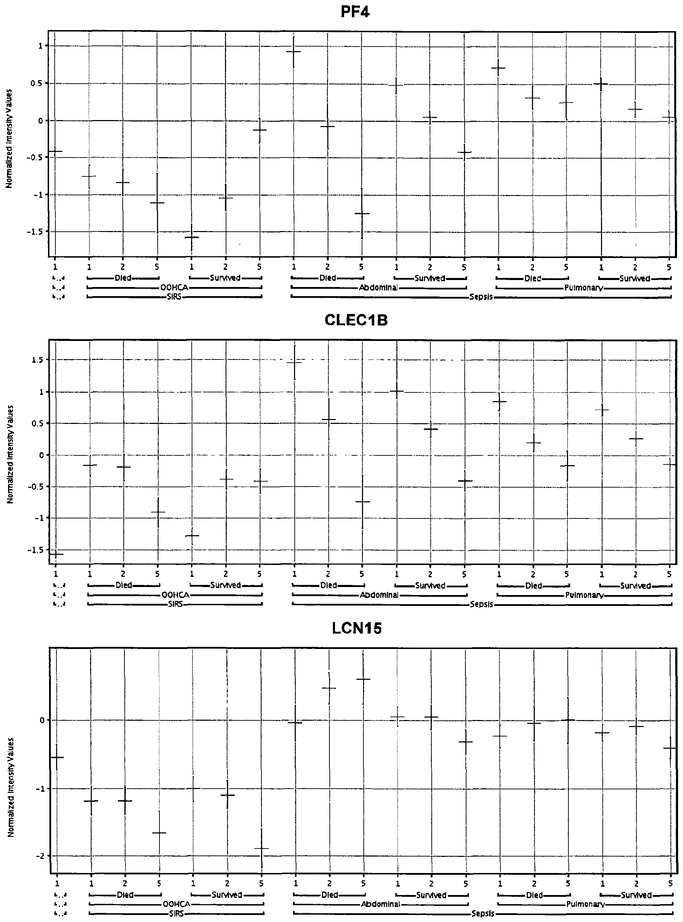

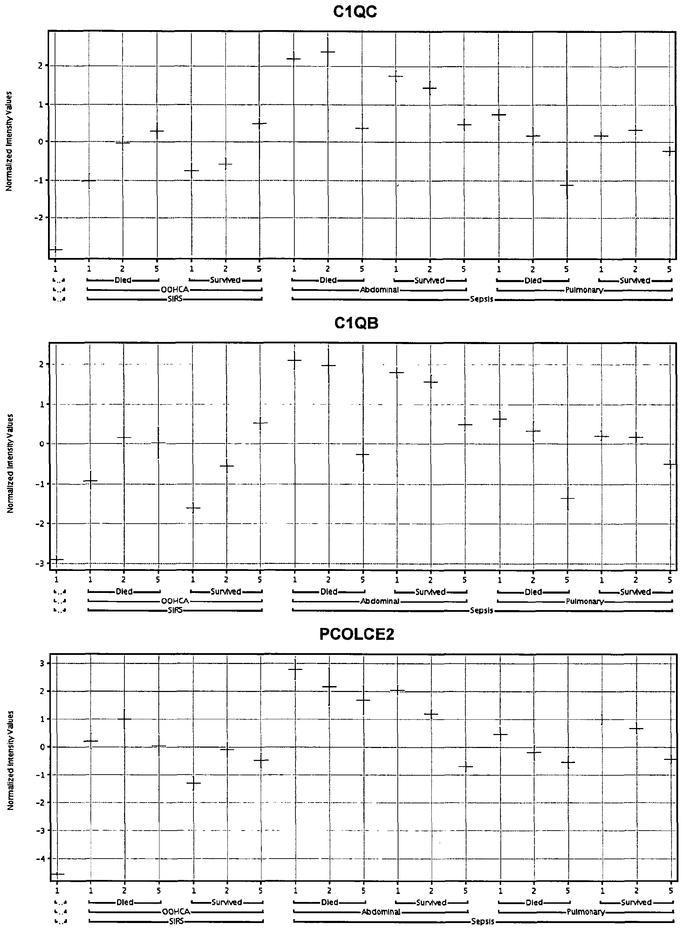

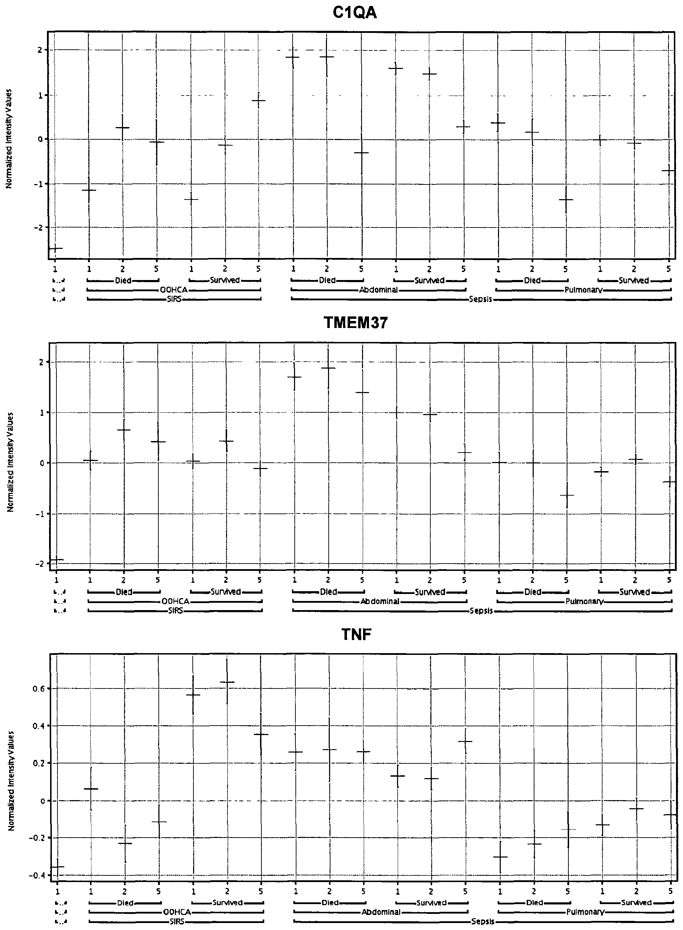

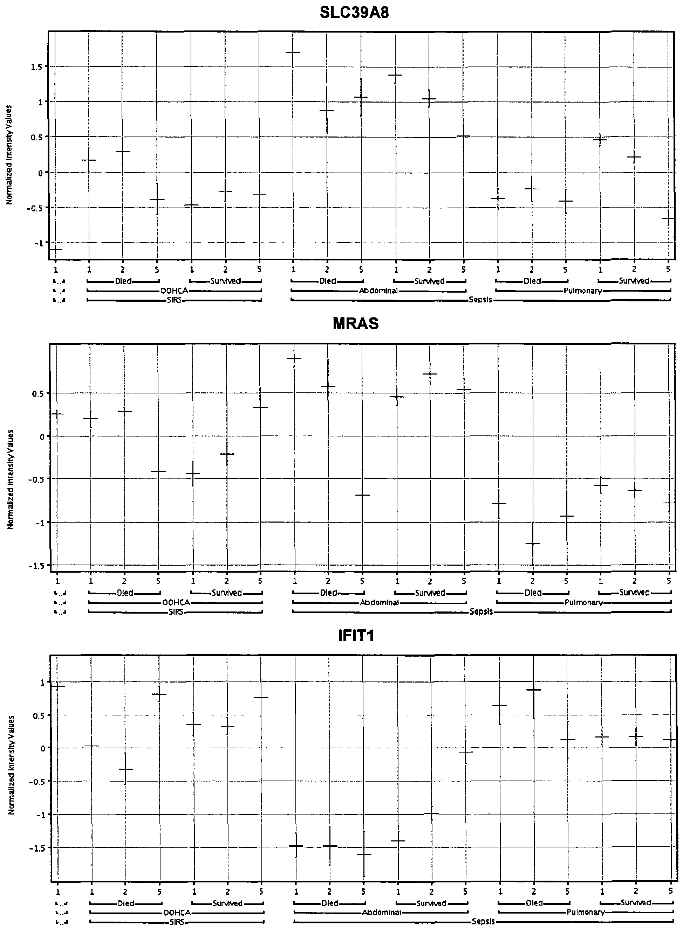

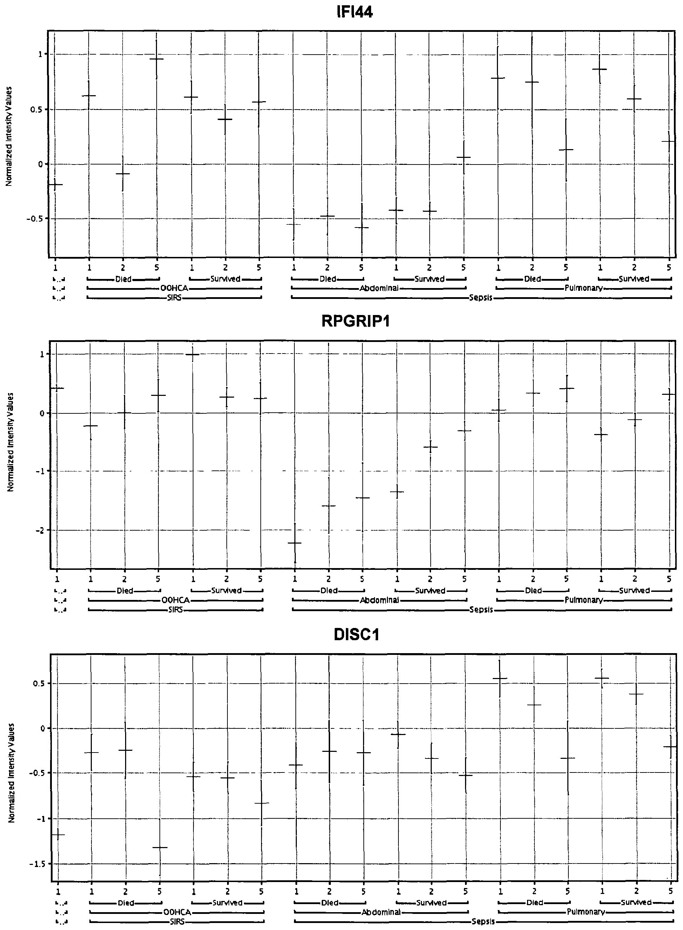

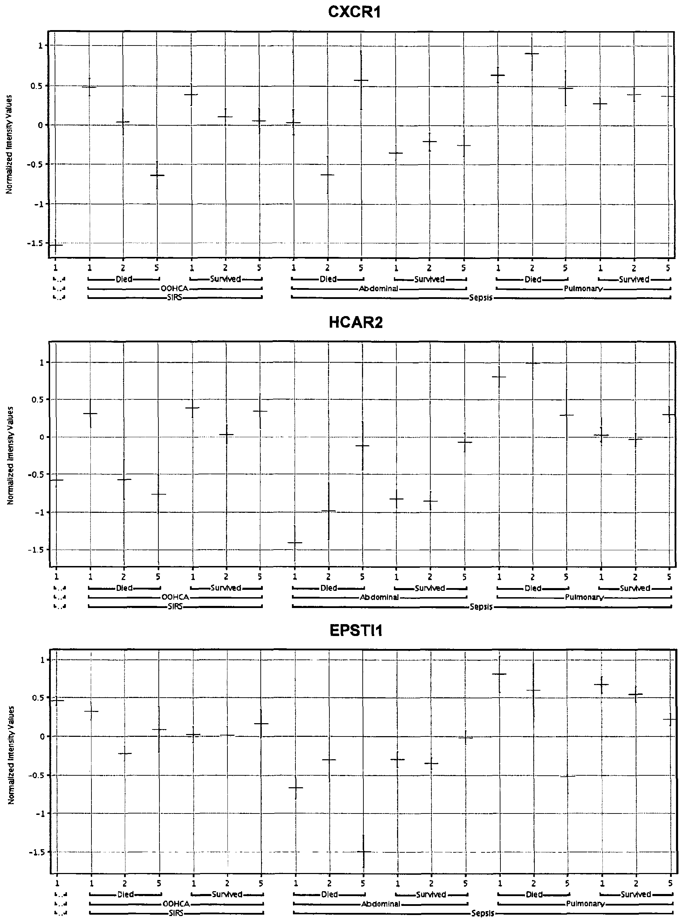

As illustrated in Figure 3, the present inventors observed that the "sepsis" biomarkers described herein increase in abundance in patients having sepsis as compared to patients having other systemic inflammatory conditions (such as SIRS), and as compared to healthy individuals. These differences in marker abundance can be used to diagnose whether an individual has or is at risk of developing sepsis.

For example, by comparing the presence and/or amount of markers quantified in a sample obtained from a patient to the presence and/or amount of markers quantified for a reference value (such as a reference value that is representative of a healthy individual (or a population of healthy individuals), or a reference value that is representative of an individual (or a population of individuals) having sepsis eg. abdominal and/or pulmonary sepsis, or a reference value that is representative of an individual (or a population of individuals) having SIRS, it is possible to diagnose the presence (or absence) of sepsis in a patient. The method permits classification of the individual as belonging to or not belonging to the reference population (ie. by determining whether the amounts of marker quantified in the individual are statistically similar to the reference population or statistically deviate from the reference population). Hence, classification of the individual's marker profile (ie. the overall pattern of change observed for the markers quantified) as corresponding to the profile derived from a particular reference population is predictive that the individual falls (or does not fall) within the reference population.

In one embodiment, an individual may be diagnosed as having or being at risk of having sepsis, when the amount of the one or more biomarker is statistically similar to the amount determined for the corresponding reference value representative of an individual (or a population of individuals) having sepsis. In one embodiment, an individual may be diagnosed as not having or not being at risk of having sepsis when the amount of the one or more biomarker is statistically similar to the amount determined for the corresponding reference value representative of a healthy individual (or a population of healthy individuals). In one embodiment, an individual may be diagnosed as not having or not being at risk of having sepsis when the amount of the one or more biomarker is statistically similar to the amount determined for the corresponding reference value representative of an individual having SIRS (or a population of individuals having SIRS).

In one embodiment, an individual may be diagnosed as not having or not being at risk of having sepsis when the amount of the one or more biomarker statistically deviates from the amount determined for the corresponding reference values representative of an individual (or a population of individuals) having sepsis. In one embodiment, an individual may be diagnosed as having or being at risk of having sepsis when the amount of the one or more biomarker statistically deviates from the amount determined for the corresponding reference value representative of a healthy individual (or a population of healthy individuals). In one embodiment, an individual may be diagnosed as having or being at risk of having sepsis when the amount of the one or more biomarker statistically deviates from the amount determined for the corresponding reference values representative of an individual having SIRS (or a population of individuals having SIRS).

All embodiments described above for the classification of a patient as having or being at risk of having a systemic inflammatory condition (or as not having or not being at risk of having a systemic inflammatory condition) apply equally to the method for diagnosing whether a patient has or is at risk of having sepsis. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may be as defined above for the method of diagnosing a systemic inflammatory condition in a patient. In one embodiment, the reference value is representative of a healthy individual (or a population of healthy individuals). In one embodiment, the reference value is representative of an individual having SIRS (or a population of individuals having SIRS). In one embodiment, the reference value is representative of an individual having sepsis (or a population of individuals having sepsis). As described above for the "method for diagnosing a systemic inflammatory condition in a patient", the method of the invention may involve the use of multiple separate reference values. For example, the reference value may include one of more (eg. two or more, or all 3) of the reference values selected from: a reference value that is representative of a healthy individual (or a population of healthy individuals); a reference value that is representative of an individual having SIRS (or a population of individuals having SIRS); and a reference value that is representative of an individual having sepsis (or a population of individuals having sepsis). The reference value that is representative of an individual having sepsis (or a population of individuals having sepsis) may be representative of an individual (or a population of individuals) having abdominal sepsis and/or pulmonary sepsis.

The present inventors observed that the sepsis biomarkers described herein each increase in abundance in samples obtained from patients having sepsis, as compared to healthy individuals. Detection of increased levels of these biomarkers in a patient as compared to the levels detected for healthy individuals can thus be used to diagnose the presence of sepsis. In one embodiment, when the reference value is representative of a healthy individual (or population of healthy individuals), an increase in the one or more biomarker for sepsis in the sample obtained from the patient relative to the corresponding reference value indicates that the patient has sepsis, or is at risk of developing sepsis. Likewise, no increase in the one or more biomarker for sepsis in the sample obtained from the patient relative to the corresponding reference value indicates that the patient does not have sepsis.

For some of the sepsis biomarkers identified by the present inventors, increased levels of these markers were also observed in patients having SIRS as compared to healthy individuals, although much bigger increases were observed for these biomarkers in the patients having sepsis. The accuracy of sepsis diagnosis can thus be improved by looking for a "minimum" fold change or % change in the levels of the one or more biomarkers as compared to the corresponding reference value that is representative of a healthy individual. The fold change or % change may be as defined above for the method for diagnosis of a systemic inflammatory condition.

In one embodiment, the patient is diagnosed as having sepsis, or being at risk of developing sepsis, when the one or more biomarker for sepsis (or the one or more additional biomarker) increases by at least 0.1 (e.g. at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, or at least 10, at least 15 fold, at least 20 fold, at least 30 fold, at least 40 fold, at least 50) fold in the sample obtained from the patient relative to the corresponding reference value representative of a healthy individual.

For example, an increase of at least 1 (eg. at least 1.1, at least 1.2, at least 1.3, at least 1.4, or at least 1.5) fold in LCN15 in the sample obtained from the patient relative to the corresponding reference value, indicates that the patient has, or is at risk of developing sepsis. In one embodiment, no increase or an increase of less than 1 (eg. less than 1.1, less than 1.2, less than 1.3, less than 1.4, less than 1.5) fold in LCN15 in the sample obtained from the patient relative to the corresponding reference value, indicates that the patient does not have, or is not at risk of developing sepsis.

For example, an increase of at least 2 (eg. at least 2.1, at least 2.2, at least 2.3, at least 2.4, at least 2.5) fold in LCN2 in the sample obtained from the patient relative to the corresponding reference value, indicates that the patient has, or is at risk of developing sepsis. In one embodiment, no increase or an increase of less than 2.1 (eg. less than 2.2, less than 2.3) fold in LCN2 in the sample obtained from the patient relative to the corresponding reference value, indicates that the patient does not have, or is not at risk of developing sepsis.

For example, an increase of at least 1.5 (eg. at least 1.6, at least 1.7, at least 1.8, at least 1.9, at least 2, at least 2.5, at least 3, or at least 3.5) fold in ITGA2B in the sample obtained from the patient relative to the corresponding reference value, indicates that the patient has, or is at risk of developing sepsis. In one embodiment, no increase or an increase of less than 1.1 (eg. less than 1.2, less than 1.3, less than 1.4, less than 1.5, less than 1.6, less than 1.7, less than 1.8, less than 1.9, less than 2, less than 2.5, or less than 3) fold in ITGA2B in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1 (eg. at least 1.1, at least 1.2, at least 1.3, at least 1.4, at least 1.5, at least 1.6, at least 1.7, at least 1.8, at least 1.9, at least 2, at least 2.1, at least 2.2, at least 2.3, at least 2.4, or at least 2.5) fold in MYL9 in the sample obtained from the patient relative to the corresponding reference value, indicates that the patient has, or is at risk of developing sepsis. In one embodiment, no increase or an increase of less than 1 (eg. less than 1.1, less than 1.2, less than 1.3, less than 1.4, less than 1.5, less than 2) fold in MYL9 in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 2 (eg. at least 2.1, at least 2.2, at least 2.3, at least 2.4, at least 2.5) fold in ITGB3 in the sample obtained from the patient relative to the corresponding reference value, indicates that the patient has, or is at risk of developing sepsis. In one embodiment, no increase or an increase of less than 2.1 (eg. less than 2, less than 1.9, less than 1.8, less than 1.7, less than 1.6, less than 1.5, less than 1.4, less than 1.3, less than 1.2) fold in ITGB3 in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.1 (eg. at least 1.2 at least 1.3, at least 1.4, at least 1.5, at least 2, at least 2.1, at least 2.2, at least 2.3, at least 2.4, or at least 2.5) fold in CMTM5 in the sample obtained from the patient relative to the corresponding reference value, indicates that the patient has, or is at risk of developing sepsis. In one embodiment, no increase or an increase of less than 1.1 (eg. less than 1.2, less than 1.3, less than 1.4, less than 1.5) fold in CMTM5 in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1 (eg. at least 1.1, at least 1.2, at least 1.3, at least 1.4, at least 1.5, at least 2 or at least 2.5) fold in PPBP in the sample obtained from the patient relative to the corresponding reference value, indicates that the patient has, or is at risk of developing sepsis. In one embodiment, no increase or an increase of less than 1 (eg. less than 1.1, less than 1.2, less than 1.3) fold in PPBP in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1 (eg. at least 1.1, at least 1.2, at least 1.3, at least 1.4, at least 1.5, at least 2, at least 2.1, at least 2.2, at least 2.3, at least 2.4, or at least 2.5) fold in TREML1 in the sample obtained from the patient relative to the corresponding reference value, indicates that the patient has, or is at risk of developing sepsis. In one embodiment, no increase or an increase of less than 1 (eg. less than 1.1, less than 1.2, less than 1.3, less than 1.4, less than 1.5, less than 2) fold in TREML1 in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1 (eg. at least 1.1, at least 1.2, at least 1.3, at least 1.4, at least 1.5, at least 1.6, at least 1.7, at least 1.8, at least 1.9, or at least 2) fold in PF4 in the sample obtained from the patient relative to the corresponding reference value, indicates that the patient has, or is at risk of developing sepsis. In one embodiment, no increase or an increase of less than 1 (eg. less than 1.1, less than 1.2, less than 1.3, less than 1.4, less than 1.5, less than 1.6, less than 1.7, or less than 1.8) fold in PF4 in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.5 (eg. at least 1.6, at least 1.7, at least 1.8, at least 1.9, at least 2, at least 2.1, at least 2.2, at least 2.3, at least 2.4, at least 2.5) fold in KIF2C in the sample obtained from the patient relative to the corresponding reference value, indicates that the patient has, or is at risk of developing sepsis (such as abdominal sepsis). In one embodiment, no increase or an increase of less than 1.5 (eg. less than 1.6, less than 1.7, less than 1.8, less than 1.9, less than 2) fold in KIF2C in the sample obtained from the patient relative to the corresponding reference value, indicates that the patient does not have, or is not at risk of developing sepsis (such as abdominal sepsis). In one embodiment, when detecting this level of fold change in the biomarker, the method is performed using a sample obtained from a patient at least 36 (eg. at least 48) hours after the patient presents with one or more clinical symptoms of a systemic inflammatory condition, or is admitted to a medical care facility. In one embodiment, when detecting this level of fold change in the biomarker, the method is performed using a sample obtained from a patient between about 24 and 48 hours after the patient presents with one or more clinical symptoms of a systemic inflammatory condition, or is admitted to a medical care facility. In one embodiment, when detecting this level of fold change in the biomarker, the method is for diagnosing abdominal sepsis in a patient.

For example, an increase of at least 1.2 (eg. at least 1.3, at least 1.4, at least 1.5, at least 1.6, at least 1.7, at least 1.8, at least 1.9, at least 2, at least 2.1, at least 2.2, at least 2.3, at least 2.4, or at least 2.5) fold in MAP1A in the sample obtained from the patient relative to the corresponding reference value, indicates that the patient has, or is at risk of developing sepsis. In one embodiment, no increase or an increase of less than 1.2 (eg. less than 1.3, less than 1.4, less than 1.5, less than 1.6, less than 1.7, less than 1.8) fold in MAP1A in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.5 (eg. at least 2, at least 2.1, at least 2.2, at least 2.3, at least 2.4, at least 2.5, at least 2.6, at least 2.7, at least 2.8, at least 2.9, or at least 3) fold in SELP in the sample obtained from the patient relative to the corresponding reference value, indicates that the patient has, or is at risk of developing sepsis. In one embodiment, no increase or an increase of less than 1.2 (eg. less than 1.3, less than 1.4, less than 1.5, less than 2) fold in SELP in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.8 (eg. at least 1.9, at least 2, at least 2.1, at least 2.2, at least 2.3, at least 2.4, or at least 2.5) fold in the amount of NEXN in the sample obtained from the patient relative to the corresponding reference value, indicates that the patient has, or is at risk of developing sepsis. In one embodiment, no increase or an increase of less than 1.1 (eg. less than 1.2, less than 1.3, less than 1.4, less than 1.5, less than 1.6, less than 1.7, less than 1.8, less than 1.9, less than 2) fold in NEXN in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 3.2 (eg. at least 3.3, at least 3.4, at least 3.5, at least 3.6, at least 3.7, at least 3.8, at least 3.9, at least 4) fold in NLRC4 in the sample obtained from the patient relative to the corresponding reference value, indicates that the patient has, or is at risk of developing sepsis. In one embodiment, no increase or an increase of less than 2.5 (eg. less than 2.6, less than 2.7, less than 2.8, less than 2.9, less than 3, less than 3.1, less than 3.2) fold in NLRC4 in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 2.7 (eg. at least 2.8, at least 2.9, at least 3, at least 3.1, at least 3.2, at least 3.3, at least 3.4, at least 3.5, at least 4) fold in CLEC1B in the sample obtained from the patient relative to the corresponding reference value, indicates that the patient has, or is at risk of developing sepsis. In one embodiment, no increase or an increase of less than 1.8 (eg. less than 1.9, less than 2, less than 2.1, less than 2.2, less than 2.3, less than 2.4, less than 2.5, less than 2.6, less than 2.7, less than 2.8, less than 2.9, less than 3) fold in CLEC1B in the sample obtained from the patient relative to the corresponding reference value, indicates that the patient does not have, or is not at risk of developing sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

As described herein, the present inventors observed that the levels of the one or more sepsis biomarkers were elevated in patients having sepsis as compared to patients having SIRS. Detection of increased levels of these biomarkers in a patient as compared to the levels detected for patients having SIRS can thus be used to diagnose the presence of sepsis. Thus, in one embodiment, when the reference value is representative of an individual having SIRS, an increase in the one or more biomarker (and/ or one or more additional biomarker) in the sample obtained from the patient relative to the corresponding reference value representative of an individual having SIRS, indicates that the patient has or is at risk of developing sepsis. Likewise, no increase in the one or more biomarker (and/ or one or more additional biomarker) in the sample obtained from the patient relative to the corresponding reference value representative of an individual having SIRS, indicates that the patient does not have or is not at risk of having sepsis. In one embodiment, the increase may be a minimum fold increase or a minimum % increase as defined above for the method for diagnosis of a systemic inflammatory condition.

In a related aspect, the present invention provides the use of one or more of: ITGB3, ITGA2B, MYL9, LCN2, TREML1, LCN15, CMTM5, PPBP, PF4, KIF2C, MAP1A, SELP, NEXN, NLRC4, CLEC1B, SLC39A8, CIQC, CIQA, MRAS, TMEM37, CIQB, PCOLCE2, KIF2C, TNF, IFI44, IFIT1, RPGRIP1, HCAR2, CXCR1, DISC1, and EPSTI1, as a biomarker for sepsis. For example, the use is of one or more of: ITGB3, ITGA2B, MYL9, LCN2, TREML1, LCN15, CMTM5, PPBP, PF4, KIF2C, MAP1A, SELP, NEXN, NLRC4, and CLEC1B. For example, the use is of one or more of: ITGB3, ITGA2B, MYL9, LCN2, TREML1, LCN15, CMTM5, PPBP, and PF4. For example, the use is of one or more of: ITGB3, ITGA2B, MYL9, LCN2, and TREML1. For example, the use is of the combination of: ITGB3, ITGA2B, MYL9, LCN2, and TREML1. In one embodiment, the use is of the one or more biomarker in the diagnosis of sepsis in a patient. In one embodiment, the sepsis is abdominal sepsis and/or pulmonary sepsis. For example, the use may comprise (i) determining the presence and/or amount of one or more biomarker in a sample obtained from a patient; and (ii) comparing the presence and/or amount of the one or more biomarker determined in said sample in (i) to a corresponding reference value to determine whether the patient has sepsis.

All embodiments described above for the method of diagnosing sepsis in a patient apply equally to the 'use' of the invention described herein. This includes all embodiments relating to the terms "sepsis", "patient", "sample", and "the one or more biomarker" described above.

As discussed herein, the present inventors observed that MRAS, PCOLCE2, TMEM37, SLC39A8, KIF2C, CIQC, CIQB, CIQA, TNF, IFI44, IFIT1, and RPGRIP1, are biomarkers specific for abdominal sepsis (see Table 3).

The present invention thus also provides a method for diagnosing abdominal sepsis in a patient, comprising:
(i) determining the presence and/or amount of one or more biomarker in a sample obtained from a patient, wherein the one or more biomarker is selected from the group consisting of: SLC39A8, CIQC, CIQA, MRAS, TMEM37, CIQB, PCOLCE2, KIF2C,TNF, IFI44, IFIT1, and RPGRIP1
(ii) comparing the presence and/or amount of the one or more biomarker determined in said sample in (i) to a corresponding reference value; and thereby determining whether the patient has or is at risk of developing abdominal sepsis.

All embodiments described above for the "method for diagnosing a systemic inflammatory condition in a patient" apply equally to the "method for diagnosing abdominal sepsis in a patient". This includes all embodiments relating to the "sample", "patient", "biomarker", and "reference value", and all embodiments relating to the step for "determining the presence and/or amount of one or more biomarker in a sample" and for the "comparison" step for making a conclusion about the disease status of the patient.

As used herein, the phrase "diagnosis of abdominal sepsis in a patient" means determining whether the patient has or is risk of developing abdominal sepsis. The systemic inflammatory condition "abdominal sepsis" diagnosed using the method of the invention is as described above for the "method for diagnosing a systemic inflammatory condition in a patient".

The "patient" for which diagnosis is performed is as described above for the "method for diagnosing a systemic inflammatory condition in a patient" and "the method for diagnosing sepsis in a patient". In one embodiment, the patient is suspected of having or being at risk of developing a systemic inflammatory condition. In one embodiment, the patient has been diagnosed as having or being at risk of developing a systemic inflammatory condition. In one embodiment, the patient has been diagnosed as having a systemic inflammatory condition using the method described herein. In one embodiment, the patient is suspected of having or being at risk of developing sepsis. In one embodiment, the patient has been diagnosed as having or being at risk of developing sepsis (eg. using the method of diagnosing sepsis in a patient as described herein and/or using the method of distinguishing between sepsis and SIRS in a patient as described herein). In one embodiment, the patient is suspected of having or being at risk of developing abdominal sepsis.

The "sample" obtained from the patient is as defined above for the "method for diagnosing a systemic inflammatory condition in a patient", including all embodiments relating to the time point at which the sample is obtained. All embodiments of the "sample" defined above for the method for diagnosing sepsis also apply to the method for diagnosing pulmonary sepsis.

The optimum time point at which a sample is obtained from a patient may depend on the biomarker being tested. For example, when testing for the biomarkers CIQC, CIQB, CIQA, and MRAS, the sample may be obtained up to 1 hour, 2 hours, 4 hours, 6 hours, 8 hours, 12 hours, 24 hours, 36 hours, 48 hours, 72 hours, or 96 hours after the patient presents with one or more clinical symptoms of a systemic inflammatory condition, or is admitted to a medical care facility. In one embodiment, the sample is obtained up to 24 hours after the patient presents with one or more clinical symptoms of a systemic inflammatory condition, or is admitted to a medical care facility. In one embodiment, the sample is obtained up to 48 hours after the patient presents with one or more clinical symptoms of a systemic inflammatory condition, or is admitted to a medical care facility.

The "one or more biomarker" of the invention is as defined above for the "method for diagnosing a systemic inflammatory condition in a patient".

As illustrated in Example 1, the present inventors observed that SLC39A8, CIQC, CIQA, MRAS, TMEM37, CIQB, PCOLCE2 KIF2C, TNF, IFI44, IFIT1, and RPGRIP1 are biomarkers of abdominal sepsis, and thus can be used in the diagnosis of abdominal sepsis.

In one embodiment, the one or more biomarker may be selected from the group consisting of: SLC39A8, CIQC, CIQA, MRAS, TMEM37, CIQB, PCOLCE2, KIF2C,TNF, IFI44, IFIT1, and RPGRIP1.

A sub-set of the biomarkers identified (IFI44, IFIT1, and RPGRIP1) were found at decreased levels in abdominal sepsis patients as compared to healthy individuals, individuals having SIRS, and/or individuals having pulmonary sepsis. In one embodiment, the one or more biomarker may be selected from the group consisting of: IFI44, IFIT1, and RPGRIP1.

A sub-set of the biomarkers identified were found at elevated levels in abdominal sepsis patients as compared to healthy individuals, individuals having SIRS, and/or individuals having pulmonary sepsis. In one embodiment, the one or more biomarker may be selected from the group consisting of: SLC39A8, CIQC, CIQA, MRAS, TMEM37, CIQB, PCOLCE2, KIF2C, and TNF.

As described Example 2, a sub-set of the markers tested (SLC39A8, CIQC, CIQA, MRAS, TMEM37, and CIQB) was observed to provide particularly accurate diagnosis of abdominal sepsis (see the ROC curve data presented in Example 2). In one embodiment, the one or more biomarker may be selected from the group consisting of: SLC39A8, CIQC, CIQA, MRAS, TMEM37, and CIQB. In one embodiment, the one or more biomarker may be selected from the group consisting of: SLC39A8, CIQC, and CIQA.

The present inventors observed that the biomarker TNF is elevated in patients having abdominal sepsis as compared to healthy individuals and patients having pulmonary sepsis. However, the inventors observed that TNF is also elevated in patients having SIRS, and thus this marker is most useful in diagnosing abdominal sepsis when a patient has already been diagnosed as having sepsis (eg. using the methods described herein for diagnosis of sepsis, or using the method described herein for distinguishing between abdominal sepsis and pulmonary sepsis). Thus, in one embodiment, the one or more biomarker may be selected from the group consisting of: SLC39A8, CIQC, CIQA MRAS, TMEM37, CIQB, PCOLCE2, KIF2C, and optionally TNF. For example, the one or more biomarker may be selected from the group consisting of: SLC39A8, CIQC, CIQA MRAS, TMEM37, CIQB, PCOLCE2, and KIF2C. In one embodiment, the one or more biomarker may be selected from the group consisting of: SLC39A8, CIQC, CIQA, MRAS, TMEM37, CIQB, and optionally TNF.

In one embodiment, the one or more biomarker may be selected from the group consisting of: MRAS, CIQC, CIQB, and CIQA. In one embodiment, the one or more biomarker may be selected from the group consisting of: PCOLCE2, TMEM37, SLC39A8, KIF2C and TNF. In one embodiment, the one or more biomarker may be selected from the group consisting of: PCOLCE2, TMEM37, SLC39A8 and KIF2C.

Each of the biomarkers of abdominal sepsis may be used alone, or in combination with any of the abdominal sepsis biomarkers in the methods and uses of the invention. For example, any combination of 1 or more, 2 or more, 3 or more, 4 or more, 5 or more, 6 or more, 7 or more, 8 or more, 9 or more, 10 or more, 11 or more, or up to and including all of the abdominal sepsis biomarkers may be used to diagnose abdominal sepsis in a patient according to the methods and uses of the invention.

In one embodiment, any combination of 1 or more (eg. 2 or more, 3 or more, 4 or more, 5 or more, 6 or more, 7 or more, 8 or more, 9 or more, 10 or more, 11 or more, or all 12) of the biomarkers selected from the group consisting of: SLC39A8, CIQC, CIQA, MRAS, TMEM37, CIQB, PCOLCE2, KIF2C, TNF, IFI44, IFIT1, and RPGRIP1 may be used to diagnose abdominal sepsis in a patient. In one embodiment, any combination of 1 or more (eg. 2 or more, 3 or more, 4 or more, 5 or more, 6 or more, 7 or more, 8 or more or all 9) of the biomarkers selected from the group consisting of: SLC39A8, CIQC, CIQA, MRAS, TMEM37, CIQB, PCOLCE2, KIF2C, and TNF may be used to diagnose abdominal sepsis in a patient. In one embodiment, any combination of 1 or more (eg. 2 or more, 3 or more, 4 or more, 5 or more, 6 or more, 7 or more or all 8) of the biomarkers selected from the group consisting of: SLC39A8, CIQC, CIQA, MRAS, TMEM37, CIQB, PCOLCE2, and KIF2C, may be used to diagnose abdominal sepsis in a patient. In one embodiment, any combination of 1 or more (eg. 2 or more, 3 or more, 4 or more or all 5) of the biomarkers selected from the group consisting of: MRAS, CIQC, CIQB, CIQA, and TNF may be used to diagnose abdominal sepsis in a patient. In one embodiment, when detecting one or more biomarker selected from this group, the sample is obtained from the patient up to 48 hours after the patient presents with one or more clinical symptoms of a systemic inflammatory condition, or is admitted to a medical care facility.

For example, the following combinations of abdominal sepsis biomarkers may be used to diagnose abdominal sepsis: (i) MRAS and CIQC; (ii) MRAS and CIQB; (iii) MRAS and PCOLCE2; (iv) MRAS and CIQA; (v) MRAS and TMEM37; (vi) MRAS and TNF; (vii) MRAS and SLC39A8; (viii) CIQC and CIQB; (ix) CIQC and PCOLCE2; (x) CIQC and CIQA; (xi) CIQC and TMEM37; (xii) CIQC and TNF; (xiii) CIQC and SLC39A8; (xiv) CIQB and PCOLCE2; (xv) CIQB and CIQA; (xvi) CIQB and TMEM37; (xvii) CIQB and TNF; (xviii) CIQB and SLC39A8; (xix) PCOLCE2 and CIQA; (xx) CIQA and TMEM37; (xxi) PCOLCE2 and TMEM37; (xxii) PCOLCE2 and TNF; (xxiii) PCOLCE2 and SLC39A8; (xxiv) CIQA and TNF; (xxv) CIQA and SLC39A8; (xxvi) TMEM37 and TNF; (xxvii) TMEM37 and SLC39A8; and (xxviii) TNF and SLC39A8; (xxxxix) MRAS and KIF2C; (xl) CIQC and KIF2C; (xli) CIQB and KIF2C; (xlii) CIQA and KIF2C; (xliii) TNF and KIF2C; (xliv) PCOLCE2 and KIF2C; (xlv) TMEM37 and KIF2C; (xlvi) SLC39A8 and KIF2C.

As described in Example 2, a sub-set of the biomarkers tested (SLC39A8, CIQC, CIQA, MRAS, TMEM37, and CIQB) was observed to provide particularly accurate diagnosis of abdominal sepsis (see the ROC curve data in Example 2). In one embodiment, any combination of 1 or more (eg. 2 or more, 3 or more, 4 or more, 5 or more, or all 6) of the biomarkers selected from the group consisting of: SLC39A8, CIQC, CIQA, MRAS, TMEM37, and CIQB, may be used to diagnose abdominal sepsis in a patient. For example, the combination of SLC39A8, CIQC, CIQA, MRAS, TMEM37, and CIQB may be used to diagnose abdominal sepsis in a patient. For example, 2 or more biomarkers selected from the group consisting of: SLC39A8, CIQC, CIQA, MRAS, TMEM37, and CIQB, may be used to diagnose abdominal sepsis in a patient. For example, 3 or more biomarkers selected from the group consisting of: SLC39A8, CIQC, CIQA, MRAS, TMEM37, and CIQB, may be used to diagnose abdominal sepsis in a patient. For example, 4 or more biomarkers selected from the group consisting of: SLC39A8, CIQC, CIQA, MRAS, TMEM37, and CIQB, may be used to diagnose abdominal sepsis in a patient. For example, the combination of the biomarkers: SLC39A8, CIQC, CIQA, MRAS, TMEM37, and CIQB, may be used to diagnose abdominal sepsis in a patient.

In one embodiment, any combination of 1 or more (eg. 2 or more, or all 3) of the biomarkers selected from the group consisting of: SLC39A8, CIQC, and CIQA may be used to diagnose abdominal sepsis in a patient. SLC39A8, CIQC, and CIQA may be used to diagnose abdominal sepsis in a patient. For example, 2 or more biomarkers selected from the group consisting of: SLC39A8, CIQC, and CIQA, may be used to diagnose abdominal sepsis in a patient. For example, the combination of the biomarkers: SLC39A8, CIQC, and CIQA may be used to diagnose abdominal sepsis in a patient

In one embodiment, the one or more biomarker is SLC39A8. In one embodiment, the one or more biomarker is CIQC. In one embodiment, the one or more biomarker is CIQA. In one embodiment, the one or more biomarker is CIQB.In one embodiment, the one or more biomarker is MRAS. In one embodiment, the one or more biomarker is TMEM37. In one embodiment, the one or more biomarker is PCOLCE2. In one embodiment, the one or more biomarker is KIF2C.. In one embodiment, the one or more biomarker is TNF. In one embodiment, the one or more biomarker is IFI44. In one embodiment, the one or more biomarker is IFIT1. In one embodiment, the one or more biomarker is RPGRIP1.

One or more additional biomarker for abdominal sepsis may also be used in the diagnosis of abdominal sepsis according to the method of the invention. Any combination of the one or more additional biomarker may be used in combination with the one or more biomarker of the invention. For example at least 1, at least 2, at least 3, at least 4, at least 5, at least 6, at least 7, at least 9, at least 10, or all 11 additional biomakers for abdominal sepsis may be used in combination with the one or more biomarker of the invention (as described herein). Typically, the one or more additional biomarker is selected from the group consisting of: SLC39A8, CIQC, CIQA, MRAS, TMEM37, CIQB, PCOLCE2, KIF2C, TNF, IFI44, IFIT1, and RPGRIP1. In one embodiment, the one or more additional biomarker is selected from the group consisting of: SLC39A8, CIQC, CIQA, MRAS, TMEM37, CIQB, PCOLCE2, KIF2C, and TNF. In one embodiment, the one or more additional biomarker is selected from the group consisting of: SLC39A8, CIQC, CIQA, MRAS, TMEM37, and CIQB.

In one embodiment, the one or more biomarker is MRAS, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, TMEM37, CIQB, PCOLCE2, KIF2C, TNF, IFI44, IFIT1, and RPGRIP1. In one embodiment, the one or more additional biomarker is selected from the group consisting of: SLC39A8, CIQC, CIQA, TMEM37, and CIQB.

In one embodiment, the one or more biomarker is PCOLCE2, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, MRAS, TMEM37, CIQB, KIF2C, TNF, IFI44, IFIT1, and RPGRIP1.

In one embodiment, the one or more biomarker is TMEM37, and the one or more additional biomarker is selected from at least 1 (eg. at least 2, at least 3, at least 4, at least 5, at least 6, at least 7, up to and including all) of the biomarkers: SLC39A8, CIQC, CIQA, MRAS, CIQB, PCOLCE2, KIF2C, TNF, IFI44, IFIT1, and RPGRIP1. In one embodiment, the one or more additional biomarker is selected from the group consisting of: SLC39A8, CIQC, CIQA, MRAS, and CIQB.

In one embodiment, the one or more biomarker is SLC39A8, and the one or more additional biomarker is selected from at least 1 (eg. at least 2, at least 3, at least 4, at least 5, at least 6, at least 7, at least 8, at least 9, at least 10, up to and including all) of the biomarkers: CIQC, CIQA, MRAS, TMEM37, CIQB, PCOLCE2, KIF2C, TNF, IFI44, IFIT1, and RPGRIP1. In one embodiment, the one or more additional biomarker is selected from the group consisting of: CIQC, CIQA, MRAS, TMEM37, and CIQB.

In one embodiment, the one or more biomarker is KIF2C, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, MRAS, TMEM37, CIQB, PCOLCE2, TNF, IFI44, IFIT1, and RPGRIP1.

In one embodiment, the one or more biomarker is CIQA, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, MRAS, TMEM37, CIQB, PCOLCE2, KIF2C, TNF, IFI44, IFIT1, and RPGRIP1. In one embodiment, the one or more additional biomarker is selected from the group consisting of: SLC39A8, CIQC, MRAS, TMEM37, and CIQB.

In one embodiment, the one or more biomarker is CIQC, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQA, MRAS, TMEM37, CIQB, PCOLCE2, KIF2C, TNF, IFI44, IFIT1, and RPGRIP1. In one embodiment, the one or more additional biomarker is selected from the group consisting of: SLC39A8, CIQA, MRAS, TMEM37, and CIQB.

In one embodiment, the one or more biomarker is CIQB, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, MRAS, TMEM37,PCOLCE2, KIF2C, TNF, IFI44, IFIT1, and RPGRIP1. In one embodiment, the one or more additional biomarker is selected from the group consisting of: SLC39A8, CIQC, CIQA, MRAS, and TMEM37.

In one embodiment, the one or more biomarker is TNF, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, MRAS, TMEM37, CIQB, PCOLCE2, KIF2C, IFI44, IFIT1, and RPGRIP1.

In one embodiment, the one or more biomarker is IFI44, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, MRAS, CIQA, TMEM37, CIQB, PCOLCE2, KIF2C, TNF, IFIT1, and RPGRIP1.

In one embodiment, the one or more biomarker is IFIT1, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, MRAS, TMEM37, CIQB, PCOLCE2, KIF2C, TNF, IFI44, and RPGRIP1.

In one embodiment, the one or more biomarker is RPGRIP1, and the one or more additional biomarker is selected from at least 1 (eg. at least 2, at least 3, at least 4, at least 5, at least 6, at least 7, at least 8, at least 9, at least 10, up to and including all) of the biomarkers: SLC39A8, CIQC, CIQA, MRAS, CIQB, TMEM37, PCOLCE2, KIF2C, TNF, IFI44, and IFIT1.

As illustrated in Figure 3, the present inventors observed that the "abdominal sepsis" biomarkers described herein (MRAS, PCOLCE2, TMEM37, SLC39A8, KIF2C, CIQC, CIQB, CIQA, and TNF) increase in abundance in patients having abdominal sepsis as compared to patients having other systemic inflammatory conditions (such as pulmonary sepsis or SIRS), as well as healthy individuals. The inventors also observed that the biomarkers IFI44, IFIT1, and RPGRIP1 decrease in abundance in patients having abdominal sepsis as compared to patients having other systemic inflammatory conditions (such as pulmonary sepsis or SIRS), as well as healthy individuals. These differences in marker abundance can be used to diagnose whether an individual has or is at risk of developing abdominal sepsis.

For example, by comparing the presence and/or amount of markers quantified in a sample obtained from a patient to the presence and/or amount of markers quantified for a reference value (such as a reference value that is representative of a healthy individual (or a population of healthy individuals), a reference value that is representative of an individual having abdominal sepsis (or a population of individuals having abdominal sepsis), a reference value that is representative of an individual having pulmonary sepsis (or a population of individuals having pulmonary sepsis), and/or a reference value that is representative of an individual having SIRS (or a population of individuals having SIRS)), it is possible to diagnose the presence (or absence) of abdominal sepsis in a patient. The method permits classification of the individual as belonging to or not belonging to the reference population (ie. by determining whether the amounts of marker quantified in the individual are statistically similar to the reference population or statistically deviate from the reference population). Hence, classification of the individual's marker profile (ie. the overall pattern of change observed for the markers quantified) as corresponding to the profile derived from a particular reference population is predictive that the individual falls (or does not fall) within the reference population.

In one embodiment, an individual may be diagnosed as having or being at risk of having abdominal sepsis, when the amount of the one or more biomarker is statistically similar to the amount determined for the corresponding reference value representative of an individual (or a population of individuals) having abdominal sepsis. In one embodiment, an individual may be diagnosed as not having or not being at risk of having abdominal sepsis when the amount of the one or more biomarker is statistically similar to the amount determined for the corresponding reference value representative of a healthy individual (or a population of healthy individuals). In one embodiment, an individual may be diagnosed as not having or not being at risk of having abdominal sepsis when the amount of the one or more biomarker is statistically similar to the amount determined for the corresponding reference value representative of an individual having SIRS (or a population of individuals having SIRS). In one embodiment, an individual may be diagnosed as not having or not being at risk of having abdominal sepsis when the amount of the one or more biomarker is statistically similar to the amount determined for the corresponding reference value representative of an individual having pulmonary sepsis (or a population of individuals having pulmonary sepsis).

In one embodiment, an individual may be diagnosed as not having or not being at risk of having abdominal sepsis when the amount of the one or more biomarker quantified statistically deviates from the amount determined for the corresponding reference value representative of an individual (or a population of individuals) having abdominal sepsis. In one embodiment, an individual may be diagnosed as having or being at risk of having abdominal sepsis when the amount of the one or more biomarker quantified statistically deviates from the amount determined for the corresponding reference value representative of a healthy individual (or a population of healthy individuals). In one embodiment, an individual may be diagnosed as having or being at risk of having abdominal sepsis when the amount of the one or more biomarker statistically deviates from the amount determined for the corresponding reference value representative of an individual having SIRS (or a population of individuals having SIRS). In one embodiment, an individual may be diagnosed as having or being at risk of having abdominal sepsis when the amount of the one or more biomarker statistically deviates from the amount determined for the corresponding reference value representative of an individual having pulmonary sepsis (or a population of individuals having pulmonary sepsis).

All embodiments described above for the classification of a patient as having or being at risk of having a systemic inflammatory condition (or as not having or not being at risk of having a systemic inflammatory condition) apply equally to the method for diagnosing whether a patient has or is at risk of having abdominal sepsis. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may be as defined above for the method of diagnosing a systemic inflammatory condition in a patient. In one embodiment, the reference value is representative of a healthy individual (or a population of healthy individuals). In one embodiment, the reference value is representative of an individual having SIRS (or a population of individuals having SIRS). In one embodiment, the reference value is representative of an individual having abdominal sepsis (or a population of individuals having abdominal sepsis). In one embodiment, the reference value is representative of an individual having pulmonary sepsis (or a population of individuals having pulmonary sepsis).

As described above for the "method for diagnosing a systemic inflammatory condition in a patient", the method of the invention may involve the use of multiple separate reference values. For example, the reference value may include one of more (eg. two or more, three or more, or all 4) of the reference values selected from: a reference value that is representative of a healthy individual (or a population of healthy individuals); a reference value that is representative of an individual having SIRS (or a population of individuals having SIRS); and a reference value that is representative of an individual having abdominal sepsis (or a population of individuals having abdominal sepsis); and a reference value that is representative of an individual having pulmonary sepsis (or a population of individuals having pulmonary sepsis).

The present inventors observed that the biomarkers for abdominal sepsis described herein (MRAS, CIQC, CIQB, PCOLCE2, CIQA, TMEM37, SLC39A8, KIF2C, TNF) each increase in abundance in samples obtained from patients having abdominal sepsis, as compared to healthy individuals. Detection of increased levels of these biomarkers in a patient as compared to the levels detected for healthy individuals can thus be used to diagnose the presence of abdominal sepsis. Thus, in one embodiment, when the reference value is representative of a healthy individual (or population of healthy individuals), an increase in the one or more biomarker for abdominal sepsis in the sample obtained from the patient relative to the corresponding reference value representative of a healthy individual, indicates that the patient has abdominal sepsis, or may be at risk of developing abdominal sepsis. Likewise, no increase in the one or more biomarker for abdominal sepsis in the sample obtained from the patient relative to the corresponding reference value representative of a healthy individual, indicates that the patient does not have abdominal sepsis.

For some of the abdominal sepsis biomarkers identified by the present inventors, increased levels of these markers were also observed in patients having other systemic inflammatory conditions (such as pulmonary sepsis and SIRS) as compared to healthy individuals, although typically much bigger increases were observed for these biomarkers in the patients having abdominal sepsis. The accuracy of abdominal sepsis diagnosis can thus be improved by looking for a "minimum" fold change or % change in the levels of the one or more biomarkers as compared to the corresponding reference value that is representative of a healthy individual. The fold increase or % increase may be as defined above for the method for diagnosis of a systemic inflammatory condition.

For example, an increase of at least 50 (eg. at least 55, at least 60, at least 70, at least 80, at least 90, at least 95 at least 100, at least 125, at least 150) fold in PCOLCE2 in the sample obtained from the patient relative to the corresponding reference value, indicates that the patient has, or is at risk of developing abdominal sepsis. In one embodiment, no increase or an increase of less than 50 (eg. less than 55, less than 60, less than 70, less than 80, less than 90, less than 95) fold in PCOLCE2 in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 7 (eg. at least 7.5, at least 8, at least 8.5) fold in TMEM37 in the sample obtained from the patient relative to the corresponding reference value, indicates that the patient has, or is at risk of developing abdominal sepsis. In one embodiment, no increase or an increase of less than 3.5 (eg. less than 4, less than 5, less than 6, less than 7) fold in TMEM37 in the sample obtained from the patient relative to the corresponding reference value, indicates that the patient does not have, or is not at risk of developing abdominal sepsis.

For example, an increase of at least 3 (eg. at least 3.5, at least 4, at least 4.5, at least 5) fold in SLC39A8 in the sample obtained from the patient relative to the corresponding reference value, indicates that the patient has, or is at risk of developing abdominal sepsis. In one embodiment, no increase or an increase of less than 2 (eg. less than 2.5, less than 3, less than 3.5, less than 4) fold in SLC39A8 in the sample obtained from the patient relative to the corresponding reference value, indicates that the patient does not have, or is not at risk of developing abdominal sepsis.

For example, an increase of at least 2.6 (eg. at least 2.7, at least 2.8, at least 2.9, at least 3, at least 3.1) fold in KIF2C in the sample obtained from the patient relative to the corresponding reference value, indicates that the patient has, or is at risk of developing abdominal sepsis. In one embodiment, no increase or an increase of less than 2.6 (eg. less than 2.7, less than 2.8, less than 2.9, less than 3) fold in KIF2C in the sample obtained from the patient relative to the corresponding reference value, indicates that the patient does not have, or is not at risk of developing abdominal sepsis.

For example, an increase of at least 12 (eg. at least 13, at least 14, at least 15, at least 16, at least 17, at least 18, at least 19, at least 20) fold in CIQC in the sample obtained from the patient relative to the corresponding reference value, indicates that the patient has, or is at risk of developing abdominal sepsis. In one embodiment, no increase or an increase of less than 7 (eg. less than 8, less than 9, less than 10, less than 11, less than 12, less than 13, less than 14, less than 15, less than 16, less than 17, less than 18, less than 19, less than 20) fold in CIQC in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 12 (eg. at least 13, at least 14, at least 15, at least 16, at least 17, at least 18, at least 19, at least 20, at least 21, at least 22, at least 23, at least 24 or at least 25) fold in CIQB in the sample obtained from the patient relative to the corresponding reference value, indicates that the patient has, or may be at risk of developing abdominal sepsis. In one embodiment, no increase or an increase of less than 6 (eg. less than 7, less than 8, less than 9, less than 10, less than 11, less than 12, less than 13, less than 14, less than 15, less than 16, less than 17, less than 18, less than 19, less than 20) fold in CIQB in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 10 (eg. at least 11, at least 12, at least 13, at least 14, at least 15, or at least 16) fold in CIQA in the sample obtained from the patient relative to the corresponding reference value, indicates that the patient has, or is at risk of developing abdominal sepsis. In one embodiment, no increase or an increase of less than 4 (eg. less than 5, less than 6, less than 7, less than 8, less than 9, less than 10, less than 11, less than 12, less than 13, less than 14, less than 15, or less than 16) fold in CIQA in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.3 (eg. at least 1.4, or at least 1.5) fold in TNF in the sample obtained from the patient relative to the corresponding reference value, indicates that the patient has, or is at risk of developing abdominal sepsis. In one embodiment, no increase or an increase of less than 1.3 (eg. less than 1.4) fold in TNF in the sample obtained from the patient relative to the corresponding reference value, indicates that the patient does not have, or is not at risk of developing abdominal sepsis. In one embodiment, the patient from which a sample is obtained has been diagnosed as having or being at risk of developing sepsis (eg. using the method of diagnosing sepsis in a patient as described herein and/or using the method of distinguishing between sepsis and SIRS in a patient as described herein).

For example, an increase of at least 1.1 (eg. at least 1.2, at least 1.3, at least 1.4, at least 1.5, or at least 1.6) fold in MRAS in the sample obtained from the patient relative to the corresponding reference value, indicates that the patient has, or is at risk of developing abdominal sepsis. In one embodiment, no increase or an increase of less than 1.1 (less than 1, less than 0.9, less than 0.8, less than 0.7, less than 0.6, less than 0.5) fold in MRAS in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

The present inventors observed that the biomarkers IFI44, IFIT1, and RPGRIP1 each decrease in abundance in samples obtained from patients having abdominal sepsis, as compared to healthy individuals. Detection of decreased levels of these biomarkers in a patient as compared to the levels detected for healthy individuals can thus be used to diagnose the presence of abdominal sepsis.

In one embodiment, when the reference value is representative of a healthy individual, a decrease in the one or more biomarker for abdominal sepsis in the sample obtained from the patient relative to the corresponding reference value representative of a healthy individual, indicates that the patient has abdominal sepsis, or may be at risk of developing abdominal sepsis. Likewise, no decrease in the one or more biomarker for abdominal sepsis in the sample obtained from the patient relative to the corresponding reference value representative of a healthy individual, indicates that the patient does not have abdominal sepsis.

For the biomarkers IFIT1 and RPGRIP1, decreased levels of these markers were also observed in patients having other systemic inflammatory conditions (such as pulmonary sepsis and SIRS) as compared to healthy individuals, although typically much bigger decreases were observed for these biomarkers in the patients having abdominal sepsis. The accuracy of abdominal sepsis diagnosis can thus be improved by looking for a "minimum" fold decrease or % decrease in the levels of the one or more biomarkers as compared to the corresponding reference value that is representative of a healthy individual. The fold decrease or % decrease may be as defined above for the method for diagnosis of a systemic inflammatory condition.

For example, a decrease of at least 0.5 (eg. at least 0.6, at least 0.7, at least 0.8, at least 0.9, or at least 1) fold in IFI44 in the sample obtained from the patient relative to the corresponding reference value, indicates that the patient has, or is at risk of developing abdominal sepsis. In one embodiment, no decrease in IFI44 in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, a decrease of at least 2.5 (eg. at least 2.6, at least 2.7, at least 2.8, at least 2.9, or at least 3) fold in IFIT1 in the sample obtained from the patient relative to the corresponding reference value, indicates that the patient has, or is at risk of developing abdominal sepsis. In one embodiment, no decrease or a decrease of less than 1.9 (eg. less than 2, less than 2.1, less than 2.2, less than 2.3, less than 2.4, less than 2.5) fold in IFIT1 in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, a decrease of at least 1.75 (eg. at least 1.8, at least 1.9, at least 2, at least 2.5, at least 3, at least 3.5, at least 4, at least 4.5, or at least 5) fold in RPGRIP1 in the sample obtained from the patient relative to the corresponding reference value, indicates that the patient has, or is at risk of developing abdominal sepsis. In one embodiment, no decrease or a decrease of less than 1.4 (eg. less than 1.5, less than 1.6, less than 1.7, less than 1.8, less than 1.9, less than 2) fold in RPGRIP1 in the sample obtained from the patient relative to the corresponding reference value, indicates that the patient does not have, or is not at risk of developing abdominal sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

As described herein, the present inventors observed that the levels of the one or more sepsis biomarkers (MRAS, PCOLCE2, TMEM37, SLC39A8, KIF2C, CIQC, CIQB, CIQA, and TNF) were elevated in patients having abdominal sepsis as compared to patients having other systemic inflammatory conditions such as pulmonary sepsis or SIRS (with the exception of TNF which is increased in abundance as compared to patients having pulmonary sepsis only). Detection of increased levels of these biomarkers in a patient as compared to the levels detected for patients having one or more of these other systemic inflammatory conditions can thus be used to diagnose the presence of abdominal sepsis.

Thus, in one embodiment, an increase in the one or more biomarker (and/ or one or more additional biomarker) in the sample obtained from the patient relative to the corresponding reference value representative of an individual having pulmonary sepsis, indicates that the patient has or is at risk of developing abdominal sepsis. Likewise, no increase in the one or more biomarker (and/ or one or more additional biomarker) in the sample obtained from the patient relative to the corresponding reference value representative of an individual having pulmonary sepsis, indicates that the patient does not have abdominal sepsis.

In one embodiment, an increase in the one or more biomarker (and/ or one or more additional biomarker) in the sample obtained from the patient relative to the corresponding reference value representative of an individual having SIRS, indicates that the patient has or is at risk of developing abdominal sepsis. Likewise, no increase in the one or more biomarker (and/ or one or more additional biomarker) in the sample obtained from the patient relative to the corresponding reference value representative of an individual having SIRS, indicates that the patient does not have abdominal sepsis.

In one embodiment, the patient may be diagnosed as having abdominal sepsis, or being at risk of developing abdominal sepsis, when the one or more biomarker (or the one or more additional biomarker) increases by at least 1 (e.g. at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value representative of an individual having pulmonary sepsis and/or an individual having SIRS.

As described herein, the present inventors observed that the levels of the biomarkers IFI44, IFIT1, and RPGRIP1 were decreased in patients having abdominal sepsis as compared to patients having other systemic inflammatory conditions such as pulmonary sepsis or SIRS. Detection of decreased levels of these biomarkers in a patient as compared to the levels detected for patients having one or more of these other systemic inflammatory conditions can thus be used to diagnose the presence of abdominal sepsis.

Thus, in one embodiment, a decrease in the one or more biomarker (and/ or one or more additional biomarker) in the sample obtained from the patient relative to the corresponding reference value representative of an individual having pulmonary sepsis, indicates that the patient has or is at risk of developing abdominal sepsis. Likewise, no decrease in the one or more biomarker (and/ or one or more additional biomarker) in the sample obtained from the patient relative to the corresponding reference value representative of an individual having pulmonary sepsis, indicates that the patient does not have abdominal sepsis.

In one embodiment, a decrease in the one or more biomarker (and/ or one or more additional biomarker) in the sample obtained from the patient relative to the corresponding reference value representative of an individual having SIRS, indicates that the patient has or is at risk of developing abdominal sepsis. Likewise, no decrease in the one or more biomarker (and/ or one or more additional biomarker) in the sample obtained from the patient relative to the corresponding reference value representative of an individual having SIRS, indicates that the patient does not have abdominal sepsis.

In one embodiment, the patient may be diagnosed as having abdominal sepsis, or being at risk of developing abdominal sepsis, when the one or more biomarker (or the one or more additional biomarker) decreases by at least 0.1 (e.g. at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value representative of an individual having pulmonary sepsis and/or an individual having SIRS.

In a related aspect, the present invention also provides the use of one or more of: SLC39A8, CIQC, CIQA, MRAS, TMEM37, CIQB, PCOLCE2, KIF2C, TNF, IFI44, IFIT1, and RPGRIP1 as a biomarker for abdominal sepsis. In one embodiment, the one or more biomarker is selected from the group consisting of: SLC39A8, CIQC, CIQA, MRAS, TMEM37, CIQB,PCOLCE2, KIF2C, and TNF. In one embodiment, the one or more biomarker is selected from the group consisting of: SLC39A8, CIQC, CIQA, MRAS, TMEM37, CIQB,PCOLCE2, and KIF2C. In one embodiment, the one or more biomarker is selected from the group consisting of: SLC39A8, CIQC, CIQA, MRAS, TMEM37, and CIQB. In one embodiment, the one or more biomarker is selected from the group consisting of: SLC39A8, CIQC, and CIQA. In one embodiment, the use is of the one or more biomarker in the diagnosis of abdominal sepsis in a patient.

All embodiments described above for the method of diagnosing abdominal sepsis in a patient apply equally to the 'use' of the invention described herein. This includes all embodiments relating to the terms "abdominal sepsis", "patient", "sample", and "the one or more biomarker".

As discussed herein, the present inventors have also observed that the levels of HCAR2, CXCR1, DISC1, EPSTI1, and IFI44, are elevated in patients having pulmonary sepsis, and are thus suitable for use as biomarkers for pulmonary sepsis (see Table 3).

The present invention therefore also provides a method for diagnosing pulmonary sepsis in a patient, comprising:
(i) determining the presence and/or amount of one or more biomarker in a sample obtained from a patient, wherein the one or more biomarker is selected from the group consisting of: HCAR2, CXCR1, DISC1, EPSTI1, and IFI44;
(ii) comparing the presence and/or amount of the one or more biomarker determined in said sample in (i) to a corresponding reference value; and thereby determining whether the patient has or is at risk of developing pulmonary sepsis.

All embodiments described above for the "method for diagnosing a systemic inflammatory condition in a patient" apply equally to the "method for diagnosing pulmonary sepsis in a patient". This includes all embodiments relating to the "sample", "patient", "biomarker", and "reference value", and all embodiments relating to the step for "determining the presence and/or amount of one or more biomarker in a sample" and for the "comparison" step for determining whether the patient has or is at risk of developing pulmonary sepsis.

As used herein, the phrase "diagnosis of pulmonary sepsis in a patient" means determining whether the patient has or is risk of developing pulmonary sepsis. The term "pulmonary sepsis" is as described above for the "method for diagnosing a systemic inflammatory condition in a patient".

The "patient" for which diagnosis is performed is as defined above for the "method for diagnosing a systemic inflammatory condition in a patient", and the "method for diagnosing sepsis in a patient". In one embodiment, the patient is suspected of having or being at risk of developing a systemic inflammatory condition. In one embodiment, the patient has been diagnosed as having or being at risk of developing a systemic inflammatory condition. In one embodiment, the patient has been diagnosed as having a systemic inflammatory condition using the method described herein. In one embodiment, the patient is suspected of having or being at risk of developing sepsis. In one embodiment, the patient has been diagnosed as having or being at risk of developing sepsis (eg. using the method of diagnosing sepsis in a patient as described herein and/or using the method of distinguishing between sepsis and SIRS in a patient as described herein). In one embodiment, the patient is suspected of having or being at risk of developing pulmonary sepsis.

The "sample" obtained from the patient is as defined above for the "method for diagnosing a systemic inflammatory condition in a patient", including all embodiments relating to the time point at which the sample is obtained. All embodiments of the "sample" described above for the method for diagnosing sepsis also apply to the method for diagnosing pulmonary sepsis.

The "one or more biomarker" of the invention is as defined above for the "method for diagnosing a systemic inflammatory condition in a patient".

In one embodiment, the one or more biomarker may be selected from the group consisting of: HCAR2, CXCR1, DISC1, EPSTI1, and IFI44. For example, the one or more biomarker may be selected from the group consisting of: EPSTI1 and DISC1. For example, the one or more biomarker may be selected from the group consisting of: CXCR1, HCAR2, and IFI44.

The present inventors observed that the biomarkers CXCR1, HCAR2, and IFI44 are elevated in patients having pulmonary sepsis as compared to patients having abdominal sepsis. However, these biomarkers were also observed as being elevated in patients having SIRS, and thus these biomarkers are particularly useful for diagnosing pulmonary sepsis in patients already diagnosed as having sepsis (eg. using the methods described herein for diagnosis of sepsis, or using the method described herein for distinguishing between abdominal sepsis and pulmonary sepsis). Thus, when the patient has already been diagnosed as having sepsis, the one or more biomarker may be selected from the group consisting of: CXCR1, HCAR2, and IFI44.

Each of the biomarkers of pulmonary sepsis may be used alone, or in combination with any of the pulmonary sepsis biomarkers in the method of the invention. For example, any combination of 1 or more, 2 or more, 3 or more, 4 or more, or up to and including all of the pulmonary sepsis biomarkers may be used to diagnose pulmonary sepsis in a patient according to the method of the invention.

In one embodiment, the one or more biomarker is HCAR2. In one embodiment, the one or more biomarker is CXCR1. In one embodiment, the one or more biomarker is DISC1. In one embodiment, the one or more biomarker is EPSTI1. In one embodiment, the one or more biomarker is IFI44.

In one embodiment, any combination of 1 or more (eg. 2 or more, 3 or more, 4 or more, or all 5) of the biomarkers selected from the group consisting of: HCAR2, CXCR1, DISC1, EPSTI1, and IFI44, may be used to diagnose pulmonary sepsis in a patient. In one embodiment, any combination of 1 or more (eg. 2 or more, or all 3) of the biomarkers selected from the group consisting of: CXCR1, HCAR2, and IFI44 may be used to diagnose pulmonary sepsis in a patient. In one embodiment, any combination of 1 or more (eg. or both) of the biomarkers selected from the group consisting of: EPSTI1 and DISC1, may be used to diagnose pulmonary sepsis in a patient.

For example, the following combinations of pulmonary sepsis biomarkers may be used to diagnose pulmonary sepsis: (i) EPSTI1 and HCAR2; (ii) EPSTI1 and DISC1; (iii) EPSTI1 and CXCR1; (iv) EPSTI1 and IFI44; (v) DISC1 and CXCR1; (vi) DISC1 and HCAR2; (vii) DISC1 and IFI44; (viii) CXCR1 and HCAR2; (ix) CXCR1 and IFI44; (x) HCAR2 and IFI44.

As described in Example 2, a sub-set of the biomarkers tested (HCAR2, CXCR1, DISC1) was observed to provide particularly accurate diagnosis of pulmonary sepsis (see the ROC curve data in Example 2). In one embodiment, any combination of 1 or more (eg. 2 or more, or all 3) of the biomarkers selected from the group consisting of: HCAR2, CXCR1, DISC1, may be used to diagnose pulmonary sepsis in a patient. For example, the combination of HCAR2, CXCR1, and DISC1 may be used to diagnose abdominal sepsis in a patient. For example, the combination of HCAR2 and CXCR1 may be used to diagnose abdominal sepsis in a patient.

One or more additional biomarker for pulmonary sepsis may also be used in the diagnosis of pulmonary sepsis according to the method of the invention. Any combination of the one or more additional biomarker may be used in combination with the one or more biomarker of the invention. For example at least 1, at least 2, at least 3, or all 4 additional biomakers for pulmonary sepsis may be used in combination with the one or more biomarker of the invention (as described herein). Typically, the one or more additional biomarker is selected from the group consisting of: HCAR2, CXCR1, DISC1, EPSTI1, and IFI44. In one embodiment, one or more additional biomarker is selected from the group consisting of: HCAR2, CXCR1, and DISC1.

In one embodiment, the one or more biomarker is HCAR2, and the one or more additional biomarker is selected from at least 1 (eg. at least 2, at least 3, up to and including all) of the biomarkers: EPSTI1, DISC1, CXCR1, and IFI44. In one embodiment, the one or more additional biomarker is selected from CXCR1 and/or DISC1.

In one embodiment, the one or more biomarker is CXCR1, and the one or more additional biomarker is selected from at least 1 (eg. at least 2, at least 3, up to and including all) of the biomarkers: EPSTI1, DISC1, HCAR2, and IFI44. In one embodiment, the one or more additional biomarker is selected from HCAR2 and/or DISC1.

In one embodiment, the one or more biomarker is DISC1, and the one or more additional biomarker is selected from at least 1 (eg. at least 2, at least 3, up to and including all) of the biomarkers: EPSTI1, HCAR2, CXCR1, and IFI44. In one embodiment, the one or more additional biomarker is selected from HCAR2 and/or CXCR1.

In one embodiment, the one or more biomarker is EPSTI1, and the one or more additional biomarker is selected from at least 1 (eg. at least 2, at least 3, up to and including all) of the biomarkers: DISC1, CXCR1, HCAR2, and IFI44.

In one embodiment, the one or more biomarker is IFI44, and the one or more additional biomarker is selected from at least 1 (eg. at least 2, at least 3, up to and including all) of the biomarkers: EPSTI1, DISC1, CXCR1, and HCAR2.

As illustrated in Figure 3, the present inventors observed that the "pulmonary sepsis" biomarkers described herein increased in abundance in patients having pulmonary sepsis as compared to patients having other systemic inflammatory conditions (such as abdominal sepsis or SIRS), as well as healthy individuals. These differences in marker abundance can be used to diagnose whether an individual has or is at risk of developing pulmonary sepsis.

For example, by comparing the amount of markers quantified in a sample obtained from a patient to the amount of markers quantified for a reference value (such as a reference value that is representative of a healthy individual (or a population of healthy individuals), a reference value that is representative of an individual having abdominal sepsis (or a population of individuals having abdominal sepsis), a reference value that is representative of an individual having pulmonary sepsis (or a population of individuals having pulmonary sepsis), and/or a reference value that is representative of an individual having SIRS (or a population of individuals having SIRS)), it is possible to diagnose the presence (or absence) of pulmonary sepsis in a patient. The method permits classification of the individual as belonging to or not belonging to the reference population (ie. by determining whether the amounts of marker quantified in the individual are statistically similar to the reference population or statistically deviate from the reference population). Hence, classification of the individual's marker profile (ie. the overall pattern of change observed for the markers quantified) as corresponding to the profile derived from a particular reference population is predictive that the individual falls (or does not fall) within the reference population.

In one embodiment, an individual may be diagnosed as having or being at risk of having pulmonary sepsis, when the amount of the one or more biomarker is statistically similar to the amount determined for the corresponding reference value representative of an individual (or a population of individuals) having pulmonary sepsis. In one embodiment, an individual may be diagnosed as not having or not being at risk of having pulmonary sepsis when the amount of the one or more biomarker is statistically similar to the amount determined for the corresponding reference value representative of a healthy individual (or a population of healthy individuals). In one embodiment, an individual may be diagnosed as not having or not being at risk of having pulmonary sepsis when the amount of the one or more biomarker is statistically similar to the amount determined for the corresponding reference value representative of an individual having abdominal sepsis (or a population of individuals having abdominal sepsis). In one embodiment, an individual may be diagnosed as not having or not being at risk of having pulmonary sepsis when the amount of the one or more biomarker is statistically similar to the amount determined for the corresponding reference values representative of an individual having SIRS (or a population of individuals having SIRS).

In one embodiment, an individual may be diagnosed as not having or not being at risk of having pulmonary sepsis when the amount of the one or more biomarker statistically deviates from the amount determined for the corresponding reference value representative of an individual (or a population of individuals) having pulmonary sepsis. In one embodiment, an individual may be diagnosed as having or being at risk of having pulmonary sepsis when the amount of the one or more biomarker statistically deviates from the amount determined for the corresponding reference values representative of a healthy individual (or a population of healthy individuals). In one embodiment, an individual may be diagnosed as having or being at risk of having pulmonary sepsis when the amount of the one or more biomarkers statistically deviates from the amount determined for the corresponding reference value representative of an individual having abdominal sepsis (or a population of individuals having abdominal sepsis). In one embodiment, an individual may be diagnosed as having or being at risk of having pulmonary sepsis when the amount of the one or more biomarker statistically deviates from the amount determined for the corresponding reference values representative of an individual having SIRS (or a population of individuals having SIRS).

All embodiments described above for the classification of a patient as having or being at risk of having a systemic inflammatory condition (or as not having or not being at risk of having a systemic inflammatory condition) apply equally to the method for diagnosing whether a patient has or is at risk of having pulmonary sepsis. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may be as defined above for the method of diagnosing a systemic inflammatory condition in a patient. In one embodiment, the reference value is representative of a healthy individual (or a population of healthy individuals). In one embodiment, the reference value is representative of an individual having SIRS (or a population of individuals having SIRS). In one embodiment, the reference value is representative of an individual having abdominal sepsis (or a population of individuals having abdominal sepsis). In one embodiment, the reference value is representative of an individual having pulmonary sepsis (or a population of individuals having pulmonary sepsis).

As described above for the "method for diagnosing a systemic inflammatory condition in a patient", the method of the invention may involve the use of multiple separate reference values. For example, the reference value may include one of more (eg. two or more, three of more, or all 4) of the reference values selected from: a reference value that is representative of a healthy individual (or a population of healthy individuals); a reference value that is representative of an individual having SIRS (or a population of individuals having SIRS); a reference value that is representative of an individual having abdominal sepsis (or a population of individuals having abdominal sepsis), and a reference value that is representative of an individual having pulmonary sepsis (or a population of individuals having pulmonary sepsis).

The present inventors observed that the pulmonary sepsis biomarkers EPSTI1, DISC1, CXCR1, HCAR2 and IFI44 each increase in abundance in samples obtained from patients having pulmonary sepsis, as compared to healthy individuals. Detection of increased levels of these biomarkers in a patient as compared to the levels detected for healthy individuals can thus be used to diagnose the presence of pulmonary sepsis.

In one embodiment, an increase in the one or more biomarker for pulmonary sepsis in the sample obtained from the patient relative to the corresponding reference value representative of a healthy individual, indicates that the patient has pulmonary sepsis, or is at risk of developing pulmonary sepsis. Likewise, no increase in the one or more biomarker for pulmonary sepsis in the sample obtained from the patient relative to the corresponding reference value representative of a healthy individual, indicates that the patient does not have pulmonary sepsis.

For some of the pulmonary sepsis biomarkers identified by the present inventors (DISC1, CXCR1, HCAR2, and IFI44), increased levels of these markers were also observed in patients having other systemic inflammatory conditions (abdominal sepsis or SIRS) as compared to healthy individuals, although much bigger increases were observed for these biomarkers in the patients having pulmonary sepsis. The accuracy of pulmonary sepsis diagnosis can thus be improved by looking for a "minimum" fold increase or % increase in the levels of the one or more biomarkers as compared to the corresponding reference value that is representative of a healthy individual. The fold increase or % increase may be as defined above for the method for diagnosis of a systemic inflammatory condition.

For example, an increase of at least 1 (eg. at least 1.05, at least 1.1, at least 1.15. at least 1.2, at least 1.25, at least 1.3) fold in EPSTI1 in the sample obtained from the patient relative to the corresponding reference value, indicates that the patient has, or is at risk of developing pulmonary sepsis. In one embodiment, no increase or an increase of less than 1 (eg. less than 1.05, less than 1.1) fold in EPSTI1 in the sample obtained from the patient relative to the corresponding reference value, indicates that the patient does not have, or is not at risk of developing pulmonary sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 1.8 (eg. at least 2, at least 2.1, at least 2.2, at least 2.3, at least 2.4, at least 2.5, at least 3) fold in DISC1 in the sample obtained from the patient relative to the corresponding reference value, indicates that the patient has, or is at risk of developing pulmonary sepsis. In one embodiment, no increase or an increase of less than 1.3 (eg. less than 1.4, less than 1.5, less than 1.6, less than 1.7, less than 1.8, less than 1.9, less than 2) fold in DISC1 in the sample obtained from the patient relative to the corresponding reference value, indicates that the patient does not have, or is not at risk of developing pulmonary sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

For example, an increase of at least 3.5 (eg. at least 3.6, at least 3.7, at least 3.8, at least 3.9, at least 4) fold in CXCR1 in the sample obtained from the patient relative to the corresponding reference value, indicates that the patient has, or is at risk of developing pulmonary sepsis. In one embodiment, no increase or an increase of less than 3.5 (eg. less than 3, less than 2.5, less than 2) fold in CXCR1 in the sample obtained from the patient relative to the corresponding reference value, indicates that the patient does not have, or is not at risk of developing pulmonary sepsis. In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility. In one embodiment, the patient from which a sample is obtained has been diagnosed as having or being at risk of developing sepsis (eg. using the method of diagnosing sepsis in a patient as described herein and/or using the method of distinguishing between sepsis and SIRS in a patient as described herein).

For example, an increase of at least 1.4 (eg. at least 1.5, at least 1.6, at least 1.7, at least 1.8, at least 1.9, at least 2) fold in HCAR2 in the sample obtained from the patient relative to the corresponding reference value, indicates that the patient has, or is at risk of developing pulmonary sepsis. In one embodiment, no increase or an increase of less than 1.5 (eg. less than 1.4, less than 1.3, less than 1.2, less than 1.1, less than 1) fold in HCAR2 in the sample obtained from the patient relative to the corresponding reference value, indicates that the patient does not have, or is not at risk of developing pulmonary sepsis. In one embodiment, the patient from which a sample is obtained has been diagnosed as having or being at risk of developing sepsis (eg. using the method of diagnosing sepsis in a patient as described herein and/or using the method of distinguishing between sepsis and SIRS in a patient as described herein).

For example, an increase of at least 1.4 (eg. at least 1.5, at least 1.6, at least 1.7, at least 1.8, at least fold 1.9, or at least 2) fold in IFI44 in the sample obtained from the patient relative to the corresponding reference value, indicates that the patient has, or is at risk of developing pulmonary sepsis. In one embodiment, no increase or an increase of less than 1.7 (eg. less than 1.6, less than 1.5, less than 1.4, less than 1.3, less than 1.2, less than 1.1) fold in IFI44 in the sample obtained from the patient relative to the corresponding reference value, indicates that the patient does not have, or is not at risk of developing pulmonary sepsis. In one embodiment, the patient from which a sample is obtained has been diagnosed as having or being at risk of developing sepsis (eg. using the method of diagnosing sepsis in a patient as described herein and/or using the method of distinguishing between sepsis and SIRS in a patient as described herein). In one embodiment, when detecting this level of fold change in the biomarker, the method is performed using a sample obtained from a patient up to 24 (eg. up to 36, up to 48, up to 72, or up to 96) hours after the patient presents with one or more clinical symptoms of a systemic inflammatory condition, or is admitted to a medical care facility.

As described herein, the present inventors observed that the levels of the one or more "pulmonary sepsis" biomarkers were elevated in patients having pulmonary sepsis as compared to patients having other systemic inflammatory conditions such as abdominal sepsis or SIRS. Detection of increased levels of these biomarkers in a patient as compared to the levels detected for patients having one or more of these other systemic inflammatory conditions can thus be used to diagnose the presence of pulmonary sepsis.

In one embodiment, when the reference value is representative of an individual having abdominal sepsis, an increase in the one or more biomarker (and/ or one or more additional biomarker) in the sample obtained from the patient relative to the corresponding reference value indicates that the patient has or is at risk of developing pulmonary sepsis. Likewise, no increase in the one or more biomarker (and/ or one or more additional biomarker) in the sample obtained from the patient relative to the corresponding reference value, indicates that the patient does not have pulmonary sepsis.

In one embodiment, when the reference value is representative of an individual having SIRS, an increase in the one or more biomarker (and/ or one or more additional biomarker) in the sample obtained from the patient relative to the corresponding reference value, indicates that the patient has or is at risk of developing pulmonary sepsis. Likewise, no increase in the one or more biomarker (and/ or one or more additional biomarker) in the sample obtained from the patient relative to the corresponding reference value, indicates that the patient does not have pulmonary sepsis.

In one embodiment, the patient may be diagnosed as having pulmonary sepsis, or being at risk of developing pulmonary sepsis, when the one or more biomarker (or the one or more additional biomarker) increases by at least 0.1 (e.g. at least 0.2, at least 0.3, at least 0.4, at least 0.5, at least 0.6, at least 0.7, at least 0.9, at least 1, at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, or at least 50) fold in the sample obtained from the patient relative to the corresponding reference value representative of an individual having abdominal sepsis and/or an individual having SIRS.

In a related aspect, the present invention also provides the use of one or more of: HCAR2, CXCR1, DISC1, EPSTI1, and IFI44 as a biomarker for pulmonary sepsis. In one embodiment, the one or more biomarker is selected from the group consisting of: HCAR2, CXCR1, and DISC1. In one embodiment, the one or more biomarker is selected from the group consisting of: HCAR2, and CXCR1. In one embodiment, the present invention provides the use of a combination of HCAR2, CXCR1, and optionally DISC1 as biomarkers for pulmonary sepsis. In one embodiment, the use is of the one or more biomarker in the diagnosis of pulmonary sepsis in a patient.

All embodiments described above for the method of diagnosing pulmonary sepsis in a patient apply equally to the 'use' of the invention described herein. This includes all embodiments relating to the terms "pulmonary sepsis", "patient", "sample", and "the one or more biomarker".

The method for diagnosis of sepsis, the method for diagnosis of abdominal sepsis and/or the method for diagnosis of pulmonary sepsis as described herein can be used in a decision tree process to investigate the health of a patient having or suspected of having a systemic inflammatory condition. For example, the method for diagnosis of sepsis, the method for diagnosis of abdominal sepsis and/or the method for diagnosis pulmonary sepsis as described herein in a patient can be performed before, after, or in addition to any of the methods of the invention described herein.

In one embodiment, the method of the invention for diagnosing sepsis in a patient (as described herein) can be performed subsequent to (or in addition to) the method for diagnosing whether a patient has a systemic inflammatory condition (as described herein). If the patient tests positive for a systemic inflammatory condition (using the method for diagnosing whether a patient has a systemic inflammatory condition), they may be tested for sepsis using the diagnostic method described herein. In one embodiment, the above combination of methods are performed as described, and if the patient tests positive for sepsis, the patient may be further tested for abdominal sepsis and/or pulmonary sepsis using the diagnostic methods of the invention described herein, so as to determine whether the patient has or is at risk of developing abdominal and/or pulmonary sepsis.

In one embodiment, the method of the invention for diagnosing sepsis in a patient (as described herein) can be performed subsequent to (or in addition to) the method for distinguishing between sepsis and SIRS in a patient (as described herein). If the patient tests positive for sepsis using the distinguishing method of the invention, they may be tested for sepsis using the diagnostic method described herein, so as to further confirm whether the patient has or is at risk of developing sepsis. In one embodiment, the above combination of methods are performed as described, and if the patient tests positive for sepsis, the patient may be further tested for abdominal sepsis and/or pulmonary sepsis using the diagnostic methods of the invention described herein, so as to detemine whether the paitent has or is at risk of developing abdominal and/or pulmonary sepsis.

In one embodiment, the method for diagnosis of sepsis may be performed subsequent to (or in addition to) the method for diagnosing whether a patient has a systemic inflammatory condition (as described herein), and the method for distinguishing between sepsis and SIRS in a patient (as described herein). For example, the patient may be tested using the method for diagnosing whether a patient has a systemic inflammatory condition (as described herein). If the patient tests positive for a systemic inflammatory condition, they may be tested using the distinguishing method of the invention (as described herein) to determine whether them patient has or is at risk of developing sepsis and/or SIRS. If the patient tests positive for sepsis using the distinguishing method of the invention, they may be tested for sepsis using the diagnostic method described herein, so as to further confirm the diagnosis. In one embodiment, the above combination of methods are performed as described, and if the patient tests positive for sepsis, the patient may be further tested for abdominal sepsis and/or pulmonary sepsis using the diagnostic methods of the invention described herein, so as to determine whether the paitent has or is at risk of developing abdominal and/or pulmonary sepsis.

In one embodiment, the method for diagnosing abdominal sepsis and/or pulmonary sepsis in a patient (as described herein) may be performed subsequent to (or in addition to) the method for diagnosing whether a patient has a systemic inflammatory condition (as described herein). If the patient tests positive for a systemic inflammatory condition (using the method of the invention for diagnosing whether a patient has a systemic inflammatory condition), they may be tested for abdominal sepsis and/or pulmonary sepsis using the diagnostic methods described herein to determine whether the patient has or is at risk of developing abdominal sepsis and/or pulmonary sepsis.

In one embodiment, the method of the invention for diagnosing abdominal sepsis and/or pulmonary sepsis in a patient (as described herein) can be performed subsequent to (or in addition to) the method for distinguishing between sepsis and SIRS in a patient (as described herein). If the patient tests positive for sepsis using the distinguishing method of the invention, they may be tested for abdominal sepsis and/or pulmonary sepsis using the diagnostic methods described herein, so as to further determine whether the patient has or is at risk of developing abdominal sepsis and/or pulmonary sepsis.

In one embodiment, the method for diagnosing abdominal sepsis and/or pulmonary sepsis in a patient (as described herein) may be performed subsequent to (or in addition to) the method for diagnosing whether a patient has a systemic inflammatory condition (as described herein), and the method for distinguishing between sepsis and SIRS in a patient (as described herein). For example, the patient may be tested using the method for diagnosing whether a patient has a systemic inflammatory condition (as described herein). If the patient tests positive for a systemic inflammatory condition, they may be tested using the distinguishing method of the invention (as described herein) to determine whether the patient has or is at risk of developing sepsis and/or SIRS. If the patient tests positive for sepsis using the distinguishing method of the invention, they may be tested for abdominal sepsis and/or pulmonary sepsis using the diagnostic methods described herein, so as to determine whether the patient has or is at risk of developing abdominal sepsis and/or pulmonary sepsis.

Furthermore, in one embodiment, each of above combination of methods may be performed as described, and if the patient tests postive for a systemic inflammatory condition, they may be tested for SIRS using the diagnostic method described herein, in addition to being tested for sepsis, abdominal sepsis and/or pulmonary sepsis using the methods described herein.

The above described combination of methods may be performed in parallel to determine the disease status of a patient by simultaneously (or substantially simultaneously) investigating the expression of all the biomarkers in a sample obtained from the patient, and determining whether the patient has or is at risk of having sepsis (such as abdominal or pulmonary sepsis).

When performing these different methods in a decision tree process, the sample used in each step of the method may be the same sample obtained from the patient (as described herein). When the method comprises multiple quantification steps, all the steps may be performed at the same time (e.g. in parallel) and/or using the same sample.

### Methods for distinguishing between different types of systemic inflammatory conditions

Sepsis and SIRS are both systemic inflammatory conditions associated with overlapping clinical symptoms. Distinguishing between these conditions is important, because different treatments are required for the two conditions. As described herein, the present inventors have identified a set of biomarkers that is predictive of sepsis and a separate set of biomarkers that is predictive of SIRS in patients. Using these distinct sets of biomarkers, the present inventors have developed a rapid and sensitive way to distinguish between SIRS and sepsis in a patient by quantifying one or more biomarker for sepsis and/or one or more biomarker for SIRS in a sample obtained from a patient, so as to determine whether the patient has a biomarker profile that is predictive of sepsis or SIRS.

The present invention therefore provides a method for distinguishing between sepsis and SIRS in a patient, comprising:
(i) determining the presence and/or amount of one or more biomarker for sepsis (as described herein), and/or one or more biomarker for SIRS (as described herein) in a sample obtained from a patient,
(ii) comparing the presence and/or amount of the one or more biomarker for sepsis and/or the one or more biomarker for SIRS determined in said sample in (i) to a corresponding reference value; and thereby determining whether the patient has sepsis and/or SIRS.

As illustrated in Example 2, the distinguishing method of the invention can be performed using only one or more of the sepsis biomarker described herein, or only one or more of the SIRS biomarkers described herein. These biomarkers can be used on their own to distinguish sepsis and SIRS because their expression correlates with the patient's disease condition (i.e. the presence and/or amount of these biomarkers depends on whether a patient has sepsis or SIRS or is healthy). Determining the presence and/or amount of either of these biomarkers and comparing this to a corresponding reference value (such as a reference value that is representative of a healthy individual, a sepsis patient and/or a SIRS patient) therefore allows the disease status of the patient to be determined.

In one embodiment, the present invention provides a method for distinguishing between sepsis and SIRS in a patient, comprising:
(i) determining the presence and/or amount of one or more biomarker for sepsis (as described herein) in a sample obtained from a patient,
(ii) comparing the presence and/or amount of the one or more biomarker for sepsis determined in said sample in (i) to a corresponding reference value; and thereby determining whether the patient has sepsis and/or SIRS.

In one embodiment, the present invention provides a method for distinguishing between sepsis and SIRS in a patient, comprising:
(i) determining the presence and/or amount of one or more biomarker for SIRS (as described herein) in a sample obtained from a patient,
(ii) comparing the presence and/or amount of the one or more biomarker for SIRS determined in said sample in (i) to a corresponding reference value; and thereby determining whether the patient has sepsis and/or SIRS.

Alternatively, the one or more biomarker for sepsis may used in combination with the one or more biomarker for SIRS to distinguish between sepsis and SIRS in a patient.

In one embodiment, the present invention provides a method for distinguishing between sepsis and SIRS in a patient, comprising:
(i) determining the presence and/or amount of one or more biomarker for sepsis (as described herein), and one or more biomarker for SIRS (as described herein) in a sample obtained from a patient,
(ii) comparing the presence and/or amount of the one or more biomarker for sepsis determined in said sample in (i) to a corresponding reference value;
(iii) comparing the presence and/or amount of the one or more biomarker for SIRS in said sample in (i) to a corresponding reference value; and thereby determining whether the patient has sepsis and/or SIRS.

All embodiments described above for the "method for diagnosing a systemic inflammatory condition", the "method for diagnosing SIRS", and the "method for diagnosing sepsis" (including the methods for diagnosing abdominal sepsis and pulmonary sepsis) apply equally to the "method for distinguishing between sepsis and SIRS in a patient". This includes all embodiments relating to the "sample", "patient", "biomarker", and "reference value", and all embodiments relating to the step for "determining the presence and/or amount of one or more biomarker in a sample" and for the "comparison" step for making a conclusion about the disease status of the patient.

As used herein, "distinguishing between sepsis and SIRS" means to determine whether a patient has or is at risk of developing sepsis and/or SIRS. For example, it may involve determining whether a patient has or is at risk of developing sepsis or SIRS. For example, it may involve determining whether a patient has or is at risk of developing sepsis and SIRS. This may involve distinguishing between a group (ie. one or more) of patients having sepsis and a group (ie. one or more) of patients having SIRS. In one embodiment, this may involve diagnosing or determining whether a patient has or is at risk of developing one or more systemic inflammatory condition selected from: sepsis and SIRS.

The systemic inflammatory conditions "sepsis" and "SIRS" are as described above for the "method for diagnosing a systemic inflammatory condition in a patient".

The "patient" for which diagnosis is performed is as described above for the "method for diagnosing a systemic inflammatory condition in a patient". In one embodiment, the patient is suspected of having or being at risk of developing a systemic inflammatory condition. In one embodiment, the patient has been diagnosed as having or being at risk of developing a systemic inflammatory condition. In one embodiment, the patient has been diagnosed as having or being at risk of developing a systemic inflammatory condition using the method described herein for diagnosis of a systemic inflammatory condition in a patient. In one embodiment, the patient is suspected of having or being at risk of developing sepsis and/or SIRS. In one embodiment, the patient is suspected of having or being at risk of developing sepsis. In one embodiment, the patient is suspected of having or being at risk of developing SIRS.

The "sample" obtained from the patient is as defined above for the "method for diagnosing a systemic inflammatory condition in a patient", the "method for diagnosing SIRS in a patient" and the "method for diagnosing sepsis in a patient", including all embodiments relating to the time point at which the sample is obtained.

The "one or more biomarker" of the invention is as defined above for the "method for diagnosing a systemic inflammatory condition in a patient".

The "one or more biomarker for sepsis" may be as defined above for the method for diagnosing sepsis in a patient, and includes any of the one or more sepsis biomarkers described herein (with or without the one or more additional biomarker) and further includes any of the combinations of sepsis biomarkers described herein.

For example, the one or more biomarker for sepsis may be selected from the group consisting of: ITGB3, ITGA2B, MYL9, LCN2, TREML1, LCN15, CMTM5, PPBP, PF4, KIF2C, MAP1A, SELP, NEXN, NLRC4, and CLEC1B. In a further example, the one or more biomarker for sepsis may be selected from the group consisting of: ITGB3, ITGA2B, MYL9, LCN2, TREML1, LCN15, CMTM5, PPBP, and PF4. In a further example, the one or more biomarker for sepsis may be selected from the group consisting of: ITGB3, ITGA2B, MYL9, LCN2, and TREML1.

Each of the biomarkers of sepsis may be used alone, or in combination with any of the sepsis biomarkers in the method of the invention. For example, any combination of 1 or more, 2 or more, 3 or more, 4 or more, 5 or more, 6 or more, 7 or more, 8 or more, 9 or more, 10 or more, 11 or more, 12 or more, 13 or more, 14 or more, up to and including all of the sepsis biomarkers may be used to distinguish between sepsis and SIRS in a patient.

For example, the method may be performed using 1 or more biomarker for sepsis selected from the group consisting of: ITGB3, ITGA2B, MYL9, LCN2, and TREML1. For example, the method may be performed using 2 or more biomarkers for sepsis selected from the group consisting of: ITGB3, ITGA2B, MYL9, LCN2, and TREML1. For example, the method may be performed using 3 or more biomarkers for sepsis selected from the group consisting of: ITGB3, ITGA2B, MYL9, LCN2, and TREML1. For example, the method may be performed using 4 or more biomarkers for sepsis selected from the group consisting of: ITGB3, ITGA2B, MYL9, LCN2, and TREML1. For example, the method may be performed using the combination of: ITGB3, ITGA2B, MYL9, LCN2, and TREML1. This combination of sepsis biomarkers was shown to be particularly effective in distinguishing sepsis from SIRS when tested by ROC analysis (see Example 2).

In one embodiment, the one or more biomarker for sepsis is LCN2. In one embodiment, the one or more biomarker for sepsis is ITGA2B. In one embodiment, the one or more biomarker for sepsis is MYL9. In one embodiment, the one or more biomarker for sepsis is ITGB3. In one embodiment, the one or more biomarker for sepsis is TREML1. In one embodiment, the one or more biomarker for sepsis is LCN15. In one embodiment, the one or more biomarker for sepsis is CMTM5. In one embodiment, the one or more biomarker for sepsis is PPBP. In one embodiment, the one or more biomarker for sepsis is PF4. In one embodiment, the one or more biomarker for sepsis is MAP1A. In one embodiment, the one or more biomarker for sepsis is SELP. In one embodiment, the one or more biomarker for sepsis is NEXN. In one embodiment, the one or more biomarker for sepsis is NLRC4. In one embodiment, the one or more biomarker for sepsis is CLEC1B.

As described above for the method for diagnosis of sepsis in a patient, one or more additional biomarker for sepsis may also be used in the distinguishing method. All embodiments described above for the one or more additional biomarker used in the method for diagnosis of sepsis in a patient apply equally to the method for distinguishing between sepsis and SIRS in a patient. For example, if the one or more biomarker is LCN15, the one or more additional biomarker may be selected from at least 1 (eg. at least 2, at least 3, at least 4, at least 5, at least 6, at least 7, at least 8, at least 9, at least 10, at least 11, up to and including all) of the biomarkers: ITGB3, ITGA2B, MYL9, LCN2, TREML1, CMTM5, PPBP, PF4, KIF2C, MAP1A, SELP, NEXN, NLRC4, and CLEC1B. In one embodiment, the one or more additional biomarker is selected from at least 1 (eg. at least 2, at least 3, at least 4, at least 5, at least 6, at least 7, up to and including all) of the biomarkers: ITGB3, ITGA2B, MYL9, LCN2, TREML1, CMTM5, PPBP, and PF4.

In a preferred embodiment, the method for distinguishing between sepsis and SIRS in a patient may comprise:
(i) determining the presence and/or amount of one or more biomarker for sepsis in a sample obtained from a patient, wherein the one or more biomarker for sepsis is selected from the group consisting of: ITGB3, ITGA2B, MYL9, LCN2, and TREML1,
(ii) comparing the presence and/or amount of the one or more biomarker for sepsis determined in said sample in (i) to a corresponding reference value (such as a reference value that is representative of a healthy individual); and thereby determining whether the patient has or is at risk of having sepsis and/or SIRS.

Preferably, the one or more biomarker for sepsis comprises the combination of: ITGB3, ITGA2B, MYL9, LCN2, and TREML1. The patient used in these methods has preferably been diagnosed as having a systemic inflammatory condition (e.g preferably using the method described for diagnosis of a systemic inflammatory condition).

The "one or more biomarker for SIRS" is as defined above for the method for diagnosing SIRS in a patient, and includes any of the one or more SIRS biomarkers described herein (with or without the one or more additional biomarker), and further includes any of the combinations of SIRS biomarkers described herein.

For example, the one or more biomarker for SIRS may be selected from the group consisting of: PLA2G7, ARHGEF10L, MYCL, TGFBI, GPR124, IL1RN, NLRP3, RBP4, and MPP3. For example, the one or more biomarker for SIRS may be selected from the group consisting of: PLA2G7, ARHGEF10L, MYCL, TGFBI, and GPR124. For example, the one or more biomarker for SIRS may be selected from the group consisting of: PLA2G7, ARHGEF10L, MYCL, and TGFBI.

Each of the biomarkers of SIRS may be used alone, or in combination with any of the SIRS biomarkers in the method of the invention. For example, any combination of 1 or more, 2 or more, 3 or more, 4 or more, 5 or more, 6 or more, 7 or more, 8 or more, up to and including all of the SIRS biomarkers may be used to distinguish between sepsis and SIRS in a patient.

For example, the distinguishing method may be performed using 1 or more biomarker for SIRS selected from the group consisting of: PLA2G7, ARHGEF10L, MYCL, and TGFBI. For example, the distinguishing method may be performed using 2 or more biomarkers for SIRS selected from the group consisting of: PLA2G7, ARHGEF10L, MYCL, and TGFBI. For example, the distinguishing method may be performed using 3 or more biomarkers for SIRS selected from the group consisting of: PLA2G7, ARHGEF10L, MYCL, and TGFBI. For example, the distinguishing method may be performed using all 4 biomarkers for SIRS selected from the group consisting of: PLA2G7, ARHGEF10L, MYCL, and TGFBI. This combination of SIRS biomarkers was shown to be particularly effective in distinguishing sepsis from SIRS when tested by ROC analysis (see Example 2).

In one embodiment, the one or more biomarker for SIRS is TGFBI. In one embodiment, the one or more biomarker for SIRS is PLA2G7. In one embodiment, the one or more biomarker for SIRS is MYCL. In one embodiment, the one or more biomarker for SIRS is ARHGEF10L. In one embodiment, the one or more biomarker for SIRS is GPR124. In one embodiment, the one or more biomarker for SIRS is IL1RN. In one embodiment, the one or more biomarker for SIRS is NLRP3. In one embodiment, the one or more biomarker for SIRS is RBP4. In one embodiment, the one or more biomarker for SIRS is MPP3.

As described above for the method for diagnosis of SIRS in a patient, one or more additional biomarker for SIRS may also be used in the distinguishing method. All embodiments described above for the one or more additional biomarker used in the method for diagnosis of SIRS in a patient apply equally to the method for distinguishing between sepsis and SIRS in a patient. For example, if the one or more biomarker is GPR124, the one or more additional biomarker may be selected from at least 1 (eg. at least 2, at least 3, at least 4, at least 5, at least 6, at least 7, up to and including all) of the biomarkers: PLA2G7, ARHGEF10L, MYCL, TGFBI, IL1RN, NLRP3, RBP4, and MPP3. In one embodiment, the one or more additional biomarker is selected from at least 1 (eg. at least 2, at least 3, up to and including all) of the biomarkers: PLA2G7, ARHGEF10L, MYCL, and TGFBI.

In a preferred embodiment, the method for distinguishing between sepsis and SIRS in a patient may comprise:
(i) determining the presence and/or amount of one or more biomarker for SIRS in a sample obtained from a patient, wherein the one or more biomarker for SIRS is selected from the group consisting of: PLA2G7, ARHGEF10L, MYCL, and TGFBI,
(ii) comparing the presence and/or amount of the one or more biomarker for SIRS determined in said sample in (i) to a corresponding reference value (such as a reference value that is representative of a healthy individual); and thereby determining whether the patient has or is at risk of having sepsis and/or SIRS.

Preferably, the one or more biomarker for SIRS comprises the combination of: PLA2G7, ARHGEF10L, MYCL, and TGFBI. The patient used in this method has preferably been diagnosed as having a systemic inflammatory condition (e.g preferably using the method described for diagnosis of a systemic inflammatory condition).

Any combination of the one or more biomarker for sepsis described herein (including the one or more additional biomarker for sepsis) may be used in conjunction with any combination of the one or more biomarker for SIRS described herein (including the one or more additional biomarker for SIRS) in the method of the invention for distinguishing between sepsis and SIRS in a patient.

For example, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, up to and including all) of the sepsis biomarkers selected from the group consisting of: ITGB3, ITGA2B, MYL9, LCN2, TREML1, LCN15, CMTM5, PPBP, PF4, KIF2C, MAP1A, SELP, NEXN, NLRC4, and CLEC1B, may be used in conjunction with any combination of 1 or more (eg. 2 or more, 3 or more, 4 or more, 5 or more, 6 or more, 7 or more, 8 or more, up to and including all) of the SIRS biomarkers selected from the group consisting of: PLA2G7, ARHGEF10L, MYCL, TGFBI, GPR124, IL1RN, NLRP3, RBP4, and MPP3, to distinguish between sepsis and SIRS in a patient according to the method described herein.

For example, any combination of 1 or more (eg. 2 or more, or all 3) of the sepsis biomarkers selected from the group consisting of: LCN15, LCN2, and NLRC4, may be used in conjunction with any combination of 1 or more (eg. 2 or more, 3 or more, 4 or more, up to and including all) of the SIRS biomarkers selected from the group consisting of: GPR124, TGFBI, PLA2G7, MYCL, and ARHGEF10L, to distinguish between sepsis and SIRS in a patient according to the method described herein.

For example, any combination of 1 or more (eg. 2 or more, 3 or more, 4 or more, 5 or more, 6 or more, 7 or more, 8 or more, up to and including all) of the sepsis biomarkers selected from the group consisting of: ITGB3, ITGA2B, MYL9, LCN2, TREML1, LCN15, CMTM5, PPBP, and PF4, may be used in conjunction with any combination of 1 or more (eg. 2 or more, 3 or more, 4 or more, up to and including all) of the SIRS biomarkers selected from the group consisting of: PLA2G7, ARHGEF10L, MYCL, TGFBI, and GPR124,, to distinguish between sepsis and SIRS in a patient according to the method described herein.

In one embodiment, any combination of 1 or more (eg. 2 or more, 3 or more, 4 or more, up to and including all) of the sepsis biomarkers selected from the group consisting of: ITGB3, ITGA2B, MYL9, LCN2, and TREML1, may be used in conjunction with any combination of 1 or more (eg. 2 or more, 3 or more, up to and including all) of the SIRS biomarkers selected from the group consisting of: PLA2G7, ARHGEF10L, MYCL, and TGFBI, to distinguish between sepsis and SIRS in a patient according to the method described herein.

In one embodiment, any combination of 1 or more (eg. 2 or more, 3 or more, 4 or more, up to and including all) of the sepsis biomarkers selected from the group consisting of: ITGB3, ITGA2B, MYL9, LCN2, and TREML1, may be used in conjunction with any combination of 1 or more (eg. 2 or more, up to and including all) of the SIRS biomarkers selected from the group consisting of: ARHGEF1 0L, MYCL, and TGFBI, to distinguish between sepsis and SIRS in a patient according to the method described herein.

In one embodiment, the combination of preferred sepsis biomarkers: ITGB3, ITGA2B, MYL9, LCN2, and TREML1, may be used in conjunction with the combiantion of preferred SIRS biomarkers: PLA2G7, ARHGEF10L, MYCL, and TGFBI, to distinguish between sepsis and SIRS in a patient according to the method described herein.

In one embodiment, the combination of preferred sepsis biomarkers: ITGB3, ITGA2B, MYL9, LCN2, and TREML1, may be used in conjunction with the combiantion of preferred SIRS biomarkers: ARHGEF10L, MYCL, and TGFBI, to distinguish between sepsis and SIRS in a patient according to the method described herein.

In a further example, the following combinations of sepsis and SIRS biomarkers may be used to distinguish sepsis and SIRS according to the method described herein: (i) LCN15 and TGFBI; (ii) LCN15 and PLA2G7; (iii) LCN15 and GPR124; (iv) LCN15 and MYCL; (v) LCN15 and ARHGEF10L; (vi) ITGA2B and TGFBI; (vii) ITGA2B and PLA2G7; (viii) ITGA2B and GPR124; (ix) ITGA2B and MYCL; (x) ITGA2B and ARHGEF10L; (xi) MYL9 and TGFBI; (xii) MYL9 and PLA2G7; (xiii) MYL9 and GPR124; (xiv) MYL9 and MYCL; (xv) MYL9 and ARHGEF10L; (xvi) CMTM5 and TGFBI; (xvii) CMTM5 and PLA2G7; (xviii) CMTM5 and GPR124; (xix) CMTM5 and MYCL; (xx) CMTM5 and ARHGEF10L; (xxi) PPBP and TGFBI; (xxii) PPBP and PLA2G7; (xxiii) PPBP and GPR124; (xxiv) PPBP and MYCL; (xxv) PPBP and ARHGEF10L; (xxvi) TREML1 and TGFBI; (xxvii) TREML1 and PLA2G7; (xxviii) TREML1 and GPR124; (xxxix) TREML1 and MYCL; (xl) TREML1 and ARHGEF10L; (xli) PF4 and TGFBI; (xlii) PF4 and PLA2G7;(xliii) PF4 and GPR124; (xliv) PF4 and MYCL; and (xlv) PF4 and ARHGEF10L; (xlvi) LCN2 and GPR124; (xlvii) LCN2 and TGFBI; (xlviii) LCN2 and PLA2G7; (xlix) LCN2 and MYCL; (I) LCN2 and ARHGEF10L; (li) ITGB3 and GPR124; (Iii) ITGB3 and TGFBI; (liii) ITGB3 and PLA2G7; (liv) ITGB3 and MYCL; and (Iv) ITGB3 and ARHGEF10L.

The one or more additional biomarker for sepsis (described herein) and/or the one or more additional biomarker SIRS (described herein) may also be used together with these combinations of biomarkers in the distinguishing method described herein.

In a preferred embodiment, the method for distinguishing between sepsis and SIRS in a patient may comprise:
(i) determining the presence and/or amount of one or more biomarker for sepsis, and one or more biomarker for SIRS in a sample obtained from a patient, wherein the one or more biomarker for sepsis is selected from the group consisting of: ITGB3, ITGA2B, MYL9, LCN2, and TREML1 and the one or more biomarker for SIRS is selected from the group consisting of: PLA2G7, ARHGEF10L, MYCL, and TGFBI,
(ii) comparing the presence and/or amount of the one or more biomarker for sepsis determined in said sample in (i) to a corresponding reference value (such as a reference value that is representative of a healthy individual);
(iii) comparing the presence and/or amount of the one or more biomarker for SIRS in said sample in (i) to a corresponding reference value (such as a reference value that is representative of a healthy individual); and thereby determining whether the patient has sepsis and/or SIRS.

Preferably, the one or more biomarker for sepsis comprises the combination of ITGB3, ITGA2B, MYL9, LCN2, and TREML1 and the one or more biomarker for SIRS comprises the combination of: PLA2G7, ARHGEF10L, MYCL, and TGFB. The patient used in this method has preferably been diagnosed as having a systemic inflammatory condition (e.g preferably using the method described for diagnosis of a systemic inflammatory condition).

All embodiments relating to the step for "determining the presence and/or amount of one or more biomarker in a sample" and for the "comparison" step for making a conclusion about the disease status of the patient as defined above for the "method for diagnosing SIRS" and the "method for diagnosing sepsis" apply equally to the "method for distinguishing between sepsis and SIRS in a patient". This includes all embodiments relating to the reference value used in these methods.

As described herein, the present inventors observed that the sepsis biomarkers increased in abundance in patients having sepsis as compared to patients having SIRS, as well as healthy individuals. Likewise, the SIRS biomarkers were observed to increase in abundance in patients having SIRS as compared to patients having sepsis, as well as healthy individuals. These differences in marker abundance can be used to determine whether an individual has or is at risk of developing sepsis and/or SIRS.

For example, by comparing the presence and/or amount of markers quantified in a sample obtained from a patient to the presence and/or amount of markers quantified for a reference value (such as a reference value that is representative of a healthy individual (or a population of healthy individuals), a reference value that is representative of an individual having sepsis (or a population of individuals having sepsis), and/or a reference value that is representative of an individual having SIRS (or a population of individuals having SIRS)), it is possible to diagnose the presence (or absence) of sepsis and/or SIRS in a patient. The method permits classification of the individual as belonging to or not belonging to the reference population (ie. by determining whether the amounts of marker quantified in the individual are statistically similar to the reference population or statistically deviate from the reference population). Hence, classification of the individual's marker profile (ie. the overall pattern of change observed for the markers quantified) as corresponding to the profile derived from a particular reference population is predictive that the individual falls (or does not fall) within the reference population.

All embodiments described above (in the context of the methods for diagnosis of sepsis) for the classification of a patient as having or being at risk of having sepsis (or not having or not being at risk of having sepsis) in the method for diagnosis of sepsis in a patient apply equally to the method for distinguishing between sepsis and SIRS in a patient. Likewise, all embodiments described above (in the context of the methods for diagnosis of SIRS) for the classification of a patient as having or being at risk of having SIRS (or not having or not being at risk of having SIRS) in the method for diagnosis of SIRS in a patient apply equally to the method for distinguishing between sepsis and SIRS in a patient. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may be as defined above for the "method of diagnosing a systemic inflammatory condition in a patient", the "method for diagnosing sepsis in a patient" and the "method for diagnosing SIRS in a patient". In one embodiment, the reference value is representative of a healthy individual (or a population of healthy individuals). In one embodiment, the reference value is representative of an individual having SIRS (or a population of individuals having SIRS). In one embodiment, the reference value is representative of an individual having sepsis (or a population of individuals having sepsis). For example, the reference value may be representative of an individual having abdominal sepsis and/or an individual having pulmonary sepsis (or a population of individuals having abdominal sepsis and/or a population of individuals having pulmonary sepsis).

As described herein, the present inventors observed that the "SIRS" biomarkers described herein each increase in abundance in samples obtained from patients having SIRS, as compared to healthy individuals. Likewise, the "sepsis" biomarkers were also observed to increase in abundance in samples obtained from patients having sepsis, as compared to healthy individuals. Detection of increased levels of the "SIRS" biomarkers in a patient as compared to the levels detected for healthy individuals can thus be used to diagnose the presence of SIRS. Whilst, detection of increased levels of the "sepsis" biomarkers in a patient as compared to the levels detected for healthy individuals can thus be used to diagnose the presence of sepsis. By combining the results from these analyses, a patient can be diagnosed as having sepsis or SIRS.

Thus, in one embodiment, when the reference value is representative of a healthy individual (or a population of healthy individuals), an increase in the one or more biomarker (and/ or one or more additional biomarker) for SIRS in the sample obtained from the patient relative to the corresponding reference value representative of a healthy individual, indicates that the patient has or is at risk of developing SIRS. Likewise, no increase or a decrease in the one or more SIRS biomarker (and/ or one or more additional biomarker) in the sample obtained from the patient relative to the corresponding reference value representative of a healthy individual, indicates that the patient does not have SIRS. Further confirmation of the diagnosis may be obtained when no increase is observed in the one or more biomarker for sepsis, in the sample obtained from the patient relative to the corresponding reference value representative of a healthy individual. This indicates that the patient has or is at risk of developing SIRS, but does not have sepsis. Furthermore, no increase or a decrease in the one or more SIRS biomarker (and/ or one or more additional biomarker) in the sample obtained from the patient relative to the corresponding reference value representative of a healthy individual, may indicate that the patient has or is at risk of having sepsis (e.g. where the patient has already been diagnosed as having a systemic inflammatory condition).

An increase in the one or more biomarker (and/ or one or more additional biomarker) for sepsis in the sample obtained from the patient relative to the corresponding reference value representative of a healthy individual, indicates that the patient has or is at risk of developing sepsis. Likewise, no increase or a decrease in the one or more sepsis biomarker (and/ or one or more additional biomarker) in the sample obtained from the patient relative to the corresponding reference value representative of a healthy individual, indicates that the patient does not have sepsis. Further confirmation of the diagnosis may be obtained when no increase is observed in the one or more biomarker for SIRS, in the sample obtained from the patient relative to the corresponding reference value representative of a healthy individual. This indicates that the patient has or is at risk of developing sepsis, but does not have SIRS. Furthermore, no increase or a decrease in the one or more sepsis biomarker (and/ or one or more additional biomarker) in the sample obtained from the patient relative to the corresponding reference value representative of a healthy individual, may indicate that the patient has or is at risk of having SIRS (e.g. where the patient has already been diagnosed as having a systemic inflammatory condition).

Furthermore, the patient may be diagnosed as having sepsis and SIRS. The patient may be diagnosed as having sepsis and SIRS when an increase is observed in the one or more biomarker for sepsis in the sample obtained from the patient relative to the corresponding reference value representative of a healthy individual; and an increase is observed in the one or more biomarker for SIRS in the sample obtained from the patient relative to the corresponding reference value representative of a healthy individual.

For some of the SIRS biomarkers identified by the present inventors, increased levels of these markers were also observed in patients having sepsis as compared to healthy individuals, although much bigger increases were observed for these biomarkers in the patients having SIRS. Similarly, for some of the sepsis biomarkers identified by the present inventors, increased levels of these markers were also observed in patients having SIRS as compared to healthy individuals, although much bigger increases were observed for these biomarkers in the patients having sepsis. The accuracy of the method for distinguishing between sepsis and SIRS in a patient can thus be improved by looking for a "minimum" fold increase or % increase in the levels of the one or more sepsis biomarker and the one or more SIRS biomarker as compared to the corresponding reference value that is representative of a healthy individual. The fold increase or % increase may be as defined above for the method for diagnosis of a systemic inflammatory condition, the method for diagnosis of sepsis and the method for diagnosis of SIRS.

In one embodiment, the minimum fold increase for the one or more sepsis biomarker (eg. LCNI5, LCN2, ITGA2B, MYL9, ITGB3, CMTM5, PPBP, TREML1, PF4, KIF2C, MAP1A, SELP, NEXN, NLRC4, and CLEC1B) is as defined above for the method for diagnosing sepsis in a patient. For example, for the biomarker LCN15, an increase of at least 1 (eg. at least 1.1, at least 1.2, at least 1.3, at least 1.4, or at least 1.5) fold in LCN15 in the sample obtained from the patient relative to the corresponding reference value, indicates that the patient has, or is at risk of developing sepsis. In one embodiment, no increase or an increase of less than 1 (eg. less than 1.1, less than 1.2, less than 1.3, less than 1.4, less than 1.5) fold in LCN15 in the sample obtained from the patient relative to the corresponding reference value, indicates that the patient does not have, or is not at risk of developing sepsis.

In one embodiment, the minimum fold increase for the one or more SIRS biomarker (eg. GPR124, TGFBI, PLA2G7, MYCL, ARHGEF10L, IL1RN, NLRP3, RBP4, and MPP3) is as defined above for the method for diagnosing SIRS in a patient. For example, for the GRP124 biomarker, an increase of at least 1.1 (eg. at least 1.2, at least 1.3, at least 1.4, at least 1.5) fold in GPR124 in the sample obtained from the patient relative to the corresponding reference value, indicates that the patient has, or is at risk of developing SIRS. In one embodiment, no increase or an increase of less than 1.1 (eg. less than 1.2, less than 1.3, less than 1.4, less than 1.5, less than 1.6) fold in GPR124 in the sample obtained from the patient relative to the corresponding reference value, indicates that the patient does not have, or is not at risk of developing SIRS.

As described herein, the present inventors observed that the levels of the one or more SIRS biomarkers were elevated in patients having SIRS as compared to patients having sepsis. Detection of increased levels of these biomarkers in a patient as compared to the levels detected for patients having sepsis can thus be used to diagnose the presence of SIRS. Thus, in one embodiment, when the reference value is representative of an individual (or population of individuals) having sepsis (such as abdominal sepsis and/or pulmonary sepsis), an increase in the one or more biomarker for SIRS (and/ or one or more additional biomarker) in the sample obtained from the patient relative to the corresponding reference value representative of an individual having sepsis, indicates that the patient has or is at risk of developing SIRS. The increase in the one or more SIRS biomarker may be as defined above for the method for diagnosing SIRS described above. Likewise, no increase in the one or more biomarker (and/ or one or more additional biomarker) in the sample obtained from the patient relative to the corresponding reference value representative of an individual having sepsis, indicates that the patient does not have SIRS.

As described herein, the present inventors observed that the levels of the one or more sepsis biomarkers were elevated in patients having sepsis as compared to patients having SIRS. Detection of increased levels of these biomarkers in a patient as compared to the levels detected for patients having SIRS can thus be used to diagnose the presence of sepsis. Thus, in one embodiment, when the reference value is representative of an individual (or population of individuals) having SIRS, an increase in the one or more sepsis biomarker (and/ or one or more additional biomarker) in the sample obtained from the patient relative to the corresponding reference value representative of an individual having SIRS, indicates that the patient has or is at risk of developing sepsis (such as abdominal sepsis and/or pulmonary sepsis). The increase in the one or more sepsis biomarker may be as defined above for the method for diagnosing sepsis described above. Likewise, no increase in the one or more biomarker (and/ or one or more additional biomarker) in the sample obtained from the patient relative to the corresponding reference value representative of an individual having SIRS, indicates that the patient does not have sepsis.

As described above for the "method for diagnosing a systemic inflammatory condition in a patient", the method of the invention may involve the use of multiple separate reference values. All combinations of reference values defined above for the "method for diagnosing a systemic inflammatory condition in a patient" apply equally to the method for distinguishing between sepsis and SIRS.

For example, the reference value used in the method may comprise: (i) a reference value that is representative of an individual (or population of individuals) having sepsis and a separate reference value that is representative of an individual (or population of individuals) having SIRS. In one embodiment, the patient may be diagnosed as having sepsis and SIRS, when an increase is observed in the one or more biomarker for sepsis in the sample obtained from the patient relative to the corresponding reference value representative of an individual having SIRS; and an increase is observed in the one or more biomarker for SIRS in the sample obtained from the patient relative to the corresponding reference value representative of an individual having sepsis.

The method for distinguishing between sepsis and SIRS in a patient as described herein can be used in a decision tree process to investigate the health of a patient having or suspected of having a systemic inflammatory condition. For example, the method for distinguishing sepsis and SIRS in a patient can be performed before, after, or in addition to any of the other methods of the invention described herein.

In one embodiment, the method for distinguishing sepsis and SIRS in a patient is performed as described herein. If the patient tests positive for sepsis, the patient may be further tested for sepsis, abdominal sepsis and/or pulmonary sepsis using the diagnostic methods described herein, so as to confirm whether the patient has or is at risk of developing sepsis, and/or determine whether the patient has or is at risk of developing abdominal sepsis and/or pulmonary sepsis. If the patient tests positive for SIRS, the patient may be further tested for SIRS using the diagnostic method described herein, so as to confirm whether the patient has or is at risk of developing SIRS.

In one embodiment, the method for distinguishing sepsis and SIRS in a patient (as described herein) can be performed subsequent to (or in addition to) the method for diagnosing whether a patient has a systemic inflammatory condition (as described herein). For example, if the patient tests positive for a systemic inflammatory condition (using the method for diagnosing whether a patient has a systemic inflammatory condition), they may be tested using the distinguishing method described herein to determine whether they have sepsis and/or SIRS. In one embodiment, the above combination of methods are performed as described, and if the patient tests positive for sepsis, the patient may be further tested for sepsis, abdominal sepsis and/or pulmonary sepsis using the diagnostic methods described herein, so as to confirm whether the patient has or is at risk of developing sepsis, and/or to determine whether the patient has or is at risk of developing abdominal sepsis and/or pulmonary sepsis. In one embodiment, the above combination of methods are performed as described, and if the patient tests positive for SIRS, the patient may be further tested for SIRS using the diagnostic method described herein, so as to confirm whether the patient has or is at risk of developing SIRS.

The above described combination of methods may also be performed in parallel to determine the disease status of a patient by simultaneously (or substantially simultaneously) investigating the expression of all the biomarkers in a sample obtained from the patient, and determining whether the patient has or is at risk of having a systemic inflammatory condition, sepsis (such as abdominal or pulmonary sepsis) and/or SIRS.

When performing these different methods in a decision tree process, the sample used in each step of the method may be the same sample obtained from the patient (as described herein). When the method comprises multiple quantification steps, these multiple steps may be performed at the same time (e.g. in parallel) and/or using the same sample. When the method comprises multiple comparison steps, these multiple steps may be performed at the same time (e.g. in parallel).

For example, the method for distinguishing between sepsis and SIRS in a patient may comprise:
(a) diagnosing a patient as having a systemic inflammatory condition by performing a method comprising:
   (i) determining the presence and/or amount of one or more inflammatory biomarker in a sample obtained from the patient, wherein the one or more inflammatory biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1;
   (ii) comparing the presence and/or amount of the one or more inflammatory biomarker determined in said sample in (i) to a corresponding reference value (such as a value that is representative of a healthy individual); and thereby determining that the patient has a systemic inflammatory condition;
(b) determining whether the patient diagnosed as having a systemic inflammatory condition has sepsis and/or SIRS by performing a method comprising:
   (i) determining the presence and/or amount of one or more biomarker for sepsis, and/or one or more biomarker for SIRS in a sample obtained from a patient, wherein the one or more biomarker for sepsis is selected from the group consisting of: ITGB3, ITGA2B, MYL9, LCN2, TREML1, LCN15, CMTM5, PPBP, and PF4; and the one or more biomarker for SIRS is selected from the group consisting of: PLA2G7, ARHGEF10L, MYCL, TGFBI, and GPR124;
   (ii) comparing the presence and/or amount of the one or more biomarker for sepsis determined in said sample in (i) to a corresponding reference value (such as a value that is representative of a healthy individual) and/or comparing the presence and/or amount of the one or more biomarker for SIRS in said sample in (i) to a corresponding reference value (such as a value that is representative of a healthy individual); and thereby determining whether the patient has sepsis and/or SIRS.

In a further example, a method may be performed to distinguish between sepsis and SIRS in a patient, comprising;
(a) diagnosing a patient as having a systemic inflammatory condition by performing a method comprising:
   (i) determining the presence and/or amount of one or more inflammatory biomarker in a sample obtained from the patient, wherein the one or more inflammatory biomarker is selected from the group consisting of: FAM20A, OLAH, and CD177;
   (ii) comparing the presence and/or amount of the one or more inflammatory biomarker determined in said sample in (i) to a corresponding reference value (such as a value that is representative of a healthy individual); and thereby determining that the patient has a systemic inflammatory condition;
(b) determining whether the patient diagnosed as having a systemic inflammatory condition has sepsis and/or SIRS by performing a method comprising:
   (i) determining the presence and/or amount of one or more biomarker for sepsis and/or one or more biomarker for SIRS in a sample obtained from a patient, wherein the one or biomarker for sepsis is selected from the group consisting of: ITGA2B, ITGB3, MYL9, LCN2, and TREML1, and the one or more biomarker for SIRS is selected from the group consisting of: PLA2G7, ARHGEF10L, MYCL, and TGFBI;
   (ii) comparing the presence and/or amount of the one or more biomarker for sepsis determined in said sample in (i) to a corresponding reference value (such as a value that is representative of a healthy individual) and/or comparing the presence and/or amount of the one or more biomarker for SIRS in said sample in (i) to a corresponding reference value (such as a value that is representative of a healthy individual); and thereby determining whether the patient has sepsis and/or SIRS.

In a related aspect, the present invention also provides the use of one or more biomarker for sepsis (as described herein), and/or one or more biomarker for SIRS (as described herein) for distinguishing between sepsis and SIRS in a patient.

In one embodiment, the invention provides the use of one or more biomarker for sepsis selected from the group consisting of: ITGA2B, ITGB3, MYL9, LCN2, TREML1, LCN15, CMTM5, PPBP, PF4, KIF2C, MAP1A, SELP, NEXN, NLRC4, and CLEC1B, and/or one or more biomarker for SIRS selected from the group consisting of: PLA2G7, ARHGEF10L, MYCL, TGFBI, GPR124, IL1RN, NLRP3, RBP4, and MPP3, for distinguishing between sepsis and SIRS in a patient. In one embodiment, the invention provides the use of one or more biomarker for sepsis selected from the group consisting of: ITGA2B, ITGB3, MYL9, LCN2, TREML1, LCN15, CMTM5, PPBP, and PF4, and/or one or more biomarker for SIRS selected from the group consisting of: PLA2G7, ARHGEF10L, MYCL, TGFBI, and GPR124, for distinguishing between sepsis and SIRS in a patient. In one embodiment, the invention provides the use of one or more biomarker for sepsis selected from the group consisting of: ITGA2B, ITGB3, MYL9, LCN2, and TREML1, and/or one or more biomarker for SIRS selected from the group consisting of: PLA2G7, ARHGEF10L, MYCL and TGFBI, for distinguishing between sepsis and SIRS in a patient. In one embodiment, the invention provides the use of the sepsis biomarkers: ITGA2B, ITGB3, MYL9, LCN2, and TREML1, and/or the SIRS biomarkers PLA2G7, ARHGEF10L, MYCL, and TGFBI, for distinguishing between sepsis and SIRS in a patient. In one embodiment, the invention provides the use of the sepsis biomarkers: ITGA2B, ITGB3, MYL9, LCN2, and TREML1, and/or the SIRS biomarkers ARHGEF10L, MYCL, and TGFBI, for distinguishing between sepsis and SIRS in a patient.

All embodiments described above for the method of distinguishing between sepsis and SIRS in a patient apply equally to the 'use' of the invention described herein. This includes all embodiments relating to the terms "sepsis", "SIRS", "patient", "sample", "the one or more biomarker for sepsis", and "the one or more biomarker for SIRS" (including all combinations of sepsis and SIRS biomarkers described above).

At present, there is no clinical test available for distinguishing between abdominal sepsis and pulmonary sepsis. Rapid diagnosis of the physiological origin of sepsis in a patient would however be useful for selecting the most appropriate treatment for patients having sepsis. The present inventors have identified a set of biomarkers that is predictive of abdominal sepsis and a separate set of biomarkers that is predictive of pulmonary sepsis in patients. Using these distinct sets of biomarkers, the present inventors have developed a rapid and sensitive way to distinguish between abdominal sepsis and pulmonary sepsis in a patient by simultaneously quantifying one or more biomarker for abdominal sepsis and/or one or more biomarker for pulmonary sepsis in a sample obtained from a patient, so as to determine whether the patient has a biomarker profile that is predictive of abdominal or pulmonary sepsis.

The present invention therefore provides a method for distinguishing between abdominal sepsis and pulmonary sepsis in a patient, comprising:
(i) determining the presence and/or amount of one or more biomarker for abdominal sepsis (as described herein), and/or one or more biomarker for pulmonary sepsis (as described herein) in a sample obtained from a patient,
(ii) comparing the presence and/or amount of the one or more biomarker for abdominal sepsis and/or the one or more for pulmonary sepsis determined in said sample in (i) to a corresponding reference value;
and thereby determining whether the patient has abdominal sepsis and/or pulmonary sepsis.

As illustrated in Example 2, the distinguishing method of the invention can be performed using only one or more of the abdominal sepsis biomarker described herein, or only one or more of the pulmonary sepsis biomarkers described herein. These biomarkers can be used on their own to distinguish abdominal and pulmonary sepsis because their expression correlates with the patient's disease condition (i.e. the presence and/or amount of these biomarkers depends on whether a patient has abdominal sepsis or pulmonary sepsis or is healthy). Determining the presence and/or amount of either of these biomarkers and comparing this to a corresponding reference value (such as a reference value that is representative of a healthy individual, an abdominal sepsis patient and/or a pulmonary sepsis patient) therefore allows the disease status of the patient to be determined.

In one embodiment, the present invention therefore provides a method for distinguishing between abdominal sepsis and pulmonary sepsis in a patient, comprising:
(i) determining the presence and/or amount of one or more biomarker for abdominal sepsis (as described herein) in a sample obtained from a patient,
(ii) comparing the presence and/or amount of the one or more biomarker for abdominal sepsis determined in said sample in (i) to a corresponding reference value; and thereby determining whether the patient has abdominal sepsis and/or pulmonary sepsis.

In one embodiment, the present invention therefore provides a method for distinguishing between abdominal sepsis and pulmonary sepsis in a patient, comprising:
(i) determining the presence and/or amount of one or more biomarker for pulmonary sepsis (as described herein) in a sample obtained from a patient,
(ii) comparing the presence and/or amount of the one or more biomarker for pulmonary sepsis determined in said sample in (i) to a corresponding reference value; and thereby determining whether the patient has abdominal sepsis and/or pulmonary sepsis.

Alternatively, the abdominal and pulmonary sepsis biomarkers can be used in combination to distinguish between abdominal sepsis and pulmonary sepsis.

In one embodiment, the present invention provides a method for distinguishing between abdominal sepsis and pulmonary sepsis in a patient, comprising:
(i) determining the presence and/or amount of one or more biomarker for abdominal sepsis (as described herein), and one or more biomarker for pulmonary sepsis (as described herein) in a sample obtained from a patient,
(ii) comparing the presence and/or amount of the one or more biomarker for abdominal sepsis determined in said sample in (i) to a corresponding reference value;
(iii) comparing the presence and/or amount of the one or more biomarker for pulmonary sepsis in said sample in (i) to a corresponding reference value; and thereby determining whether the patient has abdominal sepsis and/or pulmonary sepsis.

All embodiments described above for the "method for diagnosing a systemic inflammatory condition in a patient", the "method for diagnosing abdominal sepsis" and the "method for diagnosing pulmonary sepsis" apply equally to the "method for distinguishing between abdominal sepsis and pulmonary sepsis in a patient". This includes all embodiments relating to the "sample", "patient", "biomarker", and "reference value", and all embodiments relating to the step for "determining the presence and/or amount of one or more biomarker in a sample" and for the "comparison" step for making a conclusion as to the disease status of the patient.

As used herein, "distinguishing between abdominal sepsis and pulmonary sepsis" means to determine whether a patient has or is at risk of developing abdominal sepsis and/or pulmonary sepsis. For example, it may involve determining whether a patient has or is at risk of developing abdominal sepsis or pulmonary sepsis. For example, it may involve determining whether a patient has or is at risk of developing abdominal sepsis and pulmonary sepsis. This may involve distinguishing between a group (ie. one or more) of patients having abdominal sepsis and a group (ie. one or more) of patients having pulmonary sepsis. In one embodiment, this may involve diagnosing or determining whether a patient has or is at risk of developing one or more systemic inflammatory condition selected from: abdominal sepsis and pulmonary sepsis.

The systemic inflammatory conditions "abdominal sepsis" and "pulmonary sepsis" are as described above for the "method for diagnosing a systemic inflammatory condition in a patient".

The "patient" for which diagnosis is performed is as described above for the "method for diagnosing a systemic inflammatory condition in a patient". In one embodiment, the patient is suspected of having or being at risk of developing a systemic inflammatory condition. In one embodiment, the patient has been diagnosed as having a systemic inflammatory condition. In one embodiment, the patient has been diagnosed as having a systemic inflammatory condition using the method described herein. In one embodiment, the patient is suspected of having or being at risk of developing sepsis. In one embodiment, the patient has been diagnosed as having sepsis (eg. using the methods described herein for diagnosis of sepsis, or for distinguishing between sepsis and SIRS). In one embodiment, the patient is suspected of having or being at risk of developing abdominal sepsis and/or pulmonary sepsis. In one embodiment, the patient is suspected of having or being at risk of developing abdominal sepsis. In one embodiment, the patient is suspected of having or being at risk of developing pulmonary sepsis.

The "sample" obtained from the patient is as described above for the "method for diagnosing a systemic inflammatory condition in a patient", the "method for diagnosing abdominal sepsis" and the "method for diagnosing pulmonary sepsis", including all embodiments relating to the time point at which the sample is obtained.

The "one or more biomarker" of the invention is as described above for the "method for diagnosing a systemic inflammatory condition in a patient". In one embodiment, the "one or more biomarker" is a nucleic acid, as defined herein. In one embodiment, the "one or more biomarker" is a protein, as defined herein.

The "one or more biomarker for abdominal sepsis" is as described above for the method for diagnosis of abdominal sepsis in a patient, and includes any of the one or more abdominal sepsis biomarkers described herein (with or without the one or more additional biomarker) and further includes any of the combinations of abdominal sepsis biomarkers described herein.

For example, the one or more biomarker for abdominal sepsis may be selected from the group consisting of: SLC39A8, CIQC, CIQA, MRAS, TMEM37, CIQB,PCOLCE2, KIF2C, TNF, IFI44, IFIT1, and RPGRIP1. For example, the one or more biomarker for abdominal sepsis may be selected from the group consisting of: SLC39A8, CIQC, CIQA, MRAS, TMEM37, CIQB,PCOLCE2, KIF2C, and TNF. For example, the one or more biomarker for abdominal sepsis may be selected from the group consisting of: SLC39A8, CIQC, CIQA, MRAS, TMEM37, and CIQB. For example, the one or more biomarker for abdominal sepsis may be selected from the group consisting of: SLC39A8, CIQC, and CIQA.

Each of the biomarkers of abdominal sepsis may be used alone, or in combination with any of the abdominal sepsis biomarkers in the method of the invention. For example, any combination of 1 or more, 2 or more, 3 or more, 4 or more, 5 or more, 6 or more, 7 or more, 8 or more, 9 or more, 10 or more, 11 or more, or up to and including all of the abdominal sepsis biomarkers may be used to diagnose abdominal sepsis in a patient according to the method of the invention.

In one embodiment, any combination of 1 or more (eg. 2 or more, 3 or more, 4 or more, 5 or more, or all 6) of the biomarkers selected from the group consisting of: SLC39A8, CIQC, CIQA, MRAS, TMEM37, and CIQB, may be used as a biomarker for abdominal sepsis in the distinguishing method. For example, the combination of SLC39A8, CIQC, CIQA, MRAS, TMEM37, and CIQB may be used. In one embodiment, any combination of 1 or more (eg. 2 or more, or all 3) of the biomarkers selected from the group consisting of: SLC39A8, CIQC, and CIQA may be used as a biomarker for abdominal sepsis in the distinguishing method. For example, the combination of SLC39A8, CIQC, and CIQA may be used.

As described above for the method for diagnosis of abdominal sepsis in a patient, one or more additional biomarker for abdominal sepsis may also be used in the distinguishing method for determining the presence (or absence) of abdominal sepsis in a patient. All embodiments described above for the one or more additional biomarker used in the method for diagnosis of abdominal sepsis in a patient apply equally to the method for distinguishing between abdominal sepsis and pulmonary sepsis in a patient.

In a preferred embodiment, the method for distinguishing between abdominal sepsis and pulmonary sepsis in a patient, may comprise:
(i) determining the presence and/or amount of one or more biomarker for abdominal sepsis in a sample obtained from a patient, wherein the one or more biomarker for abdominal sepsis is selected from the group consisting of: SLC39A8, CIQC, CIQA, TMEM37, and CIQB;
(ii) comparing the presence and/or amount of the one or more biomarker for abdominal sepsis determined in said sample in (i) to a corresponding reference value; and thereby determining whether the patient has abdominal sepsis and/or pulmonary sepsis.

The "one or more biomarker for pulmonary sepsis" is as described above for the method for diagnosis of pulmonary sepsis in a patient, and includes any of the one or more pulmonary sepsis biomarkers described herein (with or without the one or more additional biomarker), and further includes any of the combinations of pulmonary sepsis biomarkers described herein.

For example, the one or more biomarker for pulmonary sepsis may be selected from the group consisting of: HCAR2, CXCR1, DISC1, EPSTI1, and IFI44. For example, the one or more biomarker for pulmonary sepsis may be selected from the group consisting of: HCAR2, CXCR1, and DISC1. For example, the one or more biomarker for pulmonary sepsis may be selected from the group consisting of: HCAR2 and CXCR1.

Each of the biomarkers of pulmonary sepsis may be used alone, or in combination with any of the pulmonary sepsis biomarkers in the method of the invention. For example, any combination of 1 or more, 2 or more, 3 or more, 4 or more, or up to and including all of the pulmonary sepsis biomarkers may be used to diagnose pulmonary sepsis in a patient according to the method of the invention.

In one embodiment, any combination of 1 or more (eg. 2 or more, or all 3) of the biomarkers selected from the group consisting of: HCAR2, CXCR1, and DISC1, may be used as a biomarker for pulmonary sepsis in the distinguishing method. For example, the combination of HCAR2, CXCR1, and DISC1 may be used. In one embodiment, HCAR2 and/or CXCR1 may be used as a biomarker for pulmonary sepsis in the distinguishing method.

As described above for the method for diagnosis of pulmonary sepsis in a patient, one or more additional biomarker for pulmonary sepsis may also be used in the distinguishing method for determining the presence (or absence) of pulmonary sepsis in a patient. All embodiments described above for the one or more additional biomarker used in the method for diagnosis of pulmonary sepsis in a patient apply equally to the method for distinguishing between abdominal sepsis and pulmonary sepsis in a patient.

In a preferred embodiment, the method for distinguishing between abdominal sepsis and pulmonary sepsis in a patient, may comprise:
(i) determining the presence and/or amount of one or more biomarker for pulmonary sepsis in a sample obtained from a patient, wherein the one or more biomarker for pulmonary sepsis is selected from the group consisting of: HCAR2, CXCR1, and DISC1,
(ii) comparing the presence and/or amount of the one or more biomarker for pulmonary sepsis determined in said sample in (i) to a corresponding reference value; and thereby determining whether the patient has abdominal sepsis and/or pulmonary sepsis.

As illustrated in Example 2, effective results for distinguishing between abdominal and pulmonary sepsis can be achieved by using the abdominal sepsis in conjunction with the pulmonary sepsis biomarkers. Any combination of the one or more biomarker for abdominal sepsis described herein (including the one or more additional biomarker for abdominal sepsis) may be used in conjunction with any combination of the one or more biomarker for pulmonary sepsis described herein (including the one or more additional biomarker for pulmonary sepsis) in the method for distinguishing between abdominal sepsis and pulmonary sepsis in a patient.

In one embodiment, any combination of 1 or more (eg. 2 or more, 3 or more, 4 or more, 5 or more, 6 or more, 7 or more, 8 or more, 9 or more, 10 or more, 11 or more up to and including all) of the abdominal sepsis biomarkers selected from the group consisting of: SLC39A8, CIQC, CIQA, MRAS, TMEM37, CIQB, PCOLCE2, KIF2C, TNF, IFI44, IFIT1, and RPGRIP1, may be used in conjunction with any combination of 1 or more (eg. 2 or more, 3 or more, 4 or more, up to and including all) of the pulmonary sepsis biomarkers selected from the group consisting of: HCAR2, CXCR1, DISC1, EPSTI1, and IFI44, to distinguish between sepsis and SIRS in a patient according to the method described herein.

In one embodiment, any combination of 1 or more (eg. 2 or more, 3 or more, 4 or more, 5 or more, 6 or more, 7 or more, 8 or more, 9 or more, 10 or more, 11 or more up to and including all) of the abdominal sepsis biomarkers selected from the group consisting of: SLC39A8, CIQC, CIQA, MRAS, TMEM37, CIQB, PCOLCE2, and KIF2C, may be used in conjunction with any combination of 1 or more (eg. 2 or more, 3 or more, 4 or more, up to and including all) of the pulmonary sepsis biomarkers selected from the group consisting of: HCAR2, CXCR1, DISC1, EPSTI1, and IFI44, to distinguish between sepsis and SIRS in a patient according to the method described herein.

In one embodiment, any combination of 1 or more (eg. 2 or more, 3 or more, 4 or more, 5 or more, up to and including all) of the abdominal sepsis biomarkers selected from the group consisting of: SLC39A8, CIQC, CIQA, MRAS, TMEM37, and CIQB, may be used in conjunction with any combination of 1 or more (eg. 2 or more, up to and including all) of the pulmonary sepsis biomarkers selected from the group consisting of: HCAR2, CXCR1, and DISC1, to distinguish between sepsis and SIRS in a patient according to the method described herein. For example, the combination of abdominal sepsis biomarkers SLC39A8, CIQC, CIQA, MRAS, TMEM37, and CIQB, may be used in conjunction with the combination of pulmonary sepsis biomarkers HCAR2, CXCR1, and DISC1, to distinguish between sepsis and SIRS in a patient according to the method described herein.

In one embodiment, any combination of 1 or more (eg. 2 or more, up to and including all) of the abdominal sepsis biomarkers selected from the group consisting of: SLC39A8, CIQC, and CIQA, may be used in conjunction with the pulmonary sepsis biomarkers HCAR2 and/or CXCR1, to distinguish between sepsis and SIRS in a patient according to the method described herein. For example, the combination of the abdominal sepsis biomarkers SLC39A8, CIQC, and CIQA may be used in conjunction with the combination of the pulmonary sepsis biomarkers HCAR2, CXCR1, and DISC1, to distinguish between sepsis and SIRS in a patient according to the method described herein. For example, the combination of abdominal sepsis biomarkers SLC39A8, CIQC, and CIQA may be used in conjunction with the combination of pulmonary sepsis biomarkers HCAR2 and CXCR1 to distinguish between sepsis and SIRS in a patient according to the method described herein.

For example, the following combinations of abdominal sepsis and pulmonary sepsis biomarkers may be used to distinguish between abdominal sepsis and pulmonary sepsis according to the method described herein: (i) MRAS and EPSTI1; (ii) MRAS and DISC1; (iii) MRAS and CXCR1; (iv) MRAS and HCAR2; (v) MRAS and IFI44; (vi) PCOLCE2 and EPSTI1; (vii) PCOLCE2 and DISC1; (viii) PCOLCE2 and CXCR1; (ix) PCOLCE2 and HCAR2; (x) PCOLCE2 and IFI44; (xi) (xii) TMEM37 and EPSTI1; (xiv) TMEM37 and DISC1; (xv) TMEM37 and CXCR1; (xvi) TMEM37 and HCAR2; (xvii) TMEM37 and IFI44; (xviii) SLC39A8 and EPSTI1; (xix) SLC39A8 and DISC1; (xx) SLC39A8 and CXCR1; (xxi) SLC39A8 and HCAR2; (xxii) SLC39A8 and IFI44; (xxiii) KIF2C and EPSTI1; (xxiv) KIF2C and DISC1; (xxv) KIF2C and CXCR1; (xxvi) KIF2C and HCAR2; (xxvii) KIF2C and IFI44; (xxviii) CIQC and EPSTI1; (xxxix) CIQC and DISC1; (xl) CIQC and CXCR1; (xli) CIQC and HCAR2; (xlii) CIQC and IFI44; (xliii) CIQB and EPST11; (xliv) CIQB and DISC1; (xlivi) CIQB and CXCR1; (xlivii) CIQB and HCAR2; (xliviii) CIQB and IFI44; (xlix) CIQA and EPSTI1; (I) CIQA and DISC1; (li) CIQA and CXCR1; (Iii) CIQA and HCAR2; (liii) CIQA and IFI44; (liv) TNF and EPSTI1; (Iv) TNF and DISC1; (Ivi) TNF and CXCR1; (Ivii) TNF and HCAR2; (Iviii) TNF and IFI44.

In one embodiment, the biomarker IFI44 may be used to distinguish between abdominal sepsis and pulmonary sepsis in a patient (such as patient that has been diagnosed as having sepsis).

The one or more additional biomarker for abdominal sepsis (described herein) and/or the one or more additional biomarker pulmonary sepsis (described herein) may also be used together with these combinations of biomarkers in the distinguishing method described herein.

In a preferred embodiment, the method for distinguishing between abdominal sepsis and pulmonary sepsis in a patient, may comprise:
(i) determining the presence and/or amount of one or more biomarker for abdominal sepsis, and one or more biomarker for pulmonary sepsis in a sample obtained from a patient, wherein the one or more biomarker for abdominal sepsis is selected from the group consisting of: SLC39A8, CIQC, CIQA, TMEM37, and CIQB, and the one or more biomarker for pulmonary sepsis is selected from the group consisting of: HCAR2, CXCR1, and DISC1,
(ii) comparing the presence and/or amount of the one or more biomarker for abdominal sepsis determined in said sample in (i) to a corresponding reference value;
(iii) comparing the presence and/or amount of the one or more biomarker for pulmonary sepsis in said sample in (i) to a corresponding reference value; and thereby determining whether the patient has abdominal sepsis and/or pulmonary sepsis.

All embodiments relating to the step for "determining the presence and/or amount of one or more biomarker in a sample" and for the "comparison" step as described above for the "method for diagnosing abdominal sepsis" and the "method for diagnosing pulmonary sepsis" apply equally to the "method for distinguishing between abdominal sepsis and pulmonary sepsis in a patient". This includes all embodiments relating to the reference value used in these methods.

As described herein, the present inventors observed that some of the "abdominal sepsis" biomarkers (MRAS, PCOLCE2, TMEM37, SLC39A8, KIF2C, CIQC, CIQB, CIQA, TNF) described herein each increase in abundance in samples obtained from patients having abdominal sepsis, as compared to patients having pulmonary sepsis and/or healthy individuals, whilst others (IFI44, IFIT1, and RPGRIP1) decreased in abundance in samples obtained from patients having abdominal sepsis, as compared to patients having pulmonary sepsis and/or healthy individuals. Likewise, the "pulmonary sepsis" biomarkers described herein were also observed to increase in abundance in samples obtained from patients having pulmonary sepsis, as compared to patients having abdominal sepsis and/or healthy individuals. These differences in marker abundance can be used to determine whether an individual has or is at risk of developing abdominal sepsis and/or pulmonary sepsis.

For example, by comparing the amount of markers quantified in a sample obtained from a patient to the amount of markers quantified for a reference value (such as a reference value that is representative of a healthy individual (or a population of healthy individuals), a reference value that is representative of an individual having abdominal sepsis (or a population of individuals having abdominal sepsis), a reference value that is representative of an individual having pulmonary sepsis (or a population of individuals having pulmonary sepsis), and/or a reference value that is representative of an individual having SIRS (or a population of individuals having SIRS)), it is possible to diagnose the presence (or absence) of abdominal sepsis and/or pulmonary sepsis in a patient. The method permits classification of the individual as belonging to or not belonging to the reference population (ie. by determining whether the amounts of marker quantified in the individual are statistically similar to the reference population or statistically deviate from the reference population). Hence, classification of the individual's marker profile (ie. the overall pattern of change observed for the markers quantified) as corresponding to the profile derived from a particular reference population is predictive that the individual falls (or does not fall) within the reference population.

All embodiments described above (in the context of the method for diagnosis of abdominal sepsis) for the classification of a patient as having or being at risk of having (or not having or not being at risk of having) abdominal sepsis in the context of the method for diagnosis of abdominal sepsis apply equally to the method for distinguishing between abdominal sepsis and pulmonary sepsis. All embodiments described above (in the context of the method for diagnosis of pulmonary sepsis) for the classification as a patient as having or being at risk of having (or not having or not being at risk of having) pulmonary sepsis in the context of the method for diagnosis of pulmonary sepsis apply equally to the method for distinguishing between abdominal sepsis and pulmonary sepsis. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may be as defined above for the "method of diagnosing a systemic inflammatory condition in a patient", the "method for diagnosing abdominal sepsis", and the "method for diagnosing pulmonary sepsis". In one embodiment, the reference value is representative of a healthy individual (or a population of healthy individuals). In one embodiment, the reference value is representative of an individual having SIRS (or a population of individuals having SIRS). In one embodiment, the reference value is representative of an individual having abdominal sepsis (or a population of individuals having abdominal sepsis). In one embodiment, the reference value is representative of an individual having pulmonary sepsis (or a population of individuals having pulmonary sepsis).

As described herein, the present inventors observed that the "abdominal sepsis" biomarkers SLC39A8, CIQC, CIQA, MRAS, TMEM37, CIQB, PCOLCE2, KIF2C, TNF each increase in abundance in samples obtained from patients having abdominal sepsis, as compared to healthy individuals. Thus, in one embodiment, when the reference value is representative of a healthy individual (or a population of healthy individuals), an increase in the one or more biomarker (and/ or one or more additional biomarker) for abdominal sepsis in the sample obtained from the patient relative to the corresponding reference value representative of a healthy individual, indicates that the patient has or is at risk of developing abdominal sepsis. Likewise, no increase in the one or more abdominal sepsis biomarker (and/ or one or more additional biomarker) in the sample obtained from the patient relative to the corresponding reference value representative of a healthy individual, indicates that the patient does not have abdominal sepsis.

The inventors also observed a decrease in the abdominal sepsis biomarkers IFI44, IFIT1, and RPGRIP1 in samples obtained from patients having abdominal sepsis, as compared to healthy individuals. Thus, in one embodiment, when the reference value is representative of a healthy individual (or a population of healthy individuals), a decrease in the one or more biomarker (and/ or one or more additional biomarker) for abdominal sepsis in the sample obtained from the patient relative to the corresponding reference value representative of a healthy individual, indicates that the patient has or is at risk of developing abdominal sepsis. Likewise, no decrease in the one or more abdominal sepsis biomarker (and/ or one or more additional biomarker) in the sample obtained from the patient relative to the corresponding reference value representative of a healthy individual, indicates that the patient does not have abdominal sepsis.

Further confirmation of the diagnosis may be obtained when no increase is observed in the one or more biomarker for pulmonary sepsis, in the sample obtained from the patient relative to the corresponding reference value representative of a healthy individual. This indicates that the patient has or is at risk of developing abdominal sepsis, but does not have pulmonary sepsis.

As described above, the inventors observed an increase in the "pulmonary sepsis" biomarkers (HCAR2, CXCR1, DISC1, EPSTI1, and IFI44) in samples obtained from patients having pulmonary sepsis, as compared to healthy individuals. Thus, in one embodiment, when the reference value is representative of a healthy individual (or a population of healthy individuals), an increase in the one or more biomarker (and/ or one or more additional biomarker) for pulmonary sepsis in the sample obtained from the patient relative to the corresponding reference value representative of a healthy individual, indicates that the patient has or is at risk of developing pulmonary sepsis. Likewise, no increase in the one or more pulmonary sepsis biomarker (and/ or one or more additional biomarker) in the sample obtained from the patient relative to the corresponding reference value representative of a healthy individual, indicates that the patient does not have pulmonary sepsis.

Further confirmation of the diagnosis may be obtained when no increase (eg. in any one or more of MRAS, PCOLCE2, TMEM37, SLC39A8, KIF2C, CIQC, CIQB, CIQA, TNF) and/or no decrease (eg. in any one or more of IFI44, IFIT1, and RPGRIP1) is observed in the one or more biomarker for abdominal sepsis, in the sample obtained from the patient relative to the corresponding reference value representative of a healthy individual. This indicates that the patient has or is at risk of developing pulmonary sepsis, but does not have abdominal sepsis.

Furthermore, the patient may be diagnosed as having abdominal sepsis and pulmonary sepsis when an increase is observed in any one or more of: SLC39A8, CIQC, CIQA, MRAS, TMEM37, CIQB,PCOLCE2, KIF2C, and TNF, and/or a decrease is observed in any one or more of: IFI44, IFIT1, and RPGRIP1, in the sample obtained from the patient relative to the corresponding reference value representative of a healthy individual; and an increase is observed in the one or more biomarker for pulmonary sepsis in the sample obtained from the patient relative to the corresponding reference value representative of a healthy individual.

The accuracy of the method for distinguishing between abdominal sepsis and pulmonary sepsis in a patient can be improved by looking for a "minimum" fold change in the levels of the one or more abdominal sepsis biomarker and the one or more pulmonary sepsis biomarker as compared to the corresponding reference value that is representative of a healthy individual. In one embodiment, the minimum fold change or % change for the abdominal sepsis biomarkers is as defined above for the method for diagnosing abdominal sepsis in a patient. In one embodiment, the minimum fold change increase or % increase for the pulmonary sepsis biomarkers is as defined above for the method for diagnosing pulmonary sepsis in a patient.

In one embodiment, the reference value used in the distinguishing method may include a reference value that is representative of an individual having pulmonary sepsis. All embodiments described above for the method of diagnosing abdominal sepsis when using a reference value that is representative of an individual having pulmonary sepsis apply equally to the distinguishing method described herein.

For example, an increase or decrease in the one or more biomarker for abdominal sepsis (and/ or one or more additional biomarker) in the sample obtained from the patient relative to the corresponding reference value representative of an individual having pulmonary sepsis, indicates that the patient has or is at risk of developing abdominal sepsis. The increase or decrease may be as defined above for the method for diagnosing abdominal sepsis in a patient. No increase in the one or more biomarker for abdominal sepsis (and/ or one or more additional biomarker) in the sample obtained from the patient relative to the corresponding reference value representative of an individual having pulmonary sepsis, indicates that the patient may have or is at risk of developing pulmonary sepsis (e.g where the patient has already been diagnosed as having sepsis).

In one embodiment, the reference value used in the distinguishing method may include a reference value that is representative of an individual having abdominal sepsis. All embodiments described above for the method of diagnosing pulmonary sepsis when using a reference value that is representative of an individual having abdominal sepsis apply equally to the distinguishing method described herein.

For example, an increase in the one or more biomarker for pulmonary sepsis (and/ or one or more additional biomarker) in the sample obtained from the patient relative to the corresponding reference value representative of an individual having abdominal sepsis, indicates that the patient has or is at risk of developing pulmonary sepsis. The increase may be as defined above for the method for diagnosing pulmonary sepsis in a patient. No increase in the one or more biomarker for pulmonary sepsis (and/ or one or more additional biomarker) in the sample obtained from the patient relative to the corresponding reference value representative of an individual having abdominal sepsis, indicates that the patient may have or is at risk of developing abdominal sepsis (e.g where the patient has already been diagnosed as having sepsis).

As described above for the "method for diagnosing a systemic inflammatory condition in a patient", the method of the invention may involve the use of multiple separate reference values. All combinations of reference values defined above for the "method for diagnosing a systemic inflammatory condition in a patient" apply equally to the method for distinguishing between abdominal sepsis and pulmonary sepsis. For example, the reference value used in the method may comprise: (i) a reference value that is representative of an individual (or population of individuals) having abdominal sepsis and a separate reference value that is representative of an individual (or population of individuals) having pulmonary sepsis.

In one embodiment, the patient may be diagnosed as having abdominal sepsis and pulmonary sepsis when an increase is observed in any one or more of: MRAS, PCOLCE2, TMEM37, SLC39A8, KIF2C, CIQC, CIQB, CIQA, and TNF, and/or a decrease is observed in any one or more of: IFI44, IFIT1, and RPGRIP1, in the sample obtained from the patient relative to the corresponding reference value representative of an individual having pulmonary sepsis; and an increase is observed in the one or more biomarker for pulmonary sepsis in the sample obtained from the patient relative to the corresponding reference value representative of an individual having abdominal sepsis.

The method for distinguishing between abdominal sepsis and pulmonary sepsis in a patient as described herein can be used in a decision tree process to investigate the health of a patient having or suspected of having a systemic inflammatory condition. For example, the method for distinguishing between abdominal sepsis and pulmonary sepsis in a patient can be performed before, after, or in addition to any of the other methods of the invention described herein.

In one embodiment, the method of the invention for distinguishing between abdominal sepsis and pulmonary sepsis in a patient can be performed subsequent to (or in addition to) the method for diagnosing whether a patient has a systemic inflammatory condition (as described herein). If the patient tests positive for a systemic inflammatory condition (using the method of the invention for diagnosing whether a patient has a systemic inflammatory condition), they may be tested using the distinguishing method described herein to determine whether they have abdominal sepsis and/or pulmonary sepsis.

In one embodiment, the method of the invention for distinguishing between abdominal sepsis and pulmonary sepsis in a patient can be performed subsequent to (or in addition to) the method for distinguishing between sepsis and SIRS in a patient (as described herein). If the patient tests positive for sepsis (using the method distinguishing between sepsis and SIRS in a patient), they may be tested using the distinguishing method described herein to determine whether they have abdominal sepsis and/or pulmonary sepsis.

In one embodiment, the method of the invention for distinguishing between abdominal sepsis and pulmonary sepsis in a patient can be performed subsequent to (or in addition to) the method for diagnosing sepsis in a patient (as described herein). If the patient tests positive for sepsis (using the method diagnosing sepsis), they may be tested using the distinguishing method described herein to determine whether they have abdominal sepsis and/or pulmonary sepsis.

In one embodiment, the method of the invention for distinguishing between abdominal sepsis and pulmonary sepsis in a patient can be performed subsequent to (or in addition to) the method for diagnosing whether a patient has a systemic inflammatory condition (as described herein), the method for distinguishing between sepsis and SIRS in a patient (as described herein), and/or the method for diagnosing sepsis (as described herein). If the patient tests positive for a systemic inflammatory condition (using the method of the invention for diagnosing whether a patient has a systemic inflammatory condition), they may be tested for sepsis using the method for distinguishing between sepsis and SIRS described herein, and/or the method for diagnosis of sepsis described herein. If the patients tests positive for sepsis, they may be tested using the distinguishing method described herein to determine whether they have abdominal sepsis and/or pulmonary sepsis.

In one embodiment, the patient may be tested for sepsis (using the method for distinguishing between sepsis and SIRS described herein, and/or the method for diagnosis of sepsis described herein). If the patient tests positive for sepsis, they may be tested using the distinguishing method described herein to determine whether they have abdominal sepsis and/or pulmonary sepsis.

The above described combination of methods may also be performed in parallel to determine the disease status of a patient by simultaneously (or substantially simultaneously) investigating the expression of all the biomarkers in a sample obtained from the patient, and determining whether the patient has or is at risk of having abdominal sepsis and/or pulmonary sepsis.

When performing these different methods in a decision tree process, the sample used in each step of the method may be the same sample obtained from the patient (as described herein). When the method comprises multiple quantification steps, these multiple steps may be performed at the same time (e.g. in parallel) and/or using the same sample. When the method comprises multiple comparison steps, these multiple steps may be performed at the same time (e.g. in parallel).

In a related aspect, the present invention also provides the use of one or more biomarker for abdominal sepsis (as described herein), and/or one or more biomarker for pulmonary sepsis (as described herein) for distinguishing between abdominal sepsis and pulmonary sepsis in a patient.

In one embodiment, the invention provides the use of one or more biomarker for abdominal sepsis selected from the group consisting of: SLC39A8, CIQC, CIQA, MRAS, TMEM37, CIQB, PCOLCE2, KIF2C TNF, IFI44, IFIT1 and RPGRIP1, and/or one or more biomarker for pulmonary sepsis selected from the group consisting of: HCAR2, CXCR1, DISC1, EPSTI1, and IFI44, for distinguishing between abdominal sepsis and pulmonary sepsis in a patient.

In one embodiment, the invention provides the use of one or more biomarker for abdominal sepsis selected from the group consisting of: SLC39A8, CIQC, CIQA, MRAS, TMEM37, CIQB, PCOLCE2, KIF2C and TNF, and/or one or more biomarker for pulmonary sepsis selected from the group consisting of: HCAR2, CXCR1, DISC1, EPSTI1, and IFI44, for distinguishing between abdominal sepsis and pulmonary sepsis in a patient..

In one embodiment, the invention provides the use of one or more biomarker for abdominal sepsis selected from the group consisting of: SLC39A8, CIQC, CIQA, MRAS, TMEM37, and CIQB, and/or one or more biomarker for pulmonary sepsis selected from the group consisting of: HCAR2, CXCR1, and DISC1, for distinguishing between abdominal sepsis and pulmonary sepsis in a patient. For example, the abdominal sepsis biomarkers: SLC39A8, CIQC, CIQA, MRAS, TMEM37, and CIQB may be used in combination with the pulmonary sepsis biomarkers: HCAR2, CXCR1, and DISC1.

In one embodiment, the invention provides the use may be of one or more biomarker for abdominal sepsis selected from the group consisting of: SLC39A8, CIQC, and CIQA, and/or one or more biomarker for pulmonary sepsis selected from the group consisting of: HCAR2, CXCR1, and DISC1, for distinguishing between abdominal sepsis and pulmonary sepsis in a patient. For example, the abdominal sepsis biomarkers: SLC39A8, CIQC, and CIQA may be used in combination with the pulmonary sepsis biomarkers: HCAR2, CXCR1, and DISC1.

In one embodiment, the invention provides the use of one or more biomarker for abdominal sepsis selected from the group consisting of: SLC39A8, CIQC, and CIQA, and/or one or more biomarker for pulmonary sepsis selected from the group consisting of: HCAR2 and CXCR1, for distinguishing between abdominal sepsis and pulmonary sepsis in a patient. For example, the abdominal sepsis biomarkers: SLC39A8, CIQC, and CIQA may be used in combination with the pulmonary sepsis biomarkers: HCAR2, and CXCR1.

All embodiments described above for the "method of distinguishing between abdominal sepsis and pulmonary sepsis in a patient" apply equally to the 'use' of the invention described herein. This includes all embodiments relating to the terms "abdominal sepsis", "pulmonary sepsis", "patient", "sample", "the one or more biomarker for abdominal sepsis", and "the one or more biomarker for pulmonary sepsis". All combinations of abdominal sepsis and pulmonary sepsis biomarkers described above for the method of distinguishing between abdominal sepsis and pulmonary sepsis in a patient apply equally to the 'use' of the invention described herein.

### Monitoring a systemic inflammatory condition

The progression of a patient from normalcy (ie. a condition characterised by not having a systemic inflammatory condition) to having a systemic inflammatory condition is characterised by changes in biomarkers, as certain biomarkers are expressed at increasingly higher levels and the expression of other biomarkers becomes down regulated. The present inventors have identified biomarkers that both increase and decrease in abundance as a physiological response to a systemic inflammatory condition is established or subsides. A feature of a patient's biomarker profile that is known to change in intensity as a physiological response to a systemic inflammatory condition becomes established may be therefore be selected for monitoring of a systemic inflammatory condition in a patient. A comparison of the same feature in a profile from a subsequent biological sample from the patient can establish whether the patient is developing a more severe form of the systemic inflammatory condition or is progressing towards normalcy. The present invention therefore also provides a method of monitoring a systemic inflammatory condition in a patient.

In one embodiment, the method of monitoring a systemic inflammatory condition comprises performing any of the methods of the invention for diagnosis of a systemic inflammatory condition (including those for diagnosis of sepsis, diagnosis of abdominal sepsis, diagnosis of pulmonary sepsis, diagnosis of SIRS, for distinguishing between sepsis and SIRS, and for distinguishing between abdominal sepsis and pulmonary sepsis, or any combination of these methods described herein) at a first time point, repeating the 'quantification' and 'comparison' steps of said method at one or more later time points, and comparing the presence and/or amount of each marker determined at said one or more later time point to the presence and/or amount of each marker determined at the first time point, to monitor the systemic inflammatory condition. All embodiments of the diagnostic methods described herein apply equally to the monitoring method of the invention.

By repeating the diagnostic method at one or more later time point, the disease status of the patient can be re-classified to determine whether there has been a change or no change in the disease status of the patient. For example, when the level of the one or more biomarker returns towards (or becomes increasingly statistically similar to) the level typically observed for the reference value representative of a healthy individual, and/or increasingly statistically deviates from the level typically observed for the reference value representative of a systemic inflammatory condition, this indicates that there has been an improvement or regression of the systemic inflammatory condition in the test individual. Likewise, when the level of the one or more biomarker increasingly statistically deviates from the level typically observed for the reference value representative of a healthy individual, and/or remains statistically similar to (or becomes increasingly statistically similar to) the level typically observed for the reference value representative of a systemic inflammatory condition, this indicates that there has been a worsening or progression of the systemic inflammatory condition in the test individual.

Monitoring of a systemic inflammatory condition in a patient may be used to monitor the recovery of a patient having a systemic inflammatory condition. As used herein, the term "recovery" refers to the survival of a patient having a systemic inflammatory condition. When a patient recovers from a systemic inflammatory condition, they no longer exhibit symptoms of the condition, and return to a normal (or near normal) state of health. In contrast, non-recovery from a systemic inflammatory condition means that the patient does not survive the systemic inflammatory condition. The symptoms of the condition in the patient generally worsen, and the patient may experience multiple organ failure resulting in death.

Monitoring of a systemic inflammatory condition in a patient may be used to monitor the severity of the systemic inflammatory condition in a patient. For example, the method of the invention may comprise monitoring of the progression, regression, aggravation, alleviation or recurrence of the condition. Monitoring of a systemic inflammatory condition in a patient may comprise determining whether the systemic inflammatory condition is progressing towards a more severe form of the condition, or regressing towards normalcy. Monitoring may also comprise determining whether the systemic inflammatory condition has remained stable.

As used herein, the term "progression" refers to an increase or worsening in the symptoms of a disease or disorder, and the term "regression" refers to a decrease or improvement in the symptoms of disease or disorder.

The monitoring method of the invention may be applied in the course of a medical treatment of the patient aimed at alleviating the monitored condition. In one embodiment, the monitoring method may be used to aid determination as to the correct course of treatment, permit evaluation of the effectiveness of treatment, and/or permit determination as to whether to continue or cease treatment. In a preferred embodiment, the method is used to monitor the effectiveness of a treatment regimen for a systemic inflammatory condition. Suitable therapies are as described herein for the treatment of sepsis and/or SIRS.

The monitoring method of the invention may also be used to make decisions about a patient, such as deciding whether a patient may be discharged, needs a change in treatment or needs further hospitalisation.

The monitoring method of the invention may be used to provide a means of disease staging and/or to permit determination as to clinical outcome. In one embodiment, the method may be used to monitor a patient for prognosis of recovery.

As used herein, the terms "prognosis" or "prognosticating" refers to an anticipation on the progression of a disease or condition and the prospect of recovery. A "good prognosis" (or a "prognosis of recovery") refers to an anticipation of a satisfactory partial or complete recovery from the disease or condition. A "poor prognosis" (or a "prognosis of non-recovery") encompasses anticipation of a substandard recovery and/or unsatisfactory recovery, or to substantially no recovery, or even further worsening of the disease or condition.

Monitoring of a systemic inflammatory condition can also be performed without an external reference value, by obtaining samples from the patient at different time points, and comparing the marker profile of these samples to one another.

In one embodiment, the method for monitoring a systemic inflammatory condition in a patient, comprises:
(i) determining the presence and/or amount of one or more biomarker described herein in a sample obtained from a patient at a first (or earlier) time point;
(ii) determining the presence and/or amount of the one or more biomarker in a sample obtained from the patient at one or more later time points;
(iii) comparing the presence and/or amount of the one or more biomarker determined in step (ii) to the presence and/or amount of the one or more biomarker determined in step (i).

The "systemic inflammatory condition" monitored using the method of the invention is as described above for the diagnostic methods described herein. In one embodiment, the systemic inflammatory condition is selected from one or more (eg. both) of SIRS and sepsis. In one embodiment, the systemic inflammatory condition is selected from one or more (eg. two or more or all 3) of SIRS, abdominal sepsis and pulmonary sepsis. In one embodiment, the systemic inflammatory condition is SIRS. In one embodiment, the systemic inflammatory condition is sepsis. In one embodiment, the systemic inflammatory condition is abdominal sepsis. In one embodiment, the systemic inflammatory condition is pulmonary sepsis.

In one embodiment, steps (i) and (ii) of the method involve "determining the presence and amount of the one or more biomarker in a sample obtained from a patient", and step (iii) involves "comparing the presence and amount of the one or more biomarker determined in step (ii) to the presence and amount of the one or more biomarker determined in step (i)". In one embodiment, steps (i) and (ii) of the method involve "determining the amount of the one or more biomarker in a sample obtained from a patient", and step (iii) involves "comparing the amount of the one or more biomarker determined in step (ii) to the amount of the one or more biomarker determined in step (i)".

The "patient" for which monitoring is performed is as defined above for the diagnostic methods described herein. In one embodiment, the patient is suspected of having or being at risk of developing a systemic inflammatory condition. In one embodiment, the patient has been diagnosed as having or being at risk of developing a systemic inflammatory condition. In one embodiment, the patient has been diagnosed as having or being at risk of developing a systemic inflammatory condition using the method described herein for diagnosing a systemic inflammatory condition. In one embodiment, the patient has been diagnosed as having or being at risk of developing a systemic inflammatory condition using the methods described herein for diagnosing SIRS, sepsis (such as abdominal sepsis, or pulmonary sepsis), or using the method described herein for distinguishing between sepsis and SIRS in a patient, or for distinguishing between abdominal sepsis and pulmonary sepsis, or any combination of these methods described herein.

In one embodiment, the patient has been diagnosed as having SIRS (eg. using the method described herein for diagnosis of SIRS, or for distinguishing between sepsis and SIRS in a patient). In one embodiment, the patient has been diagnosed as having sepsis, such as abdominal sepsis or pulmonary sepsis (eg. using the methods described herein for diagnosis of sepsis, abdominal sepsis or pulmonary sepsis, the method described herein for distinguishing between sepsis and SIRS in a patient, or for distinguishing between abdominal sepsis and pulmonary sepsis, or any combination of these methods described herein). The patient may be undergoing treatment for a systemic inflammatory condition.

The "sample" obtained from the patient is as defined above for the diagnostic methods.

The monitoring methods described herein allow the monitoring of a systemic inflammatory condition in a patient over time. All embodiments relating to the time point at which a sample is obtained from the patient as described above for the diagnostic methods (eg. in the method for diagnosing a systemic inflammatory condition in a patient) apply equally to the sample obtained from the patient at "a first (or earlier) time point" in the monitoring methods described herein. For example, the sample may be obtained at least 1 hour, 2 hours, 4 hours, 6 hours, 8 hours, 12 hours, 36 hours, 48 hours, 72 hours, 96 hours, or 120 hours, after the patient presents with one or more clinical symptoms of a systemic inflammatory condition, or is admitted to a medical care facility. In one embodiment, the sample may be obtained from the patient at least 24 hours after the patient presents with one or more clinical symptoms of a systemic inflammatory condition, or is admitted to a medical care facility.

The sample obtained from the patient at the "one or more later time points" may be obtained at least 6 hours (e.g. at least 12 hours, at least 18 hours, at least 24 hours, at least 48 hours, at least 72 hours, at least 96 hours, at least 120 hours, at least 1 week, at least 2 weeks, at least 3 weeks, at least 1 month) after the sample was obtained from the patient at a first (or earlier) time point.

In one embodiment, when the method is for monitoring the effectiveness of a treatment regimen for a systemic inflammatory condition in a patient, the sample obtained from the patient at a first (or earlier) time point is obtained from the patient before or during the course of treatment. For example, the sample may be obtained from the patient at least 1 hour (e.g. at least 2 hours, at least 4 hours, at least 8 hours, at least 12 hours, at least 18 hours, at least 24 hours) before treatment. The sample obtained from the patient at one or more later time points is obtained during or after a course of treatment. For example, the sample may be obtained from the patient at least 1 hour (e.g. at least 2 hours, at least 4 hours, at least 8 hours, at least 12 hours, at least 18 hours, at least 24 hours) after a treatment regimen has begun or has been completed.

The "one or more biomarker" of the invention is as described above for the diagnostic methods described herein. The one or more biomarker used in the monitoring method of the invention may include any of the biomarkers described herein (e.g. as defined in Tables 1-4), or any combination of biomarkers described herein.

In addition to the biomarkers described above, the present inventors have also identified a set of biomarkers which are particularly useful for monitoring a systemic inflammatory condition in a patient. These biomarkers include ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1 and LILRB5 (see Tables 1 and 4).

Thus, in one embodiment, the one or more biomarker is selected from: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1, and LILRB5. These biomarkers were observed to change in abundance over time in the samples obtained from patients having systemic inflammatory conditions (such as sepsis or SIRS), as the patients either recovered from the condition, or did not recover and subsequently died.

The reference to the biomarker HLA-DPB1 throughout the entire description, includes the HLA-DPB1 sequence encoded by SEQ ID NO: 30 and the transcript variant X1 of HLA-DPB1 (as encoded by SEQ ID NO:31 ). In one embodiment, the reference to the biomarker HLA-DPB1 is a reference to sequence encoded by SEQ ID NO: 30. In one embodiment, the reference to the biomarker HLA-DPB1 is a reference to the transcript variant X1 of HLA-DPB1 (as encoded by SEQ ID NO: 31).

Each of the biomarkers may be used alone, or in combination with any of the biomarkers described herein to monitor a systemic inflammatory condition in the method of the invention. For example, any combination of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, up to and including all of the biomarkers may be used to monitor a systemic inflammatory condition in a patient.

In one embodiment, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, or all 21) of the biomarkers selected from the group consisting of: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1, and LILRB5, may be used to monitor a systemic inflammatory condition in a patient (such as abdominal sepsis and/or SIRS). For example, any combination of 1 or more (eg. 2 or more, 3 or more, 4 or more, or all 5) of the biomarkers selected from the group consisting of: KLRB1, BCL11B, FCER1A, PKHD1, and LILRB5, may be used to monitor a systemic inflammatory condition in a patient (such as sepsis and/or SIRS).

A sub-set of these biomarkers is particularly useful for monitoring sepsis (eg. abdominal sepsis), including ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, and MX1. Thus, in one embodiment, the one or more biomarker is selected from: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, and MX1. In one embodiment, the method of the invention is for monitoring of sepsis (eg. abdominal sepsis) in a patient.

In one embodiment, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, or all 18) of the biomarkers selected from the group consisting of: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, and MX1, may be used to monitor a systemic inflammatory condition in a patient (such as abdominal sepsis).

Within this sub-set of biomarkers, the inventors identified markers that increase in abundance over time as the patient recovers from abdominal sepsis (returning towards the elevated level typically observed for healthy individuals), but show no increase (or a decrease) in abundance when the patient does not recover from abdominal sepsis. These include one or more biomarker selected from: ITM2A, CCL5, KLRK1, KLRB1, HLA-DRA, BCL11B, HLA-DPB1, SLAMF6, CD160, KLRF1, and MX1. The inventors also identified that the biomarker NPPC increases significantly in abundance over time when the patient does not recover from abdominal sepsis, and show no increase (or a decrease) in abundance over time when the patient does recover from abdominal sepsis (thereby returning towards the reduced level typically observed for healthy individuals). These biomarkers are particularly useful for monitoring abdominal sepsis in a patient, particularly for monitoring recovery from abdominal sepsis.

In one embodiment, the one or more biomarker is selected from: ITM2A, CCL5, NPPC, KLRK1, KLRB1, HLA-DRA, BCL11B, HLA-DPB1, SLAMF6, CD160, KLRF1, and MX1. In one embodiment, any combination of 1 or more (eg. 2 or more, 3 or more, 4 or more, 5 or more, 6 or more, 7 or more, 8 or more, 9 or more, 10 or more, 11 or more, or all 12) of the biomarkers selected from the group consisting of: ITM2A, CCL5, NPPC, KLRK1, KLRB1, HLA-DRA, BCL11B, HLA-DPB1, SLAMF6, CD160, KLRF1, and MX1, may be used to monitor a systemic inflammatory condition in a patient (such as abdominal sepsis).

In one embodiment, the one or more biomarker is selected from one or more (eg. 2 or more, 3 or more, 4 or more, 5 or more, 6 or more, 7 or more, 8 or more, 9 or more, 10 or more, or all 11) of: ITM2A, CCL5, KLRK1, KLRB1, HLA-DRA, BCL11B, HLA-DPB1, SLAMF6, CD160, KLRF1, and MX1, in combination with the biomarker NPPC.

The inventors also observed that a sub-set of these biomarkers is particularly useful for monitoring SIRS, including ITM2A, CCL5, NPPC, PKD1, KLRK1, KLRB1, HLA-DRA, BCL11B, HLA-DPB1, CD160, KLRF1, CD2, LGALS2, MYCL, NECAB1, and PKHD1. Thus, in one embodiment, the one or more biomarker is selected from: ITM2A, CCL5, NPPC, PKD1, KLRK1, KLRB1, HLA-DRA, BCL11B, HLA-DPB1, CD160, KLRF1, CD2, LGALS2, MYCL, NECAB1, and PKHD1. In one embodiment, the method of the invention is for monitoring of SIRS in a patient.

In one embodiment, any combination of 1 or more (eg. 2 or more, 3 or more, 4 or more, 5 or more, 6 or more, 7 or more, 8 or more, 9 or more, 10 or more, 11 or more, 12 or more, 13 or more, 14 or more, 15 or more, or all 16) of the biomarkers selected from the group consisting of: ITM2A, CCL5, NPPC, PKD1, KLRK1, KLRB1, HLA-DRA, BCL11B, HLA-DPB1, CD160, KLRF1, CD2, LGALS2, MYCL, NECAB1, and PKHD1, may be used to monitor a systemic inflammatory condition in a patient (such as SIRS).

Within this sub-set of biomarkers, the inventors further identified a sub-set of markers that increase in abundance over time as the patient recovers from SIRS (returning towards the elevated levels typically observed for healthy individuals), but show no increase (or a decrease) in abundance when the patient does not recover from SIRS. These include one or more biomarker selected from: ITM2A, CCL5, KLRK1, KLRB1, and BCL11B. The inventors also identified that the biomarkers NPPC, PKDI, CD2, LGALS2, MYCL, NECAB1, and PKHD1 increase significantly in abundance over time when the patient does not recover from SIRS, and show no increase (or a decrease) in abundance over time when the patient does recover from SIRS (thereby returning towards the reduced level typically observed for healthy individuals). These biomarkers are particularly useful for monitoring SIRS in a patient, particularly for monitoring recovery from SIRS.

In one embodiment, the one or more biomarker is selected from: ITM2A, CCL5, NPPC, PKD1, KLRK1, KLRB1, BCL11B, CD2, LGALS2, MYCL, NECAB1, and PKHD1. In one embodiment, any combination of 1 or more (eg. 2 or more, 3 or more, 4 or more, 5 or more, 6 or more, 7 or more, 8 or more, 9 or more, 10 or more, 11 or more or all 12) of the biomarkers selected from the group consisting of: ITM2A, CCL5, NPPC, PKD1, KLRK1, KLRB1, BCL11B, CD2, LGALS2, MYCL, NECAB1, and PKHD1, may be used to monitor a systemic inflammatory condition in a patient (such as SIRS).

For example, the one or more biomarker may be selected from: ITM2A, CCL5, NPPC, PKD1, LGALS2, MYCL, NECAB1, and PKHD1. For example, the one or more biomarker may be selected from: CCL5, NPPC, PKD1, LGALS2, MYCL, NECAB1, and PKHD1. For example, the one or more biomarker may be selected from: CCL5, NPPC, PKD1, LGALS2, NECAB1, and PKHD1. For example, the one or more biomarker may be selected from: CCL5, NPPC, PKDI, NECAB1, and PKHD1.

In one embodiment, the one or more biomarker is selected from one or more of: ITM2A, CCL5, KLRK1, KLRB1, and BCL11B, in combination with one or more biomarker selected from: NPPC, PKDI, CD2, LGALS2, MYCL, NECAB1, and PKHD1. For example, the one or more biomarker may be selected from one (eg. both) or more of: ITM2A and CCL5, in combination with one or more (eg. 2 or more, 3 or more, or all 4) biomarker selected from: NPPC, PKDI, NECAB1, and PKHD1. For example, the one or more biomarker may be CCL5 used in combination with one or more biomarker selected from: NPPC, PKD1, LGALS2, MYCL, NECAB1, and PKHD1. For example, the one or more biomarker may be CCL5 used in combination with one or more biomarker selected from: NPPC, PKD1, NECAB1, and PKHD1.

The present inventors have identified biomarkers that can be used to monitor multiple different types of systemic inflammatory condition (such SIRS and sepsis) in a single method. In one embodiment, the one or more (eg. 2 or more, 3 or more, 4 or more, 5 or more, 6 or more, 7 or more, or all 8) biomarker is selected from: ITM2A, CCL5, NPPC, KLRK1, KLRB1, HLA-DRA, BCL11B, and HLA-DPB1. In one embodiment, the one or more (2 or more, 3 or more, 4 or more, or all 5) biomarker is selected from: ITM2A, CCL5, NPPC, KLRK1, and BCL11B. In one embodiment, the one or more (2 or more, or all 3) biomarker is selected from: ITM2A, CCL5, and NPPC. For example, the one or more biomarker is selected from: CCL5 and NPPC. In one embodiment, the one or more biomarker may be one or more (or both) of: ITM2A and CCL5 in combination with NPPC.

In one embodiment, the one or more biomarker is FCER1A. In one embodiment, the one or more biomarker is KLRK1. In one embodiment, the one or more biomarker is KLRB1. In one embodiment, the one or more biomarker is DAAM2. In one embodiment, the one or more biomarker is HLA-DRA. In one embodiment, the one or more biomarker is BCL11B. In one embodiment, the one or more biomarker is SLAMF6. In one embodiment, the one or more biomarker is ITM2A. In one embodiment, the one or more biomarker is CD160. In one embodiment, the one or more biomarker is HLA-DPB1. In one embodiment, the one or more biomarker is KLRF1. In one embodiment, the one or more biomarker is CD2. In one embodiment, the one or more biomarker is LGALS2. In one embodiment, the one or more biomarker is NPPC. In one embodiment, the one or more biomarker is MYCL. In one embodiment, the one or more biomarker is MX1. In one embodiment, the one or more biomarker is NECAB1. In one embodiment, the one or more biomarker is NECAB2. In one embodiment, the one or more biomarker is PKHD1. In one embodiment, the one or more biomarker is PKD1. In one embodiment, the one or more biomarker is CCL5. In one embodiment, the one or more biomarker is LILRB5.

All embodiments of the 'quantification' and 'comparison' steps of the diagnostic methods described herein (such as the method for diagnosis of a systemic inflammatory condition) apply equally to the monitoring methods described herein.

The step of "comparing the presence and/or amount determined in step (ii) to the presence and/or amount determined in step (i)" involves determining whether there is a difference in the presence and/or amount of the one or more biomarkers between the samples. It is possible to monitor a systemic inflammatory condition by attributing the finding of a difference or no difference in the one or more biomarker to a change in the systemic inflammatory condition in the individual between the two or more successive time points.

A finding of "no difference" in the presence and/or amount of the one or more biomarker detected in the two or more successive time points indicates that there has been no change in the systemic inflammatory condition in the individual. In contrast, finding of a "difference" in the presence and/or amount of the one or more biomarker detected in the two or more successive time points indicates that there has been a change in the systemic inflammatory condition in the individual.

A difference in the presence and/or amount of the one or more biomarker measured by the monitoring methods of the present invention can comprise an increase or decrease in the one or more biomarkers over time. The increase or decrease in the biomarker can be, for example, at least 0.1 (eg. at least 0.2, at least 0.3, at least 0.4, at least 0.5, at least 0.6, at least 0.7, at least 0.8, at least 0.9, at least 1, at least 1.1, at least 1.2, at least 1.3, at least 1.4, at least 1.5, at least 1.6, at least 1.7, at least 1.8, at least 1.9, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, or at least 10) fold over time. The difference in the presence and/or amount of the biomarker is preferably statistically significant. By "statistically significant", it is meant that the alteration is greater than what might be expected to happen by chance alone.

The increase or decrease in the one or more biomarker in the patient over time can indicate progression of the disease, the lack of efficacy of one or more treatment regimens, and/or a poor prognosis of recovery (or a prognosis of non-recovery). Alternatively, the increase or decrease in the one or more biomarker in the patient over time can indicate regression of the disease, the success of one or more treatment regimens, and/or a good prognosis of recovery (or a prognosis of recovery).

For example, an increase in any one or more of ITM2A, CCL5, KLRK1, KLRB1, HLA-DRA, BCL11B, HLA-DPB1, SLAMF6, CD160, KLRF1, and MX1 in the sample obtained at the later time point relative to the sample obtained from the first time point indicates regression of the systemic inflammatory condition in the patient. No increase in ITM2A, CCL5, KLRK1, KLRB1, HLA-DRA, BCL11B, HLA-DPB1, SLAMF6, CD160, KLRF1, or MX1 in the sample obtained at the later time point relative to the sample obtained from the first time indicates no regression of the systemic inflammatory condition in the patient. In one embodiment, no increase in ITM2A, CCL5, KLRK1, KLRB1, HLA-DRA, BCL11B, HLA-DPB1, SLAMF6, CD160, KLRF1, or MX1 in the sample obtained at the later time point relative to the sample obtained from the first time point may indicate progression of the systemic inflammatory condition in the patient.

For example, an increase in any one or more of ITM2A, CCL5, KLRK1, KLRB1, HLA-DRA, BCL11B, HLA-DPB1, SLAMF6, CD160, KLRF1, and MX1 in the sample obtained at the later time point relative to the sample obtained from the first time point indicates the success of one or more treatment regimens. No increase in ITM2A, CCL5, KLRK1, KLRB1, HLA-DRA, BCL11B, HLA-DPB1, SLAMF6, CD160, KLRF1, or MX1 in the sample obtained at the later time point relative to the sample obtained from the first time point indicates the lack of efficacy of one or more treatment regimens.

For example, an increase in any one or more of ITM2A, CCL5, KLRK1, KLRB1, HLA-DRA, BCL11B, HLA-DPB1, SLAMF6, CD160, KLRF1, and MX1 in the sample obtained at the later time v relative to the sample obtained from the first time point indicates a (good) prognosis of recovery. No increase in ITM2A, CCL5, KLRK1, KLRB1, HLA-DRA, BCL11B, HLA-DPB1, SLAMF6, CD160, KLRF1, or MX1 in the sample obtained at the later time point relative to the sample obtained from the first time point indicates a poor prognosis of recovery (or a prognosis of non-recovery).

In a further example, an increase in any one or more of NPPC, PKD1, CD2, LGALS2, MYCL, NECAB1, and PKHD1 in the sample obtained at the later time point relative to the sample obtained from the first time point indicates progression of the systemic inflammatory condition in the patient. No increase in NPPC, PKD1, CD2, LGALS2, MYCL, NECAB1, or PKHD1, in the sample obtained at the later time point relative to the sample obtained from the first time point indicates no progression of the systemic inflammatory condition in the patient. In one embodiment, no increase in NPPC, PKD1, CD2, LGALS2, MYCL, NECAB1, or PKHD1, in the sample obtained at the later time point relative to the sample obtained from the first time point indicates regression of the systemic inflammatory condition in the patient.

In a further example, an increase in any one or more of NPPC, PKD1, CD2, LGALS2, MYCL, NECAB1, and PKHD1 in the sample obtained at the later time point relative to the sample obtained from the first time point indicates the lack of efficacy of one or more treatment regimens. No increase in NPPC, PKD1, CD2, LGALS2, MYCL, NECAB1, or PKHD1 in the sample obtained at the later time point relative to the sample obtained from the first time point indicates the success of one or more treatment regimens.

In a further example, an increase in any one or more of NPPC, PKD1, CD2, LGALS2, MYCL, NECAB1, and PKHD1 in the sample obtained at the later time point relative to the sample obtained from the first time point indicates a poor prognosis of recovery (or a prognosis of non-recovery). No increase in NPPC, PKD1, CD2, LGALS2, MYCL, NECAB1, and PKHD1 in the sample obtained at the later time point relative to the sample obtained from the first time point indicates a (good) prognosis of recovery.

In a related aspect, the present invention also provides the use of the one or more biomarker described herein for monitoring a systemic inflammatory condition in a patient.

In one embodiment, the use is of one or more biomarker selected from: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1, and LILRB5 for monitoring a systemic inflammatory condition in a patient.

In one embodiment, the use is of one or more biomarker selected from ITM2A, CCL5, NPPC, KLRK1, KLRB1, HLA-DRA, BCL11B, and HLA-DPB1, for monitoring a systemic inflammatory condition in a patient (such sepsis and/or SIRS). For example, the one or more biomarker may be selected from: ITM2A, CCL5, NPPC, KLRK1, and BCL11B. For example, the one or more biomarker may be selected from: ITM2A, CCL5, and NPPC.

In one embodiment, the use is of one or more biomarker selected from: ITM2A, CCL5, NPPC, KLRK1, KLRB1, HLA-DRA, BCL11B, HLA-DPB1, FCER1A, DAAM2, SLAMF6, CD160, KLRF1, CD2, LGALS2, MYCL, and MX1, for monitoring a systemic inflammatory condition in a patient (such as abdominal sepsis). For example, the one or more biomarker may be selected from: ITM2A, CCL5, NPPC, KLRK1, KLRB1, HLA-DRA, BCL11B, HLA-DPB1, SLAMF6, CD160, KLRF1, and MX1.

In one embodiment, the use is of one or more biomarker selected from: ITM2A, CCL5, NPPC, PKD1, KLRK1, KLRB1, HLA-DRA, BCL11B, HLA-DPB1, CD160, KLRF1, CD2, LGALS2, MYCL, NECAB1, and PKHD1 for monitoring a systemic inflammatory condition in a patient (such as SIRS). In one embodiment, the use is of one or more biomarker selected from: ITM2A, CCL5, NPPC, PKD1, KLRK1, KLRB1, BCL11B, CD2, LGALS2, MYCL, NECAB1, and PKHD1 for monitoring a systemic inflammatory condition in a patient (such as SIRS). For example, the one or more biomarker may be selected from: CCL5, NPPC, PKD1, LGALS2, MYCL, NECAB1, and PKHD1.

All embodiments described above for the method of monitoring a systemic inflammatory condition in a patient apply equally to the 'use' of the invention described herein. This includes all embodiments relating to the terms "systemic inflammatory condition", "patient", "sample obtained a first time point", "sample obtained at one or more later time points", and "the one or more biomarker".

### Survival biomarkers

A major issue facing clinicians is determining when a patient is suitable for release from medical care. In some cases, patients appear to physically recover (eg. from a systemic inflammatory condition), yet still do not survive after they are discharged from medical care. When studying the gene expression patterns of biomarkers in patients having a systemic inflammatory condition, the inventors surprisingly observed that several of the biomarkers described herein were present at much higher levels in patients that did not survive as compared to patients that made a full recovery. The inventors observed that the likelihood of survival of a patient could therefore be predicted by monitoring the levels of these "survival" biomarkers. Detection of the levels of these biomarkers in patients will therefore assist clinicians in determining whether a patient is suitable for discharge from medical care.

The present invention therefore provides a method for determining whether a patient is suitable for discharge from medical care, comprising:
(i) determining the presence and/or amount of one or more biomarker selected from the group consisting of: NECAB1, NECAB2, PKD1, PKHD1, LILRB4, and LILRB5 in a sample obtained from a patient,
(ii) comparing the presence and/or amount of the one or more biomarker determined in said sample in (i) to a corresponding reference value, and thereby determining whether the patient is suitable for discharge from medical care.

"Determining whether a patient is suitable for discharge from medical care" means determining whether the patient has a good prognosis of survival and can be safely discharged from medical care. The method therefore provides a way of predicting the survival of a patient (such as a patient that has been diagnosed with a systemic inflammatory condition). The method may therefore be alternatively defined as a "method for predicting the survival of a patient".

As used herein, "discharge from medical care" encompasses "discharge from high-dependency medical care". For example, it may refer to the act of moving a patient from a high dependency unit (such as an intensive care unit) to a lower dependency unit (such as an outpatient unit, a hospital ward, or home care).

In one embodiment, step (i) of the method involves "determining the presence and amount of the one or more biomarker in a sample obtained from a patient", and step (ii) involves "comparing the presence and amount of the one or more biomarker determined in said sample in (i) to a corresponding refrence value". In one embodiment, step (i) of the method involves "determining the amount of the one or more biomarker in a sample", and step (ii) involves "comparing the amount of the one or more biomarker determined in said sample in (i) to a corresponding reference value".

The "sample" obtained from the patient is as defined above for the diagnostic methods and monitoring methods described herein, including all embodiments relating to the time point at which the sample is obtained. In one embodiment, the sample may be obtained at least 48 hours after the patient presents with one or more clinical symptoms of a systemic inflammatory condition, or is admitted to a medical care facility. In one embodiment, the sample may be obtained at least 72 hours after the patient presents with one or more clinical symptoms of a systemic inflammatory condition, or is admitted to a medical care facility. In one embodiment, the sample may be obtained at least 96 hours after the patient presents with one or more clinical symptoms of a systemic inflammatory condition, or is admitted to a medical care facility. In one embodiment, the sample may be obtained at least 120 hours after the patient presents with one or more clinical symptoms of a systemic inflammatory condition, or is admitted to a medical care facility.

The method of the invention is intended to be used as a point of care monitor to determine whether it is safe to discharge a patient from medical care. In one embodiment, the sample may be obtained from a patient after treatment for a systemic inflammatory condition has been completed. In one embodiment, the sample is obtained from a patient when they have been clinically diagnosed as being suitable for discharge from medical care.

The "patient" is as described above for the diagnostic methods and monitoring methods described herein. In one embodiment, the patient has been diagnosed as having or being at risk of developing a systemic inflammatory condition. In one embodiment, the patient has been diagnosed as having or being at risk of developing a systemic inflammatory condition using the method described herein for diagnosing a systemic inflammatory condition. In one embodiment, the patient may have been diagnosed as having or being at risk of developing a systemic inflammatory condition using the methods described herein for diagnosing SIRS, sepsis (such as abdominal sepsis or pulmonary sepsis), or using the method described herein for distinguishing between sepsis and SIRS in a patient, or any combination of these methods as described herein). The patient may be undergoing (or has undergone) treatment for a systemic inflammatory condition.

In one embodiment, the patient has been diagnosed as having or being at risk of developing SIRS (eg. using any of the methods described herein for diagnosing SIRS, or for distinguishing between sepsis and SIRS in a patient). The patient may be undergoing (or has undergone) treatment for SIRS.

In one embodiment, the patient has been diagnosed as having or being at risk of developing sepsis (eg. using any of the methods described herein for diagnosing sepsis, or for distinguishing between sepsis and SIRS in a patient). The patient may be undergoing (or has undergone) treatment for sepsis.

In one embodiment, the patient has been diagnosed as having or being at risk of developing abdominal sepsis (eg. using the method described herein for diagnosing abdominal sepsis). The patient may be undergoing (or has undergone) treatment for abdominal sepsis.

In one embodiment, the patient has been diagnosed as having or being at risk of developing pulmonary sepsis (eg. using the method described herein for diagnosing pulmonary sepsis). The patient may be undergoing (or has undergone) treatment for pulmonary sepsis.

The "one or more biomarker" of the invention is as described above for the diagnostic methods and monitoring methods described herein. In one embodiment, the one or more biomarker may be selected from the group consisting of: NECAB1, NECAB2, PKD1, PKHD1, LILRB4, and LILRB5.

Each of the biomarkers may be used alone, or in combination with any of the survival biomarkers described herein in the method of the invention. For example, any combination of 1 or more, 2 or more, 3 or more, 4 or more, 5 or more or all 6 of the biomarkers may be used in the method of the invention.

In one embodiment, any combination of 1 or more (eg. 2 or more, 3 or more, 4 or more, 5 or more, or all 6) of the biomarkers selected from the group consisting of: NECAB1, NECAB2, PKD1, PKHD1, LILRB4, and LILRB5, may be used to determine whether a patient is suitable for discharge from medical care.

A combination of the 'survival' biomarkers may be used in the method to determine whether a patient is suitable for discharge from medical care. In one embodiment, the method may involve determining the presence and/or amount of one or more biomarker selected from NECAB2 and PKD1, in combination with one or more biomarker selected from: NECAB1, PKDI, PKHD1, LILRB4 and LILRB5. For example, the combination of biomarkers used in the method may be NECAB2 and NECAB1. For example, the combination of biomarkers used in the method may be NECAB2 and PKHD1. For example, the combination of biomarkers used in the method may be NECAB2 and PKD1. For example, the combination of biomarkers used in the method may be NECAB2 and LILRB4. For example, the combination of biomarkers used in the method may be NECAB2 and LILRB5. For example, the combination of biomarkers used in the method may be PKD1 and PKHD1. For example, the combination of biomarkers used in the method may be PKD1 and NECAB1. For example, the combination of biomarkers used in the method may be PKD1 and LILRB4. For example, the combination of biomarkers used in the method may be PKD1 and LILRB5.

In one embodiment, the one or more biomarker is NECAB1. In one embodiment, the one or more biomarker is NECAB2. In one embodiment, the one or more biomarker is PKD1. In one embodiment, the one or more biomarker is PKHD1. In one embodiment, the one or more biomarker is LILRB4. In one embodiment, the one or more biomarker is LILRB5.

The biomarkers NECAB1 and NECAB2 are brain specific markers, and the biomarkers PKHD1 and PKD1 are kidney specific markers. These markers are not usually expressed in peripheral blood leukocytes. The high levels of these markers in the patients that did not survive indicates that these patients are suffering from kidney damage and/or brain damage. The method described herein may therefore be used to diagnose organ damage in a patient. In one embodiment, the method is for diagnosis of organ damage in a patient. For example, the method may be for diagnosis of brain damage in a patient, when the one or more biomarker is selected from NECAB1 and/or NECAB2. By performing steps (i) and (ii) of the method described herein, the method can be used to determine whether a patient has or is at risk of developing brain damage. The method may alternativelty be for diagnosis of kidney damage in a patient, when the one or more biomarker is selected from PKHD1 and/or PKD1. By performing steps (i) and (ii) of the method described herein, the method can be used to determine whether a patient has or is at risk of developing kidney damage.

The present inventors have observed that a sub-set of these biomarkers (NECAB2, PKD1, PKHD1 and LILRB5,) are particularly useful in determining whether a patient diagnosed as having or being at risk of developing sepsis (such as abdominal sepsis and/or pulmonary sepsis) is suitable for discharge from medical care. In one embodiment, the one or more biomarkers may be selected from the group consisting of NECAB2, LILRB5, PKHD1 and PKD1. The patient may be undergoing (or has undergone) treatment for sepsis (such as abdominal sepsis and/or pulmonary sepsis). Treatment for sepsis is as described herein.

A subset of the biomarkers (NECAB2 and PKD1) are particularly useful in determining whether a patient diagnosed as having or being at risk of developing abdominal sepsis is suitable for discharge from medical care. As described in Example 2, ROC analysis demonstrated that these biomarkers could be used alone or in combination to effectively distinguish between abdominal sepsis patients that died and those that survived. In one embodiment, the one or more biomarkers may be selected from the group consisting of NECAB2 and PKD1. For example, the method may be performed using the combination of biomarkers NECAB2 and PKD1. For example, the method may be performed using NECAB2. For example, the method may be performed using PKD1. The patient may be undergoing (or has undergone) treatment for abdominal sepsis.

A subset of the biomarkers (PKHD1 and LILRB5) are particularly useful in determining whether a patient diagnosed as having or being at risk of developing pulmonary sepsis is suitable for discharge from medical care. As described in Example 2, ROC analysis demonstrated that these biomarkers could be used alone or in combination to effectively distinguish between pulmonary sepsis patients that died and those that survived. In one embodiment, the one or more biomarkers may be selected from the group consisting of PKHD1 and LILRB5. For example, the method may be performed using the combination of biomarkers PKHD1 and LILRB5. For example, the method may be performed using PKHD1. For example, the method may be performed using LILRB5. The patient may be undergoing (or has undergone) treatment for pulmonary sepsis.

The present inventors have observed that a sub-set of these biomarkers (NECAB1, PKDI, PKHD1, LILRB4, and LILRB5) are particularly useful in determining whether a patient diagnosed as having or being at risk of developing SIRS is suitable for discharge from medical care. The patient may be undergoing (or has undergone) treatment for SIRS. Treatment for SIRS is as described herein. Thus, in one embodiment, the one or more biomarker is selected from the group consisting of: NECAB1, PKDI, PKHD1, LILRB4, and LILRB5. In one embodiment, any combination of 1 or more (eg. 2 or more, 3 or more, 4 or more, or all 5) of the biomarkers selected from the group consisting of: NECAB1, PKD1, PKHD1, LILRB4, and LILRB5, may be used to determine whether a patient is suitable for discharge from medical care.

As described in Example 2, PKHD1 and NECAB1 were observed to provide the most accurate distinction between patients with SIRS that survived and those that died (see the ROC curve data in Example 2). Good results were observed when these markers were used on their own or in combination. In one embodiment, the one or more biomarker may be PKHD1 and/or NECAB1. For example, the markers for determining whether a patient diagnosed as having or being at risk of developing SIRS is suitable for discharge from medical care may comprise the combination of PKHD1 and NECAB1.

All embodiments of the 'quantification' and 'comparison' steps described above for the diagnostic and monitoring methods described herein apply equally to the method for determining whether a patient is suitable for discharge from medical care. This includes all embodiments relating to the "reference value".

The one or more biomarker measured by the methods of the present invention may increase or decrease as compared to the corresponding reference value. The increase or decrease in the amount of the one or more biomarker in the patient as compared to the reference value can indicate that the patient has a good prognosis of recovery (or survival) from the systemic inflammatory condition, and thus is suitable for discharge from medical care. Alternatively, the increase or decrease in the one or more biomarker in the patient as compared to the reference can indicate that the patient has a poor prognosis of recovery (or survival) (or a prognosis of non-recovery) from the systemic inflammatory condition, and thus is not suitable for discharge from medical care.

The increase or decrease in the one or more biomarker as compared to the reference can be, for example, at least 0.1 (eg. at least 0.2, at least 0.3, at least 0.5, at least 0.6, at least 0.7, at least 0.8, at least 0.9, at least 1, at least 1.1, at least 1.2, at least 1.3, at least 1.4, at least 1.5, at least 1.6, at least 1.7, at least 1.8, at least 1.9, at least 2, at least 2.5, at least 3, at least 3.5, at least 4, at least 4.5, at least 5, at least 5.5, at least 6, at least 7 fold, at least 7.5, at least 8, at least 8.5, at least 9, at least 9.5, or at least 10) fold. The difference in the amount of the biomarker is preferably statistically significant. By "statistically significant", it is meant that the alteration is greater than what might be expected to happen by chance alone.

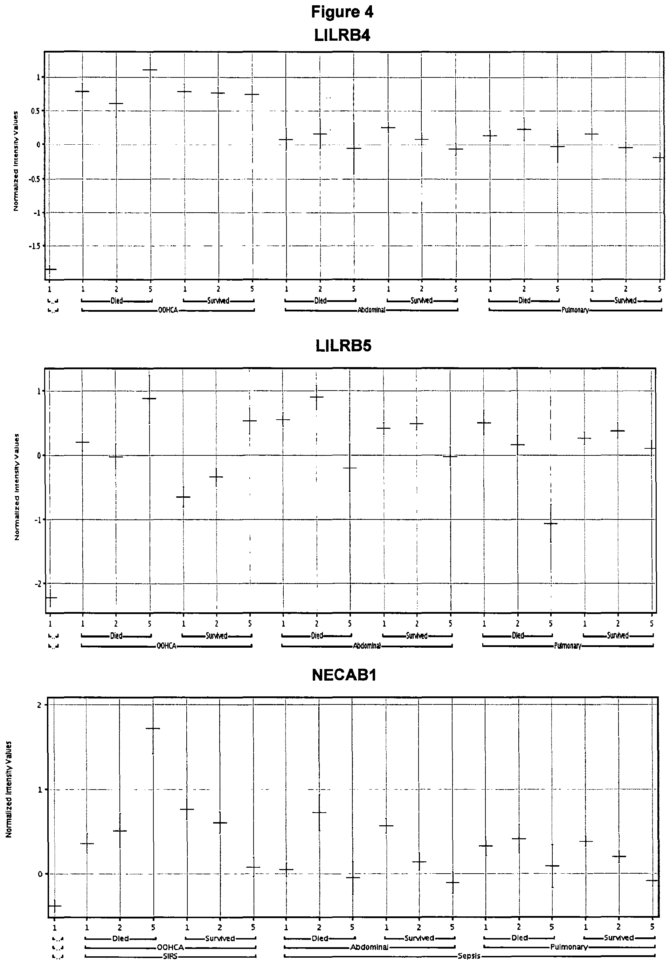

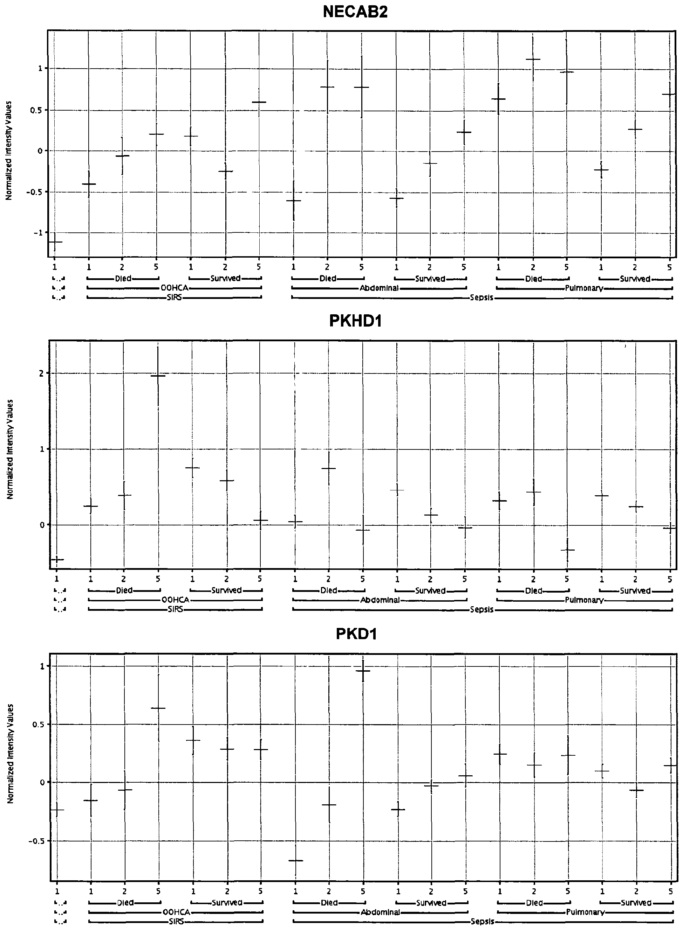

As illustrated in Figure 4, the present inventors observed that some of the "survival" biomarkers described herein increase in abundance in patients that did not survive as compared to patients that made a full recovery from a systemic inflammatory condition, and as compared to healthy individuals. These differences in marker abundance can be used to predict whether a patient is likely to survive a systemic inflammatory condition, and can thus be used to determine whether a patient is suitable for discharge from medical care. For example, the biomarkers PKHD1 and NECAB1 increased in abundance in SIRS patients that did not survive as compared to SIRS patients that made a full recovery from a systemic inflammatory condition, and as compared to healthy individuals. The biomarkers PKD1 and NECAB2 also increased in abundance in abdominal sepsis patients that did not survive as compared to abdominal sepsis patients that made a full recovery from a systemic inflammatory condition, and as compared to healthy individuals.

As illustrated in Figure 4, the present inventors observed that some of the "survival" biomarkers described herein increase in abundance in patients that made a full recovery from a systemic inflammatory condition as compared to patients that did not survive. These differences in marker abundance can be used to predict whether a patient is likely to survive a systemic inflammatory condition, and can thus be used to determine whether a patient is suitable for discharge from medical care. For example, the biomarker LILRB5 increased in abundance in pulmonary sepsis patients that made a full recovery from pulmonary sepsis as compared to pulmonary sepsis patients that did not survive.

By comparing the amount of markers quantified in a sample obtained from a patient to the amount of markers quantified for a reference value (such as that obtained from a healthy individual (or a population of healthy individuals), an individual (or population of individuals) that has a (good) prognosis of recovery (or survival) from a systemic inflammatory condition, and/or an individual (or population of individuals) that has a prognosis of non-recovery (or non-survival) from a systemic inflammatory condition), it is possible to determine whether a patient is suitable for discharge from medical care. The method permits classification of the patient as belonging to or not belonging to the reference population (ie. by determining whether the amounts of marker quantified in the patient are statistically similar to the reference population or statistically deviate from the reference population). Hence, classification of the patient's marker profile (ie. the overall pattern of change observed for the markers quantified) as corresponding to the profile derived from a particular reference population is predictive that the individual falls (or does not fall) within the reference population.

In one embodiment, a patient may be identified as being suitable for discharge from medical care, when the amount of the one or more biomarker is statistically similar to the amount determined for the corresponding reference value representative of an individual (or a population of individuals) that has a (good) prognosis of recovery (or survival) from a systemic inflammatory condition. In one embodiment, a patient may be identified as being suitable for discharge from medical care, when the amount of the one or more biomarker is statistically similar to the amount determined for the corresponding reference value representative of a healthy individual (or a population of healthy individuals). In one embodiment, a patient may be identified as being unsuitable for discharge from medical care when the amount of the one or more biomarker is statistically similar to the amount determined for the corresponding reference value representative of an individual (or a population of individuals) having a prognosis of non-recovery (or non-survival) from a systemic inflammatory condition.

In one embodiment, a patient may be identified as being unsuitable for discharge from medical care, when the amount of the one or more biomarker statistically deviates from the amount determined for the corresponding reference value representative of an individual (or a population of individuals) that has a (good) prognosis of recovery (or survival) from a systemic inflammatory condition. In one embodiment, a patient may be identified as being unsuitable for discharge from medical care, when the amount of the one or more biomarker statistically deviates from the amount determined for the corresponding reference value representative of a healthy individual (or a population of healthy individuals). In one embodiment, a patient may be identified as being suitable for discharge from medical care when the amount of the one or more biomarker statistically deviates from the amount determined for the corresponding reference value representative of an individual (or a population of individuals) that has a prognosis of non-recovery (or non-survival) from a systemic inflammatory condition.

All embodiments described above (in the context of the diagnostic methods) for classifying a patient based on their marker profile apply equally to the method for determining whether a patient is suitable for discharge from medical care. This includes all embodiments for determining whether the marker profile of the patient is "statistically similar to" or "statistically deviates from" the marker profiles observed for the corresponding reference values, and all embodiments relating to the % increase or % decrease or fold change observed in the markers as compared to the corresponding reference value.

The reference value may be as defined above for the diagnostic methods described herein. In one embodiment, the reference value is representative of a healthy individual (or a population of healthy individuals). In one embodiment, the reference value may be representative of an individual (or population of individuals) that has a (good) prognosis of recovery (or survival) from a systemic inflammatory condition. In one embodiment, the reference value may be representative of an individual (or population of individuals) that has a prognosis of non-recovery (or non-survival) from a systemic inflammatory condition).

The reference value that is representative of an individual (or population of individuals) having a (good) prognosis of recovery (or survival) from a systemic inflammatory condition is determined by quantifying the amount of the one or more biomarker in a sample obtained from an individual (or population of individuals) having a systemic inflammatory condition, wherein the individual (or population of individuals) goes on to make a full recovery from the systemic inflammatory condition. The sample may be obtained at least 1 hour, 2 hours, 4 hours, 6 hours, 8 hours, 12 hours, 36 hours, 48 hours, 72 hours, 96 hours, or 120 hours, after the individual presents with one or more clinical symptoms of a systemic inflammatory condition, or is admitted to a medical care facility, For example, the sample may be obtained at least 120 hours after the individual (or population of individuals) presents with one or more clinical symptoms of a systemic inflammatory condition, or is admitted to a medical care facility.

The reference value that is representative of an individual (or population of individuals) having a prognosis of non-recovery (or non-survival) from a systemic inflammatory condition, or a poor prognosis of recovery (or survival) is determined by quantifying the amount of biomarker in a sample obtained from an individual (or population of individuals) having a systemic inflammatory condition, wherein the individual (or population of individuals) does not recover from the systemic inflammatory condition. The sample may be obtained at least 1 hour, 2 hours, 4 hours, 6 hours, 8 hours, 12 hours, 36 hours, 48 hours, 72 hours, 96 hours, or 120 hours, after the individual presents with one or more clinical symptoms of a systemic inflammatory condition, or is admitted to a medical care facility. For example, the sample may be obtained at least 120 hours after the individual (or population of individuals) presents with one or more clinical symptoms of a systemic inflammatory condition, or is admitted to a medical care facility.

The present inventors observed that some of the 'survival' biomarkers described herein increase in abundance in non-survivors as compared to healthy individuals. Detection of increased levels of these biomarkers in a patient as compared to the levels detected for healthy individuals can thus be used to determine whether a patient is suitable for discharge from medical care.

In one embodiment, when the reference value is representative of a healthy individual (or a population of healthy individuals), an increase in the one or more biomarker in the sample obtained from the patient relative to the corresponding reference value representative of a healthy individual, indicates that the patient is not suitable for discharge from medical care. Likewise, no increase in the one or more biomarker in the sample obtained from the patient relative to the corresponding reference value representative of a healthy individual, indicates that the patient is suitable for discharge from medical care.

For some of the 'survival' biomarkers identified by the present inventors, increased levels of these markers were also observed in patients that recovered from a systemic inflammatory condition as compared to healthy individuals, although much bigger increases were observed for these biomarkers in the patients that did not survive. The accuracy of determining whether a patient is suitable for discharge from medical care can thus be improved by looking for a "minimum" fold change in the levels of the one or more biomarkers as compared to the corresponding reference value that is representative of a healthy individual.

For example, an increase of at least 2.5 (eg. at least 2.6, at least 2.7, at least 2.8, at least 2.9, at least 3, at least 3.1, at least 3.2, at least 3.3, at least 3.4, at least 3.5, at least 3.6, at least 3.7, at least 3.8, at least 3.9, at least 4) fold in NECAB1 in the sample obtained from the patient relative to the corresponding reference value representative of a healthy individual, indicates that the patient is not suitable for discharge from medical care. No increase or an increase of less than 1.5 (eg. less than 1.4, less than 1.3, less than 1.2, less than 1.1, less than 1) fold in NECAB1 in the sample obtained from the patient relative to the corresponding reference value representative of a healthy individual, indicates that the patient is suitable for discharge from medical care. In one embodiment, the patient is undergoing (or has undergone) treatment for SIRS. In one embodiment, the sample is obtained from the patient at least 72 hours (eg. at least 96 hours, at least 120 hours) after the patient presents with one or more clinical symptoms of a systemic inflammatory condition, or is admitted to a medical care facility.

For example, an increase of at least 3.5 (eg. at least 3.6, at least 3.7) fold in NECAB2 in the sample obtained from the patient relative to the corresponding reference value representative of a healthy individual, indicates that the patient is not suitable for discharge from medical care. No increase or an increase of less than 2.5 (eg. less than 2.4, less than 2.3, less than 2.2, less than 2.1, less than 2, less than 1.5, less than 1.4, less than 1.3, less than 1.2, less than 1.1, less than 1) fold in NECAB2 in the sample obtained from the patient relative to the corresponding reference value representative of a healthy individual, indicates that the patient is suitable for discharge from medical care. In one embodiment, the patient is undergoing (or has undergone) treatment for sepsis. In one embodiment, the patient is undergoing (or has undergone) treatment for abdominal sepsis and/or pulmonary sepsis. In one embodiment, the sample is obtained from the patient at least 48 hours (eg. at least 72 hours, at least 96 hours, at least 120 hours) after the patient presents with one or more clinical symptoms of a systemic inflammatory condition, or is admitted to a medical care facility.

For example, an increase of at least 1.5 (eg. at least 1.6, at least 1.7, at least 1.8, at least 1.9, at least 2, at least 2.1, at least 2.2) fold in PKD1 in the sample obtained from the patient relative to the corresponding reference value representative of a healthy individual, indicates that the patient is not suitable for discharge from medical care. No increase or an increase of less than 1.6 (eg. less than 1.5, less than 1.4, less than 1.3, less than 1.2, less than 1.1, less than 1) fold in PKD1 in the sample obtained from the patient relative to the corresponding reference value representative of a healthy individual, indicates that the patient is suitable for discharge from medical care. In one embodiment, the patient is undergoing (or has undergone) treatment for abdominal sepsis and/or SIRS. In one embodiment, the sample is obtained from the patient at least 72 hours (eg. at least 96 hours, at least 120 hours) after the patient presents with one or more clinical symptoms of a systemic inflammatory condition, or is admitted to a medical care facility.

For example, an increase of at least 2.5 (eg. at least 3, at least 3.5, at least 4, at least 4.5, at least 5, at least 5.1, at least 5.2, or at least 5.3) fold in PKHD1 in the sample obtained from the patient relative to the corresponding reference value representative of a healthy individual, indicates that the patient is not suitable for discharge from medical care. No increase or an increase of less than 1.5 (eg. less than 1.4, less than 1.3, less than 1.2, less than 1.1, less than 1) fold in PKHD1 in the sample obtained from the patient relative to the corresponding reference value representative of a healthy individual, indicates that the patient is suitable for discharge from medical care. In one embodiment, the patient is undergoing (or has undergone) treatment for SIRS. In one embodiment, the sample is obtained from the patient at least 72 hours (eg. at least 96 hours, at least 120 hours) after the patient presents with one or more clinical symptoms of a systemic inflammatory condition, or is admitted to a medical care facility.

For example, an increase of at least 6.3 (eg. at least 6.4, at least 6.5, at least 6.6, at least 6.7, at least 6.8, at least 6.9, at least 7) fold in LILRB4 in the sample obtained from the patient relative to the corresponding reference value representative of a healthy individual, indicates that the patient is not suitable for discharge from medical care. No increase or an increase of less 5.5 (eg. less than 5.4, less than 5.3, less than 5.2, less than 5.1, less than 5) fold in LILRB4 in the sample obtained from the patient relative to the corresponding reference value representative of a healthy individual, indicates that the patient is suitable for discharge from medical care. In one embodiment, the patient is undergoing (or has undergone) treatment for SIRS. In one embodiment, the sample is obtained from the patient at least 72 hours (eg. at least 96 hours, at least 120 hours) after the patient presents with one or more clinical symptoms of a systemic inflammatory condition, or is admitted to a medical care facility.

For example, an increase of at least 7 (eg. at least 7.1, at least 7.2, at least 7.3 at least 7.4, at least 7.5, at least 7.6, at least 7.7, at least 7.8, at least 7.9, at least 8) fold in LILRB5 in the sample obtained from the patient relative to the corresponding reference value representative of a healthy individual, indicates that the patient is not suitable for discharge from medical care. No increase or an increase of less than 4.5 (eg. less than 5, less than 5.5, less than 6, less than 6.5, less than 7) fold in LILRB5 in the sample obtained from the patient relative to the corresponding reference value representative of a healthy individual, indicates that the patient is suitable for discharge from medical care. In one embodiment, the patient is undergoing (or has undergone) treatment for SIRS. In one embodiment, the sample is obtained from the patient at least 72 hours (eg. at least 96 hours, at least 120 hours) after the patient presents with one or more clinical symptoms of a systemic inflammatory condition, or is admitted to a medical care facility.

As described herein, LILRB5 was also observed to increase in abundance in patients that made a full recovery from pulmonary sepsis compared to patients that did not survive. Thus, in one example, an increase of at least 2.5 (e.g at least 3, at least 3.5, at least 4, at least 4.5, at least 5) fold in LILRB5 in the sample obtained from the patient relative to the corresponding reference value representative of a healthy individual, indicates that the patient is suitable for discharge from medical care. No increase or an increase of less than 4.5 (eg. less than 4, less than 3.5, less than 3, less than 2.5, less than 2) fold in LILRB5 in the sample obtained from the patient relative to the corresponding reference value representative of a healthy individual, indicates that the patient is not suitable for discharge from medical care. In one embodiment, the patient is undergoing (or has undergone) treatment for pulmonary sepsis. In one embodiment, the sample is obtained from the patient at least 72 hours (e.g at least 96 hours or at least 120 hours) after the patient presents with one or more clinical symptoms of a systemic inflammatory condition, or is admitted to a medical care facility.

As described herein, the present inventors observed that the levels of some of the one or more survival biomarkers were elevated in patients that did not recover from (or survive) a systemic inflammatory condition as compared to patients that made a full recovery. Detection of increased levels of these biomarkers in a patient as compared to the levels detected for patients that recovered from (or survived) a systemic inflammatory condition can thus be used to determine whether a patient is suitable for discharge from medical care.

Thus, in one embodiment, when the reference value is representative of an individual (or population of individuals) having a (good) prognosis of recovery (or survival) from a systemic inflammatory condition, an increase in the one or more biomarker in the sample obtained from the patient relative to the corresponding reference value, indicates that the patient is not suitable for discharge from medical care. Likewise, no increase in the one or more biomarker in the sample obtained from the patient relative to the corresponding reference value, indicates that the patient is suitable for discharge from medical care.

In one embodiment, the patient may be identified as being unsuitable for discharge from medical care, when the one or more biomarker increases by at least 1 (e.g. at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value.

As described herein, the present inventors observed that the levels of some of the one or more survival biomarkers were elevated in patients that made a full recovery from a systemic inflammatory condition as compared to patients that did not recover from (or survive). Detection of increased levels of these biomarkers in a patient as compared to the levels detected for patients that recovered from (or survived) a systemic inflammatory condition can thus be used to determine whether a patient is suitable for discharge from medical care.

Thus, in one embodiment, when the reference value is representative of an individual (or population of individuals) having a (good) prognosis of recovery (or survival) from a systemic inflammatory condition, an increase in the one or more biomarker in the sample obtained from the patient relative to the corresponding reference value, indicates that the patient is suitable for discharge from medical care. Likewise, no increase in the one or more biomarker in the sample obtained from the patient relative to the corresponding reference value, indicates that the patient is not suitable for discharge from medical care.

In one embodiment, the patient may be identified as being suitable for discharge from medical care, when the one or more biomarker increases by at least 1 (e.g. at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value.

As described above for the diagnostic methods described herein, the method of the invention may involve the use of multiple separate reference values. For example, the method may involve the use of one or more (eg. two or more, or all three) reference values that are representative of an individual (or population of individuals) having a (good) prognosis of recovery (or survival) from a systemic inflammatory condition, an individual (or population of individuals) having a prognosis of non-recovery (or non-survival) (or a poor prognosis of recovery) from a systemic inflammatory condition; and a healthy individual (or a population of healthy individuals).

In a related aspect, the present invention also provides the use of one or more biomarker selected from: NECAB1, NECAB2, PKD1, PKHD1, LILRB4 and LILRB5 for determining whether a patient is suitable for discharge from medical care.

In one embodiment, the present invention provides the use of one or more biomarker selected from: PKHD1, PKDI, NECAB1, LILRB4, and LILRB5 for determining whether a patient is suitable for discharge from medical care. The patient may beundergoing (or has undergone) treatment for SIRS. In one embodiment, the one or more biomarker may be selected from: PKHD1 and NECAB1. For example, the one or more biomarker may comprise the combination of PKHD1 and NECAB1. For example, the one or more biomarker is PKHD1. For example, the one or more biomarker is NECAB1.

In one embodiment, the present invention provides the use of one or more biomarker selected from the group consisting of NECAB2, LILRB5, PKHD1 and PKD1 for determining whether a patient is suitable for discharge from medical care. The patient may be undergoing (or has undergone) treatment for sepsis (such as abdominal sepsis and/or pulmonary sepsis).

In one embodiment, the present invention provides the use of one or more biomarker selected from: NECAB2 and PKD1, for determining whether a patient is suitable for discharge from medical care. The patient may be undergoing (or has undergone) treatment for abdominal sepsis. For example, the one or more biomarker may comprise the combination of biomarkers NECAB2 and PKD1. For example, the one or more biomarker is NECAB2. For example, the one or more biomarker is PKD1.

In one embodiment, the present invention provides the use of one or more biomarker selected from: PKHD1 and LILRB5, for determining whether a patient is suitable for discharge from medical care. The patient may be undergoing (or has undergone) treatment for pulmonary sepsis. For example, the one or more biomarker may comprise the combination of biomarkers PKHD1 and LILRB5. For example, the one or more biomarker is PKHD1. For example, the one or more biomarker is LILRB5.

All embodiments described above for the method of determining whether a patient is suitable for discharge from medical care apply equally to the 'use' of the invention described herein. This includes all embodiments relating to the terms "systemic inflammatory condition", "patient", "sample", and "the one or more biomarker".

Systemic inflammatory conditions such as SIRS and sepsis can lead to the development of multiple organ failure in patients. Early detection of organ failure in patients may improve the chances of survival in patients having a systemic inflammatory condition.

When investigating the biomarkers associated with systemic inflammatory conditions, the present inventors surprisingly observed that various organ specific biomarkers are present in high levels in peripheral blood leukocytes (PBLs) obtained from patients having systemic inflammatory conditions that did not survive. These biomarkers include the brain specific markers NECAB1 and NECAB2, and the kidney specific markers PKHD1 and PKD1. The presence of these markers in peripheral blood leukocytes indicates that the organ is damaged. Detection of these markers in samples obtained from patients therefore provides a way of diagnosing organ damage in the patient.

The present invention provides a method for diagnosing organ damage in a patient, comprising:
(i) determining the presence (and/or amount) of one or more biomarker selected from: NECAB1, NECAB2, PKHD1, and PKD1, in a sample obtained from a patient,
(ii) comparing the presence (and/or amount) of the one or more biomarker determined in said sample in (i) to a corresponding reference value; and thereby determining the patient has or is at risk of developing organ damage.

All embodiments described above for the method of determining whether a patient is suitable for discharge from medical care apply equally to the method for diagnosing organ damage in a patient. This includes all embodiments relating to the "patient", "sample", "reference value", and the steps for "determining the presence and/or amount of the one or more biomarker" and the "comparison" for making a conclusion about the diseases state of the patient.

The term "organ damage" refers to the condition where an organ has been injured such that it does not perform its expected function. In one embodiment the organ damage is one or more of: brain damage or kidney damage.

The "patient" is as described above for the "method of determining whether a patient is suitable for discharge from medical care". The method of the invention for diagnosing organ damage is not only applicable to such patients, but may also be used to diagnose organ damage in patients having a disease or condition other than a systemic inflammatory condition. The patient may thus be an individual having any disease, condition or injury which may result in organ damage.

The "one or more biomarker" of the invention is as described above for the "method of determining whether a patient is suitable for discharge from medical care". In one embodiment, the one or more biomarker is selected from the group consisting of: NECAB1, NECAB2, PKHD1, and PKD1.

In one embodiment, the one or more biomarker is selected from NECAB1 and/or NECAB2. These biomarkers are specific for indicating the presence of brain damage in a patient. Thus when the one or more biomarker is selected from NECAB1 and/or NECAB2, the method is for diagnosing brain damage in a patient. By performing steps (i) and (ii) of the method described herein, the method can be used to determine whether a patient has or is at risk of developing brain damage.

In one embodiment, the one or more biomarker is selected from PKHD1 and/or PKD1. These biomarkers are specific for indicating the presence of kidney damage in a patient. Thus when the one or more biomarker is selected from PKHD1 and/or PKD1, the method is for diagnosing kidney damage in a patient. By performing steps (i) and (ii) of the method described herein, the method can be used to determine whether a patient has or is at risk of developing kidney damage.

Each of the biomarkers may be used alone, or in combination with any of the biomarkers described herein in the method of the invention. For example, any combination of 1 or more, 2 or more, 3 or more, or all 4 or more of the biomarkers may be used in the method of the invention.

In one embodiment, any combination of 1 or more (eg. 2 or more, 3 or more, or all 4) of the biomarkers selected from the group consisting of: NECAB1, NECAB2, PKD1, and PKHD1, may be used to diagnose organ damage in a patient.

The 'quantification' and 'comparison' steps of the method, and the "reference value" used in the 'comparison' step are as described above for the method for determining whether a patient is suitable for discharge from medical care. This includes all embodiments described for classification of a patient based on their marker profile.

As described herein, the present inventors observed that the organ specific biomarkers described herein each increase in abundance in samples obtained from patients having a systemic inflammatory condition as compared to healthy individuals. However, much higher levels of the organ specific biomarkers were observed in patients that did not survive the systemic inflammatory condition as compared to those patients that recovered.

In one embodiment, the reference value is representative of a healthy individual (or a population of healthy individuals). The patient may be diagnosed as having organ damage or being at risk of developing organ damage when an increase is observed in the one or more biomarker in the sample obtained from the patient relative to the corresponding reference value representative of a healthy individual. More accurate diagnosis can be performed by looking for a minimum fold increase in the one or more biomarker in the patient. The minimum fold change values in the biomarkers NECAB1, NECAB2, PKD1 and PKHD1 are as defined above for the method for determining whether a patient is suitable for discharge from medical care.

As described herein, the present inventors observed that the levels of the organ specific biomarkers were elevated in patients that did not recover from (or survive) a systemic inflammatory condition as compared to patients that made a full recovery. The patients that did not survive are likely to have a higher risk of organ failure as compared to patients that made a full recovery. Detection of increased levels of these biomarkers in a patient as compared to the levels detected for patients that recovered from (or survived) a systemic inflammatory condition can thus be used to determine whether a patient has organ damage.

Thus, in one embodiment, when the reference value is representative of an individual (or population of individuals) having a (good) prognosis of recovery (or survival) from a systemic inflammatory condition, an increase in the one or more biomarker in the sample obtained from the patient relative to the corresponding reference value, indicates that the patient has or is at risk of developing organ damage.

In one embodiment, the patient may be diagnosed as having or being at risk of developing organ damage, when the one or more biomarker increases by at least 1 (e.g. at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5, at least 10, at least 15 fold, at least 20 fold, at least 30 fold, at least 40 fold, at least 50) fold in the sample obtained from the patient relative to the corresponding reference value.

As described above for the method for determining whether a patient is suitable for discharge from medical care, multiple separate reference values may be used in the method for diagnosing organ damage. All combinations of reference values described above apply equally to the method for diagnosing organ damage.

In a related aspect, the present invention also provides the use of one or more of: NECAB1, NECAB2, PKHD1, and PKD1, as a biomarker for organ damage. In one embodiment, the use is of the one or more biomarker for diagnosis of organ damage in a patient.

In one embodiment, the use is of one or more biomarker selected from the group consisting of: NECAB1 and NECAB2, for diagnosis of brain damage in a patient. In one embodiment, the use is of one or more biomarker selected from the group consisting of: PKHD1 and PKD1, for diagnosis of kidney damage in a patient.

All embodiments described above for the method of diagnosing organ damage in a patient apply equally to the 'use' of the invention described herein. This includes all embodiments relating to the "patient", "sample", and "the one or more biomarker".

### Treatment

The methods described herein for diagnosis and/or monitoring of a systemic inflammatory condition in a patient may in certain embodiments also be applied to determine whether the patient is or is not in need of a therapeutic or prophylactic treatment of the systemic inflammatory condition. For example, a treatment may be indicated where the methods allow for a conclusion that the patient has or is at risk of having a systemic inflammatory condition, has a poor prognosis for the systemic inflammatory condition, displays a detrimental development of the condition, or has organ damage. Without limitation, a patient with the systemic inflammatory condition upon admission to or during stay in a medical care centre such as ICU may be tested as described herein for the necessity of continuing the treatment of the condition, and may be discharged when such treatment is no longer needed or is needed only to a given limited extent.

In a further embodiment, any of the methods described herein may further comprise treating a systemic inflammatory condition in a patient. In one embodiment, any of the methods described herein may comprise, responsive to the diagnosis of a systemic inflammatory condition in the patient, administering to the patient a therapy for a systemic inflammatory condition. For example, the therapy may be for SIRS and/or for sepsis. The methods of the invention may therefore be for treating or preventing one or more symptoms of a systemic inflammatory condition.

For example, any of the methods described herein for diagnosis of SIRS (including the method for diagnosing SIRS in a patient, and the method for distinguishing between sepsis and SIRS in a patient) may further comprise, responsive to the diagnosis of SIRS, administering to the patient a therapy for SIRS. These methods may be for treating or preventing one or more symptoms of SIRS in a patient.

The "therapy for SIRS" may include: organ support with oxygen, mechanical ventilation, circulatory support with fluid resuscitation, vasodilators, inotropes or vasopressors, renal replacement therapy.

In certain embodiments, the administering of a therapy for SIRS may comprise administering one such therapy to the patient. In certain embodiments, the administering of a therapy for SIRS may comprise administering a combination of two or more such therapies to the patient.

For example, any of the methods described herein for diagnosis of sepsis (including the method for diagnosing sepsis in a patient, the method for distinguishing between sepsis and SIRS in a patient, the method for diagnosing abdominal sepsis in a patient, the method for diagnosing pulmonary sepsis in a patient, and the method for distinguishing between abdominal sepsis and pulmonary sepsis in a patient) may further comprise, responsive to the diagnosis of sepsis, administering to the patient a therapy for sepsis. These methods may be for treating or preventing one or more symptoms of sepsis in a patient.

The "therapy for sepsis" may include anti-microbial agents (such as anti-bacterial agents e.g. antibiotics), analgesics, antipyretics, anti-inflammatory drugs (such as non-steroidal anti-inflammatory drugs), fluid resuscitation, and oxygen therapy. It may also include organ support with oxygen, mechanical ventilation, circulatory support with inotropes or vasopressors, renal replacement therapy.

In certain embodiments, the administering of a therapy for sepsis may comprise administering one such therapy to the patient. In certain embodiments, the administering of a therapy for sepsis may comprise administering a combination of two or more such therapies to the patient.

In one embodiment, the method for distinguishing between sepsis and SIRS in a patient may further comprise, responsive to the diagnosis of sepsis and/or SIRS in the patient, administering to the patient a therapy for sepsis and/or SIRS. For example, the therapy may be for SIRS as described herein. Alternatively, the therapy may be for sepsis (including abdominal sepsis and pulmonary sepsis) as described herein. The methods of the invention may therefore be for treating or preventing one or more symptoms of sepsis and/or SIRS.

In one embodiment, any of the methods described herein for diagnosis of organ damage may further comprise, responsive to the diagnosis of organ damage, administering to the patient a therapy for organ damage.

### Oligonucleotide probes and amplification primers

Any appropriate detection means can be used to detect or quantify the one or more biomarker in the methods and uses of the invention, as described herein.

Typically when the one or more biomarker of the invention is a nucleic acid, the presence of the one or more biomarker may be detected, and/or the amount of the one or more biomarker determined using an oligonucleotide probe. The methods and uses described herein may therefore use any one or more oligonucleotide probe as defined herein to detect and/or quantify the one or more biomarker of the invention. The oligonucleotide probes may be bound to a solid surface (such as a microarray). Alternatively oligonucleotide probes may be used in quantitiative real-time PCR to detect amplified target sequence from the one or more biomarker.

An oligonucleotide probe of the invention may have at least 80% sequence identity to the one or more biomarker of the invention, or a target region within said biomarker, measured over any appropriate length of sequence. Typically the % sequence identity is determined over a length of contiguous nucleic acid residues. An oligonucleotide probe of the invention may, for example, have at least 80% sequence identity to the one or more biomarker of the invention, or target region thereof, measured over at least 10, at least 20, at least 30, at least 40, at least 50, at least 60, at least 70, at least 80, at least 90, or more nucleic acid residues, up to the oligonucleotide probe having at least 80% sequence identity with the one or more biomarker of the invention, or target region thereof, over the entire length of the oligonucleotide probe.

An oligonucleotide probe of the invention may be complementary to the one or more nucleic acid biomarker of the invention, or a target region thereof. Typically the oligonucleotide probe of the invention is complementary over a length of contiguous nucleic acid residues. An oligonucleotide probe of the invention may, for example, be complementary to the one or more biomarker of the invention, or target region thereof, measured over at least 10, at least 20, at least 30, at least 40, at least 50, at least 60, at least 70, at least 80, at least 90, or more nucleic acid residues, up to the oligonucleotide probe having being complementary to the one or more biomarker of the invention, or target region thereof, over the entire length of the oligonucleotide probe.

An oligonucleotide probe of the invention may be complementary to a variant of the one or more biomarker of the invention, or a variant of a target region of said biomarker. Typically the oligonucleotide probe is complementary to a variant having at least 80% sequence identity to the one or more biomarker of the invention, or a variant having at least 80% sequence identity to the target region of said biomarker. The % sequence identity of the variant to the one or more biomarker of the invention, or a variant of a target region of said biomarker may be calculated over any appropriate length of sequence in the one or more biomarker, as described herein.

As used herein, a "sequence identity of at least 80%" includes at least 82%, at least 84%, at least 86%, at least 88%, at least 90%, at least 91%, at least 92%, at least 93%, at least 94%, at least 95%, at least 96%, at least 97%, at least 98%, at least 99%, and 100% sequence identity (to each and every nucleic acid sequence presented herein and/ or to each and every SEQ ID NO presented herein).

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position-Specific Gap Penalties and Weight Matrix Choice, 22 (22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262 (5131 ) Science 208-214 (1993); Align-M, see, e.g., Ivo Van Walle et al., Align-M - A New Algorithm for Multiple Alignment of Highly Divergent Sequences, 20 (9) Bioinformatics:1428-1435 (2004). Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992.

Variants of the specific sequences described herein may alternatively be defined by reciting the number of nucleotides that differ between the variant sequences and the specific reference sequences provided above. Thus, in one embodiment, the sequence may comprise (or consist of) a nucleotide sequence that differs from the specific sequences provided above at no more than 2 nucleotide positions, for example at no more than 1 nucleotide position. Conservative substitutions are preferred. The term variants as defined herein also encompasses splice variants.

An oligonucleotide probe of the invention may be at least 30, at least 40, at least 50, at least 60, at least 70, at least 80, at least 90, at least 100, or more nucleotides in length. In a preferred embodiment, the oligonucleotide probe is 40 to 100 nucleotides in length, more preferably 50 to 100 nucleotides in length, even more preferably 50 to 80 nucleotides in length and most preferably 50 to 70 nucleotides in length. Such oligonucleotide probes are suitable for use in use in microarray analysis when bound to a solid surface. In one embodiment, the oligonucleotide probe is designed for detection of the one or more biomarker by microarray analysis.

Oligonucleotide probes may also be designed for detection of the one or more biomarker by quantitative PCR (or real-time PCR). The oligonucleotide probe may be 5-30 nucleotides long, such as at least 6, 7, 8, 9 or 10 nucleotides long. The oligonucleotide probe may be up to 25 nucleotides long, such as up to 20, 18, 16, 15, 14, 13, 12, 11 or 10 nucleotides long. The oligonucleotide probe may be 10-25 nucleotides long, such as 10-20 nucleotides long or 10-15 nucleotides long, and may be preferably about 10 nucleotides long. In this regard, the use of short probes enables faster annealing to the target nucleic acid.

The target nucleotide sequence to which the oligonucleotide probe hybridises within the amplification product may be at least 5, 6, 7, 8, 9 or 10 nucleotides long. The target sequence for the probe may be up to 30 nucleotides long, such as up to 25, 20, 18, 16, 15, 14, 13, 12, or 11 nucleotides long. The probe target sequence may be 10-25 nucleotides long or 10-15 nucleotides long, and may be preferably about 10 nucleotides long.

The probes of the invention are typically designed to hybridise to their target nucleic acid sequence present in the one or more biomarker of the invention.

A probe may comprise or be complementary to a nucleic acid sequence within a target nucleic acid sequence from the one or more biomarker of the invention, or to a nucleic acid sequence having at least 80% identity to said target nucleic acid sequence. Any suitable probe which comprises or is complementary (as defined herein) to a nucleic acid sequence within a target nucleic acid sequence of one or more biomarker of the invention may be used.

In embodiments wherein the one or more biomarker is ADM, a target nucleic acid sequence may comprise bases 751 to 1590 of SEQ ID NO: 1, and a probe typically comprises or is complementary to a nucleic acid sequence having at least 80% sequence identity to a nucleic acid sequence from this target sequence.

In embodiments wherein the one or more biomarker is CD177, a target nucleic acid sequence may comprise bases 1351 to 2220 of SEQ ID NO: 2, and a probe typically comprises or is complementary to a nucleic acid sequence having at least 80% sequence identity to a nucleic acid sequence from this target sequence.

In embodiments wherein the one or more biomarker is FAM20A, a target nucleic acid sequence may comprise bases 1331 to 3700 of SEQ ID NO: 3, and a probe typically comprises or is complementary to a nucleic acid sequence having at least 80% sequence identity to a nucleic acid sequence from this target sequence. For example, a target nucleic acid sequence may comprise bases 1460 to 1531 of SEQ ID NO: 3 or bases 1486 to 1551 of SEQ ID NO: 3, and a probe typically comprises or is complementary to a nucleic acid sequence having at least 80% sequence identity to a nucleic acid sequence from this target sequence.

In embodiments wherein the one or more biomarker is IL10, a target nucleic acid sequence may comprise bases 61 to 1320 of SEQ ID NO: 4, and a probe typically comprises or is complementary to a nucleic acid sequence having at least 80% sequence identity to a nucleic acid sequence from this target sequence.

In embodiments wherein the one or more biomarker is METTL7B, a target nucleic acid sequence may comprise bases 581 to 1340 of SEQ ID NO: 5, and a probe typically comprises or is complementary to a nucleic acid sequence having at least 80% sequence identity to a nucleic acid sequence from this target sequence.

In embodiments wherein the one or more biomarker is MMP9, a target nucleic acid sequence may comprise bases 1511 to 2330 of SEQ ID NO: 6, and a probe typically comprises or is complementary to a nucleic acid sequence having at least 80% sequence identity to a nucleic acid sequence from this target sequence.

In embodiments wherein the one or more biomarker is RETN, a target nucleic acid sequence may comprise bases 81 to 478 of SEQ ID NO: 7, and a probe typically comprises or is complementary to a nucleic acid sequence having at least 80% sequence identity to a nucleic acid sequence from this target sequence.

In embodiments wherein the one or more biomarker is TDRD9, a target nucleic acid sequence may comprise bases 3711 to 4400 of SEQ ID NO: 8, and a probe typically comprises or is complementary to a nucleic acid sequence having at least 80% sequence identity to a nucleic acid sequence from this target sequence.

In embodiments wherein the one or more biomarker is ITGA7, a target nucleic acid sequence may comprise bases 3181 to 4080 of SEQ ID NO: 9, and a probe typically comprises or is complementary to a nucleic acid sequence having at least 80% sequence identity to a nucleic acid sequence from this target sequence.

In embodiments wherein the one or more biomarker is BMX, a target nucleic acid sequence may comprise bases 1651 to 2430 of SEQ ID NO: 10, and a probe typically comprises or is complementary to a nucleic acid sequence having at least 80% sequence identity to a nucleic acid sequence from this target sequence.

In embodiments wherein the one or more biomarker is HP, a target nucleic acid sequence may comprise bases 821 to 1430 of SEQ ID NO: 11, and a probe typically comprises or is complementary to a nucleic acid sequence having at least 80% sequence identity to a nucleic acid sequence from this target sequence.

In embodiments wherein the one or more biomarker is IGFBP2, a target nucleic acid sequence may comprise bases 651 to 1430 of SEQ ID NO: 12, and a probe typically comprises or is complementary to a nucleic acid sequence having at least 80% sequence identity to a nucleic acid sequence from this target sequence.

In embodiments wherein the one or more biomarker is ALPL, a target nucleic acid sequence may comprise bases 1441 to 2520 of SEQ ID NO: 13, and a probe typically comprises or is complementary to a nucleic acid sequence having at least 80% sequence identity to a nucleic acid sequence from this target sequence.

In embodiments wherein the one or more biomarker is DACH1, a target nucleic acid sequence may comprise bases 2341 to 4990 of SEQ ID NO: 14, and a probe typically comprises or is complementary to a nucleic acid sequence having at least 80% sequence identity to a nucleic acid sequence from this target sequence.

In embodiments wherein the one or more biomarker is IL1R1, a target nucleic acid sequence may comprise bases 1551 to 4410 of SEQ ID NO: 15, and a probe typically comprises or is complementary to a nucleic acid sequence having at least 80% sequence identity to a nucleic acid sequence from this target sequence.

In embodiments wherein the one or more biomarker is OLAH, a target nucleic acid sequence may comprise bases 781 to 1480 of SEQ ID NO: 16, and a probe typically comprises or is complementary to a nucleic acid sequence having at least 80% sequence identity to a nucleic acid sequence from this target sequence. For example, a target nucleic acid sequence may comprise bases 901 to 960 of SEQ ID NO: 16, and a probe typically comprises or is complementary to a nucleic acid sequence having at least 80% sequence identity to a nucleic acid sequence from this target sequence. In an alternative embodiment, a target nucleic acid sequence may comprise bases 632 to 697 of SEQ ID NO: 16, and a probe typically comprises or is complementary to a nucleic acid sequence having at least 80% sequence identity to a nucleic acid sequence from this target sequence.

In embodiments wherein the one or more biomarker is IL1R2, a target nucleic acid sequence may comprise bases 681 to 1310 of SEQ ID NO: 17, and a probe typically comprises or is complementary to a nucleic acid sequence having at least 80% sequence identity to a nucleic acid sequence from this target sequence.

In embodiments wherein the one or more biomarker is CYP19A1, a target nucleic acid sequence may comprise bases 441 to 4520 of SEQ ID NO: 18, and a probe typically comprises or is complementary to a nucleic acid sequence having at least 80% sequence identity to a nucleic acid sequence from this target sequence.

In embodiments wherein the one or more biomarker is MMP8, a target nucleic acid sequence may comprise bases 1621 to 2900 of SEQ ID NO: 19, and a probe typically comprises or is complementary to a nucleic acid sequence having at least 80% sequence identity to a nucleic acid sequence from this target sequence.

In embodiments wherein the one or more biomarker is TGFA, a target nucleic acid sequence may comprise bases 3321 to 4110 of SEQ ID NO: 20, and a probe typically comprises or is complementary to a nucleic acid sequence having at least 80% sequence identity to a nucleic acid sequence from this target sequence.

In embodiments wherein the one or more biomarker is VSTM1, a target nucleic acid sequence may comprise bases 271 to 990 of SEQ ID NO: 21, and a probe typically comprises or is complementary to a nucleic acid sequence having at least 80% sequence identity to a nucleic acid sequence from this target sequence.

In embodiments wherein the one or more biomarker is FCER1A, a target nucleic acid sequence may comprise bases 141 to 1110 of SEQ ID NO: 22, and a probe typically comprises or is complementary to a nucleic acid sequence having at least 80% sequence identity to a nucleic acid sequence from this target sequence. For example, a target nucleic acid sequence may comprise bases 648 to 709 of SEQ ID NO: 22, and a probe typically comprises or is complementary to a nucleic acid sequence having at least 80% sequence identity to a nucleic acid sequence from this target sequence. In an alternative embodiment, a target nucleic acid sequence may comprise bases 36 to 100 of SEQ ID NO: 22, and a probe typically comprises or is complementary to a nucleic acid sequence having at least 80% sequence identity to a nucleic acid sequence from this target sequence.

In embodiments wherein the one or more biomarker is KLRK1, a target nucleic acid sequence may comprise bases 341 to 1590 of SEQ ID NO: 23, and a probe typically comprises or is complementary to a nucleic acid sequence having at least 80% sequence identity to a nucleic acid sequence from this target sequence.

In embodiments wherein the one or more biomarker is KLRB1, a target nucleic acid sequence may comprise bases 81 to 740 of SEQ ID NO: 24, and a probe typically comprises or is complementary to a nucleic acid sequence having at least 80% sequence identity to a nucleic acid sequence from this target sequence. For example, a target nucleic acid sequence may comprise bases 219 to 291 or 297 to 370 of SEQ ID NO: 24, and a probe typically comprises or is complementary to a nucleic acid sequence having at least 80% sequence identity to a nucleic acid sequence from this target sequence.

In embodiments wherein the one or more biomarker is DAAM2, a target nucleic acid sequence may comprise bases 5131 to 6160 of SEQ ID NO: 25, and a probe typically comprises or is complementary to a nucleic acid sequence having at least 80% sequence identity to a nucleic acid sequence from this target sequence.

In embodiments wherein the one or more biomarker is HLA-DRA, a target nucleic acid sequence may comprise bases 561 to 1210 of SEQ ID NO: 26, and a probe typically comprises or is complementary to a nucleic acid sequence having at least 80% sequence identity to a nucleic acid sequence from this target sequence.

In embodiments wherein the one or more biomarker is BCL11B, a target nucleic acid sequence may comprise bases 3301 to 7670 of SEQ ID NO: 27, and a probe typically comprises or is complementary to a nucleic acid sequence having at least 80% sequence identity to a nucleic acid sequence from this target sequence. In an alternative embodiment, a target nucleic acid sequence may comprise bases 515 to 580 or 532 to 607 of SEQ ID NO: 27, and a probe typically comprises or is complementary to a nucleic acid sequence having at least 80% sequence identity to a nucleic acid sequence from this target sequence.

In embodiments wherein the one or more biomarker is ITM2A, a target nucleic acid sequence may comprise bases 411 to 1250 of SEQ ID NO: 28, and a probe typically comprises or is complementary to a nucleic acid sequence having at least 80% sequence identity to a nucleic acid sequence from this target sequence.

In embodiments wherein the one or more biomarker is SLAMF6, a target nucleic acid sequence may comprise bases 1601 to 2700 of SEQ ID NO: 29, and a probe typically comprises or is complementary to a nucleic acid sequence having at least 80% sequence identity to a nucleic acid sequence from this target sequence.

In embodiments wherein the one or more biomarker is HLA-DPB1, a target nucleic acid sequence may comprise bases 511 to 1090 of SEQ ID NO: 30, or bases 121 to 920 of SEQ ID NO: 31, and a probe typically comprises or is complementary to a nucleic acid sequence having at least 80% sequence identity to a nucleic acid sequence from this target sequence.

In embodiments wherein the one or more biomarker is CD160, a target nucleic acid sequence may comprise bases 871 to 1460 of SEQ ID NO: 32, and a probe typically comprises or is complementary to a nucleic acid sequence having at least 80% sequence identity to a nucleic acid sequence from this target sequence.

In embodiments wherein the one or more biomarker is KLRF1, a target nucleic acid sequence may comprise bases 251 to 1240 of SEQ ID NO: 33, and a probe typically comprises or is complementary to a nucleic acid sequence having at least 80% sequence identity to a nucleic acid sequence from this target sequence.

In embodiments wherein the one or more biomarker is CD2, a target nucleic acid sequence may comprise bases 291 to 1530 of SEQ ID NO: 34, and a probe typically comprises or is complementary to a nucleic acid sequence having at least 80% sequence identity to a nucleic acid sequence from this target sequence.

In embodiments wherein the one or more biomarker is LGALS2, a target nucleic acid sequence may comprise bases 101 to 520 of SEQ ID NO: 35, and a probe typically comprises or is complementary to a nucleic acid sequence having at least 80% sequence identity to a nucleic acid sequence from this target sequence.

In embodiments wherein the one or more biomarker is NPPC, a target nucleic acid sequence may comprise bases 261 to 640 of SEQ ID NO: 36, and a probe typically comprises or is complementary to a nucleic acid sequence having at least 80% sequence identity to a nucleic acid sequence from this target sequence.

In embodiments wherein the one or more biomarker is MYCL, a target nucleic acid sequence may comprise bases 2931 to 3600 of SEQ ID NO: 37, or bases 781 to 1990 of SEQ ID NO: 38, and a probe typically comprises or is complementary to a nucleic acid sequence having at least 80% sequence identity to a nucleic acid sequence from this target sequence. In an alternative embodiment, a target nucleic acid sequence may comprise bases 1022 to 1113 of SEQ ID NO: 37, or bases 661 to 720 of SEQ ID NO: 38, and a probe typically comprises or is complementary to a nucleic acid sequence having at least 80% sequence identity to a nucleic acid sequence from this target sequence

In embodiments wherein the one or more biomarker is MX1, a target nucleic acid sequence may comprise bases 391 to 3400 of SEQ ID NO: 39, and a probe typically comprises or is complementary to a nucleic acid sequence having at least 80% sequence identity to a nucleic acid sequence from this target sequence.

In embodiments wherein the one or more biomarker is CCL5, a target nucleic acid sequence may comprise bases 311 to 1230 of SEQ ID NO: 40, and a probe typically comprises or is complementary to a nucleic acid sequence having at least 80% sequence identity to a nucleic acid sequence from this target sequence.

In embodiments wherein the one or more biomarker is TGFB1, a target nucleic acid sequence may comprise bases 2091 to 2790 of SEQ ID NO: 41, and a probe typically comprises or is complementary to a nucleic acid sequence having at least 80% sequence identity to a nucleic acid sequence from this target sequence. In an alternative embodiment, a target nucleic acid sequence may comprise bases 2228 to 2090 of SEQ ID NO: 41, and a probe typically comprises or is complementary to a nucleic acid sequence having at least 80% sequence identity to a nucleic acid sequence from this target sequence.

In embodiments wherein the one or more biomarker is PLA2G7, a target nucleic acid sequence may comprise bases 1041 to 1810 of SEQ ID NO: 42, and a probe typically comprises or is complementary to a nucleic acid sequence having at least 80% sequence identity to a nucleic acid sequence from this target sequence. In an alternative embodiment, a target nucleic acid sequence may comprise bases 356 to 421 or 608 to 674 of SEQ ID NO: 42, and a probe typically comprises or is complementary to a nucleic acid sequence having at least 80% sequence identity to a nucleic acid sequence from this target sequence.

In embodiments wherein the one or more biomarker is ARHGEF10L, a target nucleic acid sequence may comprise bases 3461 to 4490 of SEQ ID NO: 43, and a probe typically comprises or is complementary to a nucleic acid sequence having at least 80% sequence identity to a nucleic acid sequence from this target sequence. In an alternative embodiment, a target nucleic acid sequence may comprise bases 2275 to 2337 of SEQ ID NO: 43, and a probe typically comprises or is complementary to a nucleic acid sequence having at least 80% sequence identity to a nucleic acid sequence from this target sequence.

In embodiments wherein the one or more biomarker is GPR124, a target nucleic acid sequence may comprise bases 5021 to 5870 of SEQ ID NO: 44, and a probe typically comprises or is complementary to a nucleic acid sequence having at least 80% sequence identity to a nucleic acid sequence from this target sequence.

In embodiments wherein the one or more biomarker is IL1RN, a target nucleic acid sequence may comprise bases 241 to 1920 of SEQ ID NO: 45, and a probe typically comprises or is complementary to a nucleic acid sequence having at least 80% sequence identity to a nucleic acid sequence from this target sequence.

In embodiments wherein the one or more biomarker is NLRP3, a target nucleic acid sequence may comprise bases 1921 to 4160 of SEQ ID NO: 46, and a probe typically comprises or is complementary to a nucleic acid sequence having at least 80% sequence identity to a nucleic acid sequence from this target sequence.

In embodiments wherein the one or more biomarker is RBP4, a target nucleic acid sequence may comprise bases 291 to 940 of SEQ ID NO: 47, and a probe typically comprises or is complementary to a nucleic acid sequence having at least 80% sequence identity to a nucleic acid sequence from this target sequence.

In embodiments wherein the one or more biomarker is MPP3, a target nucleic acid sequence may comprise bases 531 to 2140 of SEQ ID NO: 48, and a probe typically comprises or is complementary to a nucleic acid sequence having at least 80% sequence identity to a nucleic acid sequence from this target sequence.

In embodiments wherein the one or more biomarker is KIF2C, a target nucleic acid sequence may comprise bases 721 to 2630 of SEQ ID NO: 49, and a probe typically comprises or is complementary to a nucleic acid sequence having at least 80% sequence identity to a nucleic acid sequence from this target sequence.

In embodiments wherein the one or more biomarker is MAP1A, a target nucleic acid sequence may comprise bases 9521 to 10275 of SEQ ID NO: 50, and a probe typically comprises or is complementary to a nucleic acid sequence having at least 80% sequence identity to a nucleic acid sequence from this target sequence.

In embodiments wherein the one or more biomarker is SELP, a target nucleic acid sequence may comprise bases 1801 to 3150 of SEQ ID NO: 51, and a probe typically comprises or is complementary to a nucleic acid sequence having at least 80% sequence identity to a nucleic acid sequence from this target sequence.

In embodiments wherein the one or more biomarker is NEXN, a target nucleic acid sequence may comprise bases 361 to 2330 of SEQ ID NO: 52, and a probe typically comprises or is complementary to a nucleic acid sequence having at least 80% sequence identity to a nucleic acid sequence from this target sequence.

In embodiments wherein the one or more biomarker is ITGA2B, a target nucleic acid sequence may comprise bases 2211 to 3300 of SEQ ID NO: 53, and a probe typically comprises or is complementary to a nucleic acid sequence having at least 80% sequence identity to a nucleic acid sequence from this target sequence. For example, a target nucleic acid sequence may comprise bases 2286 to 2345 of SEQ ID NO: 53, and a probe typically comprises or is complementary to a nucleic acid sequence having at least 80% sequence identity to a nucleic acid sequence from this target sequence. In an alternative embodiment, a target nucleic acid sequence may comprise bases 1480 to 1543 of SEQ ID NO: 53, and a probe typically comprises or is complementary to a nucleic acid sequence having at least 80% sequence identity to a nucleic acid sequence from this target sequence.

In embodiments wherein the one or more biomarker is MYL9, a target nucleic acid sequence may comprise bases 221 to 1030 of SEQ ID NO: 54, and a probe typically comprises or is complementary to a nucleic acid sequence having at least 80% sequence identity to a nucleic acid sequence from this target sequence. In an alternative embodiment, a target nucleic acid sequence may comprise bases 38 to 83 or 53 to 120 of SEQ ID NO: 54, and a probe typically comprises or is complementary to a nucleic acid sequence having at least 80% sequence identity to a nucleic acid sequence from this target sequence

In embodiments wherein the one or more biomarker is ITGB3, a target nucleic acid sequence may comprise bases 2611 to 4580 of SEQ ID NO: 55, and a probe typically comprises or is complementary to a nucleic acid sequence having at least 80% sequence identity to a nucleic acid sequence from this target sequence. In an alternative embodiment, a target nucleic acid sequence may comprise bases 1116 to 1182 or 1978 to 2047 of SEQ ID NO: 55, and a probe typically comprises or is complementary to a nucleic acid sequence having at least 80% sequence identity to a nucleic acid sequence from this target sequence.

In embodiments wherein the one or more biomarker is CMTM5, a target nucleic acid sequence may comprise bases 381 to 1020 of SEQ ID NO: 56, and a probe typically comprises or is complementary to a nucleic acid sequence having at least 80% sequence identity to a nucleic acid sequence from this target sequence.

In embodiments wherein the one or more biomarker is LCN2, a target nucleic acid sequence may comprise bases 131 to 710 of SEQ ID NO: 57, and a probe typically comprises or is complementary to a nucleic acid sequence having at least 80% sequence identity to a nucleic acid sequence from this target sequence. For example, a target nucleic acid sequence may comprise bases 532 to 603 or 632 to 689 of SEQ ID NO: 57, and a probe typically comprises or is complementary to a nucleic acid sequence having at least 80% sequence identity to a nucleic acid sequence from this target sequence.

In embodiments wherein the one or more biomarker is NLRC4, a target nucleic acid sequence may comprise bases 441 to 1310 of SEQ ID NO: 58, and a probe typically comprises or is complementary to a nucleic acid sequence having at least 80% sequence identity to a nucleic acid sequence from this target sequence.

In embodiments wherein the one or more biomarker is PPBP, a target nucleic acid sequence may comprise bases 241 to 1200 of SEQ ID NO: 59, and a probe typically comprises or is complementary to a nucleic acid sequence having at least 80% sequence identity to a nucleic acid sequence from this target sequence.

In embodiments wherein the one or more biomarker is TREML1, a target nucleic acid sequence may comprise bases 611 to 1340 of SEQ ID NO: 60, and a probe typically comprises or is complementary to a nucleic acid sequence having at least 80% sequence identity to a nucleic acid sequence from this target sequence. In an alternative embodiment, a target nucleic acid sequence may comprise bases 502 to 569 or 520 to 588 of SEQ ID NO: 60, and a probe typically comprises or is complementary to a nucleic acid sequence having at least 80% sequence identity to a nucleic acid sequence from this target sequence.

In embodiments wherein the one or more biomarker is PF4, a target nucleic acid sequence may comprise bases 261 to 850 of SEQ ID NO: 61, and a probe typically comprises or is complementary to a nucleic acid sequence having at least 80% sequence identity to a nucleic acid sequence from this target sequence.

In embodiments wherein the one or more biomarker is CLEC1B, a target nucleic acid sequence may comprise bases 351 to 970 of SEQ ID NO: 62, and a probe typically comprises or is complementary to a nucleic acid sequence having at least 80% sequence identity to a nucleic acid sequence from this target sequence.

In embodiments wherein the one or more biomarker is LCN15, a target nucleic acid sequence may comprise bases 71 to 762 of SEQ ID NO: 63, and a probe typically comprises or is complementary to a nucleic acid sequence having at least 80% sequence identity to a nucleic acid sequence from this target sequence.

In embodiments wherein the one or more biomarker is CIQC, a target nucleic acid sequence may comprise bases 501 to 1100 of SEQ ID NO: 64, and a probe typically comprises or is complementary to a nucleic acid sequence having at least 80% sequence identity to a nucleic acid sequence from this target sequence. In an alternative embodiment, a target nucleic acid sequence may comprise bases 39 to 302, 39 to 150, 61 to 150 or 61 to 302 of SEQ ID NO: 64, and a probe typically comprises or is complementary to a nucleic acid sequence having at least 80% sequence identity to a nucleic acid sequence from this target sequence.

In embodiments wherein the one or more biomarker is CIQB, a target nucleic acid sequence may comprise bases 321 to 1020 of SEQ ID NO: 65, and a probe typically comprises or is complementary to a nucleic acid sequence having at least 80% sequence identity to a nucleic acid sequence from this target sequence. In an alternative embodiment, a target nucleic acid sequence may comprise bases 91 to 154 or 91 to 157 of SEQ ID NO: 65, and a probe typically comprises or is complementary to a nucleic acid sequence having at least 80% sequence identity to a nucleic acid sequence from this target sequence.

In embodiments wherein the one or more biomarker is PCOLEC2, a target nucleic acid sequence may comprise bases 1091 to 2000 of SEQ ID NO: 66, and a probe typically comprises or is complementary to a nucleic acid sequence having at least 80% sequence identity to a nucleic acid sequence from this target sequence.

In embodiments wherein the one or more biomarker is CIQA, a target nucleic acid sequence may comprise bases 361 to 1098 of SEQ ID NO: 67, and a probe typically comprises or is complementary to a nucleic acid sequence having at least 80% sequence identity to a nucleic acid sequence from this target sequence. In an alternative embodiment, a target nucleic acid sequence may comprise bases 214 to 299 of SEQ ID NO: 67, and a probe typically comprises or is complementary to a nucleic acid sequence having at least 80% sequence identity to a nucleic acid sequence from this target sequence.

In embodiments wherein the one or more biomarker is TMEM37, a target nucleic acid sequence may comprise bases 471 to 1687 of SEQ ID NO: 68, and a probe typically comprises or is complementary to a nucleic acid sequence having at least 80% sequence identity to a nucleic acid sequence from this target sequence. In an alternative embodiment, a target nucleic acid sequence may comprise bases 25 to 115 of SEQ ID NO: 68, and a probe typically comprises or is complementary to a nucleic acid sequence having at least 80% sequence identity to a nucleic acid sequence from this target sequence.

In embodiments wherein the one or more biomarker is TNF, a target nucleic acid sequence may comprise bases 991 to 1670 of SEQ ID NO: 69, and a probe typically comprises or is complementary to a nucleic acid sequence having at least 80% sequence identity to a nucleic acid sequence from this target sequence.

In embodiments wherein the one or more biomarker is SLC39A8, a target nucleic acid sequence may comprise bases 2161 to 3109 of SEQ ID NO: 70, or bases 2921 to 4050 of SEQ ID NO: 71, and a probe typically comprises or is complementary to a nucleic acid sequence having at least 80% sequence identity to a nucleic acid sequence from this target sequence. In an alternative embodiment, a target nucleic acid sequence may comprise bases 1525 to 1603 or 1718 to 1787 of SEQ ID NO: 70, or bases 1360 to 1438 or 1553 to 1622 of SEQ ID NO: 71, and a probe typically comprises or is complementary to a nucleic acid sequence having at least 80% sequence identity to a nucleic acid sequence from this target sequence.

In embodiments wherein the one or more biomarker is MRAS, a target nucleic acid sequence may comprise bases 3581 to 4570 of SEQ ID NO: 72, and a probe typically comprises or is complementary to a nucleic acid sequence having at least 80% sequence identity to a nucleic acid sequence from this target sequence. In an alternative embodiment, a target nucleic acid sequence may comprise bases 1104 to 1167 or 1182 to 1246 of SEQ ID NO: 72, and a probe typically comprises or is complementary to a nucleic acid sequence having at least 80% sequence identity to a nucleic acid sequence from this target sequence.

In embodiments wherein the one or more biomarker is IFIT1, a target nucleic acid sequence may comprise bases 1501 to 3960 of SEQ ID NO: 73, and a probe typically comprises or is complementary to a nucleic acid sequence having at least 80% sequence identity to a nucleic acid sequence from this target sequence.

In embodiments wherein the one or more biomarker is IFI44, a target nucleic acid sequence may comprise bases 901 to 1650 of SEQ ID NO: 74, and a probe typically comprises or is complementary to a nucleic acid sequence having at least 80% sequence identity to a nucleic acid sequence from this target sequence.

In embodiments wherein the one or more biomarker is RPGRIP1, a target nucleic acid sequence may comprise bases 2541 to 3770 of SEQ ID NO: 75, and a probe typically comprises or is complementary to a nucleic acid sequence having at least 80% sequence identity to a nucleic acid sequence from this target sequence.

In embodiments wherein the one or more biomarker is DISC1, a target nucleic acid sequence may comprise bases 1201 to 1707 of SEQ ID NO: 76, and a probe typically comprises or is complementary to a nucleic acid sequence having at least 80% sequence identity to a nucleic acid sequence from this target sequence.

In embodiments wherein the one or more biomarker is CXCR1, a target nucleic acid sequence may comprise bases 181 to 2080 of SEQ ID NO: 77, and a probe typically comprises or is complementary to a nucleic acid sequence having at least 80% sequence identity to a nucleic acid sequence from this target sequence. In an alternative embodiment, a target nucleic acid sequence may comprise bases 44 to 113 or 70 to 136 of SEQ ID NO: 77, and a probe typically comprises or is complementary to a nucleic acid sequence having at least 80% sequence identity to a nucleic acid sequence from this target sequence.

In embodiments wherein the one or more biomarker is HCAR2, a target nucleic acid sequence may comprise bases 21 to 1810 of SEQ ID NO: 78, and a probe typically comprises or is complementary to a nucleic acid sequence having at least 80% sequence identity to a nucleic acid sequence from this target sequence. For example, a target nucleic acid sequence may comprise bases 918 to 979 or 1299 to 1356 of SEQ ID NO: 78, and a probe typically comprises or is complementary to a nucleic acid sequence having at least 80% sequence identity to a nucleic acid sequence from this target sequence

In embodiments wherein the one or more biomarker is EPSTI1, a target nucleic acid sequence may comprise bases 621 to 2990 of SEQ ID NO: 79, and a probe typically comprises or is complementary to a nucleic acid sequence having at least 80% sequence identity to a nucleic acid sequence from this target sequence.

In embodiments wherein the one or more biomarker is LILRB4, a target nucleic acid sequence may comprise bases 1081 to 3240 of SEQ ID NO: 80, and a probe typically comprises or is complementary to a nucleic acid sequence having at least 80% sequence identity to a nucleic acid sequence from this target sequence.

In embodiments wherein the one or more biomarker is LILRB5, a target nucleic acid sequence may comprise bases 341 to 2120 of SEQ ID NO: 81, and a probe typically comprises or is complementary to a nucleic acid sequence having at least 80% sequence identity to a nucleic acid sequence from this target sequence. For example, a target nucleic acid sequence may comprise bases 1633 to 1697 or 1653 to 1706 of SEQ ID NO: 81, and a probe typically comprises or is complementary to a nucleic acid sequence having at least 80% sequence identity to a nucleic acid sequence from this target sequence

In embodiments wherein the one or more biomarker is NECAB1, a target nucleic acid sequence may comprise bases 4231 to 5000 of SEQ ID NO: 82, and a probe typically comprises or is complementary to a nucleic acid sequence having at least 80% sequence identity to a nucleic acid sequence from this target sequence. In an alternative embodiment, a target nucleic acid sequence may comprise bases 763 to 845 or 1206 to 1285 of SEQ ID NO: 82, and a probe typically comprises or is complementary to a nucleic acid sequence having at least 80% sequence identity to a nucleic acid sequence from this target sequence.

In embodiments wherein the one or more biomarker is NECAB2, a target nucleic acid sequence may comprise bases 691 to 1490 of SEQ ID NO: 83, and a probe typically comprises or is complementary to a nucleic acid sequence having at least 80% sequence identity to a nucleic acid sequence from this target sequence. In an alternative embodiment, a target nucleic acid sequence may comprise bases 226 to 289 or 579 to 641 of SEQ ID NO: 83, and a probe typically comprises or is complementary to a nucleic acid sequence having at least 80% sequence identity to a nucleic acid sequence from this target sequence.

In embodiments wherein the one or more biomarker is PKHD1, a target nucleic acid sequence may comprise bases 10141 to 16040 of SEQ ID NO: 84, and a probe typically comprises or is complementary to a nucleic acid sequence having at least 80% sequence identity to a nucleic acid sequence from this target sequence. In an alternative embodiment, a target nucleic acid sequence may comprise bases 9037 to 9100 or 10262 to 10335 of SEQ ID NO: 84, and a probe typically comprises or is complementary to a nucleic acid sequence having at least 80% sequence identity to a nucleic acid sequence from this target sequence.

In embodiments wherein the one or more biomarker is PKD1, a target nucleic acid sequence may comprise bases 2201 to 14080 of SEQ ID NO: 85, and a probe typically comprises or is complementary to a nucleic acid sequence having at least 80% sequence identity to a nucleic acid sequence from this target sequence.

It is preferred that the binding conditions for a probe hybridising to its target sequence are such that a high level of specificity is provided - i.e. hybridisation of the probe occurs under *"stringent conditions".* In general, stringent conditions are selected to be about 5°C lower than the thermal melting point (Tm) for the specific sequence at a defined ionic strength and pH. The Tm is the temperature (under defined ionic strength and pH) at which 50% of the target (or complement) sequence hybridises to a perfectly matched probe. In this regard, the Tm of probes of the present invention, at a salt concentration of about 0.02M or less at pH 7, is for example above 60°C, such as about 70°C.

Premixed buffer solutions are commercially available (e.g. EXPRESSHYB Hybridisation Solution from CLONTECH Laboratories, Inc.), and hybridisation can be performed according to the manufacturer's instructions.

Probes of the present invention may be screened to minimise self-complementarity and dimer formation (probe-probe binding).

Any of the probes described herein may comprise a tag and/ or label. The tag and/ or label may, for example, be located (independently of one another) towards the middle or towards or at the 5' or 3' end of the herein described probes, for example at the 5' end.

Hence, following hybridisation of tagged/ labelled probe to target nucleic acid, the tag/ label is associated with the target nucleic acid in the one or more biomarker. Alternatively, if an amplification step is employed, the probes may act as primers during the method of the invention and the tag/ label may therefore become incorporated into the amplification product as the primer is extended.

Examples of suitable labels include detectable labels such as radiolabels or fluorescent or coloured molecules, enzymatic markers or chromogenic markers - e.g. dyes that produce a visible colour change upon hybridisation of the probe. By way of example, the label may be digoxygenin, fluorescein-isothiocyanate (FITC), R-phycoerythrin, Alexa 532 or Cy3. The probes preferably contain a Fam label (e.g. a 5' Fam label), and/ or a minor groove binder (MGB). The label may be a reporter molecule, which is detected directly, such as by exposure to photographic or X-ray film. Alternatively, the label is not directly detectable, but may be detected indirectly, for example, in a two-phase system. An example of indirect label detection is binding of an antibody to the label.

Examples of suitable tags include *"complement*/ *anti-complement pairs".* The term *"complement*/ *anti-complement pair"* denotes non-identical moieties that form a non-covalently associated, stable pair under appropriate conditions. Examples of suitable tags include biotin and streptavidin (or avidin). By way of example, a biotin tag may be captured using streptavidin, which may be coated onto a substrate or support such as a bead (for example a magnetic bead) or membrane. Likewise, a streptavidin tag may be captured using biotin, which may be coated onto a substrate or support such as a bead (for example a magnetic bead) or membrane. Other exemplary complement/ anti-complement pairs include receptor/ ligand pairs, antibody/ antigen (or hapten or epitope) pairs, and the like. Another example is a nucleic acid sequence tag that binds to a complementary sequence. The latter may itself be pre-labelled, or may be attached to a surface (e.g. a bead) which is separately labelled. An example of the latter embodiment is the well-known LuminexR bead system. Other exemplary pairs of tags and capture molecules include receptor/ ligand pairs and antibody/ antigen (or hapten or epitope) pairs. Where subsequent dissociation of the complement/ anti-complement pair is desirable, the complement/ anti-complement pair has a binding affinity of, for example, less than 10⁹ M⁻¹. One exemplary tagged probe is a biotin-labelled probe, which may be detected using horse-radish peroxidase conjugated streptavidin.

The probes of the invention may be labelled with different labels or tags, thereby allowing separate identification of each probe when used in the method of the present invention.

Any conventional method may be employed to attach nucleic acid tags to a probe of the present invention (e.g. to the 5' end of the defined binding region of the probe). Alternatively, nucleic acid probes of the invention (with pre-attached nucleic acid tags) may be constructed by commercial providers.

If an amplification step is employed, this step may be carried out using methods and platforms known in the art, for example PCR (for example, with the use of *"Fast DNA Polymerase",* Life Technologies), such as real-time PCR, block-based PCR, ligase chain reaction, glass capillaries, isothermal amplification methods including loop-mediated isothermal amplification, rolling circle amplification transcription mediated amplification, nucleic acid sequence-based amplification, signal mediated amplification of RNA technology, strand displacement amplification, isothermal multiple displacement amplification, helicase-dependent amplification, single primer isothermal amplification, and circular helicase-dependent amplification. If employed, amplification may be carried using any amplification platform. Preferably, the amplification step may comprise quantitative PCR (real-time PCR).

A general amplification step (e.g. pre-detection) may be employed to increase the amount of the one or more biomarker of the invention present in the sample. PCR amplification primers are typically employed to amplify approximately 100-400 base pair regions of the target/ complementary nucleic acid that contain the nucleotide targets of the present invention. In the presence of a suitable polymerase and DNA precursors (dATP, dCTP, dGTP and dTTP), forward and reverse primers are extended in a 5' to 3' direction, thereby initiating the synthesis of new nucleic acid strands that are complementary to the individual strands of the target nucleic acid. The primers thereby drive amplification of target nucleic acid sequences in the one or more biomarker, thereby generating amplification products comprising said target nucleic acid sequences.

An amplification step may be employed in which the probes of the present invention act as primers. In this embodiment, the probes (acting as primers) are extended from their 3' ends (i.e. in a 5'-to-'3') direction. Such an amplification step may be employed in conjunction with a general amplification step, such as the one described above.

The detection step may be carried out by any known means. In this regard, the probe or amplification product may be tagged and/ or labelled, and the detection method may therefore comprise detecting said tag and/ or label.

In one embodiment, the probe(s) may comprise a tag and/ or label. Thus, in one embodiment, following hybridisation of tagged/ labelled probe to target nucleic acid in the one or more biomarker, the tag/ label becomes associated with the target nucleic acid. Thus, in one embodiment, the assay may comprise detecting the tag/ label and correlating presence of tag/ label with presence of the one or more nucleic acid biomarker of the invention.

In one embodiment, tag and/ or label may be incorporated during extension of the probe(s). In doing so, the amplification product(s) become tagged/ labelled, and the assay may therefore comprise detecting the tag/ label and correlating presence of tag/ label with presence of amplification product, and hence the presence of one or more nucleic acid biomarker of the invention.

By way of example, in one embodiment, the amplification product may incorporate a tag/ label (e.g. via a tagged/ labelled dNTP such as biotin-dNTP) as part of the amplification process, and the assay may further comprise the use of a binding partner complementary to said tag (e.g. streptavidin) that includes a detectable tag/ label (e.g. a fluorescent label, such as R-phycoerythrin). In this way, the amplified product incorporates a detectable tag/ label (e.g. a fluorescent label, such as R-phycoerythrin).

In one embodiment, the probe(s) and/ or the amplification product(s) may include a further tag/ label (as the complement component) to allow capture of the amplification product(s).

By way of example, a *"complement*/ *anti-complement"* pairing may be employed in which an anti-complement capture component binds to said further tag/ label (complement component) and thereby permits capture of the probe(s) and/ or amplification product(s). Examples of suitable "complement/ anti-complement" partners have been described earlier in this specification, such as a complementary pair of nucleic acid sequences, a complementary antibody-antigen pair, etc. The anti-complement capture component may be attached (e.g. coated) on to a substrate or solid support - examples of suitable substrates/ supports include membranes and/ or beads (e.g. a magnetic or fluorescent bead). Capture methods are well known in the art. For example, LuminexR beads may be employed. Alternatively, the use of magnetic beads may be advantageous because the beads (plus captured, tagged/ labelled amplification product) can easily be concentrated and separated from the sample, using conventional techniques known in the art.

Immobilisation provides a physical location for the anti-complement capture component (or probes), and may serve to fix the capture component/ probe at a desired location and/ or facilitate recovery or separation of probe. The support may be a rigid solid support made from, for example, glass, plastic or silica, such as a bead (for example a fluorescent or magnetic bead). Alternatively, the support may be a membrane, such as nylon or nitrocellulose membrane. 3D matrices are also suitable supports for use with the present invention - e.g. polyacrylamide or PEG gels. Immobilisation to a support/ platform may be achieved by a variety of conventional means. By way of example, immobilisation onto a support such as a nylon membrane may be achieved by UV cross-linking. Alternatively, biotin-labelled molecules may be bound to streptavidin-coated substrates (and vice-versa), and molecules prepared with amino linkers may be immobilised on to silanised surfaces. Another means of immobilisation is via a poly-T tail or a poly-C tail, for example at the 3' or 5' end. Said immobilisation techniques apply equally to the probe component (and primer pair component, if present) of the present invention.

In one embodiment, the probes of the invention comprise a nucleic acid sequence tag/ label (e.g. attached to each probe at the 5' end of the defined sequence of the probe that binds to target/ complement nucleic acid). In more detail, each of the probes is provided with a different nucleic acid sequence tag/ label, wherein each of said tags/ labels (specifically) binds to a complementary nucleic acid sequence present on the surface of a bead. Each of the different tags/ labels binds to its complementary sequence counterpart (and not to any of the complementary sequence counterparts of the other tags), which is located on a uniquely identifiable bead. In this regard, the beads are uniquely identifiable, for example by means of fluorescence at a specific wavelength. Thus, in use, probes of the invention bind to target nucleic acid (if present in the sample). Thereafter, (only) the bound probes may be extended (in the 3' direction) in the presence of one or more labelled dNTP (e.g. biotin labelled dNTPs, such as biotin-dCTPs).

The extended primers may be contacted with a binding partner counterpart to the labelled dNTPs (e.g. a streptavidin labelled fluorophore, such as streptavidin labelled R-phycoerythrin), which binds to those labelled dNTPs that have become incorporated into the extended primers. Thereafter, the labelled extended primers may be identified by allowing them to bind to their nucleic acid counterparts present on the uniquely identifiable beads. The latter may then be "called" (e.g. to determine the type of bead present by wavelength emission) and the nature of the primer extension (and thus the type of target/ complement nucleic acid present) may be determined.

Typically, probes of the invention are oligonucleotides having sequence identity with a region of the one or more biomarker of the invention as disclosed herein. One or more probe may be immobilised on a solid support, and used to interrogate mRNA obtained from a test sample. If the mRNA from the test sample contains the one or more biomarker targeted by the immobilised probe, it will bind to the probe, and may then be detected. The biomarkers of the invention may also be detected using PCR, such as real time PCR.

Any oligonucleotide with the appropriate level of sequence identity with the one or more biomarker of the invention, or with one or more target sequences within said one or more biomarker of the invention may be used as a probe in the methods and uses described herein. Any oligonucleotide with the appropriate level of complementarity with the one or more biomarker of the invention, or with one or more target sequences within said one or more biomarker of the invention may be used as a probe in the methods and uses of the invention described herein. Exemplary sequences of the one or more biomarkers of the invention are given in SEQ ID NOs: 1 to 85 (see Tables 1-4 herein). Exemplary probe nucleic acid sequences for the biomarkers disclosed herein are set out in Table 14 (SEQ ID NOs: 86-421) and are shown as underlined and bold text in the sequences of the Sequence Information section. These probes are best suited to use in microarray detection of the nucleic acid.

Further exemplary probe nucleic acid sequences are set out in Table 15 (SEQ ID NOs: 424, 427, 430, 433, 436, 439, 442, 445, 448, 451, 454, 457, 460, 463, 466, 469, 472, 475, 478, 481, 484, 487, 490, 493, 496, 499, 502, 506, 509, 512, 515, 518, 521, 524, 525, 528, 531, 534, 537, 540, 543, 546, 549, 552, 555, 558, 561, 564, 567, 570, 573, 576, 579, 582, and 585) together with the forward and reverse primers that are preferably used to amplify the target sequence prior to detection. These probes are best suited to use in quantitative PCR.

Any one or more (eg. 2 or more, 3 or more, up to an including all) of the exemplary probe sequences may be used in the methods and uses of the invention to determine the presence and/or amount of the one or more biomarker.

In embodiments wherein the one or more biomarker is ADM, the oligonucleotide probe typically comprises or is complementary to a nucleic acid sequence having at least 80% sequence identity to the nucleic acid sequence of SEQ ID NO: 86, 87, 88 or 89, preferably SEQ ID NO: 86.

In embodiments wherein the one or more biomarker is CD177, the oligonucleotide probe typically comprises or is complementary to a nucleic acid sequence having at least 80% sequence identity to the nucleic acid sequence of SEQ ID NO: 90, 91, 92, or 93, preferably SEQ ID NO: 90.

In embodiments wherein the one or more biomarker is FAM20A, the oligonucleotide probe typically comprises or is complementary to a nucleic acid sequence having at least 80% sequence identity to the nucleic acid sequence of SEQ ID NO: 94, 95, 96 or 97, preferably SEQ ID NO: 94 or 95. Alternatively, the oligonucleotide probe may comprise or is complementary to a nucleic acid sequence having at least 80% sequence identity to the nucleic acid sequence of SEQ ID NO: 424 or 427.

In embodiments wherein the one or more biomarker is IL10, the oligonucleotide probe typically comprises or is complementary to a nucleic acid sequence having at least 80% sequence identity to the nucleic acid sequence of SEQ ID NO: 98, 99, 100 or 101, preferably SEQ ID NO: 98.

In embodiments wherein the one or more biomarker is METT7LB, the oligonucleotide probe typically comprises or is complementary to a nucleic acid sequence having at least 80% sequence identity to the nucleic acid sequence of SEQ ID NO: 102, 103, 104 or 105, preferably SEQ ID NO: 102.

In embodiments wherein the one or more biomarker is MMP9, the oligonucleotide probe typically comprises or is complementary to a nucleic acid sequence having at least 80% sequence identity to the nucleic acid sequence of SEQ ID NO: 106, 107, 108, 109, preferably SEQ ID NO:106.

In embodiments wherein the one or more biomarker is RETN, the oligonucleotide probe typically comprises or is complementary to a nucleic acid sequence having at least 80% sequence identity to the nucleic acid sequence of SEQ ID NO: 110, 111, 112, or 113, preferably SEQ ID NO: 110.

In embodiments wherein the one or more biomarker is TDRD9, the oligonucleotide probe typically comprises or is complementary to a nucleic acid sequence having at least 80% sequence identity to the nucleic acid sequence of SEQ ID NO: 114, 115, 116, or 117, preferably SEQ ID NO: 114.

In embodiments wherein the one or more biomarker is ITGA7, the oligonucleotide probe typically comprises or is complementary to a nucleic acid sequence having at least 80% sequence identity to the nucleic acid sequence of SEQ ID NO: 118, 119, 120, or 121, preferably SEQ ID NO: 118.

In embodiments wherein the one or more biomarker is BMX, the oligonucleotide probe typically comprises or is complementary to a nucleic acid sequence having at least 80% sequence identity to the nucleic acid sequence of SEQ ID NO: 122, 123, 124 or 125, preferably SEQ ID NO: 122.

In embodiments wherein the one or more biomarker is HP, the oligonucleotide probe typically comprises or is complementary to a nucleic acid sequence having at least 80% sequence identity to the nucleic acid sequence of SEQ ID NO: 126, 127, 128 or 129, preferably SEQ ID NO: 126.

In embodiments wherein the one or more biomarker is IGFBP2, the oligonucleotide probe typically comprises or is complementary to a nucleic acid sequence having at least 80% sequence identity to the nucleic acid sequence of SEQ ID NO: 130, 131, 132, or 133, preferably SEQ ID NO: 130.

In embodiments wherein the one or more biomarker is ALPL, the oligonucleotide probe typically comprises or is complementary to a nucleic acid sequence having at least 80% sequence identity to the nucleic acid sequence of SEQ ID NO: 134, 135, 136, or 137, preferably SEQ ID NO: 134.

In embodiments wherein the one or more biomarker is DACH1, the oligonucleotide probe typically comprises or is complementary to a nucleic acid sequence having at least 80% sequence identity to the nucleic acid sequence of SEQ ID NO: 138, 139, 140, or 141, preferably SEQ ID NO: 138 or 139.

In embodiments wherein the one or more biomarker is IL1R1, the oligonucleotide probe typically comprises or is complementary to a nucleic acid sequence having at least 80% sequence identity to the nucleic acid sequence of SEQ ID NO: 142, 143, 144, or 145, preferably SEQ ID NO: 142 or 143.

In embodiments wherein the one or more biomarker is OLAH, the oligonucleotide probe typically comprises or is complementary to a nucleic acid sequence having at least 80% sequence identity to the nucleic acid sequence of SEQ ID NO: 146, 147, 148, or 149, preferably SEQ ID NO: 146. Alternatively, the oligonucleotide probe may comprise or is complementary to a nucleic acid sequence having at least 80% sequence identity to the nucleic acid sequence of SEQ ID NO: 430 or 433.

In embodiments wherein the one or more biomarker is IL1R2, the oligonucleotide probe typically comprises or is complementary to a nucleic acid sequence having at least 80% sequence identity to the nucleic acid sequence of SEQ ID NO: 150, 151, 152, or 153, preferably SEQ ID NO: 150.

In embodiments wherein the one or more biomarker is CYP19A1, the oligonucleotide probe typically comprises or is complementary to a nucleic acid sequence having at least 80% sequence identity to the nucleic acid sequence of SEQ ID NO: 154, 155, 156 or 157, preferably SEQ ID NO: 154 or 155.

In embodiments wherein the one or more biomarker is MMP8, the oligonucleotide probe typically comprises or is complementary to a nucleic acid sequence having at least 80% sequence identity to the nucleic acid sequence of SEQ ID NO: 158, 159, 160, or 161, preferably SEQ ID NO: 158.

In embodiments wherein the one or more biomarker is TGFA, the oligonucleotide probe typically comprises or is complementary to a nucleic acid sequence having at least 80% sequence identity to the nucleic acid sequence of SEQ ID NO: 162, 163, 164, 165, preferably SEQ ID NO: 162.

In embodiments wherein the one or more biomarker is VSTM1, the oligonucleotide probe typically comprises or is complementary to a nucleic acid sequence having at least 80% sequence identity to the nucleic acid sequence of SEQ ID NO: 166, 167, 168, or 169, preferably SEQ ID NO:166.

In embodiments wherein the one or more biomarker is FCER1A, the oligonucleotide probe typically comprises or is complementary to a nucleic acid sequence having at least 80% sequence identity to the nucleic acid sequence of SEQ ID NO: 170, 171, 172, or 173, preferably SEQ ID NO:170. Alternatively, the oligonucleotide probe may comprise or is complementary to a nucleic acid sequence having at least 80% sequence identity to the nucleic acid sequence of SEQ ID NO: 528 or 531.

In embodiments wherein the one or more biomarker is KLRK1, the oligonucleotide probe typically comprises or is complementary to a nucleic acid sequence having at least 80% sequence identity to the nucleic acid sequence of SEQ ID NO:174, 175, 176, or 177, preferably SEQ ID NO: 174 .

In embodiments wherein the one or more biomarker is KLRB1, the oligonucleotide probe typically comprises or is complementary to a nucleic acid sequence having at least 80% sequence identity to the nucleic acid sequence of SEQ ID NO: 178, 179, 180, or 181, preferably SEQ ID NO: 178 . Alternatively, the oligonucleotide probe may comprise or is complementary to a nucleic acid sequence having at least 80% sequence identity to the nucleic acid sequence of SEQ ID NO: 546 or 549.

In embodiments wherein the one or more biomarker is DAAM2, the oligonucleotide probe typically comprises or is complementary to a nucleic acid sequence having at least 80% sequence identity to the nucleic acid sequence of SEQ ID NO: 182, 183, 184, or 185, preferably SEQ ID NO: 182 or 183.

In embodiments wherein the one or more biomarker is HLA-DRA, the oligonucleotide probe typically comprises or is complementary to a nucleic acid sequence having at least 80% sequence identity to the nucleic acid sequence of SEQ ID NO: 186, 187, 188, or 189, preferably SEQ ID NO:186.

In embodiments wherein the one or more biomarker is BCL11B, the oligonucleotide probe typically comprises or is complementary to a nucleic acid sequence having at least 80% sequence identity to the nucleic acid sequence of SEQ ID NO: 190, 191, 192, or 193, preferably SEQ ID NO: 190 or 191. Alternatively, the oligonucleotide probe may comprise or is complementary to a nucleic acid sequence having at least 80% sequence identity to the nucleic acid sequence of SEQ ID NO: 534 or 537.

In embodiments wherein the one or more biomarker is ITM2A, the oligonucleotide probe typically comprises or is complementary to a nucleic acid sequence having at least 80% sequence identity to the nucleic acid sequence of SEQ ID NO: 194, 195, 196, or 197, preferably SEQ ID NO: 194 .

In embodiments wherein the one or more biomarker is SLAMF6, the oligonucleotide probe typically comprises or is complementary to a nucleic acid sequence having at least 80% sequence identity to the nucleic acid sequence of SEQ ID NO: 198, 199, 200, or 201, preferably SEQ ID NO: 198.

In embodiments wherein the one or more biomarker is HLA-DPB1, the oligonucleotide probe typically comprises or is complementary to a nucleic acid sequence having at least 80% sequence identity to the nucleic acid sequence of SEQ ID NO: 202, 203, 204, or 205, preferably SEQ ID NO:202 or 203.

In embodiments wherein the one or more biomarker is CD160, the oligonucleotide probe typically comprises or is complementary to a nucleic acid sequence having at least 80% sequence identity to the nucleic acid sequence of SEQ ID NO: 206, 207, 208, or 209, preferably SEQ ID NO: 206.

In embodiments wherein the one or more biomarker is KLFF1, the oligonucleotide probe typically comprises or is complementary to a nucleic acid sequence having at least 80% sequence identity to the nucleic acid sequence of SEQ ID NO: 210, 211, 212, or 213, preferably SEQ ID NO: 210.

In embodiments wherein the one or more biomarker is CD2, the oligonucleotide probe typically comprises or is complementary to a nucleic acid sequence having at least 80% sequence identity to the nucleic acid sequence of SEQ ID NO:214, 215, 216 or 217, preferably SEQ ID NO: 214 .

In embodiments wherein the one or more biomarker is LGALS2, the oligonucleotide probe typically comprises or is complementary to a nucleic acid sequence having at least 80% sequence identity to the nucleic acid sequence of SEQ ID NO: 218, 219, 220, or 221, preferably SEQ ID NO: 218.

In embodiments wherein the one or more biomarker is NPPC, the oligonucleotide probe typically comprises or is complementary to a nucleic acid sequence having at least 80% sequence identity to the nucleic acid sequence of SEQ ID NO: 222, 223, 224, or 225, preferably SEQ ID NO: 222.

In embodiments wherein the one or more biomarker is MYCL, the oligonucleotide probe typically comprises or is complementary to a nucleic acid sequence having at least 80% sequence identity to the nucleic acid sequence of SEQ ID NO: 226, 227, 228, 229, 230, 231, 232, or 233. In embodiments wherein the one or more biomarker is transcript variant 3 of MYCL, the oligonucleotide probe typically comprises or is complementary to a nucleic acid sequence having at least 80% sequence identity to the nucleic acid sequence of SEQ ID NO: 226, 227, 228, or 229, preferably SEQ ID NO: 226. In embodiments wherein the one or more biomarker is transcript variant 1 of MYCL, the oligonucleotide probe typically comprises or is complementary to a nucleic acid sequence having at least 80% sequence identity to the nucleic acid sequence of SEQ ID NO: 230, 231, 232, or 233, preferably SEQ ID NO: 230. Alternatively, the oligonucleotide probe may comprise or is complementary to a nucleic acid sequence having at least 80% sequence identity to the nucleic acid sequence of SEQ ID NO: 481 or 484.

In embodiments wherein the one or more biomarker is MX1, the oligonucleotide probe typically comprises or is complementary to a nucleic acid sequence having at least 80% sequence identity to the nucleic acid sequence of SEQ ID NO: 234, 235, 236, or 237, preferably SEQ ID NO: 234 .

In embodiments wherein the one or more biomarker is CCL5, the oligonucleotide probe typically comprises or is complementary to a nucleic acid sequence having at least 80% sequence identity to the nucleic acid sequence of SEQ ID NO: 238, 239, 240, or 241, preferably SEQ ID NO: 238.

In embodiments wherein the one or more biomarker is TGFB1, the oligonucleotide probe typically comprises or is complementary to a nucleic acid sequence having at least 80% sequence identity to the nucleic acid sequence of SEQ ID NO: 242, 243, 244, or 245, preferably SEQ ID NO: 242. Alternatively, the oligonucleotide probe may comprise or is complementary to a nucleic acid sequence having at least 80% sequence identity to the nucleic acid sequence of SEQ ID NO: 475 or 478.

In embodiments wherein the one or more biomarker is PLA2G7, the oligonucleotide probe typically comprises or is complementary to a nucleic acid sequence having at least 80% sequence identity to the nucleic acid sequence of SEQ ID NO: 246, 247, 248, or 249, preferably SEQ ID NO: 246. Alternatively, the oligonucleotide probe may comprise or is complementary to a nucleic acid sequence having at least 80% sequence identity to the nucleic acid sequence of SEQ ID NO: 466 or 469.

In embodiments wherein the one or more biomarker is ARHGEF10L, the oligonucleotide probe typically comprises or is complementary to a nucleic acid sequence having at least 80% sequence identity to the nucleic acid sequence of SEQ ID NO: 250, 251, 252, or 253, preferably SEQ ID NO: 250 or 251. Alternatively, the oligonucleotide probe may comprise or is complementary to a nucleic acid sequence having at least 80% sequence identity to the nucleic acid sequence of SEQ ID NO: 472.

In embodiments wherein the one or more biomarker is GPR124, the oligonucleotide probe typically comprises or is complementary to a nucleic acid sequence having at least 80% sequence identity to the nucleic acid sequence of SEQ ID NO: 254, 255, 256, or 257, preferably SEQ ID NO: 254.

In embodiments wherein the one or more biomarker is IL1RN, the oligonucleotide probe typically comprises or is complementary to a nucleic acid sequence having at least 80% sequence identity to the nucleic acid sequence of SEQ ID NO: 258, 259, 260, or 261, preferably SEQ ID NO: 258 or 259.

In embodiments wherein the one or more biomarker is NLRP3, the oligonucleotide probe typically comprises or is complementary to a nucleic acid sequence having at least 80% sequence identity to the nucleic acid sequence of SEQ ID NO: 262, 263, 264, or 265, preferably SEQ ID NO: 262.

In embodiments wherein the one or more biomarker is RBP4, the oligonucleotide probe typically comprises or is complementary to a nucleic acid sequence having at least 80% sequence identity to the nucleic acid sequence of SEQ ID NO: 266, 267, 268, or 269, preferably SEQ ID NO: 266.

In embodiments wherein the one or more biomarker is MPP3, the oligonucleotide probe typically comprises or is complementary to a nucleic acid sequence having at least 80% sequence identity to the nucleic acid sequence of SEQ ID NO: 270, 271, 272, or 273, preferably SEQ ID NO: 270.

In embodiments wherein the one or more biomarker is KIF2C, the oligonucleotide probe typically comprises or is complementary to a nucleic acid sequence having at least 80% sequence identity to the nucleic acid sequence of SEQ ID NO: 274, 275, 276, or 277, preferably SEQ ID NO:274.

In embodiments wherein the one or more biomarker is MAP1A, the oligonucleotide probe typically comprises or is complementary to a nucleic acid sequence having at least 80% sequence identity to the nucleic acid sequence of SEQ ID NO: 278, 279, 280, or 281, preferably SEQ ID NO: 278.

In embodiments wherein the one or more biomarker is SELP, the oligonucleotide probe typically comprises or is complementary to a nucleic acid sequence having at least 80% sequence identity to the nucleic acid sequence of SEQ ID NO: 282, 283, 284, or 285, preferably SEQ ID NO: 282.

In embodiments wherein the one or more biomarker is NEXN, the oligonucleotide probe typically comprises or is complementary to a nucleic acid sequence having at least 80% sequence identity to the nucleic acid sequence of SEQ ID NO: 286, 287, 288, or 289, preferably SEQ ID NO:286 or 287.

In embodiments wherein the one or more biomarker is ITGA2B, the oligonucleotide probe typically comprises or is complementary to a nucleic acid sequence having at least 80% sequence identity to the nucleic acid sequence of SEQ ID NO: 290, 291, 292, or 293, preferably SEQ ID NO: 290. Alternatively, the oligonucleotide probe may comprise or is complementary to a nucleic acid sequence having at least 80% sequence identity to the nucleic acid sequence of SEQ ID NO: 460 or 463.

In embodiments wherein the one or more biomarker is MYL9, the oligonucleotide probe typically comprises or is complementary to a nucleic acid sequence having at least 80% sequence identity to the nucleic acid sequence of SEQ ID NO: 294, 295, 296, or 297, preferably SEQ ID NO: 294. Alternatively, the oligonucleotide probe may comprise or is complementary to a nucleic acid sequence having at least 80% sequence identity to the nucleic acid sequence of SEQ ID NO: 448 or 451.

In embodiments wherein the one or more biomarker is ITGB3, the oligonucleotide probe typically comprises or is complementary to a nucleic acid sequence having at least 80% sequence identity to the nucleic acid sequence of SEQ ID NO: 298, 299, 300, or 301, preferably SEQ ID NO: 298.

In embodiments wherein the one or more biomarker is CMTM5, the oligonucleotide probe typically comprises or is complementary to a nucleic acid sequence having at least 80% sequence identity to the nucleic acid sequence of SEQ ID NO: 302, 303, 304 or 305, preferably SEQ ID NO: 302.

In embodiments wherein the one or more biomarker is LCN2, the oligonucleotide probe typically comprises or is complementary to a nucleic acid sequence having at least 80% sequence identity to the nucleic acid sequence of SEQ ID NO: 306, 307, 308, or 309, preferably SEQ ID NO: 306. Alternatively, the oligonucleotide probe may comprise or is complementary to a nucleic acid sequence having at least 80% sequence identity to the nucleic acid sequence of SEQ ID NO: 436 or 439.

In embodiments wherein the one or more biomarker is NLRC4, the oligonucleotide probe typically comprises or is complementary to a nucleic acid sequence having at least 80% sequence identity to the nucleic acid sequence of SEQ ID NO: 310, 311, 312, or 313, preferably SEQ ID NO: 310 .

In embodiments wherein the one or more biomarker is PPBP, the oligonucleotide probe typically comprises or is complementary to a nucleic acid sequence having at least 80% sequence identity to the nucleic acid sequence of SEQ ID NO: 314, 315, 316, or 317, preferably SEQ ID NO: 314.

In embodiments wherein the one or more biomarker is TREML1, the oligonucleotide probe typically comprises or is complementary to a nucleic acid sequence having at least 80% sequence identity to the nucleic acid sequence of SEQ ID NO: 318, 319, 320, 321, preferably SEQ ID NO: 318.

In embodiments wherein the one or more biomarker is PF4, the oligonucleotide probe typically comprises or is complementary to a nucleic acid sequence having at least 80% sequence identity to the nucleic acid sequence of SEQ ID NO: 322, 323, 324, or 325, preferably SEQ ID NO: 322.

In embodiments wherein the one or more biomarker is CLEC1B, the oligonucleotide probe typically comprises or is complementary to a nucleic acid sequence having at least 80% sequence identity to the nucleic acid sequence of SEQ ID NO: 326, 327, 328, or 329, preferably SEQ ID NO: 326 or 327.

In embodiments wherein the one or more biomarker is LCN15, the oligonucleotide probe typically comprises or is complementary to a nucleic acid sequence having at least 80% sequence identity to the nucleic acid sequence of SEQ ID NO: 330, 331, 332, or 333, preferably SEQ ID NO: 330.

In embodiments wherein the one or more biomarker is CIQC, the oligonucleotide probe typically comprises or is complementary to a nucleic acid sequence having at least 80% sequence identity to the nucleic acid sequence of SEQ ID NO: 334, 335, 336, or 337, preferably SEQ ID NO: 334. Alternatively, the oligonucleotide probe may comprise or is complementary to a nucleic acid sequence having at least 80% sequence identity to the nucleic acid sequence of SEQ ID NO: 506 or 509.

In embodiments wherein the one or more biomarker is CIQB, the oligonucleotide probe typically comprises or is complementary to a nucleic acid sequence having at least 80% sequence identity to the nucleic acid sequence of SEQ ID NO: 338, 339, 340, or 341, preferably SEQ ID NO: 338. Alternatively, the oligonucleotide probe may comprise or is complementary to a nucleic acid sequence having at least 80% sequence identity to the nucleic acid sequence of SEQ ID NO: 502.

In embodiments wherein the one or more biomarker is PCOLCE2, the oligonucleotide probe typically comprises or is complementary to a nucleic acid sequence having at least 80% sequence identity to the nucleic acid sequence of SEQ ID NO: 342, 343, 344, or 345, preferably SEQ ID NO: 342.

In embodiments wherein the one or more biomarker is CIQA, the oligonucleotide probe typically comprises or is complementary to a nucleic acid sequence having at least 80% sequence identity to the nucleic acid sequence of SEQ ID NO: 346, 347, 348, or 349, preferably SEQ ID NO: 346. Alternatively, the oligonucleotide probe may comprise or is complementary to a nucleic acid sequence having at least 80% sequence identity to the nucleic acid sequence of SEQ ID NO: 499.

In embodiments wherein the one or more biomarker is TMEM37, the oligonucleotide probe typically comprises or is complementary to a nucleic acid sequence having at least 80% sequence identity to the nucleic acid sequence of SEQ ID NO: 350, 351, 352, or 353, preferably SEQ ID NO: 350. Alternatively, the oligonucleotide probe may comprise or is complementary to a nucleic acid sequence having at least 80% sequence identity to the nucleic acid sequence of SEQ ID NO: 524 or 525.

In embodiments wherein the one or more biomarker is TNF, the oligonucleotide probe typically comprises or is complementary to a nucleic acid sequence having at least 80% sequence identity to the nucleic acid sequence of SEQ ID NO: 354, 355, 356, or 357, preferably SEQ ID NO: 354 .

In embodiments wherein the one or more biomarker is SLC39A8, the oligonucleotide probe typically comprises or is complementary to a nucleic acid sequence having at least 80% sequence identity to the nucleic acid sequence of SEQ ID NO: 358, 359, 360, 361, 362, 363, 364, or 365, preferably SEQ ID NO: 358 or 362. In embodiments wherein the one or more biomarker is transcript variant 1 of SLC39A8, the oligonucleotide probe typically comprises or is complementary to a nucleic acid sequence having at least 80% sequence identity to the nucleic acid sequence of SEQ ID NO: 358, 359, 360, or 361, preferably SEQ ID NO: 358. In embodiments wherein the one or more biomarker is transcript variant 3 of SLC39A8, the oligonucleotide probe typically comprises or is complementary to a nucleic acid sequence having at least 80% sequence identity to the nucleic acid sequence of SEQ ID NO: 362, 363, 364, or 365, preferably SEQ ID NO: 362. Alternatively, the oligonucleotide probe may comprise or is complementary to a nucleic acid sequence having at least 80% sequence identity to the nucleic acid sequence of SEQ ID NO: 518 or 521.

In embodiments wherein the one or more biomarker is MRAS, the oligonucleotide probe typically comprises or is complementary to a nucleic acid sequence having at least 80% sequence identity to the nucleic acid sequence of SEQ ID NO: 366, 367, 368, or 369, preferably SEQ ID NO: 366. Alternatively, the oligonucleotide probe may comprise or is complementary to a nucleic acid sequence having at least 80% sequence identity to the nucleic acid sequence of SEQ ID NO: 512 or 515.

In embodiments wherein the one or more biomarker is IFIT1, the oligonucleotide probe typically comprises or is complementary to a nucleic acid sequence having at least 80% sequence identity to the nucleic acid sequence of SEQ ID NO: 370, 371, 372, or 373, preferably SEQ ID NO: 370.

In embodiments wherein the one or more biomarker is IFI44, the oligonucleotide probe typically comprises or is complementary to a nucleic acid sequence having at least 80% sequence identity to the nucleic acid sequence of SEQ ID NO: 374, 375, 376, or 377, preferably SEQ ID NO: 374.

In embodiments wherein the one or more biomarker is RPGRIP1, the oligonucleotide probe typically comprises or is complementary to a nucleic acid sequence having at least 80% sequence identity to the nucleic acid sequence of SEQ ID NO: 378, 379, 380, or 381, preferably SEQ ID NO: 378 .

In embodiments wherein the one or more biomarker is DISC1, the oligonucleotide probe typically comprises or is complementary to a nucleic acid sequence having at least 80% sequence identity to the nucleic acid sequence of SEQ ID NO: 382, 383, 384, or 385, preferably SEQ ID NO: 382.

In embodiments wherein the one or more biomarker is CXCR1, the oligonucleotide probe typically comprises or is complementary to a nucleic acid sequence having at least 80% sequence identity to the nucleic acid sequence of SEQ ID NO: 386, 387, 388, or 389, preferably SEQ ID NO: 386. Alternatively, the oligonucleotide probe may comprise or is complementary to a nucleic acid sequence having at least 80% sequence identity to the nucleic acid sequence of SEQ ID NO: 487 or 490.

In embodiments wherein the one or more biomarker is HCAR2, the oligonucleotide probe typically comprises or is complementary to a nucleic acid sequence having at least 80% sequence identity to the nucleic acid sequence of SEQ ID NO: 390, 391, 392, or 393, preferably SEQ ID NO: 390. Alternatively, the oligonucleotide probe may comprise or is complementary to a nucleic acid sequence having at least 80% sequence identity to the nucleic acid sequence of SEQ ID NO: 493 or 496.

In embodiments wherein the one or more biomarker is EPST1, the oligonucleotide probe typically comprises or is complementary to a nucleic acid sequence having at least 80% sequence identity to the nucleic acid sequence of SEQ ID NO: 394, 395, 396, or 397, preferably SEQ ID NO: 394.

In embodiments wherein the one or more biomarker is LILRB4, the oligonucleotide probe typically comprises or is complementary to a nucleic acid sequence having at least 80% sequence identity to the nucleic acid sequence of SEQ ID NO: 398, 399, 400, or 401, preferably SEQ ID NO: 398 and 399.

In embodiments wherein the one or more biomarker is LILRB5, the oligonucleotide probe typically comprises or is complementary to a nucleic acid sequence having at least 80% sequence identity to the nucleic acid sequence of SEQ ID NO: 402, 403, 404, or 405, preferably SEQ ID NO: 402. Alternatively, the oligonucleotide probe may comprise or is complementary to a nucleic acid sequence having at least 80% sequence identity to the nucleic acid sequence of SEQ ID NO: 552 or 555.

In embodiments wherein the one or more biomarker is NECAB1, the oligonucleotide probe typically comprises or is complementary to a nucleic acid sequence having at least 80% sequence identity to the nucleic acid sequence of SEQ ID NO: 406, 407, 408, or 409, preferably SEQ ID NO: 406. Alternatively, the oligonucleotide probe may comprise or is complementary to a nucleic acid sequence having at least 80% sequence identity to the nucleic acid sequence of SEQ ID NO: 558 or 561.

In embodiments wherein the one or more biomarker is NECAB2, the oligonucleotide probe typically comprises or is complementary to a nucleic acid sequence having at least 80% sequence identity to the nucleic acid sequence of SEQ ID NO: 410, 411, 412, or 413, preferably SEQ ID NO: 410. Alternatively, the oligonucleotide probe may comprise or is complementary to a nucleic acid sequence having at least 80% sequence identity to the nucleic acid sequence of SEQ ID NO: 564 or 567.

In embodiments wherein the one or more biomarker is PKHD1, the oligonucleotide probe typically comprises or is complementary to a nucleic acid sequence having at least 80% sequence identity to the nucleic acid sequence of SEQ ID NO: 414, 415, 416 or 417, preferably SEQ ID NO: 414 or 415. Alternatively, the oligonucleotide probe may comprise or is complementary to a nucleic acid sequence having at least 80% sequence identity to the nucleic acid sequence of SEQ ID NO: 540 or 543.

In embodiments wherein the one or more biomarker is PKD1, the oligonucleotide probe typically comprises or is complementary to a nucleic acid sequence having at least 80% sequence identity to the nucleic acid sequence of SEQ ID NO: 418, 419, 420, or 421, preferably SEQ ID NO: 418.

In all of the methods and uses described herein, the presence and/or amount of the one or more biomarker is determined using an oligonucleotide probe specific for the one or more biomarker. The oligonucleotide probe used in the methods and uses of the invention may an oligonucleotide probe of the invention as described herein.

As described above, a general amplification step (e.g. pre-detection) may be employed to increase the amount of the one or more biomarker of the invention present in the sample. As well as using the oligonucleotide probes of the invention as primers, separate forward and reverse oligonucleotide primers may be used to amplify a target nucleic acid sequence. The amplified nucleic acid may be detected using an oligonucleotide probe of the invention. For example, such primers and probes may be used when the one or more biomarker is detected and/or quantified by quantitative PCR.

The present invention therefore provides a forward oligonucleotide primer and/or a reverse oligonucleotide primer for amplification of a target nucleic acid sequence in the one or more biomarker.

In one embodiment, one or more forward oligonucleotide primer and one or more reverse oligonucleotide primer may be used to amplify the one or more nucleic acid biomarker of the invention prior to detection.

In general, a reverse primer is designed to hybridise to a target nucleic acid sequence within the coding (sense) strand of a target nucleic acid, and a forward primer is designed to hybridise to a target nucleic acid sequence within the complementary (ie. anti-sense) strand of the target nucleic acid.

The term "complement of a nucleic acid sequence" refers to a nucleic acid sequence having a complementary nucleotide sequence and reverse orientation as compared to a reference nucleotide sequence.

The forward primer hybridises to a target nucleic acid sequence (a 'forward primer target sequence') located within the sequence of the nucleic acid biomarker. In one embodiment, the forward primer target sequence has a length in the range of 10-40 consecutive nucleotides, such at least 11, 12, 13, 14, 15, 16, 17, 18, 19, 20, 21 or 22 consecutive nucleotides, and/or up to 38, 35, 32, 30, 29, 28, 27, 26, 25, 24, 23, 22, 21 or 20 consecutive nucleotides.

The reverse primer hybridises to a target nucleic acid sequence (a 'reverse primer target sequence') located within the sequence of the nucleic acid biomarker. In one embodiment, the reverse primer target sequence has a length in the range of 10-40 consecutive nucleotides, such as at least 11, 12, 13, 14, 15, 16, 17, 18, 19, 20, 21 or 22 consecutive nucleotides, and/or up to 38, 35, 32, 30, 29, 28, 27, 26, 25, 24, 23, 22, 21 or 20 consecutive nucleotides.

The present invention also provides oligonucleotide primers and probes for amplifying control (or reference) genes. In one embodiment, the control gene is selected from the group consisting of: ALAS1 (NM_000688 SEQ ID NO: 586, or NM_199166, SEQ ID NO: 587), GTF2D1 (NM_003194, SEQ ID NO: 588, and HMBS (NM_000190.3, SEQ ID NO: 589).

In one embodiment, the forward primer hybridises to a target nucleic acid sequence that comprises (or consists of) the complement of a nucleotide sequence selected from SEQ ID NOs: 1-85, or a nucleotide sequence that is at least 80% identical thereto (e.g. at least 82, 84. 86, 88, 90, 91, 92, 93, 94, 95, 96, 97, 98 or 99% identical thereto).

In one embodiment, the reverse primer hybridises to a target nucleic acid sequence that comprises (or consists of) a nucleotide sequence that is at least 80% identical to (e.g at least 82, 84, 86, 88, 90, 91, 92, 93, 94, 95, 96, 97, 98, 99 or 100% identical to) a nucleotide sequence selected from SEQ ID NOs: 1-85.

Exemplary primer and probe sequences are shown in Table 15.

In one embodiment, the forward primer hybridises to a target nucleic acid sequence that comprises (or consists of) the complement of a nucleotide sequence selected from SEQ ID NOs: 422, 425, 428, 431, 434, 437, 440, 443, 446, 449, 452, 455, 458, 461, 464, 467, 470, 473, 476, 479, 482, 485, 488, 491, 494, 497, 500, 504, 507, 510, 513, 516, 519, 522, 526, 529, 532, 535, 538, 541, 544, 547, 550, 553, 556, 559, 562, 565, 568, 571, 574, 577, 580, and 583 (as shown in Table 15), or a nucleotide sequence that is at least 80% identical thereto (e.g. at least 82, 84. 86, 88, 90, 91, 92, 93, 94, 95, 96, 97, 98 or 99% identical thereto).

In one embodiment, the reverse primer hybridises to a target nucleic acid sequence that comprises (or consists of) the complement of a nucleotide sequence that is at least 80% identical to (e.g at least 82, 84. 86, 88, 90, 91, 92, 93, 94, 95, 96, 97, 98, 99 or 100% identical to) a nucleotide sequence selected from SEQ ID NOs: 423, 426, 429, 432, 435, 438, 441, 444, 447, 450, 453, 456, 447, 450, 453, 456, 459, 462, 465, 468, 471, 474, 477, 480, 483, 486, 489, 492, 495, 498, 501, 503, 505, 508, 511, 514, 517, 520, 523, 527, 530, 533, 536, 539, 542, 545, 548, 551, 554, 557, 560, 563, 566, 569, 572, 575, 578, 581, and 584 (as shown in Table 15).

In one embodiment, the forward primer comprises (or consists of) a nucleotide sequence having at least 80% identity to (preferably at least 82, 84, 86, 88, 90 91, 92, 93, 94, 95, 96, 97, 98, 99 or 100% identity to) a nucleotide sequence selected from SEQ ID NOs: 422, 425, 428, 431, 434, 437, 440, 443, 446, 449, 452, 455, 458, 461, 464, 467, 470, 473, 476, 479, 482, 485, 488, 491, 494, 497, 500, 504, 507, 510, 513, 516, 519, 522, 526, 529, 532, 535, 538, 541, 544, 547, 550, 553, 556, 559, 562, 565, 568, 571, 574, 577, 580, and 583 (as shown in Table 15). Conservative substitutions are preferred.

In one embodiment, the reverse primer comprises (or consists of) a nucleotide sequence having at least 80% identity to (preferably at least 82, 84, 86, 88, 90 91, 92, 93, 94, 95, 96, 97, 98, 99 or 100% identity to) a nucleotide sequence selected from SEQ ID NOs: 423, 426, 429, 432, 435, 438, 441, 444, 447, 450, 453, 456, 447, 450, 453, 456, 459, 462, 465, 468, 471, 474, 477, 480, 483, 486, 489, 492, 495, 498, 501, 503, 505, 508, 511, 514, 517, 520, 523, 527, 530, 533, 536, 539, 542, 545, 548, 551, 554, 557, 560, 563, 566, 569, 572, 575, 578, 581, and 584 (as shown in Table 15). Conservative substitutions are preferred.

In embodiments wherein the one or more biomarker is FAM20A, the forward primer hybridises to a target nucleic acid sequence that comprises (or consists of) the complement of the nucleotide sequence of SEQ ID NOs: 422 or 425, or a nucleotide sequence that is at least 80% identical thereto. For example, the forward primer comprises (or consists of) a nucleotide sequence having at least 80% identity to the nucleic acid sequence of SEQ ID NO: 422 or 425. The reverse primer hybridises to a target nucleic acid sequence that comprises (or consists of) the complement of a nucleotide sequence that is at least 80% identical to the nucleic acid sequence of SEQ ID NOs: 423 or 426. For example, the reverse primer comprises (or consists of) a nucleotide sequence having at least 80% identity to the nucleic acid sequence of SEQ ID NOs: 423 or 426. The amplified target nucleic acid may be detected using an oligonucleotide probe as described herein (e.g. by reference to SEQ ID NOs: 424 or 427.

In embodiments wherein the one or more biomarker is OLAH, the forward primer hybridises to a target nucleic acid sequence that comprises (or consists of) the complement of the nucleotide sequence of SEQ ID NOs: 428 or 431, or a nucleotide sequence that is at least 80% identical thereto. For example, the forward primer comprises (or consists of) a nucleotide sequence having at least 80% identity to the nucleic acid sequence of SEQ ID NO: 428 or 431. The reverse primer hybridises to a target nucleic acid sequence that comprises (or consists of) the complement of a nucleotide sequence that is at least 80% identical to the nucleic acid sequence of SEQ ID NOs: 429 or 432. For example, the reverse primer comprises (or consists of) a nucleotide sequence having at least 80% identity to the nucleic acid sequence of SEQ ID NOs: 429 or 432. The amplified target nucleic acid may be detected using an oligonucleotide probe as described herein (e.g. by reference to SEQ ID NOs: 430 or 433).

In embodiments wherein the one or more biomarker is LCN2, the forward primer hybridises to a target nucleic acid sequence that comprises (or consists of) the complement of the nucleotide sequence of SEQ ID NOs: 434 or 437, or a nucleotide sequence that is at least 80% identical thereto. For example, the forward primer comprises (or consists of) a nucleotide sequence having at least 80% identity to the nucleic acid sequence of SEQ ID NO: 434 or 437. The reverse primer hybridises to a target nucleic acid sequence that comprises (or consists of) the complement of a nucleotide sequence that is at least 80% identical to the nucleic acid sequence of SEQ ID NOs: 435 or 438. For example, the reverse primer comprises (or consists of) a nucleotide sequence having at least 80% identity to the nucleic acid sequence of SEQ ID NOs: 435 or 438. The amplified target nucleic acid may be detected using an oligonucleotide probe as described herein (e.g. by reference to SEQ ID NOs: 436 or 439).

In embodiments wherein the one or more biomarker is ITGB3, the forward primer hybridises to a target nucleic acid sequence that comprises (or consists of) the complement of the nucleotide sequence of SEQ ID NOs: 440 or 443, or a nucleotide sequence that is at least 80% identical thereto. For example, the forward primer comprises (or consists of) a nucleotide sequence having at least 80% identity to the nucleic acid sequence of SEQ ID NO: 440 or 443. The reverse primer hybridises to a target nucleic acid sequence that comprises (or consists of) the complement of a nucleotide sequence that is at least 80% identical to the nucleic acid sequence of SEQ ID NOs: 441 or 444. For example, the reverse primer comprises (or consists of) a nucleotide sequence having at least 80% identity to the nucleic acid sequence of SEQ ID NOs: 441 or 444. The amplified target nucleic acid may be detected using an oligonucleotide probe as described herein (e.g. by reference to SEQ ID NOs: 442 or 445).

In embodiments wherein the one or more biomarker is MYL9, the forward primer hybridises to a target nucleic acid sequence that comprises (or consists of) the complement of the nucleotide sequence of SEQ ID NOs: 446 or 449, or a nucleotide sequence that is at least 80% identical thereto. For example, the forward primer comprises (or consists of) a nucleotide sequence having at least 80% identity to the nucleic acid sequence of SEQ ID NO: 446 or 449. The reverse primer hybridises to a target nucleic acid sequence that comprises (or consists of) the complement of a nucleotide sequence that is at least 80% identical to the nucleic acid sequence of SEQ ID NOs: 447 or 450. For example, the reverse primer comprises (or consists of) a nucleotide sequence having at least 80% identity to the nucleic acid sequence of SEQ ID NOs: 447 or 450. The amplified target nucleic acid may be detected using an oligonucleotide probe as described herein (e.g. by reference to SEQ ID NOs: 448 or 451).

In embodiments wherein the one or more biomarker is TREML1, the forward primer hybridises to a target nucleic acid sequence that comprises (or consists of) the complement of the nucleotide sequence of SEQ ID NOs: 452 or 455, or a nucleotide sequence that is at least 80% identical thereto. For example, the forward primer comprises (or consists of) a nucleotide sequence having at least 80% identity to the nucleic acid sequence of SEQ ID NO: 452 or 455. The reverse primer hybridises to a target nucleic acid sequence that comprises (or consists of) the complement of a nucleotide sequence that is at least 80% identical to the nucleic acid sequence of SEQ ID NOs: 453 or 456. For example, the reverse primer comprises (or consists of) a nucleotide sequence having at least 80% identity to the nucleic acid sequence of SEQ ID NOs: 453 or 456. The amplified target nucleic acid may be detected using an oligonucleotide probe as described herein (e.g. by reference to SEQ ID NOs: 454 or 457).

In embodiments wherein the one or more biomarker is ITGA2B, the forward primer hybridises to a target nucleic acid sequence that comprises (or consists of) the complement of the nucleotide sequence of SEQ ID NOs: 458 or 461, or a nucleotide sequence that is at least 80% identical thereto. For example, the forward primer comprises (or consists of) a nucleotide sequence having at least 80% identity to the nucleic acid sequence of SEQ ID NO: 458 or 461. The reverse primer hybridises to a target nucleic acid sequence that comprises (or consists of) the complement of a nucleotide sequence that is at least 80% identical to the nucleic acid sequence of SEQ ID NOs: 459 or 462. For example, the reverse primer comprises (or consists of) a nucleotide sequence having at least 80% identity to the nucleic acid sequence of SEQ ID NOs: 459 or 462. The amplified target nucleic acid may be detected using an oligonucleotide probe as described herein (e.g. by reference to SEQ ID NOs: 457 or 460).

In embodiments wherein the one or more biomarker is PLA2G7, the forward primer hybridises to a target nucleic acid sequence that comprises (or consists of) the complement of the nucleotide sequence of SEQ ID NOs: 464 or 467, or a nucleotide sequence that is at least 80% identical thereto. For example, the forward primer comprises (or consists of) a nucleotide sequence having at least 80% identity to the nucleic acid sequence of SEQ ID NO: 464 or 467. The reverse primer hybridises to a target nucleic acid sequence that comprises (or consists of) the complement of a nucleotide sequence that is at least 80% identical to the nucleic acid sequence of SEQ ID NOs: 465 or 468. For example, the reverse primer comprises (or consists of) a nucleotide sequence having at least 80% identity to the nucleic acid sequence of SEQ ID NOs: 465 or 468. The amplified target nucleic acid may be detected using an oligonucleotide probe as described herein (e.g. by reference to SEQ ID NOs: 466 or 469).

In embodiments wherein the one or more biomarker is ARHGEF10L, the forward primer hybridises to a target nucleic acid sequence that comprises (or consists of) the complement of the nucleotide sequence of SEQ ID NO: 470, or a nucleotide sequence that is at least 80% identical thereto. For example, the forward primer comprises (or consists of) a nucleotide sequence having at least 80% identity to the nucleic acid sequence of SEQ ID NO: 470. The reverse primer hybridises to a target nucleic acid sequence that comprises (or consists of) the complement of a nucleotide sequence that is at least 80% identical to the nucleic acid sequence of SEQ ID NO: 471. For example, the reverse primer comprises (or consists of) a nucleotide sequence having at least 80% identity to the nucleic acid sequence of SEQ ID NO: 471. The amplified target nucleic acid may be detected using an oligonucleotide probe as described herein (e.g. by reference to SEQ ID NO: 472).

In embodiments wherein the one or more biomarker is TGFB1, the forward primer hybridises to a target nucleic acid sequence that comprises (or consists of) the complement of the nucleotide sequence of SEQ ID NOs: 473 or 476, or a nucleotide sequence that is at least 80% identical thereto. For example, the forward primer comprises (or consists of) a nucleotide sequence having at least 80% identity to the nucleic acid sequence of SEQ ID NO: 473 or 476. The reverse primer hybridises to a target nucleic acid sequence that comprises (or consists of) the complement of a nucleotide sequence that is at least 80% identical to the nucleic acid sequence of SEQ ID NOs: 474 or 477. For example, the reverse primer comprises (or consists of) a nucleotide sequence having at least 80% identity to the nucleic acid sequence of SEQ ID NOs: 474 or 477. The amplified target nucleic acid may be detected using an oligonucleotide probe as described herein (e.g. by reference to SEQ ID NOs: 475 or 478).

In embodiments wherein the one or more biomarker is MYCL, the forward primer hybridises to a target nucleic acid sequence that comprises (or consists of) the complement of the nucleotide sequence of SEQ ID NOs: 479 or 482, or a nucleotide sequence that is at least 80% identical thereto. For example, the forward primer comprises (or consists of) a nucleotide sequence having at least 80% identity to the nucleic acid sequence of SEQ ID NO: 479 or 482. The reverse primer hybridises to a target nucleic acid sequence that comprises (or consists of) the complement of a nucleotide sequence that is at least 80% identical to the nucleic acid sequence of SEQ ID NOs: 480 or 483. For example, the reverse primer comprises (or consists of) a nucleotide sequence having at least 80% identity to the nucleic acid sequence of SEQ ID NOs: 480 or 483. The amplified target nucleic acid may be detected using an oligonucleotide probe as described herein (e.g. by reference to SEQ ID NOs: 481 or 484).

In embodiments wherein the one or more biomarker is CXCR1, the forward primer hybridises to a target nucleic acid sequence that comprises (or consists of) the complement of the nucleotide sequence of SEQ ID NOs: 485 or 488, or a nucleotide sequence that is at least 80% identical thereto. For example, the forward primer comprises (or consists of) a nucleotide sequence having at least 80% identity to the nucleic acid sequence of SEQ ID NO: 485 or 488. The reverse primer hybridises to a target nucleic acid sequence that comprises (or consists of) the complement of a nucleotide sequence that is at least 80% identical to the nucleic acid sequence of SEQ ID NOs: 486 or 489. For example, the reverse primer comprises (or consists of) a nucleotide sequence having at least 80% identity to the nucleic acid sequence of SEQ ID NOs: 486 or 489. The amplified target nucleic acid may be detected using an oligonucleotide probe as described herein (e.g. by reference to SEQ ID NOs: 487 or 490).

In embodiments wherein the one or more biomarker is HCAR2, the forward primer hybridises to a target nucleic acid sequence that comprises (or consists of) the complement of the nucleotide sequence of SEQ ID NOs: 491 or 494, or a nucleotide sequence that is at least 80% identical thereto. For example, the forward primer comprises (or consists of) a nucleotide sequence having at least 80% identity to the nucleic acid sequence of SEQ ID NO: 491 or 494. The reverse primer hybridises to a target nucleic acid sequence that comprises (or consists of) the complement of a nucleotide sequence that is at least 80% identical to the nucleic acid sequence of SEQ ID NOs: 492 or 495. For example, the reverse primer comprises (or consists of) a nucleotide sequence having at least 80% identity to the nucleic acid sequence of SEQ ID NOs: 492 or 495. The amplified target nucleic acid may be detected using an oligonucleotide probe as described herein (e.g. by reference to SEQ ID NOs: 493 or 496).

In embodiments wherein the one or more biomarker is CIQA, the forward primer hybridises to a target nucleic acid sequence that comprises (or consists of) the complement of the nucleotide sequence of SEQ ID NO: 497, or a nucleotide sequence that is at least 80% identical thereto. For example, the forward primer comprises (or consists of) a nucleotide sequence having at least 80% identity to the nucleic acid sequence of SEQ ID NO: 497. The reverse primer hybridises to a target nucleic acid sequence that comprises (or consists of) the complement of a nucleotide sequence that is at least 80% identical to the nucleic acid sequence of SEQ ID NO: 498. For example, the reverse primer comprises (or consists of) a nucleotide sequence having at least 80% identity to the nucleic acid sequence of SEQ ID NO: 498. The amplified target nucleic acid may be detected using an oligonucleotide probe as described herein (e.g. by reference to SEQ ID NO: 499).

In embodiments wherein the one or more biomarker is CIQB, the forward primer hybridises to a target nucleic acid sequence that comprises (or consists of) the complement of the nucleotide sequence of SEQ ID NO: 500, or a nucleotide sequence that is at least 80% identical thereto. For example, the forward primer comprises (or consists of) a nucleotide sequence having at least 80% identity to the nucleic acid sequence of SEQ ID NO: 500. The reverse primer hybridises to a target nucleic acid sequence that comprises (or consists of) the complement of a nucleotide sequence that is at least 80% identical to the nucleic acid sequence of SEQ ID NOs: 501 or 503. For example, the reverse primer comprises (or consists of) a nucleotide sequence having at least 80% identity to the nucleic acid sequence of SEQ ID NOs: 501 or 503. The amplified target nucleic acid may be detected using an oligonucleotide probe as described herein (e.g. by reference to SEQ ID NO: 502).

In embodiments wherein the one or more biomarker is CIQC, the forward primer hybridises to a target nucleic acid sequence that comprises (or consists of) the complement of the nucleotide sequence of SEQ ID NOs: 504 or 507, or a nucleotide sequence that is at least 80% identical thereto. For example, the forward primer comprises (or consists of) a nucleotide sequence having at least 80% identity to the nucleic acid sequence of SEQ ID NOs: 504 or 507. The reverse primer hybridises to a target nucleic acid sequence that comprises (or consists of) the complement of a nucleotide sequence that is at least 80% identical to the nucleic acid sequence of SEQ ID NOs: 505 or 508. For example, the reverse primer comprises (or consists of) a nucleotide sequence having at least 80% identity to the nucleic acid sequence of SEQ ID NOs: 505 or 508. The amplified target nucleic acid may be detected using an oligonucleotide probe as described herein (e.g. by reference to SEQ ID NO: 506 or 509).

In embodiments wherein the one or more biomarker is MRAS, the forward primer hybridises to a target nucleic acid sequence that comprises (or consists of) the complement of the nucleotide sequence of SEQ ID NOs: 510 or 513, or a nucleotide sequence that is at least 80% identical thereto. For example, the forward primer comprises (or consists of) a nucleotide sequence having at least 80% identity to the nucleic acid sequence of SEQ ID NO: 510 or 513. The reverse primer hybridises to a target nucleic acid sequence that comprises (or consists of) the complement of a nucleotide sequence that is at least 80% identical to the nucleic acid sequence of SEQ ID NOs: 511 or 514. For example, the reverse primer comprises (or consists of) a nucleotide sequence having at least 80% identity to the nucleic acid sequence of SEQ ID NOs: 511 or 514. The amplified target nucleic acid may be detected using an oligonucleotide probe as described herein (e.g. by reference to SEQ ID NOs: 512 or 515).

In embodiments wherein the one or more biomarker is SLC39A8, the forward primer hybridises to a target nucleic acid sequence that comprises (or consists of) the complement of the nucleotide sequence of SEQ ID NOs: 516 or 519, or a nucleotide sequence that is at least 80% identical thereto. For example, the forward primer comprises (or consists of) a nucleotide sequence having at least 80% identity to the nucleic acid sequence of SEQ ID NO: 516 or 519. The reverse primer hybridises to a target nucleic acid sequence that comprises (or consists of) the complement of a nucleotide sequence that is at least 80% identical to the nucleic acid sequence of SEQ ID NOs: 517 or 520. For example, the reverse primer comprises (or consists of) a nucleotide sequence having at least 80% identity to the nucleic acid sequence of SEQ ID NOs: 517 or 520. The amplified target nucleic acid may be detected using an oligonucleotide probe as described herein (e.g. by reference to SEQ ID NOs: 518 or 521).

In embodiments wherein the one or more biomarker is TMEM37, the forward primer hybridises to a target nucleic acid sequence that comprises (or consists of) the complement of the nucleotide sequence of SEQ ID NO: 522, or a nucleotide sequence that is at least 80% identical thereto. For example, the forward primer comprises (or consists of) a nucleotide sequence having at least 80% identity to the nucleic acid sequence of SEQ ID NO: 522. The reverse primer hybridises to a target nucleic acid sequence that comprises (or consists of) the complement of a nucleotide sequence that is at least 80% identical to the nucleic acid sequence of SEQ ID NO: 523. For example, the reverse primer comprises (or consists of) a nucleotide sequence having at least 80% identity to the nucleic acid sequence of SEQ ID NO: 523. The amplified target nucleic acid may be detected using an oligonucleotide probe as described herein (e.g. by reference to SEQ ID NOs: 524 or 525).

In embodiments wherein the one or more biomarker is FCER1A, the forward primer hybridises to a target nucleic acid sequence that comprises (or consists of) the complement of the nucleotide sequence of SEQ ID NOs: 526 or 529, or a nucleotide sequence that is at least 80% identical thereto. For example, the forward primer comprises (or consists of) a nucleotide sequence having at least 80% identity to the nucleic acid sequence of SEQ ID NO: 526 or 529. The reverse primer hybridises to a target nucleic acid sequence that comprises (or consists of) the complement of a nucleotide sequence that is at least 80% identical to the nucleic acid sequence of SEQ ID NOs: 527 or 530. For example, the reverse primer comprises (or consists of) a nucleotide sequence having at least 80% identity to the nucleic acid sequence of SEQ ID NOs: 527 or 530. The amplified target nucleic acid may be detected using an oligonucleotide probe as described herein (e.g. by reference to SEQ ID NOs: 528 or 531).

In embodiments wherein the one or more biomarker is BLC11B, the forward primer hybridises to a target nucleic acid sequence that comprises (or consists of) the complement of the nucleotide sequence of SEQ ID NOs: 532 or 535, or a nucleotide sequence that is at least 80% identical thereto. For example, the forward primer comprises (or consists of) a nucleotide sequence having at least 80% identity to the nucleic acid sequence of SEQ ID NO: 532 or 535. The reverse primer hybridises to a target nucleic acid sequence that comprises (or consists of) the complement of a nucleotide sequence that is at least 80% identical to the nucleic acid sequence of SEQ ID NOs: 533 or 536. For example, the reverse primer comprises (or consists of) a nucleotide sequence having at least 80% identity to the nucleic acid sequence of SEQ ID NOs: 533 or 536. The amplified target nucleic acid may be detected using an oligonucleotide probe as described herein (e.g. by reference to SEQ ID NOs: 534 or 537).

In embodiments wherein the one or more biomarker is PKHD1, the forward primer hybridises to a target nucleic acid sequence that comprises (or consists of) the complement of the nucleotide sequence of SEQ ID NOs: 538 or 541, or a nucleotide sequence that is at least 80% identical thereto. For example, the forward primer comprises (or consists of) a nucleotide sequence having at least 80% identity to the nucleic acid sequence of SEQ ID NO: 538 or 541. The reverse primer hybridises to a target nucleic acid sequence that comprises (or consists of) the complement of a nucleotide sequence that is at least 80% identical to the nucleic acid sequence of SEQ ID NOs: 539 or 542. For example, the reverse primer comprises (or consists of) a nucleotide sequence having at least 80% identity to the nucleic acid sequence of SEQ ID NOs: 539 or 542. The amplified target nucleic acid may be detected using an oligonucleotide probe as described herein (e.g. by reference to SEQ ID NOs: 540 or 543).

In embodiments wherein the one or more biomarker is KLRB1, the forward primer hybridises to a target nucleic acid sequence that comprises (or consists of) the complement of the nucleotide sequence of SEQ ID NOs: 544 or 547, or a nucleotide sequence that is at least 80% identical thereto. For example, the forward primer comprises (or consists of) a nucleotide sequence having at least 80% identity to the nucleic acid sequence of SEQ ID NO: 544 or 547. The reverse primer hybridises to a target nucleic acid sequence that comprises (or consists of) the complement of a nucleotide sequence that is at least 80% identical to the nucleic acid sequence of SEQ ID NOs: 545 or 548. For example, the reverse primer comprises (or consists of) a nucleotide sequence having at least 80% identity to the nucleic acid sequence of SEQ ID NOs: 545 or 548. The amplified target nucleic acid may be detected using an oligonucleotide probe as described herein (e.g. by reference to SEQ ID NOs: 546 or 549).

In embodiments wherein the one or more biomarker is LILRB5, the forward primer hybridises to a target nucleic acid sequence that comprises (or consists of) the complement of the nucleotide sequence of SEQ ID NOs: 550 or 553, or a nucleotide sequence that is at least 80% identical thereto. For example, the forward primer comprises (or consists of) a nucleotide sequence having at least 80% identity to the nucleic acid sequence of SEQ ID NO: 550 or 553. The reverse primer hybridises to a target nucleic acid sequence that comprises (or consists of) the complement of a nucleotide sequence that is at least 80% identical to the nucleic acid sequence of SEQ ID NOs: 551 or 554. For example, the reverse primer comprises (or consists of) a nucleotide sequence having at least 80% identity to the nucleic acid sequence of SEQ ID NOs: 551 or 554. The amplified target nucleic acid may be detected using an oligonucleotide probe as described herein (e.g. by reference to SEQ ID NOs: 552 or 555).

In embodiments wherein the one or more biomarker is NECAB1, the forward primer hybridises to a target nucleic acid sequence that comprises (or consists of) the complement of the nucleotide sequence of SEQ ID NOs: 556 or 559, or a nucleotide sequence that is at least 80% identical thereto. For example, the forward primer comprises (or consists of) a nucleotide sequence having at least 80% identity to the nucleic acid sequence of SEQ ID NO: 556 or 559. The reverse primer hybridises to a target nucleic acid sequence that comprises (or consists of) the complement of a nucleotide sequence that is at least 80% identical to the nucleic acid sequence of SEQ ID NOs: 557 or 560. For example, the reverse primer comprises (or consists of) a nucleotide sequence having at least 80% identity to the nucleic acid sequence of SEQ ID NOs: 557 or 560. The amplified target nucleic acid may be detected using an oligonucleotide probe as described herein (e.g. by reference to SEQ ID NOs: 558 or 561).

In embodiments wherein the one or more biomarker is NECAB2, the forward primer hybridises to a target nucleic acid sequence that comprises (or consists of) the complement of the nucleotide sequence of SEQ ID NOs: 562 or 565, or a nucleotide sequence that is at least 80% identical thereto. For example, the forward primer comprises (or consists of) a nucleotide sequence having at least 80% identity to the nucleic acid sequence of SEQ ID NO: 562 or 565. The reverse primer hybridises to a target nucleic acid sequence that comprises (or consists of) the complement of a nucleotide sequence that is at least 80% identical to the nucleic acid sequence of SEQ ID NOs: 563 or 566. For example, the reverse primer comprises (or consists of) a nucleotide sequence having at least 80% identity to SEQ ID NOs: 563 or 566. The amplified target nucleic acid may be detected using an oligonucleotide probe as described herein (e.g. by reference to SEQ ID NOs: 564 or 567).

In embodiments wherein the control gene is ALAS1, the forward primer comprises (or consists of) a nucleotide sequence having at least 80% identity to the nucleic acid sequence of SEQ ID NO: 568 or 571. The reverse primer comprises (or consists of) a nucleotide sequence having at least 80% identity to SEQ ID NOs: 569 or 572. The amplified target nucleic acid may be detected using an oligonucleotide probe that comprises a nucleic acid sequence having at least 80% sequence identity to the nucleic acid sequence of SEQ ID NOs:570 or 573.

In embodiments wherein the control gene is HMBS, the forward primer comprises (or consists of) a nucleotide sequence having at least 80% identity to the nucleic acid sequence of SEQ ID NO: 574 or 577. The reverse primer comprises (or consists of) a nucleotide sequence having at least 80% identity to SEQ ID NOs: 575 or 578. The amplified target nucleic acid may be detected using an oligonucleotide probe that comprises a nucleic acid sequence having at least 80% sequence identity to the nucleic acid sequence of SEQ ID NOs: 576 or 579.

In embodiments wherein the control gene is GTF2D1, the forward primer comprises (or consists of) a nucleotide sequence having at least 80% identity to the nucleic acid sequence of SEQ ID NO: 580 or 583. The reverse primer comprises (or consists of) a nucleotide sequence having at least 80% identity to SEQ ID NOs: 581 or 584. The amplified target nucleic acid may be detected using an oligonucleotide probe that comprises a nucleic acid sequence having at least 80% sequence identity to the nucleic acid sequence of SEQ ID NOs: 582 or 585.

In all of the methods and uses described herein, the presence and/or amount of the one or more biomarker may be determined using a forward oligonucleotide primer and/or reverse oligonucleotide primer specific for the one or more biomarker to amplify a target nucleic acid sequence. The forward and reverse oligonucleotide primers used in the methods and uses of the invention are as described herein.

### Kits and Devices

The present invention also provides kits and devices that are useful in diagnosing a systemic inflammatory condition (including diagnosing SIRS, sepsis, abdominal sepsis, and pulmonary sepsis), distinguishing between sepsis and SIRS, distinguishing between abdominal sepsis and pulmonary sepsis, monitoring of a systemic inflammatory condition (including monitoring of SIRS, sepsis, abdominal sepsis, and pulmonary sepsis), determining whether a patient is suitable for discharge from medical care, and diagnosing organ damage.

The kits and devices of the present invention comprise one or more biomarker of the invention and/or one or more agent for the detection of or for the determination of the amount of the one or more biomarker of the invention. For example, the kit and device may comprise one or more biomarker of the invention. For example, the kit and device may comprise one or more agent for the detection of or for the determination of the amount of the one or more biomarker of the invention. The "one or more agent" may comprise one or more binding agent specific for the one or more biomarker.

The "one or more biomarker of the invention" may be as described herein. Specific biomarkers and agents for the detection of said biomarkers useful in the present invention are set forth herein. The biomarkers of the kit or device can be used to generate biomarker profiles according to the present invention.

In one embodiment, the kit and device of the present invention may comprise one or more inflammation biomarker of the invention (e.g. as described herein) and/or one or more agent for the detection of or for the determination of the amount of the one or more inflammation biomarker of the invention. For example, the "one or more inflammation biomarker of the invention" may comprise one or more (e.g. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, or all 21) inflammation biomarker selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1. For example, the "one or more inflammation biomarker of the invention" may comprise one of more (2 or more, or all 3) of: FAM20A, OLAH, and CD177. For example, the "one or more inflammation biomarker of the invention" may comprise FAM20A and OLAH.

In one embodiment, the kit and device of the present invention may comprise one or more sepsis biomarker of the invention and/or one or more agent for the detection of or for the determination of the amount of the one or more sepsis biomarker of the invention. For example, the "one or more sepsis biomarker of the invention" may comprise one or more (e.g. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, or all 30) sepsis biomarker selected from the group consisting of: ITGB3, ITGA2B, MYL9, LCN2, TREML1, LCN15, CMTM5, PPBP, PF4, KIF2C, MAP1A, SELP, NEXN, NLRC4, CLEC1B, SLC39A8, CIQC, CIQA, MRAS, TMEM37, CIQB, PCOLCE2, KIF2C, TNF, IFI44, IFIT1, RPGRIP1, HCAR2, CXCR1, DISC1, and EPSTI1. For example, the "one or more sepsis biomarker of the invention" may comprise one of more (2 or more, 3 or more, 4 or more, 5 or more, 6 or more, 7 or more, 8 or more or all 9) of ITGB3, ITGA2B, MYL9, LCN2, TREML1, LCN15, CMTM5, PPBP, and PF4. For example, the "one or more sepsis biomarker of the invention" may comprise one of more (2 or more, 3 or more, 4 or more, or all 5) of the sepsis biomarkers ITGB3, ITGA2B, MYL9, LCN2, and TREML1.

In one embodiment, the kit and device of the present invention may comprise one or more SIRS biomarker of the invention and/or one or more agent for the detection of or for the determination of the amount of the one or more SIRS biomarker of the invention. For example, the "one or more SIRS biomarker of the invention" may comprise one or more (e.g. 2 or more, 3 or more, 4 or more, 5 or more, 6 or more, 7 or more, 8 or more, or all 9) SIRS biomarker selected from the group consisting of: of PLA2G7, ARHGEF10L, MYCL, TGFBI, and GPR124, IL1RN, NLRP3, RBP4, and MPP3. For example, the "one or more SIRS biomarker of the invention" may comprise one of more (2 or more, 3 or more, 4 or more, or all 5) of PLA2G7, ARHGEF10L, MYCL, TGFBI, and GPR124. For example, the "one or more SIRS biomarker of the invention" may comprise one of more (2 or more, 3 or more, or all 4) of PLA2G7, ARHGEF10L, MYCL, and TGFBI.

In one embodiment, the kit and device of the present invention may comprise:
(i) one or more inflammation biomarker (as described herein),
(ii) one or more sepsis marker (as described herein), and/or
(iii) one or more SIRS biomarker (as described herein);
and/or one or more agent for the detection of or for the determination of the amount of the one or more biomarker.

For example, the kit and device may comprise one or more agent for the detection of or for the determination of the amount of: (i) one or more inflammation biomarker (as described herein), (ii) one or more sepsis marker (as described herein), and/or (iii) one or more SIRS biomarker (as described herein). For example, the kit and device may comprise one or more agent for the detection of or for the determination of the amount of: (i) one of more (2 or more, or all 3) of the inflammatory biomarker selected from the group consisting of: FAM20A, OLAH, and optionally CD177; (ii) one of more (2 or more, 3 or more, 4 or more, or all 5) of the sepsis biomarker selected from the group consisting of: ITGB3, ITGA2B, MYL9, LCN2, and TREML1; and/or (iii) one of more (2 or more, 3 or more, or all 4) of the SIRS biomarker selected from the group consisting of: PLA2G7, ARHGEF10L, MYCL, and TGFBI.

In one embodiment, the kit and device of the present invention may comprise one or more abdominal sepsis biomarker of the invention and/or one or more agent for the detection of or for the determination of the amount of the one or more abdominal sepsis biomarker of the invention. For example, the "one or more abdominal sepsis biomarker of the invention" may comprise one or more (e.g. 2 or more, 3 or more, 4 or more, 5 or more, 6 or more, 7 or more, 8 or more, 9 or more, 10 or more, 11 or more or all 12) abdominal sepsis biomarker selected from the group consisting of: SLC39A8, CIQC, CIQA, MRAS, TMEM37, CIQB, PCOLCE2, KIF2C, TNF, IFI44, IFIT1, and RPGRIP1. For example, the "one or more abdominal sepsis biomarker of the invention" may comprise one or more (e.g. 2 or more, 3 or more, 4 or more, 5 or more, or all 6) of SLC39A8, CIQC, CIQA, MRAS, TMEM37, CIQB. For example, the "one or more abdominal sepsis biomarker of the invention" may comprise one of more (2 or more, or all 3) of SLC39A8, CIQC, CIQA.

In one embodiment, the kit and device of the present invention may comprise one or more pulmonary sepsis biomarker of the invention and/or one or more agent for the detection of or for the determination of the amount of the one or more pulmonary sepsis biomarker of the invention. For example, the "one or more pulmonary sepsis biomarker of the invention" may comprise one or more (e.g. 2 or more, 3 or more, 4 or more, or all 5) pulmonary sepsis biomarker selected from the group consisting of: HCAR2, CXCR1, DISC1, EPSTI1, and IFI44. For example, the "one or more pulmonary sepsis biomarker of the invention" may comprise one of more (2 or more, or all 3) of HCAR2, CXCR1, and DISC1.

In one embodiment, the kit and device of the present invention may comprise:
(i) one or more abdominal sepsis biomarker (as described herein), and/or
(ii) one or more pulmonary sepsis biomarker (as described herein),
and/or one or more agent for the detection of or for the determination of the amount of the one or more biomarker.

For example, the kit and device may comprise one or more agent for the detection of or for the determination of the amount of: (i) one or more abdominal sepsis biomarker (as described herein); and/or (ii) one or more pulmonary sepsis marker (as described herein). For example, the kit and device may comprise one or more agent for the detection of or for the determination of the amount of: (i) one or more (2 or more, 3 or more, 4 or more, 5 or more, or all 6) of the abdominal sepsis biomarker selected from the group consisting of: SLC39A8, CIQC, CIQA, MRAS, TMEM37, and CIQB; and/or (ii) one or more (e.g. 2 or more, or all 3) of the pulmonary sepsis biomarker selected from the group consisting of: HCAR2, CXCR1, and DISC1;

In one embodiment, the kit and device of the present invention may comprise one or more prognosis biomarker of the invention and/or one or more agent for the detection of or for the determination of the amount of the one or more recovery biomarker of the invention. For example, the "one or more prognosis biomarker of the invention" may comprise one or more (e.g. 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or all 20) biomarker selected from the group consisting of: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, and PKHD1. For example, the "one or more biomarker of the invention" may comprise one of more (e.g. 2 or more, 3 or more, 4 or more, 5 or more, 6 or more, 7 or more, 8 or more, 9 or more, 10 or more, 11 or more, or all 12) of ITM2A, CCL5, NPPC, KLRK1, KLRB1, HLA-DRA, BCL11B, HLA-DPB1, SLAMF6, CD160, KLRF1, and MX1. For example, the "one or more biomarker of the invention" may comprise one of more (e.g. 2 or more, 3 or more, 4 or more, 5 or more, 6 or more, or all 7) of CCL5, NPPC, PKD1, LGALS2, MYCL, NECAB1, and PKHD1.

In one embodiment, the kit and device of the present invention may comprise one or more survival biomarker of the invention and/or one or more agent for the detection of or for the determination of the amount of the one or more survival biomarker of the invention. For example, the "one or more survival biomarker of the invention" may comprise one or more (e.g. 2 or more, 3 or more, 4 or more, 5 or more, or all 6) survival biomarker selected from the group consisting of: NECAB1, NECAB2 , PKDI, PKHD1, LILRB4, and LILRB5. For example, the "one or more survival biomarker of the invention" may comprise one or more (e.g. 2 or more, 3 or more, of all 4) of NECAB1, PKDI, PKHD1, and LILRB5. For example, the "one or more survival biomarker of the invention" may comprise NECAB2 and/or PKD1. For example, the "one or more survival biomarker of the invention" may comprise PKHD1 and/or LILRB5.

The kit and device of the present invention may comprise:
(i) one or more prognosis biomarker (as described herein), and/or
(ii) one or more survival biomarker (as described herein),
and/or one or more agent for the detection of or for the determination of the amount of the one or more biomarker.

For example, the kit and device may comprise one or more agent for the detection of or for the determination of the amount of: (i) one or more prognosis biomarker (as described herein); and/or (ii) one or more survival biomarker (as described herein).

In one embodiment, the kit and device of the present invention may comprise one or more agent for the detection of or for the determination of the amount of:
(i) one or more inflammation biomarker (as described herein),
(ii) one or more sepsis biomarker (as described herein),
(iii) one or more abdominal sepsis biomarker (as described herein),
(iv) one or more pulmonary sepsis biomarker (as described herein),
(v) one or more SIRS biomarker (as described herein),
(vi) one or more prognosis biomarker (as described herein), and/or
(vii) one or more survival biomarker (as described herein).

Generally, the biomarkers and agents of the kit or device will bind, with at least some specificity, to the biomarker molecules contained in the sample from which the biomarker profile is generated. In one embodiment, the kit or device of the invention comprises one or more binding agent specific for the one or more biomarker. Examples of classes of compounds of the kit or device include, but are not limited to, proteins (including antibodies), and fragments thereof, peptides, polypeptides, proteoglycans, glycoproteins, lipoproteins, carbohydrates, lipids, nucleic acids, organic and inorganic chemicals, and natural and synthetic polymers. The biomarker(s) and/or agent(s) for the detection of the one or more biomarker may be part of an array, or the biomarker(s) and/or agent(s) may be packaged separately and/or individually. The biomarker(s) and/or agent(s) may be immobilised on an inert support. The biomarkers(s) and/or agent(s) may be immobilised on a surface to provide a binding agent array.

The device may be a lateral flow device. Lateral flow devices and methods for their construction are well known in the art, being best known as the standard pregnancy test kit. The device may be portable. The device may be disposable. The device may be suitable for use as a point of care diagnostic test. The device may be suitable for use in the home or in the clinic.

The kit or device may also comprise at least one internal standard to be used in generating the biomarker profiles of the present invention. Likewise, the internal standards can be any of the classes of compounds described above.

The kits and devices of the present invention also may contain reagents that can be used to detectably label biomarkers contained in the biological samples from which the biomarker profiles are generated. For this purpose, the kit or device may comprise a set of antibodies or functional fragments thereof that specifically bind at least two, three, four, five, 10, 20, 30, 40, 50 or more, up to all of the biomarkers set forth in any one of Tables 1 to 4 that list biomarkers for use in the invention. The antibodies themselves may be detectably labelled. The kit or device also may comprise a specific biomarker binding component, such as an aptamer.

In a preferred embodiment, a kit or device of the invention comprises (i) one or more antibody specific for the one or more biomarkers of the invention; and/or (ii) one or more oligonucleotide specific for the one or more biomarker of the invention. For example, the one or more oligonucleotide specific for the one or more biomarker is an oligonucleotide of the invention, preferably one or more of SEQ ID NOs: 86-585.

If the biomarkers comprise a nucleic acid, the kit or device may provide one or more oligonucleotide probe that is capable of forming a duplex with the one or more biomarker or with a complementary strand of said one or more biomarker. The one or more oligonucleotide probe may be detectably labelled. Typically, the one or more oligonucleotide probe used in the methods of the invention is selected from one or more of the oligonucleotide probe described herein.

In one embodiment, the one or more oligonucleotide probe is selected from an oligonucleotide probe that comprises or is complementary to at least one nucleic acid sequence having at least 80% sequence identity to the nucleic acid sequence of any one or more of SEQ ID NOs: 86-421. The oligonucleotide probe(s) may be bound to a solid support (such as a microarray).

In one embodiment, the one or more oligonucleotide probe is selected from an oligonucleotide probe that comprises or is complementary to at least one nucleic acid sequence having at least 80% sequence identity to the nucleic acid sequences of any one or more of SEQ ID NOs: 424, 427, 430, 433, 436, 439, 442, 445, 448, 451, 454, 457, 460, 463, 466, 469, 472, 475, 478, 481, 484, 487, 490, 493, 496, 499, 502, 506, 509, 512, 515, 518, 521, 524, 525, 528, 531, 534, 537, 540, 543, 546, 549, 552, 555, 558, 561, 564, 567, 570, 573, 576, 579, 582, and 585 (as described in Table 15).

The kit or device may further comprise: (i) one or more forward oligonucleotide primer; and/or (ii) one or more reverse oligonucleotide primer for amplification of the target nucleic acid sequence. The forward and reverse oligonucleotide primers may be as described herein (and exemplified in Table 15). The amplification product may be detected using the corresponding oligonucleotide probe(s) described herein (as exemplified in Table 15). The kit or device may further comprise one or more reagent for performing quantitative PCR.

The kits and devices of the present invention may also include pharmaceutical excipients, diluents and/or adjuvants when the biomarker is to be used to raise an antibody. Examples of pharmaceutical adjuvants include, but are not limited to, preservatives, wetting agents, emulsifying agents, and dispersing agents. Prevention of the action of microorganisms can be ensured by the inclusion of various antibacterial and antifungal agents, for example, paraben, chlorobutanol, phenol sorbic acid, and the like. It may also be desirable to include isotonic agents such as sugars, sodium chloride, and the like.

The present invention is discussed in more detail by means of the Examples described below, and by the Figures.

### FIGURES

**Figure 1****:** shows a plot of normalised gene expression in patients having SIRS, abdominal sepsis and pulmonary sepsis for the biomarkers identified as being associated with systemic inflammatory conditions (see Table 1). Data is included for the patients that survive ("Survived") and the patients that did not survive ("Died") and relates to samples taken at 'day 1', 'day 2' and 'day 5' post-hospitalisation (to the ICU). Data is also included for healthy control patients (shown on the far left-hand side of the plot). Data points are mean expression across all individuals, depicted with standard error bars.
**Figure 2****:** shows a plot of normalised gene expression in patients having SIRS, abdominal sepsis and pulmonary sepsis for the biomarkers identified as being associated with SIRS (see Table 2). The data shown in the plot is as described for Figure 1.
**Figure 3****:** shows a plot of normalised gene expression in patients having SIRS, abdominal sepsis and pulmonary sepsis for the biomarkers identified as being associated with sepsis (see Table 3). The data shown in the plot is as described for Figure 1.
**Figure 4****:** shows a plot of normalised gene expression in patients having SIRS, abdominal sepsis and pulmonary sepsis for the biomarkers identified as being associated with prognosis of recovery from a systemic inflammatory condition (see Tables 1 and 4). The data shown in the plot is as described for Figure 1.
**Figure 5****:** provides the results from ROC analysis of the gene expression data for the inflammation biomarkers when comparing healthy controls to disease patients.
**Figure 6****:** provides the results from ROC analysis of the gene expression data for the sepsis and SIRS biomarkers when comparing patients having sepsis to patients having SIRS.
**Figure 7****:** provides the results from ROC analysis of the gene expression data for the abdominal sepsis and pulmonary sepsis biomarkers when comparing patients having abdominal sepsis to patients having pulmonary sepsis.
**Figure 8****:** provides the results from ROC analysis of the gene expression data for the prognosis biomarker PKHD1, when comparing healthy controls to disease patients that survived SIRS.
**Figure 9****:** provides the results from ROC analysis of the gene expression data for the survival biomarkers when comparing disease patients that survived to disease patients that died.
**Figure 10****:** provides the results from ROC analysis of protein quantification data.

### EXAMPLE 1: IDENTIFICATION OF BIOMARKERS

### Patients:

Patients with severe sepsis and septic shock were recruited for the study based on the following criteria:
1. Age => 16
2. Diagnosis of severe sepsis
   - SEPSIS is defined as a (1) DEFINED FOCUS OF INFECTION AND (2) at least TWO systemic inflammatory response syndrome (SIRS) criteria.
      a) (1) DEFINED FOCUS OF INFECTION is indicated by either
         i. An organism grown in blood or sterile site OR
         ii. An abscess or infected tissue (e.g. pneumonia, peritonitis, urinary tract, vascular line infection, soft tissue, etc).
      b) (2) The 4 SIRS criteria are:
         i. CORE TEMPERATURE >38°C or <36°C. (Core temperature is rectal, urinary bladder, central line, or tympanic). If oral, inguinal or axillary temperatures are used, add 0.5°C to the measured value. Hypothermia <36°C must be confirmed by core temperature only. Use the most deranged value recorded in the 24 hours before ICU admission.
         ii. HEART RATE >90 beats/minute. If patient had an atrial arrhythmia, record the ventricular rate. If patients have a known medical condition or are receiving treatment that would prevent tachycardia (for example, heart block or beta blockers), they must meet two of the remaining three SIRS criteria. Use the most deranged value recorded in the 24 hours before ICU admission.
         iii. RESPIRATORY RATE > 20 breaths per minute or a PaCO₂ < 4.3 kPa (32 mmHg) or mechanical ventilation for an acute process. Use the most deranged respiratory rate or PaCO₂ recorded in the 24 hours before ICU admission.
         iv. WHITE BLOOD CELL COUNT of >12 × 10⁹/I or < 4 × 10⁹/I or > 10% immature neutrophils (band forms). Use the most deranged value recorded in the 24 hours before ICU admission.
   - SEVERE SEPSIS is defined as SEPSIS plus at least ONE ORGAN FAILURE, except when that organ failure was already present 48 hours before the onset of sepsis.
   - ORGAN FAILURE is defined as a Sequential Organ Failure Assessment (SOFA) score ≥ 2 for the organ in question
3. Presenting to hospital with abdominal or pulmonary sepsis of less than 72 hours duration
4. Patient already has or will require arterial cannulation as part of standard treatment

Patients with SIRS (Critically III patients without infection) were recruited based on the following criteria:
1. Patients admitted to the ICU following out-of hospital cardiac arrest
2. SIRS criteria as above
3. Organ failure criteria as above
4. Patients must not be receiving antibiotics for treatment of known or suspected infection
5. Patient already has or will require arterial cannulation as part of standard treatment

Exclusion Criteria
- age <16
- pregnant
- severe immune deficiency, for example
   a diagnosis of AIDS
   anti-rejection transplant drugs
- methotrexate
   high dose corticosteriod treatment (>10mg prednisolone/day or equivalent)
- Severe Liver Failure
   Childs III orworse

Volunteers above the age of 18 were recruited for use as healthy control individuals. Exclusion criteria included:
- Presence of current or chronic infection
- severe immune deficiency, for example a diagnosis of AIDS
   anti-rejection transplant drugs, methotrexate, high dose corticosteriod treatment (>10mg prednisolone/day or equivalent)
- severe acute or chronic liver disease
- presence of malignancy which is currently treated with chemo- or radiotherapy
- Irreversible disease with <6 months prognosis

### Sample collection and processing:

Blood samples were collected from the sepsis patients (abdominal sepsis N =54 and pulmonary sepsis patients N = 76) and SIRS patients (N =38) at day 1, day 2, and day 5 of admittance to an intensive care unit (ICU) and on discharge. One blood sample was collected from healthy volunteers (N= 30) similar to day 1 blood sampling of recruited patients.

5ml of whole heparinised blood obtained from patients was mixed with Erythrocyte Lysis (EL) Buffer (Qiagen) followed by incubation on ice for 10-15 minutes. Peripheral blood leukocytes (PBLs) were recovered from erythrocyte-lysed blood by centrifugation at 400 x g for 10 minutes at 4°C and resuspended in a further 2 ml of EL buffer. PBLs were again recovered by centrifugation as described above and processed for recovery of total RNA. RNA was then prepared from patient PBLs using a semi-automated process on the Maxwell^{®} 16 platform using the Maxwell^{®} 16 LEV simplyRNA Blood Kit. Concentration and purity (A260/A280 ratio ≥ 1.8) were then assessed by spectrophotometry using a NanoDrop ND-1000 spectrophotometer (Thermo Scientific). Human PBL mRNA samples were labelleled with Cy3 using the Agilent Quick Amp one colour labelling kit and then hybridised to Human SurePrint G3 Human Gene Expression v3 8x60K Microarrays according to the manufacturer's instructions. After hybridisation and wash steps the slides were scanned usning an Agilent SureScan Dx G5761AA Microarray Scanner using default settings.

### Gene expression analysis:

### Parametric; Analysis of Varience and Group T-tests

Raw data were exported and analysed using the bioinformatics software Genespring 12.5, for differential gene expression and statistical analyses. Raw data were normalized to the 75th percentile followed by baseline transformation to the mean of all samples. Data were assessed for quality, then filtered on gene expression where entities in all samples and all conditions had normalised expression values within the cut-off -10.699 to 7.037. Statistically significant features were identified using one-way ANOVA or T-test analyses across all entities, using the Benjamini-Hochberg False Discovery Rate (BH-FDR) multiple testing correction at a cut-off p < 0.05. Data were further analysed and depicted graphically using the heat map, hierarchical cluster analysis and other functions in Genespring 12.5, using default settings. To identify differentially expressed entities between patients having sepsis or SIRS and healthy individuals, fold change cut-off analysis was conduced using a default cut-off setting of > 2.5.

### Non Parametric; Artificial Neural Network Analyses

A stepwise Artificial Neural Network approach was used to identify an optimised gene signature panel comprising orthogonal genes from a previously established gene biomarker set for sepsis. The approach was repeated 5 times to 10 stepwise additions to assess the stability of the identified gene set given the number of cases provided. This was achieved using a stochastic data selection approach incorporating Monte Carlo cross validation.

### Architecture

The ANN modelling undertaken used a supervised learning approach applied to a three-layer multi-layer perceptron architecture. The initial weights matrix was randomised with a standard deviation of 0.1 to reduce the risk of over fitting the data. The ANN architecture was initially constrained to two hidden nodes in the hidden layer also for this reason. Hidden nodes and the output node incorporated a sigmoidal transfer function. During training weights were updated by a feed forward back propagation algorithm (Rumelhart, Hinton et al. 1986). Learning rate and momentum were set at 0.1 and 0.5 respectively. The output node was coded as 0 if the patient showed no evidence of sepsis, and 1 if sepsis was evident. Similar assessments were performed for patients with SIRS.

### Monte Carlo Cross validation

Prior to ANN training, the data was randomly divided into three subsets; 60% for training, 20% for testing (to assess model performance during the training process) and 20% for validation (to independently test the model on data completely blind to the model). This process of random sample cross validation also contributed to the reduction of over-fitting to the data and assess how well the model would perform on a blind data set.

### Stepwise model development for consistency analysis

The normalised intensity of each gene was used as an individual input in the ANN model, creating *n* individual models, where *n* was the number of genes in the provided panel. These *n* models were then split into three subsets (described above) and trained. This random resampling and training process was repeated 50 times to generate predictions and associated error values for each sample with respect to the validation (blind) data. Inputs were ranked in ascending order based on predictive error and the gene that performed with the lowest error was selected for further training. Next, each of the remaining genes were sequentially added to the previous best gene, and were used in combination in a model, creating *n-*1 models each containing two genes as inputs. Training was repeated and performance evaluated. The model with the highest modelling performance was again selected and the process repeated creating n-2 models each containing three inputs. This process was repeated until no significant gain was evident from the addition of further inputs. This resulted in a final model containing the expression signature that most accurately classified the patients according to development of sepsis or SIRS. A set of 85 biomarkers was identified as being useful for diagnosis and monitoring of the systemic inflammatory conditions sepsis and SIRS. The biomarkers identified as summarised below in Tables 1-4.

**Table 1: Biomarkers of systemic inflammation**

| Table 1 lists the genes identified as biomarkers of systemic inflammatory conditions using the above methods. The identified biomarkers are useful for diagnosis of systemic inflammatory conditions (e.g. see the biomarkers of inflammation shown in the top part of the table). The identified biomarkers are also useful for monitoring of systemic inflammatory conditions (e.g. see the biomarkers of inflammation shown in bottom part of the table). The final column gives the corrected ANOVA p value illustrating the significance of the biomarkers. | | |
|---|---|---|
| **Biomarkers of inflammation** | **Reference SEQ ID NO** | **Corrected P value** |
| ADM | 1 | 0.00000E+00 |
| CD177 | 2 | 0.00000E+00 |
| FAM20A | 3 | 0.00000E+00 |
| IL10 | 4 | 0.00000E+00 |
| METTL7B | 5 | 0.00000E+00 |
| MMP9 | 6 | 0.00000E+00 |
| RETN | 7 | 0.00000E+00 |
| TDRD9 | 8 | 0.00000E+00 |
| ITGA7 | 9 | 0.00000E+00 |
| BMX | 10 | 8.40000E-45 |
| HP | 11 | 1.62300E-42 |
| IGFBP2 | 12 | 1.06650E-40 |
| ALPL | 13 | 8.93141E-38 |
| DACH1 | 14 | 2.70941E-33 |
| IL1R1 | 15 | 9.00631E-32 |
| OLAH | 16 | 2.28395E-30 |
| IL1R2 | 17 | 3.78448E-30 |
| CYP19A1 | 18 | 7.40707E-25 |
| MMP8 | 19 | 3.19970E-23 |
| TGFA | 20 | 2.86E-17 |
| VSTM1 | 21 | 7.77E-13 |
| FCER1A | 22 | 0.00000E+00 |
| KLRK1 | 23 | 5.49388E-38 |
| KLRB1 | 24 | 2.32E-36 |
| DAAM2 | 25 | 5.58531E-34 |
| HLA-DRA | 26 | 2.54E-32 |
| BCL11B | 27 | 6.59918E-30 |
| ITM2A | 28 | 1.36751E-28 |
| SLAMF6 | 29 | 7.98235E-28 |
| HLA-DPB1 | 30 and 31 | 4.19667E-27 |
| CD160 | 32 | 1.72E-22 |
| KLRF1 | 33 | 5.17E-21 |
| CD2 | 34 | 2.62E-20 |
| LGALS2 | 35 | 3.15157E-10 |
| NPPC | 36 | 2.69E-08 |
| MYCL | 37 and 38 | 1.50E-07 |
| MX1 | 39 | 3.29E-04 |
| CCL5 | 40 | 1.81E-16 |

**Table 2: Biomarkers of SIRS**

| Table 2 lists the genes identified as biomarkers of SIRS using the above methods. The final column gives the corrected ANOVA p value illustrating the significance of the biomarkers. | | |
|---|---|---|
| **Biomarker** | **Reference SEQ ID NO** | **Corrected p value** |
| MYCL | 37 and 38 | 1.68E-21 |
| TGFBI | 41 | 1.34E-17 |
| PLA2G7 | 42 | 1.03E-14 |
| ARHGEF10L | 43 | 1.50E-14 |
| GPR124 | 44 | 2.25E-10 |
| IL1RN | 45 | 9.13350E-41 |
| NLRP3 | 46 | 6.43E-28 |
| RBP4 | 47 | 2.95E-21 |
| MPP3 | 48 | 7.99E-13 |

**Table 3: Biomarkers of sepsis**

| Table 3 lists the genes identified as biomarkers of sepsis using the above methods. The table also provides an indication as to whether the gene was observed to be elevated in abdominal or pulmonary sepsis. The final column gives the corrected ANOVA p value illustrating the significance of the biomarkers. | | | |
|---|---|---|---|
| **Level of biomarker observed in sepsis patients** | **Biomarker** | **Reference SEQ ID NO** | **Corrected p value** |
| High in abdominal and pulmonary sepsis | KIF2C | 49 | 4.83E-26 |
| High in abdominal and pulmonary sepsis | MAP1A | 50 | 2.46E-18 |
| High in abdominal and pulmonary sepsis | SELP | 51 | 2.27E-11 |
| High in abdominal and pulmonary sepsis | NEXN | 52 | 2.25E-10 |
| High in abdominal and pulmonary sepsis | ITGA2B | 53 | 4.65E-10 |
| High in abdominal and pulmonary sepsis | MYL9 | 54 | 1.06E-09 |
| High in abdominal and pulmonary sepsis | ITGB3 | 55 | 2.61E-09 |
| High in abdominal and pulmonary sepsis | CMTM5 | 56 | 4.91E-09 |
| High in abdominal and pulmonary sepsis | LCN2 | 57 | 1.04E-08 |
| High in abdominal and pulmonary sepsis | NLRC4 | 58 | 3.10376E-24 |
| High in abdominal and pulmonary sepsis | PPBP | 59 | 2.66E-08 |
| High in abdominal and pulmonary sepsis | TREML1 | 60 | 2.73E-08 |
| High in abdominal and pulmonary sepsis | PF4 | 61 | 2.83E-08 |
| High in abdominal and pulmonary sepsis | CLEC1B | 62 | 2.79E-07 |
| High in abdominal and pulmonary sepsis | LCN15 | 63 | 3.07E-06 |
| High in abdominal sepsis | C1QC | 64 | 0.00000E+00 |
| High in abdominal sepsis | C1QB | 65 | 8.49000E-43 |
| High in abdominal sepsis | PCOLCE2 | 66 | 5.85317E-36 |
| High in abdominal sepsis | C1QA | 67 | 2.12937E-30 |
| High in abdominal sepsis | TMEM37 | 68 | 2.30571E-24 |
| High in abdominal sepsis | TNF | 69 | 4.70E-07 |
| High in abdominal sepsis | SLC39A8 | 70 and 71 | 1.81E-05 |
| High in abdominal sepsis | MRAS | 72 | 1.27E-04 |
| Low in abdominal sepsis | IFIT1 | 73 | 1.48E-04 |
| Low in abdominal sepsis | IFI44 | 74 | 7.62E-04 |
| Low in abdominal sepsis | RPGRIP1 | 75 | 7.79E-04 |
| High in pulmonary sepsis | DISC1 | 76 | 6.02116E-36 |
| | | | |
| High in pulmonary sepsis | CXCR1 | 77 | 1.17E-20 |
| High in pulmonary sepsis | HCAR2 | 78 | 7.62E-04 |
| High in pulmonary sepsis | EPSTI1 | 79 | 7.62E-04 |

**Table 4: Biomarkers associated with prognosis of survival**

| Table 4 lists the genes identified as biomarkers of patient survival using the above methods. The final column gives the corrected ANOVA p value illustrating the significance of the biomarkers. | | |
|---|---|---|
| **Biomarker** | **Reference SEQ ID NO.** | **Corrected p value** |
| LILRB4 | 80 | 2.03E-32 |
| LILRB5 | 81 | 2.47E-22 |
| NECAB1 | 82 | 1.28E-07 |
| NECAB2 | 83 | 2.06E-05 |
| PKHD1 | 84 | 4.96E-05 |
| PKD1 | 85 | 3.15E-03 |

**Table 5:**

| Table 5 summarises the fold-changes observed in the amounts of the biomarkers quantified in the different patient samples as compared to the amounts quantified for the healthy control samples. | | | | | | | |
|---|---|---|---|---|---|---|---|
| Fold change data observed for the patients having non-infective SIRS: | | | | | | | |
| **Gene** | **Day 1 (Died)** | **Day 2 (Died)** | **Day 5 (Died)** | **Day 1 (Survived)** | **Day 2 (Survived)** | **Day 5 (Survived)** | **Discharged** |
| **ADM** | 12.76 | 11.22 | 5.70 | 11.68 | 14.20 | 11.25 | 12.33 |
| **ALPL** | 10.74 | 8.46 | 3.78 | 7.85 | 8.44 | 6.35 | 4.76 |
| **ARHGEF10L A33_P3799936** | 1.48 | 1.42 | 1.41 | 1.65 | 1.69 | 1.50 | 1.88 |
| **ARHGEF10L A33_P3215575** | -1.06 | -1.07 | -2.08 | -1.01 | 1.01 | 1.02 | -1.10 |
| **BMX** | 48.54 | 28.97 | 10.34 | 27.47 | 28.18 | 9.83 | 10.53 |
| **C1QA** | 2.50 | 6.64 | 5.26 | 2.18 | 5.00 | 10.21 | 6.11 |
| **C1QB** | 3.98 | 8.49 | 7.67 | 2.46 | 5.10 | 10.80 | 5.74 |
| **C1QC** | 3.52 | 7.01 | 8.80 | 4.25 | 4.84 | 10.09 | 5.01 |
| **CD177 A23 P0011751** | 84.52 | 45.72 | 6.97 | 29.94 | 53.15 | 15.96 | 7.28 |
| **CD177 A23 P259863** | 96.09 | 55.1 | 10.4 | 39.8 | 65.02 | 21.29 | 11.51 |
| **CCL5** | 3.036 | 3.61 | 10.49 | 5.49 | 4.02 | 2.55 | -3.126 |
| **CLEC1B** | 2.64 | 2.59 | 1.59 | 1.22 | 2.27 | 2.24 | 1.15 |
| **CMTM5** | 1.09 | -1.15 | -1.12 | -1.86 | -1.01 | 2.07 | 1.56 |
| **CXCR1** | 4.04 | 2.97 | 1.87 | 3.78 | 3.10 | 3.00 | 2.14 |
| **CYP19A1** | 7.60 | 8.37 | 3.88 | 4.16 | 4.05 | 2.59 | 1.36 |
| **CYP19A1** | 1.52 | 1.98 | 3.17 | 2.37 | 1.99 | 1.71 | 1.13 |
| **DAAM2** | 9.27 | 7.60 | 9.25 | 6.51 | 4.62 | 5.76 | 2.50 |
| **DAAM2** | 18.08 | 12.27 | 10.35 | 7.67 | 5.48 | 8.72 | 3.37 |
| **DACH1** | 8.03 | 5.96 | 13.08 | 4.81 | 5.44 | 5.30 | 6.75 |
| **DACH1** | 6.42 | 4.76 | 7.41 | 3.93 | 4.11 | 4.00 | 3.95 |
| **DISC1 A21 P000047** | 1.87 | 1.90 | -1.11 | 1.55 | 1.54 | 1.27 | -1.26 |
| **DISC1 A24 P83787** | -1.02 | -1.09 | -2.09 | -1.38 | -.135 | -1.31 | -2.43 |
| **DISC1 A21 P0000050** | -1.08 | 1.08 | -1.29 | 1.15 | -1.03 | -.122 | -2.45 |
| **EPSTI1** | -1.09 | -1.60 | -1.29 | -1.35 | -1.36 | -1.23 | -1.29 |
| **FAM20A A32 P108254** | 66.73 | 61.83 | 59.59 | 43.80 | 59.31 | 42.12 | 26.28 |
| **FAM20A A23 P352952** | 32.21 | 26.19 | 13.07 | 17.38 | 22.73 | 21.01 | 14.17 |
| **GPR124** | 1.99 | 1.67 | 2.22 | 2.68 | 2.40 | 1.63 | 2.05 |
| **HCAR2** | 1.86 | 1.01 | -1.14 | 1.95 | 1.53 | 1.90 | 2.05 |
| **HP** | 16.48 | 21.18 | 27.79 | 16.57 | 23.68 | 13.80 | 17.43 |
| **IFI44** | 1.76 | 1.08 | 2.21 | 1.74 | 1.51 | 1.69 | 2.23 |
| **IFIT1** | -1.87 | -2.38 | -1.08 | -1.49 | -1.52 | -1.13 | 1.24 |
| **IGFBP2** | 7.08 | 8.52 | 25.16 | 5.34 | 11.38 | 15.11 | 8.53 |
| **IL10** | 9.16 | 6.64 | 7.86 | 5.38 | 5.76 | 4.78 | 4.96 |
| **IL1R1** | 7.99 | 6.96 | 5.49 | 4.09 | 5.68 | 6.81 | 6.16 |
| **IL1R1** | 5.93 | 5.88 | 3.41 | 3.55 | 3.69 | 4.60 | 3.24 |
| **IL1R2** | 16.73 | 9.91 | 4.95 | 3.65 | 4.08 | 5.37 | 2.93 |
| **IL1RN** | 5.09 | 4.60 | 5.75 | 5.53 | 6.63 | 4.39 | 5.33 |
| **ITGA2B** | 1.05 | -1.21 | 1.21 | -1.57 | 1.09 | 2.89 | 1.56 |
| **ITGA7** | 10.23 | 11.84 | 16.50 | 5.20 | 8.16 | 9.65 | 8.53 |
| **ITGB3** | -1.20 | -1.61 | -1.74 | -2.62 | -1.49 | 2.06 | -1.14 |
| **KIF2C** | 2.04 | 1.48 | 1.07 | 1.14 | 1.27 | 1.21 | 1.09 |
| **LCN15** | -1.55 | -1.55 | -2.15 | -1.38 | -1.46 | -2.53 | -2.09 |
| **LCN2** | 1.24 | -1.09 | -1.53 | -1.46 | -1.11 | 1.99 | -1.04 |
| **LGALS2** | -3.29 | -2.05 | 1.56 | -2.01 | -1.77 | -2.26 | -1.37 |
| **MAP1A** | 1.04 | -1.03 | 1.17 | -1.22 | 1.14 | 2.35 | 1.11 |
| **METTL7B** | 19.59 | 21.29 | 27.43 | 11.40 | 15.60 | 10.67 | 11.96 |
| **MMP8** | 6.73 | 5.34 | 4.62 | 2.64 | 2.68 | 3.54 | 2.29 |
| **MMP9** | 64.93 | 40.17 | 17.27 | 66.89 | 53.46 | 17.91 | 15.55 |
| **MPP3** | 5.25 | 3.52 | 5.07 | 4.85 | 4.16 | 2.44 | 2.97 |
| **MRAS** | -1.04 | 1.02 | -1.59 | -1.62 | -1.38 | 1.05 | -1.05 |
| **MYCL** | -1.12 | 1.25 | 2.11 | 1.37 | 1.40 | 1.57 | 2.05 |
| **MYL9** | -1.43 | -2.36 | -1.56 | -2.46 | -2.09 | 2.08 | -1.19 |
| **NEXN** | 1.36 | 1.74 | 1.17 | -1.00 | 1.53 | 2.16 | 1.25 |
| **NLRC4** | 2.50 | 3.14 | 2.44 | 2.04 | 2.57 | 2.53 | 1.42 |
| **NLRP3** | 4.58 | 2.99 | 4.54 | 3.90 | 3.57 | 2.83 | 3.42 |
| **OLAH** | 14.67 | 11.01 | 21.25 | 4.87 | 5.10 | 11.28 | 6.73 |
| **PCOLCE2** | 26.63 | 46.09 | 23.79 | 9.34 | 21.46 | 16.50 | 23.87 |
| **PF4** | -1.26 | -1.33 | -1.62 | -2.23 | -1.54 | 1.22 | -1.64 |
| **PLA2G7** | 2.96 | 1.76 | 1.19 | 2.29 | 1.64 | -1.41 | 1.41 |
| **PPBP** | -1.69 | -1.99 | -1.86 | -3.14 | -2.80 | 1.06 | -1.51 |
| **RBP4** | 4.88 | 4.87 | 3.55 | 4.35 | 4.90 | 2.54 | 2.27 |
| **RETN** | 31.59 | 22.53 | 8.65 | 24.30 | 25.93 | 4.95 | 5.13 |
| **RPGRIP1** | -1.57 | -1.33 | -1.10 | 1.48 | -1.12 | -1.14 | -1.00 |
| **SELP** | 1.46 | 1.10 | 1.12 | -1.08 | 1.11 | 2.02 | 1.73 |
| **SLAMF6** | -9.03 | -6.76 | -10.21 | -8.49 | -11.62 | -6.07 | -8.10 |
| **SLC39A8** | 2.42 | 2.61 | 1.65 | 1.55 | 1.79 | 1.74 | 1.85 |
| **SLC39A8** | 3.17 | 2.99 | 2.48 | 1.45 | 2.49 | 1.91 | 2.31 |
| **TDRD9** | 9.07 | 13.78 | 12.47 | 7.01 | 12.75 | 10.53 | 10.55 |
| **TGFA** | 4.02 | 3.80 | 4.23 | 4.05 | 4.24 | 2.20 | 3.07 |
| **TGFBI** | 1.84 | 2.01 | 2.35 | 1.82 | 2.01 | 2.00 | 2.42 |
| **TMEM37** | 3.92 | 6.03 | 5.10 | 3.88 | 5.16 | 3.52 | 3.08 |
| **TNF** | 1.34 | 1.09 | 1.18 | 1.89 | 1.99 | 1.64 | 1.63 |
| **TREML1** | -1.16 | -1.58 | -1.43 | -1.88 | -1.39 | 2.12 | 1.24 |
| **VSTM1** | 2.95 | 3.20 | 4.38 | 3.46 | 4.89 | 3.52 | 3.37 |
| **LILRB4** | 6.22 | 5.48 | 7.70 | 6.22 | 6.13 | 6.01 | 3.25 |
| **LILRB5** | 5.39 | 4.65 | 8.63 | 2.99 | 3.71 | 6.79 | 5.04 |
| **NECAB1** | 1.67 | 1.86 | 4.28 | 2.21 | 1.98 | 1.37 | 1.14 |
| **NECAB2** | 1.64 | 2.07 | 2.48 | 2.44 | 1.82 | 3.26 | 1.99 |
| **PKD1 A21 P0011417** | 1.06 | 1.12 | 1.83 | 1.51 | 1.43 | 1.43 | 1.28 |
| **PKD1 A21 P0011418** | 1.19 | 1.26 | 1.65 | 1.61 | 1.51 | 1.21 | 1.29 |
| **PKD1 A21 P0011419** | 1.13 | 1.29 | 1.99 | 1.32 | 1.25 | 1.47 | 1.47 |
| **PKHD1 A23 P402187** | 1.64 | 1.80 | 5.37 | 2.31 | 2.07 | 1.43 | 1.13 |
| **PKHD1 A33 P3387420** | 1.80 | 1.88 | 4.10 | 2.39 | 2.13 | 1.54 | 1.13 |
| **PKHD1 A23 P424617** | 1.62 | 1.92 | 3.44 | 2.45 | 2.14 | 1.67 | 1.43 |
| **BCL11B** | -5.40 | -4.33 | -4.87 | -4.32 | -4.08 | -3.45 | -2.58 |
| **CD160** | -8.64 | -5.98 | -5.31 | -4.81 | -3.85 | -2.93 | -1.98 |
| **CD2** | -2.52 | -1.90 | -1.52 | -2.07 | -1.83 | -1.87 | -1.52 |
| **FCER1A** | -4.43 | -4.68 | -18.52 | -3.23 | -5.88 | -6.01 | -4.05 |
| **HLA-DPB1** | -1.48 | -1.40 | -2.63 | -1.63 | -1.89 | -1.69 | -1.46 |
| **HLA-DRA** | -2.36 | -2.51 | -4.49 | -1.83 | -2.43 | -2.23 | -2.13 |
| **ITM2A** | **-**7.47 | -5.46 | -8.00 | -8.28 | -6.37 | -4.09 | -4.10 |
| **KLRB1** | -4.25 | -4.15 | -4.68 | -3.78 | -4.37 | -3.51 | -2.22 |
| **KLRF1** | -3.91 | -2.08 | -4.00 | -3.82 | -4.33 | -3.20 | -1.80 |
| **KLRK1** | -10.31 | -9.22 | -12.30 | -11.26 | -10.70 | -7.17 | -4.84 |
| **MX1** | -1.05 | -1.39 | -1.17 | -1.10 | -1.13 | 1.06 | 1.18 |
| **NPPC** | 1.70 | 2.49 | 5.21 | 2.88 | 2.54 | 2.10 | 1.71 |

| Fold change data observed for the patients having abdominal sepsis: | | | | | | | |
|---|---|---|---|---|---|---|---|
| **Gene** | **Day 1 (Died)** | **Day 2 (Died)** | **Day 5 (Died)** | **Day 1 (Survived)** | **Day 2 (Survived)** | **Day 5 (Survived)** | **Discharged** |
| **ADM** | 18.02 | 13.38 | 11.65 | 14.42 | 12.70 | 9.65 | 8.40 |
| **ALPL** | 12.45 | 9.17 | 14.34 | 9.03 | 9.54 | 6.96 | 4.78 |
| **ARHGEF10L A33_P3799936** | -3.35 | -4.60 | -4.43 | -2.22 | -1.96 | -1.44 | -1.19 |
| **ARHGEF10L A33_P3215575** | -4.47 | -4.65 | -6.87 | -2.81 | -2.52 | -1.94 | -1.82 |
| **BMX** | 65.49 | 33.30 | 57.75 | 25.57 | 19.83 | 17.75 | 15.63 |
| **C1QA** | 19.63 | 19.88 | 4.44 | 16.80 | 15.48 | 6.77 | 6.57 |
| **C1QB** | 32.12 | 29.46 | 6.26 | 26.10 | 22.45 | 10.62 | 8.01 |
| **C1QC** | 32.73 | 37.30 | 9.33 | 24.37 | 19.57 | 9.96 | 7.33 |
| **CD177 A23 P0011751** | 361.83 | 181.90 | 166.05 | 156.28 | 123.04 | 86.65 | 37.29 |
| **CD177 A23 P259863** | 399.9 | 194.2 | 192.4 | 164.5 | 123.5 | 82.9 | 45.59 |
| **CCL5** | -2.27 | -4.01 | -9.35 | -2.89 | -3.35 | -2.65 | -1.92 |
| **CLEC1B** | 8.18 | 4.38 | 1.78 | 6.03 | 3.96 | 2.25 | 2.68 |
| **CMTM5** | 4.26 | 1.95 | 1.27 | 3.53 | 2.85 | 2.49 | 2.75 |
| **CXCR1** | 2.96 | 1.87 | 4.30 | 2.27 | 2.49 | 2.42 | 2.11 |
| **CYP19A1** | 11.69 | 11.27 | 15.25 | 10.40 | 7.53 | 2.04 | 3.10 |
| **CYP19A1** | 2.58 | 3.42 | 2.41 | 2.94 | 1.85 | 1.31 | 1.76 |
| **DAAM2** | 35.26 | 43.64 | 29.68 | 21.44 | 12.90 | 8.89 | 3.68 |
| **DAAM2** | 104.78 | 171.49 | 64.55 | 32.09 | 24.21 | 14.61 | 6.94 |
| **DACH1** | 6.08 | 4.84 | 11.87 | 6.02 | 5.30 | 4.62 | 4.12 |
| **DACH1** | 4.11 | 3.86 | 8.56 | 4.95 | 4.42 | 3.47 | 3.51 |
| **DISC1 A21 P000047** | 1.69 | 1.88 | 1.87 | 2.14 | 1.79 | 1.57 | 1.13 |
| **DISC1 A24 P83787** | 1.07 | 1.55 | -1.51 | 1.42 | 1.45 | -1.03 | -1.43 |
| **DISC1 A21 P0000050** | 1.18 | 1.44 | -1.45 | 1.40 | 1.41 | 1.03 | -1.27 |
| **EPSTI1** | -2.17 | -1.70 | -3.86 | -1.69 | -1.75 | -1.39 | -1.19 |
| **FAM20A A32 P108254** | 68.88 | 86.05 | 49.80 | 73.95 | 65.01 | 29.78 | 32.82 |
| **FAM20A A23 P352952** | 38.56 | 44.85 | 24.98 | 35.33 | 33.21 | 14.38 | 18.22 |
| **GPR124** | -1.62 | -3.48 | -1.77 | -1.49 | -1.50 | -1.97 | -1.42 |
| **HCAR2** | -1.79 | -1.33 | 1.37 | -1.19 | -1.21 | 1.42 | 1.21 |
| **HP** | 22.89 | 21.71 | 43.00 | 22.11 | 21.79 | 11.06 | 16.20 |
| **IFI44** | -1.29 | -1.22 | -1.32 | -1.18 | -1.18 | 1.19 | 1.41 |
| **IFIT1** | -5.32 | -5.32 | -5.79 | -5.06 | -3.78 | -1.99 | -1.41 |
| **IGFBP2** | 24.98 | 18.83 | 25.46 | 22.55 | 16.87 | 11.52 | 23.68 |
| **IL10** | 15.63 | 15.18 | 5.32 | 7.14 | 6.30 | 3.73 | 3.89 |
| **IL1R1** | 11.03 | 9.21 | 6.68 | 9.77 | 9.20 | 5.35 | 4.03 |
| **IL1R1** | 9.38 | 7.82 | 6.12 | 10.02 | 8.18 | 5.49 | 3.19 |
| **IL1R2** | 43.67 | 45.18 | 21.05 | 21.68 | 13.36 | 9.86 | 4.29 |
| **IL1RN** | 2.76 | 2.32 | 3.92 | 3.65 | 3.58 | 2.50 | 3.21 |
| **ITGA2B** | 6.67 | 2.29 | 1.15 | 3.99 | 3.37 | 2.44 | 3.24 |
| **ITGA7** | 37.95 | 33.99 | 33.50 | 29.68 | 30.12 | 16.43 | 12.50 |
| **ITGB3** | 4.65 | 2.13 | 1.33 | 4.18 | 3.48 | 2.49 | 3.00 |
| **KIF2C** | 5.11 | 4.09 | 7.40 | 3.07 | 3.07 | 4.40 | 2.67 |
| **LCN15** | 1.42 | 2.03 | 2.23 | 1.52 | 1.51 | 1.17 | 1.40 |
| **LCN2** | 6.56 | 3.92 | 7.78 | 2.95 | 2.49 | 3.98 | 2.76 |
| **LGALS2** | -33.71 | -26.99 | -15.62 | -8.21 | -5.38 | -2.36 | -1.30 |
| **MAP1A** | 2.85 | 1.87 | 1.25 | 2.72 | 2.18 | 1.84 | 2.60 |
| **METTL7B** | 41.83 | 22.91 | 32.65 | 36.49 | 34.87 | 11.98 | 19.14 |
| **MMP8** | 38.57 | 14.21 | 40.07 | 8.02 | 5.77 | 15.76 | 5.60 |
| **MMP9** | 69.09 | 36.91 | 103.28 | 48.51 | 33.60 | 30.38 | 19.48 |
| **MPP3** | 1.44 | -1.37 | 1.69 | 1.57 | 1.56 | 1.80 | 1.78 |
| **MRAS** | 1.56 | 1.25 | -1.92 | 1.15 | 1.38 | 1.21 | 1.20 |
| **MYCL** | -4.28 | -4.96 | -2.51 | -2.39 | -1.72 | -1.61 | -1.06 |
| **MYL9** | 4.36 | 1.44 | -1.31 | 2.78 | 2.31 | 2.60 | 3.36 |
| **NEXN** | 2.94 | 2.37 | -1.42 | 2.81 | 2.06 | 1.22 | 1.88 |
| **NLRC4** | 7.00 | 7.19 | 4.08 | 6.21 | 6.20 | 3.86 | 2.72 |
| **NLRP3** | 1.33 | 1.12 | 1.65 | 1.80 | 1.69 | 1.72 | 2.15 |
| **OLAH** | 111.81 | 79.31 | 69.24 | 35.20 | 21.17 | 10.08 | 4.35 |
| **PCOLCE2** | 158.63 | 103.55 | 74.11 | 95.70 | 53.00 | 14.20 | 9.46 |
| **PF4** | 2.53 | 1.27 | -1.78 | 1.86 | 1.39 | -1.00 | 1.41 |
| **PLA2G7** | -2.10 | -2.97 | -2.64 | -2.72 | -2.79 | -2.22 | -1.55 |
| **PPBP** | 2.70 | 1.35 | -2.03 | 1.76 | 1.47 | 1.07 | 1.37 |
| **RBP4** | 2.87 | 2.63 | 2.21 | 3.50 | 2.96 | 1.94 | 2.39 |
| **RETN** | 43.34 | 33.27 | 34.66 | 32.56 | 20.20 | 11.74 | 9.82 |
| **RPGRIP1** | -6.29 | -4.04 | -3.66 | -3.43 | -2.01 | -1.67 | 1.12 |
| **SELP** | 5.22 | 2.43 | 1.24 | 3.63 | 2.67 | 1.99 | 2.38 |
| **SLAMF6** | -3.42 | -4.23 | -5.00 | -6.26 | -4.98 | -4.31 | -4.34 |
| **SLC39A8** | 6.95 | 3.94 | 4.50 | 5.60 | 4.46 | 3.06 | 2.11 |
| **SLC39A8** | 5.54 | 4.64 | 3.69 | 4.63 | 3.77 | 2.31 | 2.28 |
| **TDRD9** | 30.63 | 21.31 | 23.78 | 21.71 | 19.72 | 12.25 | 9.00 |
| **TGFA** | 3.42 | 3.57 | 2.13 | 3.67 | 3.08 | 1.99 | 2.35 |
| **TGFBI** | -2.31 | -2.60 | -2.53 | -1.27 | -1.08 | -1.20 | 1.17 |
| **TMEM37** | 12.43 | 13.91 | 10.08 | 7.61 | 7.42 | 4.39 | 3.53 |
| **TNF** | 1.53 | 1.54 | 1.53 | 1.40 | 1.39 | 1.59 | 1.64 |
| **TREML1** | 3.51 | 1.34 | -1.71 | 2.63 | 2.07 | 1.90 | 2.14 |
| **VSTM1** | 3.04 | 2.25 | 3.96 | 3.17 | 3.40 | 2.67 | 3.03 |
| **LILRB4** | 3.80 | 4.02 | 3.46 | 4.28 | 3.81 | 3.42 | 4.00 |
| **LILRB5** | 6.91 | 8.77 | 4.08 | 6.27 | 6.64 | 4.65 | 3.23 |
| **NECAB1** | 1.34 | 2.15 | 1.25 | 1.93 | 1.44 | 1.21 | 1.57 |
| **NECAB2** | 1.42 | 3.72 | 3.72 | 1.46 | 1.95 | 2.54 | 2.10 |
| **PKD1 A21 P0011417** | -1.35 | 1.03 | 2.29 | 1.00 | 1.15 | 1.22 | 1.10 |
| **PKD1 A21 P0011418** | -1.16 | -1.05 | 1.60 | 1.11 | -1.07 | 1.07 | 1.09 |
| **PKD1 A21 P0011419** | -1.16 | 1.13 | 2.46 | -1.07 | 1.11 | 1.49 | 1.12 |
| **PKHD1 A23 P402187** | 1.41 | 2.30 | 1.31 | 1.90 | 1.50 | 1.34 | 1.60 |
| **PKHD1 A33 P3387420** | 1.52 | 2.22 | 1.25 | 2.05 | 1.42 | 1.24 | 1.48 |
| **PKHD1 A23 P424617** | 1.48 | 2.34 | 1.36 | 1.97 | 1.58 | 1.28 | 1.66 |
| **BCL11B** | -4.74 | -4.80 | -4.44 | -6.01 | -4.67 | -3.64 | -2.68 |
| **CD160** | -9.36 | -7.69 | -10.27 | -6.30 | -5.44 | -3.73 | -2.12 |
| **CD2** | -3.04 | -2.89 | -2.54 | -2.77 | -2.51 | -1.95 | -1.54 |
| **FCER1A** | -33.69 | -36.09 | -20.29 | -26.26 | -19.06 | -12.11 | -6.01 |
| **HLA-DPB1** | -3.58 | -5.27 | -6.89 | -2.79 | -2.36 | -2.46 | -1.73 |
| **HLA-DRA** | -3.54 | -4.95 | -6.57 | -4.49 | -3.39 | -3.24 | -2.17 |
| **ITM2A** | -3.57 | -4.43 | -5.24 | -4.83 | -3.79 | -3.24 | -2.81 |
| **KLRB1** | -4.85 | -5.79 | -6.96 | -5.37 | -5.08 | -3.82 | -2.66 |
| **KLRF1** | -3.12 | -3.86 | -8.47 | -4.83 | -4.73 | -3.74 | -2.95 |
| **KLRK1** | -6.45 | -6.70 | -18.02 | -9.16 | -8.49 | -7.36 | -5.40 |
| **MX1** | -2.69 | -2.51 | -2.75 | -2.20 | -2.02 | -1.36 | -1.20 |
| **NPPC** | 1.95 | 2.68 | 3.06 | 2.22 | 2.30 | 1.49 | 1.79 |

| Fold change data observed for patients having pulmonary sepsis: | | | | | | | |
|---|---|---|---|---|---|---|---|
| **Gene** | **Day 1 (Died)** | **Day 2 (Died)** | **Day 5 (Died)** | **Day 1 (Survived)** | **Day 2 (Survived)** | **Day 5 (Survived)** | **Discharged** |
| **ADM** | 12.80 | 11.51 | 9.29 | 11.42 | 11.36 | 7.53 | 5.45 |
| **ALPL** | 11.08 | 10.71 | 7.00 | 9.95 | 9.44 | 6.55 | 4.14 |
| **ARHGEF10L A33_P3799936** | -2.19 | -2.31 | -1.57 | -2.11 | -2.19 | -1.28 | 1.31 |
| **ARHGEF10L A33_P3215575** | -2.96 | -3.33 | -2.35 | -2.48 | -2.75 | -1.70 | -1.05 |
| **BMX** | 29.56 | 25.38 | 15.36 | 21.20 | 16.64 | 12.04 | 6.00 |
| **C1QA** | 7.23 | 6.19 | 2.16 | 5.44 | 5.22 | 3.44 | 3.09 |
| **C1QB** | 11.75 | 9.49 | 2.94 | 8.77 | 8.51 | 5.29 | 3.80 |
| **C1QC** | 12.02 | 8.11 | 3.30 | 8.09 | 9.05 | 6.14 | 4.59 |
| **CD177 A23 P0011751** | 79.88 | 41.82 | 39.98 | 104.30 | 77.61 | 43.90 | 5.89 |
| **CD177 A23 P259863** | 79.44 | 45.32 | 35.42 | 110.3 | 81.38 | 49.59 | 7.27 |
| **CCL5** | -2.49 | -2.24 | -1.70 | -2.40 | -2.59 | -2.12 | -1.55 |
| **CLEC1B** | 5.36 | 3.41 | 2.63 | 4.91 | 3.57 | 2.69 | 1.71 |
| **CMTM5** | 3.23 | 2.56 | 2.72 | 3.28 | 2.97 | 3.02 | 1.95 |
| **CXCR1** | 4.50 | 5.40 | 4.01 | 3.50 | 3.79 | 3.74 | 2.11 |
| **CYP19A1** | 8.14 | 5.51 | 3.89 | 6.34 | 5.62 | 2.49 | 1.42 |
| **CYP19A1** | 2.04 | 2.13 | 1.07 | 2.27 | 1.89 | 1.43 | 1.33 |
| **DAAM2** | 22.74 | 17.26 | 11.13 | 15.23 | 20.25 | 11.74 | 5.68 |
| **DAAM2** | 34.62 | 28.99 | 19.24 | 24.68 | 31.64 | 21.42 | 12.88 |
| **DACH1** | 5.19 | 4.64 | 6.99 | 5.30 | 4.87 | 4.67 | 2.24 |
| **DACH1** | 4.08 | 3.87 | 4.22 | 4.44 | 4.60 | 3.86 | 1.91 |
| **DISC1 A21 P000047** | 3.31 | 2.71 | 1.79 | 3.31 | 2.93 | 1.95 | 1.69 |
| **DISC1 A24 P83787** | 1.99 | 1.62 | 1.39 | 3.3 | 1.82 | 1.15 | 1.05 |
| **DISC1 A21 P0000050** | 2.04 | 2.13 | 1.15 | 3.62 | 1.78 | 1.15 | 1.36 |
| **EPSTI1** | 1.28 | 1.11 | -1.97 | 1.16 | 1.06 | -1.18 | 1.22 |
| **FAM20A A32 P108254** | 42.38 | 35.60 | 29.98 | 49.56 | 48.43 | 18.99 | 9.03 |
| **FAM20A A23 P352952** | 21.87 | 18.64 | 19.49 | 24.61 | 24.69 | 10.81 | 5.66 |
| **GPR124** | -1.27 | -1.22 | -1.16 | -1.26 | -1.38 | -1.28 | -1.01 |
| **HCAR2** | 2.61 | 2.96 | 1.83 | 1.53 | 1.46 | 1.85 | 2.01 |
| **HP** | 20.38 | 17.05 | 13.78 | 16.06 | 15.98 | 12.28 | 3.85 |
| **IFI44** | 1.97 | 1.92 | 1.25 | 2.08 | 1.73 | 1.31 | 1.90 |
| **IFIT1** | -1.23 | -1.04 | -1.75 | -1.71 | -1.71 | -1.76 | -1.01 |
| **IGFBP2** | 14.49 | 12.66 | 7.72 | 11.68 | 12.13 | 9.35 | 5.09 |
| **IL10** | 7.01 | 5.77 | 4.95 | 5.95 | 5.40 | 4.90 | 1.91 |
| **IL1R1** | 9.66 | 9.49 | 7.58 | 8.23 | 9.12 | 6.61 | 3.82 |
| **IL1R1** | 10.91 | 7.62 | 6.97 | 8.90 | 9.08 | 6.05 | 3.93 |
| **IL1R2** | 23.07 | 18.09 | 9.14 | 19.90 | 18.26 | 13.36 | 4.09 |
| **IL1RN** | 3.83 | 3.70 | 3.31 | 3.46 | 3.44 | 2.93 | 2.30 |
| **ITGA2B** | 3.27 | 3.21 | 3.32 | 3.12 | 2.79 | 3.09 | 2.15 |
| **ITGA7** | 13.21 | 11.13 | 8.60 | 14.92 | 16.81 | 9.64 | 3.13 |
| **ITGB3** | 3.56 | 2.41 | 2.53 | 3.21 | 3.00 | 3.02 | 1.82 |
| **KIF2C** | 1.90 | 2.08 | 1.58 | 2.56 | 2.54 | 2.55 | 1.76 |
| **LCN15** | 1.24 | 1.42 | 1.47 | 1.29 | 1.38 | 1.10 | -1.52 |
| **LCN2** | 2.31 | 2.67 | 2.71 | 3.01 | 2.85 | 3.66 | 1.91 |
| **LGALS2** | -4.81 | -3.39 | -1.80 | -6.29 | -4.79 | -2.14 | -2.15 |
| **MAP1A** | 2.47 | 2.46 | 2.30 | 2.40 | 2.20 | 2.21 | 1.66 |
| **METTL7B** | 19.19 | 19.74 | 14.88 | 14.65 | 14.85 | 8.00 | 4.33 |
| **MMP8** | 4.03 | 3.55 | 4.48 | 6.85 | 6.96 | 9.21 | 3.31 |
| **MMP9** | 30.61 | 21.38 | 16.84 | 33.13 | 27.09 | 23.30 | 8.62 |
| **MPP3** | 1.43 | 1.22 | 1.96 | 1.66 | 1.56 | 1.64 | 1.76 |
| **MRAS** | -2.07 | -2.85 | -2.27 | -1.78 | -1.86 | -2.06 | -1.49 |
| **MYCL** | -2.13 | -2.29 | -1.39 | -2.45 | -2.04 | -1.40 | -1.04 |
| **MYL9** | 2.89 | 2.56 | 3.22 | 2.30 | 2.05 | 3.08 | 2.03 |
| **NEXN** | 2.54 | 2.07 | 1.14 | 2.60 | 2.20 | 1.65 | 1.26 |
| **NLRC4** | 5.12 | 4.55 | 4.05 | 4.90 | 5.18 | 3.12 | 2.08 |
| **NLRP3** | 1.79 | 1.48 | 1.91 | 1.72 | 1.66 | 1.75 | 1.69 |
| **OLAH** | 32.24 | 30.02 | 18.21 | 30.43 | 35.43 | 18.81 | 5.40 |
| **PCOLCE2** | 31.87 | 20.26 | 15.76 | 46.07 | 36.83 | 17.08 | 5.06 |
| **PF4** | 2.21 | 1.66 | 1.59 | 1.89 | 1.50 | 1.39 | 1.17 |
| **PLA2G7** | -2.36 | -3.66 | -2.11 | -2.72 | -3.02 | -2.36 | -1.29 |
| **PPBP** | 1.71 | 1.09 | 1.61 | 1.61 | 1.37 | 1.54 | 1.24 |
| **RBP4** | 3.25 | 2.99 | 2.13 | 3.05 | 2.86 | 2.20 | 1.76 |
| **RETN** | 16.73 | 11.23 | 8.32 | 15.61 | 12.18 | 7.48 | 3.55 |
| **RPGRIP1** | -1.30 | -1.06 | -1.01 | -1.74 | -1.46 | -1.08 | 1.49 |
| **SELP** | 2.92 | 2.52 | 2.71 | 3.31 | 2.72 | 2.53 | 1.68 |
| **SLAMF6** | -5.85 | -4.75 | -3.82 | -5.94 | -5.34 | -4.80 | -1.38 |
| **SLC39A8** | 1.66 | 1.83 | 1.62 | 2.95 | 2.49 | 1.36 | 1.05 |
| **SLC39A8** | 1.84 | 1.49 | 1.35 | 2.09 | 2.16 | 1.33 | 1.10 |
| **TDRD9** | 12.54 | 9.68 | 8.48 | 14.69 | 14.74 | 10.01 | 3.96 |
| **TGFA** | 4.37 | 4.42 | 3.13 | 3.85 | 3.24 | 2.42 | 1.81 |
| **TGFBI** | -1.26 | -1.30 | -1.09 | -1.47 | -1.30 | -1.04 | 1.16 |
| **TMEM37** | 3.83 | 3.78 | 2.45 | 3.36 | 3.99 | 2.93 | 1.72 |
| **TNF** | 1.04 | 1.09 | 1.15 | 1.17 | 1.24 | 1.21 | 1.29 |
| **TREML1** | 2.25 | 2.16 | 2.57 | 2.40 | 2.07 | 2.46 | 1.65 |
| **VSTM1** | 2.05 | 2.50 | 2.54 | 2.60 | 2.97 | 2.78 | 1.87 |
| **LILRB4** | 3.94 | 4.20 | 3.52 | 4.02 | 3.48 | 3.15 | 3.31 |
| **LILRB5** | 6.68 | 5.24 | 2.24 | 5.65 | 6.12 | 5.07 | 2.69 |
| **NECAB1** | 1.63 | 1.73 | 1.38 | 1.70 | 1.49 | 1.23 | 1.32 |
| **NECAB2** | 3.38 | 4.70 | 4.23 | 1.85 | 2.61 | 3.50 | 1.90 |
| **PKD1 A21 P0011417** | 1.39 | 1.30 | 1.38 | 1.26 | 1.12 | 1.30 | 1.47 |
| **PKD1 A21 P0011418** | 1.45 | 1.43 | 1.37 | 1.04 | -1.02 | 1.02 | 1.16 |
| **PKD1 A21 P0011419** | 1.25 | 1.32 | 1.32 | -1.1 | 1.05 | 1.14 | 1.37 |
| **PKHD1 A23 P402187** | 1.72 | 1.86 | 1.09 | 1.80 | 1.64 | 1.35 | 1.43 |
| **PKHD1 A33 P3387420** | 1.97 | 1.87 | 1.01 | 1.79 | 1.63 | 1.43 | 1.41 |
| **PKHD1 A23 P424617** | 1.83 | 1.95 | 1.13 | 1.61 | 1.61 | 1.36 | 1.63 |
| **BCL11B** | -6.77 | -6.65 | -3.57 | -5.19 | -4.82 | -3.97 | -2.46 |
| **CD160** | -7.07 | -5.68 | -3.58 | -7.20 | -5.83 | -6.05 | -2.24 |
| **CD2** | -2.71 | -2.39 | -1.52 | -2.66 | -2.49 | -2.16 | -1.31 |
| **FCER1A** | -21.37 | -32.23 | -10.36 | -19.22 | -20.68 | -11.38 | -3.32 |
| **HLA-DPB1** | -3.67 | -3.40 | -4.00 | -3.28 | -3.12 | -3.06 | -1.86 |
| **HLA-DRA** | -4.57 | -5.05 | -3.90 | -4.34 | -4.22 | -3.38 | -2.24 |
| **ITM2A** | -4.69 | -5.18 | -4.13 | -3.79 | -3.91 | -3.15 | -2.67 |
| **KLRB1** | -6.68 | -6.59 | -3.09 | -5.33 | -5.70 | -3.82 | -2.42 |
| **KLRF1** | -5.83 | -4.86 | -2.46 | -4.55 | -4.95 | -3.82 | -2.44 |
| **KLRK1** | -10.84 | -9.50 | -5.69 | -9.74 | -8.63 | -6.89 | -4.47 |
| **MX1** | 1.04 | 1.12 | -1.20 | -1.03 | -1.09 | -1.31 | 1.06 |
| **NPPC** | 2.55 | 2.63 | 1.66 | 2.17 | 1.79 | 1.71 | 1.83 |

### EXAMPLE 2: ANALYSIS OF BIOMARKER PERFORMANCE

To further investigate the performance of each biomarker in the diagnosis and monitoring of systemic inflammatory disease, Receiver Operating Characteristic (ROC) analysis was used to investigate the ability of the biomarkers to discriminate between different disease conditions and/or recovery status using the gene expression expression data obtained from each group of patients.

All ROC curve analysis was performed using R software and the ROCR package using the following commands:
Each data value is assigned a predictor label where negatives (non-infected) are "0" and positives (infected) are "1" and columns are saved as .txt file to be imported into R. -(GBP <- read.table("GBP.txt", header=T))

To plot ROC CURVE:

```
 -pred <- prediction(GBP$GBP1, GBP$labels)
 -perf <- performance(pred, measure="tpr", x.measure="fpr")
 -plot(perf); abline(0,1) (# This plots FPR (1-SPEC)
 on the x axis and TPR (==SENS) on the y axis.)
```

To measure Area Under Curve Value:

```
 -auc.perf <- performance(pred, measure="auc") (# generate performance object with AUC)
 -auc.perf@y.values (# and extract the AUC value thus)
```

To plot accuracy and predict optimal accuracy cutoff values (Accuracy is (TP+TN)/(P+N) or (TP+TN)/(TP+FN+FP+FN) which is the total number of True Positives & True Negatives over the sum of the whole population

```
-acc.perf <- performance(pred, measure = "acc")
 -plot(acc.perf)
 -ind = which.max(slot(acc.perf, "y.values")[[1]])
 -acc = slot(acc.perf, "y.values")[[1]][ind]
 -cutoff = slot(acc.perf, "x.values")[[1]][ind]
 -print(c(accuracy= acc, cutoff = cutoff))
 -abline(cutoff,1)
 library(ROCR)
 data(ROCR.simple)
 pred <- prediction(ROCR.simple$predictions, ROCR.simple$labels)
 perf <- performance(pred,"tpr","fpr")
 plot(perf,colorize=TRUE)
 abline(0,1, col="red")
 auc <- performance(pred, "auc")@y.values[[1]]
 legend(0.5,0.4,paste(c("AUC = "), round(auc,2), sep=""))
```

ROC analysis as described above was used to determine the sensitivity and specificity with which biomarkers of the invention discriminate between: (i) patients having a systemic inflammatory condition and healthy controls; (ii) patients having sepsis and patients having SIRS; (iii) patients having abdominal sepsis and patients having pulmonary sepsis; and (iv) healthy controls and patients that recover from a systemic inflammatory condition; and (v) patients that recover from a systemic inflammatory condition and patients that do not recover from a systemic inflammatory condition.

### ROC analysis of the inflammatory markers

Table 1 summarises the genes identified as general biomarkers of all systemic inflammatory conditions using gene expression analysis. To demonstrate that these biomarkers permit accurate diagnosis of systemic inflammatory conditions in patients, the "Day 1" gene expression data obtained for all patients was analysed by ROC analysis to determine how well the biomarkers discriminate between patients having a systemic inflammatory condition (including patients having sepsis and patients having SIRS) and healthy controls. ROC analysis of the Day 1 fold change gene expression data was performed as described above.

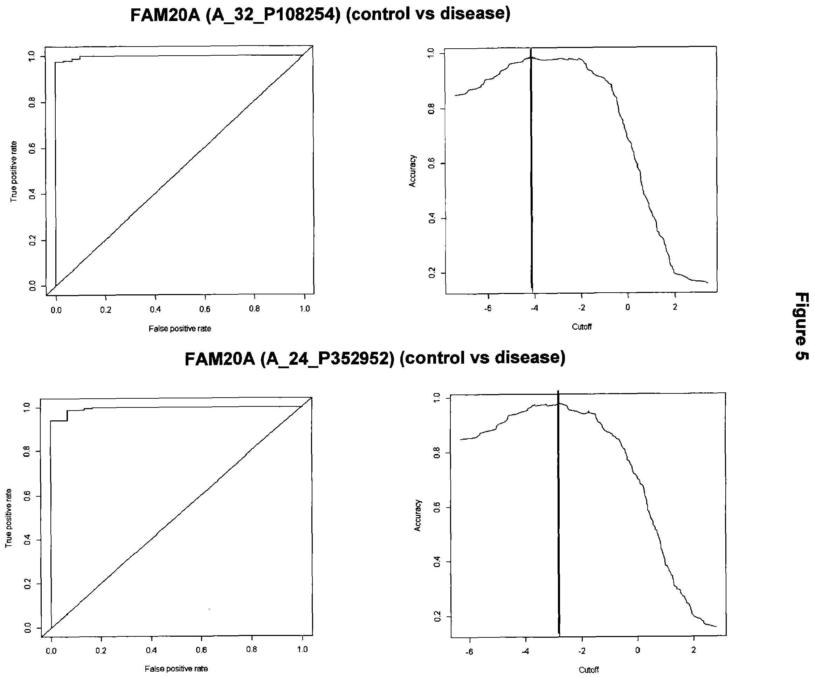

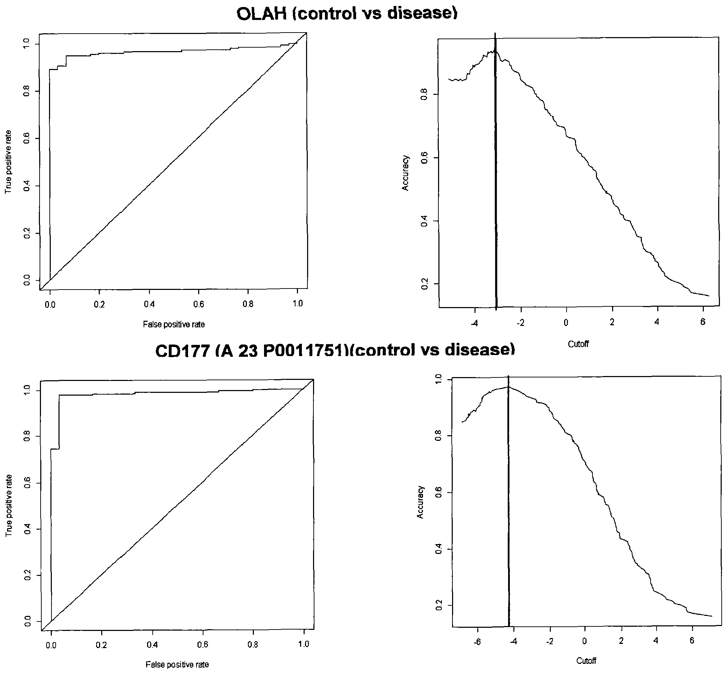

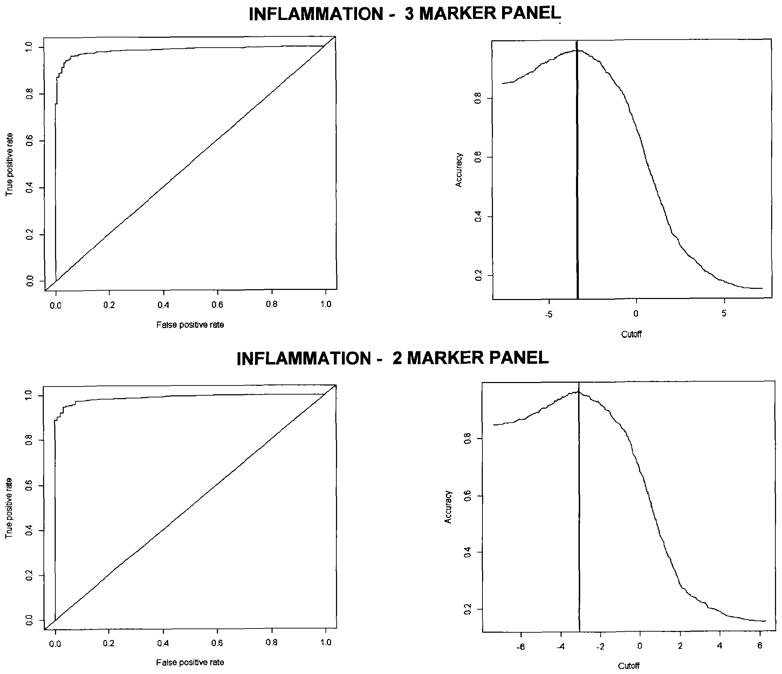

The ROC curves obtained for the best performing inflammation biomarkers (FAM20A, OLAH and CD177) are shown in Figure 5. In a ROC curve the true positive rate (Sensitivity) is plotted in function of the false positive rate (100-Specificity) for different cut-off points of a parameter. Each point on the ROC curve represents a sensitivity/specificity pair corresponding to a particular decision threshold. The area under the ROC curve (AUC) is a measure of how well a parameter can distinguish between two diagnostic groups (diseased/normal). A ROC curve plots Sensitivity on the Y axis and 1-specificity on the X axis for a "family" of threshold cut-offs (1-specificity is also known as the False Positive Rate (FPR). A test with perfect discrimination (no overlap in the two distributions) has a ROC curve that passes through the upper left corner (100% sensitivity, 100% specificity). Therefore the closer the ROC curve is to the upper left corner, the higher the overall accuracy of the test (Zweig & Campbell, 1993). AUC and ACC values calculated for the best performing biomarkers are summarised in the table below. AUC values above 0.6 indicate that the biomarker discriminates between the patient populations being tested.

**Table 6**

| **BIOMARKER** | **PROBE NUMBER** | **Area under Curve (AUC)** | **Accuracy (ACC)** | **Cut-off** |
|---|---|---|---|---|
| **FAM20A** | A_32_P108254 | 0.9982143 | 0.9848485 | -4.0993210 |
| **FAM20A** | A_24_P352952 | 0.9950397 | 0.979798 | -2.818870 |
| **OLAH** | A_23_P161458 | 0.9634921 | 0.9444444 | -3.0706300 |
| **CD177** | A_21_P0011751 | 0.9805556 | 0.9747475 | -4.2479086 |
| **CD177** | A_23_P259863 | 0.9876984 | 0.9747475 | -3.9516115 |
| **2 Marker panel (FAM20A; OLAH)** | A_32_P108254 | 0.9882496 | 0.96633 | -3.07063 |
| | A_24_P352952 | | | |
| | A_23_P161458 | | | |
| **3 MARKER PANEL (FAM20A, OLAH; CD177)** | A_32_P108254 | 0.9847222 | 0.9606061 | -3.3482232 |
| | A_24_P352952 | | | |
| | A_23_P161458 | | | |
| | A_21_P0011751 | | | |
| | A_23_P259863 | | | |

As demonstrated by the AUC and ACC values reported in the above table, FAM20A, OLAH and CD177 were observed to specifically and sensitively distinguish between healthy control patients (having no systemic inflammatory disease) and patients having a systemic inflammatory condition (i.e those having sepsis or SIRS) when used on their own. The combination of FAM20A and OLAH and the combination of FAM20A, OLAH and CD177 also specifically and sensitively distinguished between patients having a systemic inflammatory condition and healthy controls. These biomarkers may therefore be preferably used in the methods of the invention to diagnose systemic inflammation.

### ROC analysis of the sepsis and SIRS markers

Tables 2 and 3 summarise the genes identified as sepsis biomarkers and SIRS biomarkers using gene expression analysis. To demonstrate that these biomarkers permit accurate diagnosis of sepsis and SIRS (and distinguish between these specific disease conditions), the "Day 1" gene expression data obtained for all disease patients was analysed by ROC analysis to determine how well the biomarkers discriminate between patients having sepsis (including patients having abdominal sepsis and patients having pulmonary sepsis) and patients having SIRS. ROC analysis of the Day 1 fold change gene expression data was performed as described above.

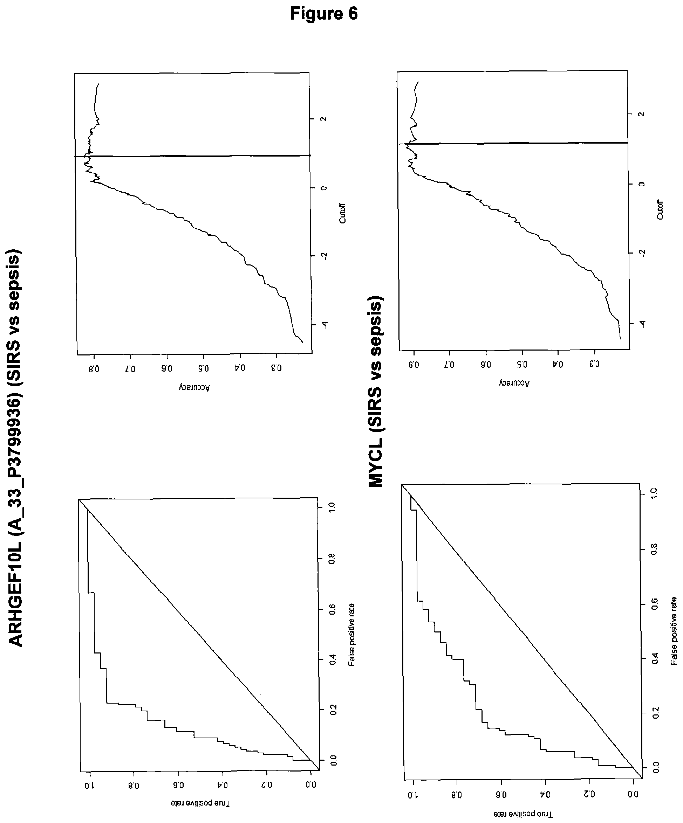

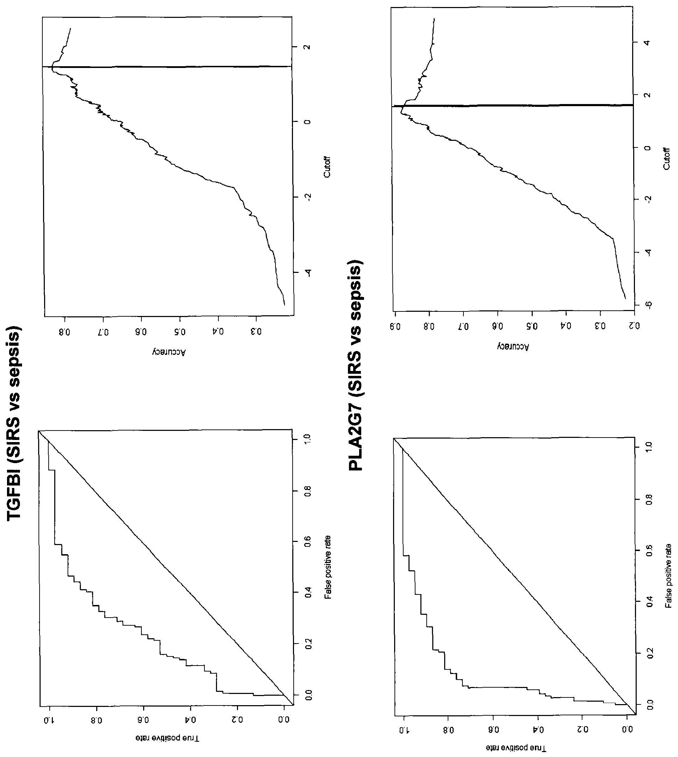

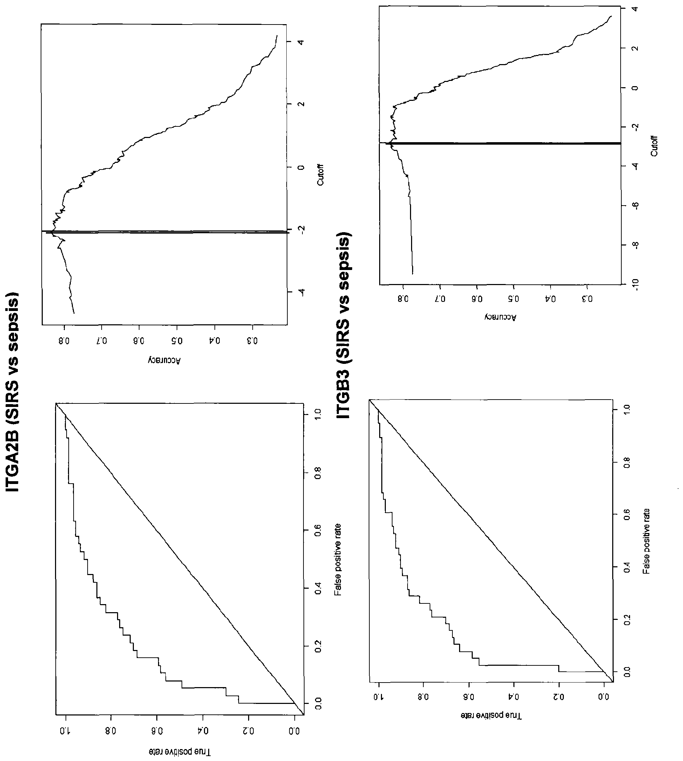

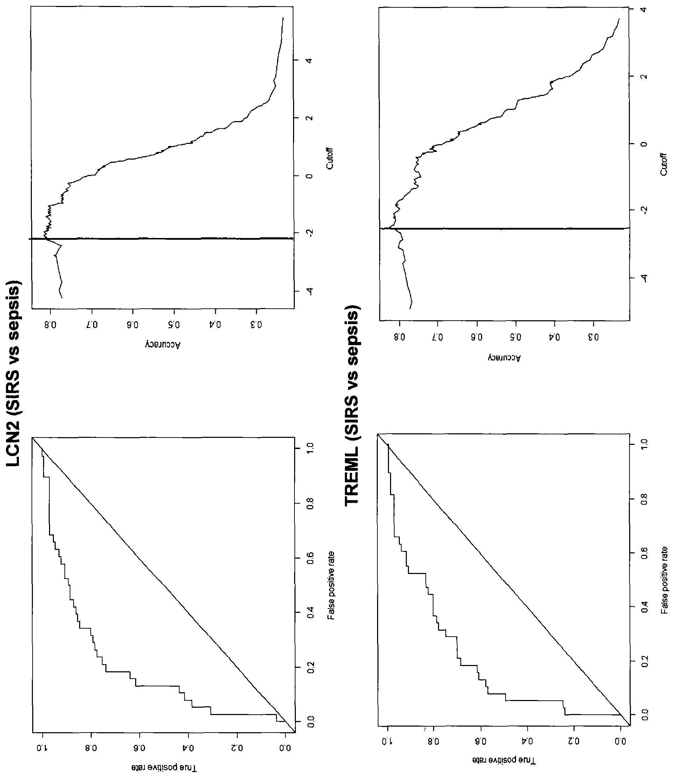

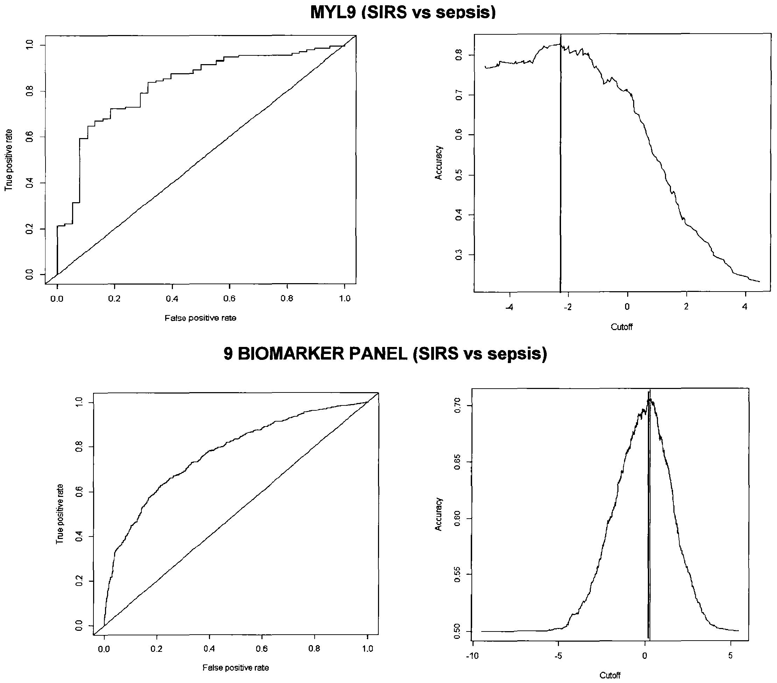

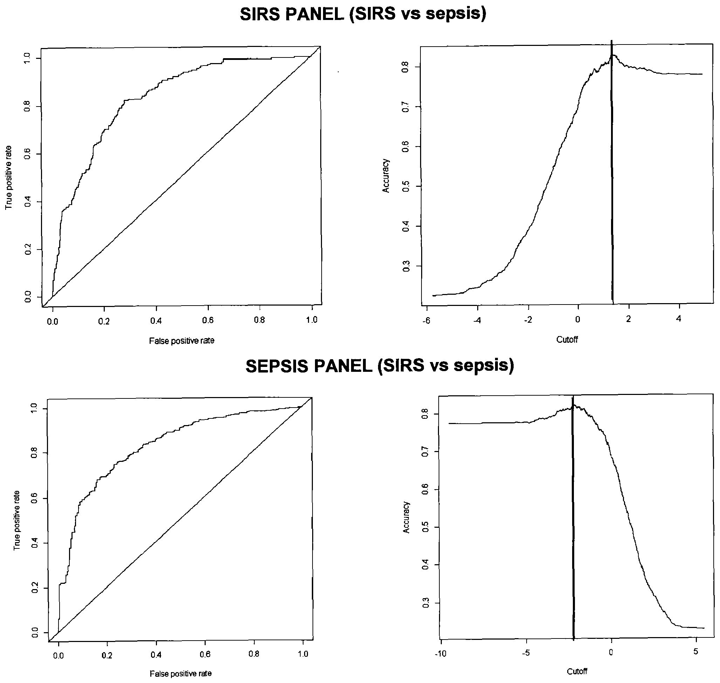

The ROC curves obtained for the best performing biomarkers are shown in Figure 6. AUC and ACC values calculated for these biomarkers are summarised in the table below.

**Table 7**

| **BIOMARKER** | **PROBE NUMBER** | **Area under Curve (AUC)** | **Accuracy (ACC)** | **Cut-off** | **SIRS/SEPSIS** |
|---|---|---|---|---|---|
| **ARGHEF10L** | A_33_P3799936 | 0.8684211 | 0.8214286 | 0.9472856 | +/- |
| **ARGHEF10L** | A_33_P3215575 | 0.8016194 | 0.8154762 | 1.4029250 | +/- |
| **MYCL** | A_33_P3306068 | 0.8008097 | 0.8154762 | 1.1467919 | +/- |
| **TGFBI** | A_23_P156327 | 0.7884615 | 0.827381 | 1.461718 | +/- |
| **PLA2G7** | A_23_P145096 | 0.890081 | 0.8809524 | 1.5761456 | +/- |
| | A_24_P145096 | | | | |
| **ITGB3** | A_24_P318656 | 0.8597166 | 0.8392857 | | -/+ |
| **ITGA2B** | A_24_P65373 | 0.8321862 | 0.8333333 | -2.05138 | -/+ |
| **MYL9** | A_23_P210425 | 0.8234818 | 0.827381 | -2.277507 | -/+ |
| **LCN2** | A_23_P169437 | 0.8125506 | 0.8214286 | -2.1682110 | -/+ |
| **TREML1** | A_33_P338177 | 0.8074899 | 0.827381 | -2.528779 | -/+ |
| | | | | | |
| **8 BIOMARKER PANEL (PLA2G7 REMOVED)** | ALL PROBES | 0.770304 | 0.7050265 | 0.1868129 | |
| **9 BIOMARKER** | ALL PROBES | 0.7691327 | 0.7065476 | 0.3061080 | |
| **PANEL** | | | | | |
| **SIRS PANEL (ARGHEF10L MYCL TGFBI PLA2G7)** | A_33_P3799936 | 0.8298785 | 0.825000 | 1.402111 | +/- |
| | A_33_P3215575 | | | | |
| | A_33_P3306068 | | | | |
| | A_23_P156327 | | | | |
| | A_23_P145096 | | | | |
| **SEPSIS PANEL (ITGB3 ITGA2B MYL9 LCN2 TREML1)** | A_24_P318656 | 0.827417 | 0.8238095 | -2.1682110 | -/+ |
| | A_24_P65373 | | | | |
| | A_23_P210425 | | | | |
| | A_23_P169437 | | | | |
| | A_33_P338177 | | | | |

As demonstrated by the AUC and ACC values reported in the above table, the following biomarkers were observed to specifically and sensitively distinguish between patients having sepsis and patients having SIRS when used on their own:
(i) The sepsis biomarkers: LCN2, ITGA2B, MYL9, ITGB3, and TREML1; and
(ii) The SIRS biomarkers: TGFBI, PLA2G7, MYCL, ARHGEF10L

The combination of all sepsis biomarkers (ITGB3, ITGA2B, MYL9, LCN2 and TREML1), the combination of all SIRS biomarkers (ARGHEF10L, MYCL, TGFB1 and PLA2G7), and the combination of all sepsis and all SIRS biomarkers also specifically and sensitively distinguished between patients having sepsis and patients having SIRS. These biomarkers may therefore be preferably used on their own or in combination in the methods of the invention to diagnose sepsis or SIRS, and to distinguish between sepsis and SIRS.

### ROC analysis of abdominal sepsis and pulmonary sepsis markers

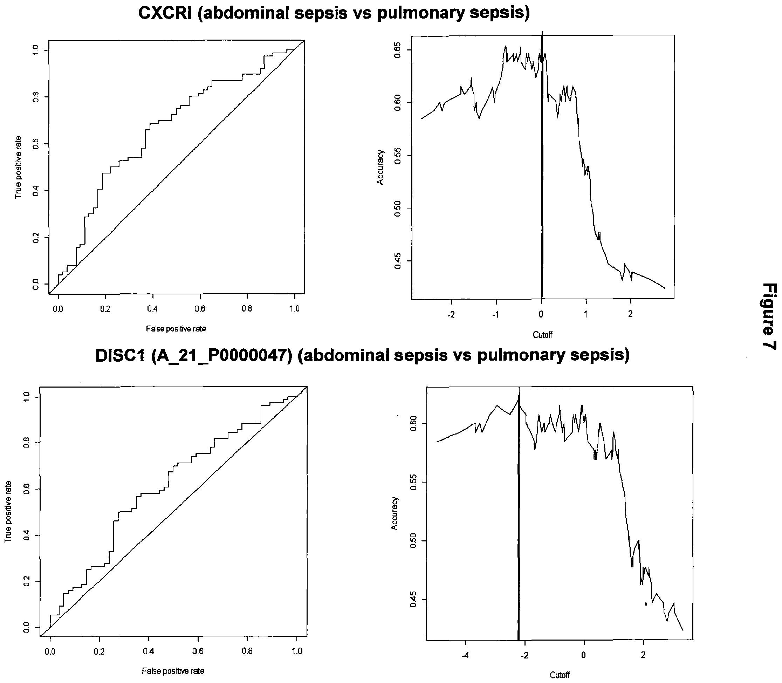

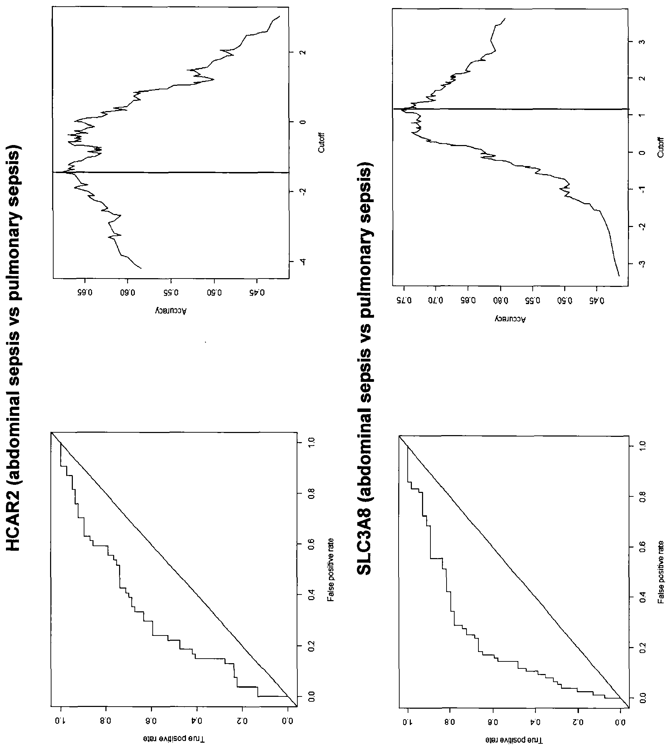

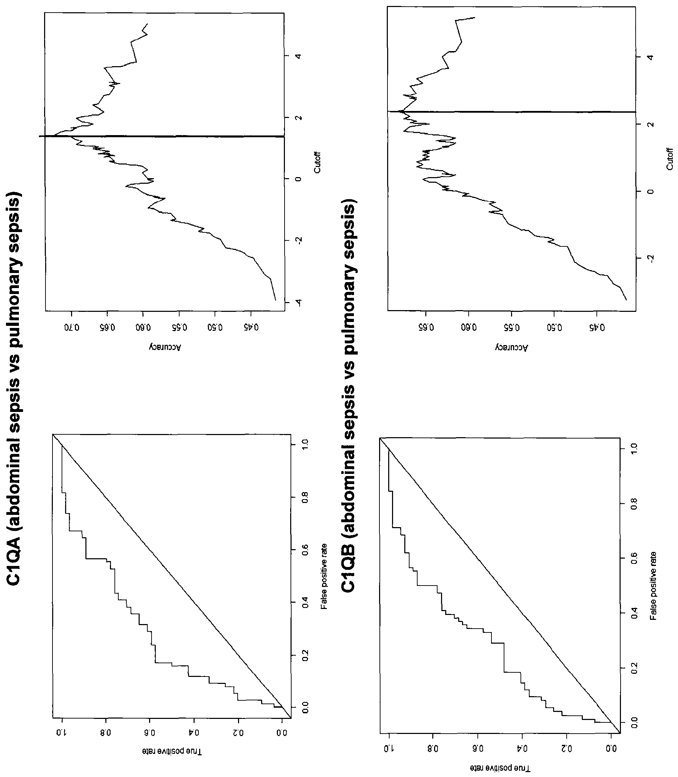

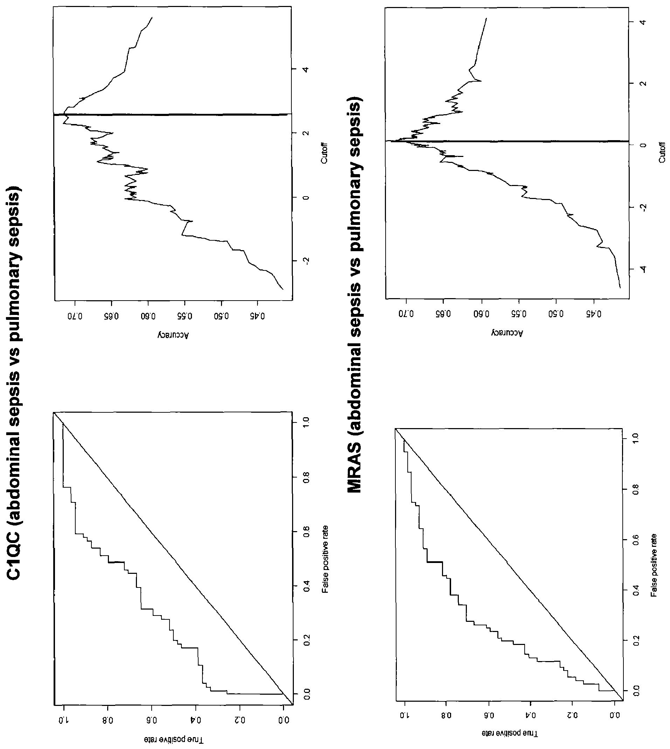

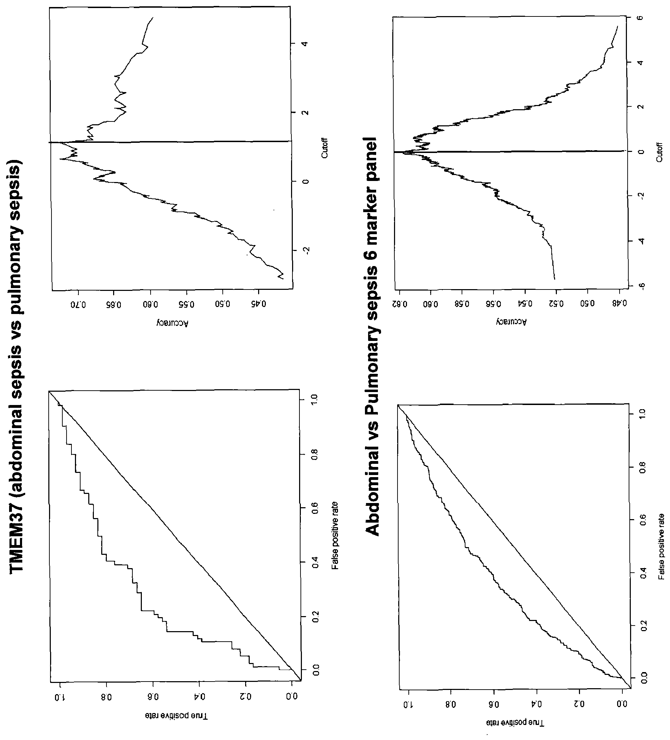

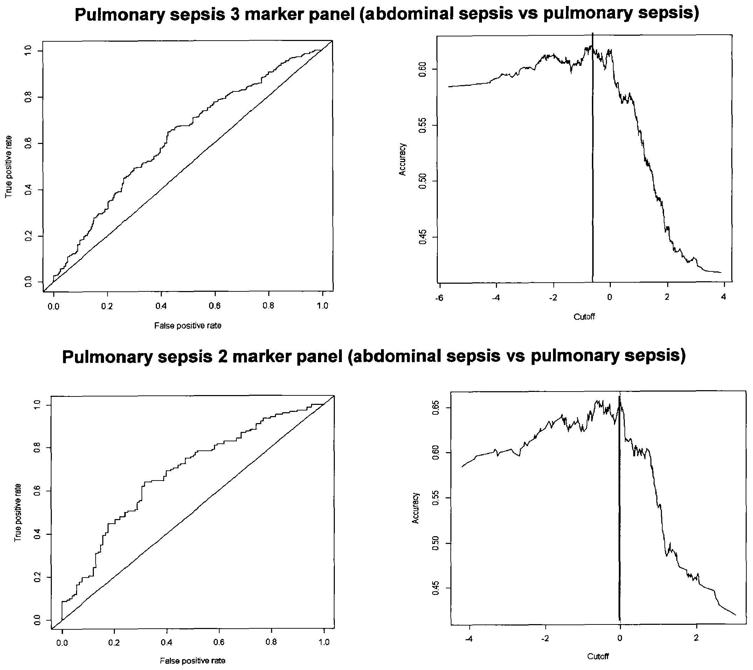

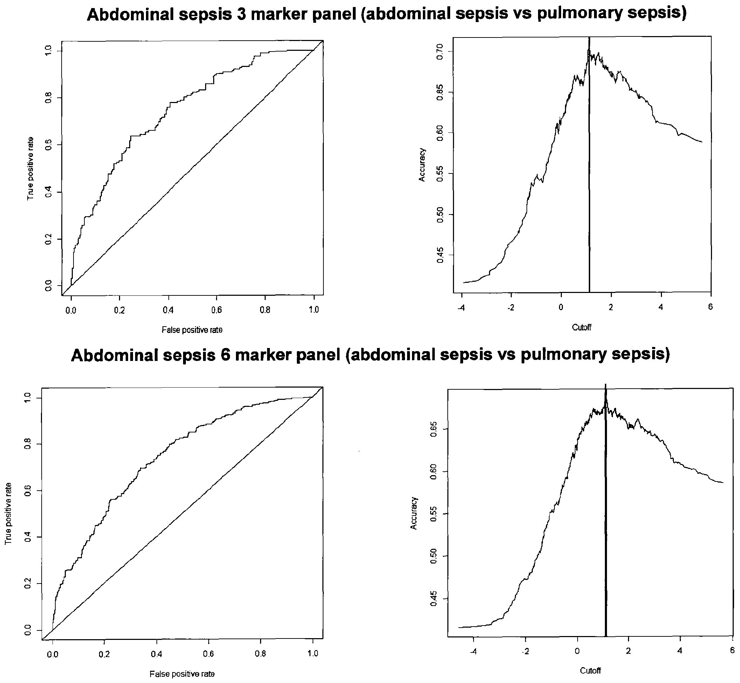

Table 3 summarises the genes identified as biomarkers of abdominal sepsis and pulmonary sepsis using gene expression analysis. To demonstrate that these biomarkers permit accurate diagnosis of abdominal and pulmonary sepsis (and distinguish between these specific disease conditions), the "Day 1" gene expression data obtained for all sepsis patients was analysed by ROC analysis to determine how well the biomarkers discriminate between patients having abdominal sepsis and patients having pulmonary sepsis. ROC analysis of the Day 1 fold change gene expression data was performed as described above. The ROC curves obtained for the best performing biomarkers are shown in Figure 7. AUC and ACC values calculated for abdominal sepsis and pulmonary sepsis biomarkers are summarised in the table below.

**Table 8**

| **BIOMARKER** | **PROBE NUMBER** | **Area under Curve (AUC)** | **Accuracy (ACC)** | **Cut-off** | **Abdominal/ Pulmonary** |
|---|---|---|---|---|---|
| **CXCR1** | A_23_P67932 | 0.6630117 | 0.6538462 | 0.00 | -/+ |
| **DISC1** | A_21_P0000047 | 0.6079435 | 0.6230769 | -2.2045078 | -/+ |
| **DISC1** | A_24_P83787 | 0.6028265 | 0.6230769 | -0.8441138 | -/+ |
| **DISC1** | A_21_P0000050 | 0.6084308 | 0.6307692 | -0.8969114 | -/+ |
| **HCAR2** | A_23_P329924 | 0.7022417 | 0.6769231 | -1.4589972 | -/+ |
| **SLC39A8 (ZIP8)** | A_23_P41424 | 0.7748538 | 0.7538462 | 1.1714854 | +/- |
| **C1QA** | A_24_P222655 | 0.7351365 | 0.7230769 | 1.4086328 | +/- |
| **C1QB** | A_23_P137366 | 0.7302632 | 0.6846154 | 2.3654090 | +/- |
| **C1QC** | A_23_P125977 | 0.7412281 | 0.7153846 | 2.5970287 | +/- |
| **MRAS** | A_24_P88850 | 0.7422027 | 0.7153846 | 0.1304908 | +/- |
| **TMEM37** | A_33_P3289296 | 0.7368421 | 0.7230769 | 1.1748223 | +/- |
| | | | | | |
| **Pulmonary sepsis 3 Marker panel (CXCR1, DISC1, HCAR2)** | A_23_P67932 | 0.6224269 | 0.6215385 | -0.6175871 | -/+ |
| | A_21_P0000047 | | | | |
| | A_24_P83787 | | | | |
| | A_21_P0000050 | | | | |
| | A_23_P329924 | | | | |
| **Pulmonary sepsis 2 Marker Panel (DISC1 REMOVED)** | A_23_P67932 | 0.6800682 | 0.6576923 | 0.000000 | -/+ |
| | A_23_P329924 | | | | |
| **Abdominal sepsis 3 Marker Panel (SLC39A8, CIQA, CIQC)** | A_23_P41424 | 0.7473468 | 0.7051282 | 1.0980682 | +/- |
| | A_24_P222655 | | | | |
| | A_23_P125977 | | | | |
| **Abdominal vs pulmonary 6 marker panel (CXCR1, DISC1, HCAR2, SLC39A8, CIQA, CIQC)** | A_23_P67932 | 0.6471384 | 0.61730769 | 0.04587579 | +/- |
| | A_21_P0000047 | | | | |
| | A_24_P83787 | | | | |
| | A_21_P0000050 | | | | |
| | A_23_P329924 | | | | |
| | A_23_P41424 | | | | |
| | A_24_P222655 | | | | |
| | A_23_P125977 | | | | |
| **Abdominal vs pulmonary 5 marker panel (DISC1 removed)** | A_23_P67932 | 0.6841542 | 0.6415385 | 0.6422954 | +/- |
| | A_23_P329924 | | | | |
| | A_23_P41424 | | | | |
| | A_24_P222655 | | | | |
| | A_23_P125977 | | | | |
| **Abdominal sepsis 6 Marker panel (SLC39A8, CIQA, CIQB, CIQC, MRAS, TMEM37)** | A_23_P41424 | 0.7356204 | 0.6858974 | 1.1297174 | +/- |
| | A_24_P222655 | | | | |
| | A_23_P125977 | | | | |
| | A_24_P88850 | | | | |
| | A_33_P3289296 | | | | |
| | A_23_P137366 | | | | |

As demonstrated by the AUC and ACC values reported in the above table, the following biomarkers were observed to specifically and sensitively distinguish between patients having abdominal sepsis and patients having pulmonary sepsis when used on their own or in combination:
(i) The abdominal sepsis biomarkers: CXCR1, DISC1, HCAR2; and
(ii) The pulmonary sepsis biomarkers: SLC39A8, CIQA, CIQB, CIQC, MRAS, TMEM37.

These biomarkers may therefore be preferably used in the methods of the invention to diagnose and distinguish between abdominal sepsis and pulmonary sepsis.

### ROC analysis of prognosis biomarkers for monitoring disease

When investigating gene expression patterns in patients that survived a systemic inflammatory condition and were deemed suitable for discharge from a high dependency unit (e.g. into a low dependency unit), the present inventors identified various biomarkers (summarised in Table 1) that altered in abundance in disease patients compared to healthy controls, but which returned towards their normal healthy levels as the patients recovered from disease. The inventors identified that these biomarkers could be used to determine the prognosis of a patient with a systemic inflammatory condition, and may be used to monitor the effectiveness of treatment in a patient or determine whether a patient is suitable for discharge.

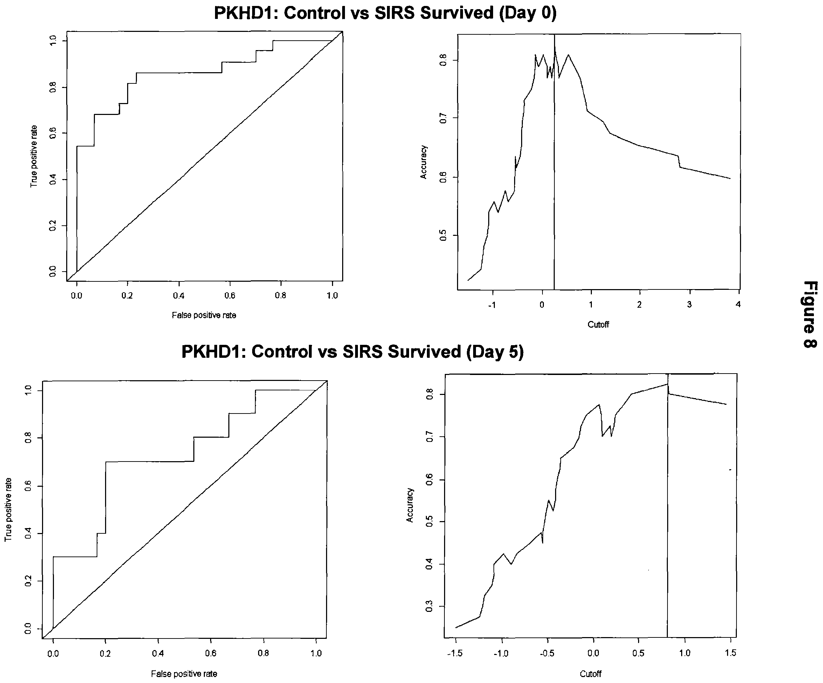

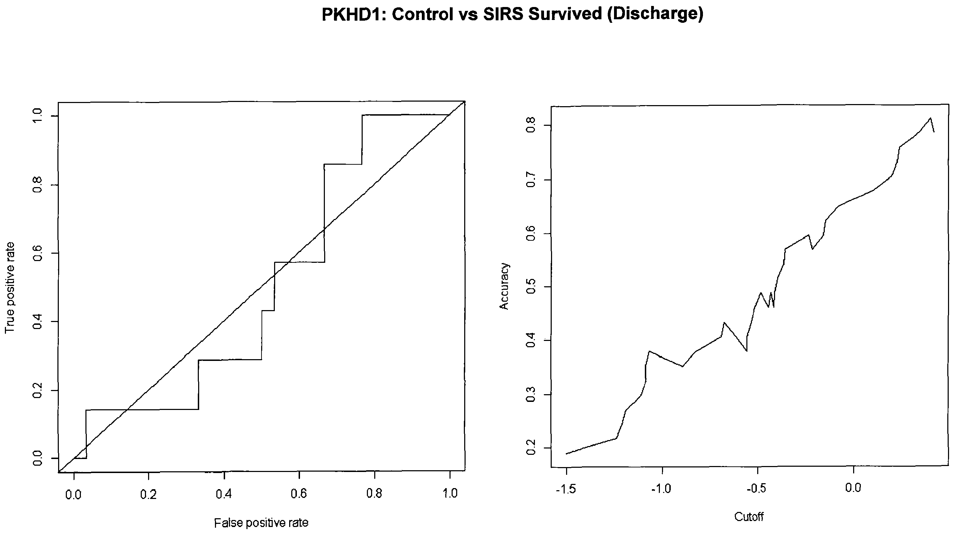

To demonstrate that these 'prognosis' biomarkers can be used to monitor the recovery of a patient from a systemic inflammatory condition, ROC analysis was performed to investigate over time the ability of the biomarkers to discriminate between healthy controls and patients that survived a systemic inflammatory condition. AUC values were calculated using the "Day 1", "Day 5" and "discharge" gene expression data, and are summarised below. An AUC value close to 1 indicates that the biomarkers discriminate well between the healthy controls and disease patient populations, whilst an AUC value close to 0.5 indicates that the two populations cannot be reliably distinguished. Representative ROC curves are plotted for PKHD1 biomarker in Figure 8.

**Table 9**

| **BIOMARKER** | **SURVIVAL vs DEATH** | **(AUC)** | **Accuracy (ACC)** | **Cut-off** |
|---|---|---|---|---|
| **FCER1A Probe: A23 P103765** | Control (1) vs OOHCA survived day 0 | 0.8681818 | 0.8269231 | 3.0441122 |
| | Control (1) vs OOHCA SURVIVED day 5 | 0.92 | 0.950000 | 2.322179 |
| | Control vs OOHCA SURVIVED DISCHARGE | 0.9857143 | 0.972973 | 2.322179 |
| | Control (1) vs Abdominal sepsis survived day 0 | 0.999187 | 0.9859155 | 2.3221793 |
| | Control (1) vs Abdominal sepsis survived day 5 | 0.9964912 | 0.9795918 | 2.3221793 |
| | Control vs Abdominal survived discharge | 0.9461538 | 0.8837209 | 2.7308435 |
| | Control (1) vs pulmonary sepsis survived | 0.9798851 | 0.9545455 | 2.7308435 |
| | day 0 | | | |
| | Control (1) vs pulmonary sepsis survived day 5 | 0.9825 | 0.9571429 | 2.7308435 |
| | Control vs pulmonary sepsis survived discharge | 0.8946667 | 0.8545455 | 2.8394332 |
| **BCL11B Probe A23 P205738** | Control vs OOHCA SURVIVAL DAY 0 | 0.9409091 | 0.9423077 | 1.3860502 |
| | Control vs OOHCA SURVIVAL DAY 5 | 1 | 1 | 1.38605 |
| | Control vs OOHCA SURVIVAL DISCHARGE | 0.9904762 | 0.972973 | 1.585580 |
| | Control vs Abdominal survived day 0 | 1 | 1 | 1.38605 |
| | Control vs Abdominal survived day 5 | 0.9964912 | 0.9795918 | 1.5855803 |
| | Control vs Abdominal survived discharge | 0.9923077 | 0.9534884 | 1.6249652 |
| | Control vs pulmonary survived day 0 | 0.9936782 | 0.9772727 | 1.5855803 |
| | Control vs pulmonary survived day 5 | 0.9983333 | 0.9857143 | 1.5855803 |
| | Control vs pulmonary survived discharge | 0.9613333 | 0.8909091 | 1.6249652 |
| **PKHD1 Probe A 33 P3387420** | Control (1) vs OOHCA survived day 0 | 0.8621212 | 0.8269231 | 0.2411327 |
| | Control (1) vs OOHCA survived day 5 | 0.7266667 | 0.8250000 | 0.8070955 |
| | Control (1) vs OOHCA survived day 5 | 0.5 | 0.8108108 | inf |
| | Control (1) vs Abdominal survived day 0 | 0.7845528 | 0.73239437 | 0.05443954 |
| | Control (1) vs Abdominal survived day 5 | 0.5350877 | 0.6938776 | 0.5116310 |
| | Control (1) vs Abdominal survived discharge | 0.6871795 | 0.8139535 | 0.4272528 |
| | Control vs Pulmonary survived day 0 | 0.7781609 | 0.7613636 | -0.4943862 |
| | Control vs Pulmonary survived day 5 | 0.6725 | 0.6714286 | -0.3561888 |
| | Control vs Pulmonary survived discharge | 0.6013333 | 0.6727273 | 0.5358357 |
| **KLRB1** | Control vs OOHCA SURVIVED DAY 0 | 0.9818182 | 0.9615385 | 1.0817766 |
| **Probe A23 P99275** | | | | |
| | Control vs OOHCA SURVIVED DAY 5 | 0.99 | 0.975000 | 1.081777 |
| | Control vs OOHCA SURVIVED discharge | 0.9285714 | 0.9189189 | 0.4236860 |
| | Control vs abdominal survived day 0 | 0.9608333 | 0.9142857 | 1.4716887 |
| | Control vs abdominal survived day 5 | 0.98 | 0.960000 | 1.081777 |
| | Control vs abdominal survived discharge | 0.8769231 | 0.8604651 | 1.0817766 |
| | Control vs pulmonary survived day 0 | 0.9816092 | 0.9431818 | 1.0817766 |
| | Control vs pulmonary survived day 5 | 0.9725 | 0.9285714 | 1.4716887 |
| | Control vs pulmonary survived discharge | 0.8826667 | 0.8727273 | 1.1410170 |
| **LILRB5 Probe A 23 P4773** | Control vs OOHCA SURVIVED DAY 0 | 0.8166667 | 0.7884615 | -0.7073317 |
| | Control vs OOHCA SURVIVED DAY 5 | 0.9266667 | 0.9250000 | 0.2927966 |
| | Control vs OOHCA SURVIVED discharge | 0.8190476 | 0.8378378 | 0.7142091 |
| | Control vs abdominal survived day 0 | 0.935 | 0.9142857 | -1.1843534 |
| | Control vs abdominal survived day 5 | 0.9035088 | 0.8367347 | -0.2738576 |
| | Control vs abdominal survived discharge | 0.8435897 | 0.8604651 | -0.2342336 |
| | Control vs pulmonary survived day 0 | 0.904023 | 0.8522727 | -1.7467065 |
| | Control vs pulmonary survived day 5 | 0.8891667 | 0.8714286 | -1.1859131 |
| | Control vs pulmonary survived discharge | 0.8026667 | 0.7636364 | -1.0275340 |

As demonstrated in the table above, the AUC values calculated for the biomarkers are observed to change over time as the patient recovers from the systemic inflammatory condition, shifting from '1' or close to '1' (for the samples taken at an early stage of the disease, such as at day 0) towards '0.5' (for the samples taken at day 5 or on discharge). This indicates that the biomarkers become less able to discriminate between healthy controls and disease patients as the patient recovers (indicating that the biomarker profile of the patient becomes more represenatative of a healthy control). For example, when investigating the gene expression data obtained for PKDH1 in the patients with SIRS, the AUC value for the 'day 0' sample is 0.86 indicating that the patient with SIRS can be readily distinguished from a healthy control. By 'day 5' the AUC value has dropped to 0.73, indicating that there is less distinction between the patient and a healthy control. Upon discharge, the AUC value has dropped to 0.5 indicating that the biomarker profile of the patient cannot be distinguished from that observed for a healthy control. By investigating the levels of these biomarkers in patients having a systemic inflammatory condition, the disease status of the patient may be monitored to determine whether the disease is progressing towards a more severe form of the disease or regressing towards normalcy.

Suprisingly, the inventors observed that for many of the prognosis biomarkers tested, the AUC values calculated for the "discharge" samples did not drop all the way to '0.5' but remained somewhere between 1 and 0.5. This indicates that patients are being discharged before they are fully immunologically recovered (ie. before their biomarker profiles are representative of a healthy control). By monitoring a patient using the 'prognosis' biomarkers of the invention, it is possible to determine more accurately when a patient has recovered fully from a systemic inflammatory condition, and can be safely discharged.

### ROC analysis of survival biomarkers

Table 4 summarises the genes identified as survival biomarkers for determining whether a patient with a systemic inflammatory condition is suitable for discharge from medical care. These markers can be used to predict whether a patient undergoing treatment for a systemic inflammatory condition is likely to survive.

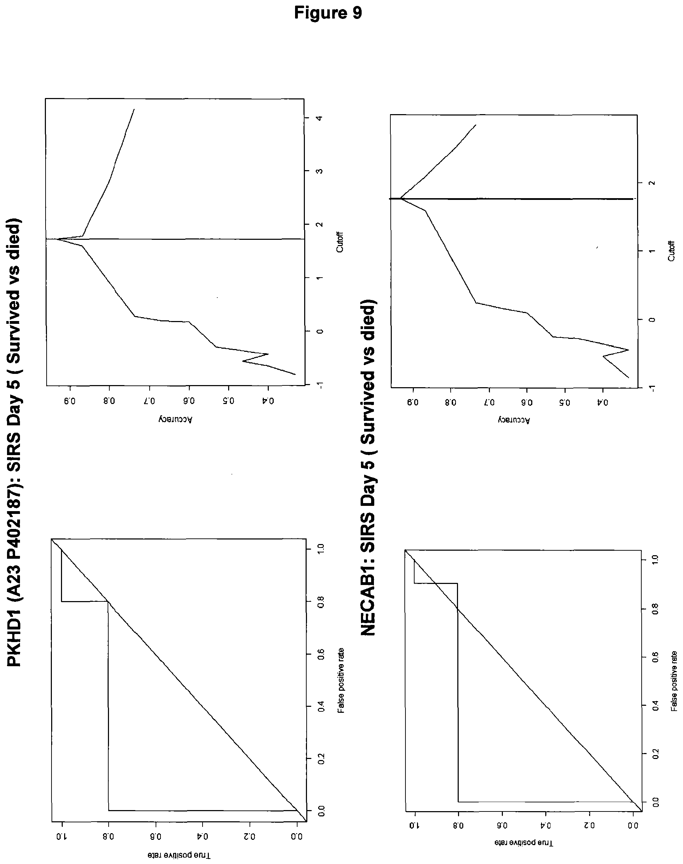

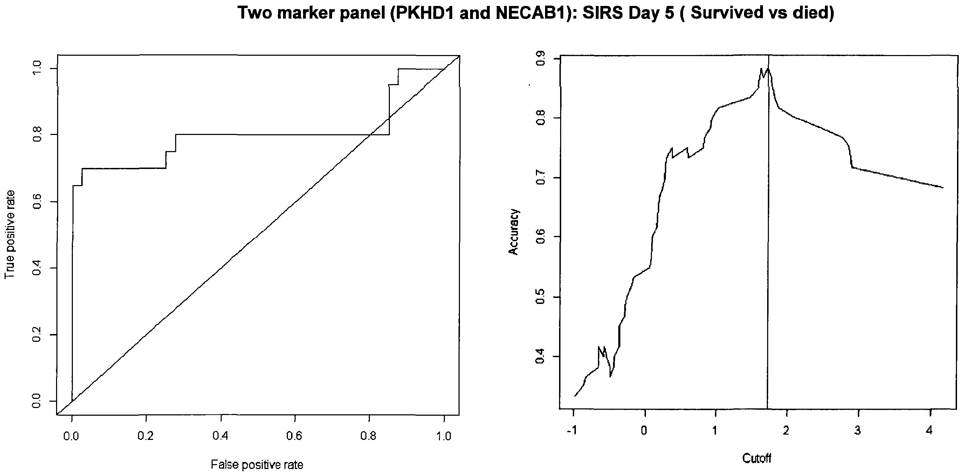

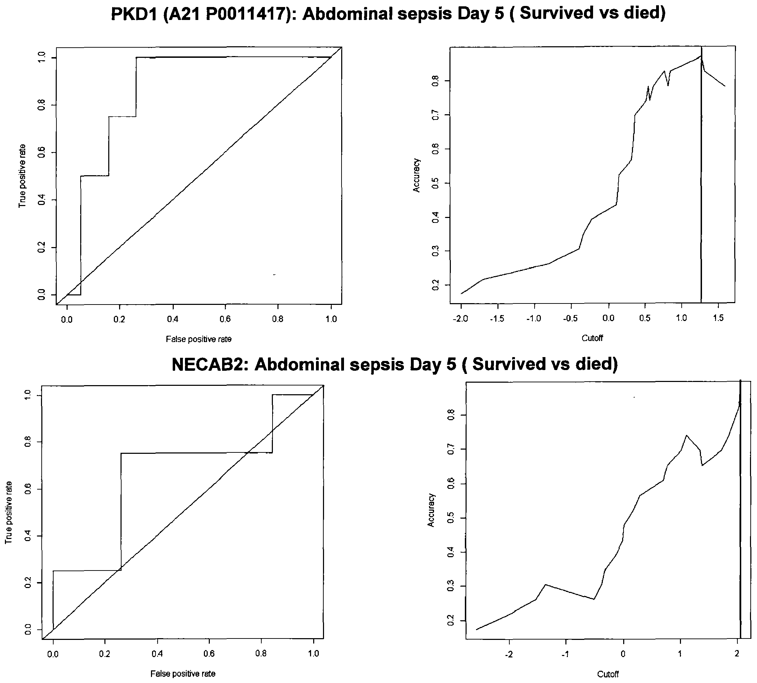

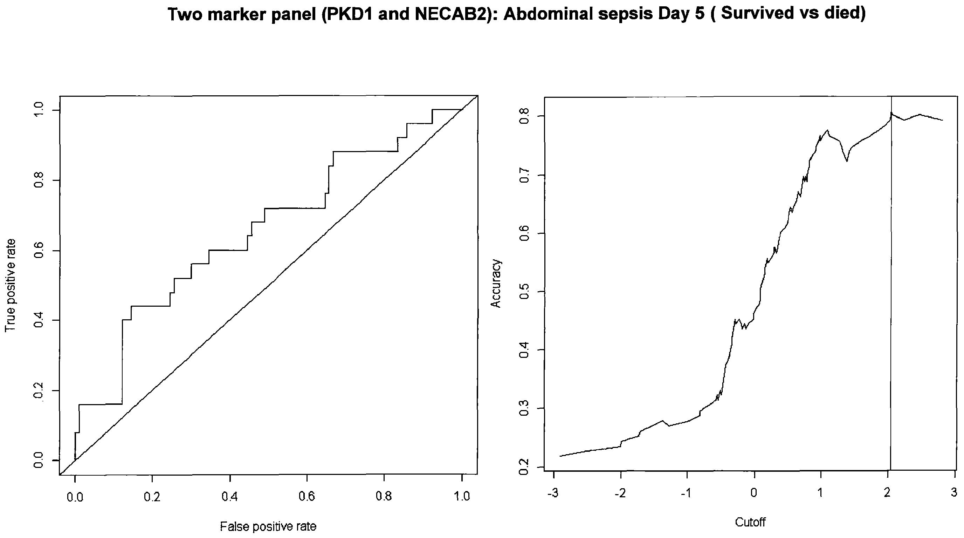

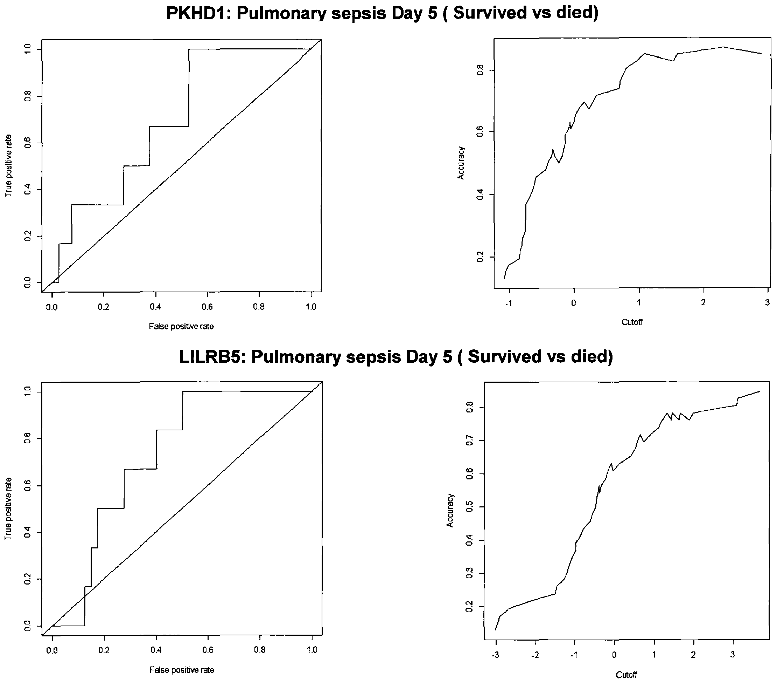

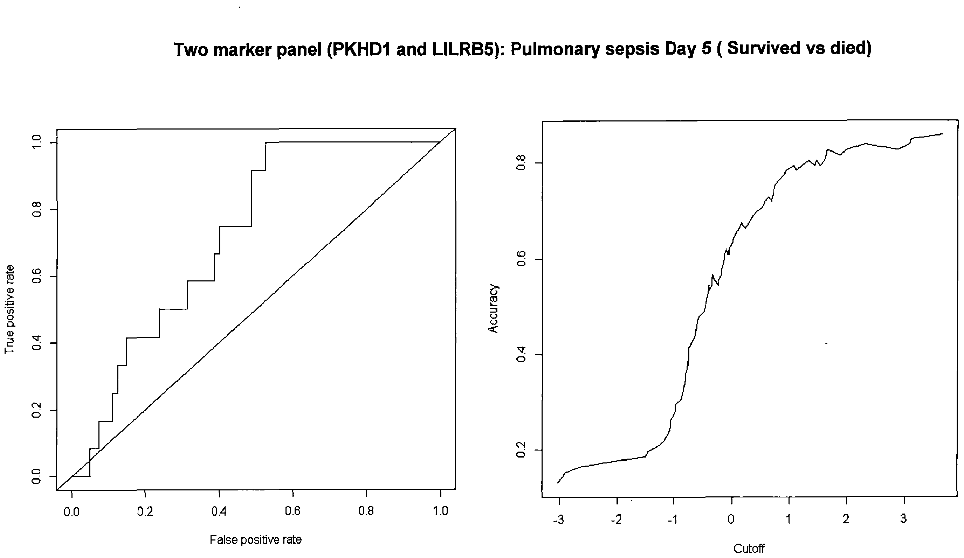

To demonstrate that these biomarkers accurately predict survival, the "Day 5" gene expression data obtained for all patients was analysed by ROC analysis to determine how well the biomarkers discriminate between patients having a systemic inflammatory condition that survive and those do not survive. ROC analysis of the Day 5 fold change gene expression data was performed as described above. The ROC curves obtained for the best performing biomarkers are shown in Figure 9. AUC and ACC values calculated for the survival biomarkers are summarised in the table below.

**Table 10: Biomarkers for predicting survival from SIRS**

| **BIOMARKER** | **PROBE NUMBER** | **Area under Curve (AUC)** | **Accuracy (ACC)** | **Cut-off** | **SURVIVAL vs DEATH** |
|---|---|---|---|---|---|
| **PKHD1** | A_23_P402187 | 0.84 | 0.9333333 | 1.7156901 | +/- |
| **PKHD1** | A_33_P3387420 | 0.76 | 0.8666667 | 1.6200123 | +/- |
| **PKHD1** | A_23_P424617 | 0.76 | 0.8666667 | 1.8112721 | +/- |
| **NECAB1** | A_24_P944756 | 0.82 | 0.9333333 | 1.7683950 | +/- |
| **2 MARKER PANEL** | A_23_P402187 | 0.80125 | 0.8833333 | 1.7156901 | +/- |
| | A_33_P3387420 | | | | |
| | A_23_P424617 | | | | |
| | A_24_P944756 | | | | |

**Table 11: Biomarkers for predicting survival from abdominal sepsis**

| **BIOMARKER** | **PROBE NUMBER** | **Area under Curve (AUC)** | **Accuracy (ACC)** | **Cut-off** | **SURVIVAL vs DEATH** |
|---|---|---|---|---|---|
| **PKD1** | A_21_P0011417 | 0.8684211 | 0.8695652 | 1.2567754 | +/- |
| **PKD1** | A_21_P0011418 | 0.7105263 | 0.8695652 | 2.0439925 | +/- |
| **PKD1** | A_21_P0011419 | 0.7368421 | 0.8695652 | 2.4574770 | +/- |
| **NECAB2** | A_23_P66011 | 0.6578947 | 0.8695652 | 2.0517770 | +/- |
| **2 MARKER PANEL** | A_21_P0011417 | 0.6533333 | 0.8086957 | 2.0439925 | +/- |
| | A_21_P0011418 | | | | |
| | A_21_P0011419 | | | | |
| | A_23_P66011 | | | | |

**Table 12: Biomarkers for predicting survival from pulmonary sepsis**

| **BIOMARKER** | **PROBE NUMBER** | **Area under Curve (AUC)** | **Accuracy (ACC)** | **Cut-off** | **SURVIVAL vs DEATH** |
|---|---|---|---|---|---|
| **PKHD1** | A_33_P3387420 | 0.7 | 0.8695652 | INF | -/+ |
| **LILRB5** | A_23_P4773 | 0.7291667 | 0.8695652 | INF | -/+ |
| **2 MARKER PANEL (PKHD1 and LILRB5)** | A_33_P3387420 | 0.7208333 | 0.8695652 | INF | -/+ |
| | A_23_P4773 | | | | |

As demonstrated by the AUC and ACC values reported in the above tables, the following biomarkers (used alone or in combination) were observed to specifically and sensitively distinguish between patients having a systemic inflammatory condition that made a full recovery and those that did not:
(i) Survival markers for predicting recovery from SIRS: PKHD1 and NECAB1
(ii) Survival markers for predicting recovery from abdominal sepsis: PKD1 and NECAB2; and
(iii) Survival markers for predicting recovery from pulmonary sepsis: PKHD1 and LILRB5.

These biomarkers may therefore be preferably used alone or in combination in the methods of the invention to determine whether a patient is suitable for discharge from medical care.

### EXAMPLE 3: QUANTIFICATION OF PROTEIN BIOMARKERS

To further investigate the biomarkers of systemic inflammation identified by gene expression analysis, a subset of the biomarkers was selected for further analysis by ELISA. Protein quantification by ELISA was performed to investigate the abundance of specific biomarkers in whole lysed blood obtained from patients at day 1 and day 5 post admittance to an intensice care unit (as described above for Example 1).

The biomarkers chosen for further analysis were: (i) the pulmonary sepsis biomarker DISC1; (ii) the abdominal sepsis biomarker SLC39A8; (iii) the SIRS biomarker GPR124; and (iv) the survival marker NECAB1 which is used to predict survival from SIRS.

ELISA protein quantification:
Blood samples were collected from patients and processed as described in Example 1. 2ml of blood sample was mixed with 8 ml of cell lysis buffer at a 1:5 dilution. The cell lysis buffer was purchased from Invitrogen (NP40 cell lysis buffer: 50 mM Tris, pH 7.4, 250 mM NaCl, 5 mM EDTA, 50 mM NaF, 1 mM Na3VO4, 1% Nonidet P40 (NP40), and 0.02% NaN3, supplemented with 1 mM PMSF and protease inhibitor cocktail). The samples were incubated on ice for 60 minutes. The samples were then centrifuged at 13000 rpm for 30 minutes to pellet debris. The supernatant was removed and passed through a 0.22 µm syringe filter. The cell free supernatant was transferred to a fresh tube and stored at -80°C.

ELISA assays were performed on neat or diluted lysed blood samples using commercial ELISA kits (https://www.mybiosource.com/).

DISC1 was quantified using ELISA kit MBS9343138, NECAB1 was quantified using ELISA kit MBS9338711, and SLC39A8 was quantified using ELISA kit MBS9381303. Briefly, 50 µl of prepared blood sample was added to the sample well of a microelisa stripplate plate. In addition, 50 µl of each standard was added to the standard wells and 50 µl of sample diluent was added to each blank/control well of the plate. 100 µl of HRP-conjugate reagent was added to each well and incubated for 60 minutes at 37°C. The plate was washed 4 times, and developed by adding 50 µl Chromagen Solution A and 50 µl Chromagen solution B and incubating for 15 minutes at 37°C. 50 µl stop solution was added to the wells, and the optical density at 450nm was read.

GPR124 was quantified using ELISA kit MBS909585. Briefly, 100 µl of prepared blood sample was added to the sample well of an ELISA plate. In addition, 100 µl of each standard was added to the standard wells and 100 µl of sample diluent was added to each blank/control well of the plate. The plate was incubated for 2 hours at 37°C. 100 µl of biotin-antibody was added to each well and incubated for 60 minutes at 37°C. The plate was washed 3 times. 100 µl of HRP-avidin was added to each well and incubated for 1 hour at 37°C. The plate washed 5 times. 90 µl TMB substrate was added and the plate was incubated for 15-30 minutes at 37°C. 50 µl stop solution was added to the wells, and the optical density at 450nm was read.

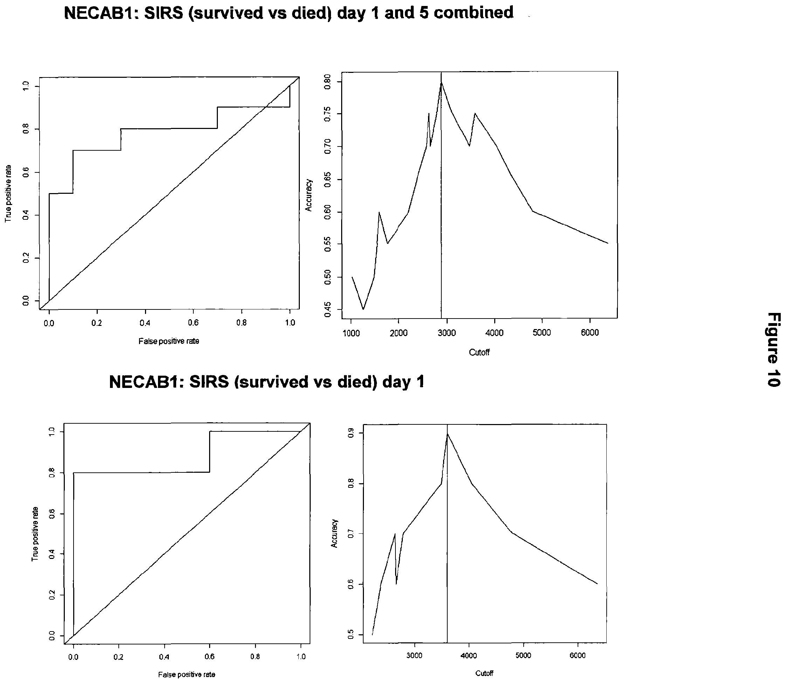

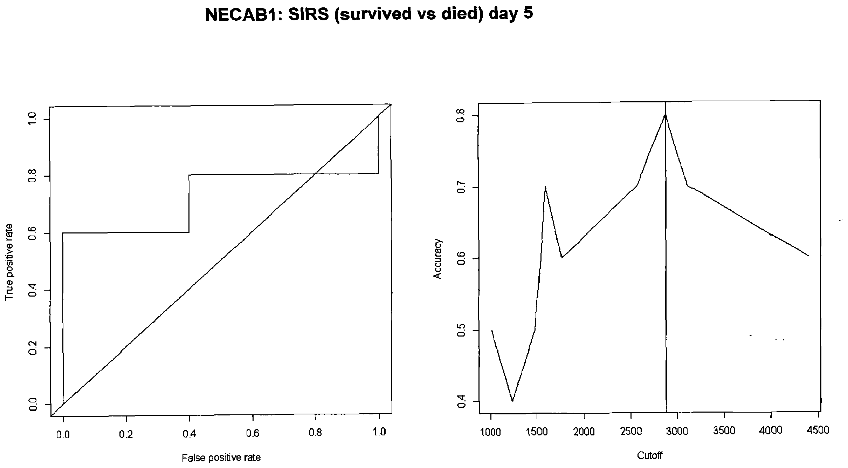

ROC analysis of protein quantification data:
The protein quantification data obtained for the patients was analysed by ROC analysis to determine how well the markers could distinguish between different types of systemic inflammatory condition and/or between patients that survived and patients that died. The results of the analysis are presented in the table below. ROC Curves are shown in Figure 10.

**Table 13:**

| **BIOMARKER/TEST** | **Area under Curve (AUC)** | **Accuracy (ACC)** | **Cut-off** |
|---|---|---|---|
| **DISC1: Pulmonary vs ALL Samples (day 0)** | 0.6935897 | 0.7959184 | ND |
| **DISC1: Pulmonary vs controls** | 0.86 | 0.8333333 | 361.5500000 ng/ml |
| **SLC39A8 (ZIP8) : Abdominal sepsis vs All** | 0.6538462 | 0.8723404 | 82.0000000 ng/ml |
| **SLC39A8 (ZIP8): Abdominal vs Controls** | 0.76875 | 0.8928571 | 57.2375000 ng/ml |
| **GPR124: SIRS(OOHCA) vs all sepsis** | 0.6608187 | 0.7272727 | 1858.6600000 pg/ml |
| **GPR124: SIRS (OOHCA) VS Pulmonary sepsis** | 0.6398892 | 0.6842105 | 1971.0400000 pg/ml |
| **GPR124: SIRS (OOHCA) vs Abdominal sepsis** | 0.6988304 | 0.7027027 | 2109.4800000 pg/ml |
| **NECAB1: SIRS: DIED VS SURVIVED** | 0.78 | 0.80 | 2876.15 ng/ml |
| **NECAB1: SIRS: DIED VS SURVIVED (DAY 0)** | 0.88 | 0.9 | 3591.25 ng/ml |
| **NECAB1: SIRS: DIED VS SURVIVED (DAY 5)** | 0.72 | 0.8 | 2876.15 ng/ml |

The pulmonary sepsis marker DISC1 was tested for its ability to distinguish between patients having pulmonary sepsis and patients having another systemic inflammatory condition (such as abdominal sepsis or SIRS). DISC1 was also tested for its ability to distinguish between patients having pulmonary sepsis and healthy controls. ROC analysis revealed that this marker performed well to identify patients having pulmonary sepsis.

The abdominal sepsis biomarker SLC39A8 was tested for its ability to distinguish between patients having abdominal sepsis and patients having another systemic inflammatory condition (such as pulmonary sepsis or SIRS). DISC1 was also tested for its ability to distinguish between patients having abdominal sepsis and healthy controls. ROC analysis revealed that this marker performed well to identify patients having abdominal sepsis.

The SIRS biomarker GPR124 was tested for its ability to distinguish between patients having SIRS and patients having sepsis (including those having abdominal sepsis and pulmonary sepsis). ROC analysis revealed that this marker performed well to distinguish patients having SIRS and patients having any type of sepsis.

The survival biomarker NECAB1 was tested for its ability to distinguish between patients having SIRS that survived and patients having SIRS that died. ROC analysis revealed that this marker performed well as a survival marker for SIRS.

### EXAMPLE 4: DIAGNOSIS OF SYSTEMIC INFLAMMATORY DISEASE IN INTENSIVE CARE

A patient presents at an intensive care unit (ICU) or is admitted to lower dependency hospital ward with unspecified illness. Within 6 hours of admission, a blood sample is obtained from the patient, and is tested with inflammation (as described in Table 1), SIRS (as described in Table 2) and sepsis (as described in Table 3) biomarker panels using qPCR. Raised expression of inflammation markers, as determined by a lower threshold Cₜ value compared with controls, indicates an ongoing systemic inflammatory condition. Raised expression of SIRS biomarkers indicates that the systemic inflammatory condition is SIRS. Raised expression of sepsis biomarkers indicates that the systemic inflammatory condition is sepsis.

### EXAMPLE 5: DIAGNOSIS OF SYSTEMIC INFLAMMATORY DISEASE AT GP SURGERY, OUT-OF-HOURS CLINIC OR EMERGENCY DEPARTMENT

A patient presents at a GP surgery, an out-of-hours clinic, or an accident and emergency department. A blood sample is obtained from the patient and tested using a rapid point of care diagnostic test for inflammation markers (as described in Table 1). The test reveals that the inflammation biomarkers are elevated in the patient. The patient is referred for further detailed investigation using a full panel of inflammation, sepsis, and SIRS biomarkers in a hospital/diagnostic laboratory setting.

### EXAMPLE 6: MONITORING OF SYSTEMIC INFLAMMATORY CONDITION

A patient is undergoing treatment for a systemic inflammatory condition in an ICU or hospital. To monitor how the patient is responding to treatment, and whether continued treatment or a change in treatment is needed, a blood sample is taken and tested using a panel of prognostic recovery biomarkers (as described in Table 1).

If the level of prognosis biomarkers detected shows regression of the systemic inflammatory condition, treatment may be continued for a short while until the patient is deemed suitable for discharge. Prior to discharge, a blood sample from the patient may be taken and tested using a panel of survival biomarkers (as described in Table 4). If the levels of survival biomarkers show that the patient has a good prognosis of recovery, the patient is discharged. If the levels of survival biomarkers show that the patient has a poor prognosis of recovery, the patient is not discharged and treatment is continued.

If the levels of prognosis biomarkers show progression or no change in the systemic inflammatory condition, the patient continues to undergo treatment and may be switched to a different treatment strategy. The patient may be monitored by testing further blood sample(s) using the panel of prognosis biomarkers to determine whether the systemic inflammatory disease is progressing or regressing towards normalcy.

After discharge, the patient may be monitored at home by community nursing staff using a prognostic marker point of care diagnostic test, to monitor ongoing response to therapy and to provide rapid indication of any relapse.

### EXAMPLE 7: PREDICTION OF DISEASE SEVERITY AND SURVIVAL IN PATIENTS

A patient presents at an intensive care unit (ICU) or is admitted to lower dependency hospital ward with unspecified illness, and is diagnosed as having a systemic inflammatory condition. To assess the severity of the disease, a blood sample is taken and tested using a panel of prognostic biomarkers (as described in Table 1). The blood sample may also be tested using a panel of survival biomarkers (as described in Table 4) to determine the degree of organ damage/failure.

The levels of prognosis biomarkers and/or survival biomarkers may be used by clinicians to inform treatment choices/tailor treatment packages and manage survival expectation in the next of kin.

### Table 14: Oligonucleotide probes

Table 14 lists various probes used to detect the various biomarkers of the invention. Preferred probe sequences are shown in bold.

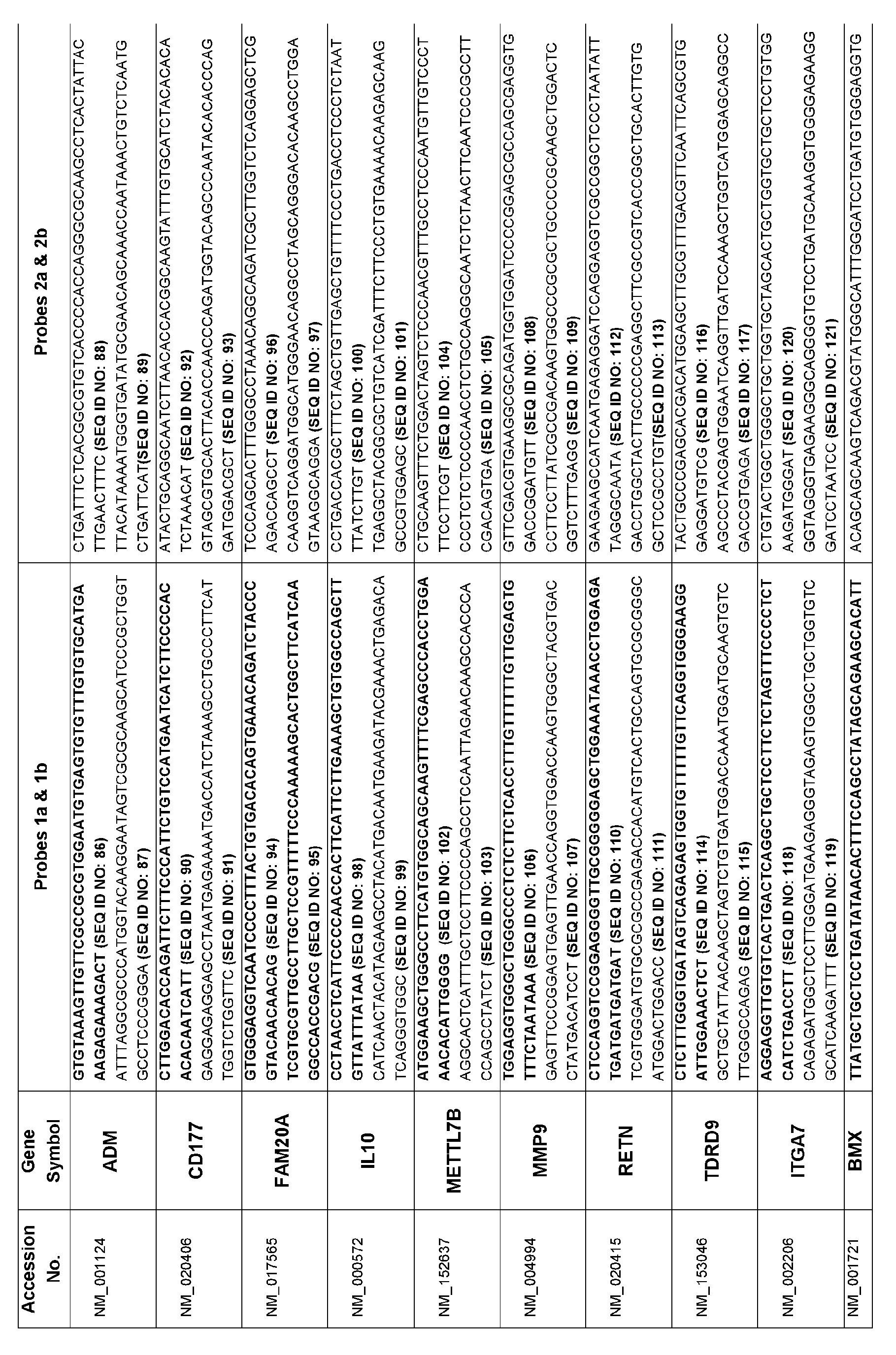

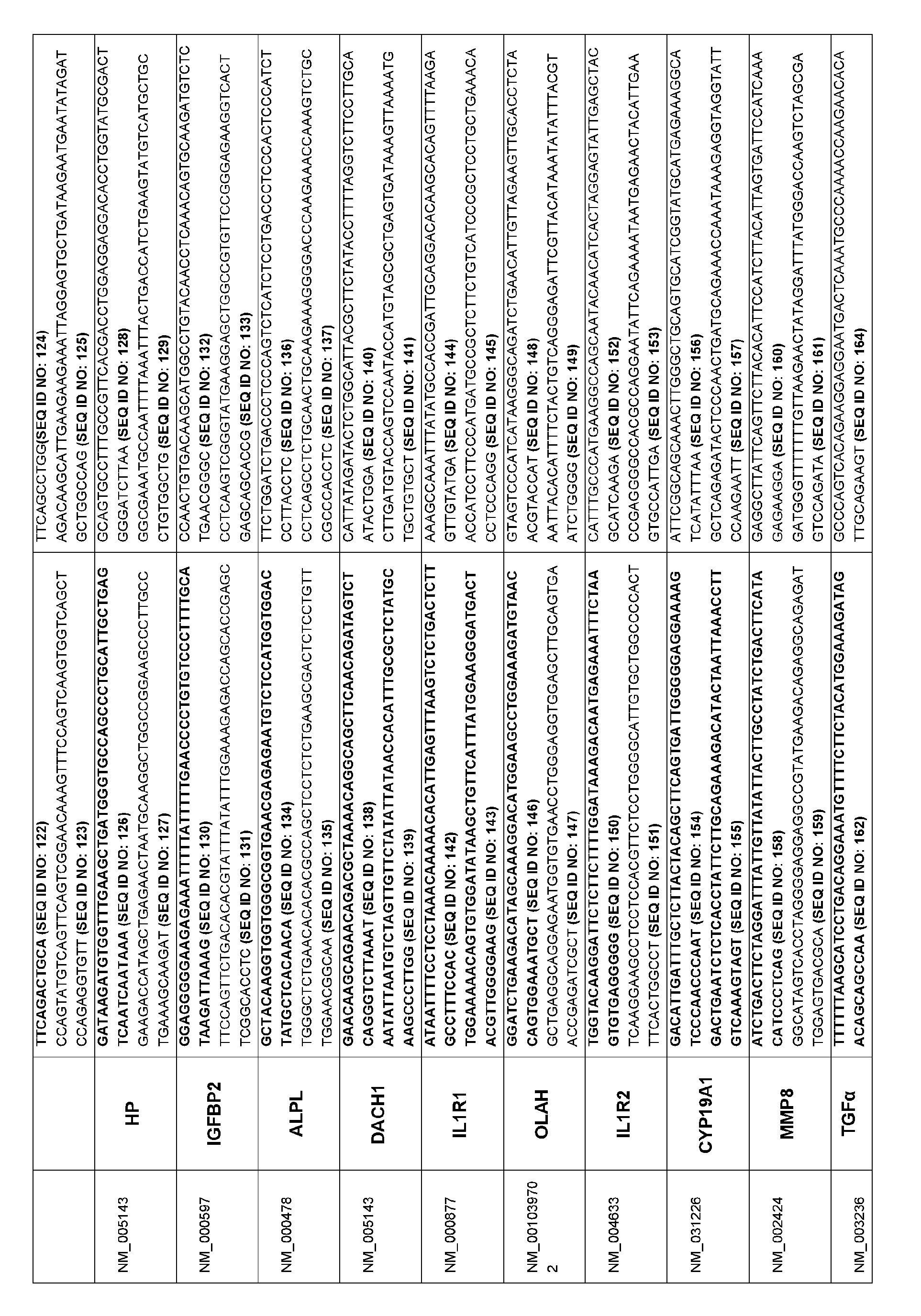

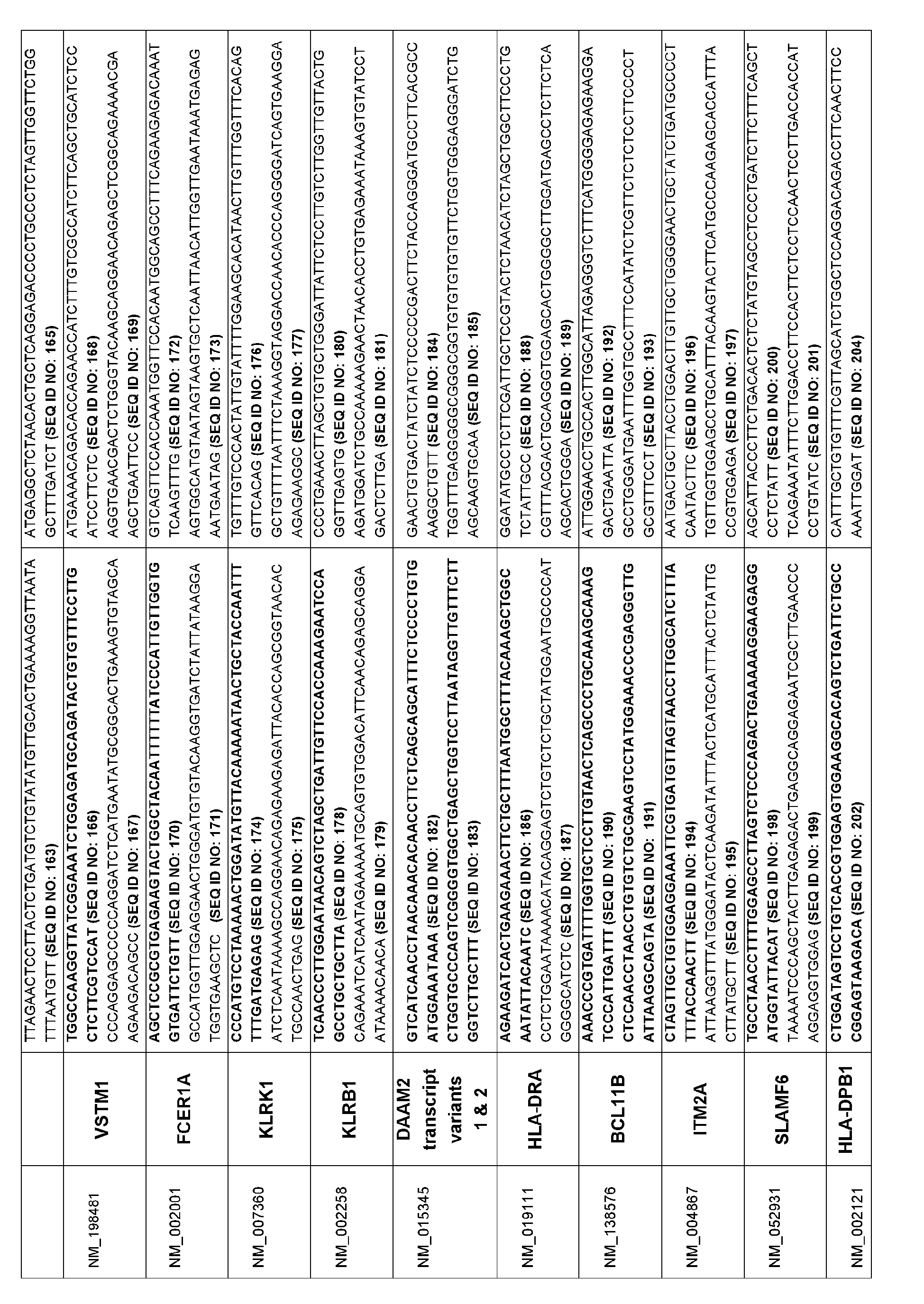

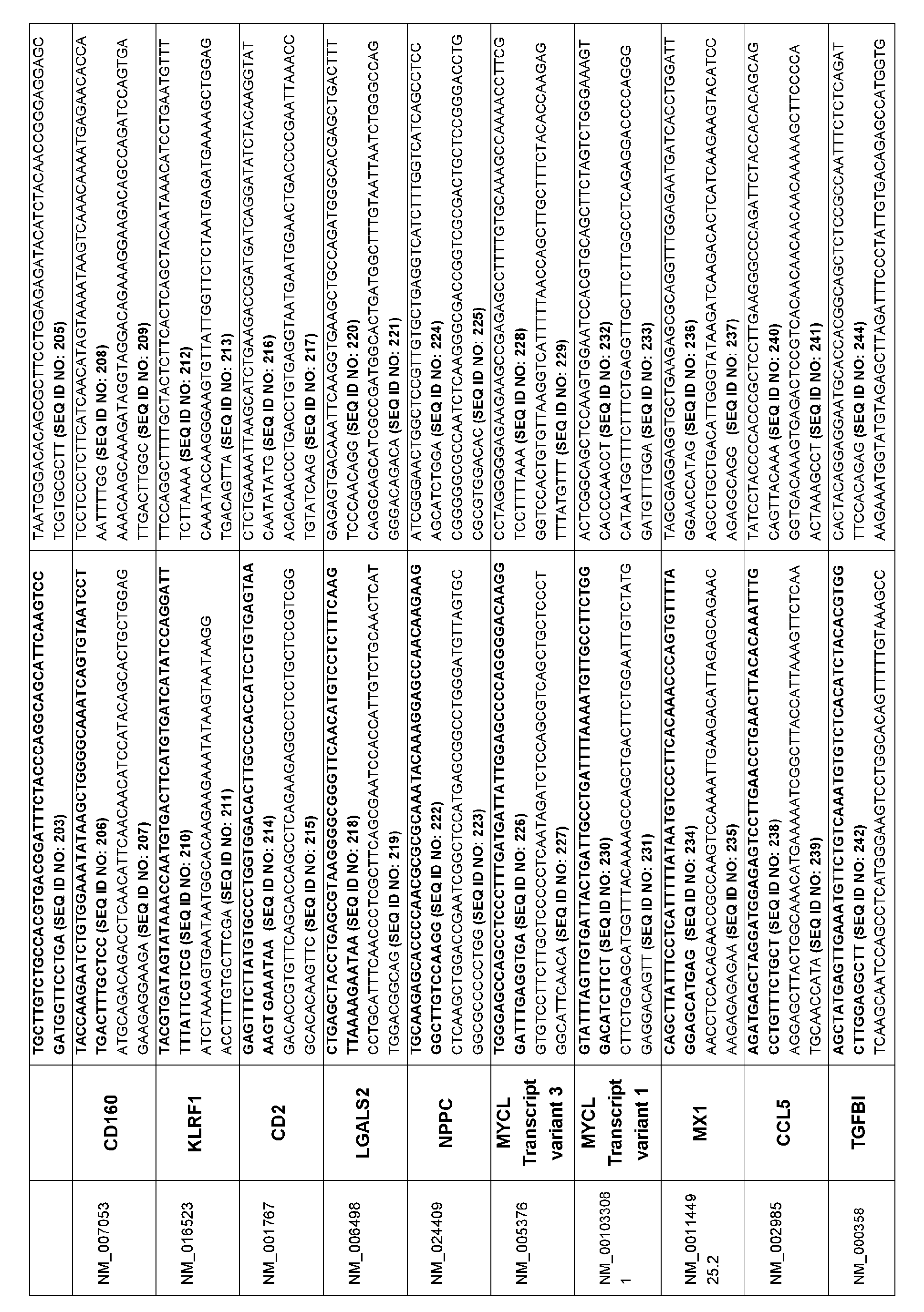

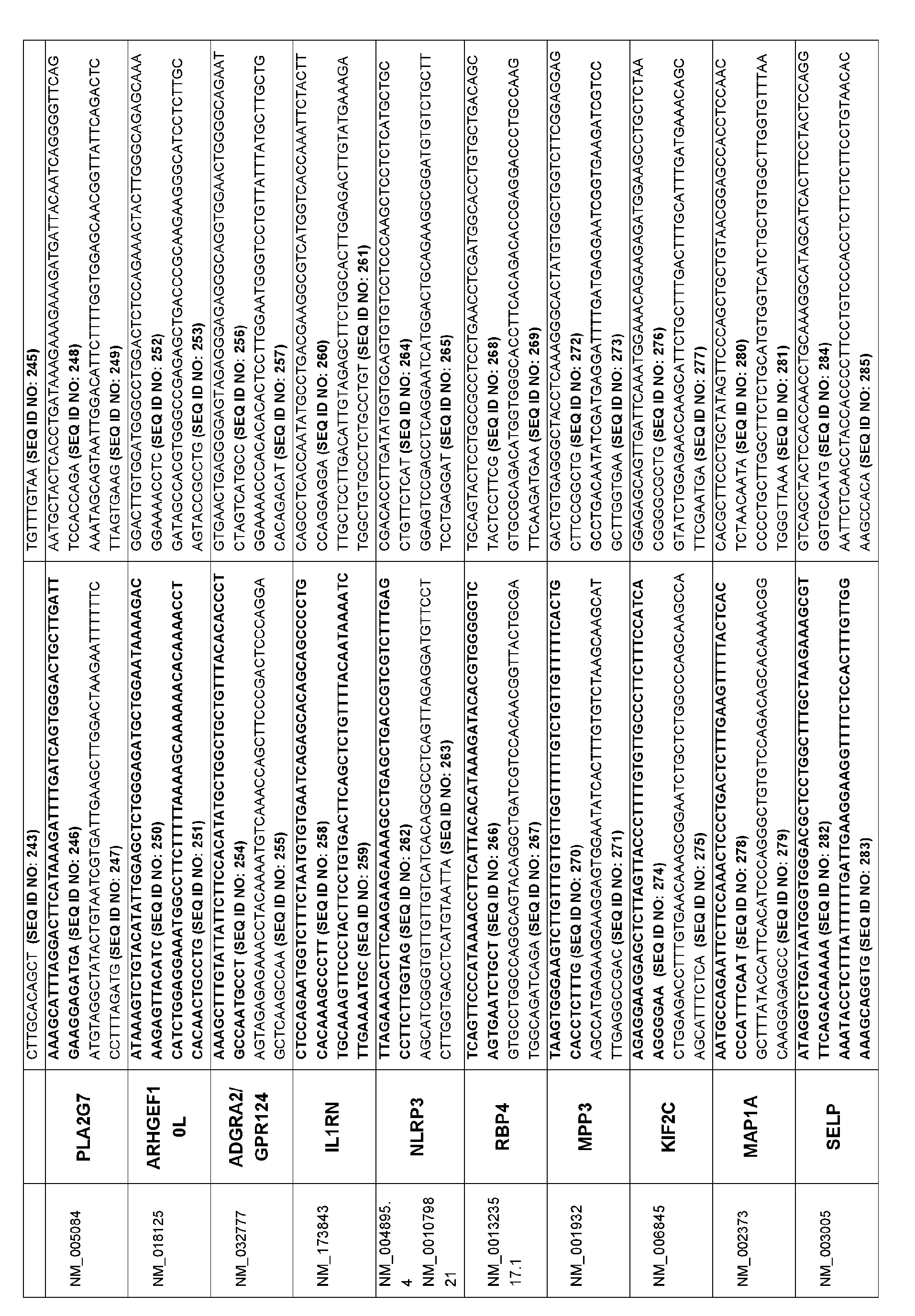

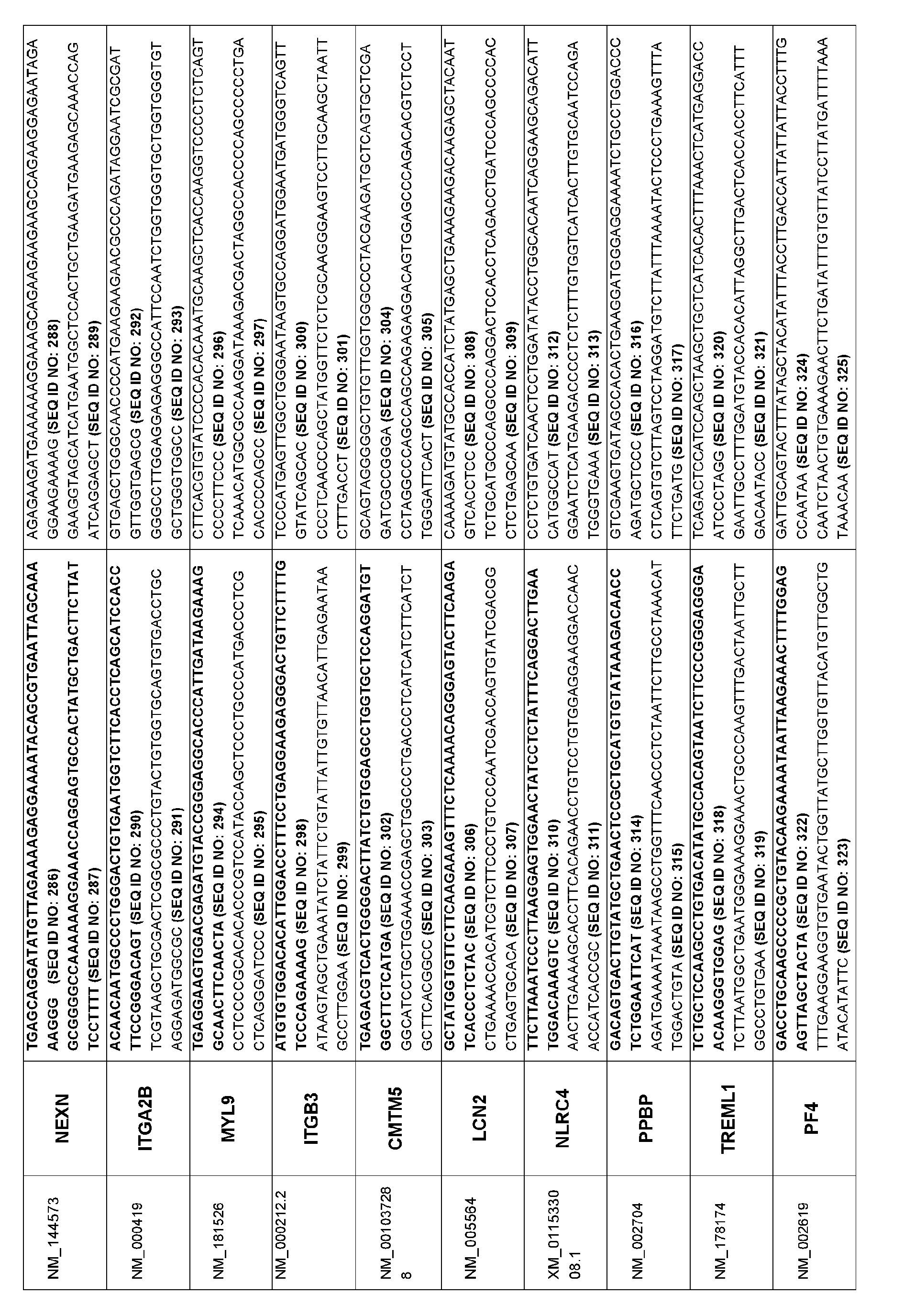

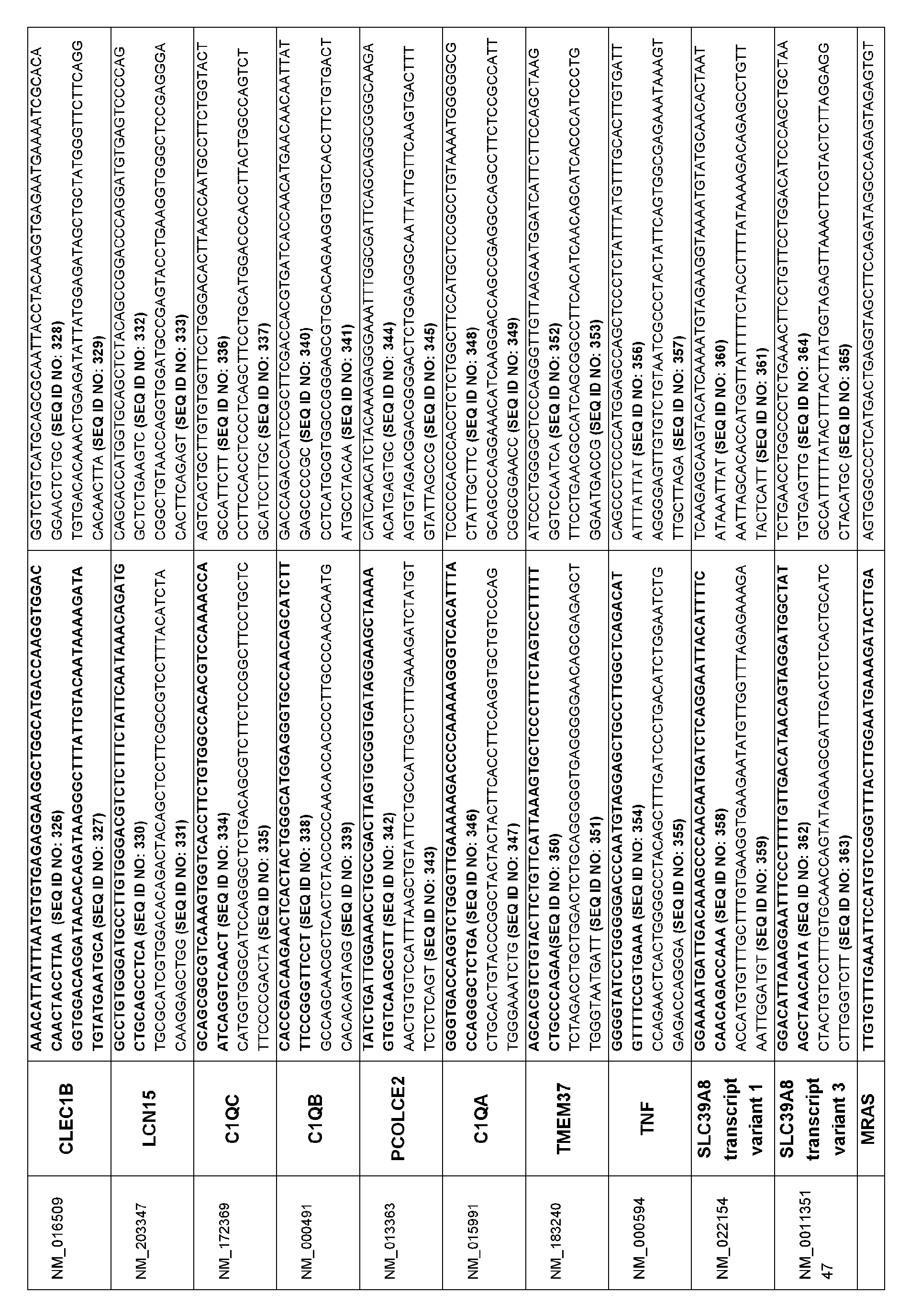

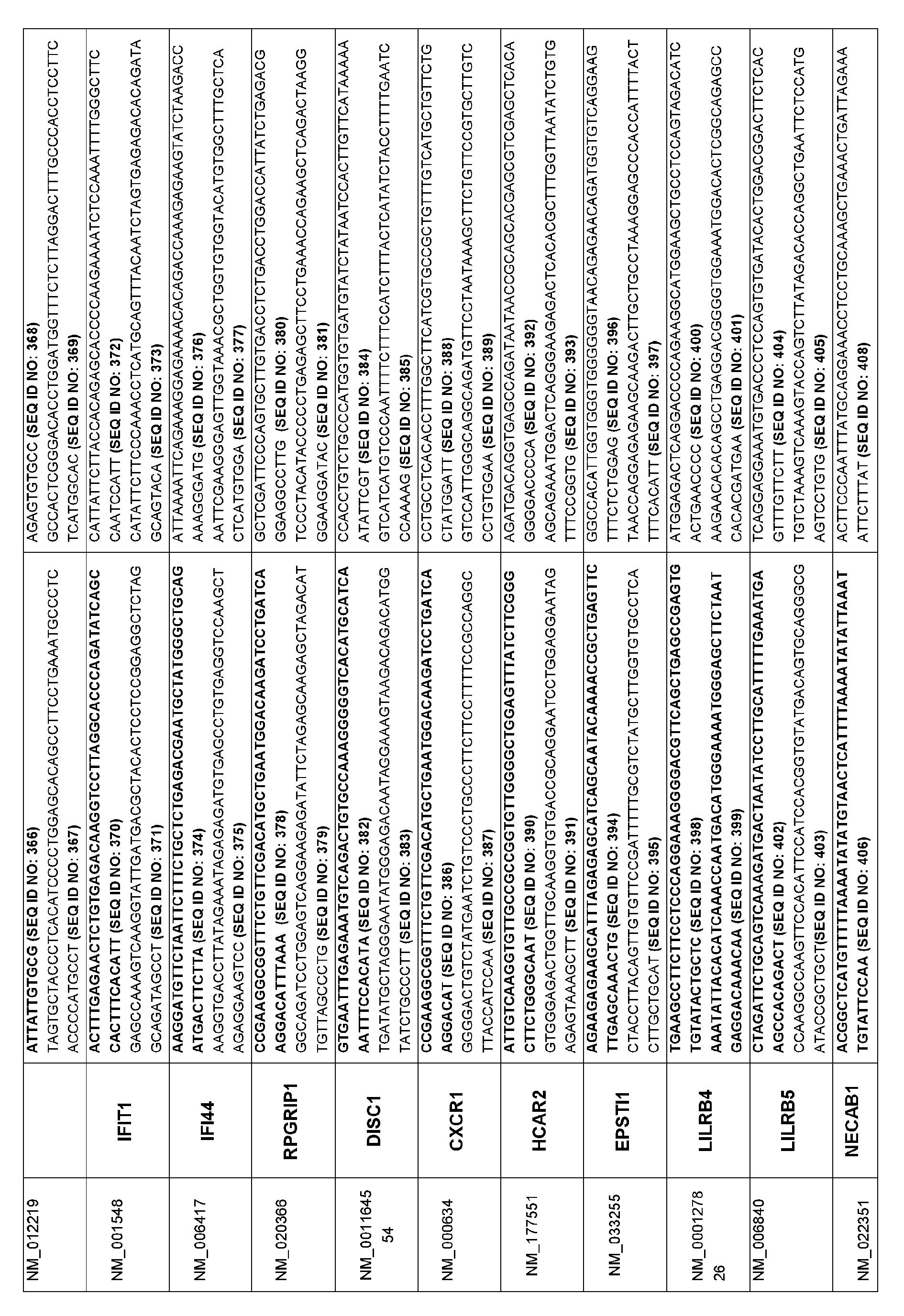

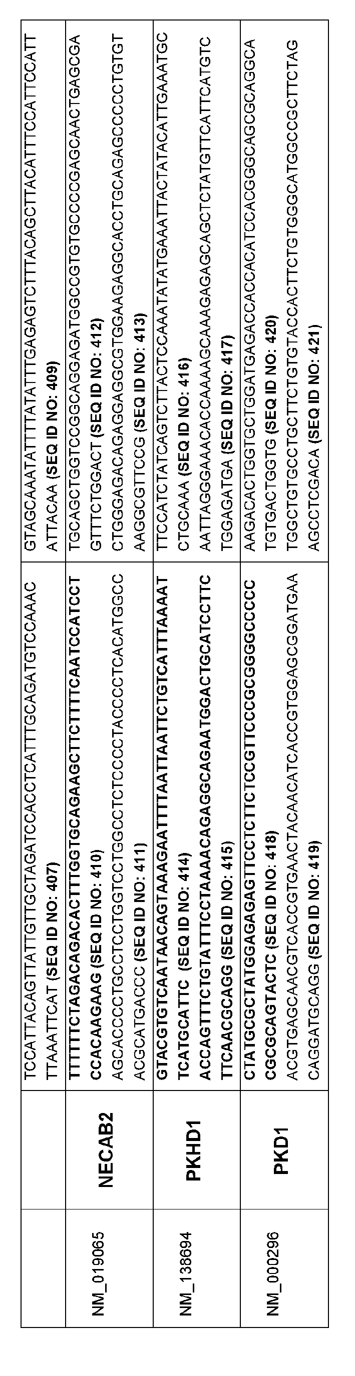

### Table 15:

Table 15 lists various oligonucleotide primers and corresponding probes used to detect the biomarkers of the invention

| **Biomarker** | **Forward primer** | **Reverse primer** | **Probe** |
|---|---|---|---|
| FAM20A | GCCATCTTCGACTTCTTGATAGG (SEQ ID NO:422) | CCCGAACTTGGTGAACATCTC (SEQ ID NO:423) | AATATGGACCGGCACCAT (SEQ ID NO:424) |
| | AGCGGCTCCTCAATGTCATC (SEQ ID NO:425) | CCGGTCCATATTCCCTATCAAG (SEQ ID NO:426) | CATGGCCATCTTCGACT (SEQ ID NO:427) |
| OLAH | ACATGGAAGCCTGGAAAGATGT (SEQ ID NO:428) | CCCCTGGAAGCTGGTAAATTT (SEQ ID NO:429) | ACCAGTGGAAATGC (SEQ ID NO:430) |
| | TTTGTCAAGTGCAACTCCTGTACA (SEQ ID NO:431) | GACAATTCATCATCTTTGGGAATG (SEQ ID NO:432) | TCAAAGGCCTGGCATC (SEQ ID NO:433) |
| LCN2 | TGGGCCTCCCTGAAAACC (SEQ ID NO:434) | CCGTCGATACACTGGTCGATT (SEQ ID NO:435) | CATCGTCTTCCCTGTCC (SEQ ID NO:436) |
| | GTTTCTCAAAACAGGGAGTACTTCAA (SEQ ID NO:437) | CTTTAGTTCCGAAGTCAGCTCCTT (SEQ ID NO:438) | ATCACCCTCTACGGGAGA (SEQ ID NO:439) |
| ITGB3 | GTCCTCCAGCTCATTGTTGATG (SEQ ID NO:440) | GGTCACGCACTTCCAGCTCTA | TTATGGGAAAATCCGTTCTAA |

| | | | |
|---|---|---|---|
| | | (SEQ ID NO:441) | (SEQ ID NO:442) |
| | ACGAAAATACCTGCAACCGTTAC (SEQ ID NO:443) | TTGCCAGTGTCCTTAAGCTCTTT (SEQ ID NO:444) | CGTGACGAGATTGAGTC (SEQ ID NO:445) |
| MYL9 | GAGCCCAAGCGCCTTCTC (SEQ ID NO:446) | CCCGCTTGCTGGACATCTT (SEQ ID NO:447) | ACCAGGGAAGCCCCA (SEQ ID NO:448) |
| | GCCGGCCCAGTTCCA (SEQ ID NO:449) | CTTCCCTGGTGCGGAGAA (SEQ ID NO:450) | ACCCAGCGAGCCCA (SEQ ID NO:451) |
| TREML1 | ACCCAGCCAGGATGAGAAGA (SEQ ID NO:452) | GCCACCAGCAGACCTACCA (SEQ ID NO:453) | ATCCCCTTGATCTGGG (SEQ ID NO:454) |
| | CAGTGCCAACCCTTTGGAA (SEQ ID NO:455) | GGAGCACAGCACCCCAGAT (SEQ ID NO:456) | CCAGCCAGGATGAG (SEQ ID NO:457) |
| ITGA2B | CTGGGCAACCCCATGAAG (SEQ ID NO:458) | CCCCACGCTCACCAACAT (SEQ ID NO:459) | AACGCCCAGATAGGA (SEQ ID NO:460) |
| | CCCTTCGAGGTGCCGTAGA (SEQ ID NO:461) | CCGTAAGCTCCCACGATCAG (SEQ ID NO:462) | TCGATGACAACGGATACC (SEQ ID NO:463) |
| PLA2G7 | TGTTGCCCATATGAAATCATCAG (SEQ ID NO:464) | AAGCTTGCAGCAGCCATCA (SEQ ID NO:465) | ATGGGTCAACAAAATACAAGT (SEQ ID NO 466) |
| | ACACTGGCTTATGGGCAACAT (SEQ ID NO:467) | ATTCCAGTTTGCAGGAGTTGTCA (SEQ ID NO:468) | TTGAGGTTACTCTTTGGTTCA (SEQ ID NO:469) |
| ARHGEF10L | ATCCACTCGGCCAACAAGTG (SEQ ID NO:470) | GCCGGACTTGTCGGGTTT (SEQ ID NO:471) | CGTCTCAGGCTCCTG (SEQ ID NO:472) |
| TGFBI | GGTCCATGTCATCACCAATGTT (SEQ ID NO:473) | CCCCTCTTTCCTGAGGTCTGT (SEQ ID NO:474) | TGCAGCCTCCAGCC (SEQ ID NO:475) |
| | GGCGTGGTCCATGTCATCA (SEQ ID NO:476) | CCCTCTTTCCTGAGGTCTGTTG (SEQ ID NO:477) | TGTTCTGCAGCCTCC (SEQ ID NO:478) |
| MYCL | GCTGGGCGAACCCAAGA (SEQ ID NO:479) | TCTTCTCTACTGTCACAACATCAATTTC (SEQ ID NO:480) | CCAGGCCTGCTCC (SEQ ID NO:481) |
| | GCTGGGCGAACCCAAGA (SEQ ID NO:482) | TCTTCTCTACTGTCACAACATCAATTTC (SEQ ID NO: 483) | CGACTCGGAGAATGA (SEQ ID NO:484) |
| CXCR1 | GACTGCAGCTCCTACTGTTGGA (SEQ ID NO:485) | TTCAGCAATGGTTTGATCTAACTGA (SEQ ID | ACACCTGGCCGGTGC (SEQ ID |

| | | | |
|---|---|---|---|
| | | NO:486) | NO:487) |
| | CCTGGCCGGTGCTTCAG (SEQ ID NO:488) | TCTGTAATATTTGACATGTCCTCTTCAG (SEQ ID NO:489) | AGATCAAACCATTGCTG (SEQ ID NO:490) |
| HCAR2 | TTAGGGAAACGGTGGCAGAT (SEQ ID NO:491) | GATTCCTGCGGTCACACCTT (SEQ ID NO:492) | AGTGGGAGACTGGTTG (SEQ ID NO:493) |
| | TTCACCTACATGAACAGCATGCT (SEQ ID NO:494) | GGAAAGGATGGGCTGGAGAA (SEQ ID NO:495) | ACCCCGTGGTGTACTA (SEQ ID NO:496) |
| C1QA | GCGGCCAGGCCTCAA (SEQ ID NO:497) | CCCCCTGGTCTCCTTTAAGG (SEQ ID NO:498) | TCCGGACAGGCATC (SEQ ID NO:499) |
| C1QB | GCCTCACAGGACACCAGCTT (SEQ ID NO:500) | CCCATGGGATCTTCATCATCA(SEQ ID NO:501) | CCAGGAGGCGTCTGA (SEQ ID NO:502) |
| | | TGCCCCATGGGATCTTCAT (SEQ ID NO:503) | |
| C1QC | TGTGCCAGGCCAGAAACC (SEQ ID NO:504) | AGCAGCTTCAGCCCAAGGT (SEQ ID NO:505) | CCTTCTCCGGGATGGA (SEQ ID NO:506) |
| | GAAGCAGATCTGAGGACATCTCTGT (SEQ ID NO:507) | CGGGAATGGCTGGGATTC (SEQ ID NO:508) | CCGCCCACCTGCA (SEQ ID NO:509) |
| MRAS | CACCAGGGAGCAAGGAAAAG (SEQ ID NO:510) | GGCACTGGTTTCTATGTACGGAAT (SEQ ID NO:511) | TGGCGACCAAACACAA (SEQ ID NO:512) |
| | CAATGTCGACAAAGCCTTCCA (SEQ ID NO:513) | GGCTTTTTTCCGGAATCTGTT (SEQ ID NO:514) | ACCTCGTTAGAGTAATTAG(SEQ ID NO:515) |
| SLC39A8 | GCACTTGCTGGAGGCATGT (SEQ ID NO:516) | TCAGCATATCATTCATCTCTGGAAA (SEQ ID NO:517) | CCTCTATATTTCTCTGGCAGATA (SEQ ID NO:518) |
| | GGGACTCAGTACTTCCATAGCAATC (SEQ ID NO:519) | TGCATTGAGTAGGATCACAAAGTCT (SEQ ID NO:520) | TGAGGAGTTTCCCCACGAG (SEQ ID NO:521) |
| TMEM37 | CGGGCGCAGCATGACT (SEQ ID NO:522) | CCGGATGAAGGATTCAAAGAAG (SEQ ID NO:523) | CCGTCGGCGTGCAG(SEQ ID NO:524) |
| | | | CCCAGAGGCCTTTG (SEQ ID NO:525) |
| FCER1A | GGCAGCTGGACTATGAGTCTGA (SEQ ID NO:526) | CTTCTCACGCGGAGCTTTTATT | CCCCTCAACATTACTG |

| | | | |
|---|---|---|---|
| | | (SEQ ID NO:527) | (SEQ ID NO:528) |
| | ACCGAGCATGGGCCTATATTT (SEQ ID NO:529) | CATGGACTCCTGGTGCTTACTG (SEQ ID NO:530) | AAGCCTTAGATCTCTCC (SEQ ID NO:531) |
| BCL11B | GCAACCCGCAGCACTTGT (SEQ ID NO:532) | TGGCGGCCTCCACATG (SEQ ID NO:533) | CCAGAGGGAGCTCAT (SEQ ID NO:534) |
| | TCCCAGAGGGAGCTCATCAC (SEQ ID NO:535) | TCTCCAGACCCTCGTCTTCTTC (SEQ ID NO:536) | AGGCTGACCATGTGGAG (SEQ ID NO:537) |
| PKHD1 | TGAGGATCTATGAACGGCTCAA (SEQ ID NO:538) | CCATCCTCCGTGACATGTACAC (SEQ ID NO:539) | CACCGGCATATTGG (SEQ ID NO:540) |
| | CATTACAACCCAGGAAAGATTTAGG (SEQ ID NO:541) | GAGATATTTTCTTGGACTTGCACAGT (SEQ ID NO:542) | AAAGTAGTCTGTCCTGAATTA (SEQ ID NO:543) |
| KLRB1 | TTGGTTGTTACTGGGTTGAGTGTT (SEQ ID NO:544) | CCACACTGCATTTTTCTATTGATGA (SEQ ID NO:545) | CAGTGACATCCTTAATACAG (SEQ ID NO:546) |
| | CAACAGAGCAGGAATAAAACAACAG (SEQ ID NO:547) | TCTCGGAGTTGCTGCCAATA (SEQ ID NO:548) | CCGGGTCTCTTAAAC (SEQ ID NO:549) |
| LILRB5 | CCAGCCCAGTTGCTGACAT (SEQ ID NO:550) | TGTCCTTCACGGCAGCATT (SEQ ID NO:551) | CAGGAGGAAATTCT (SEQ ID NO:552) |
| | CCAGGGCCTGCAGAAGAG (SEQ ID NO:553) | CGGCAGCATTGAGAATTTCC (SEQ ID NO:554) | CCAGCCCAGTTGCTGA (SEQ ID NO:555) |
| NECAB1 | TCTACAATGCTAGTTCCTGCTTCGT (SEQ ID NO:556) | GCACTTGGAACCATAAAGAAAATGT (SEQ ID NO:557) | TCCTGAACAACTAGATGTT (SEQ ID NO:558) |
| | ACAACAGCTTCTCCCCAAACA (SEQ ID NO:559) | CTGGGTCATCCACTGGTTGTC (SEQ ID NO:560) | ATGTCAGCGGTCCAGG (SEQ ID NO:561) |
| NECAB2 | GTGGAGGCCATCGAGGAA (SEQ ID NO:562) | GCTGTGGCTGGGTTTGATGT (SEQ ID NO:563) | CAGCTCCGACAGAAC (SEQ ID NO:564) |
| | GCCGTGCGGACAAAAATG (SEQ ID NO:565) | TCTGCAAAGAAGAGCTGGAATTC (SEQ ID NO:566) | TGATGGGAAGCTGTCC (SEQ ID NO:567) |
| ALAS1 (control gene) | GATGATGCCAGGCTGTGAGA (SEQ ID NO:568) | CGAATCCCTTGGATCATGGA (SEQ ID NO:569) | CTGATTCTGGGAACCAT (SEQ ID NO:570) |
| | AACCCTCTTCACCCTGGCTAA | GGCATGGTTCCCAGAATCAG | ATGATGCCAGGCTGTG |

| | | | |
|---|---|---|---|
| | (SEQ ID NO:571) | (SEQ ID NO:572) | (SEQ ID NO:573) |
| HMBS (control gene) | CCTGCCCACTGTGCTTCCT (SEQ ID NO:574) | GGTTTTCCCGCTTGCAGAT (SEQ ID NO:575) | CTGGCTTCACCATCG (SEQ ID NO:576) |
| | GCCTGTTTACCAAGGAGCTTGA (SEQ ID NO:577) | TGAACAACCAGGTCCACTTCAT (SEQ ID NO:578) | CATGCCCTGGAGAAG (SEQ ID NO:579) |
| GTF2D1 (TBP) (control gene) | GCACTTCGTGCCCGAAAC (SEQ ID NO:580) | CCTCATGATTACCGCAGCAA (SEQ ID NO:581) | CGAATATAATCCCAAGCGG (SEQ ID NO:582) |
| | GCCCGAAACGCCGAATAT (SEQ ID NO:583) | CGTGGCTCTCTTATCCTCATGA (SEQ ID NO:584) | AGCGGTTTGCTGCGGT (SEQ ID NO:585) |

### SEQUENCE INFORMATION

Set out below are the nucleotide sequences of the biomarkers described herein. Exemplary target regions within the biomarker sequences are underlined, and exemplary probe sequences are underlined and shown in bold text.
**ADM (SEQ ID NO: 1)** - Homo sapiens adrenomedullin (ADM), mRNA - NM 001124 
1 gaggaaagaa agggaaggca accgggcagc ccaggccccg ccccgccgct cccccacccg
61 tgcgcttata aagcacagga accagagctg gccactcagt ggtttcttgg tgacactgga
121 tagaacagct caagccttgc cacttcgggc ttctcactgc agctgggctt ggacttcgga
181 gttttgccat tgccagtggg acgtctgaga ctttctcctt caagtacttg gcagatcact
241 ctcttagcag ggtctgcgct tcgcagccgg gatgaagctg gtttccgtcg ccctgatgta
301 cctgggttcg ctcgccttcc taggcgctga caccgctcgg ttggatgtcg cgtcggagtt
361 tcgaaagaag tggaataagt gggctctgag tcgtgggaag agggaactgc ggatgtccag
421 cagctacccc accgggctcg ctgacgtgaa ggccgggcct gcccagaccc ttattcggcc
481 ccaggacatg aagggtgcct ctcgaagccc cgaagacagc agtccggatg ccgcccgcat
541 ccgagtcaag cgctaccgcc agagcatgaa caacttccag ggcctccgga gctttggctg
601 ccgcttcggg acgtgcacgg tgcagaagct ggcacaccag atctaccagt tcacagataa
661 ggacaaggac aacgtcgccc ccaggagcaa gatcagcccc cagggctacg gccgccggcg
721 ccggcgctcc ctgcccgagg ccggcccggg tcggactctg gtgtcttcta agccacaagc
781 acacggggct ccagcccccc cgagtggaag tgctccccac tttctttagg atttaggcgc
841 ccatggtaca aggaatagtc gcgcaagcat cccgctggtg cctcccggga cgaaggactt
901 cccgagcggt gtggggaccg ggctctgaca gccctgcgga gaccctgagt ccgggaggca
961 ccgtccggcg gcgagctctg gctttgcaag ggcccctcct tctgggggct tcgcttcctt
1021 agccttgctc aggtgcaagt gccccagggg gcggggtgca gaagaatccg agtgtttgcc
1081 aggcttaagg agaggagaaa ctgagaaatg aatgctgaga cccccggagc aggggtctga
1141 gccacagccg tgctcgccca caaactgatt tctcacggcg tgtcacccca ccagggcgca
1201 agcctcacta ttacttgaac tttccaaaac ctaaagagga aaagtgcaat gcgtgttgta
1261 catacagagg taactatcaa tatttaagtt tgttgctgtc aagatttttt ttgtaacttc
1321 aaatatagag atatttttgt acgttatata ttgtattaag ggcattttaa aagcaattat
1381 attgtcctcc cctattttaa gacgtgaatg tctcagcgag gtgtaaagtt gttcgccgcg
1441 tggaatgtga gtgtgtttgt gtgcatgaaa gagaaagact gattacctcc tgtgtggaag
1501 aaggaaacac cgagtctctg tataatctat ttacataaaa tgggtgatat gcgaacagca
1561 aaccaataaa ctgtctcaat gctgattcat aaaaaaaaaa aaaaaa
**CD177 (SEQ ID NO: 2)** - Homo sapiens CD117 molecule (CD177), mRNA - NM 020406 
1 aaaggacttg tttcctgctg aaaaagcaga aagagattac cagccacaga cgggtcatga
61 gcgcggtatt actgctggcc ctcctggggt tcatcctccc actgccagga gtgcaggcgc
121 tgctctgcca gtttgggaca gttcagcatg tgtggaaggt gtccgacctg ccccggcaat
181 ggacccctaa gaacaccagc tgcgacagcg gcttggggtg ccaggacacg ttgatgctca
241 ttgagagcgg accccaagtg agcctggtgc tctccaaggg ctgcacggag gccaaggacc
301 aggagccccg cgtcactgag caccggatgg gccccggcct ctccctgatc tcctacacct
361 tcgtgtgccg ccaggaggac ttctgcaaca acctcgttaa ctccctcccg ctttgggccc
421 cacagccccc agcagaccca ggatccttga ggtgcccagt ctgcttgtct atggaaggct
481 gtctggaggg gacaacagaa gagatctgcc ccaaggggac cacacactgt tatgatggcc
541 tcctcaggct caggggagga ggcatcttct ccaatctgag agtccaggga tgcatgcccc
601 agccagtttg caacctgctc aatgggacac aggaaattgg gcccgtgggt atgactgaga
661 actgcgatat gaaagatttt ctgacctgtc atcgggggac caccattatg acacacggaa
721 acttggctca agaacccact gattggacca catcgaatac cgagatgtgc gaggtggggc
781 aggtgtgtca ggagacgctg ctgctcctag atgtaggact cacatcaacc ctggtgggga
841 caaaaggctg cagcactgtt ggggctcaaa attcccagaa gaccaccatc cactcagccc
901 ctcctggggt gcttgtggcc tcctataccc acttctgctc ctcggacctg tgcaatagtg
961 ccagcagcag cagcgttctg ctgaactccc tccctcctca agctgcccct gtcccaggag
1021 accggcagtg tcctacctgt gtgcagcccc ttggaacctg ttcaagtggc tccccccgaa
 
1081 tgacctgccc caggggcgcc actcattgtt atgatgggta cattcatctc tcaggaggtg
1141 ggctgtccac caaaatgagc attcagggct gcgtggccca accttccagc ttcttgttga
1201 accacaccag acaaatcggg atcttctctg cgcgtgagaa gcgtgatgtg cagcctcctg
1261 cctctcagca tgagggaggt ggggctgagg gcctggagtc tctcacttgg ggggtggggc
1321 tggcactggc cccagcgctg tggtggggag tggtttgccc ttcctgctaa ctctattacc
1381 cccacgattc ttcaccgctg ctgaccaccc acactcaacc tccctctgac ctcataacct
1441 aatggccttg gacaccagat tctttcccat tctgtccatg aatcatcttc cccacacaca
1501 atcattcata tctactcacc taacagcaac actggggaga gcctggagca tccggacttg
1561 ccctatggga gaggggacgc tggaggagtg gctgcatgta tctgataata cagaccctgt
1621 cctttctccc agtgctggga tttctccatg tgagggggca gcaggacacc cagggatcta
1681 gcgtggggga ggagaggagc ctaatgagaa aatgaccatc taaagcctgc ccttcattgg
1741 tctggttcac gtctccaaac cagcttggat ggtagcagag acttcagggt gctccagcca
1801 aacgtatttg ggcatcacca tgacctggga ggggaagatg cactgagacg tatgaggctt
1861 ccagcctagc agccagggcc ctagcacaaa caggaggctc gccccatctg agcaactgca
1921 ggagaggtta gtacagtcat gcattgctta acgacaggga cgtgtcgtta gaaatgtgtc
1981 gttaggtgat tttatgacca taggaacatt gtagcgtgca cttacaccaa cccagatggt
2041 acagcccaat acacacccag gatggacgct agagtcgact gctcctaggc tacaagcctg
2101 cagtgcatgt tatggtgtga atactgcagg caatcttaac accacggcaa gtatttgtgc
2161 atctacacac atctaaacat agaaaaggta cagcataaat acactattgt catctcagca
2221 gaaaaaaaaa aaaaaaaa
**FAM20A (SEQ ID NO: 3)** - Homo sapiens family with sequence similarity 20 member A (FAM20A), transcript variant 1, mRNA - NM 017565 
1 aaaggcgcaa gaagcgggca ccccgggaac cccattccct cggctcactc ggcgcggaga
61 agcgacgccc gctgactccg agagccccgg tgctccgtgc acctggtccc caagttgagg
121 agcgacaccc ctccacaggg gactagcccg cgcggggagc attccggtct cactgacccc
181 ggcccacccg cgggactcca ggcacctctt ctgcccgcac cccgcgaccc ctcccgggac
241 cccggagaca gccggcctgc ccccggcgtc ccccttggcc agcacgccat gccggggctg
301 cgccgggacc gcctactgac tctgctgctg ctgggcgcgc tgctctccgc cgacctctac
361 ttccacctct ggccccaagt acagcgccag ctgcggcctc gggagcgccc gcgggggtgc
421 ccgtgcaccg gccgcgcctc ctccctggcg cgggactcgg ccgcagctgc ctcggacccc
481 ggcacgatcg tgcacaactt ttcccgaacc gagccccgga ctgaaccggc tggcggcagc
541 cacagcgggt cgagctccaa gttgcaggcc ctcttcgccc acccgctgta caacgtcccg
601 gaggagccgc ctctcctggg agccgaggac tcgctcctgg ccagccagga ggcgctgcgg
661 tattaccgga ggaaggtggc ccgctggaac aggcgacaca agatgtacag agagcagatg
721 aaccttacct ccctggaccc cccactgcag ctccgactcg aggccagctg ggtccagttc
781 cacctgggta ttaaccgcca tgggctctac tcccggtcca gccctgttgt cagcaaactt
841 ctgcaagaca tgaggcactt tcccaccatc agtgctgatt acagtcaaga tgagaaagcc
901 ttgctggggg catgtgactg cacccagatt gtgaaaccca gtggggtcca cctcaagctg
961 gtgctgaggt tctcggattt cgggaaggcc atgttcaaac ccatgagaca gcagcgagat
1021 gaggagacac cagtggactt cttctacttc attgactttc agagacacaa tgctgagatc
1081 gcagctttcc atctggacag gattctggac ttccgacggg tgccgccaac agtggggagg
1141 atagtaaatg tcaccaagga aatcctagag gtcaccaaga atgaaatcct gcagagtgtt
1201 ttctttgtct ctccagcgag caacgtgtgc ttcttcgcca agtgtccata catgtgcaag
1261 acggagtatg ctgtctgtgg caacccacac ctgctggagg gttccctctc tgccttcctg
1321 ccgtccctca acctggcccc caggctgtct gtgcccaacc cctggatccg ctcctacaca
1381 ctggcaggaa aagaggagtg ggaggtcaat cccctttact gtgacacagt gaaacagatc
1441 tacccgtaca acaacagcca gcggctcctc aatgtcatcg acatggccat cttcgacttc
1501 ttgataggga atatggaccg gcaccattat gagatgttca ccaagttcgg ggatgatggg
1561 ttccttattc accttgacaa cgccagaggg ttcggacgac actcccatga tgaaatctcc
1621 atcctctcgc ctctctccca gtgctgcatg ataaaaaaga aaacactttt gcacctgcag
1681 ctgctggccc aagctgacta cagactcagc gatgtgatgc gagaatcact gctggaagac
1741 cagctcagcc ctgtcctcac tgaaccccac ctccttgccc tggatcgaag gctccaaacc
1801 atcctaagga cagtggaggg gtgcatagtg gcccatggac agcagagtgt catagtcgac
1861 ggcccagtgg aacagttggc cccagactct ggccaggcta acttgacaag ctaagggctg
 
1921 gcagagtcca gtttcagaaa atacgcctgg agccagagca gtcgactcga gtgccgaccc
1981 tgcgtcctca ctcccacctg ttactgctgg gagtcaagtc agctaggaag gaagcaggac
2041 attttctcaa acagcaagtg gggcccatgg aactgaatct ttactccttg gtgcaccgct
2101 tctgtcgtgc gttgccttgc tccgtttttc ccaaaaagca ctggcttcat caaggccacc
2161 gacgatctcc tgagtgcact gggaaatctg ggtataggtc aggcttggca gccttgatcc
2221 caggagagta ctaatggtaa caagtcaaat aaaaggacat caagtggata cctgacttct
2281 caggatcctt attctagcta caagtcaaag ataactcctg gtccagacaa aacacctggc
2341 ctatcacaag ctgactaaaa atctgcactt tgggccagcg caggcaacag taactctgac
2401 aggttcaaat tagacctcac actttctact catattctag tcactggacc catctgaatc
2461 agtaatccct actgcccggt cctggagtaa cttcttagag atattataac aagtggcaaa
2521 aataaaagag ggatttgcta agaatatcag aaaaggagtg ttccaatttg aagagtatta
2581 caattgaaat aacatcaaat atgtcacact aagcagccag taacagaata aataattaca
2641 acgaaggaaa aaaaaaggaa agtcctccaa ggtcaggatg gcatgggaac aggcctagca
2701 gggacacaag cctggagtaa ggcaggaaaa gagccaaggc tgactacagc ccaccaacca
2761 caatcttctt ccctaagacc ccaggattgt ccccggccca tccccaaaag aagaaagggg
2821 ctatgtggaa aggtgaggcc ctctagatgc ccctccctgt gacggctggc tcaaaaaaga
2881 ctaagtcagg ttttttattg aagcctccct tagaagaaag tgtagtaggg attccttgtc
2941 tcctctacct tctccctgac ttgttgatat ctgaaactcc ttttaatagg aggctttgtt
3001 tcttattcca taattaatga ccgaaaacac aaaggacagt aaggtcttca ttccatggag
3061 tgactagtca gtcaacaaaa gtaaaaatgc ctcatttcat caaatcaact tttccagtaa
3121 aggccagagt tcaaatactg taagcatgag aatagaatgg agctgtcact aaagagagta
3181 caaggtcaag aaccagaaat gtctccaagc tttctgcagt gtgagcaaag atagcctcga
3241 ctggacaaat aacacttctg accccgtggt acccccacat gactggtatg ctgcccctgg
3301 actttgtgtc tctctaaaat cactaccatt cactgagatc tgaccaggtg aggcactcac
3361 caacgctaca gaagcaacac acaggtttcc aatcctctca aacacagata caacccccat
3421 cctccaatgt gcatggaaga actttaaatc tcagagagag taagcaaaat cacacaattt
3481 agtggtttag acctattcca aagtcttctt ttcactgtaa cacaaaaccc aacagtatcc
3541 cacccaattt atgaagtcaa atttgtttgc ttaaaaaaaa aatcagctgg gcatgggctc
3601 acacctgtaa tcccagcact ttgggcctaa acaggcagat cgcttggtct caggagctcg
3661 agaccagcct gggtaacatg acaaaaccct gtctctacaa aaaaatacaa aaaaaaaaaa
3721 attttttttt aaatcaactc tttaacagag aaaccttaat cccaaagatt tccttaactg
3781 ttgtggccct actgcagcat agggctcccc acttcattca ctgtctctcc tgcttcaaga
3841 tgtttcttcc ctgtggatga agaaccagga gatcacacac agaaatggga aaatatctgg
3901 gtaaaaacat tcaaagaata gcacaaaatg gtttgggact tccaaataag actctcagga
3961 aagcaggcta cagctctacc tcaaaacccg ccacctccta taacgaagga ctggttttcc
4021 aatggcctat atacacattt tgctgcactc tacaaacaga aagcaatagg cttgaatttc
4081 accatgatat gtggtacacc taccttatta ttcacttatc tttcaacctt ccattacttt
4141 ttgattagta acaaaattaa gactcatttc tcaaaaatat cagaaaacca accgtttcat
4201 tcttactcga gttcttccag catgttcatt ttgaactgtt tacctcccat gaaataaaaa
4261 gtctcttgac tctgaaaaaa aaaaa
**IL10 (SEQ ID NO: 4)** - Homo sapiens interleukin 10 (IL10), mRNA - NM 000572 
1 acacatcagg ggcttgctct tgcaaaacca aaccacaaga cagacttgca aaagaaggca
61 tgcacagctc agcactgctc tgttgcctgg tcctcctgac tggggtgagg gccagcccag
121 gccagggcac ccagtctgag aacagctgca cccacttccc aggcaacctg cctaacatgc
181 ttcgagatct ccgagatgcc ttcagcagag tgaagacttt ctttcaaatg aaggatcagc
241 tggacaactt gttgttaaag gagtccttgc tggaggactt taagggttac ctgggttgcc
301 aagccttgtc tgagatgatc cagttttacc tggaggaggt gatgccccaa gctgagaacc
361 aagacccaga catcaaggcg catgtgaact ccctggggga gaacctgaag accctcaggc
421 tgaggctacg gcgctgtcat cgatttcttc cctgtgaaaa caagagcaag gccgtggagc
481 aggtgaagaa tgcctttaat aagctccaag agaaaggcat ctacaaagcc atgagtgagt
541 ttgacatctt catcaactac atagaagcct acatgacaat gaagatacga aactgagaca
601 tcagggtggc gactctatag actctaggac ataaattaga ggtctccaaa atcggatctg
661 gggctctggg atagctgacc cagccccttg agaaacctta ttgtacctct cttatagaat
721 atttattacc tctgatacct caacccccat ttctatttat ttactgagct tctctgtgaa
 
781 cgatttagaa agaagcccaa tattataatt tttttcaata tttattattt tcacctgttt
841 ttaagctgtt tccatagggt gacacactat ggtatttgag tgttttaaga taaattataa
901 gttacataag ggaggaaaaa aaatgttctt tggggagcca acagaagctt ccattccaag
961 cctgaccacg ctttctagct gttgagctgt tttccctgac ctccctctaa tttatcttgt
1021 ctctgggctt ggggcttcct aactgctaca aatactctta ggaagagaaa ccagggagcc
1081 cctttgatga ttaattcacc ttccagtgtc tcggagggat tcccctaacc tcattcccca
1141 accacttcat tcttgaaagc tgtggccagc ttgttattta taacaaccta aatttggttc
1201 taggccgggc gcggtggctc acgcctgtaa tcccagcact ttgggaggct gaggcgggtg
1261 gatcacttga ggtcaggagt tcctaaccag cctggtcaac atggtgaaac cccgtctcta
1321 ctaaaaatac aaaaattagc cgggcatggt ggcgcgcacc tgtaatccca gctacttggg
1381 aggctgaggc aagagaattg cttgaaccca ggagatggaa gttgcagtga gctgatatca
1441 tgcccctgta ctccagcctg ggtgacagag caagactctg tctcaaaaaa taaaaataaa
1501 aataaatttg gttctaatag aactcagttt taactagaat ttattcaatt cctctgggaa
1561 tgttacattg tttgtctgtc ttcatagcag attttaattt tgaataaata aatgtatctt
1621 attcacatc
**METTL7B (SEQ ID NO: 5)** - Homo sapiens methyltransferase like 7B (METTL7B), mRNA - NM_152637 
1 aaaagtcatt gaagagcttg tggggctgtg ggacctgcgc cctctggagg aattccatac
61 acccactcaa ctctggcaaa taggaaattg tcaagtagga gacaaggagc aaagtcctat
121 cacagcggga ggggacgcca gcgcctgcag aggctgagca gggaaaaagc cagtgcccca
181 gcggaagcac agctcagagc tggtctgcca tggacatcct ggtcccactc ctgcagctgc
241 tggtgctgct tcttaccctg cccctgcacc tcatggctct gctgggctgc tggcagcccc
301 tgtgcaaaag ctacttcccc tacctgatgg ccgtgctgac tcccaagagc aaccgcaaga
361 tggagagcaa gaaacgggag ctcttcagcc agataaaggg gcttacagga gcctccggga
421 aagtggccct actggagctg ggctgcggaa ccggagccaa ctttcagttc tacccaccgg
481 gctgcagggt cacctgccta gacccaaatc cccactttga gaagttcctg acaaagagca
541 tggctgagaa caggcacctc caatatgagc ggtttgtggt ggctcctgga gaggacatga
601 gacagctggc tgatggctcc atggatgtgg tggtctgcac tctggtgctg tgctctgtgc
661 agagcccaag gaaggtcctg caggaggtcc ggagagtact gagaccggga ggtgtgctct
721 ttttctggga gcatgtggca gaaccatatg gaagctgggc cttcatgtgg cagcaagttt
781 tcgagcccac ctggaaacac attggggatg gctgctgcct caccagagag acctggaagg
841 atcttgagaa cgcccagttc tccgaaatcc aaatggaacg acagccccct cccttgaagt
901 ggctacctgt tgggccccac atcatgggaa aggctgtcaa ataatctttc ccaagctcca
961 aggcactcat ttgctccttc cccagcctcc aattagaaca agccacccac cagcctatct
1021 atcttccact gagagggacc tagcagaatg agagaagaca ttcatgtacc acctactagt
1081 ccctctctcc ccaacctctg ccagggcaat ctctaacttc aatcccgcct tcgacagtga
1141 aaaagctcta cttctacgct gacccaggga ggaaacacta ggaccctgtt gtatcctcaa
1201 ctgcaagttt ctggactagt ctcccaacgt ttgcctccca atgttgtccc tttccttcgt
1261 tcccatggta aagctcctct cgctttcctc ctgaggctac acccatgcgt ctctaggaac
1321 tggtcacaaa agtcatggtg cctgcatccc tgccaagccc ccctgaccct ctctccccac
1381 taccaccttc ttcctgagct gggggcacca gggagaatca gagatgctgg ggatgccaga
1441 gcaagactca aagaggcaga ggttttgttc tcaaatattt tttaataaat agacgaaacc
1501 acgaaaaaaa aaaaaaaaaa aaaaaaaaaa a
**MMP9 (SEQ ID NO: 6)** - Homo sapiens matrix metallopeptidase 9 (MMP9), mRNA - NM_004994 
1 agacacctct gccctcacca tgagcctctg gcagcccctg gtcctggtgc tcctggtgct
61 gggctgctgc tttgctgccc ccagacagcg ccagtccacc cttgtgctct tccctggaga
121 cctgagaacc aatctcaccg acaggcagct ggcagaggaa tacctgtacc gctatggtta
181 cactcgggtg gcagagatgc gtggagagtc gaaatctctg gggcctgcgc tgctgcttct
241 ccagaagcaa ctgtccctgc ccgagaccgg tgagctggat agcgccacgc tgaaggccat
301 gcgaacccca cggtgcgggg tcccagacct gggcagattc caaacctttg agggcgacct
361 caagtggcac caccacaaca tcacctattg gatccaaaac tactcggaag acttgccgcg
421 ggcggtgatt gacgacgcct ttgcccgcgc cttcgcactg tggagcgcgg tgacgccgct
481 caccttcact cgcgtgtaca gccgggacgc agacatcgtc atccagtttg gtgtcgcgga
 
541 gcacggagac gggtatccct tcgacgggaa ggacgggctc ctggcacacg cctttcctcc
601 tggccccggc attcagggag acgcccattt cgacgatgac gagttgtggt ccctgggcaa
661 gggcgtcgtg gttccaactc ggtttggaaa cgcagatggc gcggcctgcc acttcccctt
721 catcttcgag ggccgctcct actctgcctg caccaccgac ggtcgctccg acggcttgcc
781 ctggtgcagt accacggcca actacgacac cgacgaccgg tttggcttct gccccagcga
841 gagactctac acccaggacg gcaatgctga tgggaaaccc tgccagtttc cattcatctt
901 ccaaggccaa tcctactccg cctgcaccac ggacggtcgc tccgacggct accgctggtg
961 cgccaccacc gccaactacg accgggacaa gctcttcggc ttctgcccga cccgagctga
1021 ctcgacggtg atggggggca actcggcggg ggagctgtgc gtcttcccct tcactttcct
1081 gggtaaggag tactcgacct gtaccagcga gggccgcgga gatgggcgcc tctggtgcgc
1141 taccacctcg aactttgaca gcgacaagaa gtggggcttc tgcccggacc aaggatacag
1201 tttgttcctc gtggcggcgc atgagttcgg ccacgcgctg ggcttagatc attcctcagt
1261 gccggaggcg ctcatgtacc ctatgtaccg cttcactgag gggcccccct tgcataagga
1321 cgacgtgaat ggcatccggc acctctatgg tcctcgccct gaacctgagc cacggcctcc
1381 aaccaccacc acaccgcagc ccacggctcc cccgacggtc tgccccaccg gaccccccac
1441 tgtccacccc tcagagcgcc ccacagctgg ccccacaggt cccccctcag ctggccccac
1501 aggtcccccc actgctggcc cttctacggc cactactgtg cctttgagtc cggtggacga
1561 tgcctgcaac gtgaacatct tcgacgccat cgcggagatt gggaaccagc tgtatttgtt
1621 caaggatggg aagtactggc gattctctga gggcaggggg agccggccgc agggcccctt
1681 ccttatcgcc gacaagtggc ccgcgctgcc ccgcaagctg gactcggtct ttgaggagcg
1741 gctctccaag aagcttttct tcttctctgg gcgccaggtg tgggtgtaca caggcgcgtc
1801 ggtgctgggc ccgaggcgtc tggacaagct gggcctggga gccgacgtgg cccaggtgac
1861 cggggccctc cggagtggca gggggaagat gctgctgttc agcgggcggc gcctctggag
1921 gttcgacgtg aaggcgcaga tggtggatcc ccggagcgcc agcgaggtgg accggatgtt
1981 ccccggggtg cctttggaca cgcacgacgt cttccagtac cgagagaaag cctatttctg
2041 ccaggaccgc ttctactggc gcgtgagttc ccggagtgag ttgaaccagg tggaccaagt
2101 gggctacgtg acctatgaca tcctgcagtg ccctgaggac tagggctccc gtcctgcttt
2161 ggcagtgcca tgtaaatccc cactgggacc aaccctgggg aaggagccag tttgccggat
2221 acaaactggt attctgttct ggaggaaagg gaggagtgga ggtgggctgg gccctctctt
2281 ctcacctttg ttttttgttg gagtgtttct aataaacttg gattctctaa cctttaaaaa
2341 aaaaaaaaaa aaaaaaaaaa aaaaaaaaaa aaaaaaaaaa aaaaaaa
**RETN (SEQ ID NO: 7)** - Homo sapiens resistin, transcript variant 1 (RENT), mRNA - NM 020415 
1 gtgtgccgga tttggttagc tgagcccacc gagaggcgcc tgcaggatga aagctctctg
61 tctcctcctc ctccctgtcc tggggctgtt ggtgtctagc aagaccctgt gctccatgga
121 agaagccatc aatgagagga tccaggaggt cgccggctcc ctaatattta gggcaataag
181 cagcattggc ctggagtgcc agagcgtcac ctccaggggg gacctggcta cttgcccccg
241 aggcttcgcc gtcaccggct gcacttgtgg ctccgcctgt ggctcgtggg atgtgcgcgc
301 cgagaccaca tgtcactgcc agtgcgcggg catggactgg accggagcgc gctgctgtcg
361 tgtgcagccc tgaggtcgcg cgcagcgcgt gcacagcgcg ggcggaggcg gctccaggtc
421 cggaggggtt gcgggggagc tggaaataaa cctggagatg atgatgatga tgatgatg
**TDRD9 (SEQ ID NO: 8)** - Homo sapiens tudor domain containing 9 (TDRD9), mRNA - NM 153046 
1 ttgggggatg ccgacgcctg ggccttgagg atgctgcgga agctcaccat cgagcagatc
61 aacgactggt tcaccatcgg caagacggtg accaatgtgg agctgctggg cgcgccgccc
121 gccttcccgg caggggcggc cagggaggag gtgcagcgcc aggacgtggc ccccggcgct
181 ggtcccgcgg cccaggctcc ggctctggcc caagctccgg cccggccggc cgctgcgttc
241 gaaaggtcac tcagccaaag gagctcagaa gtagagtata ttaacaaata cagacagctc
301 gaagcacaag agcttgatgt gtgtcgcagt gtccaaccaa ccagtgggcc aggtccaagg
361 ccatctttgg ctaaattaag cagtgtgacg tgcatcccag ggacaactta taaatatcct
421 gatttgccta taagtcgata caaggaagag gttgtgtctt tgatagaaag taattccgtg
481 gtgattatcc atgggccacg ggaagcggt aaaagcactc agctcccgca gtatatcttg
541 gaccactacg ttcagcgctc cgcctactgc agcattgtgg tcacccagcc ccggaagata
601 ggggcaagca gcatcgccag gtggatcagt aaagagcgtg cctggaccct gggaggtgtg
 
661 gtgggctacc aggtagggct agagaaaata gcaacagagg acaccaggct aatttatatg
721 acaactggag tcctgcttca gaaaatagtt agtgccaaga gtttgatgga attcacacat
781 atcatcattg atgaagtaca cgaacgaaca gaagaaatgg atttcctgct attggtagtc
841 cgcaaactct taagaacaaa ttcacgtttt gtgaaggtgg tcctgatgtc ggctaccatc
901 agctgtaaag agtttgcaga ctactttgct gttcctgttc aaaacaagat gaatcctgca
961 tatatttttg aagtggaagg caagccccat tcagttgaag agtattatct taatgatttg
1021 gagcacattc atcatagcaa gctctctcct catctcctgg aggaaccggt gataactaag
1081 gatatatatg aagttgctgt ctctctcatt cagatgtttg atgacttgga tatgaaggag
1141 agtgggaaca aggcttggtc gggggcccag tttgtgttgg agcgaagcag tgtgttggtg
1201 tttttgccag gtctgggtga aataaattat atgcatgaac ttctcacaag cctggttcat
1261 aaaaggttgc aggtctatcc actccattca agtgtggctt tagaagaaca gaataatgtc
1321 tttttaagtc cagtccctgg gtacagaaag attattctgt ccaccaatat tgcagagagt
1381 tctgtcacag ttccagatgt caaatatgtt atagattttt gtttgactag aactttggtc
1441 tgtgatgaag atacaaatta tcagagtctg cgattgagtt gggcttctaa aaccagctgt
1501 aatcagagaa aaggccgtgc tggacgagtg tctagagggt actgttaccg gctggtacac
1561 aaggatttct gggacaactc catccctgat catgttgttc ctgagatgtt gcgttgtcca
1621 ttaggaagca cgatcttgaa agtgaaatta cttgacatgg gtgagccgag agctctgctg
1681 gccactgccc tttccccgcc tggtctgagt gacattgagc gcaccatcct tctactaaag
1741 gaggttggag cacttgcagt gagtgggcag agagaagatg aaaaccccca tgatggtgaa
1801 ttgaccttct taggaagagt tttagcccaa cttcctgtaa atcagcaact tggtaaactc
1861 atagtccttg gacatgtatt tggatgtcta gatgaatgtc ttattatagc ggcagctctt
1921 tctttgaaga atttttttgc aatgcctttc cggcagcatc tcgatggata taggaacaaa
1981 gtgaatttct ctggcagtag caagagtgac tgtattgcac ttgttgaggc atttaaaaca
2041 tggaaggctt gcagacagac aggggagctg cggtacccga aggatgaact taattgggga
2101 cggttaaatt acattcaaat caagagaatt agagaggtgg ctgaattata tgaagaattg
2161 aagactagaa tctcacagtt caacatgcat gttgattctc ggcgacctgt catggaccaa
2221 gagtatatat ataagcagcg attcatccta caggttgtat tggcaggtgc tttctatcca
2281 aattacttta cttttggaca gccggatgag gagatggcgg tgagggagct ggctggcaag
2341 gaccccaaga caactgtcgt gttgaaacac attcctccct atggatttct ttactataaa
2401 caactacagt ctctctttag acagtgtggt caagtcaaat ccattgtatt tgatggtgca
2461 aaagcctttg tggaattctc acgaaatcca acagagagat ttaaaaccct tcctgcagta
2521 tatatggcaa ttaagatgtc tcaactaaaa gtttcacttg aactcagcgt tcattctgca
2581 gaggaaattg aagggaaggt gcaaggcatg aacgtctcaa agctcaggaa cacaagggtg
2641 aatgtggact tccagaagca gacggtagat cctatgcaag tctcctttaa cacatcagac
2701 aggtcccaga cagttacaga tctccttcta actattgatg tcacagaggt ggttgaagtg
2761 ggacactttt ggggatacag gattgatgaa aacaactcag agattctgaa aaagcttact
2821 gctgaaatca accaactgac gctggtgccc ttgcccactc acccacatcc agacttggtc
2881 tgtctggcac cttttgctga ttttgataaa caacgctact ttagagctca agtcctttat
2941 gtttctggaa attctgctga ggtattcttt gtagattatg gcaataagtc tcatgtagat
3001 ctacatcttt tgatggagat tccctgtcaa tttcttgaac ttcctttcca ggctttggaa
3061 tttaagattt gcaaaatgag accatcagca aagtctcttg tttgtggcaa gcactggagt
3121 gacggggcca gccagtggtt cgcctctctg gtgagcggct gcaccctcct tgtgaaggtc
3181 ttctctgtgg tgcacagcgt cctgcacgtg gatgtgtacc agtactcagg ggtccaggat
3241 gccatcaaca taagagacgt cctcatccag cagggctatg ccgagctcac ggaggagtcc
3301 tacgagtcca agcaaagcca tgaagttctc aagggcctct tttccaagtc agtagaaaac
3361 atgacagatg gctctgtgcc ctttcccatg aaagacgacg agaaatatct catccggatt
3421 ttgttagaga gcttttctac caataaactg ggtactccaa actgtaaggc agaacttcac
3481 gggcctttta acccttatga actaaagtgc catagtttga ccagaatatc caaattcagg
3541 tgtgtttgga ttgagaagga gagcatcaac tctgtcatta tcagtgacgc ccctgaagac
3601 cttcaccaga gaatgctggt tgcagcttcc ctttccatca atgcgactgg atctacgatg
3661 ctgctgagag aaacctctct gatgcctcat atccctggcc tcccggctct cctcagcatg
3721 ttattcgcac cggtgataga gttaaggatt gatcagaatg gcaagtacta tactggagtc
3781 ctttgtggtt tggggtggaa tccagctaca ggggcttcca tactgcccga gcacgacatg
3841 gagcttgcgt ttgacgttca attcagcgtg gaggatgtcg tcgaggttaa tattctcagg
3901 gctgctatta acaagctagt ctgtgatgga ccaaatggat gcaagtgtct tgggccagag
3961 agagttgcgc agcttcaaga cattgcccgt cagaagcttt taggtttgtt ctgtcagtca
 
4021 aaaccaaggg agaagattgt tcccaagtgg catgaaaagc cctacgagtg gaatcaggtt
4081 gatccaaagc tggtcatgga gcaggccgac cgtgagagca gcagagggaa gaacaccttt
4141 ctctaccagc tccacaaact ggttgtgctc ggcacctgag catgtccaca ggtggcctcc
4201 agcacacccc tcaggaagct gtggaggctg gattccaggc tccctccgca gactgacttt
4261 cctctgtgtc tgggtgttac agtctgtgcc cactgcatcc taaaggcctt ttctttcttc
4321 ttttctcttt gggtgatagt cagagagtgg tgtttttgtt caggtgggaa ggattggaaa
4381 ctctagtctt ttctagaaac agaaaatcac tgtattaaat attttggaaa gattgttctg
4441 aaagaagtct gtttggataa agagctgtat tttgctttaa atttattaag gtaaatataa
4501 gtagttaatc ttagatgtaa ggttccagaa tgtgcttaca tattctgttc tgttacagtg
4561 atttaaacca gtagtatagg aaaaaactta aaaaacaaaa aaaccatgta gtattttctg
4621 attttttttt ccatgaggga aaatatctaa tttttataag actaagttga gttatacttc
4681 ttggttcaca ttttggaaat cagagattac agattacatg gccatagctt atctgtgtta
4741 aaacaataaa agcattaaat gaaaaaaaaa aaaaaaaaaa aa
**ITGA7 (SEQ ID NO: 9)** - Homo sapiens integrin subunit alpha 7 (ITGA7), transcript variant 2, mRNA - NM 002206 
1 gggcgccgga gctgcggctg ctgtagttgt cctagccggt gctggggcgg cggggtggcg
61 gagcggcggg cgggcgggag ggctggcggg gcgaacgtct gggagacgtc tgaaagacca
121 acgagacttt ggagaccaga gacgcgcctg gggggacctg gggcttgggg cgtgcgagat
181 ttcccttgca ttcgctggga gctcgcgcag ggatcgtccc atggccgggg ctcggagccg
241 cgacccttgg ggggcctccg ggatttgcta cctttttggc tccctgctcg tcgaactgct
301 cttctcacgg gctgtcgcct tcaatctgga cgtgatgggt gccttgcgca aggagggcga
361 gccaggcagc ctcttcggct tctctgtggc cctgcaccgg cagttgcagc cccgacccca
421 gagctggctg ctggtgggtg ctccccaggc cctggctctt cctgggcagc aggcgaatcg
481 cactggaggc ctcttcgctt gcccgttgag cctggaggag actgactgct acagagtgga
541 catcgaccag ggagctgata tgcaaaagga aagcaaggag aaccagtggt tgggagtcag
601 tgttcggagc caggggcctg ggggcaagat tgttacctgt gcacaccgat atgaggcaag
661 gcagcgagtg gaccagatcc tggagacgcg ggatatgatt ggtcgctgct ttgtgctcag
721 ccaggacctg gccatccggg atgagttgga tggtggggaa tggaagttct gtgagggacg
781 cccccaaggc catgaacaat ttgggttctg ccagcagggc acagctgccg ccttctcccc
841 tgatagccac tacctcctct ttggggcccc aggaacctat aattggaagg ggttgctttt
901 tgtgaccaac attgatagct cagaccccga ccagctggtg tataaaactt tggaccctgc
961 tgaccggctc ccaggaccag ccggagactt ggccctcaat agctacttag gcttctctat
1021 tgactcgggg aaaggtctgg tgcgtgcaga agagctgagc tttgtggctg gagccccccg
1081 cgccaaccac aagggtgctg tggtcatcct gcgcaaggac agcgccagtc gcctggtgcc
1141 cgaggttatg ctgtctgggg agcgcctgac ctccggcttt ggctactcac tggctgtggc
1201 tgacctcaac agtgatggct ggccagacct gatagtgggt gccccctact tctttgagcg
1261 ccaagaagag ctggggggtg ctgtgtatgt gtacttgaac caggggggtc actgggctgg
1321 gatctcccct ctccggctct gcggctcccc tgactccatg ttcgggatca gcctggctgt
1381 cctgggggac ctcaaccaag atggctttcc agatattgca gtgggtgccc cctttgatgg
1441 tgatgggaaa gtcttcatct accatgggag cagcctgggg gttgtcgcca aaccttcaca
1501 ggtgctggag ggcgaggctg tgggcatcaa gagcttcggc tactccctgt caggcagctt
1561 ggatatggat gggaaccaat accctgacct gctggtgggc tccctggctg acaccgcagt
1621 gctcttcagg gccagaccca tcctccatgt ctcccatgag gtctctattg ctccacgaag
1681 catcgacctg gagcagccca actgtgctgg cggccactcg gtctgtgtgg acctaagggt
1741 ctgtttcagc tacattgcag tccccagcag ctatagccct actgtggccc tggactatgt
1801 gttagatgcg gacacagacc ggaggctccg gggccaggtt ccccgtgtga cgttcctgag
1861 ccgtaacctg gaagaaccca agcaccaggc ctcgggcacc gtgtggctga agcaccagca
1921 tgaccgagtc tgtggagacg ccatgttcca gctccaggaa aatgtcaaag acaagcttcg
1981 ggccattgta gtgaccttgt cctacagtct ccagacccct cggctccggc gacaggctcc
2041 tggccagggg ctgcctccag tggcccccat cctcaatgcc caccagccca gcacccagcg
2101 ggcagagatc cacttcctga agcaaggctg tggtgaagac aagatctgcc agagcaatct
2161 gcagctggtc cgcgcccgct tctgtacccg ggtcagcgac acggaattcc aacctctgcc
2221 catggatgtg gatggaacaa cagccctgtt tgcactgagt gggcagccag tcattggcct
2281 ggagctgatg gtcaccaacc tgccatcgga cccagcccag ccccaggctg atggggatga
 
2341 tgcccatgaa gcccagctcc tggtcatgct tcctgactca ctgcactact caggggtccg
2401 ggccctggac cctgcggaga agccactctg cctgtccaat gagaatgcct cccatgttga
2461 gtgtgagctg ggaaccccca tgaagagagg tgcccaggtc accttctacc tcatccttag
2521 cacctccggg atcagcattg agaccacgga actggaggta gagctgctgt tggccacgat
2581 cagtgagcag gagctgcatc cagtctctgc acgagcccgt gtcttcattg agctgccact
2641 gtccattgca ggaatggcca ttccccagca actcttcttc tctggtgtgg tgagggcgga
2701 gagagccatg cagtctgagc gggatgtggg cagcaaggtc aagtatgagg tcacggtttc
2761 caaccaaggc cagtcgctca gaaccctggg ctctgccttc ctcaacatca tgtggcctca
2821 tgagattgcc aatgggaagt ggttgctgta cccaatgcag gttgagctgg agggcgggca
2881 ggggcctggg cagaaagggc tttgctctcc caggcccaac atcctccacc tggatgtgga
2941 cagtagggat aggaggcggc gggagctgga gccacctgag cagcaggagc ctggtgagcg
3001 gcaggagccc agcatgtcct ggtggccagt gtcctctgct gagaagaaga aaaacatcac
3061 cctggactgc gcccggggca cggccaactg tgtggtgttc agctgcccac tctacagctt
3121 tgaccgcgcg gctgtgctgc atgtctgggg ccgtctctgg aacagcacct ttctggagga
3181 gtactcagct gtgaagtccc tggaagtgat tgtccgggcc aacatcacag tgaagtcctc
3241 cataaagaac ttgatgctcc gagatgcctc cacagtgatc ccagtgatgg tatacttgga
3301 ccccatggct gtggtggcag aaggagtgcc ctggtgggtc atcctcctgg ctgtactggc
3361 tgggctgctg gtgctagcac tgctggtgct gctcctgtgg aagatgggat tcttcaaacg
3421 ggcgaagcac cccgaggcca ccgtgcccca gtaccatgcg gtgaagattc ctcgggaaga
3481 ccgacagcag ttcaaggagg agaagacggg caccatcctg aggaacaact ggggcagccc
3541 ccggcgggag ggcccggatg cacaccccat cctggctgct gacgggcatc ccgagctggg
3601 ccccgatggg catccagggc caggcaccgc ctaggttccc atgtcccagc ctggcctgtg
3661 gctgccctcc atcccttccc cagagatggc tccttgggat gaagagggta gagtgggctg
3721 ctggtgtcgc atcaagattt ggcaggatcg gcttcctcag gggcacagac ctctcccacc
3781 cacaagaact cctcccaccc aacttcccct tagagtgctg tgagatgaga gtgggtaaat
3841 cagggacagg gccatggggt agggtgagaa gggcaggggt gtcctgatgc aaaggtgggg
3901 agaaggatcc ctaatccctt cctctcccat tcaccctgtg taacaggacc ccaaggacct
3961 gcctccccgg aagtgcctta acctagaggg tcggggagga ggttgtgtca ctgactcagg
4021 ctgctccttc tctagtttcc cctctcatct gaccttagtt tgctgccatc agtctagtgg
4081 tttcgtggtt tcgtctattt attaaaaaat atttgagaac aaaaaaaaaa aaaaaaaa
**BMX (SEQ ID NO: 10)** - Homo sapiens BMX non-receptor tyrosine kinase (BMX), transcript variant 2, mRNA - NM 001721 
1 gggaatatga gtgatggtgc ctcaaagcag taactttttg cttagagctt gagagtcaaa
61 gttaaggacc cacatgtata cttcggctct agcgagtcta aggatgataa tatggataca
121 aaatctattc tagaagaact tcttctcaaa agatcacagc aaaagaagaa aatgtcacca
181 aataattaca aagaacggct ttttgttttg accaaaacaa acctttccta ctatgaatat
241 gacaaaatga aaaggggcag cagaaaagga tccattgaaa ttaagaaaat cagatgtgtg
301 gagaaagtaa atctcgagga gcagacgcct gtagagagac agtacccatt tcagattgtc
361 tataaagatg ggcttctcta tgtctatgca tcaaatgaag agagccgaag tcagtggttg
421 aaagcattac aaaaagagat aaggggtaac ccccacctgc tggtcaagta ccatagtggg
481 ttcttcgtgg acgggaagtt cctgtgttgc cagcagagct gtaaagcagc cccaggatgt
541 accctctggg aagcatatgc taatctgcat actgcagtca atgaagagaa acacagagtt
601 cccaccttcc cagacagagt gctgaagata cctcgggcag ttcctgttct caaaatggat
661 gcaccatctt caagtaccac tctagcccaa tatgacaacg aatcaaagaa aaactatggc
721 tcccagccac catcttcaag taccagtcta gcgcaatatg acagcaactc aaagaaaatc
781 tatggctccc agccaaactt caacatgcag tatattccaa gggaagactt ccctgactgg
841 tggcaagtaa gaaaactgaa aagtagcagc agcagtgaag atgttgcaag cagtaaccaa
901 aaagaaagaa atgtgaatca caccacctca aagatttcat gggaattccc tgagtcaagt
961 tcatctgaag aagaggaaaa cctggatgat tatgactggt ttgctggtaa catctccaga
1021 tcacaatctg aacagttact cagacaaaag ggaaaagaag gagcatttat ggttagaaat
1081 tcgagccaag tgggaatgta cacagtgtcc ttatttagta aggctgtgaa tgataaaaaa
1141 ggaactgtca aacattacca cgtgcataca aatgctgaga acaaattata cctggcagaa
1201 aactactgtt ttgattccat tccaaagctt attcattatc atcaacacaa ttcagcaggc
1261 atgatcacac ggctccgcca ccctgtgtca acaaaggcca acaaggtccc cgactctgtg
 
1321 tccctgggaa atggaatctg ggaactgaaa agagaagaga ttaccttgtt gaaggagctg
1381 ggaagtggcc agtttggagt ggtccagctg ggcaagtgga aggggcagta tgatgttgct
1441 gttaagatga tcaaggaggg ctccatgtca gaagatgaat tctttcagga ggcccagact
1501 atgatgaaac tcagccatcc caagctggtt aaattctatg gagtgtgttc aaaggaatac
1561 cccatataca tagtgactga atatataagc aatggctgct tgctgaatta cctgaggagt
1621 cacggaaaag gacttgaacc ttcccagctc ttagaaatgt gctacgatgt ctgtgaaggc
1681 atggccttct tggagagtca ccaattcata caccgggact tggctgctcg taactgcttg
1741 gtggacagag atctctgtgt gaaagtatct gactttggaa tgacaaggta tgttcttgat
1801 gaccagtatg tcagttcagt cggaacaaag tttccagtca agtggtcagc tccagaggtg
1861 tttcattact tcaaatacag cagcaagtca gacgtatggg catttgggat cctgatgtgg
1921 gaggtgttca gcctggggaa gcagccctat gacttgtatg acaactccca ggtggttctg
1981 aaggtctccc agggccacag gctttaccgg ccccacctgg catcggacac catctaccag
2041 atcatgtaca gctgctggca cgagcttcca gaaaagcgtc ccacatttca gcaactcctg
2101 tcttccattg aaccacttcg ggaaaaagac aagcattgaa gaagaaatta ggagtgctga
2161 taagaatgaa tatagatgct ggccagcatt ttcattcatt ttaaggaaag tagcaaggca
2221 taatgtaatt tagctagttt ttaatagtgt tctctgtatt gtctattatt tagaaatgaa
2281 caaggcagga aacaaaagat tcccttgaaa tttagatcaa attagtaatt ttgtttatgc
2341 tgctcctgat ataacacttt ccagcctata gcagaagcac attttcagac tgcaatatag
2401 agactgtgtt catgtgtaaa gactgagcag aactgaaaaa ttacttattg gatattcatt
2461 cttttcttta tattgtcatt gtcacaacaa ttaaatatac taccaagtac agaaatgtgg
2521 aaaaaaaaaa
**HP (SEQ ID NO: 11)** - Homo sapiens haptoglobin (HP), transcript variant 1, mRNA - NM 005143 
1 agcataaaaa gaccagcaga tgccccacag cactgctctt ccagaggcaa gaccaaccaa
61 gatgagtgcc ctgggagctg tcattgccct cctgctctgg ggacagcttt ttgcagtgga
121 ctcaggcaat gatgtcacgg atatcgcaga tgacggctgc ccgaagcccc ccgagattgc
181 acatggctat gtggagcact cggttcgcta ccagtgtaag aactactaca aactgcgcac
241 agaaggagat ggagtataca ccttaaatga taagaagcag tggataaata aggctgttgg
301 agataaactt cctgaatgtg aagcagatga cggctgcccg aagccccccg agattgcaca
361 tggctatgtg gagcactcgg ttcgctacca gtgtaagaac tactacaaac tgcgcacaga
421 aggagatgga gtgtacacct taaacaatga gaagcagtgg ataaataagg ctgttggaga
481 taaacttcct gaatgtgaag cagtatgtgg gaagcccaag aatccggcaa acccagtgca
541 gcggatcctg ggtggacacc tggatgccaa aggcagcttt ccctggcagg ctaagatggt
601 ttcccaccat aatctcacca caggtgccac gctgatcaat gaacaatggc tgctgaccac
661 ggctaaaaat ctcttcctga accattcaga aaatgcaaca gcgaaagaca ttgcccctac
721 tttaacactc tatgtgggga aaaagcagct tgtagagatt gagaaggttg ttctacaccc
781 taactactcc caggtagata ttgggctcat caaactcaaa cagaaggtgt ctgttaatga
841 gagagtgatg cccatctgcc taccttcaaa ggattatgca gaagtagggc gtgtgggtta
901 tgtttctggc tgggggcgaa atgccaattt taaatttact gaccatctga agtatgtcat
961 gctgcctgtg gctgaccaag accaatgcat aaggcattat gaaggcagca cagtccccga
1021 aaagaagaca ccgaagagcc ctgtaggggt gcagcccata ctgaatgaac acaccttctg
1081 tgctggcatg tctaagtacc aagaagacac ctgctatggc gatgcgggca gtgcctttgc
1141 cgttcacgac ctggaggagg acacctggta tgcgactggg atcttaagct ttgataagag
1201 ctgtgctgtg gctgagtatg gtgtgtatgt gaaggtgact tccatccagg actgggttca
1261 gaagaccata gctgagaact aatgcaaggc tggccggaag cccttgcctg aaagcaagat
1321 ttcagcctgg aagagggcaa agtggacggg agtggacagg agtggatgcg ataagatgtg
1381 gtttgaagct gatgggtgcc agccctgcat tgctgagtca atcaataaag agctttcttt
1441 tgacccataa aaaaaaaaaa aaaaaaaaaa aaaaaaaa
**IGFBP2 (SEQ ID NO: 12)** - Homo sapiens insulin like growth factor binding protein 2, 36kDa (IGFBP2), transcript variant 1, mRNA - NM 000597 
1 tgcggcggcg agggaggagg aagaagcgga ggaggcggct cccgcgctcg cagggccgtg
61 ccacctgccc gcccgcccgc tcgctcgctc gcccgccgcg ccgcgctgcc gaccgccagc
121 atgctgccga gagtgggctg ccccgcgctg ccgctgccgc cgccgccgct gctgccgctg
 
181 ctgccgctgc tgctgctgct actgggcgcg agtggcggcg gcggcggggc gcgcgcggag
241 gtgctgttcc gctgcccgcc ctgcacaccc gagcgcctgg ccgcctgcgg gcccccgccg
301 gttgcgccgc ccgccgcggt ggccgcagtg gccggaggcg cccgcatgcc atgcgcggag
361 ctcgtccggg agccgggctg cggctgctgc tcggtgtgcg cccggctgga gggcgaggcg
421 tgcggcgtct acaccccgcg ctgcggccag gggctgcgct gctatcccca cccgggctcc
481 gagctgcccc tgcaggcgct ggtcatgggc gagggcactt gtgagaagcg ccgggacgcc
541 gagtatggcg ccagcccgga gcaggttgca gacaatggcg atgaccactc agaaggaggc
601 ctggtggaga accacgtgga cagcaccatg aacatgttgg gcgggggagg cagtgctggc
661 cggaagcccc tcaagtcggg tatgaaggag ctggccgtgt tccgggagaa ggtcactgag
721 cagcaccggc agatgggcaa gggtggcaag catcaccttg gcctggagga gcccaagaag
781 ctgcgaccac cccctgccag gactccctgc caacaggaac tggaccaggt cctggagcgg
841 atctccacca tgcgccttcc ggatgagcgg ggccctctgg agcacctcta ctccctgcac
901 atccccaact gtgacaagca tggcctgtac aacctcaaac agtgcaagat gtctctgaac
961 gggcagcgtg gggagtgctg gtgtgtgaac cccaacaccg ggaagctgat ccagggagcc
1021 cccaccatcc ggggggaccc cgagtgtcat ctcttctaca atgagcagca ggaggctcgc
1081 ggggtgcaca cccagcggat gcagtagacc gcagccagcc ggtgcctggc gcccctgccc
1141 cccgcccctc tccaaacacc ggcagaaaac ggagagtgct tgggtggtgg gtgctggagg
1201 attttccagt tctgacacac gtatttatat ttggaaagag accagcaccg agctcggcac
1261 ctccccggcc tctctcttcc cagctgcaga tgccacacct gctccttctt gctttccccg
1321 ggggaggaag ggggttgtgg tcggggagct ggggtacagg tttggggagg gggaagagaa
1381 atttttattt ttgaacccct gtgtcccttt tgcataagat taaaggaagg aaaagtaaa
**ALPL (SEQ ID NO: 13)** - Homo sapiens alkaline phosphatase, liver/bone/kidney (ALPL),transcript variant 1, mRNA - NM 000478 
1 gggctgcccg ggcctcactc gggccccgcg gccgccttta taaggcggcg ggggtggtgg
61 cccgggccgc gttgcgctcc cgccactccg cgcccgctat cctggctccg tgctcccacg
121 cgcttgtgcc tggacggacc ctcgccagtg ctctgcgcag gattggaaca tcagttaaca
181 tctgaccact gccagcccac cccctcccac ccacgtcgat tgcatctctg ggctccaggg
241 ataaagcagg tcttggggtg caccatgatt tcaccattct tagtactggc cattggcacc
301 tgccttacta actccttagt gccagagaaa gagaaagacc ccaagtactg gcgagaccaa
361 gcgcaagaga cactgaaata tgccctggag cttcagaagc tcaacaccaa cgtggctaag
421 aatgtcatca tgttcctggg agatgggatg ggtgtctcca cagtgacggc tgcccgcatc
481 ctcaagggtc agctccacca caaccctggg gaggagacca ggctggagat ggacaagttc
541 cccttcgtgg ccctctccaa gacgtacaac accaatgccc aggtccctga cagcgccggc
601 accgccaccg cctacctgtg tggggtgaag gccaatgagg gcaccgtggg ggtaagcgca
661 gccactgagc gttcccggtg caacaccacc caggggaacg aggtcacctc catcctgcgc
721 tgggccaagg acgctgggaa atctgtgggc attgtgacca ccacgagagt gaaccatgcc
781 acccccagcg ccgcctacgc ccactcggct gaccgggact ggtactcaga caacgagatg
841 ccccctgagg ccttgagcca gggctgtaag gacatcgcct accagctcat gcataacatc
901 agggacattg acgtgatcat ggggggtggc cggaaataca tgtaccccaa gaataaaact
961 gatgtggagt atgagagtga cgagaaagcc aggggcacga ggctggacgg cctggacctc
1021 gttgacacct ggaagagctt caaaccgaga tacaagcact cccacttcat ctggaaccgc
1081 acggaactcc tgacccttga cccccacaat gtggactacc tattgggtct cttcgagcca
1141 ggggacatgc agtacgagct gaacaggaac aacgtgacgg acccgtcact ctccgagatg
1201 gtggtggtgg ccatccagat cctgcggaag aaccccaaag gcttcttctt gctggtggaa
1261 ggaggcagaa ttgaccacgg gcaccatgaa ggaaaagcca agcaggccct gcatgaggcg
1321 gtggagatgg accgggccat cgggcaggca ggcagcttga cctcctcgga agacactctg
1381 accgtggtca ctgcggacca ttcccacgtc ttcacatttg gtggatacac cccccgtggc
1441 aactctatct ttggtctggc ccccatgctg agtgacacag acaagaagcc cttcactgcc
1501 atcctgtatg gcaatgggcc tggctacaag gtggtgggcg gtgaacgaga gaatgtctcc
1561 atggtggact atgctcacaa caactaccag gcgcagtctg ctgtgcccct gcgccacgag
1621 acccacggcg gggaggacgt ggccgtcttc tccaagggcc ccatggcgca cctgctgcac
1681 ggcgtccacg agcagaacta cgtcccccac gtgatggcgt atgcagcctg catcggggcc
1741 aacctcggcc actgtgctcc tgccagctcg gcaggcagcc ttgctgcagg ccccctgctg
1801 ctcgcgctgg ccctctaccc cctgagcgtc ctgttctgag ggcccagggc ccgggcaccc
 
1861 acaagcccgt gacagatgcc aacttcccac acggcagccc ccccctcaag gggcagggag
1921 gtgggggcct cctcagcctc tgcaactgca agaaagggga cccaagaaac caaagtctgc
1981 cgcccacctc gctcccctct ggaatcttcc ccaagggcca aacccacttc tggcctccag
2041 cctttgctcc ctccccgctg ccctttggcc aacagggtag atttctcttg ggcaggcaga
2101 gagtacagac tgcagacatt ctcaaagcct cttatttttc tagcgaacgt atttctccag
2161 acccagaggc cctgaagcct ccgtggaaca ttctggatct gaccctccca gtctcatctc
2221 ctgaccctcc cactcccatc tccttacctc tggaaccccc caggccctac aatgctcatg
2281 tccctgtccc caggcccagc cctccttcag gggagttgag gtctttctcc tcaggacaag
2341 gccttgctca ctcactcact ccaagaccac cagggtccca ggaagccggt gcctgggtgg
2401 ccatcctacc cagcgtggcc caggccggga agagccacct ggcagggctc acactcctgg
2461 gctctgaaca cacacgccag ctcctctctg aagcgactct cctgtttgga acggcaaaaa
2521 aaaatttttt tttctctttt tggtggtggt taaaagggaa cacaaaacat ttaaataaaa
2581 ctttccaaat atttccgagg acaaaaaaaa aaa
**DACH1 (SEQ ID NO: 14)** - Homo sapiens dachshund family transcription factor 1 (DACH1), transcript variant 1, mRNA - NM_005143 
1 atctttgatc aatgtacttg ccagggagag cccaagtcct tcaaacctcc tccttttcac
61 cttcatcctt aactttgtgc tagagcgaga cccacacaac aacagccgac cctccccgcc
121 ccacccccac ccccaaacca gccctcgatc ccagcccccg gagaggactc gcatttcgac
181 ttgcgggaca cttttgtgcg ttcctctcca gagcgcctct cgtgctcgcc cctcttgcgc
241 tcgctcttta ttaccttcac ctccttttct cccccttctc tccctttctc cttctcgttc
301 tctcccggag ttgttgttgc ccccctcgct ccttctcccc ccttttttcc ccttcccctc
361 ccgggggtgt gtggcaactt ttcctctcgc ttctcctccg tctgtttccc cttatatgtg
421 accatggcag tgccggcggc tttgatccct ccgacccagc tggtcccccc tcaaccccca
481 atctccacgt ctgcttcctc ctctggcacc accacctcca cctcttcggc gacttcgtct
541 ccggctcctt ccatcggacc cccggcgtcc tctgggccaa ctctgttccg cccggagccc
601 atcgcttcgg cggcggcggc ggcggccaca gtcacctcta ccggcggcgg cggcggcggc
661 ggcggcagcg gaggcggcgg cggcagcagc ggcaacggag gcggcggtgg cggcggcggc
721 ggtggcagca actgcaaccc caacctggcg gccgcgagca acggcagcgg cggcggcggc
781 ggcggcatca gcgctggcgg cggcgtcgct tccagcaccc ccatcaacgc cagcaccggc
841 agcagcagca gcagcagtag cagcagcagc agcagcagca gtagtagcag cagcagcagt
901 agcagcagca gctgcggccc cctccccggg aaacccgtgt actcaacccc gtccccagtg
961 gaaaacaccc ctcagaataa tgagtgcaaa atggtggatc tgaggggggc caaagtggct
1021 tccttcacgg tggagggctg cgagctgatc tgcctgcccc aggctttcga cctgttcctg
1081 aagcacttgg tggggggctt gcatacggtc tacaccaagc tgaagcggct ggagatcacg
1141 ccggtggtgt gcaatgtgga acaagttcgc atcctgaggg gactgggcgc catccagcca
1201 ggagtgaacc gctgcaaact catctccagg aaggacttcg agaccctcta caatgactgc
1261 accaacgcaa gttctagacc tggaaggcct cctaagagga ctcaaagtgt cacctcccca
1321 gagaactctc acatcatgcc gcattctgtc cctggtctca tgtctcctgg gataattcca
1381 ccaacaggtc tgacagcagc cgctgcagca gctgctgctg ctaccaatgc agctattgct
1441 gaagcaatga aggtgaaaaa aatcaaatta gaagccatga gcaactatca tgccagtaat
1501 aaccaacatg gagcagactc tgaaaacggg gacatgaatt caagtgtcgg actggaactt
1561 ccttttatga tgatgcccca ccctctaatt cctgtcagcc tacctccagc atctgtcacc
1621 atggcaatga gccagatgaa ccacctcagc accattgcaa atatggcagc agcagcacaa
1681 gttcagagtc ccccatccag agttgagaca tcagttatta aggagcgtgt tcctgatagc
1741 ccctcacctg ccccctctct ggaggagggg agaaggcctg gcagtcaccc atcatcacat
1801 cgcagcagca gcgtgtccag ctcccctgct cggactgaga gctcttctga cagaatcccg
1861 gtccatcaga atgggttgtc catgaaccag atgctgatgg gcttatcacc aaatgtactt
1921 cctgggccca aagagggaga tttggccggt catgacatgg gacatgagtc aaaaaggatg
1981 catattgaaa aagatgagac cccgctttct acaccaaccg caagagacag ccttgacaaa
2041 ctctctctaa ctgggcatgg acaaccactg cctccaggtt ttccatctcc ttttctgttt
2101 cctgatggac tgtcttccat cgagactctt ctgactaaca tacaggggct gttgaaagtt
2161 gccatagata atgccagagc tcaagagaaa caggtccaac tggaaaaaac tgagctgaag
2221 atggattttt taagggaaag agaactaagg gaaacacttg agaagcagtt ggctatggaa
2281 caaaagaata gagccatagt tcaaaagagg ctaaagaagg agaagaaggc aaagagaaaa
 
2341 ttgcaggaag cacttgagtt tgagacgaaa cggcgtgaac aagcagaaca gacgctaaaa
2401 caggcagctt caacagatag tctcagggtc ttaaatgact ctctgacccc agagatagag
2461 gctgaccgca gtggcggcag aacagatgct gaaaggacaa tacaagatgg aagactgtat
2521 ttgaaaacta ctgtcatgta ctgaatcttt cctgttgaag aaatccatgt tatagaaaag
2581 aactttgcag tcagacattc gtcatgggaa agttcagaaa aaaataaagt ccttttaagg
2641 gaacttcctg aattttgtgt attaatgttc tttaaaagtt taagtattct acaaaaaaaa
2701 aaaaagtttt ctccattgat tttcacctgt ggttcatacc agagacctga gaatgtttgt
2761 aaatgtacaa gtatcaaagt tcttacagtt aattactgca acttgctgct ggacaattgt
2821 atacagagtt aaaggcaggt ctgaataaga cctagctttg tttttttcta atggaatgaa
2881 ccattttcct cttctgaaaa ttctgtatct gagcacatca agagactctt gtagcagtgg
2941 ttacccagac ttacagaatt atgtcctcca gaaaccagca agaacacttg gaatgaacga
3001 atgaacttgt agggggcata gaggattctt gaaaaaaaaa aatgcaagag tgattttctg
3061 ttacattcaa tttcaaactc tctaattgtg ggttttctcc tgaagaattt tttttcacat
3121 actttccaaa agaccaacaa atggatgttg acaacaaccc aatgaaataa cattttgcat
3181 atctgaaaag aagcattgaa tataagccaa aagctttcac tgaaggtttt tttttcttaa
3241 aaataaaaaa aaatatataa gtgtaacatg ttttcattcc aaactggtag tggtatatag
3301 aattaaagat aataatgttg cttcttattc aaactgttgg tcatatgtac agtatataaa
3361 cataaaacac acaaggaagg tattatgtat gcagtagtat actagagttt aggaaaatga
3421 aaattttaga aaatatgttt tgtcaccctg ttggtcagaa agatgtcttt ctggttttaa
3481 cgcatgcagg catgtaaata tttgtctgga gtcacagtat taatgaatga gatcttaagc
3541 atctggtgac atcagaactc tgtgtcagcc acttttattt gtatattgaa ccctagctag
3601 tgccccaagc tgcactattg ggaatggatt gtggctgaac agcaaatcaa aacaccagaa
3661 atatttttat atgttaacgt catattatgt taatgttgct gaaaacaaaa cctaacaaac
3721 cttgatgtac cagtccaata ccatgtagcg ctgagtgata aagttaaaat gtgctgtgct
3781 tcccaccctt gtcagaggga agggtggcta tgtgttattt tcactgtctt tttgaaagtt
3841 acagtatgtg ttttcacttt cgtgcagata actggaagta aagcggcaaa cagtgcttat
3901 tacatgctaa agttaccttc tctttgtttt ttgcatatct ggaattacac ctttaaagac
3961 tgatatgaat cagtacggtc actatacatt ttatgatttt tctgtcatct taaaattgta
4021 tgatcgtaac attatttatt accacaaaac agcaaaatct tcaatgtcta agaaaactag
4081 cttaaaatgt ttaaatatag ttctgattgg gtattaatta cttgattaag aaaaaattaa
4141 cattatagat actctggcat tacgcttcta taccttttag gtcttccttg caatactgga
4201 acataattct tttgtgtagc tcactattag ccagctaagt tcatcttttt aataccataa
4261 aaaggttata tgtacagttc ctattttagc ttgcttacaa agggagcatt atttttattt
4321 aaagtattgc tagtaaatga tttgtagaaa cttggttttc taagcatagt tcttccataa
4381 ccaccttttg ttgtttgagc acaagggatt cttttcctag ttctatgtgt ttgtttccct
4441 atatgcagtc tttaaaggat tacaacactt aaaattgaat ggacttgtgt caagcttttt
4501 gcatcataca ttttttgaaa gatttttaaa aaagcctaca acttacatat gtagtagaat
4561 cagccattgc tctgctcctg gcatagagtc acctgtttgt tatgtggatt aaatagtttt
4621 aaaatacata tttgaagacc tttgagaatg ctttagtgtt tgatttgaaa taaaaggaaa
4681 ttttagcaag gattaaagaa aaaagctatc agctgtatgt taagagagac tcttactaac
4741 atgttgtaaa tattacaatt catgaaatgt tattgtaagt ctgtaactta attttttccc
4801 tgttttagtt atacaggttg gtttggaaat ttgtgttttg gcataaacaa gtaaaatgtg
4861 cccattttat ggtttccatg cttttgtaat cctaaaaata ttaatgtcta gttgttctat
4921 attataacca catttgcgct ctatgcaagc ccttggaaca gaacatactc atcttcatgt
4981 aggacctatg aaaattgtct atttttatct atatatttaa agttttctaa aaatgataaa
5041 aggttattac gaattttgtt gtacaaaatc tgtacaaaaa tctgttttta catcataatg
5101 caagaattgg aaatttttct atggtagcct agttatttga gcctggtttc aatgtgagaa
5161 ccacgtttac tgttattgta tttaattttc ttttcctttt caacaatctc ctaataaaac
5221 tgtctgaaat ctcaaaaaaa
**IL1R1 (SEQ ID NO: 15)** - Homo sapiens interleukin 1 receptor type 1 (IL1R1), transcript variant 1, mRNA - NM 000877 
1 gtggccggcg gccggagccg actcggagcg cgcggcgccg gccgggagga gccggagagc
61 ggccgggccg ggcggtgggg gcgccggcct gccccgcgcg ccccagggag cggcaggaat
121 gtgacaatcg cgcgcccgcg caccgaagca ctcctcgctc ggctcctagg gctctcgccc
 
181 ctctgagctg agccgggttc cgcccggggc tgggatccca tcaccctcca cggccgtccg
241 tccaggtaga cgcaccctct gaagatggtg actccctcct gagaagctgg accccttggt
301 aaaagacaag gccttctcca agaagaatat gaaagtgtta ctcagactta tttgtttcat
361 agctctactg atttcttctc tggaggctga taaatgcaag gaacgtgaag aaaaaataat
421 tttagtgtca tctgcaaatg aaattgatgt tcgtccctgt cctcttaacc caaatgaaca
481 caaaggcact ataacttggt ataaagatga cagcaagaca cctgtatcta cagaacaagc
541 ctccaggatt catcaacaca aagagaaact ttggtttgtt cctgctaagg tggaggattc
601 aggacattac tattgcgtgg taagaaattc atcttactgc ctcagaatta aaataagtgc
661 aaaatttgtg gagaatgagc ctaacttatg ttataatgca caagccatat ttaagcagaa
721 actacccgtt gcaggagacg gaggacttgt gtgcccttat atggagtttt ttaaaaatga
781 aaataatgag ttacctaaat tacagtggta taaggattgc aaacctctac ttcttgacaa
841 tatacacttt agtggagtca aagataggct catcgtgatg aatgtggctg aaaagcatag
901 agggaactat acttgtcatg catcctacac atacttgggc aagcaatatc ctattacccg
961 ggtaatagaa tttattactc tagaggaaaa caaacccaca aggcctgtga ttgtgagccc
1021 agctaatgag acaatggaag tagacttggg atcccagata caattgatct gtaatgtcac
1081 cggccagttg agtgacattg cttactggaa gtggaatggg tcagtaattg atgaagatga
1141 cccagtgcta ggggaagact attacagtgt ggaaaatcct gcaaacaaaa gaaggagtac
1201 cctcatcaca gtgcttaata tatcggaaat tgaaagtaga ttttataaac atccatttac
1261 ctgttttgcc aagaatacac atggtataga tgcagcatat atccagttaa tatatccagt
1321 cactaatttc cagaagcaca tgattggtat atgtgtcacg ttgacagtca taattgtgtg
1381 ttctgttttc atctataaaa tcttcaagat tgacattgtg ctttggtaca gggattcctg
1441 ctatgatttt ctcccaataa aagcttcaga tggaaagacc tatgacgcat atatactgta
1501 tccaaagact gttggggaag ggtctacctc tgactgtgat atttttgtgt ttaaagtctt
1561 gcctgaggtc ttggaaaaac agtgtggata taagctgttc atttatggaa gggatgacta
1621 cgttggggaa gacattgttg aggtcattaa tgaaaacgta aagaaaagca gaagactgat
1681 tatcatttta gtcagagaaa catcaggctt cagctggctg ggtggttcat ctgaagagca
1741 aatagccatg tataatgctc ttgttcagga tggaattaaa gttgtcctgc ttgagctgga
1801 gaaaatccaa gactatgaga aaatgccaga atcgattaaa ttcattaagc agaaacatgg
1861 ggctatccgc tggtcagggg actttacaca gggaccacag tctgcaaaga caaggttctg
1921 gaagaatgtc aggtaccaca tgccagtcca gcgacggtca ccttcatcta aacaccagtt
1981 actgtcacca gccactaagg agaaactgca aagagaggct cacgtgcctc tcgggtagca
2041 tggagaagtt gccaagagtt ctttaggtgc ctcctgtctt atggcgttgc aggccaggtt
2101 atgcctcatg ctgacttgca gagttcatgg aatgtaacta tatcatcctt tatccctgag
2161 gtcacctgga atcagattat taagggaata agccatgacg tcaatagcag cccagggcac
2221 ttcagagtag agggcttggg aagatctttt aaaaaggcag taggcccggt gtggtggctc
2281 acgcctataa tcccagcact ttgggaggct gaagtgggtg gatcaccaga ggtcaggagt
2341 tcgagaccag cccagccaac atggcaaaac cccatctcta ctaaaaatac aaaaatgagc
2401 taggcatggt ggcacacgcc tgtaatccca gctacacctg aggctgaggc aggagaattg
2461 cttgaaccgg ggagacggag gttgcagtga gccgagtttg ggccactgca ctctagcctg
2521 gcaacagagc aagactccgt ctcaaaaaaa gggcaataaa tgccctctct gaatgtttga
2581 actgccaaga aaaggcatgg agacagcgaa ctagaagaaa gggcaagaag gaaatagcca
2641 ccgtctacag atggcttagt taagtcatcc acagcccaag ggcggggcta tgccttgtct
2701 ggggaccctg tagagtcact gaccctggag cggctctcct gagaggtgct gcaggcaaag
2761 tgagactgac acctcactga ggaagggaga catattcttg gagaactttc catctgcttg
2821 tattttccat acacatcccc agccagaagt tagtgtccga agaccgaatt ttattttaca
2881 gagcttgaaa actcacttca atgaacaaag ggattctcca ggattccaaa gttttgaagt
2941 catcttagct ttccacagga gggagagaac ttaaaaaagc aacagtagca gggaattgat
3001 ccacttctta atgctttcct ccctggcatg accatcctgt cctttgttat tatcctgcat
3061 tttacgtctt tggaggaaca gctccctagt ggcttcctcc gtctgcaatg tcccttgcac
3121 agcccacaca tgaaccatcc ttcccatgat gccgctcttc tgtcatcccg ctcctgctga
3181 aacacctccc aggggctcca cctgttcagg agctgaagcc catgctttcc caccagcatg
3241 tcactcccag accacctccc tgccctgtcc tccagcttcc cctcgctgtc ctgctgtgtg
3301 aattcccagg ttggcctggt ggccatgtcg cctgccccca gcactcctct gtctctgctc
3361 ttgcctgcac ccttcctcct cctttgccta ggaggccttc tcgcattttc tctagctgat
3421 cagaatttta ccaaaattca gaacatcctc caattccaca gtctctggga gactttccct
3481 aagaggcgac ttcctctcca gccttctctc tctggtcagg cccactgcag agatggtggt
 
3541 gagcacatct gggaggctgg tctccctcca gctggaattg ctgctctctg agggagaggc
3601 tgtggtggct gtctctgtcc ctcactgcct tccaggagca atttgcacat gtaacataga
3661 tttatgtaat gctttatgtt taaaaacatt ccccaattat cttatttaat ttttgcaatt
3721 attctaattt tatatataga gaaagtgacc tattttttaa aaaaatcaca ctctaagttc
3781 tattgaacct aggacttgag cctccatttc tggcttctag tctggtgttc tgagtacttg
3841 atttcaggtc aataacggtc ccccctcact ccacactggc acgtttgtga gaagaaatga
3901 cattttgcta ggaagtgacc gagtctagga atgcttttat tcaagacacc aaattccaaa
3961 cttctaaatg ttggaatttt caaaaattgt gtttagattt tatgaaaaac tcttctactt
4021 tcatctattc tttccctaga ggcaaacatt tcttaaaatg tttcattttc attaaaaatg
4081 aaagccaaat ttatatgcca ccgattgcag gacacaagca cagttttaag agttgtatga
4141 acatggagag gacttttggt ttttatattt ctcgtattta atatgggtga acaccaactt
4201 ttatttggaa taataatttt cctcctaaac aaaaacacat tgagtttaag tctctgactc
4261 ttgcctttcc acctgctttc tcctgggccc gctttgcctg cttgaaggaa cagtgctgtt
4321 ctggagctgc tgttccaaca gacagggcct agctttcatt tgacacacag actacagcca
4381 gaagcccatg gagcagggat gtcacgtctt gaaaagccta ttagatgttt tacaaattta
4441 attttgcaga ttattttagt ctgtcatcca gaaaatgtgt cagcatgcat agtgctaaga
4501 aagcaagcca atttggaaac ttaggttagt gacaaaattg gccagagagt gggggtgatg
4561 atgaccaaga attacaagta gaatggcagc tggaatttaa ggagggacaa gaatcaatgg
4621 ataagcgtgg gtggaggaag atccaaacag aaaagtgcaa agttattccc catcttccaa
4681 gggttgaatt ctggaggaag aagacacatt cctagttccc cgtgaacttc ctttgactta
4741 ttgtccccac taaaacaaaa caaaaaactt ttaatgcctt ccacattaat tagattttct
4801 tgcagttttt ttatggcatt tttttaaaga tgccctaagt gttgaagaag agtttgcaaa
4861 tgcaacaaaa tatttaatta ccggttgtta aaactggttt agcacaattt atattttccc
4921 tctcttgcct ttcttatttg caataaaagg tattgagcca ttttttaaat gacatttttg
4981 ataaattatg tttgtactag ttgatgaagg agtttttttt aacctgttta tataattttg
5041 cagcagaagc caaatttttt gtatattaaa gcaccaaatt catgtacagc atgcatcacg
5101 gatcaataga ctgtacttat tttccaataa aattttcaaa ctttgtactg ttaaaaaaaa
5161 aaaaaaaaaa
**OLAH (SEQ ID NO: 16)** - Homo sapiens oleoyl-ACP hydrolase (OLAH), transcript variant 2, mRNA - NM 001039702 
1 acctcatttc ctgtgtcctc tctttctttg gcaatccaaa gaaagtcatc tttcagattg
61 tctgctcaga gttcatctca aagcctggca aggattggag aggtcaataa gagtcagcgc
121 ctttaaaaag aaatctactc actcttctgt gtgcataagg ccgagcagag gttcttcgtc
181 tcaagaggaa ctgacttctg ttgagcactc aacacgccac agagaccagc catcttgcaa
241 cctcacctca cagcatggag agaggagacc aacctaagag aaccaggaat gaaaacattt
301 tcaactgctt atacaaaaac cctgaggcaa cttttaagct gatttgcttt ccctggatgg
361 gaggtggctc cactcatttt gccaaatggg gccaagatac tcatgatttg ctggaagtgc
421 actccttaag gcttcctgga agagaaagca gagttgaaga acctcttgaa aatgacatct
481 cccagttagt tgatgaagtt gtttgtgctc tgcagccagt catccaggat aaaccatttg
541 cattttttgg ccacagtatg ggatcctaca ttgcttttag gactgcacta ggtctaaaag
601 aaaacaatca accagaacca ttgcatttat ttttgtcaag tgcaactcct gtacattcaa
661 aggcctggca tcgcattccc aaagatgatg aattgtcaga agaacaaata agtcattacc
721 ttatggaatt tggaggcacc cccaagcatt ttgctgaagc caaggaattt gtgaaacaat
781 gtagtcccat cataagggca gatctgaaca ttgttagaag ttgcacctct aacgtaccat
841 ctaaggctgt tctttcctgt gacttgacat gttttgttgg atctgaagac atagcaaagg
901 acatggaagc ctggaaagat gtaaccagtg gaaatgctaa aatttaccag cttccagggg
961 gtcactttta tcttctggat cctgcgaacg agaaattaat caagaactac ataatcaagt
1021 gtctagaagt atcatcgata tccaattttt agatattttc cctttcactt ttaaaataat
1081 caaagtaata tcatactctt ctcagttatt cagatatagc tcagttttat tcagattgga
1141 aattacacat tttctactgt cagggagatt cgttacataa atatatttac gtatctgggg
1201 acaaaggtca agccagtaaa gaatacttct ggcagcactt tgggaggcca aggcgggcgg
1261 atcacgaggt caggagatcg agaccgtcct ggctaacacc gtgaaacccc atctctacta
1321 aaaatacaca aaattagccg ggcgtggtgg tgggcacctg tagtcccagc tactcgggag
1381 gctgaggcag gagaatggtg tgaacctggg aggtggagct tgcagtgaac cgagatcgct
 
1441 ccactgcact ccagcctggg tgacagatcc agactctgtc tcaaaaaaaa aaaaaaaaaa
1501 aatacttctg gcagagtctt ttatcttcct attaaaatct cacttgattc tcctttatgg
1561 gaagtttgtc gacaaaattc atgattagta aattatccat tttttccttc agttagttta
1621 atggtgaaga tgattaacag gggaaatgct tgaagtaaat gattgtttca atggc
**IL1R2 (SEQ ID NO: 17)** - Homo sapiens interleukin 1 receptor type 2 (IRL1R2), transcript variant 1, mRNA- NM 004633 
1 cccgtgagga ggaaaaggtg tgtccgctgc cacccagtgt gagcgggtga caccacccgg
61 ttaggaaatc ccagctccca agagggtata aatccctgct ttactgctga gctcctgctg
121 gaggtgaaag tctggcctgg cagccttccc caggtgagca gcaacaaggc cacgtgctgc
181 tgggtctcag tcctccactt cccgtgtcct ctggaagttg tcaggagcaa tgttgcgctt
241 gtacgtgttg gtaatgggag tttctgcctt cacccttcag cctgcggcac acacaggggc
301 tgccagaagc tgccggtttc gtgggaggca ttacaagcgg gagttcaggc tggaagggga
361 gcctgtagcc ctgaggtgcc cccaggtgcc ctactggttg tgggcctctg tcagcccccg
421 catcaacctg acatggcata aaaatgactc tgctaggacg gtcccaggag aagaagagac
481 acggatgtgg gcccaggacg gtgctctgtg gcttctgcca gccttgcagg aggactctgg
541 cacctacgtc tgcactacta gaaatgcttc ttactgtgac aaaatgtcca ttgagctcag
601 agtttttgag aatacagatg ctttcctgcc gttcatctca tacccgcaaa ttttaacctt
661 gtcaacctct ggggtattag tatgccctga cctgagtgaa ttcacccgtg acaaaactga
721 cgtgaagatt caatggtaca aggattctct tcttttggat aaagacaatg agaaatttct
781 aagtgtgagg gggaccactc acttactcgt acacgatgtg gccctggaag atgctggcta
841 ttaccgctgt gtcctgacat ttgcccatga aggccagcaa tacaacatca ctaggagtat
901 tgagctacgc atcaagaaaa aaaaagaaga gaccattcct gtgatcattt cccccctcaa
961 gaccatatca gcttctctgg ggtcaagact gacaatcccg tgtaaggtgt ttctgggaac
1021 cggcacaccc ttaaccacca tgctgtggtg gacggccaat gacacccaca tagagagcgc
1081 ctacccggga ggccgcgtga ccgaggggcc acgccaggaa tattcagaaa ataatgagaa
1141 ctacattgaa gtgccattga tttttgatcc tgtcacaaga gaggatttgc acatggattt
1201 taaatgtgtt gtccataata ccctgagttt tcagacacta cgcaccacag tcaaggaagc
1261 ctcctccacg ttctcctggg gcattgtgct ggccccactt tcactggcct tcttggtttt
1321 ggggggaata tggatgcaca gacggtgcaa acacagaact ggaaaagcag atggtctgac
1381 tgtgctatgg cctcatcatc aagactttca atcctatccc aagtgaaata aatggaatga
1441 aataattcaa acacaaaaaa aaaaaaaaaa aaaaaaaaaa aaaa
**CYP19A1 (SEQ ID NO: 18)** - Homo sapiens cytochrome P450 family 19 subfamily A member 1 (CYP19A1), transcript variant 2, mRNA - NM 031226 
1 gggagtttct ggagggctga acacgtggag gcaaacagga aggtgaagaa gaacttatcc
61 tatcaggacg gaaggtcctg tgctcgggat cttccagacg tcgcgcggtg tcagaaaccc
121 tgtggtgaaa ttcagcctgt ggattccaga aatttggagt gttcttgggg ggaaaaatcc
181 gcacacacaa agcaacattt ggaaatccct gtggactcta aattgccccc tctgaggtca
241 aggaacacaa gatggttttg gaaatgctga acccgataca ttataacatc accagcatcg
301 tgcctgaagc catgcctgct gccaccatgc cagtcctgct cctcactggc ctttttctct
361 tggtgtggaa ttatgagggc acatcctcaa taccaggtcc tggctactgc atgggaattg
421 gacccctcat ctcccacggc agattcctgt ggatggggat cggcagtgcc tgcaactact
481 acaaccgggt atatggagaa ttcatgcgag tctggatctc tggagaggaa acactcatta
541 tcagcaagtc ctcaagtatg ttccacataa tgaagcacaa tcattacagc tctcgattcg
601 gcagcaaact tgggctgcag tgcatcggta tgcatgagaa aggcatcata tttaacaaca
661 atccagagct ctggaaaaca actcgaccct tctttatgaa agctctgtca ggccccggcc
721 ttgttcgtat ggtcacagtc tgtgctgaat ccctcaaaac acatctggac aggttggagg
781 aggtgaccaa tgaatcgggc tatgtggacg tgttgaccct tctgcgtcgt gtcatgctgg
841 acacctctaa cacgctcttc ttgaggatcc ctttggacga aagtgctatc gtggttaaaa
901 tccaaggtta ttttgatgca tggcaagctc tcctcatcaa accagacatc ttctttaaga
961 tttcttggct atacaaaaag tatgagaagt ctgtcaagga tttgaaagat gccatagaag
1021 ttctgatagc agaaaaaaga cgcaggattt ccacagaaga gaaactggaa gaatgtatgg
1081 actttgccac tgagttgatt ttagcagaga aacgtggtga cctgacaaga gagaatgtga
 
1141 accagtgcat attggaaatg ctgatcgcag ctcctgacac catgtctgtc tctttgttct
1201 tcatgctatt tctcattgca aagcacccta atgttgaaga ggcaataata aaggaaatcc
1261 agactgttat tggtgagaga gacataaaga ttgatgatat acaaaaatta aaagtgatgg
1321 aaaacttcat ttatgagagc atgcggtacc agcctgtcgt ggacttggtc atgcgcaaag
1381 ccttagaaga tgatgtaatc gatggctacc cagtgaaaaa ggggacaaac attatcctga
1441 atattggaag gatgcacaga ctcgagtttt tccccaaacc caatgaattt actcttgaaa
1501 attttgcaaa gaatgttcct tataggtact ttcagccatt tggctttggg ccccgtggct
1561 gtgcaggaaa gtacatcgcc atggtgatga tgaaagccat cctcgttaca cttctgagac
1621 gattccacgt gaagacattg caaggacagt gtgttgagag catacagaag atacacgact
1681 tgtccttgca cccagatgag actaaaaaca tgctggaaat gatctttacc ccaagaaact
1741 cagacaggtg tctggaacac tagagaaggc tggtcagtac ccactctgga gcatttctca
1801 tcagtagttc acatacaaat catccatcct tgccaatagt gtcatcctca cagtgaacac
1861 tcagtggccc atggcatttt ataggcatac ctcctatggg ttgtcaccaa gctaggtgct
1921 atttgtcatc tgctcctgtt cacaccagag aaccaggcta caagagaaaa agcagaggcc
1981 aagagtttga gggagaaata gtcggtgaag aaaccgtatc cataaagacc cgattccacc
2041 aaatgtgctt tgagaaggat aggccttcat taacaaaatg tatgtctggt tccccagtag
2101 agctctactg cctcaaccca aggggatttt tatgtctggg gcagaaacac tcaagttgat
2161 tagaaagacc aggccaatgt cagggtacct ggggccaaac ccacctgcta gtgtgaatta
2221 aagtacttta attttgtttt ctgtggaggt ggaaaagcaa cattcatagt ctttggagaa
2281 atgcttagaa attcagcatt tgacccttgc tgtgaattaa gcccaattaa ttcctgtttg
2341 tctacatatg atctgtctgt ggcaaaagtt taatcagagg aaattctttc ccagtctgtc
2401 gatttatgcc tcagccactt gcctgtgcta caattcattg tgttacctgt agattcaggt
2461 aatacaaact atatataatc atcaagtaat acaaactaat ttagtaatag cctgggttaa
2521 gtattattag ggccctgtgt ctgctgtaga aaaaaaaatt cacatgatgc acttcaaatt
2581 caaataaaaa tccttttggc atgttcccat ttttgcttag ctcaattagt gtggctaacc
2641 aagagataac tgtaaatgtg acattgattt gctcttacta cagcttcagt gattggggga
2701 ggaaaagtcc caacccaatg ggctcaaact tctaaggggt actcctctca tccccttatc
2761 cttctccctc gacattttct ccctctttct tcccatgacc ccaaagccaa gggcaacaga
2821 tcagtaaaga acgtggtcag agtagaaccc ctgaagtatt ttttaatcct acctcaaaat
2881 ttaacagtta cctgagagat ttaacattat ctagttcatt gaatcattgt atgtggtcat
2941 ggataaattg cacaccttgg aattcgcttt ctaaaggaaa tcaaatgaat ggaggaactt
3001 tccaaacacc actttacttg tgttatatag ccaatataac tatctctact gaatgtcatt
3061 gaaaaactaa aaaattaaac ttatttacaa ataggtaaat atttgtcatt gaatccattg
3121 ccatcccatt tgactgttct tttcatccta ctgtctagta ataagctgag tataagatga
3181 cagtgtaatc tccctgaaag caggagctac tttctttctt ttgtaatcta tttccatccc
3241 catttccctg tcctgtctcc ctgtattcac tcccaagctc agttctgaat agacattcct
3301 gctcagagat actcccaact gatgcagaaa ccaaataaag aggtaggtat tccaagaatt
3361 caagaatgga cattagtaaa gaataaaaca tttatttgag cttggaatta tttggatcat
3421 ctatatggcc taaaaatata tggactatgc ctgtgtacct gaatacgtat gtagtcaggt
3481 caagacaatc atccaaataa cttagacccc taaaagcaag gccaggattt gcaatttaat
3541 gtgtcccaat taattcactt gaaaattagt aacactctgt ttacgttgcc tctggctgga
3601 gctgcatggt ggaagaagcc caactttgga tccatgtact tcacccatcc aatactcttg
3661 ggacatttat gtgtatttta tctgtatata tgaagccaat gtctatgtct acacagtcaa
3721 agtgaaatgc atgtttgata tagctgtaca tagatatcta ttttgcaggt acaaaaatat
3781 cctgggggaa aactgggagt ggaagggtgg ggggtgggag tgagggacat gggggaggga
3841 caggaagagg agaagtgttg gtttgaacga tccaagcaaa ctctcccaga atcaaattac
3901 ctgggtagtt gttcaacttt tcactctgct tagcctgtat agacaaaccc catatatttg
3961 tagaggcttg gccttggaat tctggaatac cattggcttt tcagtaggct gatgaacaca
4021 ttttgaaaat tctattatct tcagaatttt gccccattgt taagtgctta accgtcactc
4081 ttgaatgtgc aatgtgctgt ggattccatt ttcatcagtt ctgaaagaac tgcaatgtgt
4141 aaattatcag tgaaatgcat gcatataagg gctctatcat tatcaaattg taaggacaat
4201 tgtacccttc tatatctttg ggcatgctag acacccccat gccttcattg agatcccatt
4261 ttccccctct caagtggaaa ataatcacat ccagcaagct ctctcattat tgagaaatac
4321 catttggaaa ttgccacttt ttattcctaa gcagcacctt tcactgttca tgatgctaat
4381 gttccacaaa agcatgtgcc attggcccac tgaaggatag agggaccctt ttcaatctat
4441 atcagctggg ctctgggact gaatctctca cctattcttg cagaaagaca tactaattaa
 4501 accttgtcaa agtaaaaaaa aaaaaaaaaa a
**MMP8 (SEQ ID NO: 19)** - Homo sapiens matrix metallopeptidase 8 (MMP8), transcript variant 1, mRNA - NM 002424 
1 gacacatgat gctgtgaacg tcagggtgct cgccagggaa gggccctacc cagagggaca
61 gaaagaaagc caggaggggt agagtttgaa gagaagatca tgttctccct gaagacgctt
121 ccatttctgc tcttactcca tgtgcagatt tccaaggcct ttcctgtatc ttctaaagag
181 aaaaatacaa aaactgttca ggactacctg gaaaagttct accaattacc aagcaaccag
241 tatcagtcta caaggaagaa tggcactaat gtgatcgttg aaaagcttaa agaaatgcag
301 cgattttttg ggttgaatgt gacggggaag ccaaatgagg aaactctgga catgatgaaa
361 aagcctcgct gtggagtgcc tgacagtggt ggttttatgt taaccccagg aaaccccaag
421 tgggaacgca ctaacttgac ctacaggatt cgaaactata ccccacagct gtcagaggct
481 gaggtagaaa gagctatcaa ggatgccttt gaactctgga gtgttgcatc acctctcatc
541 ttcaccagga tctcacaggg agaggcagat atcaacattg ctttttacca aagagatcac
601 ggtgacaatt ctccatttga tggacccaat ggaatccttg ctcatgcctt tcagccaggc
661 caaggtattg gaggagatgc tcattttgat gccgaagaaa catggaccaa cacctccgca
721 aattacaact tgtttcttgt tgctgctcat gaatttggcc attctttggg gctcgctcac
781 tcctctgacc ctggtgcctt gatgtatccc aactatgctt tcagggaaac cagcaactac
841 tcactccctc aagatgacat cgatggcatt caggccatct atggactttc aagcaaccct
901 atccaaccta ctggaccaag cacacccaaa ccctgtgacc ccagtttgac atttgatgct
961 atcaccacac tccgtggaga aatacttttc tttaaagaca ggtacttctg gagaaggcat
1021 cctcagctac aaagagtcga aatgaatttt atttctctat tctggccatc ccttccaact
1081 ggtatacagg ctgcttatga agattttgac agagacctca ttttcctatt taaaggcaac
1141 caatactggg ctctgagtgg ctatgatatt ctgcaaggtt atcccaagga tatatcaaac
1201 tatggcttcc ccagcagcgt ccaagcaatt gacgcagctg ttttctacag aagtaaaaca
1261 tacttctttg taaatgacca attctggaga tatgataacc aaagacaatt catggagcca
1321 ggttatccca aaagcatatc aggtgccttt ccaggaatag agagtaaagt tgatgcagtt
1381 ttccagcaag aacatttctt ccatgtcttc agtggaccaa gatattacgc atttgatctt
1441 attgctcaga gagttaccag agttgcaaga ggcaataaat ggcttaactg tagatatggc
1501 tgaagcaaaa tcaaatgtgg ctgtatccac tttcagaatg ttgaagggaa gttcagcaag
1561 cattttcgtt acattgtgtc ctgcttatac ttttctcaat attaagtcat tgtttcccat
1621 cactgtatcc attctacctg tcctccgtga aaatatgttt ggaatattcc actatttgca
1681 gaggcttatt cagttcttac acattccatc ttacattagt gattccatca aagagaagga
1741 aagtaagcct tttgtcacct tcaatattta ctatttcaat acttacatat ctgacttcta
1801 ggatttattg ttatattact tgcctatctg acttcataca tccctcagtt tcttaaaatg
1861 tcctatgtat atcttctaca tgcaatttag aactagattt tggttagaag taaggattat
1921 aaacaaccta gacagtaccc ttggccttta cagaaaatat ggtgctgttt tctacccttg
1981 gaaagaaatg tagatgatat gtttcgtggg ttgaattgtg tcccccataa aagatatgtt
2041 gaagttctaa ccccaggtac ccatgaatgt gagcttacca gggtctttgc agatgtaatt
2101 agttaagtta aggtgagatc acactgaatt agggtgggct ctaaatccat tatgactgtt
2161 gttcttataa gaagaagaga ggcatagtca cctaggggag gaggccgtat gaagacagag
2221 gcagagattg gagtgacgca tctccaagcc aaggaattcc aaggactgta agccaccagt
2281 agaagctttg aagaggcaag gaaggattcc ctccaatagc cttcaagtgt gaccctgctg
2341 acacctgcag aattcggact tctatcctcc aaaaccgtga gggaataaat ttcctttgtt
2401 ttaagccacc aactttgcaa tactttgtta cagcaaccct agacatgagg tactagacac
2461 agtacatcta cacatatgaa aatgaatcaa cacagaatgc agaagtagaa cccttgctaa
2521 ggactactgg gcatcttccc aggacagcag ccaaaagaga accaccactt cctctcctgc
2581 ctcctccttg ctctctccta gagtccaaac ccaaatgggc cagttggatc tgatgttcgt
2641 cagttcttta cttctatttc ctggggtact caggagggca cacactatag ataacttggg
2701 ttagctgcat aaaattcaat gtctcattaa gttgcattaa actgagctta gatgtgtaag
2761 tttgctaacg gatgggtttt tttgttaaga actataggat ttatgggacc aagtctagcg
2821 agtccagata tcaaaatcat tataatgtta tatttgctgt tattagaata taatatagct
2881 tattatacaa taaatatgta gactgtaaaa tatatttctc actagtacct cctattttct
2941 ttctctgttg aagtttttaa atcccacaga taattaaatt ggcaccttta tgcttgttca
3001 aaaattaaaa taatctatta aataagttca aattaaagat ttttacttca aatgac
**TGFA (SEQ ID NO: 20)** - Homo sapiens transforming growth factor alpha (TGFA), transcript variant 1, mRNA - NM 003236 
1 gtcagctgtg ccccggtcgc cgagtggcga ggaggtgacg gtagccgcct tcctatttcc
61 gcccggcggg cagcgctgcg gggcgagtgc cagcagagag gcgctcggtc ctccctccgc
121 cctcccgcgc cgggggcagg ccctgcctag tctgcgtctt tttcccccgc accgcggcgc
181 cgctccgcca ctcgggcacc gcaggtaggg caggaggctg gagagcctgc tgcccgcccg
241 cccgtaaaat ggtcccctcg gctggacagc tcgccctgtt cgctctgggt attgtgttgg
301 ctgcgtgcca ggccttggag aacagcacgt ccccgctgag tgcagacccg cccgtggctg
361 cagcagtggt gtcccatttt aatgactgcc cagattccca cactcagttc tgcttccatg
421 gaacctgcag gtttttggtg caggaggaca agccagcatg tgtctgccat tctgggtacg
481 ttggtgcacg ctgtgagcat gcggacctcc tggccgtggt ggctgccagc cagaagaagc
541 aggccatcac cgccttggtg gtggtctcca tcgtggccct ggctgtcctt atcatcacat
601 gtgtgctgat acactgctgc caggtccgaa aacactgtga gtggtgccgg gccctcatct
661 gccggcacga gaagcccagc gccctcctga agggaagaac cgcttgctgc cactcagaaa
721 cagtggtctg aagagcccag aggaggagtt tggccaggtg gactgtggca gatcaataaa
781 gaaaggcttc ttcaggacag cactgccaga gatgcctggg tgtgccacag accttcctac
841 ttggcctgta atcacctgtg cagccttttg tgggccttca aaactctgtc aagaactccg
901 tctgcttggg gttattcagt gtgacctaga gaagaaatca gcggaccacg atttcaagac
961 ttgttaaaaa agaactgcaa agagacggac tcctgttcac ctaggtgagg tgtgtgcagc
1021 agttggtgtc tgagtccaca tgtgtgcagt tgtcttctgc cagccatgga ttccaggcta
1081 tatatttctt tttaatgggc cacctcccca caacagaatt ctgcccaaca caggagattt
1141 ctatagttat tgttttctgt catttgccta ctggggaaga aagtgaagga ggggaaactg
1201 tttaatatca catgaagacc ctagctttaa gagaagctgt atcctctaac cacgagaccc
1261 tcaaccagcc caacatcttc catggacaca tgacattgaa gaccatccca agctatcgcc
1321 acccttggag atgatgtctt atttattaga tggataatgg ttttattttt aatctcttaa
1381 gtcaatgtaa aaagtataaa accccttcag acttctacat taatgatgta tgtgttgctg
1441 actgaaaagc tatactgatt agaaatgtct ggcctcttca agacagctaa ggcttgggaa
1501 aagtcttcca gggtgcggag atggaaccag aggctgggtt actggtagga ataaaggtag
1561 gggttcagaa atggtgccat tgaagccaca aagccggtaa atgcctcaat acgttctggg
1621 agaaaactta gcaaatccat cagcagggat ctgtcccctc tgttggggag agaggaagag
1681 tgtgtgtgtc tacacaggat aaacccaata catattgtac tgctcagtga ttaaatgggt
1741 tcacttcctc gtgagccctc ggtaagtatg tttagaaata gaacattagc cacgagccat
1801 aggcatttca ggccaaatcc atgaaagggg gaccagtcat ttattttcca ttttgttgct
1861 tggttggttt gttgctttat ttttaaaagg agaagtttaa ctttgctatt tattttcgag
1921 cactaggaaa actattccag taattttttt ttcctcattt ccattcagga tgccggcttt
1981 attaacaaaa actctaacaa gtcacctcca ctatgtgggt cttcctttcc cctcaagaga
2041 aggagcaatt gttcccctga gcatctgggt ccatctgacc catggggcct gcctgtgaga
2101 aacagtgggt cccttcaaat acatagtgga tagctcatcc ctaggaattt tcattaaaat
2161 ttggaaacag agtaatgaag aaataatata taaactcctt atgtgaggaa atgctactaa
2221 tatctgaaaa gtgaaagatt tctatgtatt aactcttaag tgcacctagc ttattacatc
2281 gtgaaaggta catttaaaat atgttaaatt ggcttgaaat tttcagagaa ttttgtcttc
2341 ccctaattct tcttccttgg tctggaagaa caatttctat gaattttctc tttatttttt
2401 tttataattc agacaattct atgacccgtg tcttcatttt tggcactctt atttaacaat
2461 gccacacctg aagcacttgg atctgttcag agctgacccc ctagcaacgt agttgacaca
2521 gctccaggtt tttaaattac taaaataagt tcaagtttac atcccttggg ccagatatgt
2581 gggttgaggc ttgactgtag catcctgctt agagaccaat caacggacac tggtttttag
2641 acctctatca atcagtagtt agcatccaag agactttgca gaggcgtagg aatgaggctg
2701 gacagatggc ggaagcagag gttccctgcg aagacttgag atttagtgtc tgtgaatgtt
2761 ctagttccta ggtccagcaa gtcacacctg ccagtgccct catccttatg cctgtaacac
2821 acatgcagtg agaggcctca catatacgcc tccctagaag tgccttccaa gtcagtcctt
2881 tggaaaccag caggtctgaa aaagaggctg catcaatgca agcctggttg gaccattgtc
2941 catgcctcag gatagaacag cctggcttat ttggggattt ttcttctaga aatcaaatga
3001 ctgataagca ttggatccct ctgccattta atggcaatgg tagtctttgg ttagctgcaa
3061 aaatactcca tttcaagtta aaaatgcatc ttctaatcca tctctgcaag ctccctgtgt
 
3121 ttccttgccc tttagaaaat gaattgttca ctacaattag agaatcattt aacatcctga
3181 cctggtaagc tgccacacac ctggcagtgg ggagcatcgc tgtttccaat ggctcaggag
3241 acaatgaaaa gcccccattt aaaaaaataa caaacatttt ttaaaaggcc tccaatactc
3301 ttatggagcc tggatttttc ccactgctct acaggctgtg acttttttta agcatcctga
3361 caggaaatgt tttcttctac atggaaagat agacagcagc caaccctgat ctggaagaca
3421 gggccccggc tggacacacg tggaaccaag ccagggatgg gctggccatt gtgtccccgc
3481 aggagagatg ggcagaatgg ccctagagtt cttttccctg agaaaggaga aaaagatggg
3541 attgccactc acccacccac actggtaagg gaggagaatt tgtgcttctg gagcttctca
3601 agggattgtg ttttgcaggt acagaaaact gcctgttatc ttcaagccag gttttcgagg
3661 gcacatgggt caccagttgc tttttcagtc aatttggccg ggatggacta atgaggctct
3721 aacactgctc aggagacccc tgccctctag ttggttctgg gctttgatct cttccaacct
3781 gcccagtcac agaaggagga atgactcaaa tgcccaaaac caagaacaca ttgcagaagt
3841 aagacaaaca tgtatatttt taaatgttct aacataagac ctgttctctc tagccattga
3901 tttaccaggc tttctgaaag atctagtggt tcacacagag agagagagag tactgaaaaa
3961 gcaactcctc ttcttagtct taataattta ctaaaatggt caacttttca ttatctttat
4021 tataataaac ctgatgcttt tttttagaac tccttactct gatgtctgta tatgttgcac
4081 tgaaaaggtt aatatttaat gttttaattt attttgtgtg gtaagttaat tttgatttct
4141 gtaatgtgtt aatgtgatta gcagttattt tccttaatat ctgaattata cttaaagagt
4201 agtgagcaat ataagacgca attgtgtttt tcagtaatgt gcattgttat tgagttgtac
4261 tgtaccttat ttggaaggat gaaggaatga atcttttttt cctaaatcaa aaaaaaaaaa
4321 aaaaaa
**VSTM1 (SEQ ID NO: 21)** - Homo sapiens V-set and transmembrane domain containing 1 (VSTM1), transcript variant 1, mRNA - NM 198481 
1 gtgagaagga aactgcaaga gtggggcaga gaaccagagt gtcagagcaa aacctcctct
61 atctgcacat cctggggacg aaccgggcag ccggagagct gcggccggcc cagtcccgct
121 ccgcctttga agggtaaaac ccaaggcggg gccttggttc tggcagaagg gacgctatga
181 ccgcagaatt cctctccctg ctttgcctcg ggctgtgtct gggctacgaa gatgagaaaa
241 agaatgagaa accgcccaag ccctccctcc acgcctggcc cagctcggtg gttgaagccg
301 agagcaatgt gaccctgaag tgtcaggctc attcccagaa tgtgacattt gtgctgcgca
361 aggtgaacga ctctgggtac aagcaggaac agagctcggc agaaaacgaa gctgaattcc
421 ccttcacgga cctgaagcct aaggatgctg ggaggtactt ttgtgcctac aagacaacag
481 cctcccatga gtggtcagaa agcagtgaac acttgcagct ggtggtcaca gataaacacg
541 atgaacttga agctccctca atgaaaacag acaccagaac catctttgtc gccatcttca
601 gctgcatctc catccttctc ctcttcctct cagtcttcat catctacaga tgcagccagc
661 acagttcatc atctgaggaa tccaccaaga gaaccagcca ttccaaactt ccggagcagg
721 aggctgccga ggcagattta tccaatatgg aaagggtatc tctctcgacg gcagaccccc
781 aaggagtgac ctatgctgag ctaagcacca gcgccctgtc tgaggcagct tcagacacca
841 cccaggagcc cccaggatct catgaatatg cggcactgaa agtgtagcaa gaagacagcc
901 ctggccacta aaggaggggg gatcgtgctg gccaaggtta tcggaaatct ggagatgcag
961 atactgtgtt tccttgctct tcgtccatat caataaaatt aagtttctcg tcttaaaaag
1021 aaaaaaaaaa aaaa
**FCER1A (SEQ ID NO: 22)** Fc fragment of IgE receptor Ia, NM 002001.3 
1 tactaagagt ctccagcatc ctccacctgt ctaccaccga gcatgggcct atatttgaag
61 ccttagatct ctccagcaca gtaagcacca ggagtccatg aagaagatgg ctcctgccat
121 ggaatcccct actctactgt gtgtagcctt actgttcttc gctccagatg gcgtgttagc
181 agtccctcag aaacctaagg tctccttgaa ccctccatgg aatagaatat ttaaaggaga
241 gaatgtgact cttacatgta atgggaacaa tttctttgaa gtcagttcca ccaaatggtt
301 ccacaatggc agcctttcag aagagacaaa ttcaagtttg aatattgtga atgccaaatt
361 tgaagacagt ggagaataca aatgtcagca ccaacaagtt aatgagagtg aacctgtgta
421 cctggaagtc ttcagtgact ggctgctcct tcaggcctct gctgaggtgg tgatggaggg
481 ccagcccctc ttcctcaggt gccatggttg gaggaactgg gatgtgtaca aggtgatcta
541 ttataaggat ggtgaagctc tcaagtactg gtatgagaac cacaacatct ccattacaaa
 
601 tgccacagtt gaagacagtg gaacctacta ctgtacgggc aaagtgtggc agctggacta
661 tgagtctgag cccctcaaca ttactgtaat aaaagctccg cgtgagaagt actggctaca
721 attttttatc ccattgttgg tggtgattct gtttgctgtg gacacaggat tatttatctc
781 aactcagcag caggtcacat ttctcttgaa gattaagaga accaggaaag gcttcagact
841 tctgaaccca catcctaagc caaaccccaa aaacaactga tataattact caagaaatat
901 ttgcaacatt agtttttttc cagcatcagc aattgctact caattgtcaa acacagcttg
961 caatatacat agaaacgtct gtgctcaagg atttatagaa atgcttcatt aaactgagtg
1021 aaactggtta agtggcatgt aatagtaagt gctcaattaa cattggttga ataaatgaga
1081 gaatgaatag attcatttat tagcatttgt aaaagagatg ttcaatttca ataaaataaa
1141 tataaaacca tgtaacagaa tgcttctgag taaaaaaaaa aaaaaaaaaa aaaaaaaa
**KLRK1 (SEQ ID NO: 23)** - Homo sapiens killer cell lectin like receptor K1 (KLRK1), mRNA - NM 007360 
1 actaagtatc tccactttca attctagatc aggaactgag gacatatcta aattttctag
61 ttttatagaa ggcttttatc cacaagaatc aagatcttcc ctctctgagc aggaatcctt
121 tgtgcattga agactttaga ttcctctctg cggtagacgt gcacttataa gtatttgatg
181 gggtggattc gtggtcggag gtctcgacac agctgggaga tgagtgaatt tcataattat
241 aacttggatc tgaagaagag tgatttttca acacgatggc aaaagcaaag atgtccagta
301 gtcaaaagca aatgtagaga aaatgcatct ccattttttt tctgctgctt catcgctgta
361 gccatgggaa tccgtttcat tattatggta acaatatgga gtgctgtatt cctaaactca
421 ttattcaacc aagaagttca aattcccttg accgaaagtt actgtggccc atgtcctaaa
481 aactggatat gttacaaaaa taactgctac caattttttg atgagagtaa aaactggtat
541 gagagccagg cttcttgtat gtctcaaaat gccagccttc tgaaagtata cagcaaagag
601 gaccaggatt tacttaaact ggtgaagtca tatcattgga tgggactagt acacattcca
661 acaaatggat cttggcagtg ggaagatggc tccattctct cacccaacct actaacaata
721 attgaaatgc agaagggaga ctgtgcactc tatgcctcga gctttaaagg ctatatagaa
781 aactgttcaa ctccaaatac gtacatctgc atgcaaagga ctgtgtaaag atgatcaacc
841 atctcaataa aagccaggaa cagagaagag attacaccag cggtaacact gccaactgag
901 actaaaggaa acaaacaaaa acaggacaaa atgaccaaag actgtcagat ttcttagact
961 ccacaggacc aaaccataga acaatttcac tgcaaacatg catgattctc caagacaaaa
1021 gaagagagat cctaaaggca attcagatat ccccaaggct gcctctccca ccacaagccc
1081 agagtggatg ggctggggga ggggtgctgt tttaatttct aaaggtagga ccaacaccca
1141 ggggatcagt gaaggaagag aaggccagca gatcactgag agtgcaaccc caccctccac
1201 aggaaattgc ctcatgggca gggccacagc agagagacac agcatgggca gtgccttccc
1261 tgcctgtggg ggtcatgctg ccacttttaa tgggtcctcc acccaacggg gtcagggagg
1321 tggtgctgcc ccagtgggcc atgattatct taaaggcatt attctccagc cttaagtaag
1381 atcttaggac gtttcctttg ctatgatttg tacttgcttg agtcccatga ctgtttctct
1441 tcctctcttt cttccttttg gaatagtaat atccatccta tgtttgtccc actattgtat
1501 tttggaagca cataacttgt ttggtttcac aggttcacag ttaagaagga attttgcctc
1561 tgaataaata gaatcttgag tctcatgcaa aaaaaaaaaa aaaaaa
**KLRB1 (SEQ ID NO: 24)** - Homo sapiens killer cell lectin like receptor B1 (KLRB1), mRNA - NM 002258 
1 gcctcacaga attgagagtt tgttcttaca cacaagttta atgccacctt cctctgtctg
61 ccatggacca acaagcaata tatgctgagt taaacttacc cacagactca ggcccagaaa
121 gttcttcacc ttcatctctt cctcgggatg tctgtcaggg ttcaccttgg catcaatttg
181 ccctgaaact tagctgtgct gggattattc tccttgtctt ggttgttact gggttgagtg
241 tttcagtgac atccttaata cagaaatcat caatagaaaa atgcagtgtg gacattcaac
301 agagcaggaa taaaacaaca gagagaccgg gtctcttaaa ctgcccaata tattggcagc
361 aactccgaga gaaatgcttg ttattttctc acactgtcaa cccttggaat aacagtctag
421 ctgattgttc caccaaagaa tccagcctgc tgcttattcg agataaggat gaattgatac
481 acacacagaa cctgatacgt gacaaagcaa ttctgttttg gattggatta aatttttcat
 
541 tatcagaaaa gaactggaag tggataaacg gctctttttt aaattctaat gacttagaaa
601 ttagaggtga tgctaaagaa aacagctgta tttccatctc acagacatct gtgtattctg
661 agtactgtag tacagaaatc agatggatct gccaaaaaga actaacacct gtgagaaata
721 aagtgtatcc tgactcttga
**DAAM2 (SEQ ID NO: 25)** - Homo sapiens dishevelled associated activator of morphogenesis 2 (DAAM2), transcript variant 2, mRNA - NM 015345 
1 gggcggggga aggagcatct caggaaaggg gtccccggac tctggggctc tcagcacctg
61 cggtcgcaaa ccaacctcat gccctgactt taccaggcgt cgggactctg acttaaccgg
121 ggaatgaggg acttggtctg gcggcagatc acaatgagga cctagggcat ctgtctgctg
181 acgccccctg gcctgcagtg accatggccc cccgcaagag gagccaccat ggcctgggct
241 tcctgtgctg cttcgggggc agtgacatcc ccgaaatcaa cctccgggac aaccaccctc
301 tgcagttcat ggagttctcc agccccatcc cgaacgcaga ggagctcaac atccgctttg
361 cagagctggt ggatgaattg gatctcactg acaaaaaccg agaggctatg tttgcactgc
421 cccctgagaa gaaatggcag atctactgca gcaagaagaa ggagcaggag gaccccaaca
481 agctggcaac cagctggcct gactattaca tcgaccgcat caattccatg gctgcgatgc
541 agagtctgta cgcgtttgat gaggaggaga cggagatgag gaaccaagtc gtggaagacc
601 tgaagacagc cctccggaca cagcctatga ggtttgtgac ccgcttcatt gagctggagg
661 gcttgacctg tctgctaaat ttcctccgga gcatggacca cgccacctgt gagagccgca
721 tccacacctc actcattggc tgcatcaaag cattgatgaa caactcccag gggcgggcac
781 atgtgctggc acagcctgag gccattagta ccatagccca gagcctacgc acagagaaca
841 gcaagaccaa ggtggctgtg ctggagatcc tgggtgctgt gtgcctcgtg cctggtggcc
901 acaagaaggt gctgcaggcc atgctgcact accaggtgta tgcagcagag cgaacccgct
961 tccagaccct gctgaacgag ctagaccgaa gtctgggccg gtaccgggat gaagtgaatc
1021 tgaaaacagc catcatgtcc ttcatcaatg ctgtcctcaa tgctggagct ggagaggata
1081 atctggagtt ccgcctacat ctacggtatg aattcctgat gctgggtata cagcctgtga
1141 ttgacaagct ccggcaacat gaaaatgcca tcctggacaa acatttagac ttcttcgaga
1201 tggtgcggaa tgaggatgac ctggagctag ccaggaggtt tgacatggtc cacatcgaca
1261 ccaagagtgc ttcccagatg tttgagttga tccacaagaa gctgaagtac acggaggcct
1321 acccctgcct gctctctgtg ctgcaccact gcctgcagat gccctacaaa cggaacggtg
1381 gctacttcca gcagtggcag ctcctggacc gcatcctcca gcagattgtc ctccaggatg
1441 agcggggtgt ggaccctgac ctggctccct tggagaactt caatgtcaag aacatcgtca
1501 acatgctcat caacgagaat gaagtgaaac agtggcgaga ccaggcagag aagttccgga
1561 aagaacacat ggagcttgtg agccgtctgg agaggaagga gcgggaatgc gagacaaaga
1621 cattggagaa ggaagagatg atgcggacgc tgaacaaaat gaaggacaag ctggcccggg
1681 agtcccagga gctgcgccag gctcggggac aagtggcaga gctggtagcc cagctcagtg
1741 aactctcaac aggccctgta tcttccccac caccccctgg gggcccactc accttgtctt
1801 cctcaatgac aaccaatgac ctgcctccac cccctcctcc tctgcccttt gcctgttgtc
1861 cccctccccc accaccaccc cttcctcccg gggggacccc gactccccca ggtgccccac
1921 cttgcctcgg catgggcctg cccctccctc aggaccccta ccccagcagt gacgtcccac
1981 tcaggaaaaa gcgtgtcccc cagccttctc acccactgaa gtccttcaac tgggtgaagc
2041 tgaatgagga gcgtgtccct ggcaccgtat ggaatgagat tgatgacatg caggtatttc
2101 ggatcctgga cctagaggat tttgaaaaga tgttttcagc ctaccagagg caccagaaag
2161 agctgggctc cactgaagac atctacctgg cttcccgcaa ggtcaaagag ctgtcggtca
2221 ttgatggccg gagggcccaa aactgcatca tccttctttc caagttgaag ctttctaacg
2281 aggagatccg gcaggccatc ttgaagatgg atgagcagga ggaccttgct aaggacatgc
2341 tggagcagct cctcaagttc atcccagaga agagtgacat tgacctcctg gaggagcaca
2401 agcatgaaat tgagcggatg gcccgtgctg accgcttcct ctatgaaatg agcaggattg
2461 accactacca gcagcgactg caagccctct tcttcaagaa gaaattccag gagcggctgg
2521 ctgaggcaaa gcccaaagtg gaagccatcc tgttggcctc ccgggagctg gtccgcagca
2581 agcgtcttag acagatgcta gaggtcatcc tagccatagg caacttcatg aacaaagggc
2641 agcgtggggg cgcctacggg ttccgggtgg ccagcctcaa caagatcgct gacaccaagt
2701 ccagcatcga cagaaacatc tctctgctcc attacctgat catgatcctg gagaagcatt
2761 ttcctgatat tctaaacatg ccttcagagc tgcaacatct tccagaagct gccaaagtca
2821 acctagcaga actggagaag gaggtgggca acctcaggag gggcctgaga gcggtggagg
 
2881 tgctggagta tcagaggcgc caggtacggg agcccagtga caagtttgtc cctgtcatga
2941 gcgacttcat cacggtgtcc agcttcagct tctccgagct ggaggaccag ctaaatgagg
3001 ccagggacaa gttcgccaag gccttgatgc acttcgggga gcatgacagc aagatgcagc
3061 cagacgaatt ctttggcatc tttgatacct tcttgcaggc cttctcagag gcccggcagg
3121 atctagaggc catgaggagg aggaaggagg aggaggagcg gcgggcgcgc atggaagcca
3181 tgctgaagga gcagagggaa cgtgagcggt ggcagcggca gcggaaggtc ctggctgcag
3241 gcagctcgct ggaggaggga ggagagttcg atgacctggt gtcggccctg cgctctgggg
3301 aggtcttcga caaggactta tgcaagctca agcgcagccg caagcgatca gggagccagg
3361 ccctggaagt tacccgggag cgggcaataa accggctaaa ttattgacct ggggaactag
3421 ccacacagga ggccgggaga cagggactgg tgagaatggg gctgagtgga ggaggtggtg
3481 atatttaaac catttggtgc ttggtttaga gccttgggct gggtcctggg atggggggct
3541 gtgtgtggct ggaccaggtg tctccccacg cttaccttaa ggggctcctc ttatctcccc
3601 ttcacatgat tccttctgtg ccctggcccc aggtattatt ctgaggctgc cttggatggc
3661 ctcaggccag gtaaccccag gctgaagggg ccctgctccc catcccctac catgggcacc
3721 catgtgctgg cacagaacag ttccagatct agactggaga ggtccacagc cttgtccaga
3781 gttcctgtgt agcacgggga gcaatgatgg agggagcccc tgagagggaa tctggtgagg
3841 gaatccagac tcccttctct caaggggagg ctcaacagaa cattgacctg ggggcaaact
3901 ttcctcttga atgggaacag aggaggcatt atatattcta gttagatcag ctctggtagg
3961 ttccagagaa cagtcaatgt tggaaggatg atgcagggac caaagccatc aggacagagt
4021 agcagtgtct gtttcccatg tcacaagtcc tctggcctct ccctgcatgt cttaagtatc
4081 tttcccttcc ttctctaccc tcacctccat cctgtctact aatccacagt cctagaagac
4141 tcaccttggg tttccacagc tatggctcac taccaggtgc ttgatgaatc tggcgagggg
4201 ctcaagacag acctcatgca tcaccacacc tcatgccttt tgggcatctc ccatgtcccc
4261 atctcctgga cacctggcca ttgttgtgaa gccagacagt gacctcaaat gttgccttgg
4321 agtcccctac agcccctcag cagagggcag cacttgaatg cttagctcca tcccatagtt
4381 ctctacttca tataaattgc tcaggccctc ccaccccttc tctaacacta gcttcaaggc
4441 agaagccaca gcagcctctg tccagcctgc aggtggccac ttggaaccat gtgtccactg
4501 gcgttgggga gttggttcct gagaggtctg agggccagag ctgccctcta cattaacatg
4561 ctgtctctaa gggtggcccc tcctctcagg cgttcagatg gtgcgaacag cagagcaggc
4621 aagggaaact ggggagatgg ggatggagga ggaaggctga tatcctctgg ggagcacatc
4681 acctgaaggt gccaaggagg aaggctgaga ggggggccac cccatttctg gtacccaatt
4741 tggttcttca gcccaacttg caaggggttc cttctggtcc tcccatccac tgccaccttc
4801 cattttgtcc atctcatgct ggccttggtg gatgggatgg ctgtatctag acaaaatttt
4861 tctaaaactc catcaaggct cttattcaat accacgttcc gagttggcct ttcatcttct
4921 ttgagactgg ccctgcctaa cctctaccat caatgagctc ttggcccttc tgcccttccc
4981 tgtgtttctc actttccaac ctaatccctg gctcagggtt attgccagtg gagactggtg
5041 agctgggcct actctcagct gcctatcttc tgcctttcac ttgcatccaa ctcctggggc
5101 tgggaccgta gtagctgcgg gggggaagaa acacagggtc ggtgagccca gcatgtgcgt
5161 tggtttgagg gggcgggcgg tgtgtgtgtg ttctggtggg agggatctga gcaagtgcaa
5221 gcctggctga cacaggtgtg aagaggccat cctggaaccc aggtgagggc aagatgaagg
5281 cttccaggca gaacagctgc agagagtttg gctatatgca tctgcagccc caagagctcc
5341 cactgcaaga caagtgttgg ggaagatggg aggttgtggg tgaggcctct aaaggtcctc
5401 tcccaaactg accaggctga tgtcaaccta accccctcag gggcagggaa caggggaggg
5461 ctccacaagc gtgtctggca ttcccaccca ccatggaaga ctggatacgc acctggaaac
5521 aaaaggacta tggaagctgt tcaagataca tttgatcttc agaaaagcag aatttggttc
5581 aactgttgac agaggacaca aatacgttgt tccagagctc agccttctca ctctaaaaga
5641 aagatatttt tctatttatt ttctacatct ggccagtggc tctggtgcta gatgccactg
5701 tagccagatc tccaacagtg ccttggacca tggactcata ctcaactgag taagaagggg
5761 ctggtgccca gtcgggtggg ctgagctggt ccttaatagg ttgtttcttg gtcttgcttt
5821 cttcatgccc tccccactgc tcctgccacc tttagataag tttctctagc taattttgtg
5881 gccaatgtaa aattcgtcat caacctaaca aacacaacct tctcagcagc atttctcccc
5941 tgtgatggaa ataaagtgtt tagggcagtg ggaggagaaa attctccagg tgaatgggga
6001 agggtctgtt ccagcctctc cctactccca tcccatttcc accaactggg gaactgtgac
6061 tatctatctc ccccgacttc taccagggat gccttcacgc caaggctgtt ctcaccagct
6121 gcctcagatg acaaatgagg ctaatggaca taatctacag tgtccttttt cacttgcacc
6181 ttttttataa gaatatattg taatactaaa aaatattaaa ttcataccat ccctacccag
 6241 tctgcctaaa aa
**HLA-DRA (SEQ ID NO: 26)** - Homo sapiens major histocompatibility complex, class II, DR alpha (HLA-DRA), mRNA - NM 019111 
1 ttttaatggt cagactctat tacaccccac attctctttt cttttattct tgtctgttct
61 gcctcactcc cgagctctac tgactcccaa cagagcgccc aagaagaaaa tggccataag
121 tggagtccct gtgctaggat ttttcatcat agctgtgctg atgagcgctc aggaatcatg
181 ggctatcaaa gaagaacatg tgatcatcca ggccgagttc tatctgaatc ctgaccaatc
241 aggcgagttt atgtttgact ttgatggtga tgagattttc catgtggata tggcaaagaa
301 ggagacggtc tggcggcttg aagaatttgg acgatttgcc agctttgagg ctcaaggtgc
361 attggccaac atagctgtgg acaaagccaa cctggaaatc atgacaaagc gctccaacta
421 tactccgatc accaatgtac ctccagaggt aactgtgctc acaaacagcc ctgtggaact
481 gagagagccc aacgtcctca tctgtttcat agacaagttc accccaccag tggtcaatgt
541 cacgtggctt cgaaatggaa aacctgtcac cacaggagtg tcagagacag tcttcctgcc
601 cagggaagac caccttttcc gcaagttcca ctatctcccc ttcctgccct caactgagga
661 cgtttacgac tgcagggtgg agcactgggg cttggatgag cctcttctca agcactggga
721 gtttgatgct ccaagccctc tcccagagac tacagagaac gtggtgtgtg ccctgggcct
781 gactgtgggt ctggtgggca tcattattgg gaccatcttc atcatcaagg gattgcgcaa
841 aagcaatgca gcagaacgca gggggcctct gtaaggcaca tggaggtgat ggtgtttctt
901 agagagaaga tcactgaaga aacttctgct ttaatggctt tacaaagctg gcaatattac
961 aatccttgac ctcagtgaaa gcagtcatct tcagcatttt ccagccctat agccacccca
1021 agtgtggata tgcctcttcg attgctccgt actctaacat ctagctggct tccctgtcta
1081 ttgccttttc ctgtatctat tttcctctat ttcctatcat tttattatca ccatgcaatg
1141 cctctggaat aaaacataca ggagtctgtc tctgctatgg aatgccccat ggggcatctc
1201 ttgtgtactt attgtttaag gtttcctcaa actgtgattt ttctgaacac aataaactat
1261 tttgatgatc ttgggtggaa aaaaaaaaaa aaaaaaaaaa aaaaaaaaaa aa
**BCL11B (SEQ ID NO: 27)** - Homo sapiens B-cell CLL/lymphoma 11B (BCL11B), transcript variant 1, mRNA - NM 138576 
1 tgcgctttcc acctaccaga ccctgaaaga aagtgtcagg agccggtgca aaacccagtt
61 taagttcaag aagacatttg caagtgcaag aggccaagca gtttgaagaa gtgtaagaga
121 ttttttttcc ttcgaaagaa tatattttta aagaaaccag ccagtccgcg gaaagcaaca
181 gcagtttttt ttttttttgc ctctttttct tattttagat cgagaggttt ttcttgcttt
241 tcttcccttt tttttctttt tgcaaacaaa acaaaaaaca gcatagaaga aagagcaaaa
301 taaagaagaa gaagaggagg aagagaggga aagagaggaa gggaaaaaaa acaccaaccc
361 gggcagagga ggaggtgcgg cggcggcggc ggcggcggca gcggcggcag cggcgcggcg
421 gcggctcgga ccccctcccc cggctccccc catcagtgca gctctccggg cgatgccaga
481 atagatgccg gggcaatgtc ccgccgcaaa cagggcaacc cgcagcactt gtcccagagg
541 gagctcatca ccccagaggc tgaccatgtg gaggccgcca tcctcgaaga agacgagggt
601 ctggagatag aggagccaag tggcctgggg ctgatggtgg gtggccccga ccctgacctg
661 ctcacctgtg gccagtgtca aatgaacttc cccttggggg acatcctggt ttttatagag
721 cacaaaagga agcagtgtgg cggcagcttg ggtgcctgct atgacaaggc cctggacaag
781 gacagcccgc caccctcctc acgctccgag ctcaggaaag tgtccgagcc ggtggagatc
841 gggatccaag tcacccccga cgaagatgac cacctgctct cacccacgaa aggcatctgt
901 cccaagcagg agaacattgc agggccgtgc aggcctgccc agctgccagc ggtggccccc
961 atagctgcct cctcccaccc tcactcatcc gtgatcactt cacctctgcg tgccctgggc
1021 gctctcccgc cctgcctccc cctgccgtgc tgcagcgcgc gcccggtctc gggtgacggg
1081 actcagggtg agggtcagac ggaggctccc tttggatgcc agtgtcagtt gtcaggtaaa
1141 gatgagcctt ccagctacat ttgcacaaca tgcaagcagc ccttcaacag cgcgtggttc
1201 ctgctgcagc acgcgcagaa cacgcacggc ttccgcatct acctggagcc cgggccggcc
1261 agcagctcgc tcacgccgcg gctcaccatc ccgccgccgc tcgggccgga ggccgtggcg
1321 cagtccccgc tcatgaattt cctgggcgac agcaacccct tcaacctgct gcgcatgacg
1381 ggccccatcc tgcgggacca cccgggcttc ggcgagggcc gcctgccggg cacgccgcct
1441 ctcttcagtc ccccgccgcg ccaccacctg gacccgcacc gcctcagtgc cgaggagatg
 
1501 gggctcgtcg cccagcaccc cagtgccttc gaccgagtca tgcgcctgaa ccccatggcc
1561 atcgactcgc ccgccatgga cttctcgcgg cggctccgcg agctggcggg caacagctcc
1621 acgccgccgc ccgtgtcccc gggccgcggc aaccctatgc accggctcct gaaccccttc
1681 cagcccagcc ccaagtcccc gttcctgagc acgccgccgc tgccgcccat gccccctggc
1741 ggcacgccgc ccccgcagcc gccagccaag agcaagtcgt gcgagttctg cggcaagacc
1801 ttcaagttcc agagcaatct catcgtgcac cggcgcagtc acacgggcga gaagccctac
1861 aagtgccagc tgtgcgacca cgcgtgctcg caggccagca agctcaagcg ccacatgaag
1921 acgcacatgc acaaggccgg ctcgctggcc ggccgctccg acgacgggct ctcggccgcc
1981 agctcccccg agcccggcac cagcgagctg gcgggcgagg gcctcaaggc ggccgacggt
2041 gacttccgcc accacgagag cgacccgtcg ctgggccacg agccggagga ggaggacgag
2101 gaggaggagg aggaggagga ggagctgcta ctggagaacg agagccggcc cgagtcgagc
2161 ttcagcatgg actcggagct gagccgcaac cgcgagaacg gcggtggtgg ggtgcccggg
2221 gtcccgggcg cgggggcggg cgcggccaag gcgctggctg acgagaaggc gctggtgctg
2281 ggcaaggtca tggagaacgt gggcctaggc gcactgccgc agtacggcga gctcctggcc
2341 gacaagcaga agcgcggcgc cttcctgaag cgtgcggcgg gcggcgggga cgcgggcgac
2401 gacgacgacg cgggcggctg cggggacgcg ggcgcgggcg gcgcggtcaa cgggcgcggg
2461 ggcggcttcg cgccaggcac cgagcccttc cccgggctct tcccgcgcaa gcccgcgccg
2521 ctgcccagcc ccgggctcaa cagcgccgcc aagcgcatca aggtggagaa ggacctggag
2581 ctgccgcccg ccgcgctcat cccgtccgag aacgtgtact cgcagtggct ggtgggctac
2641 gcggcgtcgc ggcacttcat gaaggacccc ttcctgggct tcacggacgc acgacagtcg
2701 cccttcgcca cgtcgtccga gcactcgtcc gagaacggca gcctgcgctt ctccacgccg
2761 cccggggacc tgctggacgg cggcctctcg ggccgcagcg gcacggccag cggaggcagc
2821 accccgcacc tgggcggccc gggccccggg cggcccagct ccaaggaggg ccgccgcagc
2881 gacacgtgcg agtactgcgg caaggtgttc aagaactgca gcaacttgac ggtgcaccgg
2941 cggagccaca ccggcgagcg gccttacaag tgcgagctgt gcaactacgc gtgcgcgcag
3001 agcagcaagc tcacgcgcca catgaagacg cacgggcaga tcggcaagga ggtgtaccgc
3061 tgcgacatct gccagatgcc cttcagcgtc tacagcaccc tggagaaaca catgaaaaag
3121 tggcacggcg agcacttgct gactaacgac gtcaaaatcg agcaggccga gaggagctaa
3181 gcgcgcgggc cccggcgccc cgcacctgta cagtggaacc gttgccaacc gagagaatgc
3241 tgacctgact tgcctccgtg tcaccgccac ccgcaccccc gcgtgtcccc ggggcccagg
3301 ggaggcggca ctccaaccta acctgtgtct gcgaagtcct atggaaaccc gagggttgat
3361 taaggcagta caaattgtgg agccttttaa ctgtgcaata atttctgtat ttattgggtt
3421 ttgtaatttt tttggcatgt gcaggtactt tttattatta ttttttctgt ttgaattcct
3481 ttaagagatt ttgttgggta tccatccctt ctttgttttt tttttaaccc ggtagtagcc
3541 tgagcaatga ctcgcaagca atgttagagg ggaagcatat cttttaaatt ataatttggg
3601 gggaggggtg gtgctgcttt tttgaaattt aagctaagca tgtgtaattt cttgtgaaga
3661 agccaacact caaatgactt ttaaagttgt ttactttttc attccttcct tttttttgtc
3721 ctgaaataaa aagtggcatg cagttttttt tttaattatt ttttaatttt tttttttggtt
3781 tttgtttttg gggtgggggg tgtggatgta cagcggataa caatctttca agtcgtagca
3841 ctttgtttca gaactggaat ggagatgtag cactcatgtc gtcccgagtc aagcggcctt
3901 ttctgtgttg atttcggctt tcatattaca taagggaaac cttgagtggt ggtgctgggg
3961 gaggcacccc acagactcag cgccgccaga gatagggttt ttggagggct cctctgggaa
4021 atggcccgac agcattctga ggttgtgcat gaccagcaga tactatcctg ttggtgtgcc
4081 ctggggtgcc atggctgcta ttcgctgtag attaggctac ataaaatggg ctgagggtac
4141 ctttttgggg agatggggtg gcctgcagtg acacagaaag gaagaaacta gcggtgttct
4201 tttaggcgtt ttctggcttg acggcttctc tcttttttta aatcaccccc accacataaa
4261 tctcaaatcc tatgttgcta caaggggtca tccatcattt cccaagcaga cgaatgccct
4321 aattaattga agttagtgtt ctctcattta atgcacactg atgatattgt agggatgggt
4381 ggggtgggga tcttgcaaat ttctattctc ttttactgaa aaagcagggg atgagttcca
4441 tcagaaggtg cccagcgcta cttcccaggt ttttattttt tttttcctat ctcattaggt
4501 tggaaggtac taaatattga actgttaaga ttagacattt gaattctgtt gacccgcact
4561 ttaaagcttt tgtttgcatt taaattaaat ggcttctaaa caagaaattg cagcatattc
4621 ttctctttgg cccagaggtg ggttaaactg taagggacag ctgagattga gtgtcagtat
4681 tgctaagcgt ggcattcaca atactggcac tataaagaac aaaataaaat aataatttat
4741 aggacagttt ttctactgcc attcaatttg atgtgagtgc cttgaaaact gatcttccta
4801 tttgagtctc ttgagacaaa tgcaaaactt tttttttgaa atgaaaagac tttttaaaaa
 
4861 agtaaaacaa gaaaagtaca ttctttagaa actaacaaag ccacatttac tttaagtaaa
4921 aaaaaaaaaa attctggttg aagatagagg atatgaaatg ccataagacc caatcaaatg
4981 aagaaataaa cccagcacaa ccttggacat ccattagctg aattatcctc agcccctttt
5041 gtttttggga caacgctgct tagatatgga gtggaggtga tttactgctg aattaaaact
5101 caagtgacac aagttacaag ttgatatcgt tgaatgaaaa gcaaaacaaa aacaattcag
5161 gaacaacggc taattttttc taaagttaaa tttagtgcac tctgtcttaa aaatacgttt
5221 acagtattgg gtacatacaa gggtaaaaaa aaaattgtgt gtatgtgtgt tggagcgatc
5281 tttttttttc aaagtttgct taataggtta tacaaaaatg ccacagtggc cgcgtgtata
5341 ttgttttctt ttggtgacgg ggttttagta tatattatat atattaaaat ttcttgatta
5401 ctgtaaaagt ggaccagtat ttgtaataat cgagaatgcc tgggcatttt acaaaacaag
5461 aaaaaaaata cccttttctt ttccttgaaa atgttgcagt aaaatttaaa tggtgggtct
5521 ataaatttgt tcttgttaca gtaactgtaa agtcggagtt ttagtaaatt tttttctgcc
5581 ttgggtgttg aatttttatt tcaaaaaaaa tgtatagaaa cttgtatttg gggattcaaa
5641 ggggattgct acaccatgta gaaaaagtat gtagaaaaaa agtgcttaat attgttattg
5701 ctttgcagaa aaaaaaaaaa tcacatttct gacctgtact tatttttctc ttcccgcctc
5761 cctctggaat ggatatattg gttggttcat atgatgtagg cacttgctgt atttttactg
5821 gagctcgtaa ttttttaact gtaagcttgt ccttttaaag ggatttaatg tacctttttg
5881 ttagtgaatt tggaaataaa aagaaaaaaa aaacaaaaac aaacaggctg ccataatata
5941 tttttttaat ttggcaggat aaaatattgc aaaaaaaaca catttgtatg ttaagtccta
6001 ttgtacagga gaaaaagggt tgtttgacaa cctttgagaa aaagaaacaa aaggaagtag
6061 ttaaatgctt tggttcacaa atcatttagt tgtatatatt ttttgtcgga attggcctac
6121 acagagaacc gttcgtgttg ggcttctctc tgaacgcccc gaaccttgca tcaaggctcc
6181 ttggtgtggc cacagcagac cagatgggaa attatttgtg ttgagtggaa aaaaatcagt
6241 ttttgtaaag atgtcagtaa cattccacat cgtcctccct ttctctaaga ggccatctct
6301 aagatgtcag atgtagagga gagagagcga gagaacatct tccttctcta ccatcactcc
6361 tgtggcggtc accaccacca cctctcccgc ccttaccagc agaaagcaat gcaaactgag
6421 ctgctttagt ccttgagaaa ttgtgaaaca aacacaaata tcataaaagg agctggtgat
6481 tcagctgggt ccaggtgaag tgacctgctg ttgagaccgg tacaaattgg atttcaggaa
6541 ggagactcca tcacagccag gacctttcgt gccatggaga gtgttggcct cttgtctttc
6601 ttccctgctt tgctgctttg ctctctgaaa cctacattcc gtcagtttcc gaatgcgagg
6661 gcctgggatg aatttggtgc ctttccatat ctcgttctct ctccttcccc tgcgtttcct
6721 ctccatcctt catcctccat tggtcctttt tttttctttc atttttattt taatttcttt
6781 tcttcctgtc tgttcctccc ctaatcctct attttatttt tattttttgt aaagccaagt
6841 agctttaaga taaagtggtg gtcttttgga tgagggaata atgcattttt aaataaaata
6901 ccaatatcag gaagccattt tttatttcag gaaatgtaag aaaccattat ttcaggttat
6961 gaaagtataa ccaagcatcc ttttgggcaa ttccttacca aatgcagaag cttttctgtt
7021 cgatgcactc tttcctcctt gccacttacc tttgcaaagt taaaaaaaag gggggaggga
7081 atgggagaga aagctgagat ttcagtttcc tactgcagtt tcctacctgc agatccaggg
7141 gctgctgttg cctttggatg ccccactgag gtcctagagt gcctccaggg tggtcttcct
7201 gtagtcataa cagctagcca gtgctcacca gcttaccaga ttgccaggac taagccatcc
7261 caaagcacaa gcattgtgtg tctctgtgac tgcagagaag agagaatttt gcttctgttt
7321 tgtgtttaaa aaaccaacac ggaagcagat gatcccgaga gagaggcctc tagcatgggt
7381 gacccagccg acctcaggcc ggtttccgca ctgccacaac tttgttcaaa gttgccccca
7441 attggaacct gccacttggc attagagggt ctttcatggg gagagaagga gactgaatta
7501 ctctaagcaa aatgtgaaaa gtaaggaaat cagcctttca tcccggtcct aagtaaccgt
7561 cagccgaagg tctcgtggaa cacaggcaaa cccgtgattt tggtgctcct tgtaactcag
7621 ccctgcaaag caaagtccca ttgatttaag ttgtttgcat ttgtactggc aaggcaaaat
7681 atttttatta ccttttctat tacttattgt atgagctttt gttgtttact tggaggtttt
7741 gtcttttact acaagtttgg aactatttat tattgcttgg tatttgtgct ctgtttaaga
7801 aacaggcact tttttttatt atggataaaa tgttgagatg acaggaggtc atttcaatat
7861 ggcttagtaa aatatttatt gttcctttat tctctgtaca agattttggg cctctttttt
7921 tccttaatgt cacaatgttg agttcagcat gtgtctgcca tttcatttgt acgcttgttc
7981 aaaaccaagt ttgttctggt ttcaagttat aaaaataaat tggacattta acttgatctc
8041 caaa
**ITM2A (SEQ ID NO: 28)** - Homo sapiens integral membrane protein 2A (ITM2A), transcript variant 1, mRNA - NM_004367 
1 gtgcaggtga ggcacgttta gcctgagccg gccacggact ccacttcccc tgctcttccc
61 ccagtggcaa atccgcgcca cctcgcaaac ccccaactca ggcacttggg ccccttttgg
121 gccccctctc gctcctccct ttaggcacct ccctgggccc gcccacggtc tccccccagt
181 ttgggactgc gtcataagta tcccagacct cggcttgcag tagtgttaga ctgaagataa
241 agtaagtgct gtttgggcta acaggatctc ctcttgcagt ctgcagccca ggacgctgat
301 tccagcagcg ccttaccgcg cagcccgaag attcactatg gtgaaaatcg ccttcaatac
361 ccctaccgcc gtgcaaaagg aggaggcgcg gcaagacgtg gaggccctcc tgagccgcac
421 ggtcagaact cagatactga ccggcaagga gctccgagtt gccacccagg aaaaagaggg
481 ctcctctggg agatgtatgc ttactctctt aggcctttca ttcatcttgg caggacttat
541 tgttggtgga gcctgcattt acaagtactt catgcccaag agcaccattt accgtggaga
601 gatgtgcttt tttgattctg aggatcctgc aaattccctt cgtggaggag agcctaactt
661 cctgcctgtg actgaggagg ctgacattcg tgaggatgac aacattgcaa tcattgatgt
721 gcctgtcccc agtttctctg atagtgaccc tgcagcaatt attcatgact ttgaaaaggg
781 aatgactgct tacctggact tgttgctggg gaactgctat ctgatgcccc tcaatacttc
841 tattgttatg cctccaaaaa atctggtaga gctctttggc aaactggcga gtggcagata
901 tctgcctcaa acttatgtgg ttcgagaaga cctagttgct gtggaggaaa ttcgtgatgt
961 tagtaacctt ggcatcttta tttaccaact ttgcaataac agaaagtcct tccgccttcg
1021 tcgcagagac ctcttgctgg gtttcaacaa acgtgccatt gataaatgct ggaagattag
1081 acacttcccc aacgaattta ttgttgagac caagatctgt caagagtaag aggcaacaga
1141 tagagtgtcc ttggtaataa gaagtcagag atttacaata tgactttaac attaaggttt
1201 atgggatact caagatattt actcatgcat ttactctatt gcttatgctt taaaaaaagg
1261 aaaaaaaaaa actactaacc actgcaagct cttgtcaaat tttagtttaa ttggcattgc
1321 ttgttttttg aaactgaaat tacatgagtt tcattttttc tttgaattta tagggtttag
1381 atttctgaaa gcagcatgaa tatatcacct aacatcctga caataaattc catccgttgt
1441 tttttttgtt tgtttgtttt ttcttttcct ttaagtaagc tctttattca tcttatggtg
1501 gagcaatttt aaaatttgaa atattttaaa ttgtttttga actttttgtg taaaatatat
1561 cagatctcaa cattgttggt ttcttttgtt tttcattttg tacaactttc ttgaatttag
1621 aaattacatc tttgcagttc tgttaggtgc tctgtaatta acctgactta tatgtgaaca
1681 attttcatga gacagtcatt tttaactaat gcagtgattc tttctcacta ctatctgtat
1741 tgtggaatgc acaaaattgt gtaggtgctg aatgctgtaa ggagtttagg ttgtatgaat
1801 tctacaaccc tataataaat tttactctat acaaaaaaaa aaaaaaaaaa a
**SLAMF6 (SEQ ID NO: 29)** - Homo sapiens SLAM family member 6 (SLAMF6), transcript variant 2, mRNA - NM 052931 
1 agtttatgac agaagggcaa aaacattgac tgcctcaagg tctcaagcac cagtcttcac
61 cgcggaaagc atgttgtggc tgttccaatc gctcctgttt gtcttctgct ttggcccagg
121 gaatgtagtt tcacaaagca gcttaacccc attgatggtg aacgggattc tgggggagtc
181 agtaactctt cccctggagt ttcctgcagg agagaaggtc aacttcatca cttggctttt
241 caatgaaaca tctcttgcct tcatagtacc ccatgaaacc aaaagtccag aaatccacgt
301 gactaatccg aaacagggaa agcgactgaa cttcacccag tcctactccc tgcaactcag
361 caacctgaag atggaagaca caggctctta cagagcccag atatccacaa agacctctgc
421 aaagctgtcc agttacactc tgaggatatt aagacaactg aggaacatac aagttaccaa
481 tcacagtcag ctatttcaga atatgacctg tgagctccat ctgacttgct ctgtggagga
541 tgcagatgac aatgtctcat tcagatggga ggccttggga aacacacttt caagtcagcc
601 aaacctcact gtctcctggg accccaggat ttccagtgaa caggactaca cctgcatagc
661 agagaatgct gtcagtaatt tatccttctc tgtctctgcc cagaagcttt gcgaagatgt
721 taaaattcaa tatacagata ccaaaatgat tctgtttatg gtttctggga tatgcatagt
781 cttcggtttc atcatactgc tgttacttgt tttgaggaaa agaagagatt ccctatcttt
841 gtctactcag cgaacacagg gccccgagtc cgcaaggaac ctagagtatg tttcagtgtc
901 tccaacgaac aacactgtgt atgcttcagt cactcattca aacagggaaa cagaaatctg
961 gacacctaga gaaaatgata ctatcacaat ttactccaca attaatcatt ccaaagagag
1021 taaacccact ttttccaggg caactgccct tgacaatgtc gtgtaagttg ctgaaaggcc
 
1081 tcagaggaat tcgggaatga cacgtcttct gatcccatga gacagaacaa agaacaggaa
1141 gcttggttcc tgttgttcct ggcaacagaa tttgaatatc taggatagga tgatcacctc
1201 cagtccttcg gacttaaacc tgcctacctg agtcaaacac ctaaggataa catcatttcc
1261 agcatgtggt tcaaataata ttttccaatc cacttcaggc caaaacatgc taaagataac
1321 acaccagcac attgactctc tctttgataa ctaagcaaat ggaattatgg ttgacagaga
1381 gtttatgatc cagaagacaa ccacttctct ccttttagaa agcagcagga ttgacttatt
1441 gagaaataat gcagtgtgtt ggttacatgt gtagtctctg gagttggatg ggcccatcct
1501 gatacaagtt gagcatccct tgtctgaaat gcttgggatt agaaatgttt cagatttcaa
1561 ttttttttca gattttggaa tatttgcatt atatttagcg gttgagtatc caaatccaaa
1621 aatccaaaat tcaaaatgct ccaataagca tttcccttga gtttcattga tgtcgatgca
1681 gtgctcaaaa tctcagattt tggagcattt tggatattgg atttttggat ttgggatgct
1741 caacttgtac aatgtttatt agacacatct cctgggacat actgcctaac cttttggagc
1801 cttagtctcc cagactgaaa aaggaagagg atggtattac atcagctcca ttgtttgagc
1861 caagaatcta agtcatccct gactccagtg tctttgtcac caggcccttt ggactctacc
1921 tcagaaatat ttcttggacc ttccacttct cctccaactc cttgaccacc atcctgtatc
1981 caaccatcac cacctctaac ctgaatccta ccttaagatc agaacagttg tcctcacttt
2041 tgttcttgtc cctctccaac ccactctcca caagatggcc agagtaatgt ttttaatata
2101 aattggatcc ttcagtttcc tgcttaaaac cctgcaggtt tcccaatgca ctcagaaaga
2161 aatccagttt ccatggccct ggatggtctg gcccacctcc agcctcagct agcattaccc
2221 ttctgacact ctctatgtag cctccctgat cttctttcag ctcctctatt aaaggaaaag
2281 ttctttatgt taattattta catcttcctg caggcccttc ctctgcctgc tggggtcctc
2341 ctattcttta ggtttaattt taaatatgtc acctcctaag agaaaccttc ccagaccact
2401 ctttctaaaa tgaatcttct aggctgggca tggtggctca cacctgtaat ccctgtactt
2461 tgggaggcca aggggggaga tcacttgagg tcaggagttc aagaccagcc tggccaactt
2521 ggtgaaaccc cgtctttact aaaaatacaa aaaaattagc caggcgtggt ggtgcacccc
2581 taaaatccca gctacttgag agactgaggc aggagaatcg cttgaaccca ggaggtggag
2641 gttccagtga gccaaaatca tgccaatgta ttccagtctg ggtgacagag tgagactctg
2701 tctcaaaaaa taaataaata aaataaaatg aaatagatct tataaaaaaa a
**HLA-DPB1 (SEQ ID NO: 30)** - Homo sapiens major histocompatibility complex, class II, DP beta 1, mRNA - NM 002121 
1 gtcacagaag actacttggg ttcatggtct ctaatatttc aaacaggagc tccctttagc
61 gagtccttct tttcctgact gcagctcttt tcattttgcc atccttttcc agctccatga
121 tggttctgca ggtttctgcg gcccccggga cagtggctct gacggcgtta ctgatggtgc
181 tgctcacatc tgtggtccag ggcagggcca ctccagagaa ttaccttttc cagggacggc
241 aggaatgcta cgcgtttaat gggacacagc gcttcctgga gagatacatc tacaaccggg
301 aggagttcgc gcgcttcgac agcgacgtgg gggagttccg ggcggtgacg gagctggggc
361 ggcctgctgc ggagtactgg aacagccaga aggacatcct ggaggagaag cgggcagtgc
421 cggacaggat gtgcagacac aactacgagc tgggcgggcc catgaccctg cagcgccgag
481 tccagcctag ggtgaatgtt tccccctcca agaaggggcc cttgcagcac cacaacctgc
541 ttgtctgcca cgtgacggat ttctacccag gcagcattca agtccgatgg ttcctgaatg
601 gacaggagga aacagctggg gtcgtgtcca ccaacctgat ccgtaatgga gactggacct
661 tccagatcct ggtgatgctg gaaatgaccc cccagcaggg agatgtctac acctgccaag
721 tggagcacac cagcctggat agtcctgtca ccgtggagtg gaaggcacag tctgattctg
781 cccggagtaa gacattgacg ggagctgggg gcttcgtgct ggggctcatc atctgtggag
841 tgggcatctt catgcacagg aggagcaaga aagttcaacg aggatctgca taaacagggt
901 tcctgagctc actgaaaaga ctattgtgcc ttaggaaaag catttgctgt gtttcgttag
961 catctggctc caggacagac cttcaacttc caaattggat actgctgcca agaagttgct
1021 ctgaagtcag tttctatcat tctgctcttt gattcaaagc actgtttctc tcactgggcc
1081 tccaaccatg ttcccttctt cttagcacca caaataatca aaacccaaca tgactgtttg
1141 ttttccttta aaaatatgca ccaaatcatc tctcatcact tttctctgag ggttttagta
1201 gacagtagga gttaataaag aagttcattt tggtttaaac ataggaaaga agagaaccat
1261 gaaaatgggg atatgttaac tattgtataa tggggcctgt tacacatgac actcttctga
1321 attgactgta tttcagtgag ctgcccccaa atcaagttta gtgccctcat ccatttatgt
1381 ctcagaccac tattcttaac tattcaatgg tgagcagact gcaaatctgc ctgataggac
 
1441 ccatattccc acagcactaa ttcaacatat accttactga gagcatgttt tatcattacc
1501 attaagaagt taaatgaaca tcagaattta aaatcataaa tataatctaa tacactttaa
1561 ccatttcttt tgtgtgccat cacaaatact ccttaaccaa atacggcttg gacttttgaa
1621 tgcatccaat agacgtcatt tgtcgtctaa gtctgcattc atccaccagc ctaggcctcc
1681 tgtcttaatt ttcatacaga cagaaatgac tccccactgg ggaaagagca aagcaataca
1741 tgtagcactc tttttcaaac actggtcttt ttttttttct taacaatcca acattgttat
1801 gtgttttgcg tctcatattg acaccttttg gtcaaggtag aggacatgtt tgttgtaagc
1861 tttctttttc gtgtagagga tggattcttc actcctgata cacacaatca gtgcacagca
1921 gctctcttat acatccagtt gatgccttca gtctccctgg cttcttacaa gcatcttctg
1981 ggccttgtgt gtccctgggc acctgtccct ggtcaattcc cgaaagctac tgtgctcctc
2041 ttgcccatct ccccttgcaa ataatatctt ccatcggggg accggcttcc tccaatttca
2101 ggagaggtgg ggctgaaggc acagacttgg gcgtcactgg cacagatata agtaaataca
2161 gctggagtct gcagagaggc tggactgagt cagggagtca ggaaagagaa gccacacaca
2221 aggacaacca atcatgtttc tcataatctt cttaacctag ggaataggac acaatcattt
2281 tttcttttta aaacatcttt atccctgatc agcctcattt cctcaaaaac tataaaggaa
2341 aatgctgctg acttgttttt gcgtagtaat ttcagctgtc acataataag ctaaggaaga
2401 cagtatatag taaataagga ccctttatct gtcttatttt cccttttggc ttcacaggaa
2461 acttgtgaga aacctatgca gcataaaatt aatatgattt caatccaggg attcaacgat
2521 ggaaggaggt catgagaata gcagaaagtc ttcaaatcga gatcattatg aaatcctcag
2581 acccagagca cataaatcct accctcagag tcactgagca gttaacatta caaattacaa
2641 accatatcca gtcagagtca ttctctttcc tgcttgtctc ctgtactcat gttacaggtt
2701 agggcagtac cccgagtgga gtgaacaatc tctggactaa cacttgtcag gatcagaagc
2761 tgaggtatct gcacccacat tacaggaaca ggatatgtgc tcctagggaa ctgagggtgt
2821 caggagatga ggaatgtccc tggagtcaca gaaagaaggt atcagatgtg tctcactctg
2881 acatatgcag gtgtttatga aactctggga tttctaagga aggatgcagt gcagagacag
2941 gtcccagagg agacaagagc tgagagacca tccaaactgg gaccaccttg tcactagact
3001 tcaaattttc aatattgata gagtgttttc taagagtcag gccctttgct gagtgctatg
3061 tgcagcagga tcaaaggcag ccaggaggta gaggagtctt gaggtacatc agtcattgga
3121 gttgaagagc agagattcaa aggaaagttg gaactggagc tttaaaggag atgtgaagtg
3181 ggtgactcaa cctctgactc agaaaaattg atacctgcag aagaaaaaac ccggcgggct
3241 taggactccc agctgagtgt tgtatcctcc atccctttcc acctggtccc ttcattttct
3301 acccctcaca gttccctaac gagaaggtgg tccacccaac agacaacact gcctcagatg
3361 gttatcaagg ggtaccctaa gaagaaatca tctcaccctc tctttgtccc catttgtcaa
3421 gtagcagtga ggccgagcca ggggatggtg aaagtggaag gaggtgggag ttgggcatcg
3481 ggtgtgaaga tgctcttgaa aggggtttta ataaccactt gctaccaggc cagtgaacac
3541 ttaccatagt tgatgccttt tgagcatgtt gcattgtaaa ctgtccctga aattactgtg
3601 cacttggctt atgggatgaa acatcctcct agttcttttg tctctcagct tctctgaagt
3661 ctcattgagc accttctctt caatttcttt tacacagtaa gaataggatc agctgtgcta
3721 aactaacaaa tacccagata tccaggtttg gctcatgtta cacgtccaaa gtaagtcatg
3781 caggaagctc tgctcatcat cgtactcagg aagtcaggct gacagtcttt ctcctgcaca
3841 tctgctccca gaacctcccc agcagaatga agggaaccta agaatttatt cactggcttt
3901 taatgatccc tcctagaaag aacacacttc tcgcatttca ttttccaatg taaatcatat
3961 ggctgcaact aacttcaaat aagtgggaat acttgaaggt ggaaaacatt taagaagtac
4021 acactaaata aataataaaa tacttctaca agaga
**HLA-DPB1 (SEQ ID NO: 31)** - Homo sapiens major histocompatibility complex, class II, DP beta 1 (HLA-DPB1), transcript variant X1, mRNA - XM_006725998 
1 atcactcagt gcccctgagc tcattctttt cagtaaattc tctctctgcg tggtgagaaa
61 acaggcctgg agaggctctg cgacccgctt aggaccacag aactcgagaa ttaccttttc
121 cagggacggc aggaatgcta cgcgtttaat gggacacagc gcttcctgga gagatacatc
181 tacaaccggg aggagctcgt gcgcttcgac agcgacgtgg gggagttccg ggcggtgacg
241 gagctggggc ggcctgaggc ggagtactgg aacagccaga aggacatcct ggaggaggag
301 cgggcagtgc cggacaggat gtgcagacac aactacgagc tgggcggccc catgaccctg
361 cagcgccgag tccagcctag ggtgaatgtt tccccctcca agaaggggcc cttgcagcac
421 cacaacctgc ttgtctgcca cgtgacggat ttctacccag gcagcattca agtccgatgg
 
481 ttcctgaatg gacaggagga aacagctggg gtcgtgtcca ccaacctgat ccgtaatgga
541 gactggacct tccagatcct ggtgatgctg gaaatgaccc cccagcaggg agatgtctac
601 acctgccaag tggagcacac cagcctggat agtcctgtca ccgtggagtg gaaggcacag
661 tctgattctg cccggagtaa gacattgacg ggagctgggg gcttcgtgct ggggctcatc
721 atctgtggag tgggcatctt catgcacagg aggagcaaga aagttcaacg aggatctgca
781 taaacagggt tcctgagctc actgaaaaga ctattgtgcc ttaggaaaag catttgctgt
841 gtttcgttag catctggctc caggacagac cttcaacttc caaattggat actgctgcca
901 agaagttgct ctgaagtcag tttctatcat tctgctcttt gattcaaagc actgtttctc
961 tcactgggcc tccaaccatg ttcccttctt cttagcacca caaataatca aaacccaaca
1021 tgactgtttg ttttccttta aaaatatgca ccaaatcatc tctca
**CD160 (SEQ ID NO: 32)** - CD160 molecule, transcript variant 1, mRNA - NM 007053 
1 gacgaaactg gagagatagg gttttaacaa gatgcaagga caatctgagg actgagagcc
61 atttcaacgt gagcccccag tctgagaaca agaaagaaga acttctgtct cgagggtctc
121 actgtcaacc aggccagagt gcagtgaaga tcatacctca ctacatccgt gaactcccgg
181 gctcctccca cctaagtctc ttgagtagct gggacttcag gagactgaag ccaaggatac
241 cagcagagcc aacatttgct tcaagttcct gggcctgctg acagcgtgca ggatgctgtt
301 ggaacccggc agaggctgct gtgccctggc catcctgctg gcaattgtgg acatccagtc
361 tggtggatgc attaacatca ccagctcagc ttcccaggaa ggaacgcgac taaacttaat
421 ctgtactgta tggcataaga aagaagaggc tgaggggttt gtagtgtttt tgtgcaagga
481 caggtctgga gactgttctc ctgagaccag tttaaaacag ctgagactta aaagggatcc
541 tgggatagat ggtgttggtg aaatatcatc tcagttgatg ttcaccataa gccaagtcac
601 accgttgcac agtgggacct accagtgttg tgccagaagc cagaagtcag gtatccgcct
661 tcagggccat tttttctcca ttctattcac agagacaggg aactacacag tgacgggatt
721 gaaacaaaga caacaccttg agttcagcca taatgaaggc actctcagtt caggcttcct
781 acaagaaaag gtctgggtaa tgctggtcac cagccttgtg gcccttcaag ctttgtaagc
841 cttgtgccaa aagaaacttt taaaacagct acagcaagat gagtctgact atggcttagt
901 atctttctca ttacaatagg cacagagaag aatgcaacag ggcacagggg aagagatgct
961 aaatatacca agaatctgtg gaaatataag ctggggcaaa tcagtgtaat ccttgacttt
1021 gctcctcacc atcagggcaa acttgccttc ttccctccta agctccagta aataaacaga
1081 acagctttca ccaaagtggg tagtatagtc ctcaaatatc ggataaatat atgcgttttt
1141 gtaccccaga aaaacttttc ctccctcttc atcaacatag taaaataagt caaacaaaat
1201 gagaacacca aattttgggg gaataaattt ttatttaaca ctgcaaagga aagagagaga
1261 aaacaagcaa agataggtag gacagaaagg aagacagcca gatccagtga ttgacttggc
1321 atgaaaatga gaaaatgcag acagacctca acattcaaca acatccatac agcactgctg
1381 gaggaagagg aagatttgtg cagaccaaga gcaccacaga ctacaactgc ccagcttcat
1441 ctaaatactt gttaacctct ttggtcattt ctctttttaa ataaatgccc atagcagtat
1501 ttggagtctt ttcttttctc ctaaatccac aaactctctt ctttctcttt ggacagatga
1561 cctcttgtca tagttaagca gagagtgggc aggatattcc tgataggagg aactacatga
1621 ataaaggggt aag
**KLRF1 (SEQ ID NO: 33)** - Homo sapiens killer cell lectin like receptor F1 (KLRF1), transcript variant 1, mRNA - NM 016523 
1 atttcatgtt atacttaata aaacaaaaca tacctgtata cacacacatt cactcacatt
61 gaagatgcaa gatgaagaaa gatacatgac attgaatgta cagtcaaaga aaaggagttc
121 tgcccaaaca tctcaactta catttaaaga ttattcagtg acgttgcact ggtataaaat
181 cttactggga atatctggaa ccgtgaatgg tattctcact ttgactttga tctccttgat
241 cctgttggtt tctcagggag tattgctaaa atgccaaaaa ggaagttgtt caaatgccac
301 tcagtatgag gacactggag atctaaaagt gaataatggc acaagaagaa atataagtaa
361 taaggacctt tgtgcttcga gatctgcaga ccagacagta ctatgccaat cagaatggct
421 caaataccaa gggaagtgtt attggttctc taatgagatg aaaagctgga gtgacagtta
481 tgtgtattgt ttggaaagaa aatctcatct actaatcata catgaccaac ttgaaatggc
541 ttttatacag aaaaacctaa gacaattaaa ctacgtatgg attgggctta actttacctc
601 cttgaaaatg acatggactt gggtggatgg ttctccaata gattcaaaga tattcttcat
 
661 aaagggacca gctaaagaaa acagctgtgc tgccattaag gaaagcaaaa ttttctctga
721 aacctgcagc agtgttttca aatggatttg tcagtattag agtttgacaa aattcacagt
781 gaaataatca atgatcacta tttttggcct attagtttct aatattaatc tccaggtgta
841 agattttaaa gtgcaattaa atgccaaaat ctcttctccc ttctccctcc atcatcgaca
901 ctggtctagc ctcagagtaa cccctgttaa caaactaaaa tgtacacttc aaaattttta
961 cgtgatagta taaaccaatg tgacttcatg tgatcatatc caggattttt attcgtcgct
1021 tattttatgc caaatgtgat caaattatgc ctgtttttct gtatcttgcg ttttaaattc
1081 ttaataaggt cctaaacaaa atttcttata tttctaatgg ttgaattata atgtgggttt
1141 atacattttt tacccttttg tcaaagagaa ttaactttgt ttccaggctt ttgctactct
1201 tcactcagct acaataaaca tcctgaatgt tttcttaaaa aaaaaaaaaa aaaaaaaaaa
1261 aaaaaa
**CD2 (SEQ ID NO: 34)** CD2 molecule, NM 001767.3 
1 agaatcaaaa gaggaaacca acccctaaga tgagctttcc atgtaaattt gtagccagct
61 tccttctgat tttcaatgtt tcttccaaag gtgcagtctc caaagagatt acgaatgcct
121 tggaaacctg gggtgccttg ggtcaggaca tcaacttgga cattcctagt tttcaaatga
181 gtgatgatat tgacgatata aaatgggaaa aaacttcaga caagaaaaag attgcacaat
241 tcagaaaaga gaaagagact ttcaaggaaa aagatacata taagctattt aaaaatggaa
301 ctctgaaaat taagcatctg aagaccgatg atcaggatat ctacaaggta tcaatatatg
361 atacaaaagg aaaaaatgtg ttggaaaaaa tatttgattt gaagattcaa gagagggtct
421 caaaaccaaa gatctcctgg acttgtatca acacaaccct gacctgtgag gtaatgaatg
481 gaactgaccc cgaattaaac ctgtatcaag atgggaaaca tctaaaactt tctcagaggg
541 tcatcacaca caagtggacc accagcctga gtgcaaaatt caagtgcaca gcagggaaca
601 aagtcagcaa ggaatccagt gtcgagcctg tcagctgtcc agagaaaggt ctggacatct
661 atctcatcat tggcatatgt ggaggaggca gcctcttgat ggtctttgtg gcactgctcg
721 ttttctatat caccaaaagg aaaaaacaga ggagtcggag aaatgatgag gagctggaga
781 caagagccca cagagtagct actgaagaaa ggggccggaa gccccaccaa attccagctt
841 caacccctca gaatccagca acttcccaac atcctcctcc accacctggt catcgttccc
901 aggcacctag tcatcgtccc ccgcctcctg gacaccgtgt tcagcaccag cctcagaaga
961 ggcctcctgc tccgtcgggc acacaagttc accagcagaa aggcccgccc ctccccagac
1021 ctcgagttca gccaaaacct ccccatgggg cagcagaaaa ctcattgtcc ccttcctcta
1081 attaaaaaag atagaaactg tctttttcaa taaaaagcac tgtggatttc tgccctcctg
1141 atgtgcatat ccgtacttcc atgaggtgtt ttctgtgtgc agaacattgt cacctcctga
1201 ggctgtgggc cacagccacc tctgcatctt cgaactcagc catgtggtca acatctggag
1261 tttttggtct cctcagagag ctccatcaca ccagtaagga gaagcaatat aagtgtgatt
1321 gcaagaatgg tagaggaccg agcacagaaa tcttagagat ttcttgtccc ctctcaggtc
1381 atgtgtagat gcgataaatc aagtgattgg tgtgcctggg tctcactaca agcagcctat
1441 ctgcttaaga gactctggag tttcttatgt gccctggtgg acacttgccc accatcctgt
1501 gagtaaaagt gaaataaaag ctttgactag aaaaaaaaaa aaaaaaaaaa aaaaaaaaaa
1561 aaaaaaaaaa aaaaaaaaaa aaaaaaaaaa aaaaa
**LGALS2 (SEQ ID NO: 35)** - Homo sapiens lectin, galactoside-binding, soluble, 2 (LGALS2), mRNA NM 006498 
1 gaccttgagg gagttaatgt gtaatattct aggatataag cttgaccacg agttgagacc
61 ctgagcacag gcctccagga gccgctggga gctgccgcca ggagctgtca ccatgacggg
121 ggaacttgag gttaagaaca tggacatgaa gccggggtca accctgaaga tcacaggcag
181 catcgccgat ggcactgatg gctttgtaat taatctgggc caggggacag acaagctgaa
241 cctgcatttc aaccctcgct tcagcgaatc caccattgtc tgcaactcat tggacggcag
301 caactggggg caagaacaac gggaagatca cctgtgcttc agcccagggt cagaggtcaa
361 gttcacagtg acctttgaga gtgacaaatt caaggtgaag ctgccagatg ggcacgagct
421 gacttttccc aacaggctgg gtcacagcca cctgagctac ctgagcgtaa ggggcgggtt
481 caacatgtcc tctttcaagt taaaagaata aaagacttcc agccgagaaa aaaaaaaaaa
541 aaa
**NPPC (SEQ ID NO: 36)** - Homo sapiens natriuretic peptide C, mRNA - NM 024409 
1 cgggctcaga gcgcacccag ccggcgccgc gcagcactgg gaccctgctc gccctgcagc
61 ccagccagcc tgctccgcat ccccctgctg gtctgcccgc cgacctgcgc gccctcgctg
121 ccgcccgtgt gcgcccctcg accccagcgg caccatgcat ctctcccagc tgctggcctg
181 cgccctgctg ctcacgctgc tctccctccg gccctccgaa gccaagcccg gggcgccgcc
241 gaaggtcccg cgaaccccgc cggcagagga gctggccgag ccgcaggctg cgggcggcgg
301 tcagaagaag ggcgacaagg ctcccggggg cgggggcgcc aatctcaagg gcgaccggtc
361 gcgactgctc cgggacctgc gcgtggacac caagtcgcgg gcagcgtggg ctcgccttct
421 gcaagagcac cccaacgcgc gcaaatacaa aggagccaac aagaagggct tgtccaaggg
481 ctgcttcggc ctcaagctgg accgaatcgg ctccatgagc ggcctgggat gttagtgcgg
541 cgccccctgg cggcggatcg ggaactggct ccgttgtgct gaggtcatct ttggtcatca
601 gcctccagca tctggaaaca cctccaacgc aatgtggctt ttacatttct ttctttcttt
661 cttttttttt cctggtactg ggaatacaca acaccagctg ttttattatt atttggggag
721 ggggttgtga ttttattatt tgttttttta aaatgaaaaa taaaaagtta tatatta
**MYCL (SEQ ID NO: 37)** - Homo sapiens v-myc avian myelocytomatosis viral oncogene lung carcinoma derived homolog (MYCL), transcript variant 1, mRNA - NM 001033081 
1 aatgcgcctg cagctcgcgc tcccgcgccg atcccgagag cgtccgggcc gccgtgcgcg
61 agcgagggag ggcgcgcgcg cggggggggc gcgcttgtga gtgcgggccg cgctctcggc
121 ggcgcgcatg tgcgtgtgtg ctggctgccg ggctgccccg agccggcggg gagccggtcc
181 gctccaggtg gcgggcggct ggagcgaggt gaggctgcgg gtggccaggg cacgggcgcg
241 ggtcccgcgg tgcgggctgg ctgcaggctg ccttctgggc acggcgcgcc cccgcccggc
301 cccgccgggc cctgggagct gcgctccggg cggcgctggc aaagtttgct ttgaactcgc
361 tgcccacagt cgggtccgcg cgctgcgatt ggcttcccct accactctga cccggggccc
421 ggcttcccgg gacgcgagga ctgggcgcag gctgcaagct ggtggggttg gggaggaacg
481 agagcccggc agccgactgt gccgagggac ccggggacac ctccttcgcc cggccggcac
541 ccggtcagca cgtcccccct tccctcccgc agggagcgga catggactac gactcgtacc
601 agcactattt ctacgactat gactgcgggg aggatttcta ccgctccacg gcgcccagcg
661 aggacatctg gaagaaattc gagctggtgc catcgccccc cacgtcgccg ccctggggct
721 tgggtcccgg cgcaggggac ccggcccccg ggattggtcc cccggagccg tggcccggag
781 ggtgcaccgg agacgaagcg gaatcccggg gccactcgaa aggctggggc aggaactacg
841 cctccatcat acgccgtgac tgcatgtgga gcggcttctc ggcccgggaa cggctggaga
901 gagctgtgag cgaccggctc gctcctggcg cgccccgggg gaacccgccc aaggcgtccg
961 ccgccccgga ctgcactccc agcctcgaag ccggcaaccc ggcgcccgcc gccccctgtc
1021 cgctgggcga acccaagacc caggcctgct ccgggtccga gagcccaagc gactcggaga
1081 atgaagaaat tgatgttgtg acagtagaga agaggcagtc tctgggtatt cggaagccgg
1141 tcaccatcac ggtgcgagca gaccccctgg atccctgcat gaagcatttc cacatctcca
1201 tccatcagca acagcacaac tatgctgccc gttttcctcc agaaagctgc tcccaagaag
1261 aggcttcaga gaggggtccc caagaagagg ttctggagag agatgctgca ggggaaaagg
1321 aagatgagga ggatgaagag attgtgagtc ccccacctgt agaaagtgag gctgcccagt
1381 cctgccaccc caaacctgtc agttctgata ctgaggatgt gaccaagagg aagaatcaca
1441 acttcctgga gcgcaagagg cggaatgacc tgcgttcgcg attcttggcg ctgagggacc
1501 aggtgcccac cctggccagc tgctccaagg cccccaaagt agtgatccta agcaaggcct
1561 tggaatactt gcaagccctg gtgggggctg agaagaggat ggctacagag aaaagacagc
1621 tccgatgccg gcagcagcag ttgcagaaaa gaattgcata cctcactggc tactaactga
1681 ccaaaaagcc tgacagttct gtcttacgaa gacacaagtt tattttttaa cctccctctc
1741 ccctttagta atttgcacat tttggttatg gtgggacagt ctggacagta gatcccagaa
1801 tgcattgcag ccggtgcaca cacaataaag gcttgcattc ttggaaacct tgaaacccag
1861 ctctccctct tccctgactc atgggagtgc tgtatgttct ctggcgcctt tggcttccca
1921 gcaggcagct gactgaggag ccttggggtc tgcctagctc actagctctg aagaaaaggc
1981 tgacagatgc tatgcaacag gtggtggatg ttgtcagggg ctccagcctg catgaaatct
2041 cacactctgc atgagcttta ggctaggaaa ggatgctccc aactggtgtc tctggggtga
2101 tgcaaggaca gctgggcctg gatgctctcc ctgaggctcc tttttccaga agacacacga
2161 gctgtcttgg gtgaagacaa gcttgcagac ttgatcaaca ttgaccatta cctcactgtc
 
2221 agacacttta cagtagccaa ggagttggaa acctttatat attatgatgt tagctgaccc
2281 ccttcctccc actcccaatg ctgcgaccct gggaacactt aaaaagcttg gcctctagat
2341 tctttgtctc agagccctct gggctctctc ctctgaggga gggacctttc tttcctcaca
2401 agggactttt ttgttccatt atgccttgtt atgcaatggg ctctacagca ccctttccca
2461 caggtcagaa atatttcccc aagacacagg gaaatcggtc ctagcctggg gcctggggat
2521 agcttggagt cctggcccat gaacttgatc cctgcccagg tgttttccga ggggcacttg
2581 aggcccagtc ttttctcaag gcaggtgtaa gacacctcag agggagaact gtactgctgc
2641 ctctttccca cctgcctcat ctcaatcctt gagcggcaag tttgaagttc ttctggaacc
2701 atgcaaatct gtcctcctca tgcaattcca aggagcttgc tggctctgca gccacccttg
2761 ggccccttcc agcctgccat gaatcagata tctttcccag aatctgggcg tttctgaagt
2821 tttggggaga gctgttggga ctcatccagt gctccagaag gtggacttgc ttctggtggg
2881 ttttaaagga gcctccagga gatatgctta gccaaccatg atggatttta ccccagctgg
2941 actcggcagc tccaagtgga atccacgtgc agcttctagt ctgggaaagt cacccaacct
3001 agcagttgtc atgtgggtaa cctcaggcac ctctaagcct gtcctggaag aaggaccagc
3061 agcccctcca gaactctgcc caggacagca ggtgcctgct ggctctgggt ttggaagttg
3121 gggtgggtag ggggtggtaa gtactatata tggctctgga aaaccagctg ctacttccaa
3181 atctattgtc cataatggtt tctttctgag gttgcttctt ggcctcagag gaccccaggg
3241 gatgtttgga aatagcctct ctacccttct ggagcatggt ttacaaaagc cagctgactt
3301 ctggaattgt ctatggagga cagtttgggt gtaggttact gatgtctcaa ctgaatagct
3361 tgtgttttat aagctgctgt tggctattat gctgggggag tctttttttt ttatattgta
3421 tttttgtatg ccttttgcaa agtggtgtta actgtttttg tacaaggaaa aaaactcttg
3481 gggcaatttc ctgttgcaag ggtctgattt attttgaaag gcaagttcac ctgaaatttt
3541 gtatttagtt gtgattactg attgcctgat tttaaaatgt tgccttctgg gacatcttct
3601 aataaaagat ttctcaaaca tgtc
**MYCL (SEQ ID NO: 38)** - Homo sapiens v-myc avian myelocytomatosis viral oncogene lung carcinoma derived homolog (MYCL), transcript variant 3, mRNA - NM 005376 
1 aatgcgcctg cagctcgcgc tcccgcgccg atcccgagag cgtccgggcc gccgtgcgcg
61 agcgagggag ggcgcgcgcg cggggggggc gcgcttgtga gtgcgggccg cgctctcggc
121 ggcgcgcatg tgcgtgtgtg ctggctgccg ggctgccccg agccggcggg gagccggtcc
181 gctccaggtg gcgggcggct ggagcgaggg agcggacatg gactacgact cgtaccagca
241 ctatttctac gactatgact gcggggagga tttctaccgc tccacggcgc ccagcgagga
301 catctggaag aaattcgagc tggtgccatc gccccccacg tcgccgccct ggggcttggg
361 tcccggcgca ggggacccgg cccccgggat tggtcccccg gagccgtggc ccggagggtg
421 caccggagac gaagcggaat cccgggccca ctcgaaaggc tggggcagga actacgcctc
481 catcatacgc cgtgactgca tgtggagcgg cttctcggcc cgggaacggc tggagagagc
541 tgtgagcgac cggctcgctc ctggcgcgcc ccgggggaac ccgcccaagg cgtccgccgc
601 cccggactgc actcccagcc tcgaagccgg caacccggcg cccgccgccc cctgtccgct
661 gggcgaaccc aagacccagg cctgctccgg gtccgagagc ccaagcgact cgggtaagga
721 cctccccgag ccatccaaga gggggccacc ccatgggtgg ccaaagctct gcccctgcct
781 gaggtcaggc attggctctt ctcaagctct tgggccatct ccgcctctct ttggctgaag
841 ctgcccgtgt agtccccaac cgtgtctgtc tggcacgtgg gtgtgttggt aaacagtttg
901 gaaaagtggc gtgggagcca gcctcccttt gatgattatt ggagccccag gggacaaggg
961 atttgaggtg agggttggcg cttagagagg acaatactgg ggttggactg taagggattg
1021 aagggggtac cttaagagac actccaaacc tgaagttttt ttgctgctgc ctctttccct
1081 aggaaactca cactccccta gggggagaag aagccgagag ccttttgtgc aaagccaaaa
1141 ccttcgtcct tttaaaaacc taggtctcca gttggcttta ctttaaaatg ccaataataa
1201 atgccctctt ctcgtgcctc cccaccacca cttaccactc gtgcatccct gagacaggga
1261 gggaagaatg aacactcccc attaacagat ggaaaaactg aggcttagag atagacaatc
1321 actacaagtc agctccagct ttctgccatc tagccagccc ctcttcccca atgctccatc
1381 ccaaccaggc acctcttcct tgatgtttgg ggtctttgtg gtagcttatc ttagaagcac
1441 tacaccttgc cttgctgttt gtcctgagat ggaaaagtgt ccttcttgct ccccctcaat
1501 agatctccag cgtcagctgc tccctggcat tcaacaaata ttcactggcc cctactttgt
1561 ggcaatctgt gggctacatg ctggggtcaa ggcagtagaa ctccaggccc tcctctccca
1621 tccttgatgc aagtgcaacc tcgctgaggg cagactgggg catcctgtgc cactaaacta
 
1681 cattgttctt attctggcat cttagacctc cacacccgtg agaaatcctg gagagggtat
1741 ttttgtagag tgtagactgt ggctagtgac aaataaatta ggaccaagaa agctcactgt
1801 agcttttagg aataactttt acacgaccat ttgataggga actggggaat ggggtatgga
1861 agttttccta cacttgagag aaaaaatagg ataacaaaaa ttaaaagtct tttttttcct
1921 ggtccactgt gttaaggtca tttttaacca gcttgctttc tacaccaaga gtttatgttt
1981 gtttaatggc tggaaagaga atcttgagat caaaaaacca ataaagatgt atctctacaa
2041 aaaaaaaaaa
**MX1 (SEQ ID NO: 39)** MX dynamin like GTPase 1, transcript variant 1, NM 001144925.2 
1 cgagcagaaa tgaaaccgaa actgaattgt ccgggaaatt cgcggtgggg gcggagagcg
61 cagggagaag taagcccagt gcaggatcct gaggcccgtg tttgcaggac cagggccggc
121 cttccgattc cccattcatt ccagaagcac cgaaccacgc tgtgcccgga tcccaagtgc
181 agcggcaccc agcgtgggcc tggggttgcc ggttgacccg gtcctcagcc tggtagcaga
241 ggccaggcca gtgccacaag gcacctaagt ccacctgggc ctggagcagg acaggttgca
301 aaagaaaata tctcgggacc cccaaactcc ttatgctaag ggaaacatcg agcctgggaa
361 ctgagccatc aacgctgcca ttctttttcc caaacagaac cctgttgtca gaggtacacc
421 cagagcaact ccacaccggg tgcatgccac agcaactcca tcttaaatag gagctggtaa
481 aacgaggctg atacctactg ggctgcattc ccagacggca tagcgaggag gtgctgaaga
541 gcgcaggttt ggagaatgat cacctggatt ggaaccatag ctctaccaat atggaaccca
601 gctccttagg cctcggtctt ctcatggaga acatggtgtg ataatcctac tcctctggga
661 gggtggctgt taagccttgg accgcagttg ccggccagga atcccagtgt cacggtggac
721 acgcctccct cgcgcccttg ccgcccacct gctcacccag ctcaggggct ttggaattct
781 gtggccacac tgcgaggaga tcggttctgg gtcggaggct acaggaagac tcccactccc
841 tgaaatctgg agtgaagaac gccgccatcc agccaccatt ccaaggaggt gcaggagaac
901 agctctgtga taccatttaa cttgttgaca ttacttttat ttgaaggaac gtatattaga
961 gcttactttg caaagaagga agatggttgt ttccgaagtg gacatcgcaa aagctgatcc
1021 agctgctgca tcccaccctc tattactgaa tggagatgct actgtggccc agaaaaatcc
1081 aggctcggtg gctgagaaca acctgtgcag ccagtatgag gagaaggtgc gcccctgcat
1141 cgacctcatt gactccctgc gggctctagg tgtggagcag gacctggccc tgccagccat
1201 cgccgtcatc ggggaccaga gctcgggcaa gagctccgtg ttggaggcac tgtcaggagt
1261 tgcccttccc agaggcagcg ggatcgtgac cagatgcccg ctggtgctga aactgaagaa
1321 acttgtgaac gaagataagt ggagaggcaa ggtcagttac caggactacg agattgagat
1381 ttcggatgct tcagaggtag aaaaggaaat taataaagcc cagaatgcca tcgccgggga
1441 aggaatggga atcagtcatg agctaatcac cctggagatc agctcccgag atgtcccgga
1501 tctgactcta atagaccttc ctggcataac cagagtggct gtgggcaatc agcctgctga
1561 cattgggtat aagatcaaga cactcatcaa gaagtacatc cagaggcagg agacaatcag
1621 cctggtggtg gtccccagta atgtggacat cgccaccaca gaggctctca gcatggccca
1681 ggaggtggac cccgagggag acaggaccat cggaatcttg acgaagcctg atctggtgga
1741 caaaggaact gaagacaagg ttgtggacgt ggtgcggaac ctcgtgttcc acctgaagaa
1801 gggttacatg attgtcaagt gccggggcca gcaggagatc caggaccagc tgagcctgtc
1861 cgaagccctg cagagagaga agatcttctt tgagaaccac ccatatttca gggatctgct
1921 ggaggaagga aaggccacgg ttccctgcct ggcagaaaaa cttaccagcg agctcatcac
1981 acatatctgt aaatctctgc ccctgttaga aaatcaaatc aaggagactc accagagaat
2041 aacagaggag ctacaaaagt atggtgtcga cataccggaa gacgaaaatg aaaaaatgtt
2101 cttcctgata gataaagtta atgcctttaa tcaggacatc actgctctca tgcaaggaga
2161 ggaaactgta ggggaggaag acattcggct gtttaccaga ctccgacacg agttccacaa
2221 atggagtaca ataattgaaa acaattttca agaaggccat aaaattttga gtagaaaaat
2281 ccagaaattt gaaaatcagt atcgtggtag agagctgcca ggctttgtga attacaggac
2341 atttgagaca atcgtgaaac agcaaatcaa ggcactggaa gagccggctg tggatatgct
2401 acacaccgtg acggatatgg tccggcttgc tttcacagat gtttcgataa aaaattttga
2461 agagtttttt aacctccaca gaaccgccaa gtccaaaatt gaagacatta gagcagaaca
2521 agagagagaa ggtgagaagc tgatccgcct ccacttccag atggaacaga ttgtctactg
2581 ccaggaccag gtatacaggg gtgcattgca gaaggtcaga gagaaggagc tggaagaaga
2641 aaagaagaag aaatcctggg attttggggc tttccagtcc agctcggcaa cagactcttc
2701 catggaggag atctttcagc acctgatggc ctatcaccag gaggccagca agcgcatctc
 
2761 cagccacatc cctttgatca tccagttctt catgctccag acgtacggcc agcagcttca
2821 gaaggccatg ctgcagctcc tgcaggacaa ggacacctac agctggctcc tgaaggagcg
2881 gagcgacacc agcgacaagc ggaagttcct gaaggagcgg cttgcacggc tgacgcaggc
2941 tcggcgccgg cttgcccagt tccccggtta accacactct gtccagcccc gtagacgtgc
3001 acgcacactg tctgcccccg ttcccgggta gccactggac tgacgacttg agtgctcagt
3061 agtcagactg gatagtccgt ctctgcttat ccgttagccg tggtgattta gcaggaagct
3121 gtgagagcag tttggtttct agcatgaaga cagagcccca ccctcagatg cacatgagct
3181 ggcgggattg aaggatgctg tcttcgtact gggaaaggga ttttcagccc tcagaatcgc
3241 tccaccttgc agctctcccc ttctctgtat tcctagaaac tgacacatgc tgaacatcac
3301 agcttatttc ctcattttta taatgtccct tcacaaaccc agtgttttag gagcatgagt
3361 gccgtgtgtg tgcgtcctgt cggagccctg tctcctctct ctgtaataaa ctcatttcta
3421 gcagacaaaa aaaaaaaaaa aaaa
**CCL5 (SEQ ID NO: 40)** - Homo sapiens C-C motif chemokine ligand 5 (CCL5), transcript variant 1, mRNA - NM_002985 
1 gctgcagagg attcctgcag aggatcaaga cagcacgtgg acctcgcaca gcctctccca
61 caggtaccat gaaggtctcc gcggcagccc tcgctgtcat cctcattgct actgccctct
121 gcgctcctgc atctgcctcc ccatattcct cggacaccac accctgctgc tttgcctaca
181 ttgcccgccc actgccccgt gcccacatca aggagtattt ctacaccagt ggcaagtgct
241 ccaacccagc agtcgtcttt gtcacccgaa agaaccgcca agtgtgtgcc aacccagaga
301 agaaatgggt tcgggagtac atcaactctt tggagatgag ctaggatgga gagtccttga
361 acctgaactt acacaaattt gcctgtttct gcttgctctt gtcctagctt gggaggcttc
421 ccctcactat cctaccccac ccgctccttg aagggcccag attctaccac acagcagcag
481 ttacaaaaac cttccccagg ctggacgtgg tggctcacgc ctgtaatccc agcactttgg
541 gaggccaagg tgggtggatc acttgaggtc aggagttcga gaccagcctg gccaacatga
601 tgaaacccca tctctactaa aaatacaaaa aattagccgg gcgtggtagc gggcgcctgt
661 agtcccagct actcgggagg ctgaggcagg agaatggcgt gaacccggga ggcggagctt
721 gcagtgagcc gagatcgcgc cactgcactc cagcctgggc gacagagcga gactccgtct
781 caaaaaaaaa aaaaaaaaaa aaaatacaaa aattagccgg gcgtggtggc ccacgcctgt
841 aatcccagct actcgggagg ctaaggcagg aaaattgttt gaacccagga ggtggaggct
901 gcagtgagct gagattgtgc cacttcactc cagcctgggt gacaaagtga gactccgtca
961 caacaacaac aacaaaaagc ttccccaact aaagcctaga agagcttctg aggcgctgct
1021 ttgtcaaaag gaagtctcta ggttctgagc tctggctttg ccttggcttt gccagggctc
1081 tgtgaccagg aaggaagtca gcatgcctct agaggcaagg aggggaggaa cactgcactc
1141 ttaagcttcc gccgtctcaa cccctcacag gagcttactg gcaaacatga aaaatcggct
1201 taccattaaa gttctcaatg caaccataaa aaaaaaa
**TGFBI (SEQ ID NO: 41)** - Homo sapiens transforming growth factor beta induced (TGFBI), mRNA - NM 000358 
1 ctccttgcac gggccggccc agcttccccg cccctggcgt ccgctccctc ccgctcgcag
61 cttacttaac ctggcccggg cggcggaggc gctctcactt ccctggagcc gcccgcttgc
121 ccgtcggtcg ctagctcgct cggtgcgcgt cgtcccgctc catggcgctc ttcgtgcggc
181 tgctggctct cgccctggct ctggccctgg gccccgccgc gaccctggcg ggtcccgcca
241 agtcgcccta ccagctggtg ctgcagcaca gcaggctccg gggccgccag cacggcccca
301 acgtgtgtgc tgtgcagaag gttattggca ctaataggaa gtacttcacc aactgcaagc
361 agtggtacca aaggaaaatc tgtggcaaat caacagtcat cagctacgag tgctgtcctg
421 gatatgaaaa ggtccctggg gagaagggct gtccagcagc cctaccactc tcaaaccttt
481 acgagaccct gggagtcgtt ggatccacca ccactcagct gtacacggac cgcacggaga
541 agctgaggcc tgagatggag gggcccggca gcttcaccat cttcgcccct agcaacgagg
601 cctgggcctc cttgccagct gaagtgctgg actccctggt cagcaatgtc aacattgagc
661 tgctcaatgc cctccgctac catatggtgg gcaggcgagt cctgactgat gagctgaaac
721 acggcatgac cctcacctct atgtaccaga attccaacat ccagatccac cactatccta
781 atgggattgt aactgtgaac tgtgcccggc tgctgaaagc cgaccaccat gcaaccaacg
841 gggtggtgca cctcatcgat aaggtcatct ccaccatcac caacaacatc cagcagatca
 
901 ttgagatcga ggacaccttt gagacccttc gggctgctgt ggctgcatca gggctcaaca
961 cgatgcttga aggtaacggc cagtacacgc ttttggcccc gaccaatgag gccttcgaga
1021 agatccctag tgagactttg aaccgtatcc tgggcgaccc agaagccctg agagacctgc
1081 tgaacaacca catcttgaag tcagctatgt gtgctgaagc catcgttgcg gggctgtctg
1141 tagagaccct ggagggcacg acactggagg tgggctgcag cggggacatg ctcactatca
1201 acgggaaggc gatcatctcc aataaagaca tcctagccac caacggggtg atccactaca
1261 ttgatgagct actcatccca gactcagcca agacactatt tgaattggct gcagagtctg
1321 atgtgtccac agccattgac cttttcagac aagccggcct cggcaatcat ctctctggaa
1381 gtgagcggtt gaccctcctg gctcccctga attctgtatt caaagatgga acccctccaa
1441 ttgatgccca tacaaggaat ttgcttcgga accacataat taaagaccag ctggcctcta
1501 agtatctgta ccatggacag accctggaaa ctctgggcgg caaaaaactg agagtttttg
1561 tttatcgtaa tagcctctgc attgagaaca gctgcatcgc ggcccacgac aagaggggga
1621 ggtacgggac cctgttcacg atggaccggg tgctgacccc cccaatgggg actgtcatgg
1681 atgtcctgaa gggagacaat cgctttagca tgctggtagc tgccatccag tctgcaggac
1741 tgacggagac cctcaaccgg gaaggagtct acacagtctt tgctcccaca aatgaagcct
1801 tccgagccct gccaccaaga gaacggagca gactcttggg agatgccaag gaacttgcca
1861 acatcctgaa ataccacatt ggtgatgaaa tcctggttag cggaggcatc ggggccctgg
1921 tgcggctaaa gtctctccaa ggtgacaagc tggaagtcag cttgaaaaac aatgtggtga
1981 gtgtcaacaa ggagcctgtt gccgagcctg acatcatggc cacaaatggc gtggtccatg
2041 tcatcaccaa tgttctgcag cctccagcca acagacctca ggaaagaggg gatgaacttg
2101 cagactctgc gcttgagatc ttcaaacaag catcagcgtt ttccagggct tcccagaggt
2161 ctgtgcgact agcccctgtc tatcaaaagt tattagagag gatgaagcat tagcttgaag
2221 cactacagga ggaatgcacc acggcagctc tccgccaatt tctctcagat ttccacagag
2281 actgtttgaa tgttttcaaa accaagtatc acactttaat gtacatgggc cgcaccataa
2341 tgagatgtga gccttgtgca tgtgggggag gagggagaga gatgtacttt ttaaatcatg
2401 ttccccctaa acatggctgt taacccactg catgcagaaa cttggatgtc actgcctgac
2461 attcacttcc agagaggacc tatcccaaat gtggaattga ctgcctatgc caagtccctg
2521 gaaaaggagc ttcagtattg tggggctcat aaaacatgaa tcaagcaatc cagcctcatg
2581 ggaagtcctg gcacagtttt tgtaaagccc ttgcacagct ggagaaatgg catcattata
2641 agctatgagt tgaaatgttc tgtcaaatgt gtctcacatc tacacgtggc ttggaggctt
2701 ttatggggcc ctgtccaggt agaaaagaaa tggtatgtag agcttagatt tccctattgt
2761 gacagagcca tggtgtgttt gtaataataa aaccaaagaa acata
**PLA2G7 (SEQ ID NO: 42)** - Homo sapiens phospholipase A2 group VII (PLA2G7), transcript variant 1, mRNA NM 005084 
1 gggtcggggc cacaaggccg cgctaggcgg acccaggaca cagcccgcgc gcagcccacc
61 cgcccgccgc ctgccagagc tgctcggccc gcagccaggg ggacagcggc tggtcggagg
121 ctcgcagtgc tgtcggcgag aagcagtcgg gtttggagcg cttgggtcgc gttggtgcgc
181 ggtggaacgc gcccagggac cccagttccc gcgagcagct ccgcgccgcg cctgagagac
241 taagctgaaa ctgctgctca gctcccaaga tggtgccacc caaattgcat gtgcttttct
301 gcctctgcgg ctgcctggct gtggtttatc cttttgactg gcaatacata aatcctgttg
361 cccatatgaa atcatcagca tgggtcaaca aaatacaagt actgatggct gctgcaagct
421 ttggccaaac taaaatcccc cggggaaatg ggccttattc cgttggttgt acagacttaa
481 tgtttgatca cactaataag ggcaccttct tgcgtttata ttatccatcc caagataatg
541 atcgccttga caccctttgg atcccaaata aagaatattt ttggggtctt agcaaatttc
601 ttggaacaca ctggcttatg ggcaacattt tgaggttact ctttggttca atgacaactc
661 ctgcaaactg gaattcccct ctgaggcctg gtgaaaaata tccacttgtt gttttttctc
721 atggtcttgg ggcattcagg acactttatt ctgctattgg cattgacctg gcatctcatg
781 ggtttatagt tgctgctgta gaacacagag atagatctgc atctgcaact tactatttca
841 aggaccaatc tgctgcagaa ataggggaca agtcttggct ctaccttaga accctgaaac
901 aagaggagga gacacatata cgaaatgagc aggtacggca aagagcaaaa gaatgttccc
961 aagctctcag tctgattctt gacattgatc atggaaagcc agtgaagaat gcattagatt
1021 taaagtttga tatggaacaa ctgaaggact ctattgatag ggaaaaaata gcagtaattg
1081 gacattcttt tggtggagca acggttattc agactcttag tgaagatcag agattcagat
1141 gtggtattgc cctggatgca tggatgtttc cactgggtga tgaagtatat tccagaattc
 
1201 ctcagcccct cttttttatc aactctgaat atttccaata tcctgctaat atcataaaaa
1261 tgaaaaaatg ctactcacct gataaagaaa gaaagatgat tacaatcagg ggttcagtcc
1321 accagaattt tgctgacttc acttttgcaa ctggcaaaat aattggacac atgctcaaat
1381 taaagggaga catagattca aatgtagcta ttgatcttag caacaaagct tcattagcat
1441 tcttacaaaa gcatttagga cttcataaag attttgatca gtgggactgc ttgattgaag
1501 gagatgatga gaatcttatt ccagggacca acattaacac aaccaatcaa cacatcatgt
1561 tacagaactc ttcaggaata gagaaataca attaggatta aaataggttt tttaaaagtc
1621 ttgtttcaaa actgtctaaa attatgtgtg tgtgtgtgtg tgtgtgtgtg tgtgtgagag
1681 agagagagag agagagagag agagagagag agaattttaa tgtattttcc caaaggactc
1741 atattttaaa atgtaggcta tactgtaatc gtgattgaag cttggactaa gaattttttc
1801 cctttagatg taaagaaaga atacagtata caatattcaa aaaaaaaaaa aaaaaaaaaa
1861 aaaaaaaaaa aaaaaaaaaa
**ARHGEF10L (SEQ ID NO: 43)** - Homo sapiens Rho guanine nucleotide exchange factor 10 like (ARHGEF10L), transcript variant 1, mRNA - NM 018125 
1 gcgccgtccc ggccatgggc gcccgcggcg gcctgcggag ctggaggcgc ggcgccggcc
61 gccaggcgcc tttgtgagcg gcgcggacga caaaggcgcg ggcccgggca gccgaggtgt
121 gtagctggga cggtgctggt ctgagctgga ccttgtctga tggcttcctc caaccctcct
181 ccacagcctg ccataggaga tcagctggtt ccaggagtcc caggcccctc ctctgaggca
241 gaggacgacc caggagaggc gtttgagttt gatgacagtg atgatgaaga ggacaccagc
301 gcagccctgg gcgtccccag ccttgctcct gagagggaca cagacccccc actgatccac
361 ttggactcca tccctgtcac tgacccagac ccagcagctg ctccacccgg cacaggggtg
421 ccagcctggg tgagcaatgg ggatgcagcg gacgcagcct tctccggggc ccggcactcc
481 agctggaagc ggaagagttc ccgtcgcatt gaccggttca ctttccccgc cctggaagag
541 gatgtgattt atgacgacgt cccctgcgag agcccagatg cgcatcagcc cggggcagag
601 aggaacctgc tctacgagga tgcgcaccgg gctggggccc ctcggcaggc ggaggaccta
661 ggctggagct ccagtgagtt cgagagctac agcgaggact cgggggagga ggccaagccg
721 gaggtcgagg tcgagcccgc caagcaccga gtgtccttcc agcccaagct ttctccagac
781 ctgactaggc taaaggagag atacgccagg actaagagag acatcttggc tttgagagtt
841 ggggggagag acatgcagga gctgaagcac aagtacgatt gtaagatgac ccagctcatg
901 aaggccgcca agagcgggac caaggatggg ctggagaaga cacggatggc cgtgatgcgc
961 aaagtctcct tcctgcacag gaaggacgtc ctcggtgact cggaggagga ggacatgggg
1021 ctcctggagg tcagcgtttc ggacatcaag cccccagccc cagagctggg ccccatgcca
1081 gagggcctga gccctcagca ggtggtccgg aggcatatcc tgggctccat cgtgcagagc
1141 gaaggcagct acgtggagtc tctgaagcgg atactccagg actaccgcaa ccccctgatg
1201 gagatggagc ccaaggcgct gagcgcccgc aagtgccagg tggtgttctt ccgcgtgaag
1261 gagatcctgc actgccactc catgttccag atcgccctgt cctcccgcgt ggctgagtgg
1321 gattccaccg agaagatcgg ggacctcttc gtggcctcgt tttccaagtc catggtgcta
1381 gatgtgtaca gtgactacgt gaacaacttc accagtgcca tgtccatcat caagaaggcc
1441 tgcctcacca agcctgcctt cctcgagttc ctcaagcgac ggcaggtgtg cagcccagac
1501 cgtgtcaccc tctacgggct gatggtcaag cccatccaga ggttcccaca gttcatactc
1561 ctgcttcagg acatgctgaa gaacaccccc aggggccatc cggacaggct gtcgctgcag
1621 ctggccctca cagagctgga gacgctggct gagaagctga acgagcagaa gcggctggct
1681 gaccaggtgg ctgagatcca gcagctgacc aagagcgtca gtgaccgcag cagcctcaac
1741 aagctgttga cctcaggcca gcggcagctg ctcctgtgtg agacgttgac ggagaccgtg
1801 tacggtgacc gcgggcagct aattaagtcc aaggagcgtc gggtcttcct gctcaacgac
1861 atgcttgtct gtgccaacat caacttcaag cctgccaacc acaggggcca gctggagatc
1921 agcagcctgg tgcccctggg gcccaagtat gtggtgaagt ggaacacggc gctgccccag
1981 gtgcaggtgg tggaggtggg ccaggacggt ggcacctatg acaaggacaa tgtgctcatc
2041 cagcactcag gcgccaagaa ggcctctgcc tcagggcagg ctcagaataa ggtgtacctc
2101 ggccccccac gcctcttcca ggagctgcag gacctgcaga aggacctggc cgtggtggag
2161 cagatcacgc ttctcatcag cacgctgcac ggcacctacc agaacctgaa catgactgtg
2221 gctcaagact ggtgcctggc cctgcagagg ctgatgcggg tgaaggagga agagatccac
2281 tcggccaaca agtgccgtct caggctcctg cttcctggga aacccgacaa gtccggccgc
2341 cccattagct tcatggtggt tttcatcacc cccaaccccc tgagcaagat ttcctgggtc
 
2401 aacaggttac atttggccaa aatcggactc cgggaggaga accagccagg ctggctatgc
2461 ccggatgagg acaagaagag caaagcccca ttctggtgcc cgatcctggc ctgctgcatc
2521 cctgccttct cctcccgggc actcagcctg cagcttgggg ccctggtcca cagtcctgtc
2581 aactgtcccc tgctgggttt ctcagcagtc agcacctccc ttccacaggg ctacctctgg
2641 gtcgggggcg gacaggaagg cgcagggggc caggtggaaa tcttttcctt gaaccggccc
2701 tcgccccgca ccgtcaagtc cttcccactg gcagcccctg tgctctgcat ggagtatatc
2761 ccggagctgg aggaggaggc ggagagcaga gacgagagcc cgacagttgc tgacccctcg
2821 gccacggtgc atccaaccat ctgcctcggg ctccaggatg gcagcatcct cctctacagc
2881 agtgtggaca ctggcaccca gtgcctggtg agctgcagga gcccaggtct gcagcctgtg
2941 ctctgcctgc gacacagccc cttccacctg ctcgctggcc tgcaggatgg gacccttgct
3001 gcttaccctc ggaccagcgg aggtgtcctg tgggacctgg agagccctcc cgtgtgcctg
3061 actgtggggc ccgggcctgt ccgcaccctg ttgagcctgg aggatgccgt gtgggccagc
3121 tgtgggcccc gggtcactgt cctggaagcc accaccctgc agcctcagca aagcttcgag
3181 gcgcaccagg acgaggcagt gagcgtgaca cacatggtga aggcgggcag cggcgtctgg
3241 atggccttct cctccggcac ctccatccgc ctcttccaca ctgagaccct ggagcatctg
3301 caagagatca acatcgccac caggaccacc ttcctcctgc caggccagaa gcacttgtgt
3361 gtcaccagcc tcctgatctg ccagggtctg ctctgggtgg gcactgacca gggtgtcatc
3421 gtcctgctgc ccgtgcctcg gctggaaggc atccccaaga tcacagggaa aggcatggtc
3481 tcactcaacg ggcactgtgg gcctgtggcc ttcctggctg tggctaccag catcctggcc
3541 cctgacatcc tgcggagtga ccaggaggag gctgaggggc cccggctgag ggaggacaag
3601 ccagacgggc aggcacacga gcccatgccc gatagccacg tgggccgaga gctgacccgc
3661 aagaagggca tcctcttgca gtaccgcctg cgctccaccg cacacctccc gggcccgctg
3721 ctctccatgc gggagccggc gcctgctgat ggcgcagctt tggagcacag cgaggaggac
3781 ggctccattt acgagatggc cgacgacccc gacatctggg tgcgcagccg gccctgcgcc
3841 cgcgacgccc accgcaagga gatttgctct gtggccatca tctccggcgg gcagggctac
3901 cgcaactttg gcagcgctct gggcagcagt gggaggcagg ccccgtgtgg ggagacggac
3961 agcaccctcc tcatctggca ggtgcccttg atgctatagc gcctcccctc tcccctcaga
4021 gggcacagct gcaggcctga ccaaggccac gcccggctct cgtgctctag gacctgcacg
4081 ggacttgtgg atgggcctgg actctccaga aactacttgg gcagagcaaa ggaaaacctc
4141 ttgttttaaa aaaatttttt tcagagtgtt ttggggagga gttttagggc ttggggagag
4201 ggaggacaca tctggaggaa atggccttct ttttaaaagc aaaaaacaca aaacctcaca
4261 actgcctggc aagcccagta tcacttgttt gggccctagc gggactccaa ggcagccaca
4321 cgcccctcct ggaagggtgt gtgcgtgtga gtgtgtgcga gtgtgtgggc tggtgtgtga
4381 atatctataa ataagtatat atggtgtata ttatatgtgt ataaataaag tctgtacata
4441 ttggagctct gggagatgct ggaataaaag acaagagtta catctggact tggaaaaaaa
4501 aa
**ADGRA2 (GPR124) (SEQ ID NO: 44)** - Homo sapiens Adhesion G protein-coupled receptor A2, mRNA - NM 032777 
1 atccatggca cggagcggcg gcggcggcgg cagcaggagc ccggcgcgat ccgctaggtc
61 ccagcccagc gcccagcgag caggcgacgc ggaggggccg ggcctccagt gtcccgaggg
121 ccgggcgctg agactccggc cgcgcagctg ggagctgccc gcgctgcgct gacagccgcg
181 ccgacgtcct ccccgccggg gcgctcgcag gacatgcccc cggggcgcgg cggcggggac
241 cccggggctc gcctccgccc agggcccccc tccacgccct cgggagcccc gggcccccgc
301 tgagcactcc tcccgcacgc ctgggtccct ccggccggcg cgcagcccgg ccccagcgct
361 gtgggtcccc gcggggcgat gggttgatgg gcgccggggg acgcaggatg cggggggcgc
421 ccgcgcgcct gctgctgccg ctgctgccgt ggctcctgct gctcctggcg cccgaggctc
481 ggggcgcgcc cggctgcccg ctatccatcc gcagctgcaa gtgctcgggg gagcggccca
541 aggggctgag cggcggcgtc cctggcccgg ctcggcggag ggtggtgtgc agcggcgggg
601 acctcccgga gcctcccgag cccggccttc tgcctaacgg caccgttacc ctgctcttga
661 gcaataacaa gatcacgggg ctccgcaatg gctccttcct gggactgtca ctgctggaga
721 agctggacct gaggaacaac atcatcagca cagtgcagcc gggcgccttc ctgggcctgg
781 gggagctgaa gcgtttagat ctctccaaca accggattgg ctgtctcacc tccgagacct
841 tccagggcct ccccaggctt ctccgactaa acatatctgg aaacatcttc tccagtctgc
901 aacctggggt ctttgatgag ctgccagccc ttaaggttgt ggacttgggc accgagttcc
 
961 tgacctgtga ctgccacctg cgctggctgc tgccctgggc ccagaatcgc tccctgcagc
1021 tgtcggaaca cacgctctgt gcttacccca gtgccctgca tgctcaggcc ctgggcagcc
1081 tccaggaggc ccagctctgc tgcgaggggg ccctggagct gcacacacac cacctcatcc
1141 cgtccctacg ccaagtggtg ttccaggggg atcggctgcc cttccagtgc tctgccagct
1201 acctgggcaa cgacacccgc atccgctggt accacaaccg agcccctgtg gagggtgatg
1261 agcaggcggg catcctcctg gccgagagcc tcatccacga ctgcaccttc atcaccagtg
1321 agctgacgct gtctcacatc ggcgtgtggg cctcaggcga gtgggagtgc accgtgtcca
1381 tggcccaagg caacgccagc aagaaggtgg agatcgtggt gctggagacc tctgcctcct
1441 actgccccgc cgagcgtgtt gccaacaacc gcggggactt caggtggccc cgaactctgg
1501 ctggcatcac agcctaccag tcctgcctgc agtatccctt cacctcagtg cccctgggcg
1561 ggggtgcccc gggcacccga gcctcccgcc ggtgtgaccg tgccggccgc tgggagccag
1621 gggactactc ccactgtctc tacaccaacg acatcaccag ggtgctgtac accttcgtgc
1681 tgatgcccat caatgcctcc aatgcgctga ccctggctca ccagctgcgc gtgtacacag
1741 ccgaggccgc tagcttttca gacatgatgg atgtagtcta tgtggctcag atgatccaga
1801 aatttttggg ttatgtcgac cagatcaaag agctggtaga ggtgatggtg gacatggcca
1861 gcaacctgat gctggtggac gagcacctgc tgtggctggc ccagcgcgag gacaaggcct
1921 gcagccgcat cgtgggtgcc ctggagcgca ttgggggggc cgccctcagc ccccatgccc
1981 agcacatctc agtgaatgcg aggaacgtgg cattggaggc ctacctcatc aagccgcaca
2041 gctacgtggg cctgacctgc acagccttcc agaggaggga gggaggggtg ccgggcacac
2101 ggccaggaag ccctggccag aaccccccac ctgagcccga gcccccagct gaccagcagc
2161 tccgcttccg ctgcaccacc gggaggccca atgtttctct gtcgtccttc cacatcaaga
2221 acagcgtggc cctggcctcc atccagctgc ccccgagtct attctcatcc cttccggctg
2281 ccctggctcc cccggtgccc ccagactgca ccctgcaact gctcgtcttc cgaaatggcc
2341 gcctcttcca cagccacagc aacacctccc gccctggagc tgctgggcct ggcaagaggc
2401 gtggcgtggc cacccccgtc atcttcgcag gaaccagtgg ctgtggcgtg ggaaacctga
2461 cagagccagt ggccgtttcg ctgcggcact gggctgaggg agccgaacct gtggccgctt
2521 ggtggagcca ggagggcccc ggggaggctg ggggctggac ctcggagggc tgccagctcc
2581 gctccagcca gcccaatgtc agcgccctgc actgccagca cttgggcaat gtggccgtgc
2641 tcatggagct gagcgccttt cccagggagg tggggggcgc cggggcaggg ctgcaccccg
2701 tggtataccc ctgcacggcc ttgctgctgc tctgcctctt cgccaccatc atcacctaca
2761 tcctcaacca cagctccatc cgtgtgtccc ggaaaggctg gcacatgctg ctgaacttgt
2821 gcttccacat agccatgacc tctgctgtct ttgcgggggg catcacactc accaactacc
2881 agatggtctg ccaggcggtg ggcatcaccc tgcactactc ctccctatcc acgctgctct
2941 ggatgggcgt gaaggcgcga gtgctccata aggagctcac ctggagggcac cccctccgc
3001 aagaagggga ccccgctctg cctactccca gtcctatgct ccggttctat ttgatcgctg
3061 gagggattcc actcattatc tgtggcatca cagctgcagt caacatccac aactaccggg
3121 accacagccc ctactgctgg ctggtgtggc gtccaagcct tggcgccttc tacatccctg
3181 tggctttgat tctgctcatc acctggatct atttcctgtg cgccgggcta cgcttacggg
3241 gtcctctggc acagaacccc aaggcgggca acagcagggc ctccctggag gcaggggagg
3301 agctgagggg ttccaccagg ctcaggggca gcggccccct cctgagtgac tcaggttccc
3361 ttcttgctac tgggagcgcg cgagtgggga cgcccgggcc cccggaggat ggtgacagcc
3421 tctattctcc gggagtccag ctaggggcgc tggtgaccac gcacttcctg tacttggcca
3481 tgtgggcctg cggggctctg gcagtgtccc agcgctggct gccccgggtg gtgtgcagct
3541 gcttgtacgg ggtggcagcc tccgccctgg gcctcttcgt cttcactcac cactgtgcca
3601 ggcggaggga cgtgagagcc tcgtggcgcg cctgctgccc ccctgcctct cccgcggccc
3661 cccatgcccc gccccgggcc ctgcccgccg ccgcagagga cggttccccg gtgttcgggg
3721 agggcccccc ctccctcaag tcctccccaa gcggcagcag cggccatccg ctggctctgg
3781 gcccctgcaa gctcaccaac ctgcagctgg cccagagtca ggtgtgcgag gcgggggcgg
3841 cggccggcgg ggaaggagag ccggagccgg cgggcacccg gggaaacctc gcccaccgcc
3901 accccaacaa cgtgcaccac gggcgtcggg cgcacaagag ccgggccaag ggacaccgcg
3961 cgggggaggc ctgcggcaag aaccggctca aggccctgcg cgggggcgcg gcgggggcgc
4021 tggagctgct gtccagcgag agcggcagtc tgcacaacag ccccaccgac agctacctgg
4081 gcagcagccg caacagcccg ggcgccggcc tgcagctgga aggcgagccc atgctcacgc
4141 cgtccgaggg cagcgacacc agcgccgcgc cgctttctga ggcggccggg gcaggccagc
4201 gccgcagcgc cagccgcgac agtctcaagg gcggcggcgc gctggagaag gagagccatc
4261 gccgctcgta cccgctcaac gccgccagcc taaacggcgc ccccaagggg ggcaagtacg
 
4321 acgacgtcac cctgatgggc gcggaggtag ccagcggcgg ctgcatgaag accggactct
4381 ggaagagcga aactaccgtc taaggtgggg cgggcgacgc ggtagacggg ctggccacgc
4441 ggctcgttcc cccgctcctc ggggccctcc aaggtgtctc cgtagtcagc aggttggagg
4501 cagaggagcc gatggctgga ggaagcccac aggcggatgt tccccacttg cctagagggc
4561 atccctctgg ggtagcgaca gacaatccca gaaacacgca taatacattt ccgtccagcc
4621 cggggcagtc tgactgtcgg tgccctccca ggaacgggga aggcctccgt ctgtgtgaaa
4681 gggcacagca catcccaggt gcaccctccc caagtactcc caccccgcct actgtccatg
4741 cggcctcact gggggccatc agcctcacca gcaaagcaga gatgagagcg tgggaactgt
4801 gttctttcct ccctgccctc tactgatttc agcccagccc ctgcctagat cctaggtccc
4861 ttttcctccc gagtttggct ggcacgagag ctagcccagc acatgaagca ggtgatgtta
4921 agtcacaagg tgctgctttt cagatccact atgcaagagg ggagggtggg gccacgtgaa
4981 aggcagctct agacatcaac cagtcctggg ggaggggagt gggaaccggg cacaactagg
5041 aacaatgcca ccattcccac aggagtggta cttaaaccag acagcagggt tcagaggtgg
5101 cacaccggga caaagctgag gccctgcacc tcaacagctg actgccaggt gcctgtgggt
5161 gaactgaggg gagtagaggg agagggcagg tggaactggg gcagaatcta gtcatgccct
5221 aaagctagtc ctgtaaacaa tggtgcccca gaaagctgca ggtggtgttt ggagaagcag
5281 ttacttttca gttacaagac ccatctccct agtctcagcc ttacaacacc acgggactaa
5341 ggaagagcac ttccttgcct ccgtaaggcc agaggaagaa ccatcccaat catttgatct
5401 ccagctccac agtagagaga aacctacaaa atgtcaaacc agcttcccga ctcccaggag
5461 ctcaagccaa gcccagaggc agtggctggg gtccctgcag gtcatgaggg gcctatgcct
5521 ttactccttt taaacaccag cacccgtctt ttccccaacc taaaaccaac caccagcatt
5581 tcactacagg accaaatgga aaccgaggga accctgggtc ttgggaagaa caacaggaaa
5641 ccaaggtctg acctagggtt ccctcccagt cttcacatca ctctggcctc atcaccaagg
5701 tgacagagga cacaggggag ggggaaaacc cacacacact ccttggaatg ggtcctgtta
5761 tttatgcttg ctgcacagac atattagaag aaaaaaaaaa gctttgtatt attcttccac
5821 atatgctggc tgctgtttac acaccctgcc aatgccttag cactggagag ctttttgcaa
5881 tatgctgggg aaagggaggg gagggaatga aagtgccaaa gaaaacatgt ttttaagaac
5941 tcgggtttta tacaatagaa tgttttctag cagatgcctc ttgttttaat atattaaaat
6001 tttgcaaagc cctttgagct actgccttag tctaaaaaaa aaaaaaaaaa
**IL1RN (SEQ ID NO: 45)** interleukin-1 receptor antagonist protein isoform 4 NM_173843.2 
1 gggcagctcc accctgggag ggactgtggc ccaggtactg cccgggtgct actttatggg
61 cagcagctca gttgagttag agtctggaag acctcagaag acctcctgtc ctatgaggcc
121 ctccccatgg ctttaggggg attataaaac taatcatcaa agccaagaag gcaagagcaa
181 gcatgtaccg ctgaaaacac aagataactg cataagtaat gactttcagt gcagattcat
241 agctaaccca taaactgctg gggcaaaaat catcttggaa ggctctgaac ctcagaaagg
301 attcacaaga cgatctgccg accctctggg agaaaatcca gcaagatgca agccttcaga
361 atctgggatg ttaaccagaa gaccttctat ctgaggaaca accaactagt tgctggatac
421 ttgcaaggac caaatgtcaa tttagaagaa aagatagatg tggtacccat tgagcctcat
481 gctctgttct tgggaatcca tggagggaag atgtgcctgt cctgtgtcaa gtctggtgat
541 gagaccagac tccagctgga ggcagttaac atcactgacc tgagcgagaa cagaaagcag
601 gacaagcgct tcgccttcat ccgctcagac agtggcccca ccaccagttt tgagtctgcc
661 gcctgccccg gttggttcct ctgcacagcg atggaagctg accagcccgt cagcctcacc
721 aatatgcctg acgaaggcgt catggtcacc aaattctact tccaggagga cgagtagtac
781 tgcccaggcc tgcctgttcc cattcttgca tggcaaggac tgcagggact gccagtcccc
841 ctgccccagg gctcccggct atgggggcac tgaggaccag ccattgaggg gtggaccctc
901 agaaggcgtc acaacaacct ggtcacagga ctctgcctcc tcttcaactg accagcctcc
961 atgctgcctc cagaatggtc tttctaatgt gtgaatcaga gcacagcagc ccctgcacaa
1021 agcccttcca tgtcgcctct gcattcagga tcaaaccccg accacctgcc caacctgctc
1081 tcctcttgcc actgcctctt cctccctcat tccaccttcc catgccctgg atccatcagg
1141 ccacttgatg acccccaacc aagtggctcc cacaccctgt tttacaaaaa agaaaagacc
1201 agtccatgag ggaggttttt aagggtttgt ggaaaatgaa aattaggatt tcatgatttt
1261 tttttttcag tccccgtgaa ggagagccct tcatttggag attatgttct ttcggggaga
1321 ggctgaggac ttaaaatatt cctgcatttg tgaaatgatg gtgaaagtaa gtggtagctt
1381 ttcccttctt tttcttcttt ttttgtgatg tcccaacttg taaaaattaa aagttatggt
1441 actatgttag ccccataatt ttttttttcc ttttaaaaca cttccataat ctggactcct
 
1501 ctgtccaggc actgctgccc agcctccaag ctccatctcc actccagatt ttttacagct
1561 gcctgcagta ctttacctcc tatcagaagt ttctcagctc ccaaggctct gagcaaatgt
1621 ggctcctggg ggttctttct tcctctgctg aaggaataaa ttgctccttg acattgtaga
1681 gcttctggca cttggagact tgtatgaaag atggctgtgc ctctgcctgt ctcccccacc
1741 gggctgggag ctctgcagag caggaaacat gactcgtata tgtctcaggt ccctgcaggg
1801 ccaagcacct agcctcgctc ttggcaggta ctcagcgaat gaatgctgta tatgttgggt
1861 gcaaagttcc ctacttcctg tgacttcagc tctgttttac aataaaatct tgaaaatgcc
1921 taaaaaaaaa aaaaaaaaaa aaaaaaaaaa aaaaaaaaaa aaaaaaaaaa aaa
**NLRP3 (SEQ ID NO: 46)** NACHT, LRR and PYD domains-containing protein 3 isoform a, transcript variant 1 NM 004895.4 
1 gtagatgagg aaactgaagt tgaggaatag tgaagagttt gtccaatgtc atagccccgt
61 aatcaacggg acaaaaattt tcttgctgat gggtcaagat ggcatcgtga agtggttgtt
121 caccgtaaac tgtaatacaa tcctgtttat ggatttgttt gcatattttt ccctccatag
181 ggaaaccttt cttccatggc tcaggacaca ctcctggatc gagccaacag gagaactttc
241 tggtaagcat ttggctaact tttttttttt tgagatggag tcttgctgtg tcgcctaggc
301 tggagtgcag tggcgtgatc ttggctcact gcagcctcca cttcccgggt tcaatcaatt
361 ctcctacctc aacttcctga gtagctggga ttacaggcgc ccgccaccac acccggctca
421 tttttgtact tttagtagag acacagtttt gccatgttgg ccaggctggt cttgaattcc
481 tcagctcagg tgatctgcct gccttggcct ctcaaagtgc tgggattaca ggcgtgagcc
541 actgtgcccg gccttggcta acttttcaaa attaaagatt ttgacttgtt acagtcatgt
601 gacatttttt tctttctgtt tgctgagttt ttgataattt atatctctca aagtggagac
661 tttaaaaaag actcatccgt gtgccgtgtt cactgcctgg tatcttagtg tggaccgaag
721 cctaaggacc ctgaaaacag ctgcagatga agatggcaag cacccgctgc aagctggcca
781 ggtacctgga ggacctggag gatgtggact tgaagaaatt taagatgcac ttagaggact
841 atcctcccca gaagggctgc atccccctcc cgaggggtca gacagagaag gcagaccatg
901 tggatctagc cacgctaatg atcgacttca atggggagga gaaggcgtgg gccatggccg
961 tgtggatctt cgctgcgatc aacaggagag acctttatga gaaagcaaaa agagatgagc
1021 cgaagtgggg ttcagataat gcacgtgttt cgaatcccac tgtgatatgc caggaagaca
1081 gcattgaaga ggagtggatg ggtttactgg agtacctttc gagaatctct atttgtaaaa
1141 tgaagaaaga ttaccgtaag aagtacagaa agtacgtgag aagcagattc cagtgcattg
1201 aagacaggaa tgcccgtctg ggtgagagtg tgagcctcaa caaacgctac acacgactgc
1261 gtctcatcaa ggagcaccgg agccagcagg agagggagca ggagcttctg gccatcggca
1321 agaccaagac gtgtgagagc cccgtgagtc ccattaagat ggagttgctg tttgaccccg
1381 atgatgagca ttctgagcct gtgcacaccg tggtgttcca gggggcggca gggattggga
1441 aaacaatcct ggccaggaag atgatgttgg actgggcgtc ggggacactc taccaagaca
1501 ggtttgacta tctgttctat atccactgtc gggaggtgag ccttgtgaca cagaggagcc
1561 tgggggacct gatcatgagc tgctgccccg acccaaaccc acccatccac aagatcgtga
1621 gaaaaccctc cagaatcctc ttcctcatgg acggcttcga tgagctgcaa ggtgcctttg
1681 acgagcacat aggaccgctc tgcactgact ggcagaaggc cgagcgggga gacattctcc
1741 tgagcagcct catcagaaag aagctgcttc ccgaggcctc tctgctcatc accacgagac
1801 ctgtggccct ggagaaactg cagcacttgc tggaccatcc tcggcatgtg gagatcctgg
1861 gtttctccga ggccaaaagg aaagagtact tcttcaagta cttctctgat gaggcccaag
1921 ccagggcagc cttcagtctg attcaggaga acgaggtcct cttcaccatg tgcttcatcc
1981 ccctggtctg ctggatcgtg tgcactggac tgaaacagca gatggagagt ggcaagagcc
2041 ttgcccagac atccaagacc accaccgcgg tgtacgtctt cttcctttcc agtttgctgc
2101 agccccgggg agggagccag gagcacggcc tctgcgccca cctctggggg ctctgctctt
2161 tggctgcaga tggaatctgg aaccagaaaa tcctgtttga ggagtccgac ctcaggaatc
2221 atggactgca gaaggcggat gtgtctgctt tcctgaggat gaacctgttc caaaaggaag
2281 tggactgcga gaagttctac agcttcatcc acatgacttt ccaggagttc tttgccgcca
2341 tgtactacct gctggaagag gaaaaggaag gaaggacgaa cgttccaggg agtcgtttga
2401 agcttcccag ccgagacgtg acagtccttc tggaaaacta tggcaaattc gaaaaggggt
2461 atttgatttt tgttgtacgt ttcctctttg gcctggtaaa ccaggagagg acctcctact
2521 tggagaagaa attaagttgc aagatctctc agcaaatcag gctggagctg ctgaaatgga
2581 ttgaagtgaa agccaaagct aaaaagctgc agatccagcc cagccagctg gaattgttct
 
2641 actgtttgta cgagatgcag gaggaggact tcgtgcaaag ggccatggac tatttcccca
2701 agattgagat caatctctcc accagaatgg accacatggt ttcttccttt tgcattgaga
2761 actgtcatcg ggtggagtca ctgtccctgg ggtttctcca taacatgccc aaggaggaag
2821 aggaggagga aaaggaaggc cgacaccttg atatggtgca gtgtgtcctc ccaagctcct
2881 ctcatgctgc ctgttctcat ggattggtga acagccacct cacttccagt tttgccgggg
2941 gcctcttttc agttctgagc accagccaga gtctaactga attggacctc agtgacaatt
3001 ctctggggga cccagggatg agagtgttgt gtgaaacgct ccagcatcct ggctgtaaca
3061 ttcggagatt gtggttgggg cgctgtggcc tctcgcatga gtgctgcttc gacatctcct
3121 tggtcctcag cagcaaccag aagctggtgg agctggacct gagtgacaac gccctcggtg
3181 acttcggaat cagacttctg tgtgtgggac tgaagcacct gttgtgcaat ctgaagaagc
3241 tctggttggt cagctgctgc ctcacatcag catgttgtca ggatcttgca tcagtattga
3301 gcaccagcca ttccctgacc agactctatg tgggggagaa tgccttggga gactcaggag
3361 tcgcaatttt atgtgaaaaa gccaagaatc cacagtgtaa cctgcagaaa ctggggttgg
3421 tgaattctgg ccttacgtca gtctgttgtt cagctttgtc ctcggtactc agcactaatc
3481 agaatctcac gcacctttac ctgcgaggca acactctcgg agacaagggg atcaaactac
3541 tctgtgaggg actcttgcac cccgactgca agcttcaggt gttggaatta gacaactgca
3601 acctcacgtc acactgctgc tgggatcttt ccacacttct gacctccagc cagagcctgc
3661 gaaagctgag cctgggcaac aatgacctgg gcgacctggg ggtcatgatg ttctgtgaag
3721 tgctgaaaca gcagagctgc ctcctgcaga acctggggtt gtctgaaatg tatttcaatt
3781 atgagacaaa aagtgcgtta gaaacacttc aagaagaaaa gcctgagctg accgtcgtct
3841 ttgagccttc ttggtaggag tggaaacggg gctgccagac gccagtgttc tccggtccct
3901 ccagctgggg gccctcaggt ggagagagct gcgatccatc caggccaaga ccacagctct
3961 gtgatccttc cggtggagtg tcggagaaga gagcttgccg acgatgcctt cctgtgcaga
4021 gcttgggcat ctcctttacg ccagggtgag gaagacacca ggacaatgac agcatcgggt
4081 gttgttgtca tcacagcgcc tcagttagag gatgttcctc ttggtgacct catgtaatta
4141 gctcattcaa taaagcactt tctttatttt tctcttctct gtctaacttt ctttttccta
4201 tcttttttct tctttgttct gtttactttt gctcatatca tcattcccgc tatctttcta
4261 ttaactgacc ataacacaga actagttgac tatatattat gttgaaattt tatggcagct
4321 atttatttat ttaaattttt tgtaacagtt ttgttttcta ataagaaaaa tccatgcttt
4381 ttgtagctgg ttgaaaattc aggaatatgt aaaacttttt ggtatttaat taaattgatt
4441 ccttttctta attttaaaaa aaaaaaaaaa
RBP4 **(SEQ ID NO: 47)** retinol binding protein 4 NM 001323517.1 
1 ggcggccgct ggcacgagtg cagggtaact gagccagggc cgctggcgca tttggcctgg
61 ccgaggccac cccgcgcggc cgctccactg tgcccgaggc tgtcctggag gtgaggccgg
121 cccacaggga ccctgcccgt gcccgggctc cgggcggatt cctgggcaag atgaagtggg
181 tgtgggcgct cttgctgttg gcggcgctgg gcagcggccg cgcggagcgc gactgccgag
241 tgagcagctt ccgagtcaag gagaacttcg acaaggctcg cttctctggg acctggtacg
301 ccatggccaa gaaggacccc gagggcctct ttctgcagga caacatcgtc gcggagttct
361 ccgtggacga gaccggccag atgagcgcca cagccaaggg ccgagtccgt cttttgaata
421 actgggacgt gtgcgcagac atggtgggca ccttcacaga caccgaggac cctgccaagt
481 tcaagatgaa gtactggggc gtagcctcct ttctccagaa aggaaatgat gaccactgga
541 tcgtcgacac agactacgac acgtatgccg tgcagtactc ctgccgcctc ctgaacctcg
601 atggcacctg tgctgacagc tactccttcg tgttttcccg ggaccccaac ggcctgcccc
661 cagaagcgca gaagattgta aggcagcggc aggaggagct gtgcctggcc aggcagtaca
721 ggctgatcgt ccacaacggt tactgcgatg gcagatcaga aagaaacctt ttgtagcaat
781 atcaagaatc tagtttcatc tgagaacttc tgattagctc tcagtcttca gctctattta
841 tcttaggagt ttaatttgcc cttctctccc catcttccct cagttcccat aaaaccttca
901 ttacacataa agatacacgt gggggtcagt gaatctgctt gcctttcctg aaagtttctg
961 gggcttaaga ttccagactc tgattcatta aactatagtc acccgtgtcc tgtga
**MPP3 (SEQ ID NO: 48)** membrane palmitoylated protein 3, transcript variant 1, mRNA NM 001932.4 
1 aaggcggagc cagcggcggc ccaggctctg ggcactcgcg cggctcccgt tcgcggagca
61 cacatcccgg cgcgacgagg ttccttcgga gagcgagcgg gagtcggtgc tcggcctcct
 
121 gcggggagca gcccgaggaa tctgcaggga gaggtcggga ggtgacaacg ccagcatgcc
181 agtgctatcg gaggactctg gtttgcatga aaccctggcc ctgctgacct cccagctcag
241 acctgactcc aaccacaagg aggagatggg cttcctgagg gatgttttca gtgaaaaaag
301 cctcagttac ttaatgaaga ttcatgagaa gcttcgctat tatgaaaggc aaagtccaac
361 cccagttctg cacagcgctg tggccctcgc tgaggacgtg atggaggagt tgcaggccgc
421 ctccgtgcac agtgatgaga gggagctgct ccagctgctg tccaccccgc acctgagggc
481 tgtgctcatg gtacatgaca cggttgccca gaagaatttt gaccccgttc tcccgcctct
541 gcctgacaat atcgatgagg attttgatga ggaatcggtg aagatcgtcc gcttggtgaa
601 gaacaaggaa cccctgggtg ccaccatccg gcgggacgag cactcagggg ctgttgtggt
661 ggccaggatc atgcgaggag gcgcagcaga caggagcggc ctggtccacg ttggagatga
721 gctccgagaa gtgaacggga tcgcagtcct gcacaagcgg cccgacgaga tcagccagat
781 tctggcccag tcccagggat ccatcaccct aaaaatcatc ccagccaccc aggaggaaga
841 tcgcttaaag gagagcaagg tgttcatgcg cgccctcttc cactacaacc ctcgggagga
901 ccgggccatc ccttgccagg aggcgggcct gcccttccag cgcaggcagg tcctggaggt
961 ggtgagccag gacgacccca cgtggtggca ggccaagcga gtcggggaca ccaaccttcg
1021 agccggcctc atcccctcca aggggttcca ggagagacga ctaagctacc ggagagccgc
1081 gggcaccctg ccgagccccc agagcctcag gaagcccccc tatgatcagc cttgtgacaa
1141 agagacctgt gactgtgagg gctacctcaa agggcactat gtggctggtc ttcggaggag
1201 cttccggctg ggctgtaggg agagactggg tggctcgcag gaaggaaaga tgtcctccgg
1261 agctgagtct ccggagctgc tgacttacga agaggtggcc aggtaccaac accagcccgg
1321 agagcggccc cgcctggtgg ttctgatcgg gtctctggga gcccgactgc acgagctgaa
1381 gcaaaaggtg gtggctgaga acccacagca ctttggcgtc gctgttccac ataccaccag
1441 gccccgaaag agccatgaga aggaaggagt ggaatatcac tttgtgtcta agcaagcatt
1501 tgaggccgac ttacatcaca acaagttcct ggaacatggt gaatataagg aaaatctgta
1561 tggaaccagc ctggaggcca ttcaggctgt tatggccaaa aacaaagttt gtttggtgga
1621 tgtggagcca gaagcactga aacaactgag gacctcagaa tttaaaccct atattatatt
1681 tgtaaagcct gcaattcagg aaaaaagaaa aacgccacct atgtccccag cttgtgagga
1741 cacagcagcc ccatttgatg agcagcagca agagatggcc gcttctgccg ccttcataga
1801 ccggcattac gggcacctgg tagacgccgt gctggtgaag gaggatctcc agggtgccta
1861 cagccagctc aaagtggtct tagagaagct gagcaaggac actcactggg tacctgttag
1921 ttgggtcagg taactttatc ccagaacatc caagctggac gggaccttga agatcatcta
1981 gtccagactc cctcatttta ccatcaagga atctcaagcg cagagaggga gagaattctc
2041 cacaaattcc atcatcgaga agagtataag tgggaagtct tgtttgttgt tggtttttgt
2101 ctgttgtttt tcactgcacc tctttggatc atgatttgaa aggggcatat cagaaaacaa
2161 cacatttcat ttattaaagt atcacaggca agctgaccct gattctttgt accaaagtta
2221 agtagccact gtcttttgtg ggtggtagtg gttaatttat acagtactga ttcgcagaat
2281 gtttaagctt tttaaacata gtgacgctta gtagtttttt tggaagctaa cttgttttat
2341 ccaaggggat tttacatgta actgaagttc ccctgtcttc aagcactaaa acgttgatct
2401 taaccttttt tttgaagtgc ttgcctggta atagaaaacg ggttctctgc ctattttaaa
2461 atagtgaata tacgtaaatt ttctctggaa ggctgaggca cacttcacca tcaacatgaa
2521 ttactgtact atcctgtact gcagtggtgc cttcagggac tcgaggaatg taaggttgcc
2581 tttccccttt ctaaataccc tcagattcct aacatcgagc ccatgctttg tttgatttgt
2641 tctattccat ccattgtccc ttttgttact gacagttgcc ttggtcctag ccagtccctg
2701 ccatgagatc ataggggttc ccattgtgct agatcttggg aaaccagatg actctccctg
2761 tcaaaactat ggctacgtca ctgtaaacca tttctgtcaa gaataaaagt atgtagaccc
2821 agagtgtggg cctaaaaaaa aaaaaaaaaa a
**KIF2C (SEQ ID NO: 49)** kinesin-like protein KIF2C isoform 1 NM 006845.3 
1 acgcttgcgc gcgggattta aactgcggcg gtttacgcgg cgttaagact tcgtagggtt
61 agcgaaattg aggtttcttg gtattgcgcg tttctcttcc ttgctgactc tccgaatggc
121 catggactcg tcgcttcagg cccgcctgtt tcccggtctc gctatcaaga tccaacgcag
181 taatggttta attcacagtg ccaatgtaag gactgtgaac ttggagaaat cctgtgtttc
241 agtggaatgg gcagaaggag gtgccacaaa gggcaaagag attgattttg atgatgtggc
301 tgcaataaac ccagaactct tacagcttct tcccttacat ccgaaggaca atctgccctt
 
361 gcaggaaaat gtaacaatcc agaaacaaaa acggagatcc gtcaactcca aaattcctgc
421 tccaaaagaa agtcttcgaa gccgctccac tcgcatgtcc actgtctcag agcttcgcat
481 cacggctcag gagaatgaca tggaggtgga gctgcctgca gctgcaaact cccgcaagca
541 gttttcagtt cctcctgccc ccactaggcc ttcctgccct gcagtggctg aaataccatt
601 gaggatggtc agcgaggaga tggaagagca agtccattcc atccgaggca gctcttctgc
661 aaaccctgtg aactcagttc ggaggaaatc atgtcttgtg aaggaagtgg aaaaaatgaa
721 gaacaagcga gaagagaaga aggcccagaa ctctgaaatg agaatgaaga gagctcagga
781 gtatgacagt agttttccaa actgggaatt tgcccgaatg attaaagaat ttcgggctac
841 tttggaatgt catccactta ctatgactga tcctatcgaa gagcacagaa tatgtgtctg
901 tgttaggaaa cgcccactga ataagcaaga attggccaag aaagaaattg atgtgatttc
961 cattcctagc aagtgtctcc tcttggtaca tgaacccaag ttgaaagtgg acttaacaaa
1021 gtatctggag aaccaagcat tctgctttga ctttgcattt gatgaaacag cttcgaatga
1081 agttgtctac aggttcacag caaggccact ggtacagaca atctttgaag gtggaaaagc
1141 aacttgtttt gcatatggcc agacaggaag tggcaagaca catactatgg gcggagacct
1201 ctctgggaaa gcccagaatg catccaaagg gatctatgcc atggcctccc gggacgtctt
1261 cctcctgaag aatcaaccct gctaccggaa gttgggcctg gaagtctatg tgacattctt
1321 cgagatctac aatgggaagc tgtttgacct gctcaacaag aaggccaagc tgcgcgtgct
1381 ggaggacggc aagcaacagg tgcaagtggt ggggctgcag gagcatctgg ttaactctgc
1441 tgatgatgtc atcaagatga tcgacatggg cagcgcctgc agaacctctg ggcagacatt
1501 tgccaactcc aattcctccc gctcccacgc gtgcttccaa attattcttc gagctaaagg
1561 gagaatgcat ggcaagttct ctttggtaga tctggcaggg aatgagcgag gcgcggacac
1621 ttccagtgct gaccggcaga cccgcatgga gggcgcagaa atcaacaaga gtctcttagc
1681 cctgaaggag tgcatcaggg ccctgggaca gaacaaggct cacaccccgt tccgtgagag
1741 caagctgaca caggtgctga gggactcctt cattggggag aactctagga cttgcatgat
1801 tgccacgatc tcaccaggca taagctcctg tgaatatact ttaaacaccc tgagatatgc
1861 agacagggtc aaggagctga gcccccacag tgggcccagt ggagagcagt tgattcaaat
1921 ggaaacagaa gagatggaag cctgctctaa cggggcgctg attccaggca atttatccaa
1981 ggaagaggag gaactgtctt cccagatgtc cagctttaac gaagccatga ctcagatcag
2041 ggagctggag gagaaggcta tggaagagct caaggagatc atacagcaag gaccagactg
2101 gcttgagctc tctgagatga ccgagcagcc agactatgac ctggagacct ttgtgaacaa
2161 agcggaatct gctctggccc agcaagccaa gcatttctca gccctgcgag atgtcatcaa
2221 ggccttgcgc ctggccatgc agctggaaga gcaggctagc agacaaataa gcagcaagaa
2281 acggccccag tgacgactgc aaataaaaat ctgtttggtt tgacacccag cctcttccct
2341 ggccctcccc agagaacttt gggtacctgg tgggtctagg cagggtctga gctgggacag
2401 gttctggtaa atgccaagta tgggggcatc tgggcccagg gcagctgggg agggggtcag
2461 agtgacatgg gacactcctt ttctgttcct cagttgtcgc cctcacgaga ggaaggagct
2521 cttagttacc cttttgtgtt gcccttcttt ccatcaaggg gaatgttctc agcatagagc
2581 tttctccgca gcatcctgcc tgcgtggact ggctgctaat ggagagctcc ctggggttgt
2641 cctggctctg gggagagaga cggagccttt agtacagcta tctgctggct ctaaaccttc
2701 tacgcctttg ggccgagcac tgaatgtctt gtactttaaa aaaatgtttc tgagacctct
2761 ttctacttta ctgtctccct agagatccta gaggatccct actgttttct gttttatgtg
2821 tttatacatt gtatgtaaca ataaagagaa aaaataaatc agctgtttaa gtgtgtggaa
2881 aaaaaaaaaa aaaaaa
**MAP1A (SEQ ID NO: 50)** - Homo sapiens microtubule associated protein 1A (MAP1A), mRNA - NM 002373 
1 actcccaccc taagtgctgc agactcttcc ctgaagctgc cggctgaggc cggagctgcc
61 gcctccatga gaggcttcct cctacacccc agggccagag gaccctttgc caccagagtg
121 agatcctaga gaccatcatc ctggtaaatc ccagtgcaga cagcatcagc tctgaggttc
181 atcatcttct tagcagctca tcagcttata aactactaat cttgagtggg caaagtttag
241 agcctggggg agacctcatc ctacagagtg gcacctactc atatgaaaac tttgcccagg
301 tccttcacaa ccccgagatt tcccaattgc tcagcaatag agaccctggg atacaggcct
361 tccttaccgt gtcctgctta ggggaaggtg attggagcca cctgggatta tccagttccc
421 aagagaccct gcacctccgg ctaaaccctg agcccactct gcccaccatg gacggcgtgg
481 ctgagttctc cgagtatgtc tctgagactg tggacgtgcc atccccattt gacctactag
 
541 agccccccac ctcagggggc ttcctcaagc tctccaagcc ttgttgctac atcttcccag
601 gtggtcgtgg ggactctgcc ctctttgctg tcaatggttt caacatcctg gtggatggtg
661 gctctgatcg caagtcctgt ttttggaagc tggtacggca cttggaccgc attgactcgg
721 tgctactcac acacattggg gcagacaacc tgccaggcat caatggacta ctgcagcgca
781 aagtggcaga gctagaggag gagcagtccc agggctctag cagttacagc gactgggtga
841 agaaccttat ctctcctgag cttggagttg tctttttcaa cgtgcctgag aagctgcggc
901 ttcctgatgc ctcccggaaa gccaagcgta gcattgagga ggcctgcctc actctgcagc
961 acttaaaccg cctgggcatc caggctgagc ctctatatcg tgtggtcagc aataccattg
1021 agccactgac cctcttccac aaaatgggtg tgggccggct ggacatgtat gtcctcaacc
1081 ctgtcaagga cagcaaggag atgcagttcc tcatgcaaaa gtgggcaggc aatagtaaag
1141 ccaagacagg catcgtgctg cccaatggga aggaggctga gatctccgtg ccctacctta
1201 cctctatcac tgctctggtg gtctggctac cagccaatcc cactgagaag attgtgcgtg
1261 tgctttttcc aggaaatgct ccccaaaaca agatcttgga gggcctagaa aagcttcggc
1321 atctggactt cctgcgttac cctgtggcca cgcagaagga cctggcttct ggggctgtgc
1381 ctaccaacct caagcccagc aaaatcaaac agcgggctga tagcaaggag agcctcaaag
1441 ccactaccaa gacggccgtg agcaagttgg ccaaacggga ggaggtggta gaagagggag
1501 ccaaggaggc acgttcagag ctggccaagg agttagccaa gacagagaag aaggcaaaag
1561 agtcatctga gaagccccca gagaagcctg ccaagcctga gagggtgaag acagagtcaa
1621 gtgaggcact gaaggcagag aagcgaaagc tgatcaaaga caaggtaggg aaaaagcacc
1681 ttaaagaaaa gatatcaaag ctggaagaaa aaaaagacaa ggagaaaaaa gagatcaaaa
1741 aggagaggaa agagctcaag aaggatgaag gaaggaagga ggagaagaag gatgccaaga
1801 aggaggagaa gaggaaagat accaaacctg agctcaagaa gatttccaag ccagacctaa
1861 agccctttac tcctgaggta cgtaagaccc tctataaagc caaggtccct ggaagagtca
1921 aaatagacag gagccgtgct atccgtgggg agaaggagct gtcttctgag ccccagacac
1981 ccccagccca gaagggaact gtaccactcc caaccatcag tgggcacagg gagctggtcc
2041 tatcctcacc agaggacctc acacaggact ttgaggagat gaagcgtgag gagagggctt
2101 tgctggctga acaaagggac acaggactag gagataagcc attccctcta gacactgcag
2161 aggagggacc cccaagtaca gctatccagg gaacaccacc ctctgttcca gggctgggac
2221 aagaagaaca tgtgatgaag gagaaagagc ttgtcccaga ggtccctgag gaacaaggca
2281 gcaaggacag aggcctagac tctggggctg aaacagagga agagaaagat acctgggagg
2341 aaaagaagca gagggaagca gagaggctcc cagacagaac agaagccaga gaggaaagtg
2401 aacctgaagt aaaggaggat gtgatagaaa aggctgagtt agaagaaatg gaggaggtac
2461 acccttcaga tgaggaggaa gaggacgcga caaaagctga gggtttttac caaaaacata
2521 tgcaggaacc cttgaaggta actccaagga gccgggaggc ttttgggggt cgggaattgg
2581 gactccaggg caaggcccct gagaaggaga cctcgttatt cctaagcagc ctgaccacac
2641 ctgcaggagc cactgagcat gtctcttaca tccaggatga gacaatccct ggctactcag
2701 agactgagca gaccatctca gatgaggaga tccatgatga gccggaggag cgcccagctc
2761 cacccagatt tcatacaagt acatatgacc tgcccgggcc tgaaggtgct ggcccattcg
2821 aagccagcca acctgccgat agtgctgttc ctgctacctc tggcaaagtc tatggaacgc
2881 cagagactga actcacctac cccactaaca tagtggctgc ccctttggct gaagaggaac
2941 atgtgtcctc ggccacttca atcactgagt gtgacaaact ttcttccttt gccacatcag
3001 tggctgagga ccaatctgtg gcctcactta cagctcccca gacagaggag acaggcaaga
3061 gctccctgct gcttgacaca gtcacaagca tcccttcctc ccgtactgaa gctacgcagg
3121 gcttggacta tgtgccatca gctggtacca tctcacccac ctcctcactg gaagaagaca
3181 agggcttcaa atcaccaccc tgtgaggact tctctgtgac tggggagtca gagaagagag
3241 gagagatcat agggaaaggc ttgtctggag agagagctgt ggaagaggaa gaggaggaga
3301 cagcaaacgt agagatgtct gagaaacttt gcagtcaata tggaactcca gtgtttagtg
3361 cccctgggca tgccctacat ccaggagaac cagcccttgg agaagcagag gagcggtgcc
3421 ttagcccaga tgacagcaca gtgaagatgg cttctcctcc accatctggc ccacccagtg
3481 ccacccacac accctttcat cagtccccag tggaagaaaa gtctgagccc caagactttc
3541 aggaggcaga ctcctgggga gacactaagc gcacaccagg tgtgggcaaa gaagatgctg
3601 ctgaggagac agtcaagcca gggcctgaag agggcacact agagaaggaa gagaaagttc
3661 ctcctcccag gagcccccag gcccaggaag cacctgtcaa cattgatgag gggcttacag
3721 gctgtaccat tcaactgttg ccagcacagg ataaagcaat agtctttgag attatggagg
3781 caggagagcc cacaggccca attctgggag cagaagccct tcccggaggt ttgaggactt
3841 taccccaaga acctggcaaa cctcagaaag atgaggtgct cagatatcct gaccgaagcc
 
3901 tctctcctga agatgcagaa tccctctctg tcctcagcgt gccctcccca gacactgcca
3961 accaagagcc tacccccaag tctccctgtg gcctgacaga acagtaccta cacaaagacc
4021 gttggccaga ggtatctcca gaagacaccc agtcactttc tctgtcagaa gagagtccca
4081 gcaaggagac ctccctggat gtctcttcta agcagctctc tccagaaagc cttggcaccc
4141 tccagtttgg ggaactaaac cttgggaagg aagaaatggg gcatctgatg caggccgagg
4201 atacctctca ccacacagct cccatgtctg ttccagagcc ccatgcagcc acagcgtcac
4261 ctcccacaga tgggacaact cgatactctg cacagacaga catcacagat gacagccttg
4321 acaggaagtc acctgccagc tcattctctc actctacacc ttcaggaaat gggaagtact
4381 tacctggggc gatcacaagc cctgatgaac acattctgac acctgatagc tccttctcca
4441 agagtcctga gtctttgcca ggccctgcct tggaggacat tgccataaag tgggaagata
4501 aagttccagg gttgaaagac agaacctcag aacagaagaa ggaacctgag ccaaaggatg
4561 aagttttaca gcagaaagac aaaactctgg agcacaagga ggtggtagag ccgaaggata
4621 cagccatcta tcagaaagat gaggctctgc atgtaaagaa tgaggctgtg aaacagcagg
4681 ataaggcttt agaacaaaag ggcagagact tagagcaaaa agacacagcc ctagaacaga
4741 aggacaaggc cctggaacca aaagacaaag acttagaaga aaaagacaag gccctggaac
4801 agaaggataa gattccagaa gagaaagaca aagccttaga acaaaaggat acagccctgg
4861 aacagaagga caaggccctg gaaccaaaag ataaagactt ggaacaaaag gacagggtcc
4921 tagaacagaa ggagaagatc ccagaagaga aagacaaagc cttagatcaa aaagtcagaa
4981 gtgttgaaca taaggctccg gaggacacgg tcgctgaaat gaaggacaga gacctagaac
5041 agacagacaa agcccctgaa cagaaacacc aggcccagga acaaaaggat aaagtctcag
5101 aaaagaagga tcaggcctta gaacaaaaat actgggcttt gggacagaag gatgaagccc
5161 tggaacaaaa cattcaggct ctggaagaga accaccaaac tcaggagcag gagagcctag
5221 tgcaggagga taaaaccagg aaaccaaaga tgctagagga aaaatcccca gaaaaggtca
5281 aggccatgga agagaagtta gaagctcttc tggagaagac caaagctctg ggcctggaag
5341 agagcctagt gcaggagggc agggccagag agcaggaaga aaagtactgg agggggcagg
5401 atgtggtcca ggagtggcaa gaaacatctc ctaccagaga ggagccggct ggagaacaga
5461 aagagcttgc cccggcatgg gaggacacat ctcctgagca ggacaatagg tattggaggg
5521 gcagagagga tgtggccttg gaacaggaca catactggag ggagctaagc tgtgagcgga
5581 aggtctggtt ccctcacgag ctggatggcc aggggcccgg cccacactac actgaggaac
5641 gggaaagcac tttcctagat gagggcccag atgatgagca agaagtaccc ctgcgggaac
5701 acgcaacccg gagcccctgg gcctcagact tcaaggattt ccaggaatcc tcaccacaga
5761 aggggctaga ggtggagcgc tggcttgctg aatcaccagt tgggttgcca ccagaggaag
5821 aggacaaact gacccgctct ccctttgaga tcatctcccc tccagcttcc ccacctgaga
5881 tggttggaca aagggttcct tcagccccag gacaagagag tcctatccca gaccctaagc
5941 tcatgccaca catgaagaat gaacccacta ctccctcatg gctggctgac atcccaccct
6001 gggtgcccaa ggacagaccc ctcccccctg cacccctctc cccagctcct ggtcccccca
6061 cacctgcccc ggaatcccat actcctgcac ccttctcttg gggcacagcc gagtatgaca
6121 gtgtggtggc tgcagtgcag gagggggcag ctgagttgga aggtgggcca tactcccccc
6181 tggggaagga ctaccgcaag gctgaagggg aaagggaaga agaaggtagg gctgaggctc
6241 ctgacaaaag ctcacacagc tcaaaggtac cagaggccag caaaagccat gccaccacgg
6301 agcctgagca gactgagccg gagcagagag agcccacacc ctatcctgat gagagaagct
6361 ttcagtatgc agacatctat gagcagatga tgcttactgg gcttggccct gcatgcccca
6421 ctagagagcc tccacttgga gcagctgggg attggccccc atgcctctca accaaggagg
6481 cagctgccgg ccgaaacaca tctgcagaga aggagctttc atctcctatc tcacccaaga
6541 gcctccagtc tgacactcca accttcagct atgcagccct ggcaggaccc actgtacccc
6601 caaggccaga gccagggcca agtatggagc ccagcctcac cccacctgca gttccccccc
6661 gtgctcctat cctgagcaaa ggcccaagcc cccctcttaa tggtaacatc ctgagctgca
6721 gcccagatag gaggtcccca tcccccaagg aatcaggccg gagtcactgg gatgacagca
6781 ctagtgactc agaactggag aagggggctc gggaacagcc agaaaaagag gcccaatccc
6841 caagtcctcc tcaccccatt cctatggggt cccccacatt atggccagaa actgaggcac
6901 atgttagccc tcccttggac tcacacctgg ggcctgcccg acccagtctg gacttccctg
6961 cttcagcctt tggcttctcc tcattgcagc cagctccccc acagctgccc tctccagctg
7021 aaccccgctc ggcaccctgt ggctcccttg ccttctctgg ggatcgagct ctggctctgg
7081 ctccaggacc ccccaccaga acccggcatg atgaatacct ggaagtgacc aaggccccca
7141 gcctggattc ctcactgccc cagctcccat cacccagttc tcctggggcc cctctcctct
7201 ccaatctgcc acgacctgcc tcaccagccc tgtctgaggg ctcctcctct gaggctacca
 
7261 cgcctgtgat ttcaagtgtg gcggagcgct tctctccaag ccttgaggct gcagaacagg
7321 agtctggaga gctggaccca ggaatggaac cagctgccca cagcctctgg gacctcactc
7381 ctctgagccc agcaccccca gcttcactgg acttggccct agctccagct ccaagcctgc
7441 ctggagacat gggtgatggc atcctgccgt gccacctgga gtgctcagag gcagccacgg
7501 agaagccaag ccccttccag gttccctctg aggattgtgc agccaatggc ccaactgaaa
7561 ccagccctaa ccccccaggc cctgccccag ccaaggctga aaatgaagag gctgcggctt
7621 gccctgcctg ggaacgtggg gcctggcctg aaggagctga gaggagctcc cggcctgaca
7681 cattgctctc ccctgagcag ccagtgtgtc ctgcaggggg ctccgggggc ccacccagca
7741 gtgcctctcc tgaggtcgaa gctgggcccc agggatgtgc cactgagcct cggccccatc
7801 gtggggagct ctccccatcc ttcctgaacc cacctctgcc cccatccata gatgataggg
7861 acctctcaac tgaggaagtt cggctagtag gaagaggggg gcggcgccgg gtaggggggc
7921 cagggaccac tgggggccca tgccctgtga ctgatgagac accccctaca tcagccagtg
7981 actcaggctc ctcacagtca gattctgatg tcccgccaga aactgaggag tgtccgtcca
8041 tcacagctga ggcagccctc gactcagatg aagatggaga cttcctacct gtggacaaag
8101 ctgggggtgt cagtggtact caccacccca ggcctggcca tgacccacct cctctcccac
8161 agccagaccc ccgcccatcc cctccccgcc ctgatgtgtg catggctgac cccgaggggc
8221 tcagctcaga gtctgggaga gtagagaggc tacgggagaa ggaaaaggtt caggggcgag
8281 tagggcgcag ggccccaggc aaggccaagc cagcgtcccc tgcacggcgt ctggatcttc
8341 ggggaaaacg ctcacccacc cctggtaaag ggcctgcaga tcgagcatcc cgggccccac
8401 ctcgaccacg cagcaccaca agccaggtca ccccagcaga ggaaaaggat ggacacagcc
8461 ccatgtccaa aggcctagtc aatggactca aggcaggacc aatggccttg agttccaagg
8521 gcagctctgg tgcccctgta tatgtggatc tcgcctacat cccgaatcat tgcagtggca
8581 agactgctga ccttgacttc ttccgtcgag tgcgtgcatc ctactatgtg gtcagtggga
8641 atgaccctgc caatggcgag ccaagccggg ctgtgctgga tgccctgctg gagggcaagg
8701 cccagtgggg ggagaatctt caggtgactc tgatccctac tcatgacacg gaggtgactc
8761 gtgagtggta ccaacaaact catgagcagc agcaacaact gaatgtcctg gtcctggcta
8821 gcagcagcac cgtggtgatg caggatgagt ccttccctgc ctgcaagatt gagttctgaa
8881 agagccgccc tcccttcccc aaggatccac tcccccagct cctttagaga atggctactg
8941 ctgagtcctt tggggttgag ggagatggga gctaggggga ggggagggag atgtcttgtt
9001 gtggggactt gggctgggct aaatgggagg ggttgtccct ccccatcatc cattcctgtg
9061 aggtgtctca aaccaaagtt aacagggaga ggatggggga ggggacaaat tagaatagga
9121 tagcatctga tgcctgagaa ccctctccta gcactgtcaa atgctggtat tgaatgggga
9181 ctgaggatgg gtctcagaga gcaacctcct ccctcgtaga gggagattat atccccaact
9241 ccagggacct ctttatctca atctatttat ttggcatcct gggagggatt tccaatagta
9301 atttatgtga cctggggcag gataccgtca gtgaggtgcc cagagctgca ccctttcctc
9361 catttcccat cccccatctc ctcaaccacc agggtctgag ttctagcagg gtcctggggg
9421 tatcccactg ctatactgtt ctactgcttc cctcagtatc tgaatgtctc aatttaaaac
9481 ttgaagctct ttagaccaat agactggtga gaggagaaag gagcttatcc cccagaccct
9541 gctttatacc attcacatcc cagggctgtg tccagacagc acaaaacggc aaggagagcc
9601 caagccccaa tgccagaatt cttccaaact ccctgactct ttgaagtttt tactcacccc
9661 atttcaatta tcctgatccc ttctcatccc ctgcttggct tctctgcatg tggtcatctg
9721 ctgtggcttg gtgtttaatg ggttaaaaat aagccactgc ctgacatccc aacatttgac
9781 accccagcaa tgtgtgactc ccccaacatt ccactatgcc atcctgcagc tgaaatggga
9841 acactggctg cctctccaaa cccgctcttg gacagaggat ctgggaggtg gaagccaggc
9901 cagaggactt ggggaaaatg agatggagga aggaaaaagg gagaagctga gccacagctt
9961 aactcctaca gagtgaaatg aaaacgggct gaaaatacca ccccaggaga ggacctcgcc
10021 ccaagcaagc cagtgagcag ccctgccaga ctactgccag actgagaaac ccagaagctg
10081 gtagtcatgt gggcttgcct tctctgccaa acgactggga aaccaaaatg agcccacctt
10141 gtgttcttcc tagctccacc ctccccgtgc tgctgtgttc tgctcctccc cacgcttccc
10201 tgctatagtt cccagctgct gtaacggagc cacctccaac tctaacaata aaccaagttc
10261 attgcagata gtgta
**SELP (SEQ ID NO: 51)** - Homo sapiens selectin P (SELP), mRNA - NM 003005 
1 ggcagtgaga ctgtaagcag tctgggttgg gcagaaggca gaaaaccagc agagtcacag
61 aggagatggc caactgccaa atagccatct tgtaccagag attccagaga gtggtctttg
 
121 gaatttccca actcctttgc ttcagtgccc tgatctctga actaacaaac cagaaagaag
181 tggcagcatg gacttatcat tacagcacaa aagcatactc atggaatatt tcccgtaaat
241 actgccagaa tcgctacaca gacttagtgg ccatccagaa taaaaatgaa attgattacc
301 tcaataaggt cctaccctac tacagctcct actactggat tgggatccga aagaacaata
361 agacatggac atgggtggga accaaaaagg ctctcaccaa cgaggctgag aactgggctg
421 ataatgaacc taacaacaaa aggaacaacg aggactgcgt ggagatatac atcaagagtc
481 cgtcagcccc tggcaagtgg aatgatgagc actgcttgaa gaaaaagcac gcattgtgtt
541 acacagcctc ctgccaggac atgtcctgca gcaaacaagg agagtgcctc gagaccatcg
601 ggaactacac ctgctcctgt taccctggat tctatgggcc agaatgtgaa tacgtgagag
661 agtgtggaga acttgagctc cctcaacacg tgctcatgaa ctgcagccac cctctgggaa
721 acttctcttt taactcgcag tgcagcttcc actgcactga cgggtaccaa gtaaatgggc
781 ccagcaagct ggaatgcttg gcttctggaa tctggacaaa taagcctcca cagtgtttag
841 ctgcccagtg cccacccctg aagattcctg aacgaggaaa catgacctgc cttcattctg
901 caaaagcatt ccagcatcag tctagctgca gcttcagttg tgaagaggga tttgcattag
961 ttggaccgga agtggtgcaa tgcacagcct cgggggtatg gacagcccca gccccagtgt
1021 gtaaagctgt gcagtgtcag cacctggaag cccccagtga aggaaccatg gactgtgttc
1081 atccgctcac tgcttttgcc tatggctcca gctgtaaatt tgagtgccag cccggctaca
1141 gagtgagggg cttggacatg ctccgctgca ttgactctgg acactggtct gcacccttgc
1201 caacctgtga ggctatttcg tgtgagccgc tggagagtcc tgtccacgga agcatggatt
1261 gctctccatc cttgagagcg tttcagtatg acaccaactg tagcttccgc tgtgctgaag
1321 gtttcatgct gagaggagcc gatatagttc ggtgtgataa cttgggacag tggacagcac
1381 cagccccagt ctgtcaagct ttgcagtgcc aggatctccc agttccaaat gaggcccggg
1441 tgaactgctc ccaccccttc ggtgccttta ggtaccagtc agtctgcagc ttcacctgca
1501 atgaaggctt gctcctggtg ggagcaagtg tgctacagtg cttggctact ggaaactgga
1561 attctgttcc tccagaatgc caagccattc cctgcacacc tttgctaagc cctcagaatg
1621 gaacaatgac ctgtgttcaa cctcttggaa gttccagtta taaatccaca tgtcaattca
1681 tctgtgacga gggatattct ttgtctggac cagaaagatt ggattgtact cgatcgggac
1741 gctggacaga ctccccacca atgtgtgaag ccatcaagtg cccagaactc tttgccccag
1801 agcagggcag cctggattgt tctgacactc gtggagaatt caatgttggc tccacctgcc
1861 atttctcttg tgacaacggc tttaagctgg aggggcccaa taatgtggaa tgcacaactt
1921 ctggaagatg gtcagctact ccaccaacct gcaaaggcat agcatcactt cctactccag
1981 gggtgcaatg tccagccctc accactcctg ggcagggaac catgtactgt aggcatcatc
2041 cgggaacctt tggttttaat accacttgtt actttggctg caacgctgga ttcacactca
2101 taggagacag cactctcagc tgcagacctt caggacaatg gacagcagta actccagcat
2161 gcagagctgt gaaatgctca gaactacatg ttaataagcc aatagcgatg aactgctcca
2221 acctctgggg aaacttcagt tatggatcaa tctgctcttt ccattgtcta gagggccagt
2281 tacttaatgg ctctgcacaa acagcatgcc aagagaatgg ccactggtca actaccgtgc
2341 caacctgcca agcaggacca ttgactatcc aggaagccct gacttacttt ggtggagcgg
2401 tggcttctac gataggtctg ataatgggtg ggacgctcct ggctttgcta agaaagcgtt
2461 tcagacaaaa agatgatggg aaatgcccct tgaatcctca cagccaccta ggaacatatg
2521 gagtttttac aaacgctgca tttgacccga gtccttaagg tttccataaa cacccatgaa
2581 tcaaagacat ggaattacct tagattagct ctggaccagc ctgttggacc cgctctggac
2641 caaccctgtt tcctgagttt gggattgtgg tacaatctca aattctcaac ctaccacccc
2701 ttcctgtccc acctcttctc ttcctgtaac acaagccaca gaagccagga gcaaatgttt
2761 ctgcagtagt ctctgtgctt tgactcacct gttacttgaa ataccagtga accaaagaga
2821 ctggagcatc tgactcacaa gaagaccaga ctgtggagaa ataaaaatac ctctttattt
2881 tttgattgaa ggaaggtttt ctccactttg ttggaaagca ggtggcatct ctaattggaa
2941 gaaattcctg tagcatcttc tggagtctcc agtggttgct gttgatgagg cctcttggac
3001 ctctgctctg aggcttccag agagtcctct ggatggcacc agaggctgca gaaggccaag
3061 aatcaagcta gaaggccaca tgtcaccgtg gaccttcctg ccaccagtca ctgtccctca
3121 aatgacccaa agaccaatat tcaaatgcgt aattaaaaga attttcccca aaaaaaaaaa
3181 aaaaa
**NEXN (SEQ ID NO: 52)** nexilin F-actin binding protein, transcript variant 1, mRNA NM 144573.3 1 aacagctgca gccggcgctg ggcccgcctg gaatgcggga acaggctgca caccaggact 
61 tttatggaaa cttgctgctg gagacggcgg cggcggcggc ggcagcggca gccagaggac
121 tcccagcggc tggagcagaa gtgttagcgg ccagagctcc cagaccccta cccacagcca
181 ggcgggacgc gcacagtccc tccacgcgga aagaagtacc ttcgccggtc accggctcct
241 gcagggtgca aatatataca gagcttcata atcagcccaa gaccacatag agcaaacatg
301 aatgatattt cccaaaaggc tgagattctg ctttcttcat ctaaacctgt cccaaaaacc
361 tatgtaccaa aacttggcaa gggtgatgta aaggataagt ttgaagccat gcagagagcc
421 agggaagaaa gaaatcaaag gagatctaga gacgaaaaac aaagaagaaa agaacaatat
481 attagagaga gagaatggaa caggagaaag caggagatta aagaaatgct tgcttctgat
541 gatgaggaag atgtatcttc taaagtagaa aaggcttatg ttccaaaatt aacaggaact
601 gtgaagggta gatttgctga aatggagaaa caaagacaag aggaacaaag gaagagaacg
661 gaggaggaac gaaaacgcag aattgagcag gatatgttag aaaagaggaa aatacagcgt
721 gaattagcaa aaagggctga acagattgag gacataaaca atacgggaac tgaatcagca
781 tcagaggaag gagatgattc actacttata actgtggtac ctgtcaaatc atataaaaca
841 tctggaaaaa tgaaaaagaa ttttgaggat ctagaaaaag aacgtgaaga gaaagaaagg
901 atcaagtacg aggaagataa aagaataaga tatgaagaac aacgaccatc tctcaaggaa
961 gcaaagtgtc tttcattagt tatggatgat gaaatagaaa gtgaagcaaa aaaagaatca
1021 ctttctcccg gaaaattgaa actaactttt gaagaactgg agcgacaaag acaagaaaac
1081 cgaaagaagc aagctgaaga ggaagcaaga aaacgtttag aagaagagaa gcgtgctttt
1141 gaagaagcaa ggcggcaaat ggtaaatgaa gatgaggaaa accaagacac agcaaaaatt
1201 tttaaagggt accgccctgg taaactcaaa ctcagttttg aagaaatgga aaggcaaaga
1261 agagaagatg aaaaaaggaa agcagaagaa gaagccagaa ggagaataga ggaagaaaag
1321 aaggcgtttg ctgaagcaag gagaaatatg gtagtagatg atgactcccc agagatgtat
1381 aagacaatct ctcaagaatt tcttacaccg ggaaaactgg aaattaattt tgaagaatta
1441 ttaaaacaaa aaatggaaga agaaaaacga cgaacagagg aggaacggaa gcataagcta
1501 gaaatggaga aacaagaatt tgaacaactg agacaggaaa tgggagagga agaggaagaa
1561 aatgaaacct ttggattgag cagagaatat gaagaactga tcaaattaaa aaggagtggc
1621 tctattcaag ctaaaaacct aaaaagcaag tttgaaaaaa ttggacagtt gtctgaaaaa
1681 gaaatacaga aaaaaataga agaagagcga gcaagaagga gagcaattga ccttgaaatt
1741 aaagagcgag aagctgaaaa ttttcatgag gaagatgatg ttgatgttag gcctgcaaga
1801 aaaagcgagg ctccatttac tcacaaagtg aatatgaaag ctagatttga acaaatggct
1861 aaggcaagag aagaagaaga acaaagaaga attgaagaac aaaagttact acgcatgcag
1921 tttgaacaaa gggaaattga tgcagcacta caaaagaaaa gagaagagga ggaggaggaa
1981 gaaggtagca tcatgaatgg ctccactgct gaagatgaag agcaaaccag atcaggagct
2041 ccatggttca agaagcctct taaaaacaca tcagttgtag acagtgagcc agtcagattt
2101 acggttaaag taacaggaga acccaaacca gaaattacat ggtggtttga aggagaaata
2161 ctgcaggatg gagaagacta tcaatatatt gaaaggggag aaacttactg cctttactta
2221 ccagaaactt tcccagaaga tggaggagag tatatgtgta aagcagtcaa caataaagga
2281 tctgcagcta gtacctgtat tcttaccatt gaaagtaaga attaatcact ctttttatct
2341 tttattctat taattttttt ttccttaaaa tcacttttct tcttctcttt tttagctgat
2401 gactactagc tcccctcccc tctccctgga actttctctt tcactccaac tttcttacta
2461 catccatctt ttctgtggcg gggccaaaaa aggaaaccag gagtgccact atgctgactt
2521 cttattcctt ttcataacag tcttcaaagc acagctcatc taaagaatgc ctacttcttt
2581 tccaaataag catcagattt atcgcctatt atgcagtaac agtcaataaa atgtacttat
2641 gggggggaat tactcaatta ttctatcaga acctattata aagactgtat ttcccataga
2701 cgtttacagc aactatgttt aaaaaacaaa aacaaaaaaa aaacacacaa acctaagtag
2761 aatacattat tttgcatgaa ggaatgtcat ttctgagctt tttacaccta aaattaggct
2821 gaaatagctg agataattaa tttggaacct atcaatttga gtggactttt tctttagtag
2881 tacaccattt tggttgttgt agtttcaaag tctttctgaa gcagatatat tgggattgga
2941 gcggggtggg gaaaactgtc actcctttca gaggaaaagg ggaggagcat ggagaaaaac
3001 aaaaattaaa ggacttaaag aatggctata cagtgttgag tgttgaggat attaaacatg
3061 ttatttttca aacgtatgta atatatatta aatttataaa gcaaatttat gttgtgatct
3121 tgcctgaaca aattatattt taatgaaaaa actttctatt aatagttcac gcaagagaaa
3181 acactttcaa catagtcgaa ggcttcaaga tctaagtgta tcagacttag ggaaaaagtg
3241 gcacaacctt cgatttaaaa ttctagtctt taaaatgagt ttgtaaataa ttagctatta
3301 cgttctatta agttgtttta tattttaatt ttctggaaga caattttatt ttacaacgtg
3361 aacccaaata aagtaacttc tgtatttaaa aaaaaaaaaa aaaaa
**ITGA2B (SEQ ID NO: 53)** - Homo sapiens integrin subunit alpha 2b (ITGA2B), mRNA - NM 000419 
1 gctctgcccg ttgctcagca agttacttgg ggttccagtt tgataagaaa agacttcctg
61 tggaggaatc tgaagggaag gaggaggagc tggcccattc ctgcctggga ggttgtggaa
121 gaaggaagat ggccagagct ttgtgtccac tgcaagccct ctggcttctg gagtgggtgc
181 tgctgctctt gggaccttgt gctgcccctc cagcctgggc cttgaacctg gacccagtgc
241 agctcacctt ctatgcaggc cccaatggca gccagtttgg attttcactg gacttccaca
301 aggacagcca tgggagagtg gccatcgtgg tgggcgcccc gcggaccctg ggccccagcc
361 aggaggagac gggcggcgtg ttcctgtgcc cctggagggc cgagggcggc cagtgcccct
421 cgctgctctt tgacctccgt gatgagaccc gaaatgtagg ctcccaaact ttacaaacct
481 tcaaggcccg ccaaggactg ggggcgtcgg tcgtcagctg gagcgacgtc attgtggcct
541 gcgccccctg gcagcactgg aacgtcctag aaaagactga ggaggctgag aagacgcccg
601 taggtagctg ctttttggct cagccagaga gcggccgccg cgccgagtac tccccctgtc
661 gcgggaacac cctgagccgc atttacgtgg aaaatgattt tagctgggac aagcgttact
721 gtgaagcggg cttcagctcc gtggtcactc aggccggaga gctggtgctt ggggctcctg
781 gcggctatta tttcttaggt ctcctggccc aggctccagt tgcggatatt ttctcgagtt
841 accgcccagg catccttttg tggcacgtgt cctcccagag cctctccttt gactccagca
901 acccagagta cttcgacggc tactgggggt actcggtggc cgtgggcgag ttcgacgggg
961 atctcaacac tacagaatat gtcgtcggtg cccccacttg gagctggacc ctgggagcgg
1021 tggaaatttt ggattcctac taccagaggc tgcatcggct gcgcggagag cagatggcgt
1081 cgtattttgg gcattcagtg gctgtcactg acgtcaacgg ggatgggagg catgatctgc
1141 tggtgggcgc tccactgtat atggagagcc gggcagaccg aaaactggcc gaagtggggc
1201 gtgtgtattt gttcctgcag ccgcgaggcc cccacgcgct gggtgccccc agcctcctgc
1261 tgactggcac acagctctat gggcgattcg gctctgccat cgcacccctg ggcgacctcg
1321 accgggatgg ctacaatgac attgcagtgg ctgcccccta cgggggtccc agtggccggg
1381 gccaagtgct ggtgttcctg ggtcagagtg aggggctgag gtcacgtccc tcccaggtcc
1441 tggacagccc cttccccaca ggctctgcct ttggcttctc ccttcgaggt gccgtagaca
1501 tcgatgacaa cggataccca gacctgatcg tgggagctta cggggccaac caggtggctg
1561 tgtacagagc tcagccagtg gtgaaggcct ctgtccagct actggtgcaa gattcactga
1621 atcctgctgt gaagagctgt gtcctacctc agaccaagac acccgtgagc tgcttcaaca
1681 tccagatgtg tgttggagcc actgggcaca acattcctca gaagctatcc ctaaatgccg
1741 agctgcagct ggaccggcag aagccccgcc agggccggcg ggtgctgctg ctgggctctc
1801 aacaggcagg caccaccctg aacctggatc tgggcggaaa gcacagcccc atctgccaca
1861 ccaccatggc cttccttcga gatgaggcag acttccggga caagctgagc cccattgtgc
1921 Lcagcclcaa LgLgLcccLa ccgcccacgg aggcLggaaL ggccccLgcL gLcgLgcLgc
1981 atggagacac ccatgtgcag gagcagacac gaatcgtcct ggactgtggg gaagatgacg
2041 tatgtgtgcc ccagcttcag ctcactgcca gcgtgacggg ctccccgctc ctagttgggg
2101 cagataatgt cctggagctg cagatggacg cagccaacga gggcgagggg gcctatgaag
2161 cagagctggc cgtgcacctg ccccagggcg cccactacat gcgggcccta agcaatgtcg
2221 agggctttga gagactcatc tgtaatcaga agaaggagaa tgagaccagg gtggtgctgt
2281 gtgagctggg caaccccatg aagaagaacg cccagatagg aatcgcgatg ttggtgagcg
2341 tggggaatct ggaagaggct ggggagtctg tgtccttcca gctgcagata cggagcaaga
2401 acagccagaa tccaaacagc aagattgtgc tgctggacgt gccggtccgg gcagaggccc
2461 aagtggagct gcgagggaac tcctttccag cctccctggt ggtggcagca gaagaaggtg
2521 agagggagca gaacagcttg gacagctggg gacccaaagt ggagcacacc tatgagctcc
2581 acaacaatgg ccctgggact gtgaatggtc ttcacctcag catccacctt ccgggacagt
2641 cccagccctc cgacctgctc tacatcctgg atatacagcc ccaggggggc cttcagtgct
2701 tcccacagcc tcctgtcaac cctctcaagg tggactgggg gctgcccatc cccagcccct
2761 cccccattca cccggcccat cacaagcggg atcgcagaca gatcttcctg ccagagcccg
2821 agcagccctc gaggcttcag gatccagttc tcgtaagctg cgactcggcg ccctgtactg
2881 tggtgcagtg tgacctgcag gagatggcgc gcgggcagcg ggccatggtc acggtgctgg
2941 ccttcctgtg gctgcccagc ctctaccaga ggcctctgga tcagtttgtg ctgcagtcgc
3001 acgcatggtt caacgtgtcc tccctcccct atgcggtgcc cccgctcagc ctgccccgag
3061 gggaagctca ggtgtggaca cagctgctcc gggccttgga ggagagggcc attccaatct
3121 ggtgggtgct ggtgggtgtg ctgggtggcc tgctgctgct caccatcctg gtcctggcca
 
3181 tgtggaaggt cggcttcttc aagcggaacc ggccacccct ggaagaagat gatgaagagg
3241 gggagtgatg gtgcagccta cactattcta gcaggagggt tgggcgtgct acctgcaccg
3301 ccccttctcc aacaagttgc ctccaagctt tgggttggag ctgttccatt gggtcctctt
3361 ggtgtcgttt ccctcccaac agagctgggc taccccccct cctgctgcct aataaagaga
3421 ctgagccctg aaaaaaaaaa aaaaaaaaa
**MYL9 (SEQ ID NO: 54)** - Homo sapiens myosin light chain 9 (MYL9), transcript variant 2, mRNA - NM 181526 
1 gcccccgcct ggagtccaga cccgacggcc ggcccagttc cacgcaccca gcgagcccaa
61 gcgccttctc cgcaccaggg aagccccacc caccagaagc caagatgtcc agcaagcggg
121 ccaaagccaa gaccaccaag aagcggccac agcgggccac atccaatgtc ttcgcaatgt
181 ttgaccagtc ccagatccag gagtttaagg aggctttcaa catgattgac cagaaccgtg
241 atggcttcat tgacaaggag gacctgcacg acatgctggc ctcgctgggt ttcatccatg
301 aggaccacct ccgggagctg ctcaccacca tgggtgaccg cttcacagat gaggaagtgg
361 acgagatgta ccgggaggca cccattgata agaaaggcaa cttcaactac gtggagttca
421 cccgcatcct caaacatggc gccaaggata aagacgacta ggccacccca gccccctgac
481 accccagccc ccgccagtca cccctccccg cacacacccg tccataccag ctccctgccc
541 atgaccctcg ctcagggatc cccctttgag gggttagggt cccagttccc agtggaagaa
601 acaggccagg agaagtgcgt gccgagctga ggcagatgtt cccacagtga ccccagagcc
661 ctgggctata gtctctgacc cctccaagga aagaccacct tctggggaca tgggctggag
721 ggcaggacct agaggcacca agggaaggcc ccattccggg gctgttcccc gaggaggaag
781 ggaaggggct ctgtgtgccc cccaggagga agaggccctg agtcctggga tcagacaccc
841 cttcacgtgt atccccacac aaatgcaagc tcaccaaggt cccctctcag tccccttccc
901 tacaccctga ccggccactg ccgcacaccc acccagagca cgccacccgc catgggagtg
961 tgctcaggag tcgcgggcag cgtggacatc tgtcccagag ggggcagaat ctccaataga
1021 ggactgagca ctgctaaaaa aaaaaaaaaa aaaa
**ITGB3 (SEQ ID NO: 55)** integrin subunit beta 3, NM 000212.2 
1 cgccgcggga ggcggacgag atgcgagcgc ggccgcggcc ccggccgctc tgggcgactg
61 tgctggcgct gggggcgctg gcgggcgttg gcgtaggagg gcccaacatc tgtaccacgc
121 gaggtgtgag ctcctgccag cagtgcctgg ctgtgagccc catgtgtgcc tggtgctctg
181 atgaggccct gcctctgggc tcacctcgct gtgacctgaa ggagaatctg ctgaaggata
241 actgtgcccc agaatccatc gagttcccag tgagtgaggc ccgagtacta gaggacaggc
301 ccctcagcga caagggctct ggagacagct cccaggtcac tcaagtcagt ccccagagga
361 ttgcactccg gctccggcca gatgattcga agaatttctc catccaagtg cggcaggtgg
421 aggattaccc tgtggacatc tactacttga tggacctgtc ttactccatg aaggatgatc
481 tgtggagcat ccagaacctg ggtaccaagc tggccaccca gatgcgaaag ctcaccagta
541 acctgcggat tggcttcggg gcatttgtgg acaagcctgt gtcaccatac atgtatatct
601 ccccaccaga ggccctcgaa aacccctgct atgatatgaa gaccacctgc ttgcccatgt
661 ttggctacaa acacgtgctg acgctaactg accaggtgac ccgcttcaat gaggaagtga
721 agaagcagag tgtgtcacgg aaccgagatg ccccagaggg tggctttgat gccatcatgc
781 aggctacagt ctgtgatgaa aagattggct ggaggaatga tgcatcccac ttgctggtgt
841 ttaccactga tgccaagact catatagcat tggacggaag gctggcaggc attgtccagc
901 ctaatgacgg gcagtgtcat gttggtagtg acaatcatta ctctgcctcc actaccatgg
961 attatccctc tttggggctg atgactgaga agctatccca gaaaaacatc aatttgatct
1021 ttgcagtgac tgaaaatgta gtcaatctct atcagaacta tagtgagctc atcccaggga
1081 ccacagttgg ggttctgtcc atggattcca gcaatgtcct ccagctcatt gttgatgctt
1141 atgggaaaat ccgttctaaa gtagagctgg aagtgcgtga cctccctgaa gagttgtctc
1201 tatccttcaa tgccacctgc ctcaacaatg aggtcatccc tggcctcaag tcttgtatgg
1261 gactcaagat tggagacacg gtgagcttca gcattgaggc caaggtgcga ggctgtcccc
1321 aggagaagga gaagtccttt accataaagc ccgtgggctt caaggacagc ctgatcgtcc
1381 aggtcacctt tgattgtgac tgtgcctgcc aggcccaagc tgaacctaat agccatcgct
1441 gcaacaatgg caatgggacc tttgagtgtg gggtatgccg ttgtgggcct ggctggctgg
1501 gatcccagtg tgagtgctca gaggaggact atcgcccttc ccagcaggac gaatgcagcc
 
1561 cccgggaggg tcagcccgtc tgcagccagc ggggcgagtg cctctgtggt caatgtgtct
1621 gccacagcag tgactttggc aagatcacgg gcaagtactg cgagtgtgac gacttctcct
1681 gtgtccgcta caagggggag atgtgctcag gccatggcca gtgcagctgt ggggactgcc
1741 tgtgtgactc cgactggacc ggctactact gcaactgtac cacgcgtact gacacctgca
1801 tgtccagcaa tgggctgctg tgcagcggcc gcggcaagtg tgaatgtggc agctgtgtct
1861 gtatccagcc gggctcctat ggggacacct gtgagaagtg ccccacctgc ccagatgcct
1921 gcacctttaa gaaagaatgt gtggagtgta agaagtttga ccggggagcc ctacatgacg
1981 aaaatacctg caaccgttac tgccgtgacg agattgagtc agtgaaagag cttaaggaca
2041 ctggcaagga tgcagtgaat tgtacctata agaatgagga tgactgtgtc gtcagattcc
2101 agtactatga agattctagt ggaaagtcca tcctgtatgt ggtagaagag ccagagtgtc
2161 ccaagggccc tgacatcctg gtggtcctgc tctcagtgat gggggccatt ctgctcattg
2221 gccttgccgc cctgctcatc tggaaactcc tcatcaccat ccacgaccga aaagaattcg
2281 ctaaatttga ggaagaacgc gccagagcaa aatgggacac agccaacaac ccactgtata
2341 aagaggccac gtctaccttc accaatatca cgtaccgggg cacttaatga taagcagtca
2401 tcctcagatc attatcagcc tgtgccacga ttgcaggagt ccctgccatc atgtttacag
2461 aggacagtat ttgtggggag ggatttgggg ctcagagtgg ggtaggttgg gagaatgtca
2521 gtatgtggaa gtgtgggtct gtgtgtgtgt atgtgggggt ctgtgtgttt atgtgtgtgt
2581 gttgtgtgtg ggagtgtgta atttaaaatt gtgatgtgtc ctgataagct gagctcctta
2641 gcctttgtcc cagaatgcct cctgcaggga ttcttcctgc ttagcttgag ggtgactatg
2701 gagctgagca ggtgttcttc attacctcag tgagaagcca gctttcctca tcaggccatt
2761 gtccctgaag agaagggcag ggctgaggcc tctcattcca gaggaaggga caccaagcct
2821 tggctctacc ctgagttcat aaatttatgg ttctcaggcc tgactctcag cagctatggt
2881 aggaactgct gggcttggca gcccgggtca tctgtacctc tgcctccttt cccctccctc
2941 aggccgaagg aggagtcagg gagagctgaa ctattagagc tgcctgtgcc ttttgccatc
3001 ccctcaaccc agctatggtt ctctcgcaag ggaagtcctt gcaagctaat tctttgacct
3061 gttgggagtg aggatgtctg ggccactcag gggtcattca tggcctgggg gatgtaccag
3121 catctcccag ttcataatca caacccttca gatttgcctt attggcagct ctactctgga
3181 ggtttgttta gaagaagtgt gtcaccctta ggccagcacc atctctttac ctcctaattc
3241 cacaccctca ctgctgtaga catttgctat gagctgggga tgtctctcat gaccaaatgc
3301 ttttcctcaa agggagagag tgctattgta gagccagagg tctggcccta tgcttccggc
3361 ctcctgtccc tcatccatag cacctccaca tacctggccc tgtgccttgg tgtgctgtat
3421 ccatccatgg ggctgattgt atttaccttc tacctcttgg ctgccttgtg aaggaattat
3481 tcccatgagt tggctgggaa taagtgccag gatggaatga tgggtcagtt gtatcagcac
3541 gtgtggcctg ttcttctatg ggttggacaa cctcatttta actcagtctt taatctgaga
3601 ggccacagtg caattttatt ttatttttct catgatgagg ttttcttaac ttaaaagaac
3661 atgtatataa acatgcttgc attatatttg taaatttatg tgatggcaaa gaaggagagc
3721 ataggaaacc acacagactt gggcagggta cagacactcc cacttggcat cattcacagc
3781 aagtcactgg ccagtggctg gatctgtgag gggctctctc atgatagaag gctatgggga
3841 tagatgtgtg gacacattgg acctttcctg aggaagaggg actgttcttt tgtcccagaa
3901 aagcagtggc tccattggtg ttgacataca tccaacatta aaagccaccc ccaaatgccc
3961 aagaaaaaaa gaaagactta tcaacatttg ttccatgagc agaaaactgg agctctggcc
4021 tcagtgttac agctaaataa tctttaatta aggcaagtca ctttcttctt cttaaagctg
4081 ttttctagtt tgagaaatga tgggatttta gcagccagtc ttgaaggtct ctttcagtat
4141 caacattcta agatgctggg acttactgtg tcatcaaatg tgcggttaag attctctggg
4201 atattgatac tgtttgtgtt tttagttggg agatctgaga gacctggctt tggcaagagc
4261 agatgtcatt ccatatcacc tttctcaatg aaagtctcat tctatcctct ctccaaaccc
4321 gttttccaac atttgttaat agttacgtct ctcctgatgt agcacttaag cttcatttag
4381 ttattatttc tttcttcact ttgcacacat ttgcatccac atattaggga agaggaatcc
4441 ataagtagct gaaatatcta ttctgtatta ttgtgttaac attgagaata agccttggaa
4501 ttagatatgg ggcaatgact gagccctgtc tcacccatgg attactcctt actgtaggga
4561 atggcagtat ggtagaggga taaatagggg gcggggaggg atagtcatgg atccaagaag
4621 tccttagaaa tagtggcagg gaacaggtgt ggaagctcat gcctgtaatt ataaccttca
4681 gctactaaga caggtgtggt ggctcacgcc tgtgattata atcttcagtt actaagacag
4741 agtccatgag agtgttaatg ggacattttc tttagataag atgttttata tgaagaaact
4801 gtatcaaagg gggaagaaaa tgtatttaac aggtgaatca aatcaggaat cttgtctgag
4861 ctactggaat gaagttcaca ggtcttgaag acca
**CMTM5 (SEQ ID NO: 56)** - Homo sapiens CKLF like MARVEL transmembrane domain containing 5 (CMTM5), transcript variant 3, mRNA - NM 001037288 
1 gttagagaag ggggacacaa atgtcttcag gcttaggtcc ctgggggctc ccagtcctgc
61 ccctgttcct ctactcacat cccagctcct cccagtttct tttcggacct cctcctctcc
121 ccttccttct ctattccagg ctgggttggg ctctaagcaa ggggagggat tagagcctcc
181 ttcctctctg cccctcccca tgggtctcta gggggctggt gcaggcagca gcagaggcac
241 tctgggcagc tgggtgaggg cccatctggg caaggccccc agcgcctgcc ttctctcccg
301 gggccctgtg ggcaagcctc ctgcttcact ttcaggtttc tcgaagtgcc ttcttgctcc
361 tgtctgtttc cccatcctgc cagatttctg tttctcttgc tgggcttttg gcagtagggg
421 gctgtgttgg tgggccctac gaagatgctc agtgctcgag atcgccggga ccggcaccct
481 gaggaggggg tagttgcaga gctccagggc ttcgcggtgg acaaggcctt cctcacctcc
541 cacaagggca tcctgctgga aaccgagctg gccctgaccc tcatcatctt catctgcttc
601 acggcctcca tctctgccta catggccgcg gcgctactgg agttcttcat cacacttgcc
661 ttcctcttcc tctatgccac ccagtactac cagcgcttcg accgaattaa ctggccctgt
721 ctggtttttg gcatcatcct ggtttccatc tttgcctatg atgccttcaa gatctaccgg
781 actgagatgg cacccggggc cagccagggg gaccagcagt gactctgggg ctacctggct
841 cctaggccca gccagccaga gaggacagtg gagcccagac acgtctcctt gggattcact
901 agcccccagc ccgccaaacc ccaccccagc cctacacagc agtctggcct gagacgtcac
961 tggggactta tctgtggagc ctggtgctcc aggatgtggc ttctcatgaa gctctggcca
1021 gaggagggga acttattggg ggaggggggg tggaggggag gaatctggac ctctaagtca
1081 ttcccaaatt aaaatattca aattctaaaa aaaaaaaaaa a
**LCN2 (SEQ ID NO: 57)** Homo sapiens lipocalin 2 (LCN2), mRNA NM 005564.4 
1 agggccaccc aggtgagcct ctcactcgcc acctcctctt ccacccctgc caggcccagc
61 agccaccaca gcgcctgctt cctcggccct gaaatcatgc ccctaggtct cctgtggctg
121 ggcctagccc tgttgggggc tctgcatgcc caggcccagg actccacctc agacctgatc
181 ccagccccac ctctgagcaa ggtccctctg cagcagaact tccaggacaa ccaattccag
241 gggaagtggt atgtggtagg cctggcaggg aatgcaattc tcagagaaga caaagacccg
301 caaaagatgt atgccaccat ctatgagctg aaagaagaca agagctacaa tgtcacctcc
361 gtcctgttta ggaaaaagaa gtgtgactac tggatcagga cttttgttcc aggttgccag
421 cccggcgagt tcacgctggg caacattaag agttaccctg gattaacgag ttacctcgtc
481 cgagtggtga gcaccaacta caaccagcat gctatggtgt tcttcaagaa agtttctcaa
541 aacagggagt acttcaagat caccctctac gggagaacca aggagctgac ttcggaacta
601 aaggagaact tcatccgctt ctccaaatct ctgggcctcc ctgaaaacca catcgtcttc
661 cctgtcccaa tcgaccagtg tatcgacggc tgagtgcaca ggtgccgcca gctgccgcac
721 cagcccgaac accattgagg gagctgggag accctcccca cagtgccacc catgcagctg
781 ctccccaggc caccccgctg atggagcccc accttgtctg ctaaataaac atgtgccctc
841 aggccaaaaa aaaaaaaaaa aaa
**NLRC4 (SEQ ID NO: 58)** NLR family CARD domain containing 4 XM_011533008.1 
1 agaacaagaa ggtatctggt ctacaagaac tcgaggcctc actgaaacgg aaagcaaata
61 caaagaaact ttattttaaa aacatgtctt ggtctcccaa gaagagggca attggattgc
121 tcagccagaa tgaagagtag ttttacagaa aaaagaggac aatattggga tcacctttga
181 cctttccatt tggaaataat attttctatt gtgttataga aaggtgggaa gctttcatcc
241 agaacaatga atttcataaa ggacaatagc cgagccctta ttcaaagaat gggaatgact
301 gttataaagc aaatcacaga tgacctattt gtatggaatg ttctgaatcg cgaagaagta
361 aacatcattt gctgcgagaa ggtggagcag gatgctgcta gagggatcat tcacatgatt
421 ttgaaaaagg gttcagagtc ctgtaacctc tttcttaaat cccttaagga gtggaactat
481 cctctatttc aggacttgaa tggacaaagt ctttttcatc agacatcaga aggagacttg
541 gacgatttgg ctcaggattt aaaggacttg taccataccc catcttttct gaacttttat
601 ccccttggtg aagatattga cattattttt aacttgaaaa gcaccttcac agaacctgtc
661 ctgtggagga aggaccaaca ccatcaccgc gtggagcagc tgaccctgaa tggcctcctg
721 caggctcttc agagcccctg catcattgaa ggggaatctg gcaaaggcaa gtccactctg
 
781 ctgcagcgaa ttgccatgct ctggggctcc ggaaagtgca aggctctgac caagttcaaa
841 ttcgtcttct tcctccgtct cagcagggcc cagggtggac tttttgaaac cctctgtgat
901 caactcctgg atatacctgg cacaatcagg aagcagacat tcatggccat gctgctgaag
961 ctgcggcaga gggttctttt ccttcttgat ggctacaatg aattcaagcc ccagaactgc
1021 ccagaaatcg aagccctgat aaaggaaaac caccgcttca agaacatggt catcgtcacc
1081 actaccactg agtgcctgag gcacatacgg cagtttggtg ccctgactgc tgaggtgggg
1141 gatatgacag aagacagcgc ccaggctctc atccgagaag tgctgatcaa ggagcttgct
1201 gaaggcttgt tgctccaaat tcagaaatcc aggtgcttga ggaatctcat gaagacccct
1261 ctctttgtgg tcatcacttg tgcaatccag atgggtgaaa gtgagttcca ctctcacaca
1321 caaacaacgc tgttccatac cttctatgat ctgttgatac agaaaaacaa acacaaacat
1381 aaaggtgtgg ctgcaagtga cttcattcgg agcctggacc actgtggaga cctagctctg
1441 gagggtgtgt tctcccacaa gtttgatttc gaactgcagg atgtgtccag cgtgaatgag
1501 gatgtcctgc tgacaactgg gctcctctgt aaatatacag ctcaaaggtt caagccaaag
1561 tataaattct ttcacaagtc attccaggag tacacagcag gacgaagact cagcagttta
1621 ttgacgtctc atgagccaga ggaggtgacc aaggggaatg gttacttgca gaaaatggtt
1681 tccatttcgg acattacatc cacttatagc agcctgctcc ggtacacctg tgggtcatct
1741 gtggaagcca ccagggctgt tatgaagcac ctcgcagcag tgtatcaaca cggctgcctt
1801 ctcggacttt ccatcgccaa gaggcctctc tggagacagg aatctttgca aagtgtgaaa
1861 aacaccactg agcaagaaat tctgaaagcc ataaacatca attcctttgt agagtgtggc
1921 atccatttat atcaagagag tacatccaaa tcagccctga gccaagaatt tgaagctttc
1981 tttcaaggta aaagcttata tatcaactca gggaacatcc ccgattactt atttgacttc
2041 tttgaacatt tgcccaattg tgcaagtgcc ctggacttca ttaaactgga cttttatggg
2101 ggagctatgg cttcatggga aaaggctgca gaagacacag gtggaatcca catggaagag
2161 gccccagaaa cctacattcc cagcagggct gtatctttgt tcttcaactg gaagcaggaa
2221 ttcaggactc tggaggtcac actccgggat ttcagcaagt tgaataagca agatatcaga
2281 tatctgggga aaatattcag ctctgccaca agcctcaggc tgcaaataaa gagatgtgct
2341 ggtgtggctg gaagcctcag tttggtcctc agcacctgta agaacattta ttctctcatg
2401 gtggaagcca gtcccctcac catagaagat gagaggcaca tcacatctgt aacaaacctg
2461 aaaaccttga gtattcatga cctacagaat caacggctgc cgggtattgt ggtctgactg
2521 acagcttggg taacttgaag aaccttacaa agctcataat ggataacata aagatgaatg
2581 aagaagatgc tataa
**PPBP (SEQ ID NO: 59)** - Homo sapiens pro-platelet basic protein (PPBP), mRNA - NM 002704 
1 acttatctgc agacttgtag gcagcaactc accctcactc agaggtcttc tggttctgga
61 aacaactcta gctcagcctt ctccaccatg agcctcagac ttgataccac cccttcctgt
121 aacagtgcga gaccacttca tgccttgcag gtgctgctgc ttctgtcatt gctgctgact
181 gctctggctt cctccaccaa aggacaaact aagagaaact tggcgaaagg caaagaggaa
241 agtctagaca gtgacttgta tgctgaactc cgctgcatgt gtataaagac aacctctgga
301 attcatccca aaaacatcca aagtttggaa gtgatcggga aaggaaccca ttgcaaccaa
361 gtcgaagtga tagccacact gaaggatggg aggaaaatct gcctggaccc agatgctccc
421 agaatcaaga aaattgtaca gaaaaaattg gcaggtgatg aatctgctga ttaatttgtt
481 ctgtttctgc caaacttctt taactcccag gaagggtaga attttgaaac cttgattttc
541 tagagttctc atttattcag gatacctatt cttactgtat taaaatttgg atatgtgttt
601 cattctgtct caaaaatcac attttattct gagaaggttg gttaaaagat ggcagaaaga
661 agatgaaaat aaataagcct ggtttcaacc ctctaattct tgcctaaaca ttggactgta
721 ctttgcattt ttttctttaa aaatttctat tctaacacaa cttggttgat ttttcctggt
781 ctactttatg gttattagac atactcatgg gtattattag atttcataat ggtcaatgat
841 aataggaatt acatggagcc caacagagaa tatttgctca atacattttt gttaatatat
901 ttaggaactt aatggagtct ctcagtgtct tagtcctagg atgtcttatt taaaatactc
961 cctgaaagtt tattctgatg tttattttag ccatcaaaca ctaaaataat aaattggtga
1021 atatgaatct tataaactgt ggttagctgg tttaaagtga atatatttgc cactagtaga
1081 acaaaaatag atgatgaaaa tgaattaaca tatctacata gttataattc tatcattaga
1141 atgagcctta taaataagta caatatagga cttcaacctt actagactcc taattctaaa
1201 ttctactttt ttcatcaaca gaactttcat tcatttttta aaccctaaaa cttataccca
1261 cactattctt acaaaaatat tcacatgaaa taaaaatttg ctattga
**TREML1 (SEQ ID NO: 60)** - Homo sapiens triggering receptor expressed on myeloid cells like 1 (TREML1), transcript variant 1, mRNA - NM 178174.3 
1 tcaggcgaat gctgcatcag tgcccaggca agcccaggag ttgacatttc tctgcccagc
61 catgggcctc accctgctct tgctgctgct cctgggacta gaaggtcagg gcatagttgg
121 cagcctccct gaggtgctgc aggcacccgt gggaagctcc attctggtgc agtgccacta
181 caggctccag gatgtcaaag ctcagaaggt gtggtgccgg ttcttgccgg aggggtgcca
241 gcccctggtg tcctcagctg tggatcgcag agctccagcg ggcaggcgta cgtttctcac
301 agacctgggt gggggcctgc tgcaggtgga aatggttacc ctgcaggaag aggatgctgg
361 cgagtatggc tgcatggtgg atggggccag ggggccccag attttgcaca gagtctctct
421 gaacatactg cccccagagg aagaagaaga gacccataag attggcagtc tggctgagaa
481 cgcattctca gaccctgcag gcagtgccaa ccctttggaa cccagccagg atgagaagag
541 catccccttg atctggggtg ctgtgctcct ggtaggtctg ctggtggcag cggtggtgct
601 gtttgctgtg atggccaaga ggaaacaagg gaacaggctt ggtgtctgtg gccgattcct
661 gagcagcaga gtttcaggca tgaatccctc ctcagtggtc caccacgtca gtgactctgg
721 accggctgct gaattgcctt tggatgtacc acacattagg cttgactcac caccttcatt
781 tgacaatacc acctacacca gcctacctct tgattcccca tcaggaaaac cttcactccc
841 agctccatcc tcattgcccc ctctacctcc taaggtcctg gtctgctcca agcctgtgac
901 atatgccaca gtaatcttcc cgggagggaa caagggtgga gggacctcgt gtgggccagc
961 ccagaatcca cctaacaatc agactccatc cagctaagct gctcatcaca ctttaaactc
1021 atgaggacca tccctagggg ttctgtgcat ccatccagcc agctcatgcc ctaggatcct
1081 taggatatct gagcaaccag ggactttaag atctaatcca atgtcctaac tttactaggg
1141 aaagtgacgc tcagacatga ctgagatgtc ttggggaaga cctccctgca cccaactccc
1201 ccactggttc ttctaccatt acacactggg ctaaataaac cctaataatg atgtgcaaac
1261 tcttaatggc tgaatgggaa aggaaactgc ccaagtttga ctaattgctt ggcctgtgaa
1321 tggaaaagac tctggtctaa aaaaaaaaaa aaaaaa
**PF4 (SEQ ID NO: 61)** - Homo sapiens platelet factor 4 (PF4), mRNA - NM 002619 
1 atcttagttt ccgcaccgca gttcctcggt gtccacttca ggcttccgga ctggaaggac
61 agccgggaat aaaacgtgcc ggcgaggctc aggagtcatt ggccacagag acccagcccg
121 agtttcccat cgcactgagc actgagatcc tgctggaagc tctgccgcag catgagctcc
181 gcagccgggt tctgcgcctc acgccccggg ctgctgttcc tggggttgct gctcctgcca
241 cttgtggtcg ccttcgccag cgctgaagct gaagaagatg gggacctgca gtgcctgtgt
301 gtgaagacca cctcccaggt ccgtcccagg cacatcacca gcctggaggt gatcaaggcc
361 ggaccccact gccccactgc ccaactgata gccacgctga agaatggaag gaaaatttgc
421 ttggacctgc aagccccgct gtacaagaaa ataattaaga aacttttgga gagttagcta
481 ctagctgcct acgtgtgtgc atttgctata tagcatactt ctttttttca gtttcaatct
541 aactgtgaaa gaacttctga tatttgtgtt atccttatga ttttaaataa acaaaataaa
601 tcaagttgta gtatagtcaa aatacttctt aataatagtg caaaaattgt gttgacacat
661 aacaatttca tggaagaaaa aaattccggt attttaagca aaaagtattt tgaaggaagg
721 tgtgaatact ggttatgctt ggtgttacat gttggctgat acatattcat gcatttacat
781 gattgcagta ctttatagct acatatttac cttgaccatt attattacct ttgccaataa
841 atatcagtaa cacagaaaaa aaaaaaaaaa aaaaaaaa
**CLEC1B (SEQ ID NO: 62)** C-type lectin domain family 1 member B, transcript variant 1 NM 016509.3 
1 gtccatgtat ctctgagcag ctatgaagaa gcttcctgga aaacaataag caaaggaaaa
61 caaatgtgtc ccatctcaca tggttctacc ctactaaaga caggaagatc ataaactgac
121 agatactgaa attgtaaagt tggaaactac attttgcaaa gtcattgaac tctgagctca
 
181 gttgcagtac tcgggaagcc atgcaggatg aagatggata catcacctta aatattaaaa
241 ctcggaaacc agctctcatc tccgttggct ctgcatcctc ctcctggtgg cgtgtgatgg
301 ctttgattct gctgatcctg tgcgtgggga tggttgtcgg gctggtggct ctggggattt
361 ggtctgtcat gcagcgcaat tacctacaag gtgagaatga aaatcgcaca ggaactctgc
421 aacaattagc aaagcgcttc tgtcaatatg tggtaaaaca atcagaacta aagggcactt
481 tcaaaggtca taaatgcagc ccctgtgaca caaactggag atattatgga gatagctgct
541 atgggttctt caggcacaac ttaacatggg aagagagtaa gcagtactgc actgacatga
601 atgctactct cctgaagatt gacaaccgga acattgtgga gtacatcaaa gccaggactc
661 atttaattcg ttgggtcgga ttatctcgcc agaagtcgaa tgaggtctgg aagtgggagg
721 atggctcggt tatctcagaa aatatgtttg agtttttgga agatggaaaa ggaaatatga
781 attgtgctta ttttcataat gggaaaatgc accctacctt ctgtgagaac aaacattatt
841 taatgtgtga gaggaaggct ggcatgacca aggtggacca actaccttaa tgcaaagagg
901 tggacaggat aacacagata agggctttat tgtacaataa aagatatgta tgaatgcatc
961 agtagctgaa aaaaaaaaaa aa
**LCN15 (SEQ ID NO: 63)** - Homo sapiens lipocalin 15 (LCN15), mRNA - NM 203347 
1 caggggctgg agggcagggg aggggatgat gtcattcctg ctcggcgcaa tcctgaccct
61 gctctgggcg cccacggctc aggctgaggt tctgctgcag cctgacttca atgctgaaaa
121 gttctcaggc ctctggtacg tggtctccat ggcatctgac tgcagggtct tcctgggcaa
181 gaaggaccac ctgtccatgt ccaccagggc catcaggccc acagaggagg gcggcctcca
241 cgtccacatg gagttcccgg gggcggacgg ctgtaaccag gtggatgccg agtacctgaa
301 ggtgggctcc gagggacact tcagagtccc ggccttgggc tacctggacg tgcgcatcgt
361 ggacacagac tacagctcct tcgccgtcct ttacatctac aaggagctgg agggggccct
421 cagcaccatg gtgcagctct acagccggac ccaggatgtg agtccccagg ctctgaagtc
481 cttccaggac ttctacccga ccctggggct ccccaaggac atgatggtca tgctgcccca
541 gtcagatgca tgcaaccctg agagcaagga ggcgccctga cacctccgga gccccacccc
601 cgcccttccc aggtggagcc aaagcagcag gcgcctttgc ccctggagtc aagacccaca
661 gccctcgggg accacctgga gtctctccat cctccacccc ccgcctgtgg gatgccttgt
721 gggacgtctc tttctattca ataaacagat gctgcagcct ca
**C1QC (SEQ ID NO: 64)** - Homo sapiens complement component 1, q subcomponent, C chain (C1QC), transcript variant 2, mRNA - NM 172369 
1 gaccactcag acaccgtgtc ctcttgcctg ggagagggga agcagatctg aggacatctc
61 tgtgccaggc cagaaaccgc ccacctgcag ttccttctcc gggatggacg tggggcccag
121 ctccctgccc caccttgggc tgaagctgct gctgctcctg ctgctgctgc ccctcagggg
181 ccaagccaac acaggctgct acgggatccc agggatgccc ggcctgcccg gggcaccagg
241 gaaggatggg tacgacggac tgccggggcc caagggggag ccaggaatcc cagccattcc
301 cgggatccga ggacccaaag ggcagaaggg agaacccggc ttacccggcc atcctgggaa
361 aaatggcccc atgggacccc ctgggatgcc aggggtgccc ggccccatgg gcatccctgg
421 agagccaggt gaggagggca gatacaagca gaaattccag tcagtgttca cggtcactcg
481 gcagacccac cagccccctg cacccaacag cctgatcaga ttcaacgcgg tcctcaccaa
541 cccgcaggga gattatgaca cgagcactgg caagttcacc tgcaaagtcc ccggcctcta
601 ctactttgtc taccacgcgt cgcatacagc caacctgtgc gtgctgctgt accgcagcgg
661 cgtcaaagtg gtcaccttct gtggccacac gtccaaaacc aatcaggtca actcgggcgg
721 tgtgctgctg aggttgcagg tgggcgagga ggtgtggctg gctgtcaatg actactacga
781 catggtgggc atccagggct ctgacagcgt cttctccggc ttcctgctct tccccgacta
841 gggcgggcag atgcgctcga gccccacggg ccttccacct ccctcagctt cctgcatgga
901 cccaccttac tggccagtct gcatccttgc ctagaccatt ctccccacca gatggacttc
961 tcctccaggg agcccaccct gacccacccc cactgcaccc cctccccatg ggttctctcc
1021 ttcctctgaa cttctttagg agtcactgct tgtgtggttc ctgggacact taaccaatgc
1081 cttctggtac tgccattctt tttttttttt ttttcaagta ttggaagggg tggggagata
1141 tataaataaa tcatgaaatc aatacataaa aaaaaaaaaa aa
**C1QB (SEQ ID NO: 65)** - Homo sapiens complement component 1, q subcomponent, B chain (C1QB), mRNA - NM 000491 
1 gcccttcccg cctctgggga agggaacttc cgcttcggac cgagggcagt aggctctcgg
61 ctcctggtcc cactgctgct cagcccagtg gcctcacagg acaccagctt cccaggaggc
121 gtctgacaca gtatgatgat gaagatccca tggggcagca tcccagtact gatgttgctc
181 ctgctcctgg gcctaatcga tatctcccag gcccagctca gctgcaccgg gcccccagcc
241 atccctggca tcccgggtat ccctgggaca cctggccccg atggccaacc tgggacccca
301 gggataaaag gagagaaagg gcttccaggg ctggctggag accatggtga gttcggagag
361 aagggagacc cagggattcc tgggaatcca ggaaaagtcg gccccaaggg ccccatgggc
421 cctaaaggtg gcccaggggc ccctggagcc ccaggcccca aaggtgaatc gggagactac
481 aaggccaccc agaaaatcgc cttctctgcc acaagaacca tcaacgtccc cctgcgccgg
541 gaccagacca tccgcttcga ccacgtgatc accaacatga acaacaatta tgagccccgc
601 agtggcaagt tcacctgcaa ggtgcccggt ctctactact tcacctacca cgccagctct
661 cgagggaacc tgtgcgtgaa cctcatgcgt ggccgggagc gtgcacagaa ggtggtcacc
721 ttctgtgact atgcctacaa caccttccag gtcaccaccg gtggcatggt cctcaagctg
781 gagcaggggg agaacgtctt cctgcaggcc accgacaaga actcactact gggcatggag
841 ggtgccaaca gcatcttttc cgggttcctg ctctttccag atatggaggc ctgacctgtg
901 ggctgcttca catccacccc ggctccccct gccagcaacg ctcactctac ccccaacacc
961 accccttgcc caaccaatgc acacagtagg gcttggtgaa tgctgctgag tgaatgagta
1021 aataaactct tcaaggccaa ggga
**PCOLCE2 (SEQ ID NO: 66)** - Homo sapiens procollagen C-endopeptidase enhancer 2 (PCOLCE2),mRNA - NM 013363 
1 gaacaaacgg gatattcagc agtggcctgt ggctgccaga gcagctcctc aggggaaact
61 aagcgtcgag tcagacggca ccataatcgc ctttaaaagt gcctccgccc tgccggccgc
121 gtatcccccg gctacctggg ccgccccgcg gcggtgcgcg cgtgagaggg agcgcgcggg
181 cagccgagcg ccggtgtgag ccagcgctgc tgccagtgtg agcggcggtg tgagcgcggt
241 gggtgcggag gggcgtgtgt gccggcgcgc gcgccgtggg gtgcaaaccc cgagcgtcta
301 cgctgccatg aggggcgcga acgcctgggc gccactctgc ctgctgctgg ctgccgccac
361 ccagctctcg cggcagcagt ccccagagag acctgttttc acatgtggtg gcattcttac
421 tggagagtct ggatttattg gcagtgaagg ttttcctgga gtgtaccctc caaatagcaa
481 atgtacttgg aaaatcacag ttcccgaagg aaaagtagtc gttctcaatt tccgattcat
541 agacctcgag agtgacaacc tgtgccgcta tgactttgtg gatgtgtaca atggccatgc
601 caatggccag cgcattggcc gcttctgtgg cactttccgg cctggagccc ttgtgtccag
661 tggcaacaag atgatggtgc agatgatttc tgatgccaac acagctggca atggcttcat
721 ggccatgttc tccgctgctg aaccaaacga aagaggggat cagtattgtg gaggactcct
781 tgacagacct tccggctctt ttaaaacccc caactggcca gaccgggatt accctgcagg
841 agtcacttgt gtgtggcaca ttgtagcccc aaagaatcag cttatagaat taaagtttga
901 gaagtttgat gtggagcgag ataactactg ccgatatgat tatgtggctg tgtttaatgg
961 cggggaagtc aacgatgcta gaagaattgg aaagtattgt ggtgatagtc cacctgcgcc
1021 aattgtgtct gagagaaatg aacttcttat tcagttttta tcagacttaa gtttaactgc
1081 agatgggttt attggtcact acatattcag gccaaaaaaa ctgcctacaa ctacagaaca
1141 gcctgtcacc accacattcc ctgtaaccac gggtttaaaa cccaccgtgg ccttgtgtca
1201 acaaaagtgt agacggacgg ggactctgga gggcaattat tgttcaagtg actttgtatt
1261 agccggcact gttatcacaa ccatcactcg cgatgggagt ttgcacgcca cagtctcgat
1321 catcaacatc tacaaagagg gaaatttggc gattcagcag gcgggcaaga acatgagtgc
1381 caggctgact gtcgtctgca agcagtgccc tctcctcaga agaggtctaa attacattat
1441 tatgggccaa gtaggtgaag atgggcgagg caaaatcatg ccaaacagct ttatcatgat
1501 gttcaagacc aagaatcaga agctcctgga tgccttaaaa aataagcaat gttaacagtg
1561 aactgtgtcc atttaagctg tattctgcca ttgcctttga aagatctatg ttctctcagt
1621 agaaaaaaaa atacttataa aattacatat tctgaaagag gattccgaaa gatgggactg
1681 gttgactctt cacatgatgg aggtatgagg cctccgagat agctgaggga agttctttgc
1741 ctgctgtcag aggagcagct atctgattgg aaacctgccg acttagtgcg gtgataggaa
1801 gctaaaagtg tcaagcgttg acagcttgga agcgtttatt tatacatctc tgtaaaagga
 
1861 tattttagaa ttgagttgtg tgaagatgtc aaaaaaagat tttagaagtg caatatttat
1921 agtgttattt gtttcacctt caagcctttg ccctgaggtg ttacaatctt gtcttgcgtt
1981 ttctaaatca atgcttaata aaatattttt aaaggattac agaacaacca aaaaaaaaaa
2041 aaaaaaa
**C1QA (SEQ ID NO: 67)** - Homo sapiens component 1, q subcomponent, A chain (C1QA), mRNA - NM 015991 
1 gccactcctg ctgggcagcc cacagggtcc ctgggcggag ggcaggagca tccagttgga
61 gttgacaaca ggaggcagag gcatcatgga gggtccccgg ggatggctgg tgctctgtgt
121 gctggccata tcgctggcct ctatggtgac cgaggacttg tgccgagcac cagacgggaa
181 gaaaggggag gcaggaagac ctggcagacg ggggcggcca ggcctcaagg gggagcaagg
241 ggagccgggg gcccctggca tccggacagg catccaaggc cttaaaggag accaggggga
301 acctgggccc tctggaaacc ccggcaaggt gggctaccca gggcccagcg gccccctcgg
361 agcccgtggc atcccgggaa ttaaaggcac caagggcagc ccaggaaaca tcaaggacca
421 gccgaggcca gccttctccg ccattcggcg gaacccccca atggggggca acgtggtcat
481 cttcgacacg gtcatcacca accaggaaga accgtaccag aaccactccg gccgattcgt
541 ctgcactgta cccggctact actacttcac cttccaggtg ctgtcccagt gggaaatctg
601 cctgtccatc gtctcctcct caaggggcca ggtccgacgc tccctgggct tctgtgacac
661 caccaacaag gggctcttcc aggtggtgtc agggggcatg gtgcttcagc tgcagcaggg
721 tgaccaggtc tgggttgaaa aagaccccaa aaagggtcac atttaccagg gctctgaggc
781 cgacagcgtc ttcagcggct tcctcatctt cccatctgcc tgagccaggg aaggaccccc
841 tcccccaccc acctctctgg cttccatgct ccgcctgtaa aatgggggcg ctattgcttc
901 agctgctgaa gggagggggc tggctctgag agccccagga ctggctgccc cgtgacacat
961 gctctaagaa gctcgtttct tagacctctt cctggaataa acatctgtgt ctgtgtctgc
1021 tgaacatgag cttcagttgc tactcggagc attgagaggg aggcctaaga ataataacaa
1081 tccagtgctt aagagtca
**TMEM37 (SEQ ID NO: 68)** - Homo sapiens transmembrane protein 37 (TMEM37), mRNA - NM 183240 
1 cgatcgaggc tgcagcgcgg ccgccgggcg cagcatgact gccgtcggcg tgcaggccca
61 gaggcctttg ggccaaaggc agccccgccg gtccttcttt gaatccttca tccggaccct
121 catcatcacg tgtgtggccc tggctgtggt cctgtcctcg gtctccattt gtgatgggca
181 ctggctcctg gctgaggacc gcctcttcgg gctctggcac ttctgcacca ccaccaacca
241 gacgatctgc ttcagagacc tgggccaggc ccatgtgccc gggctggccg tgggcatggg
301 cctggtacgc agcgtgggcg ccttggccgt ggtggccgcc atttttggcc tggagttcct
361 catggtgtcc cagttgtgcg aggacaaaca ctcacagtgc aagtgggtca tgggttccat
421 cctcctcctg gtgtctttcg tcctctcctc cggcgggctc ctgggttttg tgatcctcct
481 caggaaccaa gtcacactca tcggcttcac cctaatgttt tggtgcgaat tcactgcctc
541 cttcctcctc ttcctgaacg ccatcagcgg ccttcacatc aacagcatca cccatccctg
601 ggaatgaccg tggaaatttt aggccccctc cagggacatc agattccaca agaaaatatg
661 gtcaaaatgg gacttttcca gcatgtggcc tctggtgggg ctgggttgga caagggcctt
721 gaaacggctg cctgtttgcc gataacttgt gggtggtcag ccagaaatgg cccgggggcc
781 tctgcacctg gtctgcaggg ccagaggcca ggagggtgcc tcagtgccac caactgcaca
841 ggcttagcca gatgttgatt ttagaggaag aaaaaaacat tttaaaactc cttcttgaat
901 tttcttccct ggactggaat acagttggaa gcacaggggt aactggtacc tgagctagct
961 gcacagccaa ggatagttca tgcctgtttc attgacacgt gctgggatag gggctgcaga
1021 atccctgggg ctcccagggt tgttaagaat ggatcattct tccagctaag ggtccaatca
1081 gtgcctagga ctttcttcca ccagctcaaa gggccttcgt atgtatgtcc ctggcttcag
1141 ctttggtcat gccaaagagg cagagttcag gattccctca gaatgccctg cacacagtag
1201 gtttccaaac catttgactc ggtttgcctc cctgcccgtt gtttaaacct tacaaaccct
1261 ggataacccc atcttctagc agctggctgt gcctctggga gctctgccta tcagaaccct
1321 accttaaggt gggtttcctt ccgagaagag ttcttgagca agctctccca ggagggccca
1381 cctgactgct aatacacagc cctccccaag gcccgtgtgt gcatgtgtct gtcttttgtg
1441 agggttagac agcctcaggg caccattttt aatcccagaa cacatttcaa agagcacgta
1501 tctagacctg ctggactctg cagggggtga gggggaacag cgagagcttg ggtaatgatt
 
1561 aacacccatg ctggggatgc atggaggtga agggggccag gaaccagtgg agatttccat
1621 ccttgccagc acgtctgtac ttctgttcat taaagtgctc cctttctagt cctttttctg
1681 cccagaa
**TNF (SEQ ID NO: 69)** - Homo sapiens tumor necrosis factor (TNF), mRNA - NM 000594 
1 cagacgctcc ctcagcaagg acagcagagg accagctaag agggagagaa gcaactacag
61 accccccctg aaaacaaccc tcagacgcca catcccctga caagctgcca ggcaggttct
121 cttcctctca catactgacc cacggctcca ccctctctcc cctggaaagg acaccatgag
181 cactgaaagc atgatccggg acgtggagct ggccgaggag gcgctcccca agaagacagg
241 ggggccccag ggctccaggc ggtgcttgtt cctcagcctc ttctccttcc tgatcgtggc
301 aggcgccacc acgctcttct gcctgctgca ctttggagtg atcggccccc agagggaaga
361 gttccccagg gacctctctc taatcagccc tctggcccag gcagtcagat catcttctcg
421 aaccccgagt gacaagcctg tagcccatgt tgtagcaaac cctcaagctg aggggcagct
481 ccagtggctg aaccgccggg ccaatgccct cctggccaat ggcgtggagc tgagagataa
541 ccagctggtg gtgccatcag agggcctgta cctcatctac tcccaggtcc tcttcaaggg
601 ccaaggctgc ccctccaccc atgtgctcct cacccacacc atcagccgca tcgccgtctc
661 ctaccagacc aaggtcaacc tcctctctgc catcaagagc ccctgccaga gggagacccc
721 agagggggct gaggccaagc cctggtatga gcccatctat ctgggagggg tcttccagct
781 ggagaagggt gaccgactca gcgctgagat caatcggccc gactatctcg actttgccga
841 gtctgggcag gtctactttg ggatcattgc cctgtgagga ggacgaacat ccaaccttcc
901 caaacgcctc ccctgcccca atccctttat taccccctcc ttcagacacc ctcaacctct
961 tctggctcaa aaagagaatt gggggcttag ggtcggaacc caagcttaga actttaagca
1021 acaagaccac cacttcgaaa cctgggattc aggaatgtgt ggcctgcaca gtgaagtgct
1081 ggcaaccact aagaattcaa actggggcct ccagaactca ctggggccta cagctttgat
1141 ccctgacatc tggaatctgg agaccaggga gcctttggtt ctggccagaa tgctgcagga
1201 cttgagaaga cctcacctag aaattgacac aagtggacct taggccttcc tctctccaga
1261 tgtttccaga cttccttgag acacggagcc cagccctccc catggagcca gctccctcta
1321 tttatgtttg cacttgtgat tatttattat ttatttatta tttatttatt tacagatgaa
1381 tgtatttatt tgggagaccg gggtatcctg ggggacccaa tgtaggagct gccttggctc
1441 agacatgttt tccgtgaaaa cggagctgaa caataggctg ttcccatgta gccccctggc
1501 ctctgtgcct tcttttgatt atgtttttta aaatatttat ctgattaagt tgtctaaaca
1561 atgctgattt ggtgaccaac tgtcactcat tgctgagcct ctgctcccca ggggagttgt
1621 gtctgtaatc gccctactat tcagtggcga gaaataaagt ttgcttagaa aagaaaaaaa
1681 aaaaaa
**SLC39A8 (SEQ ID NO: 70)** - Homo sapiens solute carrier family 39 member 8 (SLC39A8), transcript variant 1, mRNA - NM_022154 
1 ggcggagaga ataaagcaga aagacaggaa gaggaggtgg agttctacag ttagtgtggt
61 tttagttttt cctaagaagt ggcgtggttt ggggctttat atccgggagg agcatatgta
121 cgcaaatcct ggggcgtttg caaacccgga tccggggcgt ctggccccat gcccggccgg
181 gcgtttgagg gctactgcca cgcagcgttt ctggagcctg ccggctggtg ccctggtggc
241 ctttatctct gtcccccttt gtcctcttta tctcaggctc tccaggaggc cgggggcccc
301 actccgccta tcgctcccct cggctacgct gccacttcaa tgccccgcag gtcgcgagct
361 gctgttcttt cgaaggcgtc ggagaaccag gggcgtcccg cgccacctct gactcggagc
421 agcgccgagc actgacgctc ccgcccttgg gcaaggacgc cagtgcgccc gcgcgcgtcc
481 ctctgcgcgg cagcccgtcg cgggccctca aggggaagcc caggccagga tggccccggg
541 tcgcgcggtg gccgggctcc tgttgctggc ggccgccggc ctcggaggag tggcggaggg
601 gccagggcta gccttcagcg aggatgtgct gagcgtgttc ggcgcgaatc tgagcctgtc
661 ggcggcgcag ctccagcact tgctggagca gatgggagcc gcctcccgcg tgggcgtccc
721 ggagcctggc cagctgcact tcaaccagtg tttaactgct gaagagatct tttcccttca
781 tggcttttca aatgctaccc aaataaccag ctccaaattc tctgtcatct gtccagcagt
841 cttacagcaa ttgaactttc acccatgtga ggatcggccc aagcacaaaa caagaccaag
901 tcattcagaa gtttggggat atggattcct gtcagtgacg attattaatc tggcatctct
961 cctcggattg attttgactc cactgataaa gaaatcttat ttcccaaaga ttttgacctt
 
1021 ttttgtgggg ctggctattg ggactctttt ttcaaatgca attttccaac ttattccaga
1081 ggcatttgga tttgatccca aagtcgacag ttatgttgag aaggcagttg ctgtgtttgg
1141 tggattttac ctacttttct tttttgaaag aatgctaaag atgttattaa agacatatgg
1201 tcagaatggt catacccact ttggaaatga taactttggt cctcaagaaa aaactcatca
1261 acctaaagca ttacctgcca tcaatggtgt gacatgctat gcaaatcctg ctgtcacaga
1321 agctaatgga catatccatt ttgataatgt cagtgtggta tctctacagg atggaaaaaa
1381 agagccaagt tcatgtacct gtttgaaggg gcccaaactg tcagaaatag ggacgattgc
1441 ctggatgata acgctctgcg atgccctcca caatttcatc gatggcctgg cgattggggc
1501 ttcctgcacc ttgtctctcc ttcagggact cagtacttcc atagcaatcc tatgtgagga
1561 gtttccccac gagttaggag actttgtgat cctactcaat gcaggatgaa gcactcgaca
1621 agccttgcta ttcaacttcc tttctgcatg ttcctgctat gttgggctag cttttggcat
1681 tttggtgggc aacaatttcg ctccaaatat tatatttgca cttgctggag gcatgttcct
1741 ctatatttct ctggcagata tgtttccaga gatgaatgat atgctgagag aaaaggtaac
1801 tggaagaaaa accgatttca ccttcttcat gattcagaat gctggaatgt taactggatt
1861 cacagccatt ctactcatta ccttgtatgc aggagaaatc gaattggagt aatagaaaat
1921 ggaagatggt gttgttaata aaggcattta atagataaaa acatctccaa aaaggatttt
1981 gaagctgatc ctatttagtt aaaaagataa ttttgctttc aactgtaggt ccagaaaact
2041 aattattggc atcagtctgt gaaatagtcc attatttgtt gttaaaaatg cttcaaaagg
2101 ttttcagtgt cagtctgaga tgcctggtat ataggagcct ttgggaaata cctatttttc
2161 agtattccat gcatattaga tatcaccatg aagcaagaga catgcattct ataatcatgt
2221 agacactcag actcagggga aaatacaagt tatatcctga aagcctttaa aactctatgg
2281 taggatcaaa gattcaaatg gtttcagaga ggttttattt caattaattt gttctagtgc
2341 tttcaagagc aagtacatca aaatgtagaa ggtaaaatgt atgcaacact aatataaatt
2401 attccaagtc tttaaggagc caaagaaaaa aaagatttct cacagctttt tgttctgttt
2461 tgtatttcaa ttaggaactt gcagtattat tttgaaaacc attctaaaat aataggagtt
2521 aggaaataaa taaagttttg ctagccctgc taagttcagg cttagaggct tatcgctaag
2581 tataaacttc accagattcc acgaaaagct ggatagcttt ttttctgact tatgttgtgg
2641 ttgcacccct cacaaatggc agaacagtat gtaaagctgg taacacctcg gtttcagtgc
2701 accatgtgtt tgctttgtga aggtgaagaa tatgttggtt tagagaaaga aattggatgt
2761 aattttatgc aatttacttt taaagacaaa cataactatt tagcagagaa tattttaata
2821 aatgcaaaac aacagctgga ctgctgtaca tcaaggacag attaactgga aaacatatgt
2881 tccttatgtg tgatcgagag ccattcagaa aagacttcct ttgtgttcag cctatacttt
2941 tccatatggt ataccttgaa aaaaattagc acaccatggt tatttttcta ccttttataa
3001 aagacagagc ctgtttactc atttagaaga tagagaaaat tggtctaaaa ttgaacatcc
3061 tagattcaca ctcccaagtc acttaaggtg atttgatggt gaggaaaatg attgacaaag
3121 cccaacaatg atctcaggaa ttacattttc caacagacca aaaaatgttt tcatgtagca
3181 gcaatgcaga tttggtgaat atttaatata tattttagta tgtatttcac tttatgactg
3241 acaattaaaa aatattgttt ggccaaatag taaacaccct tttgaaacca tgaaaaaaaa
3301 aaaaaaaaa
**SLC39A8 (SEQ ID NO: 71)** - Homo sapiens solute carrier family 39 member 8 (SLC39A8), transcript variant 3, mRNA - NM_001135147 
1 gactccggaa gagccctttt ctgcagctcc ttggggactg cacgtctcac ttctaagttt
61 gcggcggaga gaataaagca gaaagacagg aagaggaggt ggagttctac agctctccag
121 gaggccgggg ggcccactcc gcctatcgct cccctcggct acgctgccac ttcaatgccc
181 cgcaggtcgc gagctgctgt tctttcgaag gcgtcggaga accagggcgg tcccgcgcca
241 cctctgactc ggagcagcgc cgagcactga cgctcccgcc cttgggcaag gacgccagtg
301 cgcccgcgcg cgtccctctg cgcggcagcc cgtcgcgggc cctcaagggg aagcccaggc
361 caggatggcc ccgggtcgcg cggtggccgg gctcctgttg ctggcggccg ccggcctcgg
421 aggagtggcg gaggggccag ggctagcctt cagcgaggat gtgctgagcg tgttcggcgc
481 gaatctgagc ctgtcggcgg cgcagctcca gcacttgctg gagcagatgg gagccgcctc
541 ccgcgtgggc gtcccggagc ctggccagct gcacttcaac cagtgtttaa ctgctgaaga
601 gatcttttcc cttcatggct tttcaaatgc tacccaaata accagctcca aattctctgt
661 catctgtcca gcagtcttac agcaattgaa ctttcaccca tgtgaggatc ggcccaagca
721 caaaacaaga ccaagtcatt cagaagtttg gggatatgga ttcctgtcag tgacgattat
 
781 taatctggca tctctcctcg gattgatttt gactccactg ataaagaaat cttatttccc
841 aaagattttg accttttttg tggggctggc tattgggact cttttttcaa atgcaatttt
901 ccaacttatt ccagaggcat ttggatttga tcccaaagtc gacagttatg ttgagaaggc
961 agttgctgtg tttggtggat tttacctact tttctttttt gaaagaatgc taaagatgtt
1021 attaaagaca tatggtcaga atggtcatac ccactttgga aatgataact ttggtcctca
1081 agaaaaaact catcaaccta aagcattacc tgccatcaat ggtgtgacat gctatgcaaa
1141 tcctgctgtc acagaagcta atggacatat ccattttgat aatgtcagtg tggtatctct
1201 acaggatgga aaaaaagagc caagttcatg tacctgtttg aaggggccca aactgtcaga
1261 aatagggacg attgcctgga tgataacgct ctgcgatgcc ctccacaatt tcatcgatgg
1321 cctggcgatt ggggcttcct gcaccttgtc tctccttcag ggactcagta cttccatagc
1381 aatcctatgt gaggagtttc cccacgagtt aggagacttt gtgatcctac tcaatgcagg
1441 gatgagcact cgacaagcct tgctattcaa cttcctttct gcatgttcct gctatgttgg
1501 gctagctttt ggcattttgg tgggcaacaa tttcgctcca aatattatat ttgcacttgc
1561 tggaggcatg ttcctctata tttctctggc agatatgttt ccagagatga atgatatgct
1621 gagagaaaag ataatcaaat gggctactga tgacatcaaa tctcaacttc atttactttg
1681 gatatatacg gcaaggtagt aatttcatta tctggagtat ttcacctcca catctctgca
1741 ataccagcac cttcaacagt gattggttag aggttggcat atgacatagt tatgaccatt
1801 gagacgtgat gctgtgtgtg ctgtgaagtt tctcaaaagg agtaaggaag atacagcctc
1861 ttctttctag ggactttgtt tgtaggtgtg acacctggag cagccatttt gctactgttt
1921 tgtttctaat tgtgttcccc gccgccctcc ccgctcgccc cgaaaaacga catgtttgag
1981 ttctaatccc cagtatttca gaatgtgacc ttatttggaa atagagtcat tgcaaatgta
2041 atttgttaag atgagctcat actggagtag agtgggttcc tgatctagta tgactggtat
2101 tcttattaaa aagagaaatt tgaatactag ctacaccatg taaagatgaa ggcagagatc
2161 gtggtgatgc atctacaatg tcaacaattg ccagcaaatc atcagaatct gggagagagg
2221 tatggaatga attctctctc gcagccctaa gaaggaacca atcctgtgca cacctgaatc
2281 ttggacttcc agcctccaca actgtgagac aatacatttt tgttatttaa gctacccagt
2341 tgtggcactt tattactgca gttctagccg actaatacag ctaccttgag aggaaatagc
2401 atggggatga aggtgatgtg ctgaagaagc acagcagaaa gtggaaagat ggtccatgag
2461 gacatcctta gccactgaat tgacaagcct cttccctgga gtccctcttc cttgggattt
2521 tttttggtgt gtgtaataat cacttttcct aatggtgcaa accattgtga gttgctcttt
2581 gactgctagt aattaaagca aactaaccaa gtcagccctc cccagtgttc ctttttgcaa
2641 tctaaattct gatgtccaac tcctccctgg gccactccct gtccccagat gtctctttgt
2701 atatctaaag aagaaaaaga aggataaatc ctttttctcc caagagatct ccctgctttt
2761 gtgaaaagca caaagaaggg cattatctaa cttttctcat cattagaagt caagcctggc
2821 cgtgactccc gttatgacat tttgatggcc acattttatg agaaaagccc aaacagacag
2881 acctgtctgt aaattctttc atcctttgca ttctttcttt gactgtacct tagagagtgt
2941 ttgattattg caggggagat ggaaaagaag caggttctgg cctatgctct agttcctcac
3001 tttctggagt tatcttttgg gcctggagtc tcttggagtg aggaaaggaa gccattttta
3061 tactttactt atggtagagt taaacttcgt actcttagga ggctacatgc aatctccaag
3121 aggaagagag tgaagaggtt aagaattatg ccatcaaaat gtgctgctct ggcatattga
3181 ctatttgaat tacagatatt ttaaaaacat cagatatgaa gaggtcactc tgatcttaat
3241 tctgtgtctt aacagcagga gatgaaattc ccacatggga gatggcctcc ctgtcacaga
3301 aggaatacca ttctccctat caatgatgag aaattggaac tgagagaatt ctgtatagac
3361 cttgttcaag taattcttct attttaaacc tccccacata atttagctgc cttttcaaaa
3421 ctactgtcct ttgtgcaacc agtatagaag cgattgactc tcactgcatc cttgggtctt
3481 cttttcccta tatgggctct tttcccatat gggctcttat gtcctgtaaa acatgtatta
3541 aataaatttg tatgcttttc tcccgttaac ctgtcttaca tcaatttaat tcttgggccc
3601 aggtaggacc ctaagaggac agaggtcgag ttttgccacc tctgcaagag aaaagagaga
3661 tgcccagacc accaggcctg ggaaatcttt gatttcccag gctttgaagt gggcactgtg
3721 tctgaacctg gccctctgaa acttcctgtt cctggacatc ccagctgcta atgtgagttg
3781 cccttgcact ggctcagggc tgggacatta aaggaatttc ccttttgttg acataacagt
3841 aggatggcta tagctaacaa taatatatta tttagtttca agtagctaga aggaacatat
3901 gaatgttccc aacacaaaga aatgacaact atttgagatg atggatatgc taataacctt
3961 aatctgattc ctatacatta tatgcatcac atatacatac attatatgta taacatcact
4021 atgtacccca taaatatgtg caattattgt caattaaaca aataaattta aacaaacatt
4081 aaaaaaaaaa aaaaaaaa
**MRAS (SEQ ID NO: 72)** - Homo sapiens muscle RAS oncogene homolog (MRAS), transcript variant 1, mRNA - NM 012219 
1 agtagcgcaa tctcggctca ctacaacctc tgcctcccgg gttcacgtga ttctcctgcc
61 tcagcctccc gagtagatga gatcacaggc acgtgccgcc atgccgggtt gacttttgta
121 tttttagtag agacggggtt tcaccatgtt gcctaggctg gtcttgaact cctgacctca
181 ggcgattcac ccgcctcggc ctcccaaagt gttaggatta caggcgtgag ccaccgcgcc
241 cggcttgaat tgtacacttc aaaaggtgga attttatggt gttgaattat atctttattt
301 ttttaacggg gggaaaatga cgccgctgga gaggagttag cggaactgaa acaatgaaat
361 ggtgcgcgag tgtcgcctgt ccccgtcgca tccatcccaa cgaagtttgg gccctggaac
421 ggtgcaccca gaaggcctgc ggggagagac gctggggcat gatctggaag aaagacgtct
481 caggattcga agggaatgca gctaaggtgg cggcggaggt tcgcctagga ctggggaggc
541 gtccctaggc tcagaagttg gcccggccgg agcggagatt taaaggttgg agcgcagagg
601 ctcttaaaga ggccgagtcg aattcccact cggcgtccac cttaaagcca gctccccggc
661 accacggatc tgacccgggt ctgacctacg agaaacatgg caaccagcgc cgtccccagt
721 gacaacctcc ccacatacaa gctggtggtg gtgggggatg gggtgtgggg caaaagtgcc
781 ctcaccatcc agtttttcca gaagatcttt gtgcctgact atgaccccac cattgaagac
841 tcctacctga aacatacgga gattgacaat caatgggcca tcttggacgt tctggacaca
901 gctgggcagg aggaattcag cgccatgcgg gagcaataca tgcgcacggg ggatggcttc
961 ctcatcgtct actccgtcac tgacaaggcc agctttgagc acgtggaccg cttccaccag
1021 cttatcctgc gcgtcaaaga cagggagtca ttcccgatga tcctcgtggc caacaaggtc
1081 gatttgatgc acttgaggaa gatcaccagg gagcaaggaa aagaaatggc gaccaaacac
1141 aatattccgt acatagaaac cagtgccaag gacccacctc tcaatgtcga caaagccttc
1201 catgacctcg ttagagtaat taggcaacag attccggaaa aaagccagaa gaagaagaag
1261 aaaaccaaat ggcggggaga ccgggccaca ggcacccaca aactgcaatg tgtgatcttg
1321 tgacaggcct gaggccctgg gcacagtgac ggtggcctgg ccagccctcg ggacccctcc
1381 ccacctaact gcactgaaac catttctaac cacaaccctt ggcccaagga cttggtacag
1441 gaagggagaa gggcaggtgg gcagggagca gacagggtct ggctttgccc agagggcacg
1501 ggctttccca cctctcaaag agacaaggaa gccacctgta agcagaagca gcatccaagt
1561 gcccctggcc cccccatgtg ttgattcaac ccggttcctc cccctctctc ggtgggtgtg
1621 ttgtttattg taactacata gtgttggttt gatgtggaag tgtttatcca catacaaagt
1681 acaaaacaag ccatgaacaa gcttctttcc cttacccccc atccacaatg tctgagcttg
1741 gatgtctttt atagattttt aaattatttt agtgattatt attttattaa aggggtctgg
1801 gctcactgcc tggtgaagtt tcaagtgttc agcagacctc tctggtaaca tatctggaat
1861 attgttgttg ttttttaacc gagttttccc atcagtgcca aaactcaact caatctgaaa
1921 gtagagtgtc tgagaggaca gaaggtaatg ggaactgtag ctggaggcct caggccatgg
1981 gtcaaacctg ggagggaaag agaccctaca catggcctag aaatgagaga agagagaggt
2041 atttacccag aggattttcc tatggttggg gatgcaaata ttagaaaaca gattgtattt
2101 tgctgagggg agtggctgtc atgagcatgt cagttctaaa aggggttttc attatcctgg
2161 aaatgtataa actaaagtaa gctgattggc tttgcaaaca tgttcatttg tttttcagac
2221 agtatgggtt aagttctctg ccctccccag gggtctgagg aggctctggg tttctcagat
2281 ctgtctcttg ctgcgttttc acatcagctg tgctgcttgg tgcctctctg atacgaatac
2341 actgacacgt caaagtaacc taatgtggac accatccaga aaactccagt tcatgctgga
2401 tcttaaccaa aaatgattca atactgttat cactaaaaca gcaccaagac ctgaagccat
2461 cttcccttgg agtcaactga ctaccacctc tataagccta gtcaatgagc agaccccttc
2521 cagtatttgt aaaagtagta ctaggttgcc tttttggcaa tttttattga cctgttgaat
2581 cttgactata aaatgatctg agaagtaagg aaggctgggc tgatgtgtgg ctctcatata
2641 ccttctgcaa gggggcagtc tccccagctc cctgatgatg ctcacccccg cccccccacc
2701 tcaggtgctg ctggtgtgag ccaaagactg gagtttttcc agctggggtg ggagtggaga
2761 gacaacagga acaacgctgc accaaagaaa aggtcagaat aaaaggcagc acagctggtg
2821 accttatttt ctagatgtta caaatcaggt cactatgcaa actagaatat cctcagcagg
2881 tggcctggcc actctggaga aagaaaccca aggaaagtga gcacccaact ggatgccaag
2941 acacccgggt tctgaaaatg tgctgtgttc ctacctcggc aagatcacca gcactgaggg
3001 gcccagctgg agaatgattc tgctacaaaa ggagacagtt gagacttttg cttgttggaa
3061 atcaaacttc ttatttgtct aaattgcccc tttttctgtt cctaaaagga aggataagag
 
3121 agaacattcc aggtgaggca cttcaaagtt tccttagacc ctatagtgtt aagaggtatt
3181 ttaaacacta aaaggacaaa gctcttccca atccttatgc ttccctaagt ggtatctgca
3241 gcagtttgtt gtgtgcagtt tgatggcagc tgcaaactgg aggtgaggcg gaggaaaggc
3301 aggtaggaag gagtaaggat ggagatgctc agaatcaaga gcatggcgga gtaggagaag
3361 aagccctgca cacagggcag tgtccacagc cagaaaactc ctgctgggca ccaaccacta
3421 cgagcatacc ccatgcccac cgtggagctg caactcctcg acagcactga gtttgatagt
3481 ctcactggaa gcagatcagc tgatgtagaa cagagacctc ggccataaag gtgagaagac
3541 atagggattt caaccacaca gttgggacag aagggacagt gcatctgttc atccatcctg
3601 cacttggccc acgttgaact ccatggtgcc tgagagagac tagttaaggg ttggtcttct
3661 gtatcctctg ctgttgagcc tctggtaagc tttcatctcc catgaactca tttccccata
3721 aatgaaatgg gtaaataatg ccccatttgt agaagtgggc cctcatgact gaggtagctt
3781 ccagataggc cagagtagag tgtagagtgt gccccgtgac atccctccat cttctcctcc
3841 attatcatct agcagggtca gactgggaaa cctggttggc cacgccacac catgaccgag
3901 gagccaactg ggacttctgg ctgtttgaca tcctcatgtt cccgttggtc ttccggagaa
3961 tagtgctacc ctcacatccc ctggagcaca gccttcctga aatgccctca ccccatgcct
4021 ttgccattgt gtgctctcag atttcttcca ctgtttgaca ccctccttag agggctgctc
4081 ttttttttcc agagataatc ctagccatcc tctccactcc cacggctggg gacaatggcc
4141 acttactacc tgtgcacttt gccactcggg acacctggat ggtttctctt aggactttgc
4201 ccacctcctt ctcatggcac ttgctgtgga aaatgcctgg ctggcctcgt ggggcctgtc
4261 tcacttttcc aggagacatg acccactaac gtggcaactt taacccaaag gcccctcaga
4321 catgttacag caaatctgga gccacagaca ggttccctcc attggcagcc cattgtgttt
4381 gaaattccat gtcgggttta cttggaatga aagatacttg aattattgtg cgcctgtgag
4441 cgcccagctt ctgtttcata gtcttaacag gtggccattg tcgtgaaacg agtgatgcct
4501 gaagatctca gtgatgtttg aaccttctgt gtaacttttt attaagtctt tgtatctctc
4561 gactgattaa taaagaagag aaacacgtaa aaaaaaaaaa aaaaaaaaa
**IFIT1 (SEQ ID NO: 73)** - Homo sapiens interferon induced protein with tetratricopeptide repeats 1 (IFIT1), transcript variant 1, mRNA - NM 001543 
1 gcaaggacac acccacagct tacaccattg gctgctgttt agctccctta tataacactg
61 tcttggggtt taaacgtaac tgaaaatcca caagacagaa tagccagatc tcagaggagc
121 ctggctaagc aaaaccctgc agaacggctg cctaatttac agcaaccatg agtacaaatg
181 gtgatgatca tcaggtcaag gatagtctgg agcaattgag atgtcacttt acatgggagt
241 tatccattga tgacgatgaa atgcctgatt tagaaaacag agtcttggat cagattgaat
301 tcctagacac caaatacagt gtgggaatac acaacctact agcctatgtg aaacacctga
361 aaggccagaa tgaggaagcc ctgaagagct taaaagaagc tgaaaactta atgcaggaag
421 aacatgacaa ccaagcaaat gtgaggagtc tggtgacctg gggcaacttt gcctggatgt
481 attaccacat gggcagactg gcagaagccc agacttacct ggacaaggtg gagaacattt
541 gcaagaagct ttcaaatccc ttccgctata gaatggagtg tccagaaata gactgtgagg
601 aaggatgggc cttgctgaag tgtggaggaa aaaattatga acgggccaag gcctgctttg
661 aaaaggtgct tgaagtggac cctgaaaacc ctgaatccag cgctgggtat gcgatctctg
721 cctatcgcct ggatggcttt aaattagcca caaaaaatca caagccattt tctttgcttc
781 ccctaaggca ggctgtccgc ttaaatccag acaatggata tattaaggtt ctccttgccc
841 tgaagcttca ggatgaagga caggaagctg aaggagaaaa gtacattgaa gaagctctag
901 ccaacatgtc ctcacagacc tatgtctttc gatatgcagc caagttttac cgaagaaaag
961 gctctgtgga taaagctctt gagttattaa aaaaggcctt gcaggaaaca cccacttctg
1021 tcttactgca tcaccagata gggctttgct acaaggcaca aatgatccaa atcaaggagg
1081 ctacaaaagg gcagcctaga gggcagaaca gagaaaagct agacaaaatg ataagatcag
1141 ccatatttca ttttgaatct gcagtggaaa aaaagcccac atttgaggtg gctcatctag
1201 acctggcaag aatgtatata gaagcaggca atcacagaaa agctgaagag aattttcaaa
1261 aattgttatg catgaaacca gtggtagaag aaacaatgca agacatacat ttccactatg
1321 gtcggtttca ggaatttcaa aagaaatctg acgtcaatgc aattatccat tatttaaaag
1381 ctataaaaat agaacaggca tcattaacaa gggataaaag tatcaattct ttgaagaaat
1441 tggttttaag gaaacttcgg agaaaggcat tagatctgga aagcttgagc ctccttgggt
1501 tcgtctacaa attggaagga aatatgaatg aagccctgga gtactatgag cgggccctga
1561 gactggctgc tgactttgag aactctgtga gacaaggtcc ttaggcaccc agatatcagc
 
1621 cactttcaca tttcatttca ttttatgcta acatttacta atcatctttt ctgcttactg
1681 ttttcagaaa cattataatt cactgtaatg atgtaattct tgaataataa atctgacaaa
1741 atattagttg tgttcaacaa ttagtgaaac agaatgtgtg tatgcatgta agaaagagaa
1801 atcatttgta tgagtgctat gtagtagaga aaaaatgtta gttaactttg taggaaataa
1861 aacattggac ttacactaaa tgtttaattc attcatttta ttgtgaaata aaaataaaat
1921 ccttagctcc tccaccaact gaacagaccc tcttggccaa ggagacccca gaaaccttaa
1981 aaactaagtt tcccaaccat gacaagatga gagatcattc acacctcatt atattccctc
2041 ccttgctaac tgccattgga ctttttccac tgagttaaac agaaacccat ggaaaacaaa
2101 gaacagaaga ctcactcctt ggctgacttc acctagctca ctccacgtag cgccacagcc
2161 agactcccct cccctcttgc ggtttccaca tgacaactga tcagccttcc ctcctgataa
2221 gtgaccactg cccacagact ggttctggcc agtccatgga ggctgcacac agggtgcctc
2281 tatgtccttt gtttcacctt ttgatataga aaggctaatt ttgctgtatt ttaatgttaa
2341 gtctccacca cagagtgaac acagaatgca tgtgacatac atgtttacat accactattg
2401 tgtgactgcc cctcatgaat attcatagcc ccccataacc tgttaactat gtgtgtctag
2461 ccaatccacc aaccataaaa cttctgtaat accctccctt cctccaagag cctgcttttg
2521 gttgctgtgg taggctctgc ttcccaggct gcaggttgca ggagaggagg ctgcagtggc
2581 tcacgcctgt aatctcagca cttcgatggg acgaggcagg cagatcacct gaacccagga
2641 gttcgagagc agccttggca atggcaaaac caaccgtctc tacaaaaaat gcaaaaactt
2701 agctgggtgt ggtggcatgc acctgtagct tcagttccag ctactcagga ggctgaggtg
2761 agtggactgc tggagccagg gagttcgagg ctgcagtgtc gagatcttgc cactgcactc
2821 cattctggat gatagaacga gaccccatct caaaaaaaaa aaaagttctc tccaattgta
2881 tatagcttgt gattttatgt caacactatc aataaatagc tttcagtgca agaaaccaaa
2941 aatactgtaa taaacaggca catattcttc ccaaacctca tgcagtttac aatctagtga
3001 gagacacaga tagcagtaca gagtcaatta aaggttagtt ttcttcatga agatgtttta
3061 attttaattc aatgtgaaag ggttccaagg agtttatctt gttttatgcc attttatttg
3121 aagcactact tactaagtca tttgctgata ttaatctagt taaatcaaga aatattacat
3181 gaaaatgttg ctaaatcaga gatcatgggt aacaatcacc tttgattatg aataatcata
3241 ttttattgaa aggcaaggca caacaaataa taagaaggaa aaaataaata agcaatgtta
3301 ttgatctttc attctgtata tgttttgggg ggaatatact agtttctttt agtggctgta
3361 acaaattacc acaaacttgg tgacttaaaa tttcacagat ttactctttc ttacagttct
3421 ggaggtcaga agtctgaaat gggtttcaat gagccaaagt caaggtattg atgacgctac
3481 actcctccgg aggctctagg cagatagcct tttccagctt ccagaggctg cctgaattct
3541 ttcatccatc ttaaaaacca acagtgtagt agcctcaaat ctctctctct gcttccttct
3601 tcacatctcc ttctctcctc tgactctttt gcctctttct tctaaggacg caccaggtcc
3661 acctgcataa tccagaataa ttgccccatc cgcaaatcct taatttaata acatctgcaa
3721 agtccctttt gctatgtaaa gtagcatgtt cacaggttct ggagacttgg ccatggatac
3781 gattgcgggg ggggcattat tcttaccaca gagcacccca agaaaatctc caattttggg
3841 gcttccaatc cattttgctt caattattta atatttttac tccttccagt agatactgat
3901 ttcatccatt gcccttaaga aggtaggaca gagattatgg cacatctcac attaaatgct
3961 atattttcgt tggaaataca ttttttgctt caacttttat tttaaattca agggtacatg
4021 tgcaggatgt tcaggtttgt tacacaggta aacgtgtgcc atggcggttt gctgaacaga
4081 tcatcccatc accaacagat catcccattg agaggtgaag ccggctgggc ttctgggttg
4141 ggtggggact tggagaactt ttctgtctag ctaaagtatt gtaaaatgga ccagtcaaca
4201 ctctgtaaaa tggaccaatc agctctctgt aaaatggacc aatcagcagg atgtgggtgg
4261 ggccaagtaa gggaataaaa gcaggccacc cgagctggca gcggcaaccc gctcgggtcc
4321 ccttccatgc tgtggaagtt ttgttctttc gctctttcaa taaatcttgc tgctgctcaa
4381 aaaaaaaaaa aaaaaa
**IFI44 (SEQ ID NO: 74)** - Homo sapiens interferon induced protein 44 (IFI44), mRNA - NM 006417 
1 tctttgaagc ttcaaggctg ctgaataatt tccttctccc attttgtgcc tgcctagcta
61 tccagacaga gcagctaccc tcagctctag ctgatactac agacagtaca acagatcaag
121 aagtatggca gtgacaactc gtttgacatg gttgcacgaa aagatcctgc aaaatcattt
181 tggagggaag cggcttagcc ttctctataa gggtagtgtc catggattcc gtaatggagt
241 tttgcttgac agatgttgta atcaagggcc tactctaaca gtgatttata gtgaagatca
301 tattattgga gcatatgcag aagagagtta ccaggaagga aagtatgctt ccatcatcct
 
361 ttttgcactt caagatacta aaatttcaga atggaaacta ggactatgta caccagaaac
421 actgttttgt tgtgatgtta caaaatataa ctccccaact aatttccaga tagatggaag
481 aaatagaaaa gtgattatgg acttaaagac aatggaaaat cttggacttg ctcaaaattg
541 tactatctct attcaggatt atgaagtttt tcgatgcgaa gattcactgg atgaaagaaa
601 gataaaaggg gtcattgagc tcaggaagag cttactgtct gccttgagaa cttatgaacc
661 atatggatcc ctggttcaac aaatacgaat tctgctgctg ggtccaattg gagctgggaa
721 gtccagcttt ttcaactcag tgaggtctgt tttccaaggg catgtaacgc atcaggcttt
781 ggtgggcact aatacaactg ggatatctga gaagtatagg acatactcta ttagagacgg
841 gaaagatggc aaatacctgc cgtttattct gtgtgactca ctggggctga gtgagaaaga
901 aggcggcctg tgcagggatg acatattcta tatcttgaac ggtaacattc gtgatagata
961 ccagtttaat cccatggaat caatcaaatt aaatcatcat gactacattg attccccatc
1021 gctgaaggac agaattcatt gtgtggcatt tgtatttgat gccagctcta ttcaatactt
1081 ctcctctcag atgatagtaa agatcaaaag aattcgaagg gagttggtaa acgctggtgt
1141 ggtacatgtg gctttgctca ctcatgtgga tagcatggat ttgattacaa aaggtgacct
1201 tatagaaata gagagatgtg agcctgtgag gtccaagcta gaggaagtcc aaagaaaact
1261 tggatttgct ctttctgaca tctcggtggt tagcaattat tcctctgagt gggagctgga
1321 ccctgtaaag gatgttctaa ttctttctgc tctgagacga atgctatggg ctgcagatga
1381 cttcttagag gatttgcctt ttgagcaaat agggaatcta agggaggaaa ttatcaactg
1441 tgcacaagga aaaaaataga tatgtgaaag gttcacgtaa atttcctcac atcacagaag
1501 attaaaattc agaaaggaga aaacacagac caaagagaag tatctaagac caaagggatg
1561 tgttttatta atgtctagga tgaagaaatg catagaacat tgtagtactt gtaaataact
1621 agaaataaca tgatttagtc ataattgtga aaaataataa taatttttct tggatttatg
1681 ttctgtatct gtgaaaaaat aaatttctta taaaactcgg gtctaaaaaa aaaaaaaaaa
1741 aa
**RPGRIP1 (SEQ ID NO: 75)** (retinitis pigmentosa GTPase regulator interacting protein 1, NM 020466.4 
1 atgtcacatc tggtggaccc tacatcagga gacttgccag ttagagacat agatgctata
61 cctctggtgc taccagcctc aaaaggtaag aatatgaaaa ctcaaccacc cttgagcagg
121 atgaaccggg aggaattgga ggacagtttc tttcgacttc gcgaagatca catgttggtg
181 aaggagcttt cttggaagca acaggatgag atcaaaaggc tgaggaccac cttgctgcgg
241 ttgaccgctg ctggccggga cctgcgggtc gcggaggagg cggcgccgct ctcggagacc
301 gcaaggcgcg ggcagaaggc gggatggcgg cagcgcctct ccatgcacca gcgcccccag
361 atgcaccgac tgcaaggcca tttccactgc gtcggccctg ccagcccccg ccgcgcccag
421 cctcgcgtcc aagtgggaca cagacagctc cacacagccg gtgcaccggt gccggagaaa
481 cccaagaggg ggccaaggga caggctgagc tacacagccc ctccatcgtt taaggagcat
541 gcgacaaatg aaaacagagg tgaagtagcc agtaaaccca gtgaacttgt ttctggttct
601 aacagcataa tttctttcag cagtgtcata agtatggcta aacccattgg tctatgcatg
661 cctaacagtg cccacatcat ggccagcaat accatgcaag tggaagagcc acccaagtct
721 cctgagaaaa tgtggcctaa agatgaaaat tttgaacaga gaagctcatt ggagtgtgct
781 cagaaggctg cagagcttcg agcttccatt aaagagaagg tagagctgat tcgacttaag
841 aagctcttac atgaaagaaa tgcttcattg gttatgacaa aagcacaatt aacagaagtt
901 caagaggcat acgaaacctt gctccagaag aatcaggaaa tcctgagtgc agcccatgag
961 gccctcctca agcaagtgaa tgagctcagg gcagagctga aggaagaaag caagaaggct
1021 gtgagcttga agagccaact ggaagatgtg tctatcttgc agatgactct gaaggagttt
1081 caggagagag ttgaagattt ggaaaaagaa cgaaaattgc tgaatgacaa ttatgacaaa
1141 ctcttagaaa gcatgctgga cagcagtgac agctccagtc agccccactg gagcaacgag
1201 ctcatagcgg aacagctaca gcagcaagtc tctcagctgc aggatcagct ggatgctgag
1261 ctggaggaca agagaaaagt tttacttgag ctgtccaggg agaaagccca aaatgaggat
1321 ctgaagcttg aagtcaccaa catacttcag aagcataaac aggaagtaga gctcctccaa
1381 aatgcagcca caatttccca acctcctgac aggcaatctg aaccagccac tcacccagct
1441 gtattgcaag agaacactca gatcgagcca agtgaaccca aaaaccaaga agaaaagaaa
1501 ctgtcccagg tgctaaatga gttgcaagta tcacacgcag agaccacatt ggaactagaa
1561 aagaccaggg acatgcttat tctgcagcgc aaaatcaacg tgtgttatca ggaggaactg
 
1621 gaggcaatga tgacaaaagc tgacaatgat aatagagatc acaaagaaaa gctggagagg
1681 ttgactcgac tactagacct caagaataac cgtatcaagc agctggaagg tattttaaga
1741 agccatgacc ttccaacatc tgaacagctc aaagatgttg cttatggcac ccgaccgttg
1801 tcgttatgtt tggaaacact gccagcccat ggagatgagg ataaagtgga tatttctctg
1861 ctgcatcagg gtgagaatct ttttgaactg cacatccacc aggccttcct gacatctgcc
1921 gccctagctc aggctggaga tacccaacct accactttct gcacctattc cttctatgac
1981 tttgaaaccc actgtacccc attatctgtg gggccacagc ccctctatga cttcacctcc
2041 cagtatgtga tggagacaga ttcgcttttc ttacactacc ttcaagaggc ttcagcccgg
2101 cttgacatac accaggccat ggccagtgaa cacagcactc ttgctgcagg atggatttgc
2161 tttgacaggg tgctagagac tgtggagaaa gtccatggct tggccacact gattggagct
2221 ggtggagaag agttcggggt tctagagtac tggatgaggc tgcgtttccc cataaaaccc
2281 agcctacagg cgtgcaataa acgaaagaaa gcccaggtct acctgtcaac cgatgtgctt
2341 ggaggccgga aggcccagga agaggagttc agatcggagt cttgggaacc tcagaacgag
2401 ctgtggattg aaatcaccaa gtgctgtggc ctccggagtc gatggctggg aactcaaccc
2461 agtccatatg ctgtgtaccg cttcttcacc ttttctgacc atgacactgc catcattcca
2521 gccagtaaca acccctactt tagagaccag gctcgattcc cagtgcttgt gacctctgac
2581 ctggaccatt atctgagacg ggaggccttg tctatacatg tttttgatga tgaagactta
2641 gagcctggct cgtatcttgg ccgagcccga gtgcctttac tgcctcttgc aaaaaatgaa
2701 tctatcaaag gtgattttaa cctcactgac cctgcagaga aacccaacgg atctattcaa
2761 gtgcaactgg attggaagtt tccctacata ccccctgaga gcttcctgaa accagaagct
2821 cagactaagg ggaaggatac caaggacagt tcaaagatct catctgaaga ggaaaaggct
2881 tcatttcctt cccaggatca gatggcatct cctgaggttc ccattgaagc tggccagtat
2941 cgatctaaga gaaaacctcc tcatggggga gaaagaaagg agaaggagca ccaggttgtg
3001 agctactcaa gaagaaaaca tggcaaaaga ataggtgttc aaggaaagaa tagaatggag
3061 tatcttagcc ttaacatctt aaatggaaat acaccagagc aggtgaatta cactgagtgg
3121 aagttctcag agactaacag cttcataggt gatggcttta aaaatcagca cgaggaagag
3181 gaaatgacat tatcccattc agcactgaaa cagaaggaac ctctacatcc tgtaaatgac
3241 aaagaatcct ctgaacaagg ttctgaagtc agtgaagcac aaactaccga cagtgatgat
3301 gtcatagtgc cacccatgtc tcagaaatat cctaaggcag attcagagaa gatgtgcatt
3361 gaaattgtct ccctggcctt ctacccagag gcagaagtga tgtctgatga gaacataaaa
3421 caggtgtatg tggagtacaa attctacgac ctacccttgt cggagacaga gactccagtg
3481 tccctaagga agcctagggc aggagaagaa atccactttc actttagcaa ggtaatagac
3541 ctggacccac aggagcagca aggccgaagg cggtttctgt tcgacatgct gaatggacaa
3601 gatcctgatc aaggacattt aaagtttaca gtggtaagtg atcctctgga tgaagaaaag
3661 aaagaatgtg aagaagtggg atatgcatat cttcaactgt ggcagatcct ggagtcagga
3721 agagatattc tagagcaaga gctagacatt gttagccctg aagatctggc taccccaata
3781 ggaaggctga aggtttccct tcaagcagct gctgtcctcc atgctattta caaggagatg
3841 actgaagatt tgttttcatg aaggaacaag tgctattcca atctaaaagt ctctgaggga
3901 accatagtaa aaagtctctt ataaagttag cttgctataa catgaaaaaa
**DISC1 (SEQ ID NO:76)** - Homo sapiens disrupted in schizophrenia 1 (DISC1), transcript variant q, mRNA - NM_001164554 
1 ggaaggagca ggaggcagcc caggcggagc gggaggagct ggcagcgggg cgcatgccag
61 gcgggggtcc tcaggcgccc ccagccgccg ccggcggcgg cggcgtgagc caccgcgcag
121 gcagccggga ttgcttacca cctgcagcgt gctttcggag gcggcggctg gcacggaggc
181 cgggctacat gagaagctcg acagggcctg ggatcgggtt cctttcccca gcagtgggca
241 cactgttccg gttcccagga ggggtgtctg gcgaggagtc ccaccactcg gagtccaggg
301 ccagacagtg tggccttgac tcgagaggcc tcttggtccg gagccctgtt tccaagagtg
361 cagcagcccc tactgtgacc tctgtgagag gaacctcggc gcactttggg attcagctca
421 gaggtggcac cagattgcct gacaggctta gctggccgtg tggccctggg agtgctgggt
481 ggcagcaaga gtttgcagcc atggatagtt ctgagaccct ggacgccagc tgggaggcag
541 cctgcagcga tggagcaagg cgtgtccggg cagcaggctc tctgccatca gcagagttga
601 gtagcaacag ctgcagccct ggctgtggcc ctgaggtccc cccaacccct cctggctctc
661 acagtgcctt tacctcaagc tttagcttta ttcggctctc gcttggctct gccggggaac
721 gtggagaagc agaaggctgc ccaccatcca gagaggctga gtcccattgc cagagccccc
 
781 aggagatggg agccaaagct gccagcttgg acgggcctca cgaggacccg cgatgtctct
841 ctcggccctt cagtctcttg gctacacggg tctctgcaga cttggcccag gccgcaagga
901 acagctccag gccagagcgt gacatgcatt ctttaccaga catggaccct ggctcctcca
961 gttctctgga tccctcactg gctggctgtg gtggtgatgg gagcagcggc tcaggggatg
1021 cccactcttg ggacaccctg ctcaggaaat gggagccagt gctgcgggac tgcctgctga
1081 gaaaccggag gcagatggag gtaatatcct taagattaaa acttcagaaa cttcaggaag
1141 atgcagttga gaatgatgat tatgataaag gtgagtttta atttgtttat tgattgtttt
1201 gtcatcatgt cccaattttc tttccatctt tactcatatc taccttttga atcccaaaag
1261 aattgtacaa tctgttcctc tgatcatctc taccagggaa tagttgactc ttttacagca
1321 ttattgtttt gtagatttca aagtacttca tgaacattaa tcctttgggt tataaatata
1381 accttatcaa ttgtccagaa acacttagca tactcacaca ataaaaatta tattagcttg
1441 ccacctgtct gcccatggtg tgatgtatct ataatccact tgttcataaa aaatattcgt
1501 ctgacaccta tgatatgcta gggaatatgg gagacaatag gaaagtaaga cagacatggt
1561 atctgccctt gtgatgccag taaacttaag ccttcatggg gctctctgac ttcataattt
1621 ccagaaccag agataggaaa tgaggtgaat ttgagaaatg tcagactgtg ccaaaggggg
1681 tcacatgcat caaatttcca catacat
**CXCR1 (SEQ ID NO: 77)** C-X-C motif chemokine receptor 1, NM_000634.2 
1 tattcatcaa gtgccctcta gctgttaagt cactctgatc tctgactgca gctcctactg
61 ttggacacac ctggccggtg cttcagttag atcaaaccat tgctgaaact gaagaggaca
121 tgtcaaatat tacagatcca cagatgtggg attttgatga tctaaatttc actggcatgc
181 cacctgcaga tgaagattac agcccctgta tgctagaaac tgagacactc aacaagtatg
241 ttgtgatcat cgcctatgcc ctagtgttcc tgctgagcct gctgggaaac tccctggtga
301 tgctggtcat cttatacagc agggtcggcc gctccgtcac tgatgtctac ctgctgaacc
361 tggccttggc cgacctactc tttgccctga ccttgcccat ctgggccgcc tccaaggtga
421 atggctggat ttttggcaca ttcctgtgca aggtggtctc actcctgaag gaagtcaact
481 tctacagtgg catcctgctg ttggcctgca tcagtgtgga ccgttacctg gccattgtcc
541 atgccacacg cacactgacc cagaagcgtc acttggtcaa gtttgtttgt cttggctgct
601 ggggactgtc tatgaatctg tccctgccct tcttcctttt ccgccaggct taccatccaa
661 acaattccag tccagtttgc tatgaggtcc tgggaaatga cacagcaaaa tggcggatgg
721 tgttgcggat cctgcctcac acctttggct tcatcgtgcc gctgtttgtc atgctgttct
781 gctatggatt caccctgcgt acactgttta aggcccacat ggggcagaag caccgagcca
841 tgagggtcat ctttgctgtc gtcctcatct tcctgctttg ctggctgccc tacaacctgg
901 tcctgctggc agacaccctc atgaggaccc aggtgatcca ggagagctgt gagcgccgca
961 acaacatcgg ccgggccctg gatgccactg agattctggg atttctccat agctgcctca
1021 accccatcat ctacgccttc atcggccaaa attttcgcca tggattcctc aagatcctgg
1081 ctatgcatgg cctggtcagc aaggagttct tggcacgtca tcgtgttacc tcctacactt
1141 cttcgtctgt caatgtctct tccaacctct gaaaaccatc gatgaaggaa tatctcttct
1201 cagaaggaaa gaataaccaa caccctgagg ttgtgtgtgg aaggtgatct ggctctggac
1261 aggcactatc tgggttttgg ggggacgcta taggatgtgg ggaagttagg aactggtgtc
1321 ttcaggggcc acaccaacct tctgaggagc tgttgaggta cctccaagga ccggcctttg
1381 cacctccatg gaaacgaagc accatcattc ccgttgaacg tcacatcttt aacccactaa
1441 ctggctaatt agcatggcca catctgagcc ccgaatctga cattagatga gagaacaggg
1501 ctgaagctgt gtcctcatga gggctggatg ctctcgttga ccctcacagg agcatctcct
1561 caactctgag tgttaagcgt tgagccacca agctggtggc tctgtgtgct ctgatccgag
1621 ctcagggggg tggttttccc atctcaggtg tgttgcagtg tctgctggag acattgaggc
1681 aggcactgcc aaaacatcaa cctgccagct ggccttgtga ggagctggaa acacatgttc
1741 ccccttgggg tggtggatga acaaagagaa agagggtttg gaagccagat ctatgccaca
1801 agaaccccct ttacccccat gaccaacatc gcagacacat gtgctggcca cctgctgagc
1861 cccaagtgga acgagacaag cagcccttag cccttcccct ctgcagcttc caggctggcg
1921 tgcagcatca gcatccctag aaagccatgt gcagccacca gtccattggg caggcagatg
1981 ttcctaataa agcttctgtt ccgtgcttgt ccctgtggaa gtatcttggt tgtgacagag
2041 tcaagggtgt gtgcagcatt gttggctgtt cctgcagtag aatgggggca gcacctccta
2101 agaaggcacc tctctgggtt gaagggcagt gttccctggg gctttaactc ctgctagaac
2161 agtctcttga ggcacagaaa ctcctgttca tgcccatacc cctggccaag gaagatccct
2221 ttgtccacaa gtaaaaggaa atgctcctcc agggagtctc agcttcaccc tgaggtgagc
 
2281 atcatcttct gggttaggcc ttgcctaggc atagccctgc ctcaagctat gtgagctcac
2341 cagtccctcc ccaaatgctt tccatgagtt gcagtttttt cctagtctgt tttccctcct
2401 tggagacagg gccctgtcgg tttattcact gtatgtcctt ggtgcctgga gcctactaaa
2461 tgctcaataa ataatgatca caggaaaaaa aaaaaaaaaa aa
**HCAR2 (SEQ ID NO: 78)** - Homo sapiens hydroxycarboxylic acid receptor 2 (HCAR2), mRNA - NM_177551 
1 accacacaga cacacacctc cttgctggag cattcactag gcgaggcgct ccatcggact
61 cactagccgc actcatgaat cggcaccatc tgcaggatca ctttctggaa atagacaaga
121 agaactgctg tgtgttccga gatgacttca ttgtcaaggt gttgccgccg gtgttggggc
181 tggagtttat cttcgggctt ctgggcaatg gccttgccct gtggattttc tgtttccacc
241 tcaagtcctg gaaatccagc cggattttcc tgttcaacct ggcagtggct gactttctac
301 tgatcatctg cctgcccttc ctgatggaca actatgtgag gcgttgggac tggaagtttg
361 gggacatccc ttgccggctg atgctcttca tgttggctat gaaccgccag ggcagcatca
421 tcttcctcac ggtggtggcg gtagacaggt atttccgggt ggtccatccc caccacgccc
481 tgaacaagat ctccaatcgg acagcagcca tcatctcttg ccttctgtgg ggcatcacta
541 ttggcctgac agtccacctc ctgaagaaga agatgccgat ccagaatggc ggtgcaaatt
601 tgtgcagcag cttcagcatc tgccatacct tccagtggca cgaagccatg ttcctcctgg
661 agttcttcct gcccctgggc atcatcctgt tctgctcagc cagaattatc tggagcctgc
721 ggcagagaca aatggaccgg catgccaaga tcaagagagc catcaccttc atcatggtgg
781 tggccatcgt ctttgtcatc tgcttccttc ccagcgtggt tgtgcggatc cgcatcttct
841 ggctcctgca cacttcgggc acgcagaatt gtgaagtgta ccgctcggtg gacctggcgt
901 tctttatcac tctcagcttc acctacatga acagcatgct ggaccccgtg gtgtactact
961 tctccagccc atcctttccc aacttcttct ccactttgat caaccgctgc ctccagagga
1021 agatgacagg tgagccagat aataaccgca gcacgagcgt cgagctcaca ggggacccca
1081 acaaaaccag aggcgctcca gaggcgttaa tggccaactc cggtgagcca tggagcccct
1141 cttatctggg cccaacctct ccttaaataa ccatgccaag aagggacatt gtcaccaaga
1201 accaggatct ctggagaaac agttgggctg ttgcatcgag taatgtcact ggactcggcc
1261 taaggtttcc tggaacttcc agattcagag aatgcgattt agggaaacgg tggcagatga
1321 gtgggagact ggttgcaagg tgtgaccgca ggaatcctgg aggaatagag agtaaagctt
1381 ctaggcatct gaaacttttg cttcatctct gacgctcgca ggactgaaga tgggcaaatt
1441 gtaggcattt ctgctgagca gagttggagc cagagatcta cttgtgactt gttggccttc
1501 ttcccacatc tgcctcagac tggagggggc tcagctcctg gggtgatatc tagcctgctt
1561 gtgagctcta gcagggataa ggagagctga gattggaggg aattgtgttg ctcctggagg
1621 gagcccaggc atcattaaac aagccagtag gtcacctggc ttccgtggac caattcatct
1681 ttcagacaag ctttagcaga aatggactca gggaagagac tcacacgctt tggttaatat
1741 ctgtgtttcc ggtgggtgta ataggggatt agccccagaa gggactgagc taaacagtgt
1801 tattatggga aaggaaatgg cattgctgct ttcaaccagc gactaatgca atccattcct
1861 ctcttgttta tagtaatcta agggttgggc agttaaaacg gcttcaggat agaaagctgt
1921 ttcccacctc tgtttgcttt taacattaaa agggaaatgt gcctctgccc cacagttaga
1981 ggggtgcacg ttcctcctgg ttccttcgct tgtgtttctg tacttaccaa aaatctacca
2041 tttcaataaa ttttgatagg agacaaaaaa aaaaaaaaaa aa
**EPSTI1 (SEQ ID NO:79)** - Homo sapiens epithelial stromal interaction 1 (breast)(EPSTIL1), transcript variant 2, mRNA - NM_033255 
1 aaaaccgctg cctctgcact ttggaatccc atcttgagac tcgctaagcg tcccagccgc
61 atccctcccg cagcgacggc ggcccgggac ccgcgggctg tgaaccatga acacccgcaa
121 tagagtggtg aactccgggc tcggcgcctc ccctgcctcc cgcccgaccc gggatcccca
181 ggacccttct gggcggcaag gggagctgag ccccgtggaa gaccagagag agggtttgga
241 ggcagcccct aagggccctt cgcgggagag cgtcgtgcac gcgggccaga ggcgcacaag
301 tgcatacacc ttgatagcac caaatataaa ccggagaaat gagatacaaa gaattgcgga
361 gcaggagctg gccaacctgg agaagtggaa ggagcagaac agagctaaac cggttcacct
421 ggtgcccaga cggctaggtg gaagccagtc agaaactgaa gtcagacaga aacaacaact
 
481 ccagctgatg caatctaaat acaagcaaaa gctaaaaaga gaagaatctg taagaatcaa
541 gaaggaagct gaagaagctg aactccaaaa aatgaaggca attcagagag agaagagcaa
601 taaactggag gagaaaaaaa gacttcaaga aaaccttaga agagaagcat ttagagagca
661 tcagcaatac aaaaccgctg agttcttgag caaactgaac acagaatcgc cagacagaag
721 tgcctgtcaa agtgctgttt gtggcccaca atcctcaaca tgggccagaa gctgggctta
781 cagagattct ctaaaggcag aagaaaacag aaaattgcaa aagatgaagg atgaacaaca
841 tcaaaagagt gaattactgg aactgaaacg gcagcagcaa gagcaagaaa gagccaaaat
901 ccaccagact gaacacagga gggtaaataa tgcttttctg gaccgactcc aaggcaaaag
961 tcaaccaggt ggcctcgagc aatctggagg ctgttggaat atgaatagcg gtaacagctg
1021 gggtatatga gaaaatattg actcctatct ggccttcatc aactgacctc gaaaagcctc
1081 atgagatgct ttttcttaat gtgattttgt tcagcctcac tgtttttacc ttaatttcaa
1141 ctgcccacac acttgaccgt gcagtcagga gtgactggct tctccttgtc ctcatttatg
1201 catgtttgga ggagctgatt cctgaactca tatttaatct ctactgccag ggaaatgcta
1261 cattattttt ctaattggaa gtataattag agtgatgttg gtagggtaga aaaagaggga
1321 gtcacttgat gctttcaggt taatcagagc tatgggtgct acaggcttgt ctttctaagt
1381 gacatattct tatctaattc tcagatcagg ttttgaaagc tttgggggtc tttttagatt
1441 ttaatcccta ctttctttat ggtacaaata tgtacaaaag aaaaaggtct tatattcttt
1501 tacacaaatt tataaataaa ttttgaactc cttctgtata aatgggtcat ttttattttt
1561 aatgaaaagt tattggggtt ttctctcttg aagggtctca ttttaattcc cttttccagg
1621 ccgtatagat caaatatagt actgtcatta ctgttggctc ttgttttggt cttgacttac
1681 taatagtgtt accctgattt tcagaggggg acagtttatc tccagaaagg ccaatgtttg
1741 tatacacatc agctagacac aaatatagac atcatatgta gtttgtacat gtttcagaaa
1801 cttgtttttt ctttgctctg tgtaacctat ttcctattgc tagttcagtt ggctttctta
1861 ttcacttctg tgaccctgaa ccagttctca gaccctagag tgtaagagca ttgattttct
1921 acgctgtgta atctagctca atccctctgt cccctccgcc tcaccgtccc ccagccacca
1981 cattgtatag caaaagcatt acattcaatc ctagaataaa ggtaaataca acaaatcatc
2041 tttgcagctg gacaactaat aatactttgc agcattaaga gatcttctgt gttaccagtc
2101 actctgttga aatgaacttt ccgaatctct ttattcagga aaacatgggg ttttgaaatt
2161 cttgggccaa gagacataac tgaggggttc gcagagctag gcaagggtgc actaggaaag
2221 ggccacattg gtgggtgggg ggtaacagag aacagatggt gtcaggaagt ttctctggag
2281 taaataatgt ggatattctt ggtttccctc tcctccgcca gctgaagctg tgttagtgct
2341 gttgacacta atataaaatg tttggtccat ttgaaatcct tgtcattgcc ttatatgggg
2401 gaaactcaat cccccagcct gtgttggaaa tatcaccaaa ctgattgtaa atgtgcggct
2461 gtagcagaca ttttagtgtg gtggtgtgca gccatttcgg ccctacacct gccagcctgg
2521 ctaccttaca gttgtgttcc gatttttgcg tctatgcttg gtgtgcctca cttgctgcat
2581 tttccagcat gcaaccagga gttgacgtag gaaaaaggga tgctttctta ctttggaagc
2641 tctcagggaa gttggtgtca atttctcctc cactgcctgg cctaccctgc actcccaaag
2701 attttgtgca gatgggtagt tccatttttt aaaaattgtg cagatatgga aaattgtgac
2761 ttacttcatg accagaacta tctagaatat gtgtgggggt ataaacatct tgcttaacca
2821 aatatctatg taggcagagg taaccaggag agaagcaaga cttgctgcct aaaggagccc
2881 accattttac ttttcacatt taatctgcca cgttgaatca attggaataa aacctgactc
2941 gcaggtgact ggacaggaaa tcccaaagtt ccaccatttc tatgcttaat tttaatgtcc
3001 ccccgctttt ttttttgtag aaaataaaaa caagaaaatc gttccaatgt aagatgtttg
3061 ttatagaaac tttaggcaat acaggtgtgt aataaaatgt ttaataaact tctaaacact
3121 tttgtatttg gattta
**LILRB4 (SEQ ID NO: 80)** Homo sapiens leukocyte immunoglobulin like receptor B4 (LILRB4), transcript variant 1, mRNA NM 001278426.3 
1 agaacctggt gcctgcctca gccctagctc tggggaaatg aaagccaggc tggggttcaa
61 atgagggcag tttcccttcc tgtgggctgc tgatggaaca accccatgac gagaaggacc
121 cagcctccaa gcggccacac cctgtgtgtc tctttgtcct gccggcactg aggactcatc
181 catctgcaca gctggggccc ctgggaggag acgccatgat ccccaccttc acggctctgc
241 tctgcctcgg gctgagtctg ggccccagga cccacatgca ggcagggccc ctccccaaac
301 ccaccctctg ggctgagcca ggctctgtga tcagctgggg gaactctgtg accatctggt
361 gtcaggggac cctggaggct cgggagtacc gtctggataa agaggaaagc ccagcaccct
 
421 gggacagaca gaacccactg gagcccaaga acaaggccag attctccatc ccatccatga
481 cagaggacta tgcagggaga taccgctgtt actatcgcag ccctgtaggc tggtcacagc
541 ccagtgaccc cctggagctg gtgatgacag gagcctacag taaacccacc ctttcagccc
601 tgccgagtcc tcttgtgacc tcaggaaaga gcgtgaccct gctgtgtcag tcacggagcc
661 caatggacac ttttcttctg atcaaggagc gggcagccca tcccctactg catctgagat
721 cagagcacgg agctcagcag caccaggctg aattccccat gagtcctgtg acctcagtgc
781 acggggggac ctacaggtgc ttcagctcac acggcttctc ccactacctg ctgtcacacc
841 ccagtgaccc cctggagctc atagtctcag gatccttgga gggtcccagg ccctcaccca
901 caaggtccgt ctcaacagct gcaggccctg aggaccagcc cctcatgcct acagggtcag
961 tcccccacag tggtctgaga aggcactggg aggtactgat cggggtcttg gtggtctcca
1021 tcctgcttct ctccctcctc ctcttcctcc tcctccaaca ctggcgtcag ggaaaacaca
1081 ggacattggc ccagagacag gctgatttcc aacgtcctcc aggggctgcc gagccagagc
1141 ccaaggacgg gggcctacag aggaggtcca gcccagctgc tgacgtccag ggagaaaact
1201 tctgtgctgc cgtgaagaac acacagcctg aggacggggt ggaaatggac actcggcaga
1261 gcccacacga tgaagacccc caggcagtga cgtatgccaa ggtgaaacac tccagaccta
1321 ggagagaaat ggcctctcct ccctccccac tgtctgggga attcctggac acaaaggaca
1381 gacaggcaga agaggacaga cagatggaca ctgaggctgc tgcatctgaa gccccccagg
1441 atgtgaccta cgcccggctg cacagcttta ccctcagaca gaaggcaact gagcctcctc
1501 catcccagga aggggcctct ccagctgagc ccagtgtcta tgccactctg gccatccact
1561 aatccagggg ggacccagac cccacaagcc atggagactc aggaccccag aaggcatgga
1621 agctgcctcc agtagacatc actgaacccc agccagccca gacccctgac acagaccact
1681 agaagattcc gggaacgttg ggagtcacct gattctgcaa agataaataa tatccctgca
1741 ttatcaaaat aaagtagcag acctctcaat tcacaatgag ttaactgata aaacaaaaca
1801 gaagtcagac aatgttttaa attgaatgat catgtaaata ttacacatca aaccaatgac
1861 atgggaaaat gggagcttct aatgaggaca aacaaaaaat agagaaaaat taataaagtc
1921 aaaatgttta ttcttgaaaa cattaatgat acatgaatct tggccacaat gagaaaaata
1981 aaaatgaaaa aagagcaggc atccatttcc atacaggaac aaaataggag gcagcactac
2041 agaccctaca cacagcttta cagaggtgaa agaaaactgt cagcaattct atgctgacat
2101 aacagaaaat gtagatgaga tagatgaaat acgaaaaatt acagtttact taatgaacat
2161 aaggataaat agaaaaactg aatcatcata cataaacata tataaaatgc attgatcctg
2221 taatcaaaaa tgttcccaca aagtaaatgc cacttcagca aggtttgttg gtggtttttt
2281 caaactctta tgcactcatg aaacacacag acacacacac acacaaactt gcataaattt
2341 tccctgagaa tattttgtat atatttacac aaatacattt gatcagacta ggaacaagtt
2401 gataccaaaa cctgaaaagg aaactacaga atgggaaagt catagaagat ctctcacaga
2461 aatataaatc ccttaacaaa tattaacaag taagattcat gtctctataa aatagacagt
2521 atatcatgac cacactggtt ttttgttatc ctttgatttt gtttatgaaa agcaaggata
2581 gcttaatttt caaaaactca atcaatgtaa ttcagtattt taacaaaagg aatgaaaaat
2641 tatcatctca atagacaaag cttttgtctg agcacctttt catatagctg ctgaccattt
2701 gtatgtcttc ttttgagaaa tgcctgttca gctactttgc ccatgtttca agtagttttt
2761 ggtttcttgc tgttgctttg ttttagttcc ttacatattt ttgcatatta accctttatc
2821 aggtatacag cttgcaacta ttttctccca tttctgagtt gtctcttcat tctgtttgca
2881 gaagctgttt agaagccaca ccttttgtct atttttgctt ttgttgcttg tgttttcagg
2941 gccatatcca aaaaaacctt gcccggacca acgtcttgaa gcttttctcc cacccatttt
3001 tgtatatggg ataagggttc aatttcattc ttcttcatat gaatatcccc aggatgtgtc
3061 ctatgcccag ctgcacagct taccctcaaa cagaaaataa tgaagccttc ttcctcccag
3121 gaaaggggac gttcagctga gccgagtgtg tatactgctc tggccatcca ctagcccagg
3181 gaggacccag acctccacac tccatggaga ctcagttctc ctaggaccat ttattcaaaa
3241 ggactgccct ctcttgttct tggaaacttt gttgaggatc aattcaccat aaatatgtgt
3301 gtttccttct ttgctttcat ccctgttgca ctgatcactg tacctgtttc tattccagtt
3361 ccatgatgtc ttcctggctg tagctttgta ggatatttgg ggattccata gtgtgatatc
3421 cccttcttcc ctttgctcaa gattgttttg gctatttggg gtccttttgt agtcccattc
3481 aaattttagg attgtttttc tatttctgtg gaaaacgacc ttggaatttt gttaggaatt
3541 gcattgagtc tgcaggtatg aacttttttt taaagttcca gggcacatgt acaggacctg
3601 cagctttgtt acataggtag gcttgtgcca tggtggtttg ctgcacctat caacccatta
3661 cctagttatt aagcccagca tgcattagct ctttttcctg atgctctccc tcccttcatc
3721 atccgccctc ccactacaag ccccagtgtg tgttgttccc ctccctgtgt ccatgtgttc
 
3781 tcattgttat acgaacattt taacaatgtt aattcttgca gaccatgaac ataagctacc
3841 ttcccattta tatgcgtctt gttcaatttc attcatcaat gttataaaga ttttagtgca
3901 ga
**LILRB5 (SEQ ID NO: 81**)Homo sapiens leukocyte immunoglobulin like receptor B5 (LILRB5), transcript variant 2, mRNA NM_006840.4 
1 cagtcttgtg acagggagaa cccagcctcc agtccacact ctgcgtgttt ttgtgtcctg
61 ccaggcaccg tggtctcatc cgcctgcaca gctgagtcca gtgggagctg acgccatgac
121 cctcaccctc tcagtcctga tttgcctcgg gctgagtgtg ggccccagga cctgcgtgca
181 ggcaggcacc ctccccaaac ccaccctctg ggctgagcca gcctctgtga tagctcgggg
241 gaagcccgtg accctctggt gtcaggggcc cctggagact gaggagtacc gtctggataa
301 ggagggactc ccatgggccc ggaagagaca gaacccactg gagcctggag ccaaggccaa
361 gttccacatt ccatccacgg tgtatgacag tgcagggcga taccgctgct actatgagac
421 ccctgcaggc tggtcagagc ccagtgaccc cctggagctg gtggcgacag gattctatgc
481 agaacccact cttttagccc tgccgagtcc tgtggtggcc tcaggaggaa atgtgaccct
541 ccagtgtgat acactggacg gacttctcac gtttgttctt gttgaggaag aacagaagct
601 ccccaggacc ctgtactcac agaagctccc caaagggcca tcccaggccc tgttccctgt
661 gggtcccgtg acccccagct gcaggtggag gttcagatgc tattactatt acaggaaaaa
721 ccctcaggtg tggtcgaacc ccagtgacct cctggagatt ctggtcccag gcgtgtctag
781 gaagccctcc ctcctgatcc cgcagggctc tgtcgtggcc cgcggaggca gcctgaccct
841 gcagtgtcgc tctgatgtcg gctatgacat attcgttctg tacaaggagg gggaacatga
901 cctcgtccag ggctctggcc agcagcccca ggctgggctc tcccaggcca acttcaccct
961 gggccctgtg agccgctccc acgggggcca gtacagatgc tacggtgcac acaacctctc
1021 ccctaggtgg tcggccccca gcgaccccct ggacatcctg atcgcaggac tgatccctga
1081 catacccgcc ctctcggtgc agccgggccc caaggtggcc tcaggagaga acgtgaccct
1141 gctgtgtcag tcatggcatc agatagacac tttctttttg accaaggagg gggcagccca
1201 tcccccgctg tgtctaaagt caaagtacca gtcttataga caccaggctg aattctccat
1261 gagtcctgtg acctcagccc agggtggaac ctaccgatgc tacagcgcaa tcaggtccta
1321 cccctacctg ctgtccagcc ctagttaccc ccaggagctc gtggtctcag gaccctctgg
1381 ggatcccagc ctctcaccta caggctccac ccccacacct ggccctgagg accagcccct
1441 cacccccacg gggttggatc cccagagtgg tctgggaagg cacctggggg ttgtgactgg
1501 ggtctcagtg gccttcgtcc tgctgctgtt cctcctcctc ttcctcctcc tccgacatcg
1561 gcatcagagc aaacacagga catcggccca tttctaccgt cctgcagggg ctgcggggcc
1621 agagcccaag gaccagggcc tgcagaagag ggccagccca gttgctgaca tccaggagga
1681 aattctcaat gctgccgtga aggacacaca gcccaaggac ggggtggaga tggatgctcg
1741 ggctgctgca tctgaagccc cccaggatgt gacctacgcc cagctgcaca gcttgaccct
1801 cagacgggag gcaactgagc ctcctccatc ccaggaaagg gaacctccag ctgaacccag
1861 catctacgcc cccctggcca tccactagcc cacgggggac ccagatctca tactcaacag
1921 aaggagactc agagactcca gaaggcacag gagctgcccc cagtggacac caatgaaccc
1981 cagccagcct ggacccctaa caaagaccac caggacatcc tgggaactct gggactcact
2041 agattctgca gtcaaagatg actaatatcc ttgcattttt gaaatgaagc cacagacttc
2101 tcaataaatc aatgagctga gaaaactgaa acagaaatta gagcatggta taaatttgga
2161 atgataatgt aaatattaca cattaaatga tgaaatcgga aaactacaaa tgagcgaatg
2221 aattagaaaa gaataaaacc tacgtaatta atgaccttgg caatgacag
**NECAB1 (SEQ ID NO: 82)** - Homo sapiens N-terminal EF-hand calcium binding protein 1 (NECAB1), mRNA - NM 022351 
1 agaggccgga ggagggaggg ggggacaccg agcgcggaga gcgcggagag cgcggaggga
61 ggcgcgcgcg ggagcgaaca ccctcccgga tccagagccc ggcggcggcg aagcagcagc
121 tgcggccgcg cccttgccag agccggtgcg tccgcctagc cccgctccgc ctgaggccgt
181 caggctcccc gaggatggaa gattcccagg agacatcgcc gtcctccaac aactcctcgg
241 aggagctcag ctctgctctg cacctgtcca agggcatgtc gatcttcctc gacatactga
301 ggagagcaga caaaaatgat gatggaaaat tatcctttga agaattcaaa gcatattttg
361 cagatggtgt tctcagtgga gaagaattac acgagctttt ccataccatt gatacacata
421 atactaataa tcttgacaca gaagagctat gtgaatattt ttctcagcac ttgggcgagt
481 atgagaatgt actagcagca cttgaagacc tgaatctttc catcctgaag gcaatgggca
541 aaacaaagaa agactaccaa gaagcctcca atttggaaca attcgtaact agatttttat
601 tgaaggaaac cctgaatcag ctgcagtctc tccagaattc cctggaatgt gccatggaaa
661 ctactgagga gcaaacccgt caagaaaggc aagggccagc caagccagaa gtcctgtcga
721 ttcaatggcc tggaaaacga tcaagccgcc gagtccagag acacaacagc ttctccccaa
781 acagccctca gtttaatgtc agcggtccag gcttattaga agaagacaac cagtggatga
841 cccagataaa tagactccag aaattaattg atagactgga aaagaaggat ctcaaactcg
901 aaccaccaga agaagaaatt attgaaggga atactaaatc tcacatcatg cttgtgcagc
961 ggcagatgtc tgtgatagaa gaggacctgg aagaattcca gctcgctctg aaacactacg
1021 tggagagtgc ttcctcccaa agtggatgct tgcgtatttc tatacagaag ctttcaaatg
1081 aatctcgcta catgatctat gagttctggg agaatagtag tgtatggaat agccaccttc
1141 agacaaatta tagcaagaca ttccaaagaa gtaatgtgga tttcttggaa actccagaac
1201 tcacatctac aatgctagtt cctgcttcgt ggtggatcct gaacaactag atgttcctag
1261 acattttctt tatggttcca agtgcaaaac aggtgttctt atctaaaacg tcaattagaa
1321 aattatctgc ggttgttaat ctactgtata tttttgtttg gtatatttac taagtgcact
1381 ctttcaaaac ttattctata actttatcaa ttcatgtgaa ttttagctca attttcaaag
1441 ttcactaata ttctcaatat ttaatgctaa atgctttgct acattgtaac tcacctaaaa
1501 ccttttagtg acaaaatcct aatatgtgga aaaaagcata tgcataaagg aataatattg
1561 tgaaaatgaa tctgttatga taaagaaaaa ataaagtgga aacttttaga gtattacttc
1621 atagggcaga ttttgtaaac tgtcgtatac tgtaaagggt taaatcagcg ttttgtgatt
1681 tttaagtaac tgtgagtgaa gtttattctt caacaatgtc tactccatcc ccaacccaac
1741 tcacagccct atgactacta tctttgcatt agttaaaaag ttagtatata ggcatcaaac
1801 aaccttggct gtaacctata gaatctctat ccacgtatca ggttatagac tggtttttca
1861 aaagtgaaca atcctgtgat aagttggagt accatttagt aatacagcaa cattgtgtca
1921 tttattagca tcataattct ttgttatgta agttaaatat atcaagaaag aagagactgt
1981 ttggaaaaat gtggttcaag ttttatgcta tatagttttg gtatgcgata cagacagcta
2041 acttttctta tgaaaaatac atatttgcat gtaaacaatg atttcaaaat acttgaaaaa
2101 taaaatttta acccaaatga ataactaaga aatataaaac aagcacaaaa tcttagggaa
2161 gtcataaaat agtagtgaaa gtattagaca gaagacatct gttttcgaat ttcaacacta
2221 gaatgactaa aactatctac ctatagaact atctgtagat agtatactat ctacactctg
2281 ctcaacaagc tcagaaatta aatattttta ataataaaaa tctgttctgg ttataaacct
2341 tgctaatgaa aatacaatac atataaaaat gtatagccat gttattttct agtataaatt
2401 cctttgaaac tataagtctt tgaggaaaat tataaggtaa aattttcctg tttttccccc
2461 tttgaaaaac tcaggaaaaa aggaagattg aactaataaa attttatttc ttaaatataa
2521 atttgaccta aaatattttc tcaaactaat tcatgaaaca gcaactttta ccaatacctt
2581 tgtatactct cagttctcat tcagtataaa taaaatttta aaatcctttc atagttctat
2641 tagaaataag tagtaaattt tgatatattg tacatacaca cgtgtgtgtg tgtgtgtgtg
2701 tgtgtgtgtg tgtgtgtgtg tgtatttgtg tgcctctggt caactctaag gatgacagac
2761 actgtgtaac aacacctggg tcaactcttt taatttatat acaaagcaaa gaacaacatt
2821 aatggagatg cacaatgatt attcaaacaa gctatatata tgtacaaagg caaacagaca
2881 cataacagtc tctgcagact gattgtatat agtaagaaaa gatcaaaaga ctttaaaacc
2941 taaatgactt ttgacataca aactcttctt gagaatgttt gttgtaaatg gtttcaaaaa
3001 tacaaattat agccaatcaa aacattgctt tggttggtgc atttaagtat ccaactcaaa
3061 aagcatatca aatattttgg gtactaggca gtttccaaag tagcatggta gtattacttg
3121 ttaaaagggt tctgttttca ttaacagtac taagtggaag ggatctgcag attccaaact
 
3181 ggaataagct ctatcatatt ctgaaacaag aattagaatg acttgagaac gggcaaataa
3241 caaagcaaac caatataatt atatggtcat tctgacccca gctcttatac aaattataca
3301 tgtatttttg tgtatgtttg tgagagttgt atgtatgtga atgtgtgtga gtgtgtattc
3361 acatacacat atatactgga acctatagta gaaaaggaaa ctagtagggc caaaaaaaaa
3421 aagaaaaaga aaaagaaaaa agaaaaaaaa agaaaaaact gggacctaag tataaatatc
3481 tcatcctaaa gtaaacaata agtttatagt taacgaagat ttttttctat ttaaaacccc
3541 attttcctaa agaacaaagt agtaaaataa aaaaaaattt aaaaaattaa aaaataaaaa
3601 aaagaggtca ctaaaagacc aagatgggaa acgtaacatg gaatacaagc ttgaatctgg
3661 gaaacaagct caaaaaacag tgaaaaaaaa agtactggtt taggagttca aaattcagtg
3721 aaacaaactt tttgccaata gacctaggga tctaagaaat agaattaggg agagtttaca
3781 cttacttacc tatatatcag cagatttttc tggaagaaac cctttttttt ttttttttta
3841 aacagtaaca tccagagtga caaattgcag gagatttaat gtataaaatt tctaggaatt
3901 aaaagcttaa acccaaaaat tgttcatcca tatataagaa attatcgaat taaaacttaa
3961 tgtatgtcaa ttattttcaa atgtctaaat tcctttggaa ataaaaatat cagttttact
4021 ttgaaattgc tcaacttctg ctattcatat ggtctgatta gtgacataag tagcagccgt
4081 ttttcaactt cagtttcatt cactaccatt gtttccaaat tatcaatctc tgctagtaat
4141 tatgaattta agaccatatt attatcaaaa acctagagac caatcatata tctgaaaaga
4201 aatacccatt aaaaattctg cctcctgttt attgagaact atgcattgaa cattctgaat
4261 cattctaagc ctctggagag actgtaatga tccattcatg agctggtatg aaatcagtgg
4321 gaagcagaac aacagaagga actttaaaag caacaagcaa ttatgtacca tatatacact
4381 gtagcaaata ttttatattt gagagtcttt acagcttaca tttccattcc attattacaa
4441 gtgatgaaaa acaaaaaatt gcaagtagta tgcaaattat aaatatacag tcttcccact
4501 tcactaacca aattcctact ttccagtgtt acttcccaat ttatgcagga aacctcctgc
4561 aaagctgaaa ctgattagaa aattctttat attttaaaat agctctttct catttttaga
4621 gaagtcaaat agccaaccat caaaattaag aataaattga attgtcacag tccattacag
4681 ttattgttgc tagatccacc tcatttgcag atgtccaaac ttaaattcat ctgttcttaa
4741 aatgctactt aaaactttgg ttgttttcct gtaatataaa agaaaaagtt aatttatcaa
4801 ttgattgaat acagttttta ctaattagtt tatcaaacca aatactgtga acgtaccagg
4861 tgtttacaga tttaaatgca tgttaccata gaaactatta aagtaactag aactgtcaaa
4921 taacaaaacg gctcatgttt ttaaaatata tgtaactcat tttaaaatat attaaattgt
4981 attccaaacc tgttcttctg tttctgtggc acctaggttt aaaatatgta ttaatgtgta
5041 aatcacaagt aaaatgaatt ctaatgtaca agtttgtttt aaaaagtgta tgtcaagctt
5101 ttatttacac aataaaatgt tattaaagat ggaaagtcta
**NECAB2 (SEQ ID NO: 83)** - Homo sapiens N-terminal EF-hand calcium binding protein 2 (NECAB2), mRNA - NM 019065 
1 gcggcgggcg cggcgcgatg tgcgagcggg cggcgcgcct gtgcagggcc ggcgcgcaca
61 ggctgctccg ggagccgccg cagcagggcc gggcgctggg cgggctgctg cgctgggtgg
121 gcgccaggat gggcgagccc cgggagtcgc tggcccccgc cgcccccgcg gaccccggcc
181 cagcctcgcc gcgcgggggc accgccgtca tcctggacat tttccgccgt gcggacaaaa
241 atgatgatgg gaagctgtcc ttggaggaat tccagctctt ctttgcagat ggcgtcctta
301 atgagaaaga actggaggat ctctttcaca cgattgactc tgacaacacc aaccatgtgg
361 acaccaagga gctgtgtgat tactttgtgg accacatggg tgactatgag gatgtcctgg
421 cctccctgga gaccttgaat cactctgtcc tgaaggccat gggttatacc aagaaggtat
481 atgagggtgg gagcaacgtg gaccagtttg tgacccgctt cctcctgaag gagacggcca
541 atcagatcca gtcgctgctg agctcagtgg agagtgcggt ggaggccatc gaggaacaga
601 ccagccagct ccgacagaac cacatcaaac ccagccacag cgcggcacag acctggtgtg
661 gaagccccac tcccgcctct gcccccaacc acaagctcat ggctatggaa caaggcaaga
721 cccttccatc tgccacggag gatgcaaagg aagagggtct ggaagcccag atcagccgct
781 tggcagagct gattgggagg ctggagagca aagcactgtg gttcgacctg cagcagcgcc
841 tgtcagatga agatggcacc aacatgcacc tgcagctggt ccggcaggag atggccgtgt
901 gccccgagca actgagcgag tttctggact ctctgcgcca gtatctgcgg gggaccactg
961 gcgtgaggaa ctgcttccac atcactgccg tgaggctctc agatggcttc acctttgtca
1021 tctatgagtt ctgggagaca gaggaggcgt ggaagaggca cctgcagagc cccctgtgta
1081 aggcgttccg gcacgtcaag gtggacacac tgagccagcc tgaggccctc tccaggatct
 
1141 tggtgccagc tgcttggtgc acggtgggac gggactgaca gcctcccaga ggcccgtgga
1201 ggagcccacc agccccttct tcttgtgaag gaaatcccgt ttttttctag acagacactt
1261 tggtgcagaa gcttcttttc aatccatcct ccacaagaag gtgtttccct gttgttaagt
1321 gaaggaggcc gcccctgccc ccacctgaga aggcagagca gtgtctgtgc tgccaggtcc
1381 tggtgaagcc caaggttgaa gggggcggct tcctggagcc agcacccctg cctcctggtc
1441 ctggcctctc ccctacccct cacatggcca cgcatgaccc acactgacca caccctgccc
1501 tcttcggtga cattcttcta cctagtagga gtcatgcccc tgtagtgccc aacctagcca
1561 ggtagccacc actgtgccca ggcgccaaat aaaccctggt tgggaaaaaa aaaaaaaaaa
1621 aaaaaaaaaa aaaaaa
**PKHD1 (SEQ ID NO: 84)** - Homo sapiens polycystic kidney and hepatic disease 1 (autosomal Recessive)(PKHD1), transcript variant 1, mRNA - NM_138694 
1 acatttggct gggacacaaa cgagttaggt gagttatagt ctaacgtgag tgagttacct
61 gcgtggtggc tgcaggctga gctctaacca gataacatgt ccacggattc ttgacagaga
121 gaaataagat cacttagaaa gaaaggatca tttctccctt gagtcacaag gagacagaaa
181 cagaaaaaaa agcacaaaag ctatgctgct ccaatcaaaa ctgaaaatgc ttttaatgtc
241 tgagcaatct taagagtatt gatttaagtg gacagaatga ctgcctggct gatctctctg
301 atgagtattg aagtactact tttggcagta cgtcacctga gtttacatat tgaacctgaa
361 gaaggtagcc ttgcaggggg aacgtggatc acagtcattt ttgatggttt ggagttgggt
421 gttctttacc ccaacaatgg ctctcaattg gagatacacc tggtgaacgt gaacatggtg
481 gtgcccgcac tgcggagtgt tccctgtgac gtctttcctg ttttcttgga tttgcctgtg
541 gtgacatgcc ggaccagatc tgtgctgtct gaagcacatg agggtctgta cttcctggaa
601 gcatacttcg ggggacagct ggtaagcagt ccaaatccag gaccacgaga tagctgtact
661 ttcaagtttt ccaaggcgca gacacccatc gttcaccaag tttatccacc aagtggtgtt
721 ccaggaaaac taatacatgt atatggctgg attatcactg gaagattgga aacttttgat
781 tttgatgctg agtacattga tagcccagtg atcttggaag ctcaaggaga caaatgggtt
841 actccttgct ctcttataaa taggcagatg ggaagctgtt atcctattca ggaggaccat
901 ggtcttggga ctctgcagtg ccatgtggaa ggcgactaca tcggctccca gaatgttagc
961 ttctcagtat ttaacaaagg aaagtcaatg gtccacaaga aggcatggct gatcagtgct
1021 aaacaggatc ttttcctata ccagacacac tcagaaatat tatctgtgtt tccagaaact
1081 gggagccttg ggggaagaac aaacatcaca attacaggag acttttttga caattctgcc
1141 caggttacca ttgcaggcat tccatgtgat attagacacg tgtctcccag gaagattgag
1201 tgcaccactc gggctccagg aaaagatgtg aggctcacca cccctcagcc aggcaatcga
1261 gggcttcttt ttgaagttgg agatgctgtt gagggactgg aactgactga agccacccca
1321 gggtacaggt ggcagattgt ccctaatgcc agttctccat ttgggttttg gtcacaggaa
1381 ggacaacctt tcagagcacg gctcagtggg ttctttgtgg ctccagagac aaataattac
1441 actttctgga ttcaggcaga tagccaagct tccttgcatt tcagttggtc agaggaacca
1501 aggactaagg tgaaagtggc ctccatcagc gtcggcactg ctgactggtt tgactcctgg
1561 gagcagaata gggatgaagg gacctggcag cagaagactc ccaagttgga gctgttgggt
1621 ggagccatgt actacctgga agcagagcat catgggatag ccccaagcag gggatgagg
1681 attggtgtcc agattcacaa cacctggctg aatcctgatg tggtcaccac ttacctacgg
1741 gagaagcacc agatccgagt ccgagcccag aggcttccag aagtacaggt gctgaatgta
1801 tcaggcagag gaaacttctt ccttacttgg gacaatgtct ctagtcagcc aatccctgca
1861 aatgccacag cccatctgat tcaaacaacc attgaggagt tacttgcagt aaaatgcaaa
1921 ctggaacccc tttggtctaa catccttctc cggcttggat ttgaacgagg cccagaagtt
1981 tccaactctg atggggacct caccagtggg acggagccct tctgtggcag gttcagcctc
2041 cgtcagcctc gacaccttgt ccttactccc ccggctgccc agaagggcta tcggctagat
2101 cagtatacac acctgtgtct tgcatacaaa ggccacatga acaagatcct gaagatgatt
2161 gtgtccttca caatcggctt tcaaaacatg gtaaagaata ccacctgtga ctggagtctc
2221 acgaggacca gccccgagag ctggcagttc gattgcactg acctctggga gacttgtgtg
2281 cgttgcttcg gggatctcca gccccctccg gcaaactccc cagtgctggt tcatcagatc
2341 aaccttctcc ctctggccca ggagacgggc ctgttctatg tggatgaaat tattattgca
2401 gacacaaacg taacagtttc tcaagctgat tctggaacgg ctcgcccagg gggcaatctg
2461 gtggaatcag tctctgtggt gggatcccct ccggtctaca gtgtcacctc ctggctggcg
2521 gggtgtggca cggagctccc gctcatcact gcacgctctg tgcccactga aggaacagaa
 
2581 gagggatctg gactggtcct ggtgacgaca cagagacgac agcggacaag tccacctcta
2641 ggaggacact ttcgcatcca gcttcctaat acagtgattt ctgatgtccc tgtacaaatt
2701 tctgctcatc accttcacca gctcttacag aataatgccg atgacttcac atccaggtac
2761 ctcaatgcca gtgacttcac tgtgaaggag gatctataca cttgctacga acacgtgtgg
2821 accttgtcct ggtccactca gattggggat ttgcccaatt ttatcagggt ctctgatgaa
2881 aaccttactg gagtgaatcc tgctgcagcc acgcgtgtgg tatatgatgg tggagttttt
2941 cttggaccca tatttggaga catgttggct actgccaacc agcatactca ggtggttgtg
3001 cgagtgaatg atgtaccagc tcattgccca ggttcctgct ctttccagta cctccaaggg
3061 tcaactccct gtgtccattc tgtgtggtac tccattgatg gtgacatcaa cctaatgatt
3121 tacattaccg gaactggttt ctctggtgac tcccagttct tgcaggttac agtgaacaaa
3181 acgagttgca aagttatttt ctcaaaccag accaatgtag tctgtcagac agatttgcta
3241 cctgttggaa tgcatcggat cttgatgttg gtgagaccct ctggtcttgc catcagtgcc
3301 actggagaag acctcttcct aaatgtgaaa cctagactgg atatggtgga gccttccaga
3361 gctgcggata ttggagggct ctgggccacc atccgaggct ctagtttgga aggtgttagc
3421 ctgatattat ttggatctta ctcgtgtgcc atcaatgtcg ctacaagcaa ttcaagcaga
3481 attcagtgca aagttccacc cagggggaaa gatggacgca ttgtgaatgt gactgtgatc
3541 agaggggact attctgcagt tcttcccaga gcatttacat atgtctcttc cttaaatcca
3601 gttattgtga ctctgagcag aaacataagc aatatagcag gcggtgagac cctggtcatt
3661 ggagtggcga ggctgatgaa ctatacggat ttggatgtgg aagtccacgt ccaggatgcc
3721 ttggctccgg ttcacacaca gtcggcttgg ggcctggagg tggcactgcc cccactgcca
3781 gctggtctcc acagaatttc cgtctctatc aatggggtca gcattcactc acaaggggtt
3841 gatctccaca tccagtacct cacagaagtt ttcagcatcg agccttgctg tgggtccctg
3901 ctgggaggga ccatcctcag catctcagga ataggcttca gcagggaccc agctttggtt
3961 tgggtacttg tgggcaatcg gtcctgtgac attgtgaact taacggaggc gagcatctgg
4021 tgtgaaaccc tgccagcccc ccagataccc gatgcgggcg ctcccactgt tccagctgcc
4081 gtggaggtct gggctggcaa caggttcttc gcccgtggtc cttcaccaag cttggtgggg
4141 aaaggcttca ccttcatgta tgaagcggca gcaacaccag tagtcactgc catgcaagga
4201 gaaatcacaa atagcagcct gagcctgcat gtgggaggaa gtaacctctc caactcagtc
4261 atccttctgg ggaacctgaa ctgtgatgtt gagacacagt ccttccaggg caacgtgagc
4321 ctgtctggat gctccatccc tcttcacagt ctggaggctg gcatctatcc tctccaagta
4381 cgtcagaagc agatgggatt tgctaatatg tctgtggtgc tccagcaatt tgcagtgatg
4441 cctcggataa tggccatctt cccatcgcag ggttcggcat gtggtgggac catacttact
4501 gtgagggggt tgcttcttaa ctctagaagg aggtcagttc gggttgacct ctcgggtcct
4561 tttacttgtg tgattttgag tttgggagac cacaccattc tctgccaggt tagcctggag
4621 ggtgacccct tgcctggagc ttccttctcc ctgaacgtca cagtcctggt caatgggcta
4681 accagcgagt gtcaggggaa ttgcactctt ttcataaggg aagaggcaag tcctgtcatg
4741 gatgccttgt ccacaaacac cagtgggtct ctgaccactg tgctgattag gggtcagagg
4801 ttagccacca cagctgatga gccgatggta tttgtggatg atcaacttcc ttgcaatgta
4861 acttttttta atgcaagcca cgttgtgtgc cagacaagag acttggcccc aggaccccac
4921 tacctgtcag ttttttatac aagaaatggg tatgcttgtt ctggtaatgt ttccagacac
4981 ttctacatta tgccccaagt gtttcattat tttcctaaga atttcagctt acatggtgga
5041 agcctcttga ccatagaggg cacaggcctg agaggacaga acaccacgtc agtctatatt
5101 gaccagcaga cctgcctgac ggtgaacatc ggtgctgagc tcatccggtg cattgttccc
5161 acagggaatg gctctgttgc cctggaaata gaggtagatg gactttggta tcacatagga
5221 gtcattggtt ataacaaggc ctttacccca gaattgatct ctatttctca gagcgatgac
5281 atcttaacct ttgcagtggc ccagatctca ggagctgcaa acattgacat ttttatagga
5341 atgtcaccct gtgtgggtgt ctctggtaac cacaccgttc ttcagtgcgt ggtcccttcc
5401 cttccggccg gggagtacca cgtcagaggc tatgactgca tcagagggtg ggcctcatct
5461 gccctggtgt tcacctcaag agttattatt acagcagtga cggagaactt cggctgcctg
5521 ggtggaaggc tggtgcatgt gtttggagcg ggattttctc cagggaatgt ctcagctgct
5581 gtgtgtggtg ctccctgccg agtcctggct aatgctacag tgtctgcctt cagctgcttg
5641 gttctgcccc tggatgtgtc cttggccttc ctgtgtggcc tgaagcgtga ggaggacagc
5701 tgtgaggctg ccagacacac ctatgtgcag tgtgatttga cagttgccat ggcgacagag
5761 caactgcttg aatcgtggcc ttacctctac atttgcgagg aaagttccca atgcctcttt
5821 gtgccagatc attgggcaga gtcaatgttt ccatcattct cgggcctctt tatcagccct
5881 aaattggaaa gagatgaagt tctcatctat aatagctccc gtaacattac catggaaact
 
5941 gaggcagaga tggagtgtga gacgcccaat cagccaatta ccgtcaagat tactgagata
6001 cggaaacgct ggggccagaa cactcagggc aacttttctt tacagttctg ccggagatgg
6061 tccaggactc acagctggtt tcctgaaagg ctgccacaag atggcgacaa cgtcacagtg
6121 gagaatggcc aattgcttct gctggacact aacacaagca tcctcaactt actgcacatt
6181 aaagggggca agctgatttt catggcccca ggacccatcg agctcagggc acacgccatc
6241 cttgtttctg atggtggaga gctccggatt ggatccgaag acaagccctt ccaaggcaga
6301 gctcagatca cactctacgg gagttcctac tcaactccct tctttcccta tggagtcaag
6361 ttcctggctg tgaggaatgg aactctttct ctgcacggtt cactaccaga agtaattgtc
6421 acctgtctta gagcaactgc ccatgcccta gacacagtgc tggctttaga agatgctgtg
6481 gactggaacc ctggggatga agttgtcatc atcagtggaa caggtgttaa aggtgccaaa
6541 ccgatggaag agattgtcac tgtggaaact gtgcaggata cagacctcta tcttaagtca
6601 cctttgagat attctcacaa ctttacagag aattgggtgg ctggagagca ccatatttta
6661 aaggccactg tggctctgct cagcaggagt attaccatac aaggaaatct cactaatgag
6721 agggagaagc tgcttgtttc atgccaggag gccaatgctc cagaaggtaa tctgcagcac
6781 tgtttgtatt ccatgagtga gaagatgcta ggatccaggg atatgggagc cagagtgatc
6841 gttcagtcct tcccagaaga gcccagccag gtccagttga agggagtgca gtttcaagtc
6901 ttggggcaag ccttccataa gcatctgagc tcactcactc tggtgggagc tatgagagag
6961 tctttcatac agggctgcac agtgaggaac tccttcagta gaggcctcag catgtgcggg
7021 accttgggcc tgaaggtgga cagtaatgta ttctacaata ttttaggtca tgcgctgcta
7081 gttgggacat gcacggagat gagatatatc tcctgggagg caattcatgg aaggaaagat
7141 gactggtcag gacatggaaa tataataaga aacaacgtga tcatccaggt ttctggtgcc
7201 gagggactct ccaatcctga aatgttgaca ccatctggca tctatatctg cagtcccacc
7261 aatgttatag aggggaacag agtgtgtggt gctggctatg gctacttttt ccatctcatg
7321 accaaccaaa catcacaagc tccgcttctt tccttcactc agaacattgc acattcttgt
7381 accaggtatg gtctctttgt ataccctaaa tttcagccac cttgggataa tgtcactggc
7441 accactctgt tccagagctt cacagtttgg gaaagtgcag gtggtgccca gatttttaga
7501 agtagcaatc ttcgcctgaa aaacttcaaa gtttattcat gcagagattt tggaattgac
7561 gtcttggaaa gtgatgcaaa tacttcagtt actgacagct tattacttgg tcattttgcc
7621 cacaaggaaa gtctgtgtat gtcatctggg attaaaactc ctaaaagatg ggaactgatg
7681 gtgtctaaca caacctttgt taattttgat ctcatcaact gtgtggccat tagaacctgt
7741 tcagactgtt cccaaggaca aggtggattt actgtgaaga ccagccagtt gaagtttaca
7801 aactcttcaa acttagtggc atttccattt cctcatgcag caattttgga agacttggat
7861 gggtctctgt ctgggaaaaa cagaagtcac attcttgctt ctatggaaac cctttcagct
7921 tcttgtttgg tcaattcaag ctttggtcgg gttgtccatg gcagtgcctg tggaggaggt
7981 gttctttttc atcgtatgtc tattggttta gcgaatactc ctgaagtttc ttatgattta
8041 accatgactg acagcagaaa taaaacaacc actgtcaatt atgtacgtga tacattgtct
8101 aaccctcgtg gctggatggc tctgctcttg gaccaagaga cctactcatt gcaatctgag
8161 aacctttgga tcaacagatc tctgcagtac tcagcaacct ttgacaactt tgctcctggt
8221 aattacctac tgctggtgca cacagatttg ccgccttacc ctgacatcct cctaagatgt
8281 gggagtcgag tgggtctgtc ttttccattt cttccatcac caggtcagaa ccaaggctgt
8341 gactggttct tcaatagcca gctgaggcaa ctcacctatc tggtttcagg tgaaggccaa
8401 gttcaagtca ttctccgggt gaaggaaggt atgcccccaa ctatttcagc ttctacctct
8461 gcccctgaat cagctttaaa atggtccctc cctgaaacat ggcaaggtgt tgaagaaggc
8521 tggggaggat acaacaatac cattccaggc cctggggatg acgttctcat tttacccaac
8581 agaactgtcc ttgtggatac agatcttcca ttcttcaaag ggctgtatgt gatggggacc
8641 ttagacttcc ctgtggacag aagcaatgtt ctgagtgtgg catgcatggt cattgcaggc
8701 ggggagctga aagttggtac tttagaaaat cccttagaaa aggaacaaaa gcttctgatt
8761 ctccttagag cctcagaggg agtcttttgt gaccgtatga atggaattca tattgaccca
8821 ggaacaattg ggtttatggg gaaagttcat ctttacagtg cttatcctaa gaactcctgg
8881 acacatcttg gagctgatat tgcctcagga aatgagagaa ttatagtaga agatgcagtg
8941 gattggcgcc cccatgacaa aatagtcctt agctcctctt cttatgagcc tcatgaagca
9001 gaggtcctca ctgtgaaaga agtcaagggc caccatgtga ggatctatga acggctcaaa
9061 caccggcata ttggaagtgt acatgtcacg gaggatggcc gacacattcg tttggctgct
9121 gaggttggac tgttgacccg aaatatacaa attcagcctg acgtatcatg tagggggaga
9181 ctgtttgtgg ggtccttcag gaagtccagc cgagaagaat tttcaggtgt ccttcaactt
9241 cttaatgtgg aaattcagaa cttcgggtca ccattgtact catctgttga attcagtaat
 
9301 gtgtcagcag gatcctggat catatcatct actctgcacc agagctgtgg cgggggcatt
9361 catgcagctg ccagtcatgg agtactttta aatgacaata ttgtgtttgg cacagctggc
9421 catggcatag atttagaggg tcaggcctat actgtcacta ataaccttgt ggttctgatg
9481 acacagccag cgtggtccac catttgggtg gcgggaatca aagtgaacca ggtaaaggac
9541 atcaacctcc atggcaacgt tgtggcagga tcagagagac ttggctttca catccgaggc
9601 cacaagtgct cctcttgtga actgctttgg tctgacaatg tggcgcattc aagtcttcat
9661 ggccttcatc tctataagga aagtggactt gacaactgta ccagaatctc tggcttcttg
9721 gctttcaaga actttgacta tggtgccatg ttacatgtag agaacagcgt ggagatagag
9781 aacattactc tggtagacaa tactattggt cttttggcag tagtgtatgt attttctgct
9841 ccacaaaatt ccgtcaaaaa agtgcagatt gtgcttagga attcagtcat tgtggccacc
9901 agctcttctt ttgactgcat tcaggacaaa gtgaagccgc actcagccaa cttgacatca
9961 acagatagag ctccctccaa tccaagagga ggtcgaattg gtattctgtg gcctgtattc
10021 acctcagaac caaatcagtg gcctcaggag ccatggcaca aagtgaggaa tgatcattca
10081 atttcaggaa tcatgaaact tcaagatgtt accttttcta gttttgtgaa gagttgctat
10141 agcgatgacc tggatgtctg cattctacca aatgcagaga acagtggaat tatgcaccca
10201 ataacagcag agaggaccag gatgctaaag ataaaagata aaaacaagtt ctactttcct
10261 tcattacaac ccaggaaaga tttaggaaaa gtagtctgtc ctgaattaga ctgtgcaagt
10321 ccaagaaaat atctcttcaa ggatctggat gggagagccc tgggtctgcc tccaccagtt
10381 tctgtatttc ctaaaacaga ggcagaatgg actgcatcct tcttcaacgc aggtacattt
10441 agagaagaac agaaatgtac ataccaattt ctgatgcaag gattcatctg caaacagact
10501 gaccaagtgg tcctaattct tgatagcgct gatgccattt gggcaattca gaagttatat
10561 ccagttgtat ctgtgactag tggttttgtt gatgtcttta gcagtgtaaa tgccaatatt
10621 ccctgctcta cttctgggtc agtgtctact ttctattcta tcttacccat caggcaaatc
10681 accaaagtct gcttcatgga tcaaactcct caagttttgc gcttttttct attggggaac
10741 aaaagtacct ccaagcttct cttggctgta ttctaccatg agctccagag cccccacgtc
10801 ttcttagggg aaagttttat tccacccact ctggttcagt cagcttcctt attgctgaat
10861 gaatctattg gtgccaacta tttcaacatc atggataacc tcttgtatgt tgtcctacaa
10921 ggagaggagc ccattgaaat acgctcaggt gtttccattc acttggccct cactgtgatg
10981 gtttcagtct tagaaaaagg ctgggaaata gtaatactcg aaagactaac taacttctta
11041 cagattggcc aaaaccaaat caggtttatt cacgagatgc ctggccatga agagacctta
11101 aaggccattg ctgacagtag agcaaaaaga aagcgcaatt gccctactgt gacttgcact
11161 agtcattata gaagagttgg tcaacgtagg cctctcatga tggaaatgaa ctcacatagg
11221 gcttcacccc caatgactgt ggaaactatc tcaaaagtga ttgtcattga aattggtgat
11281 tcgccaacag taaggagcac tggaatgatt tcatccttat caagtaacaa attacagaat
11341 ttggctcatc gagtcatcac tgctcaacag actggggtac tagagaatgt tctgaatatg
11401 actatcgggg ccttactagt tactcagtca aagggagtca ttggctatgg aaatacaagc
11461 agttttaaaa ctgggaactt gatatatatt cggccctatg cactttccat cctagtccag
11521 ccttcagatg gagaagtggg aaatgagctt ccagtgcagc cacaattggt atttttggat
11581 gagcagaatc gaagagtaga gtccctggga cctccttcag agccatggac aatttcagct
11641 tccctggaag gagcatcaga ctcagtgcta aaagggtgca cccaggcaga aactcaagat
11701 ggttatgtta gcttctacaa cttggcagtc ttgatctctg ggtcaaactg gcactttatt
11761 tttactgtca cttctcctcc aggagtcaat tttacagctc gatccaagcc atttgctgtc
11821 ttgcctgtga ctaggaagga gaagtcgacc atcatcctgg ctgcttccct gtcctctgtg
11881 gcctcatggc tggctctgag ctgtctggtg tgctgttggc ttaaaagaag caaaagcaga
11941 aaaacaaaac ctgaagagat tcctgaatcc cagactaata atcaaaatat tcatatccac
12001 atctcatcca aacgccgaga atcacaaggg cccaaaaaag aagacactgt ggtgggagaa
12061 gatatgagaa tgaaggtcat gctgggcaag gtgaaccagt gcccccacca gttgatgaat
12121 ggagtgtcca gaaggaaagt tagccgccac attgtccgag aggaagaggc tgctgtgcct
12181 gctcctggta ctactggcat cacatcccat gggcacatct gtgctccagg tgctcctgct
12241 cagcaggtgt acctgcaaga gactgggaac tggaaggagg gccaagagca gttgctcaga
12301 taccagctgg caggccaaaa tcagctgctg ctgctatgcc cagacttcag acaagagagg
12361 cagcagttgc cagggcaaag tcggctgagt aagcaaagtg gcagcttggg gctttcccaa
12421 gagaagaaag cctcctgcgg ggccactgag gcattctgcc ttcattcagt acacccggaa
12481 actattcagg agcaactgtg atcagggaag ttgggggcat ttggcctgaa aggcagaatg
12541 ttcccagtat ttctggataa taagctgggg aagtgaggac tgtcctgctg ggacaactaa
12601 gaagagagaa tgtggactct gaatcccttt ttcaacttta aaatggaaaa cagtcatata
 
12661 aatgcttaca gactgaaaaa tgtctcatac atattttgca gctatcatga tcctagttca
12721 atgctaggca aataatggca cttgttaatt atttaccagt ttttaactta agcctgattt
12781 taacaacttg ataatggtgt aatactgact tataccagca ggggttatta ttaagcattc
12841 tctggattga ccacccacaa tgctttgagc ttcttttata gaagggatca tgaaaaggtc
12901 tggtcagcta cagtttaatt ccacactgtt acagaaaagc atttctttat cctgtagtag
12961 catttgaagg gacagttcaa caccctgctc cctgatctgt cagagcttac cttccaacca
13021 caaggcaact tcctggcttt ttacaagtgg attttatttc atggtttaaa ttcaatcatt
13081 attgagaacc tacagtagtg tgagcgtatc aaatttgcac ttcatgagga tttacacatg
13141 aaaattgata ttctcatggg ctttgctgag agtttagtta aggacaaaga tgtaaaaatg
13201 ccaaatttgg gaaaggaaat tttgtatgac cactgttcta tttttcagtg attctctctg
13261 ttagtagctg ggtcacattt acaactggaa aagaactaca ttgggagatc agagaaagcc
13321 aagtggccaa gtcctttatc aaaaagctgc tttttggaga agatgatgca taaagctgtg
13381 atactaagga gaggagagat tatgtcatat gcaactgctc taggctgtgc agggggttta
13441 aatggagatc agaacaaagg ctcacctggt attgatacat ttggtaaggt tgttaaaggc
13501 aatttcacca aattcacaca tttgtacggg gcccatttct tttcaaactg aagaatactt
13561 taaacagcct gactgtgtcc ttggctctgg atccagaagg ccttatgctc caagcatgta
13621 aagaaaagat cgcaggaatg tggagagctt ccaaaaagag attgctctca gattttcctg
13681 aaaatcctga aaaccaggat tttccaatct ctacaatgtt aaggggttgt tgtgtagttg
13741 cattggaaga gagaaaggaa acacactctt tcattgctga attctaaata atgatgccct
13801 gaattagttc tatgagaagt actggcagac agctgttatg tctgcagggc atcaaggggc
13861 aggtgtgtct caccctacta agaattggca gaaatgtttt aacctcttct ttaggcatct
13921 ggtgagtatt gcagatcttc aggctgtccc taaaaggagg taggtagcct tctctgtggt
13981 cactgtgttg gatttggaag aaaatataag tagatgttaa ggtcagtgct ctccaggaga
14041 tttcaatcca ggctatctgt gttcagggaa tgatttgcaa gaagttggtt tatagacgtt
14101 tactctggag gcacaagctg gctgccatgc tgagtaagtc ctctttgtag atgatgtatt
14161 ttgcttgggc accaacagaa ttaaaaacga aaaacaaaaa aaaactcgaa tgacttaggc
14221 agaacatgcc ttccctagtt taattagtcc ctgttataac cagttattta tctttttttg
14281 tagattctcg gtcttgaagt catttgcctt tgcaatctct actttagtat actcattgga
14341 tcctcaaaga gccgtaagct ttttggaaaa aaacatttca gaagtgaagg cttttgaggt
14401 ttataaaatg gtagcagtct tagtccttgc cctgcctcag tttacctgca catgctttct
14461 ttcatttcct cggattcttt caccactttt taagtttttt ttccacaggt atttttcctg
14521 agctctatac catgcattta gctgcctagt gagaatatcc agtaggttat cctatagtta
14581 ctatgaagtt aacacagccc aaaaacctac ttctccttgt gtttttttcc aactcaatga
14641 atgacatcac tgcactcaga ccagaaatct agaagtcagt catgactctt cattctattt
14701 cgcccttcac aaagagttag ctgccagctc tgttatttct gccccttctt tacccttctt
14761 ccaattttta ttatcttctt atttatcctc tatgtgtcac aagaccagtc tttctgggag
14821 gcatgtttta tcctatccct ctgcgtagga ataaccataa ttatcttact aattccatcc
14881 cagaaaagcc tgtgatccct gaggccagaa ttgactcttt gggcctaaat agcagctata
14941 aacatcttca gtaacttgcc aggctctagc atgtggcatg gaattttggc atctagctaa
15001 aattcctcaa cccatagtta agctactcct ctttctccta aaaaaaaaaa aaaatgagag
15061 aattagggaa acaccaaaag caaagagagc agctctatgt tcattcatgt ctggagatga
15121 gccaatttga gcaccactgt aggccaactc taaatttatt cagatcgtta acatgggtaa
15181 cccagcaaat atttcccagc agtgacaatc tacacccttt atctaaataa atattgagat
15241 gtgctttttt cctgtgttag ttcaaagaat ctcttcttgc ctcccattgc tgattttctg
15301 tattgatgga ctcatttctc tcaagagatt ggacaagcta aaattctaga gagttcaaaa
15361 gtgtttgaaa gtacatttta agtgttctca ccacaaaaaa tgctagtgtg tgaggtgata
15421 catatgttaa ttagttcaac tgatccattc cacaatgtat acatatttca aactatcgag
15481 ttgtacatga taaatatata caatttttat ttgtcaaata aagaacatct ataaatattt
15541 atagatttca aaattgagaa agaagtatat atggagccac ggttgttttg accatttctc
15601 catgcttttt gtttggggag gggacatttt tttggggggc agggactgtg ttcagggaaa
15661 gtacctgacc aacagaactt caagtctctt ccagagtgcg agactcagct tatctgatca
15721 aggtgaactt gcccttcaat agacttgccc ttgttactgc ccagtgaggc caccaaggac
15781 caagaacagt tgagctctct cagggccttc ccctttcaac aatttagttg aatgactccc
15841 ttccatctat cagtcttact ccaaatatat gaaattacta tacattgaaa tgcctgcaaa
15901 ctatatgcaa aaatgagtat gaggaaatca aatgtaaatc atatgcaaat attattcaac
15961 caagcttcta taaaattgta cgtgtcaata acagtaaaga attttaatta attctgtcat
 
16021 ttaaaattca tgcattccat attttgcttt cagaacttat agtctatgcc aattttgaaa
16081 aaactataag cacaaggaat taaatactat tgagtataga actgttgatt tcaacataca
16141 ctatagcact ttgactaatt acagatatat ttgatagaaa agtcacattt ctacaaggta
16201 attttcaaaa agaaataaaa catttctgta aactt
**PKD1 (SEQ ID NO: 85)** - Homo sapiens polycystin kidney disease 1, transient receptor potential channel interacting (PKD1), transcript variant 2, mRNA - NM 000296 
1 gcactgcagc gccagcgtcc gagcgggcgg ccgagctccc ggagcggcct ggccccgagc
61 cccgagcggg cgtcgctcag cagcaggtcg cggccgcagc cccatccagc cccgcgcccg
121 ccatgccgtc cgcgggcccc gcctgagctg cggcctccgc gcgcgggcgg gcctggggac
181 ggcggggcca tgcgcgcgct gccctaacga tgccgcccgc cgcgcccgcc cgcctggcgc
241 tggccctggg cctgggcctg tggctcgggg cgctggcggg gggccccggg cgcggctgcg
301 ggccctgcga gcccccctgc ctctgcggcc cagcgcccgg cgccgcctgc cgcgtcaact
361 gctcgggccg cgggctgcgg acgctcggtc ccgcgctgcg catccccgcg gacgccacag
421 cgctagacgt ctcccacaac ctgctccggg cgctggacgt tgggctcctg gcgaacctct
481 cggcgctggc agagctggat ataagcaaca acaagatttc tacgttagaa gaaggaatat
541 ttgctaattt atttaattta agtgaaataa acctgagtgg gaacccgttt gagtgtgact
601 gtggcctggc gtggctgccg cgatgggcgg aggagcagca ggtgcgggtg gtgcagcccg
661 aggcagccac gtgtgctggg cctggctccc tggctggcca gcctctgctt ggcatcccct
721 tgctggacag tggctgtggt gaggagtatg tcgcctgcct ccctgacaac agctcaggca
781 ccgtggcagc agtgtccttt tcagctgccc acgaaggcct gcttcagcca gaggcctgca
841 gcgccttctg cttctccacc ggccagggcc tcgcagccct ctcggagcag ggctggtgcc
901 tgtgtggggc ggcccagccc tccagtgcct cctttgcctg cctgtccctc tgctccggcc
961 ccccgccacc tcctgccccc acctgtaggg gccccaccct cctccagcac gtcttccctg
1021 cctccccagg ggccaccctg gtggggcccc acggacctct ggcctctggc cagctagcag
1081 ccttccacat cgctgccccg ctccctgtca ctgccacacg ctgggacttc ggagacggct
1141 ccgccgaggt ggatgccgct gggccggctg cctcgcatcg ctatgtgctg cctgggcgct
1201 atcacgtgac ggccgtgctg gccctggggg ccggctcagc cctgctgggg acagacgtgc
1261 aggtggaagc ggcacctgcc gccctggagc tcgtgtgccc gtcctcggtg cagagtgacg
1321 agagccttga cctcagcatc cagaaccgcg gtggttcagg cctggaggcc gcctacagca
1381 tcgtggccct gggcgaggag ccggcccgag cggtgcaccc gctctgcccc tcggacacgg
1441 agatcttccc tggcaacggg cactgctacc gcctggtggt ggagaaggcg gcctggctgc
1501 aggcgcagga gcagtgtcag gcctgggccg gggccgccct ggcaatggtg gacagtcccg
1561 ccgtgcagcg cttcctggtc tcccgggtca ccaggagcct agacgtgtgg atcggcttct
1621 cgactgtgca gggggtggag gtgggcccag cgccgcaggg cgaggccttc agcctggaga
1681 gctgccagaa ctggctgccc ggggagccac acccagccac agccgagcac tgcgtccggc
1741 tcgggcccac cgggtggtgt aacaccgacc tgtgctcagc gccgcacagc tacgtctgcg
1801 agctgcagcc cggaggccca gtgcaggatg ccgagaacct cctcgtggga gcgcccagtg
1861 gggacctgca gggacccctg acgcctctgg cacagcagga cggcctctca gccccgcacg
1921 agcccgtgga ggtcatggta ttcccgggcc tgcgtctgag ccgtgaagcc ttcctcacca
1981 cggccgaatt tgggacccag gagctccggc ggcccgccca gctgcggctg caggtgtacc
2041 ggctcctcag cacagcaggg accccggaga acggcagcga gcctgagagc aggtccccgg
2101 acaacaggac ccagctggcc cccgcgtgca tgccaggggg acgctggtgc cctggagcca
2161 acatctgctt gccgctggac gcctcctgcc acccccaggc ctgcgccaat ggctgcacgt
2221 cagggccagg gctacccggg gccccctatg cgctatggag agagttcctc ttctccgttc
2281 ccgcggggcc ccccgcgcag tactcggtca ccctccacgg ccaggatgtc ctcatgctcc
2341 ctggtgacct cgttggcttg cagcacgacg ctggccctgg cgccctcctg cactgctcgc
2401 cggctcccgg ccaccctggt ccccaggccc cgtacctctc cgccaacgcc tcgtcatggc
2461 tgccccactt gccagcccag ctggagggca cttgggcctg ccctgcctgt gccctgcggc
2521 tgcttgcagc cacggaacag ctcaccgtgc tgctgggctt gaggcccaac cctggactgc
2581 ggctgcctgg gcgctatgag gtccgggcag aggtgggcaa tggcgtgtcc aggcacaacc
2641 tctcctgcag ctttgacgtg gtctccccag tggctgggct gcgggtcatc taccctgccc
2701 cccgcgacgg ccgcctctac gtgcccacca acggctcagc cttggtgctc caggtggact
2761 ctggtgccaa cgccacggcc acggctcgct ggcctggggg cagtgtcagc gcccgctttg
2821 agaatgtctg ccctgccctg gtggccacct tcgtgcccgg ctgcccctgg gagaccaacg
 
2881 ataccctgtt ctcagtggta gcactgccgt ggctcagtga gggggagcac gtggtggacg
2941 tggtggtgga aaacagcgcc agccgggcca acctcagcct gcgggtgacg gcggaggagc
3001 ccatctgtgg cctccgcgcc acgcccagcc ccgaggcccg tgtactgcag ggagtcctag
3061 tgaggtacag ccccgtggtg gaggccggct cggacatggt cttccggtgg accatcaacg
3121 acaagcagtc cctgaccttc cagaacgtgg tcttcaatgt catttatcag agcgcggcgg
3181 tcttcaagct ctcactgacg gcctccaacc acgtgagcaa cgtcaccgtg aactacaacg
3241 taaccgtgga gcggatgaac aggatgcagg gtctgcaggt ctccacagtg ccggccgtgc
3301 tgtcccccaa tgccacgcta gcactgacgg cgggcgtgct ggtggactcg gccgtggagg
3361 tggccttcct gtggaccttt ggggatgggg agcaggccct ccaccagttc cagcctccgt
3421 acaacgagtc cttcccggtt ccagacccct cggtggccca ggtgctggtg gagcacaatg
3481 tcatgcacac ctacgctgcc ccaggtgagt acctcctgac cgtgctggca tctaatgcct
3541 tcgagaacct gacgcagcag gtgcctgtga gcgtgcgcgc ctccctgccc tccgtggctg
3601 tgggtgtgag tgacggcgtc ctggtggccg gccggcccgt caccttctac ccgcacccgc
3661 tgccctcgcc tgggggtgtt ctttacacgt gggacttcgg ggacggctcc cctgtcctga
3721 cccagagcca gccggctgcc aaccacacct atgcctcgag gggcacctac cacgtgcgcc
3781 tggaggtcaa caacacggtg agcggtgcgg cggcccaggc ggatgtgcgc gtctttgagg
3841 agctccgcgg actcagcgtg gacatgagcc tggccgtgga gcagggcgcc cccgtggtgg
3901 tcagcgccgc ggtgcagacg ggcgacaaca tcacgtggac cttcgacatg ggggacggca
3961 ccgtgctgtc gggcccggag gcaacagtgg agcatgtgta cctgcgggca cagaactgca
4021 cagtgaccgt gggtgcggcc agccccgccg gccacctggc ccggagcctg cacgtgctgg
4081 tcttcgtcct ggaggtgctg cgcgttgaac ccgccgcctg catccccacg cagcctgacg
4141 cgcggctcac ggcctacgtc accgggaacc cggcccacta cctcttcgac tggaccttcg
4201 gggatggctc ctccaacacg accgtgcggg ggtgcccgac ggtgacacac aacttcacgc
4261 ggagcggcac gttccccctg gcgctggtgc tgtccagccg cgtgaacagg gcgcattact
4321 tcaccagcat ctgcgtggag ccagaggtgg gcaacgtcac cctgcagcca gagaggcagt
4381 ttgtgcagct cggggacgag gcctggctgg tggcatgtgc ctggcccccg ttcccctacc
4441 gctacacctg ggactttggc accgaggaag ccgcccccac ccgtgccagg ggccctgagg
4501 tgacgttcat ctaccgagac ccaggctcct atcttgtgac agtcaccgcg tccaacaaca
4561 tctctgctgc caatgactca gccctggtgg aggtgcagga gcccgtgctg gtcaccagca
4621 tcaaggtcaa tggctccctt gggctggagc tgcagcagcc gtacctgttc tctgctgtgg
4681 gccgtgggcg ccccgccagc tacctgtggg atctggggga cggtgggtgg ctcgagggtc
4741 cggaggtcac ccacgcttac aacagcacag gtgacttcac cgttagggtg gccggctgga
4801 atgaggtgag ccgcagcgag gcctggctca atgtgacggt gaagcggcgc gtgcgggggc
4861 tcgtcgtcaa tgcaagccgc acggtggtgc ccctgaatgg gagcgtgagc ttcagcacgt
4921 cgctggaggc cggcagtgat gtgcgctatt cctgggtgct ctgtgaccgc tgcacgccca
4981 tccctggggg tcctaccatc tcttacacct tccgctccgt gggcaccttc aatatcatcg
5041 tcacggctga gaacgaggtg ggctccgccc aggacagcat cttcgtctat gtcctgcagc
5101 tcatagaggg gctgcaggtg gtgggcggtg gccgctactt ccccaccaac cacacggtac
5161 agctgcaggc cgtggttagg gatggcacca acgtctccta cagctggact gcctggaggg
5221 acagggggcc ggccctggcc ggcagcggca aaggcttctc gctcaccgtg ctcgaggccg
5281 gcacctacca tgtgcagctg cgggccacca acatgctggg cagcgcctgg gccgactgca
5341 ccatggactt cgtggagcct gtggggtggc tgatggtggc cgcctccccg aacccagctg
5401 ccgtcaacac aagcgtcacc ctcagtgccg agctggctgg tggcagtggt gtcgtataca
5461 cttggtcctt ggaggagggg ctgagctggg agacctccga gccatttacc acccatagct
5521 tccccacacc cggcctgcac ttggtcacca tgacggcagg gaacccgctg ggctcagcca
5581 acgccaccgt ggaagtggat gtgcaggtgc ctgtgagtgg cctcagcatc agggccagcg
5641 agcccggagg cagcttcgtg gcggccgggt cctctgtgcc cttttggggg cagctggcca
5701 cgggcaccaa tgtgagctgg tgctgggctg tgcccggcgg cagcagcaag cgtggccctc
5761 atgtcaccat ggtcttcccg gatgctggca ccttctccat ccggctcaat gcctccaacg
5821 cagtcagctg ggtctcagcc acgtacaacc tcacggcgga ggagcccatc gtgggcctgg
5881 tgctgtgggc cagcagcaag gtggtggcgc ccgggcagct ggtccatttt cagatcctgc
5941 tggctgccgg ctcagctgtc accttccgcc tgcaggtcgg cggggccaac cccgaggtgc
6001 tccccgggcc ccgtttctcc cacagcttcc cccgcgtcgg agaccacgtg gtgagcgtgc
6061 ggggcaaaaa ccacgtgagc tgggcccagg cgcaggtgcg catcgtggtg ctggaggccg
6121 tgagtgggct gcaggtgccc aactgctgcg agcctggcat cgccacgggc actgagagga
6181 acttcacagc ccgcgtgcag cgcggctctc gggtcgccta cgcctggtac ttctcgctgc
 
6241 agaaggtcca gggcgactcg ctggtcatcc tgtcgggccg cgacgtcacc tacacgcccg
6301 tggccgcggg gctgttggag atccaggtgc gcgccttcaa cgccctgggc agtgagaacc
6361 gcacgctggt gctggaggtt caggacgccg tccagtatgt ggccctgcag agcggcccct
6421 gcttcaccaa ccgctcggcg cagtttgagg ccgccaccag ccccagcccc cggcgtgtgg
6481 cctaccactg ggactttggg gatgggtcgc cagggcagga cacagatgag cccagggccg
6541 agcactccta cctgaggcct ggggactacc gcgtgcaggt gaacgcctcc aacctggtga
6601 gcttcttcgt ggcgcaggcc acggtgaccg tccaggtgct ggcctgccgg gagccggagg
6661 tggacgtggt cctgcccctg caggtgctga tgcggcgatc acagcgcaac tacttggagg
6721 cccacgttga cctgcgcgac tgcgtcacct accagactga gtaccgctgg gaggtgtatc
6781 gcaccgccag ctgccagcgg ccggggcgcc cagcgcgtgt ggccctgccc ggcgtggacg
6841 tgagccggcc tcggctggtg ctgccgcggc tggcgctgcc tgtggggcac tactgctttg
6901 tgtttgtcgt gtcatttggg gacacgccac tgacacagag catccaggcc aatgtgacgg
6961 tggcccccga gcgcctggtg cccatcattg agggtggctc ataccgcgtg tggtcagaca
7021 cacgggacct ggtgctggat gggagcgagt cctacgaccc caacctggag gacggcgacc
7081 agacgccgct cagtttccac tgggcctgtg tggcttcgac acagagggag gctggcgggt
7141 gtgcgctgaa ctttgggccc cgcgggagca gcacggtcac cattccacgg gagcggctgg
7201 cggctggcgt ggagtacacc ttcagcctga ccgtgtggaa ggccggccgc aaggaggagg
7261 ccaccaacca gacggtgctg atccggagtg gccgggtgcc cattgtgtcc ttggagtgtg
7321 tgtcctgcaa ggcacaggcc gtgtacgaag tgagccgcag ctcctacgtg tacttggagg
7381 gccgctgcct caattgcagc agcggctcca agcgagggcg gtgggctgca cgtacgttca
7441 gcaacaagac gctggtgctg gatgagacca ccacatccac gggcagtgca ggcatgcgac
7501 tggtgctgcg gcggggcgtg ctgcgggacg gcgagggata caccttcacg ctcacggtgc
7561 tgggccgctc tggcgaggag gagggctgcg cctccatccg cctgtccccc aaccgcccgc
7621 cgctgggggg ctcttgccgc ctcttcccac tgggcgctgt gcacgccctc accaccaagg
7681 tgcacttcga atgcacgggc tggcatgacg cggaggatgc tggcgccccg ctggtgtacg
7741 ccctgctgct gcggcgctgt cgccagggcc actgcgagga gttctgtgtc tacaagggca
7801 gcctctccag ctacggagcc gtgctgcccc cgggtttcag gccacacttc gaggtgggcc
7861 tggccgtggt ggtgcaggac cagctgggag ccgctgtggt cgccctcaac aggtctttgg
7921 ccatcaccct cccagagccc aacggcagcg caacggggct cacagtctgg ctgcacgggc
7981 tcaccgctag tgtgctccca gggctgctgc ggcaggccga tccccagcac gtcatcgagt
8041 actcgttggc cctggtcacc gtgctgaacg agtacgagcg ggccctggac gtggcggcag
8101 agcccaagca cgagcggcag caccgagccc agatacgcaa gaacatcacg gagactctgg
8161 tgtccctgag ggtccacact gtggatgaca tccagcagat cgctgctgcg ctggcccagt
8221 gcatggggcc cagcagggag ctcgtatgcc gctcgtgcct gaagcagacg ctgcacaagc
8281 tggaggccat gatgctcatc ctgcaggcag agaccaccgc gggcaccgtg acgcccaccg
8341 ccatcggaga cagcatcctc aacatcacag gagacctcat ccacctggcc agctcggacg
8401 tgcgggcacc acagccctca gagctgggag ccgagtcacc atctcggatg gtggcgtccc
8461 aggcctacaa cctgacctct gccctcatgc gcatcctcat gcgctcccgc gtgctcaacg
8521 aggagcccct gacgctggcg ggcgaggaga tcgtggccca gggcaagcgc tcggacccgc
8581 ggagcctgct gtgctatggc ggcgccccag ggcctggctg ccacttctcc atccccgagg
8641 ctttcagcgg ggccctggcc aacctcagtg acgtggtgca gctcatcttt ctggtggact
8701 ccaatccctt tccctttggc tatatcagca actacaccgt ctccaccaag gtggcctcga
8761 tggcattcca gacacaggcc ggcgcccaga tccccatcga gcggctggcc tcagagcgcg
8821 ccatcaccgt gaaggtgccc aacaactcgg actgggctgc ccggggccac cgcagctccg
8881 ccaactccgc caactccgtt gtggtccagc cccaggcctc cgtcggtgct gtggtcaccc
8941 tggacagcag caaccctgcg gccgggctgc atctgcagct caactatacg ctgctggacg
9001 gccactacct gtctgaggaa cctgagccct acctggcagt ctacctacac tcggagcccc
9061 ggcccaatga gcacaactgc tcggctagca ggaggatccg cccagagtca ctccagggtg
9121 ctgaccaccg gccctacacc ttcttcattt ccccggggag cagagaccca gcggggagtt
9181 accatctgaa cctctccagc cacttccgct ggtcggcgct gcaggtgtcc gtgggcctgt
9241 acacgtccct gtgccagtac ttcagcgagg aggacatggt gtggcggaca gaggggctgc
9301 tgcccctgga ggagacctcg ccccgccagg ccgtctgcct cacccgccac ctcaccgcct
9361 tcggcgccag cctcttcgtg cccccaagcc atgtccgctt tgtgtttcct gagccgacag
9421 cggatgtaaa ctacatcgtc atgctgacat gtgctgtgtg cctggtgacc tacatggtca
9481 tggccgccat cctgcacaag ctggaccagt tggatgccag ccggggccgc gccatccctt
9541 tctgtgggca gcggggccgc ttcaagtacg agatcctcgt caagacaggc tggggccggg
 
9601 gctcaggtac cacggcccac gtgggcatca tgctgtatgg ggtggacagc cggagcggcc
9661 accggcacct ggacggcgac agagccttcc accgcaacag cctggacatc ttccggatcg
9721 ccaccccgca cagcctgggt agcgtgtgga agatccgagt gtggcacgac aacaaagggc
9781 tcagccctgc ctggttcctg cagcacgtca tcgtcaggga cctgcagacg gcacgcagcg
9841 ccttcttcct ggtcaatgac tggctttcgg tggagacgga ggccaacggg ggcctggtgg
9901 agaaggaggt gctggccgcg agcgacgcag cccttttgcg cttccggcgc ctgctggtgg
9961 ctgagctgca gcgtggcttc tttgacaagc acatctggct ctccatatgg gaccggccgc
10021 ctcgtagccg tttcactcgc atccagaggg ccacctgctg cgttctcctc atctgcctct
10081 tcctgggcgc caacgccgtg tggtacgggg ctgttggcga ctctgcctac agcacggggc
10141 atgtgtccag gctgagcccg ctgagcgtcg acacagtcgc tgttggcctg gtgtccagcg
10201 tggttgtcta tcccgtctac ctggccatcc tttttctctt ccggatgtcc cggagcaagg
10261 tggctgggag cccgagcccc acacctgccg ggcagcaggt gctggacatc gacagctgcc
10321 tggactcgtc cgtgctggac agctccttcc tcacgttctc aggcctccac gctgaggcct
10381 ttgttggaca gatgaagagt gacttgtttc tggatgattc taagagtctg gtgtgctggc
10441 cctccggcga gggaacgctc agttggccgg acctgctcag tgacccgtcc attgtgggta
10501 gcaatctgcg gcagctggca cggggccagg cgggccatgg gctgggccca gaggaggacg
10561 gcttctccct ggccagcccc tactcgcctg ccaaatcctt ctcagcatca gatgaagacc
10621 tgatccagca ggtccttgcc gagggggtca gcagcccagc ccctacccaa gacacccaca
10681 tggaaacgga cctgctcagc agcctgtcca gcactcctgg ggagaagaca gagacgctgg
10741 cgctgcagag gctgggggag ctggggccac ccagcccagg cctgaactgg gaacagcccc
10801 aggcagcgag gctgtccagg acaggactgg tggagggtct gcggaagcgc ctgctgccgg
10861 cctggtgtgc ctccctggcc cacgggctca gcctgctcct ggtggctgtg gctgtggctg
10921 tctcagggtg ggtgggtgcg agcttccccc cgggcgtgag tgttgcgtgg ctcctgtcca
10981 gcagcgccag cttcctggcc tcattcctcg gctgggagcc actgaaggtc ttgctggaag
11041 ccctgtactt ctcactggtg gccaagcggc tgcacccgga tgaagatgac accctggtag
11101 agagcccggc tgtgacgcct gtgagcgcac gtgtgccccg cgtacggcca ccccacggct
11161 ttgcactctt cctggccaag gaagaagccc gcaaggtcaa gaggctacat ggcatgctgc
11221 ggagcctcct ggtgtacatg cttttcctgc tggtgaccct gctggccagc tatggggatg
11281 cctcatgcca tgggcacgcc taccgtctgc aaagcgccat caagcaggag ctgcacagcc
11341 gggccttcct ggccatcacg cggtctgagg agctctggcc atggatggcc cacgtgctgc
11401 tgccctacgt ccacgggaac cagtccagcc cagagctggg gcccccacgg ctgcggcagg
11461 tgcggctgca ggaagcactc tacccagacc ctcccggccc cagggtccac acgtgctcgg
11521 ccgcaggagg cttcagcacc agcgattacg acgttggctg ggagagtcct cacaatggct
11581 cggggacgtg ggcctattca gcgccggatc tgctgggggc atggtcctgg ggctcctgtg
11641 ccgtgtatga cagcgggggc tacgtgcagg agctgggcct gagcctggag gagagccgcg
11701 accggctgcg cttcctgcag ctgcacaact ggctggacaa caggagccgc gctgtgttcc
11761 tggagctcac gcgctacagc ccggccgtgg ggctgcacgc cgccgtcacg ctgcgcctcg
11821 agttcccggc ggccggccgc gccctggccg ccctcagcgt ccgccccttt gcgctgcgcc
11881 gcctcagcgc gggcctctcg ctgcctctgc tcacctcggt gtgcctgctg ctgttcgccg
11941 tgcacttcgc cgtggccgag gcccgtactt ggcacaggga agggcgctgg cgcgtgctgc
12001 ggctcggagc ctgggcgcgg tggctgctgg tggcgctgac ggcggccacg gcactggtac
12061 gcctcgccca gctgggtgcc gctgaccgcc agtggacccg tttcgtgcgc ggccgcccgc
12121 gccgcttcac tagcttcgac caggtggcgc agctgagctc cgcagcccgt ggcctggcgg
12181 cctcgctgct cttcctgctt ttggtcaagg ctgcccagca gctacgcttc gtgcgccagt
12241 ggtccgtctt tggcaagaca ttatgccgag ctctgccaga gctcctgggg gtcaccttgg
12301 gcctggtggt gctcggggta gcctacgccc agctggccat cctgctcgtg tcttcctgtg
12361 tggactccct ctggagcgtg gcccaggccc tgttggtgct gtgccctggg actgggctct
12421 ctaccctgtg tcctgccgag tcctggcacc tgtcacccct gctgtgtgtg gggctctggg
12481 cactgcggct gtgggcgccc ctacggctgg gggctgttat tctccgctgg cgctaccacg
12541 ccttgcgtgg agagctgtac cggccggcct gggagcccca ggactacgag atggtggagt
12601 tgttcctgcg caggctgcgc ctctggatgg gcctcagcaa ggtcaaggag ttccgccaca
12661 aagtccgctt tgaagggatg gagccgctgc cctctcgctc ctccaggggc tccaaggtat
12721 ccccggatgt gcccccaccc agcgctggct ccgatgcctc gcacccctcc acctcctcca
12781 gccagctgga tgggctgagc gtgagcctgg gccggctggg gacaaggtgt gagcctgagc
12841 cctcccgcct ccaagccgtg ttcgaggccc tgctcaccca gtttgaccga ctcaaccagg
12901 ccacagagga cgtctaccag ctggagcagc agctgcacag cctgcaaggc cgcaggagca
 
12961 gccgggcgcc cgccggatct tcccgtggcc catccccggg cctgcggcca gcactgccca
13021 gccgccttgc ccgggccagt cggggtgtgg acctggccac tggccccagc aggacacccc
13081 ttcgggccaa gaacaaggtc caccccagca gcacttagtc ctccttcctg gcgggggtgg
13141 gccgtggagt cggagtggac accgctcagt attactttct gccgctgtca aggccgaggg
13201 ccaggcagaa tggctgcacg taggttcccc agagagcagg caggggcatc tgtctgtctg
13261 tgggcttcag cactttaaag aggctgtgtg gccaaccagg acccagggtc ccctccccag
13321 ctcccttggg aaggacacag cagtattgga cggtttctag cctctgagat gctaatttat
13381 ttccccgagt cctcaggtac agcgggctgt gcccggcccc accccctggg cagatgtccc
13441 ccactgctaa ggctgctggc ttcagggagg gttagcctgc accgccgcca ccctgcccct
13501 aagttattac ctctccagtt cctaccgtac tccctgcacc gtctcactgt gtgtctcgtg
13561 tcagtaattt atatggtgtt aaaatgtgta tatttttgta tgtcactatt ttcactaggg
13621 ctgaggggcc tgcgcccaga gctggcctcc cccaacacct gctgcgcttg gtaggtgtgg
13681 tggcgttatg gcagcccggc tgctgcttgg atgcgagctt ggccttgggc cggtgctggg
13741 ggcacagctg tctgccaggc actctcatca ccccagaggc cttgtcatcc tcccttgccc
13801 caggccaggt agcaagagag cagcgcccag gcctgctggc atcaggtctg ggcaagtagc
13861 aggactaggc atgtcagagg accccagggt ggttagagga aaagactcct cctgggggct
13921 ggctcccagg gtggaggaag gtgactgtgt gtgtgtgtgt gtgcgcgcgc gcacgcgcga
13981 gtgtgctgta tggcccaggc agcctcaagg ccctcggagc tggctgtgcc tgcttctgtg
14041 taccacttct gtgggcatgg ccgcttctag agcctcgaca cccccccaac ccccgcacca
14101 agcagacaaa gtcaataaaa gagctgtctg actgc
**ALAS1 (SEQ ID NO: 586)** Homo sapiens 5'-aminolevulinate synthase 1 (ALAS1) , transcript variant 1, mRNA.NM 000688 
1 cagaagaagg cagcgcccaa ggcgcatgcg cagcggtcac tcccgctgta tattaaggcg
61 ccggcgatcg cggcctgagg ctgctcccgg acaagggcaa cgagcgtttc gtttggactt
121 ctcgacttga gtgcccgcct ccttcgccgc cgcctctgca gtcctcagcg cagttatgcc
181 cagttcttcc cgctgtgggg acacgaccac ggaggaatcc ttgcttcagg gactcgggac
241 cctgctggac cccttcctcg ggtttagggg atgtggggac caggagaaag tcaggatccc
301 taagagtctt ccctgcctgg atggatgagt ggcttcttct ccacctagat tctttccaca
361 ggagccagca tacttcctga acatggagag tgttgttcgc cgctgcccat tcttatcccg
421 agtcccccag gcctttctgc agaaagcagg caaatctctg ttgttctatg cccaaaactg
481 ccccaagatg atggaagttg gggccaagcc agcccctcgg gcattgtcca ctgcagcagt
541 acactaccaa cagatcaaag aaacccctcc ggccagtgag aaagacaaaa ctgctaaggc
601 caaggtccaa cagactcctg atggatccca gcagagtcca gatggcacac agcttccgtc
661 tggacacccc ttgcctgcca caagccaggg cactgcaagc aaatgccctt tcctggcagc
721 acagatgaat cagagaggca gcagtgtctt ctgcaaagcc agtcttgagc ttcaggagga
781 tgtgcaggaa atgaatgccg tgaggaaaga ggttgctgaa acctcagcag gccccagtgt
841 ggttagtgtg aaaaccgatg gaggggatcc cagtggactg ctgaagaact tccaggacat
901 catgcaaaag caaagaccag aaagagtgtc tcatcttctt caagataact tgccaaaatc
961 tgtttccact tttcagtatg atcgtttctt tgagaaaaaa attgatgaga aaaagaatga
1021 ccacacctat cgagttttta aaactgtgaa ccggcgagca cacatcttcc ccatggcaga
1081 tgactattca gactccctca tcaccaaaaa gcaagtgtca gtctggtgca gtaatgacta
1141 cctaggaatg agtcgccacc cacgggtgtg tggggcagtt atggacactt tgaaacaaca
1201 tggtgctggg gcaggtggta ctagaaatat ttctggaact agtaaattcc atgtggactt
1261 agagcgggag ctggcagacc tccatgggaa agatgccgca ctcttgtttt cctcgtgctt
1321 tgtggccaat gactcaaccc tcttcaccct ggctaagatg atgccaggct gtgagattta
1381 ctctgattct gggaaccatg cctccatgat ccaagggatt cgaaacagcc gagtgccaaa
1441 gtacatcttc cgccacaatg atgtcagcca cctcagagaa ctgctgcaaa gatctgaccc
1501 ctcagtcccc aagattgtgg catttgaaac tgtccattca atggatgggg cggtgtgccc
1561 actggaagag ctgtgtgatg tggcccatga gtttggagca atcaccttcg tggatgaggt
1621 ccacgcagtg gggctttatg gggctcgagg cggagggatt ggggatcggg atggagtcat
1681 gccaaaaatg gacatcattt ctggaacact tggcaaagcc tttggttgtg ttggagggta
1741 catcgccagc acgagttctc tgattgacac cgtacggtcc tatgctgctg gcttcatctt
1801 caccacctct ctgccaccca tgctgctggc tggagccctg gagtctgtgc ggatcctgaa
1861 gagcgctgag ggacgggtgc ttcgccgcca gcaccagcgc aacgtcaaac tcatgagaca
1921 gatgctaatg gatgccggcc tccctgttgt ccactgcccc agccacatca tccctgtgcg
1981 ggttgcagat gctgctaaaa acacagaagt ctgtgatgaa ctaatgagca gacataacat
2041 ctacgtgcaa gcaatcaatt accctacggt gccccgggga gaagagctcc tacggattgc
2101 ccccacccct caccacacac cccagatgat gaactacttc cttgagaatc tgctagtcac
2161 atggaagcaa gtggggctgg aactgaagcc tcattcctca gctgagtgca acttctgcag
2221 gaggccactg cattttgaag tgatgagtga aagagagaag tcctatttct caggcttgag
2281 caagttggta tctgctcagg cctgagcatg acctcaatta tttcacttaa ccccaggcca
2341 ttatcatatc cagatggtct tcagagttgt ctttatatgt gaattaagtt atattaaatt
2401 ttaatctata gtaaaaacat agtcctggaa ataaattctt gcttaaatgg tgaaaaaa
**ALAS1 (SEQ ID NO: 587)** Homo sapiens 5'-aminolevulinate synthase 1 (ALAS1) , transcript Variant 2, mRNA. NM_199166 
1 cagaagaagg cagcgcccaa ggcgcatgcg cagcggtcac tcccgctgta tattaaggcg
61 ccggcgatcg cggcctgagg ctgctcccgg acaagggcaa cgagcgtttc gtttggactt
121 ctcgacttga gtgcccgcct ccttcgccgc cgcctctgca gtcctcagcg cagtctttcc
181 acaggagcca gcatacttcc tgaacatgga gagtgttgtt cgccgctgcc cattcttatc
241 ccgagtcccc caggcctttc tgcagaaagc aggcaaatct ctgttgttct atgcccaaaa
301 ctgccccaag atgatggaag ttggggccaa gccagcccct cgggcattgt ccactgcagc
361 agtacactac caacagatca aagaaacccc tccggccagt gagaaagaca aaactgctaa
421 ggccaaggtc caacagactc ctgatggatc ccagcagagt ccagatggca cacagcttcc
481 gtctggacac cccttgcctg ccacaagcca gggcactgca agcaaatgcc ctttcctggc
541 agcacagatg aatcagagag gcagcagtgt cttctgcaaa gccagtcttg agcttcagga
601 ggatgtgcag gaaatgaatg ccgtgaggaa agaggttgct gaaacctcag caggccccag
661 tgtggttagt gtgaaaaccg atggagggga tcccagtgga ctgctgaaga acttccagga
721 catcatgcaa aagcaaagac cagaaagagt gtctcatctt cttcaagata acttgccaaa
781 atctgtttcc acttttcagt atgatcgttt ctttgagaaa aaaattgatg agaaaaagaa
841 tgaccacacc tatcgagttt ttaaaactgt gaaccggcga gcacacatct tccccatggc
901 agatgactat tcagactccc tcatcaccaa aaagcaagtg tcagtctggt gcagtaatga
961 ctacctagga atgagtcgcc acccacgggt gtgtggggca gttatggaca ctttgaaaca
1021 acatggtgct ggggcaggtg gtactagaaa tatttctgga actagtaaat tccatgtgga
1081 cttagagcgg gagctggcag acctccatgg gaaagatgcc gcactcttgt tttcctcgtg
1141 ctttgtggcc aatgactcaa ccctcttcac cctggctaag atgatgccag gctgtgagat
1201 ttactctgat tctgggaacc atgcctccat gatccaaggg attcgaaaca gccgagtgcc
1261 aaagtacatc ttccgccaca atgatgtcag ccacctcaga gaactgctgc aaagatctga
1321 cccctcagtc cccaagattg tggcatttga aactgtccat tcaatggatg gggcggtgtg
1381 cccactggaa gagctgtgtg atgtggccca tgagtttgga gcaatcacct tcgtggatga
1441 ggtccacgca gtggggcttt atggggctcg aggcggaggg attggggatc gggatggagt
1501 catgccaaaa atggacatca tttctggaac acttggcaaa gcctttggtt gtgttggagg
1561 gtacatcgcc agcacgagtt ctctgattga caccgtacgg tcctatgctg ctggcttcat
1621 cttcaccacc tctctgccac ccatgctgct ggctggagcc ctggagtctg tgcggatcct
1681 gaagagcgct gagggacggg tgcttcgccg ccagcaccag cgcaacgtca aactcatgag
1741 acagatgcta atggatgccg gcctccctgt tgtccactgc cccagccaca tcatccctgt
1801 gcgggttgca gatgctgcta aaaacacaga agtctgtgat gaactaatga gcagacataa
1861 catctacgtg caagcaatca attaccctac ggtgccccgg ggagaagagc tcctacggat
1921 tgcccccacc cctcaccaca caccccagat gatgaactac ttccttgaga atctgctagt
1981 cacatggaag caagtggggc tggaactgaa gcctcattcc tcagctgagt gcaacttctg
2041 caggaggcca ctgcattttg aagtgatgag tgaaagagag aagtcctatt tctcaggctt
2101 gagcaagttg gtatctgctc aggcctgagc atgacctcaa ttatttcact taaccccagg
2161 ccattatcat atccagatgg tcttcagagt tgtctttata tgtgaattaa gttatattaa
2221 attttaatct atagtaaaaa catagtcctg gaaataaatt cttgcttaaa tggtgaaaaa
2281 a
**GTF2D1 (SEQ ID NO: 588)** Homo sapiens TATA-box binding protein (TBP) , transcript variant 1,NM_003194 
1 ggcggaagtg acattatcaa cgcgcgccag gggttcagtg aggtcgggca ggttcgctgt
61 ggcgggcgcc tgggccgccg gctgtttaac ttcgcttccg ctggcccata gtgatctttg
121 cagtgaccca gcatcactgt ttcttggcgt gtgaagataa cccaaggaat tgaggaagtt
181 gctgagaaga gtgtgctgga gatgctctag gaaaaaattg aatagtgaga cgagttccag
241 cgcaagggtt tctggtttgc caagaagaaa gtgaacatca tggatcagaa caacagcctg
301 ccaccttacg ctcagggctt ggcctcccct cagggtgcca tgactcccgg aatccctatc
361 tttagtccaa tgatgcctta tggcactgga ctgaccccac agcctattca gaacaccaat
421 agtctgtcta ttttggaaga gcaacaaagg cagcagcagc aacaacaaca gcagcagcag
481 cagcagcagc agcaacagca acagcagcag cagcagcagc agcagcagca gcagcagcag
541 cagcagcagc agcagcagca acaggcagtg gcagctgcag ccgttcagca gtcaacgtcc
601 cagcaggcaa cacagggaac ctcaggccag gcaccacagc tcttccactc acagactctc
661 acaactgcac ccttgccggg caccactcca ctgtatccct cccccatgac tcccatgacc
721 cccatcactc ctgccacgcc agcttcggag agttctggga ttgtaccgca gctgcaaaat
781 attgtatcca cagtgaatct tggttgtaaa cttgacctaa agaccattgc acttcgtgcc
841 cgaaacgccg aatataatcc caagcggttt gctgcggtaa tcatgaggat aagagagcca
901 cgaaccacgg cactgatttt cagttctggg aaaatggtgt gcacaggagc caagagtgaa
961 gaacagtcca gactggcagc aagaaaatat gctagagttg tacagaagtt gggttttcca
1021 gctaagttct tggacttcaa gattcagaat atggtgggga gctgtgatgt gaagtttcct
1081 ataaggttag aaggccttgt gctcacccac caacaattta gtagttatga gccagagtta
1141 tttcctggtt taatctacag aatgatcaaa cccagaattg ttctccttat ttttgtttct
1201 ggaaaagttg tattaacagg tgctaaagtc agagcagaaa tttatgaagc atttgaaaac
1261 atctacccta ttctaaaggg attcaggaag acgacgtaat ggctctcatg taccettgcc
1321 tcccccaccc ccttcttttt ttttttttaa acaaatcagt ttgttttggt acctttaaat
 
1381 ggtggtgttg tgagaagatg gatgttgagt tgcagggtgt ggcaccaggt gatgcccttc
1441 tgtaagtgcc caccgcggga tgccgggaag gggcattatt tgtgcactga gaacaccgcg
1501 cagcgtgact gtgagttgct cataccgtgc tgctatctgg gcagcgctgc ccatttattt
1561 atatgtagat tttaaacact gctgttgaca agttggtttg agggagaaaa ctttaagtgt
1621 taaagccacc tctataattg attggacttt ttaattttaa tgtttttccc catgaaccac
1681 agtttttata tttctaccag aaaagtaaaa atctttttta aaagtgttgt tttctaatt
1741 tataactcct aggggttatt tctgtgccag acacattcca cctctccagt attgcaggac
1801 agaatatatg tgttaatgaa aatgaatggc tgtacatatt tttttctttc ttcagagtac
1861 tctgtacaat aaatgcagtt tataaaagtg ttagattgtt gttaaaaaaa aaaaaaaaaa
1921 a
**HMBS (SEQ ID NO: 589)** Homo sapiens hydroxymethylbilane synthase (HMBS) , transcript variant 1. NM_000190

1 ccggaagtga cgcgaggctc tgcggagacc aggagtcaga ctgtaggacg acctcgggtc
61 ccacgtgtcc ccggtactcg ccggccggag cccccggctt cccggggccg ggggacctta
121 gcggcaccca cacacagcct actttccaag cggagccatg tctggtaacg gcaatgcggc
181 tgcaacggcg gaagaaaaca gcccaaagat gagagtgatt cgcgtgggta cccgcaagag
241 ccagcttgct cgcatacaga cggacagtgt ggtggcaaca ttgaaagcct cgtaccctgg
301 cctgcagttt gaaatcattg ctatgtccac cacaggggac aagattcttg atactgcact
361 ctctaagatt ggagagaaaa gcctgtttac caaggagctt gaacatgccc tggagaagaa
421 tgaagtggac ctggttgttc actccttgaa ggacctgccc actgtgcttc ctcctggctt
481 caccatcgga gccatctgca agcgggaaaa ccctcatgat gctgttgtct ttcacccaaa
541 atttgttggg aagaccctag aaaccctgcc agagaagagt gtggtgggaa ccagctccct
601 gcgaagagca gcccagctgc agagaaagtt cccgcatctg gagttcagga gtattcgggg
661 aaacctcaac acccggcttc ggaagctgga cgagcagcag gagttcagtg ccatcatcct
721 ggcaacagct ggcctgcagc gcatgggctg gcacaaccgg gtggggcaga tcctgcaccc
781 tgaggaatgc atgtatgctg tgggccaggg ggccttgggc gtggaagtgc gagccaagga
841 ccaggacatc ttggatctgg tgggtgtgct gcacgatccc gagactctgc ttcgctgcat
901 cgctgaaagg gccttcctga ggcacctgga aggaggctgc agtgtgccag tagccgtgca
961 tacagctatg aaggatgggc aactgtacct gactggagga gtctggagtc tagacggctc
1021 agatagcata caagagacca tgcaggctac catccatgtc cctgcccagc atgaagatgg
1081 ccctgaggat gacccacagt tggtaggcat cactgctcgt aacattccac gagggcccca
1141 gttggctgcc cagaacttgg gcatcagcct ggccaacttg ttgctgagca aaggagccaa
1201 aaacatcctg gatgttgcac ggcagcttaa cgatgcccat taactggttt gtggggcaca
1261 gatgcctggg ttgctgctgt ccagtgccta catcccgggc ctcagtgccc cattctcact
1321 gctatctggg gagtgattac cccgggagac tgaactgcag ggttcaagcc ttccagggat
1381 ttgcctcacc ttggggcctt gatgactgcc ttgcctcctc agtatgtggg ggcttcatct
1441 ctttagagaa gtccaagcaa cagcctttga atgtaaccaa tcctactaat aaaccagttc
1501 tgaaggtgta aaaaaaaaaa aaaaaa

### CLAUSES

1. A method for distinguishing between sepsis and systemic inflammatory response syndrome (SIRS) in a patient, comprising:
   (i) determining the amount of one or more biomarker for sepsis, and one or more biomarker for SIRS in a sample obtained from a patient, wherein the one or more biomarker for sepsis is selected from the group consisting of: ITGB3, ITGA2B, MYL9, LCN2, TREML1, LCN15, CMTM5, PPBP and PF4; and the one or more biomarker for SIRS is selected from the group consisting of: PLA2G7, ARHGEF10L, MYCL, TGFBI, and GPR124,
   (ii) comparing the amount of the one or more biomarker for sepsis determined in said sample in (i) to a corresponding reference value representative of a healthy individual,
   (iii) comparing the amount of the one or more biomarker for SIRS determined in said sample in (i) to a corresponding reference value representative of a healthy individual;

   wherein the patient is diagnosed as having sepsis, when an increase is observed in the one or more biomarker for sepsis, and no increase is observed in the one or more biomarker for SIRS, in the sample obtained from the patient relative to the corresponding reference value; and
   wherein the patient is diagnosed as having SIRS, when an increase is observed in the one or more biomarker for SIRS, and no increase is observed in the one or more biomarker for sepsis, in the sample obtained from the patient relative to the corresponding reference value.
2. The method according to Clause 1, wherein the patient has been diagnosed as having a systemic inflammatory condition.
3. A method for distinguishing between sepsis and systemic inflammatory response syndrome (SIRS) in a patient diagnosed as having a systemic inflammatory condition, comprising:
   (i) determining the amount of one or more biomarker for sepsis in a sample obtained from a patient, wherein the one or more biomarker for sepsis is selected from the group consisting of: ITGB3, ITGA2B, MYL9, LCN2, TREML1, LCN15, CMTM5, PPBP, and PF4,
   (ii) comparing the amount of the one or more biomarker for sepsis determined in said sample in (i) to a corresponding reference value representative of a healthy individual;
      wherein the patient is diagnosed as having sepsis, when an increase is observed in the one or more biomarker for sepsis in the sample obtained from the patient relative to the corresponding reference value; and
      wherein the patient is diagnosed as having SIRS, when no increase is observed in the one or more biomarker for sepsis, in the sample obtained from the patient relative to the corresponding reference value.
4. A method for distinguishing between sepsis and systemic inflammatory response syndrome (SIRS) in a patient diagnosed as having a systemic inflammatory condition, comprising:
   (i) determining the amount of one or more biomarker for SIRS in a sample obtained from a patient, wherein the one or more biomarker for SIRS is selected from the group consisting of: PLA2G7, ARHGEF10L, MYCL, TGFBI, and GPR124,
   (ii) comparing the amount of the one or more biomarker for SIRS determined in said sample in (i) to a corresponding reference value representative of a healthy individual;
      wherein the patient is diagnosed as having SIRS, when an increase is observed in the one or more biomarker for SIRS in the sample obtained from the patient relative to the corresponding reference value; and
      wherein the patient is diagnosed as having sepsis, when no increase is observed in the one or more biomarker for SIRS, in the sample obtained from the patient relative to the corresponding reference value.
5. The method according to any one of Clauses 1, 2 and 3, wherein the one or more biomarker for sepsis is selected from the group consisting of: ITGB3, ITGA2B, MYL9, LCN2, and TREML1.
6. The method according to Clause 5, wherein the one or more biomarker for sepsis comprises the combination of: ITGB3, ITGA2B, MYL9, LCN2, and TREML1.
7. The method according to any one of Clauses 1, 2 and 4, wherein the one or more biomarker for SIRS is selected from the group consisting of: PLA2G7, ARHGEF10L, MYCL, and TGFBI.
8. The method according to Clause 7, wherein the one or more biomarker for SIRS comprises the combination of: PLA2G7, ARHGEF10L, MYCL, and TGFBI.
9. The method according to any one of Clauses 1-8, wherein the patient has been diagnosed as having a systemic inflammatory condition using a method comprising:
   (i) determining the amount of one or more inflammation biomarker in a sample obtained from a patient, wherein the one or more inflammation biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1;
   (ii) comparing the amount of the one or more inflammation biomarker determined in said sample in (i) to a corresponding reference value; and thereby determining whether the patient has a systemic inflammatory condition.
10. The method according to Clause 9, wherein the reference value used in the method for diagnosing whether the patient has a systemic inflammatory condition, is representative of a healthy individual having no known systemic inflammatory condition.
11. The method according to Clause 10, wherein the patient is diagnosed as having a systemic inflammatory condition, when an increase is observed in the one or more inflammation biomarker in the sample obtained from the patient relative to the corresponding reference value.
12. The method according to any one of Clauses 9-11, wherein the one or more inflammation biomarker is selected from the group consisting of: FAM20A, OLAH and CD177.
13. The method according to Clause 12, wherein the one or more inflammation biomarker comprises the combination of FAM20A and OLAH, and optionally CD177.
14. A method for diagnosing whether a patient has a systemic inflammatory condition, comprising:
   (i) determining the amount of FAM20A and OLAH in a sample obtained from a patient,
   (ii) comparing the amount of FAM20A determined in said sample in (i) to a corresponding reference value representative of a healthy individual,
   (iii) comparing the amount of OLAH determined in said sample in (i) to a corresponding reference value representative of a healthy individual;
      wherein the patient is diagnosed as having a systemic inflammatory condition, when an increase is observed in FAM20A and/or OLAH in the sample obtained from the patient relative to the corresponding reference value; and
      wherein the patient is diagnosed as not having a systemic inflammatory condition, when no increase is observed in FAM20A and OLAH, in the sample obtained from the patient relative to the corresponding reference value.
15. A method for diagnosing whether a patient has abdominal sepsis, comprising:
   (i) determining the amount of one or more abdominal sepsis biomarker in a sample obtained from a patient, wherein the one or more biomarker is selected from the group consisting of: SLC39A8, CIQC, CIQA, MRAS, TMEM37, CIQB, PCOLCE2, KIF2C, TNF, IFI44, IFIT1, and RPGRIP1,
   (ii) comparing the amount of the one or more abdominal sepsis biomarker determined in said sample in (i) to a corresponding reference value; and thereby determining whether the patient has abdominal sepsis.
16. The method according to Clause 15, wherein the one or more abdominal sepsis biomarker is selected from the group consisting of: SLC39A8, CIQC, CIQA, MRAS, TMEM37, and CIQB.
17. The method according to Clause 16, wherein the one or more abdominal sepsis biomarker is selected from the group consisting of: SLC39A8, CIQC and CIQA.
18. A method for diagnosing whether a patient has pulmonary sepsis, comprising:
   (i) determining the amount of one or more pulmonary sepsis biomarker in a sample obtained from a patient, wherein the one or more biomarker is selected from the group consisting of: HCAR2, CXCR1, DISC1, EPSTI1, and IFI44,
   (ii) comparing the amount of the one or more pulmonary sepsis biomarker determined in said sample in (i) to a corresponding reference value; and thereby determining whether the patient has pulmonary sepsis.
19. The method according to Clause 18, wherein the one or more pulmonary sepsis biomarker is selected from the group consisting of: HCAR2, CXCR1, and DISC1, optionally wherein the one or more pulmonary sepsis biomarker comprises HCAR2 and/or CXCR1.
20. The method according to any one of Clauses 15-19, wherein the reference value comprises one or more value selected from: (i) a reference value that is representative of a healthy individual, (ii) a reference value that is representative of an individual having pulmonary sepsis, (iii) a reference value that is representative of an individual having abdominal sepsis, and (iv) a reference value that is representative of an individual having SIRS.
21. The method according to any one of Clauses 15-20, wherein the patient has been diagnosed as having a systemic inflammatory condition using the method of Clause 14.
22. The method according to Clause 21, wherein the patient has been diagnosed as having sepsis, optionally wherein the patient has been diagnosed as having sepsis using the method of any one of Clauses 1-13.
23. A method for monitoring a systemic inflammatory condition in a patient, comprising:
   (i) determining the amount of one or more biomarker in a sample obtained from a patient at a first time point;
   (ii) determining the amount of the one or more biomarker in a sample obtained from the patient at one or more later time points;
   (iii) comparing the amount of the one or more biomarker determined in step (ii) to the amount of the one or more biomarker determined in step (i);
   wherein the one or more biomarker is selected from the group consisting of: ITM2A, CCL5, NPPC, PKD1, KLRK1, KLRB1, HLA-DRA, BCL11B, HLA-DPB1, FCER1A, DAAM2, SLAMF6, CD160, KLRF1, CD2, LGALS2, MYCL, MX1, NECAB1, PKHD1, and LILRB5.
24. The method according to Clause 23, wherein the systemic inflammatory condition is abdominal sepsis, and wherein the one or more biomarker is selected from the group consisting of: ITM2A, CCL5, NPPC, KLRK1, KLRB1, HLA-DRA, BCL11B, HLA-DPB1, SLAMF6, CD160, KLRF1, and MX1.
25. The method according to Clause 23, wherein the systemic inflammatory condition is SIRS, and wherein the one or more biomarker is selected from the group consisting of: CCL5, NPPC, PKD1, LGALS2, MYCL, NECAB1, and PKHD1.
26. The method according to any one of Clauses 23-25, wherein an increase in any one or more of ITM2A, CCL5, KLRK1, KLRB1, HLA-DRA, BCL11B, HLA-DPB1, SLAMF6, CD160, KLRF1, and MX1 in the sample obtained at the later time point relative to the sample obtained from the first time point indicates regression of the systemic inflammatory condition in the patient, the success of one or more treatment regimens and/or a prognosis of recovery.
27. The method according to any one of Clauses 23-25, wherein an increase in any one or more of NPPC, PKDI, LGALS2, MYCL, NECAB1, and PKHD1 in the sample obtained at the later time period relative to the sample obtained from the first time period indicates progression of the systemic inflammatory condition in the patient, the lack of efficacy of one or more treatment regimens and/or a prognosis of non-recovery.
28. The method of any one of Clauses 23-27, wherein the patient has been diagnosed as having a systemic inflammatory condition using the method of Clause14.
29. The method according to any one of Clauses 24 and 26-28, wherein the patient has been diagnosed as having abdominal sepsis, optionally wherein the patient has been diagnosed as having abdominal sepsis using the method according to any one of Clauses 15-17 and 20-22.
30. The method according to any one of Clauses 25-28, wherein the patient has been diagnosed as having SIRS, optionally wherein the patient has been diagnosed as having SIRS using the method according to any of Clauses 1-13.
31. A method for determining whether a patient having a systemic inflammatory condition is suitable for discharge from medical care, comprising:
   (i) determining the amount of one or more biomarker selected from the group consisting of: NECAB1, NECAB2, PKD1, PKHD1, LILRB4, and LILRB5 in a sample obtained from a patient,
   (ii) comparing the amount of the one or more biomarker determined in said sample in (i) to a corresponding reference value; and thereby determining whether the patient is suitable for discharge from medical care.
32. The method according to Clause 31, wherein the patient has been diagnosed as having a systemic inflammatory condition using the method of Clause 14.
33. The method according to Clause 31 or 32, wherein the patient is undergoing or has undergone treatment for SIRS, and wherein the one or more biomarker is selected from the group consisting of: NECAB1, PKDI, PKHD1, LILRB4, and LILRB5.
34. The method according to Clause 33, wherein the one or more biomarker is selected from the group consisting of: NECAB1 and PKHD1.
35. The method according to Clause 31 or 32, wherein the patient is undergoing or has undergone treatment for sepsis, and wherein the one or more biomarker is selected from the group consisting of NECAB2, PKD1, PKHD1 and LILRB5.
36. The method according to Clause 35, wherein the patient is undergoing or has undergone treatment for abdominal sepsis, and wherein the one or more biomarker comprises NECAB2 and/or PKD1.
37. The method according to Clause 35, wherein the patient is undergoing or has undergone treatment for pulmonary sepsis, and wherein the one or more biomarker comprises PKHD1 and/or LILRB5.
38. The method according to any one of Clauses 31-37, wherein the reference value comprises one or more value selected from: (i) a reference value that is representative of a healthy individual, (ii) a reference value that is representative of an individual having a prognosis of recovery from a systemic inflammatory condition, and (iii) a reference value that is representative of an individual having a prognosis of non-recovery from a systemic inflammatory condition.
39. The method according to any one of Clauses 1-38, wherein the sample is a sample of blood, cerebral spinal fluid, cells, a cellular extract, a tissue specimen, or a tissue biopsy, or a combination thereof.
40. The method according to Clause 39, wherein the blood sample is a sample of whole blood, purified peripheral blood leukocytes or cell type sorted leukocytes.
41. The method according to any one of Clauses 1-40, wherein the one or more biomarker is a protein biomarker.
42. The method according to Clause 41, wherein the amount of the one or more biomarker is determined using an antibody specific for said one or more biomarker.
43. The method according to any one of Clauses 1-42, wherein the one or more biomarker is a nucleic acid biomarker.
44. The method according to Clause 43, wherein the amount of the one or more biomarker is determined using an oligonucleotide specific for said one or more biomarker.
45. A device for carrying out the method of any one of Clauses 1-44, wherein the device comprises one or more binding agent specific for the one or more biomarker.
46. The device according to Clause 45, wherein the one or more binding agent comprises one or more oligonucleotides specific for the one or more biomarker.
47. The device according to Clause 45 or 46, wherein the one or more binding agent comprises one or more antibodies specific for the one or more biomarker.
48. The device according to any of Clauses 44-47, wherein the one or more binding agent is immobilised on a surface to provide a binding agent array.

## Claims

1. A method for distinguishing between sepsis and systemic inflammatory response syndrome (SIRS) in a patient that has a systemic inflammatory condition, the method comprising:
(i) determining the amount of three or more biomarkers for sepsis in a sample obtained from a patient, wherein said three or more biomarkers comprise:
- ITGB3, and
- at least two biomarkers selected from the group consisting of ITGA2B, MYL9, LCN2, TREML1, LCN15, CMTM5, PPBP and PF4,
(ii) comparing the amount of the three or more biomarkers for sepsis determined in said sample in (i) to a corresponding reference value representative of a healthy individual,
wherein the patient is diagnosed as having sepsis, when an increase is observed in the three or more biomarkers for sepsis in the sample obtained from the patient relative to the corresponding reference value; or
wherein the patient is diagnosed as having SIRS, when no increase is observed in the three or more biomarkers for sepsis in the sample obtained from the patient relative to the corresponding reference value.

2. A method for distinguishing between sepsis and systemic inflammatory response syndrome (SIRS) in a patient that has a systemic inflammatory condition, the method comprising:
(i) determining the amount of three or more biomarkers for sepsis in a sample obtained from a patient, wherein said three or more biomarkers comprise:
- CMTM5, and
- at least two biomarkers selected from the group consisting of ITGB3, ITGA2B, MYL9, LCN2, TREML1, LCN15, PPBP and PF4, and
(ii) comparing the amount of the three or more biomarkers for sepsis determined in said sample in (i) to a corresponding reference value representative of a healthy individual,
wherein the patient is diagnosed as having sepsis, when an increase is observed in the three or more biomarkers for sepsis in the sample obtained from the patient relative to the corresponding reference value; or
wherein the patient is diagnosed as having SIRS, when no increase is observed in the three or more biomarkers for sepsis in the sample obtained from the patient relative to the corresponding reference value.

3. A method for distinguishing between sepsis and systemic inflammatory response syndrome (SIRS) in a patient that has a systemic inflammatory condition, the method comprising:
(i) determining the amount of three or more biomarkers for sepsis in a sample obtained from a patient, wherein said three or more biomarkers comprise:
- PF4, and
- at least two biomarkers selected from the group consisting of ITGB3, ITGA2B, MYL9, LCN2, TREML1, LCN15, CMTM5 and PPBP, and
(ii) comparing the amount of the three or more biomarkers for sepsis determined in said sample in (i) to a corresponding reference value representative of a healthy individual,
wherein the patient is diagnosed as having sepsis, when an increase is observed in the three or more biomarkers for sepsis in the sample obtained from the patient relative to the corresponding reference value; or
wherein the patient is diagnosed as having SIRS, when no increase is observed in the three or more biomarkers for sepsis in the sample obtained from the patient relative to the corresponding reference value.

4. A method for distinguishing between sepsis and systemic inflammatory response syndrome (SIRS) in a patient diagnosed as having a systemic inflammatory condition, comprising:
(i) determining the amount of three or more biomarkers for SIRS in a sample obtained from a patient, wherein the three or more biomarkers for SIRS comprise:
- PLA2G7, and
- at least two biomarkers selected from the group consisting of ARHGEF10L, MYCL, TGFBI and GPR124, and
wherein the patient is diagnosed as having SIRS, when an increase is observed in the three or more biomarkers for SIRS in the sample obtained from the patient relative to the corresponding reference value; or
wherein the patient is diagnosed as having sepsis, when no increase is observed in the three or more biomarkers for SIRS in the sample obtained from the patient relative to the corresponding reference value.
(ii) comparing the amount of the three or more biomarkers for SIRS determined in said sample in (i) to a corresponding reference value representative of a healthy individual;

5. A method for distinguishing between sepsis and systemic inflammatory response syndrome (SIRS) in a patient diagnosed as having a systemic inflammatory condition, comprising:
(i) determining the amount of three or more biomarkers for SIRS in a sample obtained from a patient, wherein the three or more biomarkers for SIRS comprise:
- ARHGEF10L, and
- at least two biomarkers selected from the group consisting of PLA2G7, MYCL, TGFBI, and GPR124, and
(ii) comparing the amount of the three or more biomarkers for SIRS determined in said sample in (i) to a corresponding reference value representative of a healthy individual;
wherein the patient is diagnosed as having SIRS, when an increase is observed in the three or more biomarkers for SIRS in the sample obtained from the patient relative to the corresponding reference value; or
wherein the patient is diagnosed as having sepsis, when no increase is observed in the three or more biomarkers for SIRS in the sample obtained from the patient relative to the corresponding reference value.

6. A method for distinguishing between sepsis and systemic inflammatory response syndrome (SIRS) in a patient diagnosed as having a systemic inflammatory condition, comprising:
(i) determining the amount of three or more biomarkers for SIRS in a sample obtained from a patient, wherein the three or more biomarkers for SIRS comprise:
- GPR124, and
- at least two biomarkers selected from the group consisting of ARHGEF10L, PLA2G7, MYCL, TGFBI, and
(ii) comparing the amount of the three or more biomarkers for SIRS determined in said sample in (i) to a corresponding reference value representative of a healthy individual;
wherein the patient is diagnosed as having SIRS, when an increase is observed in the three or more biomarkers for SIRS in the sample obtained from the patient relative to the corresponding reference value; or
wherein the patient is diagnosed as having sepsis, when no increase is observed in the three or more biomarkers for SIRS, in the sample obtained from the patient relative to the corresponding reference value.

7. The method according to any one of claims 1-3, further comprising
(i) determining the amount of three or more biomarkers for SIRS in a sample obtained from a patient, wherein the three or more biomarkers for SIRS comprise:
- PLA2G7, and at least two biomarkers selected from the group consisting of ARHGEF10L, MYCL, TGFBI, and GPR124; or wherein the three or more biomarkers for SIRS comprise:
- ARHGEF10L, and at least two biomarkers selected from the group consisting of PLA2G7, MYCL, TGFBI, and GPR124; or wherein the three or more biomarkers for SIRS comprise:
- GPR124, and at least two biomarkers selected from the group consisting of ARHGEF10L, PLA2G7, MYCL, and TGFBI; and
(ii) comparing the amount of the three or more biomarkers for SIRS determined in said sample in (i) to a corresponding reference value representative of a healthy individual;
wherein the patient is diagnosed as having sepsis, when an increase is observed in the three or more biomarkers for sepsis, and no increase is observed in the three or more biomarkers for SIRS, in the sample obtained from the patient relative to the corresponding reference value; and
wherein the patient is diagnosed as having SIRS, when an increase is observed in the three or more biomarkers for SIRS, and no increase is observed in the three or more biomarkers for sepsis, in the sample obtained from the patient relative to the corresponding reference value.

8. The method according to any one of claims 4-6, further comprising
(i) determining the amount of three or more biomarkers for sepsis in a sample obtained from a patient, wherein said three or more biomarkers comprise:
- ITGB3, and at least two biomarkers selected from the group consisting of ITGA2B, MYL9, LCN2, TREML1, LCN15, CMTM5, PPBP and PF4, or wherein said three or more biomarkers comprise:
- CMTM5, and at least two biomarkers selected from the group consisting of ITGB3, ITGA2B, MYL9, LCN2, TREML1, LCN15, PPBP and PF4, or wherein said three or more biomarkers comprise:
- PF4, and at least two biomarkers selected from the group consisting of ITGB3, ITGA2B, MYL9, LCN2, TREML1, LCN15, CMTM5, PPBP; and
(ii) comparing the amount of the three or more biomarkers for sepsis determined in said sample in (i) to a corresponding reference value representative of a healthy individual,
wherein the patient is diagnosed as having sepsis, when an increase is observed in the three or more biomarkers for sepsis, and no increase is observed in the three or more biomarkers for SIRS, in the sample obtained from the patient relative to the corresponding reference value; or
wherein the patient is diagnosed as having SIRS, when an increase is observed in the three or more biomarkers for SIRS, and no increase is observed in the three or more biomarkers for sepsis, in the sample obtained from the patient relative to the corresponding reference value.

9. The method according to any one of claims 1 or 7-8, comprising detecting sepsis when:
an increase in ITGB3 is present, and no increase in GPR124 is present; or
an increase in ITGB3 is present, and no increase in PLA2G7 is present; or
an increase in ITGB3 is present, and no increase in ARHGEF10L is present.

10. The method according to any one of claims 2 or 7-8, comprising detecting sepsis when:
an increase in CMTM5 is present, and no increase in PLA2G7 is present; or
an increase in CMTM5 is present, and no increase in GPR124 is present; or
an increase in CMTM5 is present, and no increase in ARHGEF10L is present.

11. The method according to any one of claims 3 or 7-8, comprising detecting sepsis when:
an increase in PF4 is present, and no increase in PLA2G7 is present; or
an increase in PF4 is present, and no increase in GPR124 is present; or
an increase in PF4 is present, and no increase in ARHGEF10L is present.

12. The method according to any one of claims 4 or 7-8, comprising detecting SIRS when:
no increase in ITGB3 is present, and an increase in GPR124 is present; or
no increase in ITGB3 is present, and an increase in PLA2G7 is present; or
no increase in ITGB3 is present, and an increase in ARHGEF10L is present.

13. The method according to any one of claims 5 or 7-8, comprising detecting SIRS when:
no increase in CMTM5 is present, and an increase in PLA2G7 is present; or
no increase in CMTM5 is present, and an increase in GPR124 is present; or
no increase in CMTM5 is present, and an increase in ARHGEF10L is present.

14. The method according to any one of claims 6 or 7-8, comprising detecting SIRS when:
no increase in PF4 is present, and an increase in PLA2G7 is present; or
no increase in PF4 is present, and an increase in GPR124 is present; or
no increase in PF4 is present, and an increase in ARHGEF10L is present.

15. The method according to any one of the preceding claims, wherein the patient has been diagnosed as having a systemic inflammatory condition using a method comprising:
(i) determining the amount of one or more inflammation biomarker in a sample obtained from a patient, wherein the one or more inflammation biomarker is selected from the group consisting of: FAM20A, OLAH, CD177, ADM, IL10, METTL7B, MMP9, RETN, TDRD9, ITGA7, BMX, HP, IGFBP2, ALPL, DACH1, IL1R1, IL1R2, CYP19A1, MMP8, TGFA and VSTM1;
(ii) comparing the amount of the one or more inflammation biomarker determined in said sample in (i) to a corresponding reference value representative of a healthy individual; and
(iii) diagnosing a systemic inflammatory condition when an increase is observed in the one or more inflammation biomarker in the sample obtained from the patient relative to the corresponding reference value;
preferably wherein the one or more inflammation biomarker is selected from the group consisting of: FAM20A, and OLAH;
more preferably wherein the one or more inflammation biomarker is OLAH.
